(19) **Europäisches Patentamt / European Patent Office / Office européen des brevets**

(11) **EP 2 279 746 B1**

(12) **EUROPEAN PATENT SPECIFICATION**

(45) Date of publication and mention
of the grant of the patent:
**02.10.2013 Bulletin 2013/40**

(21) Application number: **10004682.0**

(22) Date of filing: **17.11.2003**

(51) Int Cl.:
*A61K 39/095* (2006.01)  *C07K 14/22* (2006.01)

(54) **SURFACE PROTEINS IN NEISSERIA MENINGITIDIS**

OBERFLÄCHENPROTEINE IN NEISSERIA MENINGITIDIS

PROTEINES DE SURFACE DE NEISSERIA MENINGITIDIS

(84) Designated Contracting States:
**AT BE BG CH CY CZ DE DK EE ES FI FR GB GR
HU IE IT LI LU MC NL PT RO SE SI SK TR**

(30) Priority: **15.11.2002 GB 0226734
27.03.2003 GB 0307131**

(43) Date of publication of application:
**02.02.2011 Bulletin 2011/05**

(62) Document number(s) of the earlier application(s) in
accordance with Art. 76 EPC:
**03780503.3 / 1 562 982**

(73) Proprietor: **Novartis Vaccines and Diagnostics
S.r.l.
53100 Siena (SI) (IT)**

(72) Inventors:
• **Norais, Nathalie
53100 Siena (IT)**
• **Grandi, Guido
53100 Siena (IT)**

(74) Representative: **Carpmaels & Ransford LLP
One Southampton Row
London WC1B 5HA (GB)**

(56) References cited:
**WO-A-00/25811    WO-A-01/09350
WO-A-01/52885    WO-A2-00/71725**

• CLAASSEN I ET AL: "Production, characterization and control of a Neisseria meningitidis hexavalent class 1 outer membrane protein containing vesicle vaccine", VACCINE, BUTTERWORTH SCIENTIFIC. GUILDFORD, GB, vol. 14, no. 10, 1 July 1996 (1996-07-01), pages 1001-1008, XP004057632, ISSN: 0264-410X -& VAN DER LEY P ET AL: "Construction of Neisseria meningitidis strains carrying multiple chromosomal copies of the porA gene for use in the production of a multivalent outer membrane vesicle vaccine", VACCINE, BUTTERWORTH SCIENTIFIC. GUILDFORD, GB, vol. 13, no. 4, 1995, pages 401-407, XP004057740, ISSN: 0264-410X
• NORAIS NATHALIE ET AL: "Combined automated PCR cloning, in vitro transcription/ translation and two-dimensional electrophoresis for bacterial proteome analysis", PROTEOMICS, vol. 1, no. 11, November 2001 (2001-11), pages 1378-1389, XP009032238, ISSN: 1615-9853
• TETTELIN H ET AL: "COMPLETE GENOME SEQUENCE OF NEISSERIA MENINGITIDIS SEROGROUP B STRAIN MC58", SCIENCE, AMERICAN ASSOCIATION FOR THE ADVANCEMENT OF SCIENCE,, US, vol. 287, 2000, pages 1809-1815, XP000914963, ISSN: 0036-8075 -& DATABASE UniProt [Online] EBI; 1 October 2000 (2000-10-01), TETTELIN ET AL.: "SubName: Full=Putative uncharacterized protein", XP002610937, Database accession no. Q9JY11
• GRIFANTINI R ET AL: "Previously unrecognized vaccine candidates against group B meningococcus identified by DNA microarrays", NATURE BIOTECHNOLOGY, NATURE PUBLISHING, US, vol. 20, no. 9, September 2002 (2002-09), pages 914-921, XP002272872, ISSN: 1087-0156

- PIZZA M ET AL: "IDENTIFICATION OF VACCINE CANDIDATES AGAINST SEROGROUP B MENINGOCOCCUS BY WHOLE-GENOME SEQUENCING", SCIENCE, AMERICAN ASSOCIATION FOR THE ADVANCEMENT OF SCIENCE,, US, vol. 287, no. 5459, 10 March 2000 (2000-03-10), pages 1816-1820, XP000986271, ISSN: 0036-8075
- JOLLEY KEITH A ET AL: "Immunization with recombinant Opc outer membrane protein from Neisseria meningitidis: Influence of sequence variation and levels of expression on the bactericidal immune response against meningococci", INFECTION AND IMMUNITY, vol. 69, no. 6, June 2001 (2001-06), pages 3809-3916, XP002284891, ISSN: 0019-9567
- WRIGHT J CLAIRE ET AL: "Immunization with the recombinant PorB outer membrane protein induces a bactericidal immune response against Neisseria meningitidis", INFECTION AND IMMUNITY, vol. 70, no. 8, August 2002 (2002-08), pages 4028-4034, XP002284892, ISSN: 0019-9567
- POLLARD A J ET AL: "The meningococcus tamed?", ARCHIVES OF DISEASE IN CHILDHOOD. ENGLAND JUL 2002, vol. 87, no. 1, July 2002 (2002-07), pages 13-17, XP002284893, ISSN: 1468-2044
- GIL JEOVANIS ET AL: "Proteomic study via a non-gel based approach of meningococcal outer membrane vesicle vaccine obtained from strain CU385: a road map for discovering new antigens.", HUMAN VACCINES MAY 2009 LNKD-PUBMED:19377283, vol. 5, no. 5, May 2009 (2009-05), pages 347-356, XP009132297, ISSN: 1554-8619

**Description**

**TECHNICAL FIELD**

This invention is in the field of meningococcal biochemistry, in particular the trafficking and localisation of meningococcal proteins.

**BACKGROUND ART**

The complete genome sequence of serogroup B *N.meningitidis* has been published [1] and has been subjected to analysis in order to identify candidate vaccine antigens [2]. The complete genome sequence of serogroup A *N.meningitidis* is also known [3].

Footnote 12 of reference 2 describes the authors' approach to antigen selection. Briefly, a first screening for coding capacity of the genome sequence was performed using computer programs included in the Wisconsin package version 10.0, Genetics Computer Group (GCG). BLASTX was used to classify ORFs as coding either for known cytoplasmic functions or for other functions. The cytoplasmic ORFs were "not further investigated" as candidate antigens.

A second screening step aimed at identifying putative proteins with a cellular localization spanning from the inner membrane to outside the bacterium was also used. This screening involved F-BLAST, FASTA, MOTIFS, FINDPATTERNS, and PSORT, as well as the ProDom, Pfam, and Blocks databases. ORFs were thus selected on the basis of features typical of surface-associated proteins such as transmembrane domains, leader peptides, homologies to known surface proteins, lipoprotein signature, outer membrane anchoring motifs, and host cell binding domains such as RGD.

In total, reference 2 identified 570 ORFs within the genome as candidate vaccine antigens. 98.8% of these selected serogroup B ORFs were also found and conserved in serogroup A, and 95.3% were found and conserved in *N. gonorrhoeae.*

This "reverse genomics" approach to vaccine candidate selection is undoubtedly powerful, and has been copied for other organisms [*e.g.* refs. 4 to 10], but it does not identify all surface-exposed proteins and vaccine candidates [11]. It is therefore an object of the invention to identify surface-exposed meningococcal proteins which are not identified by computer prediction methods.

Claassen et al., 1996, Vaccine, vol. 14(10), pp. 1001-1008 describes a vesicle vaccine containing a Neisseria meningitidis hexavalent class 1 outer membrane protein.

Norais et al., 2001, Proteomics, vol. 1, pp. 1378-1389 describes mass spectrometry of Neisseria meningitidis membrane preparations on 2D gels followed by sequence analysis and expression in E. coli.

Wright et al., 2002, Infections and Immunity, vol. 70(8), pp. 4028-4034 discloses immunization with recombinant PorB outer membrane protein which induces a bacterial immune response against Neisseria meningitidis.

Jolley et al., 2001, Infection and Immunity, vol. 69(6), pp. 3809-3816 describes immunization with recombinant Opc outer membrane protein from Neisseria meningitidis.

WO00/71725 discloses compositions comprising a first biological molecule from Neisseria bacterium and a second biological molecule from Neisseria bacterium.

**DISCLOSURE OF THE INVENTION**

The invention is based on the discovery of 217 proteins which, contrary to expectations, are found in the membrane of *Neisseria meningitidis.* Of these 217, 76 in particular evade all algorithmic methods for predicting membrane localisation. Existing knowledge of protein trafficking pathways in meningococcus does not explain how or why these proteins are located in the bacterial membrane *e.g.* there is no apparent biochemical reason for a DNA helicase or a chromosomal replication initiator protein to be found in the membrane.

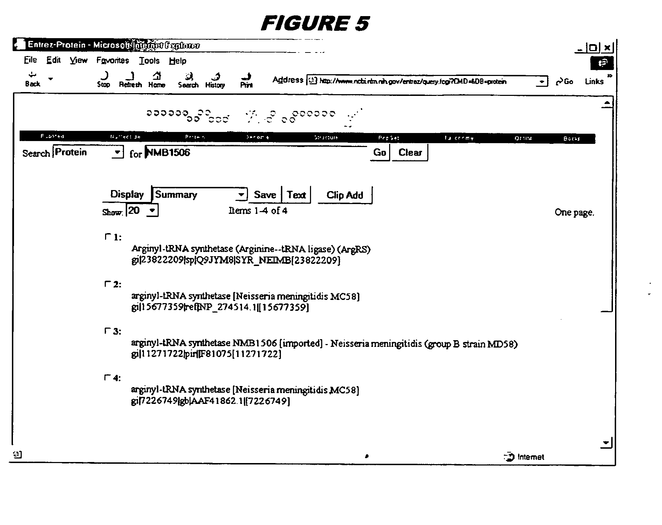

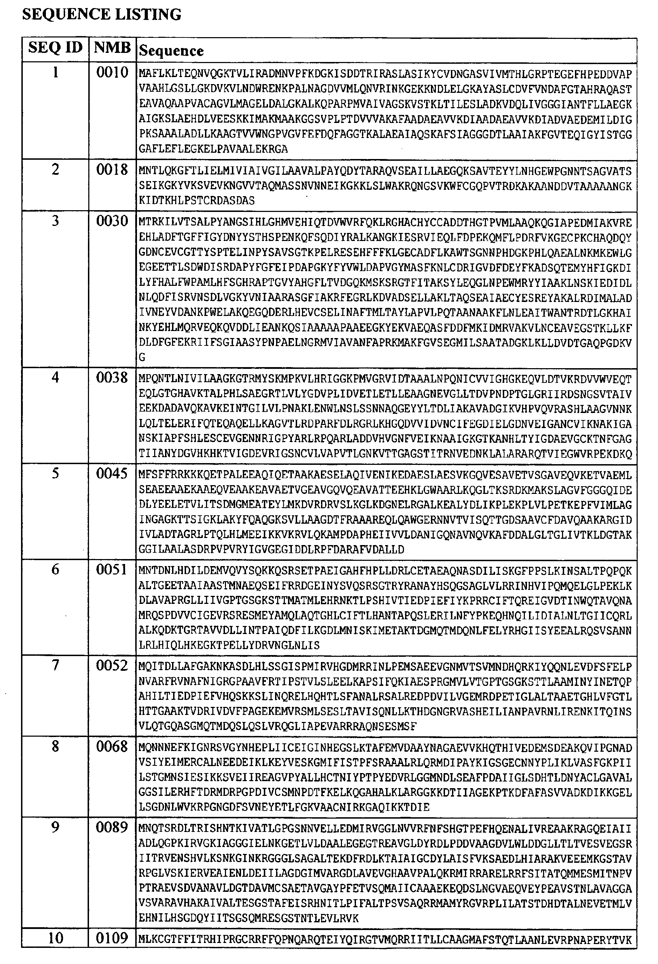

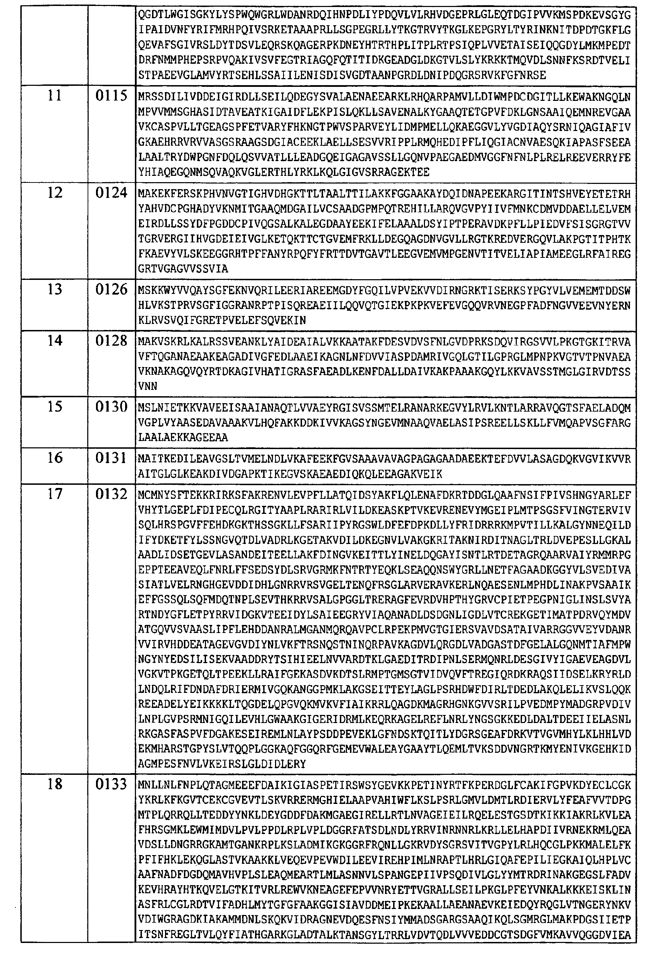

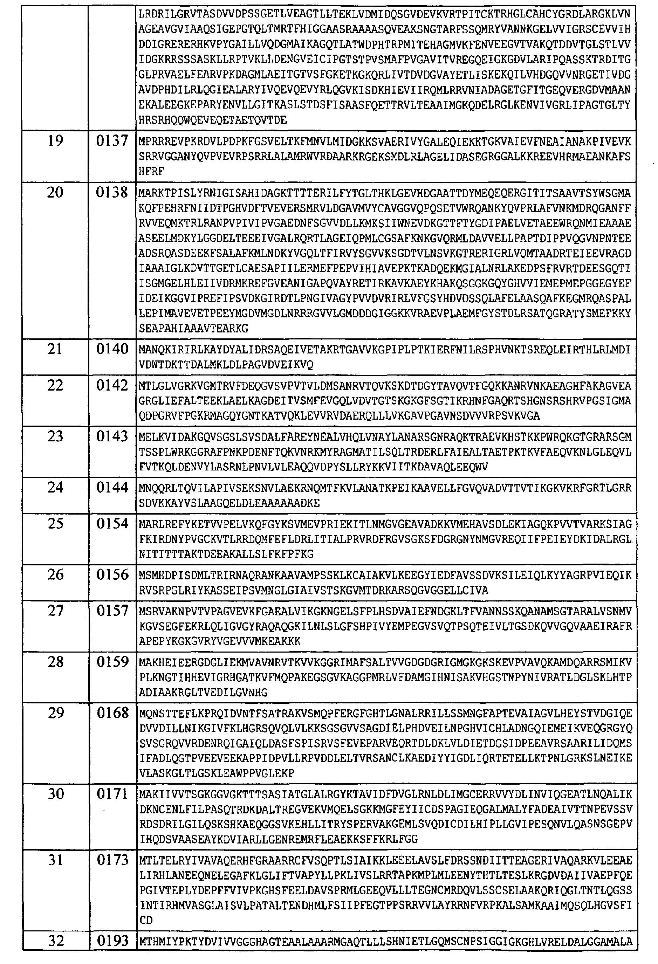

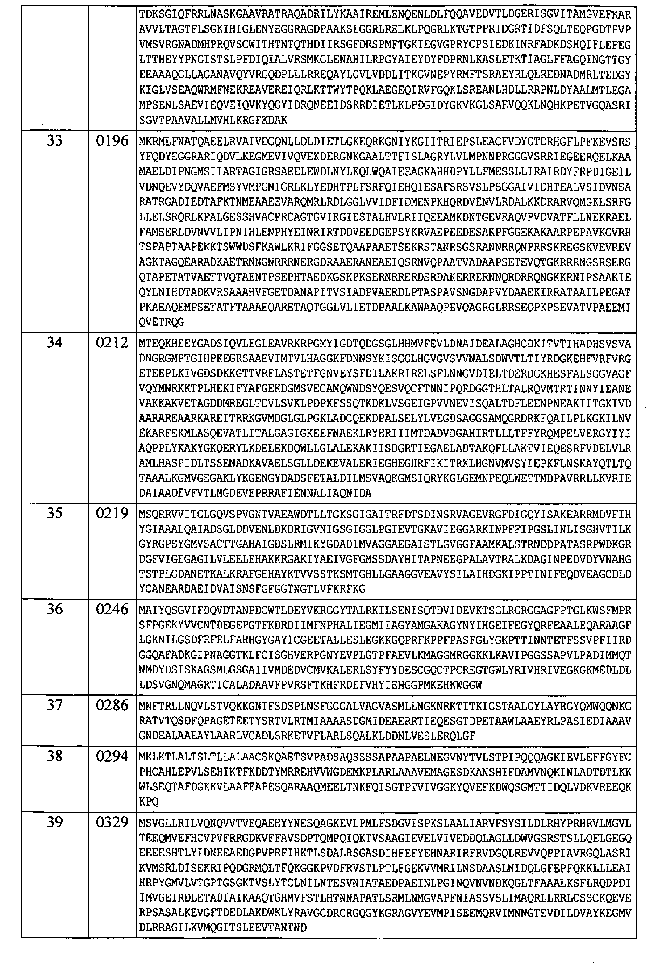

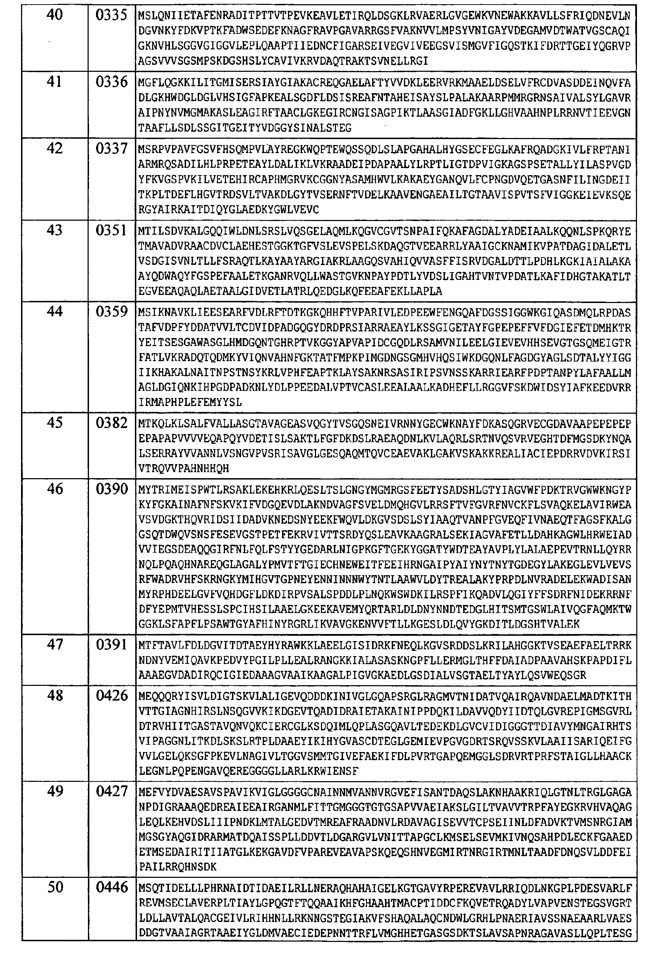

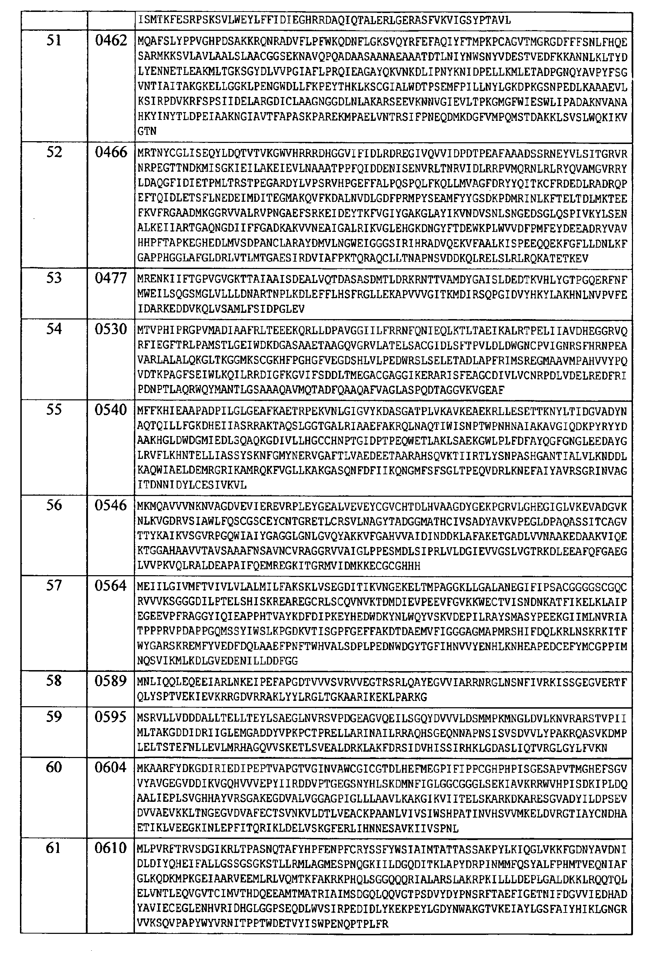

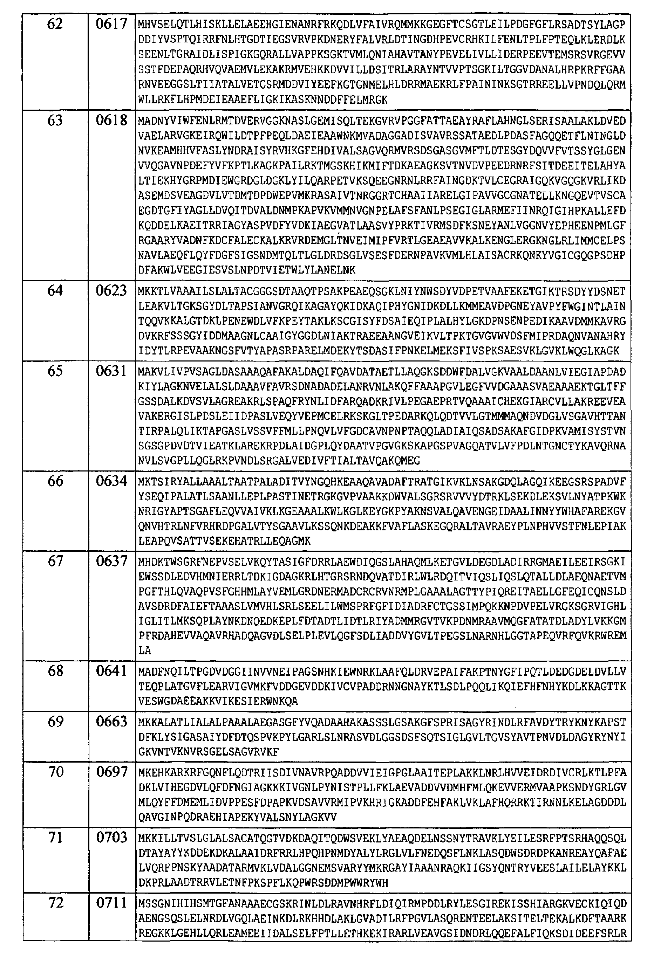

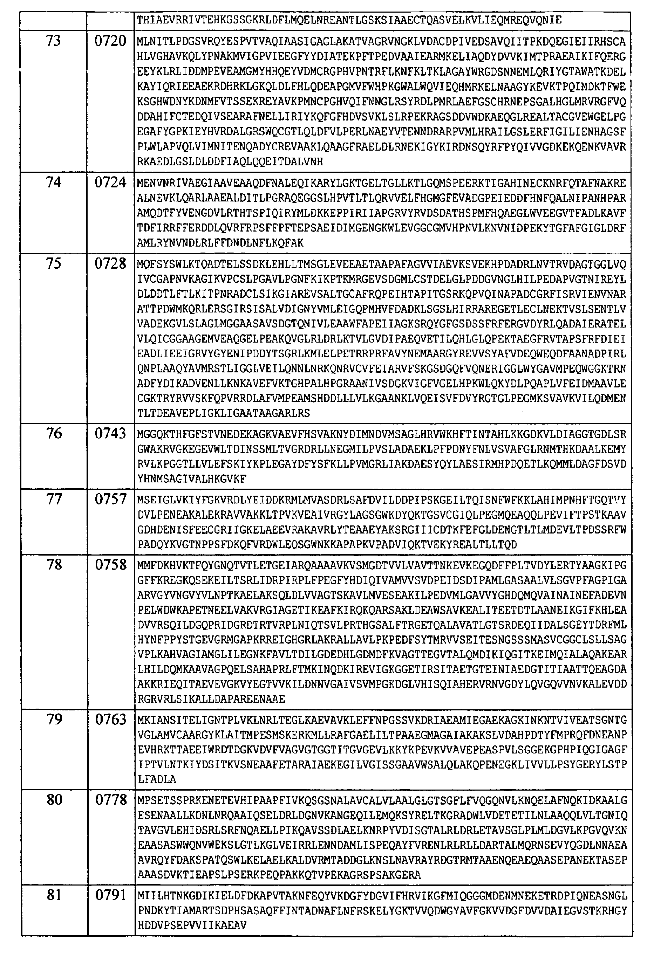

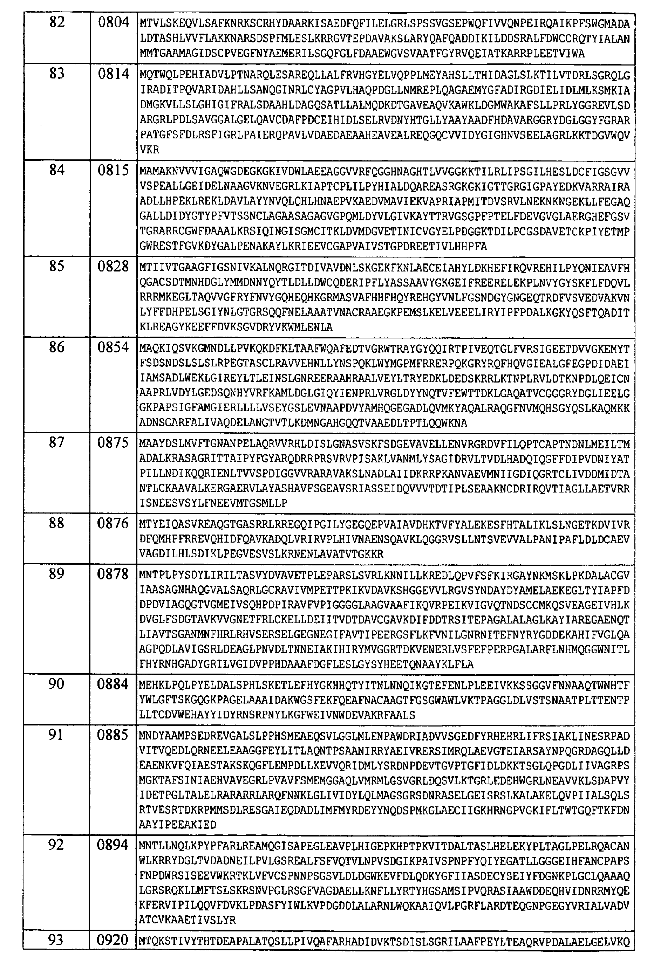

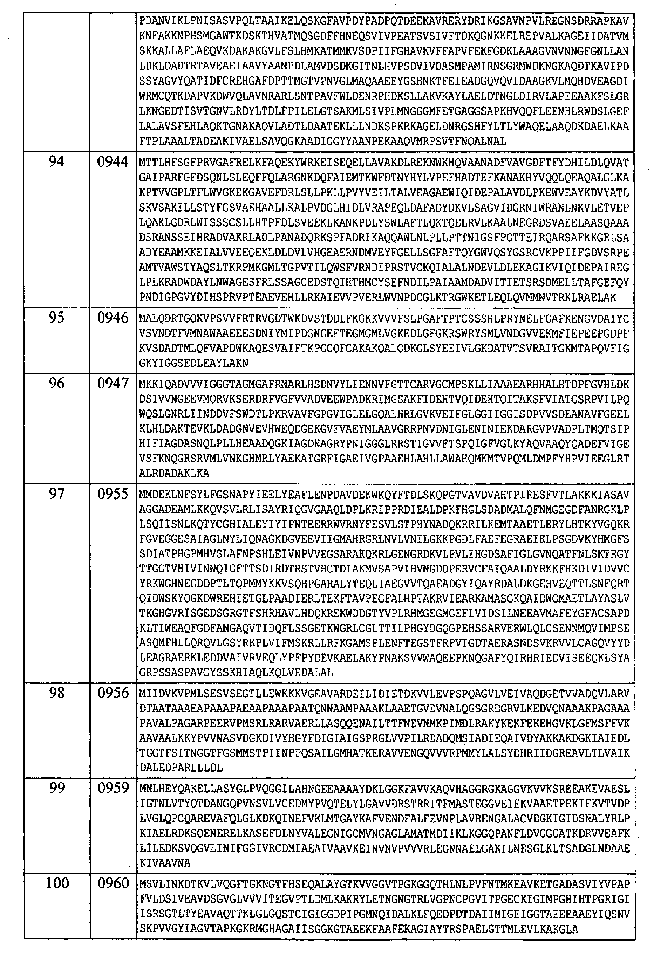

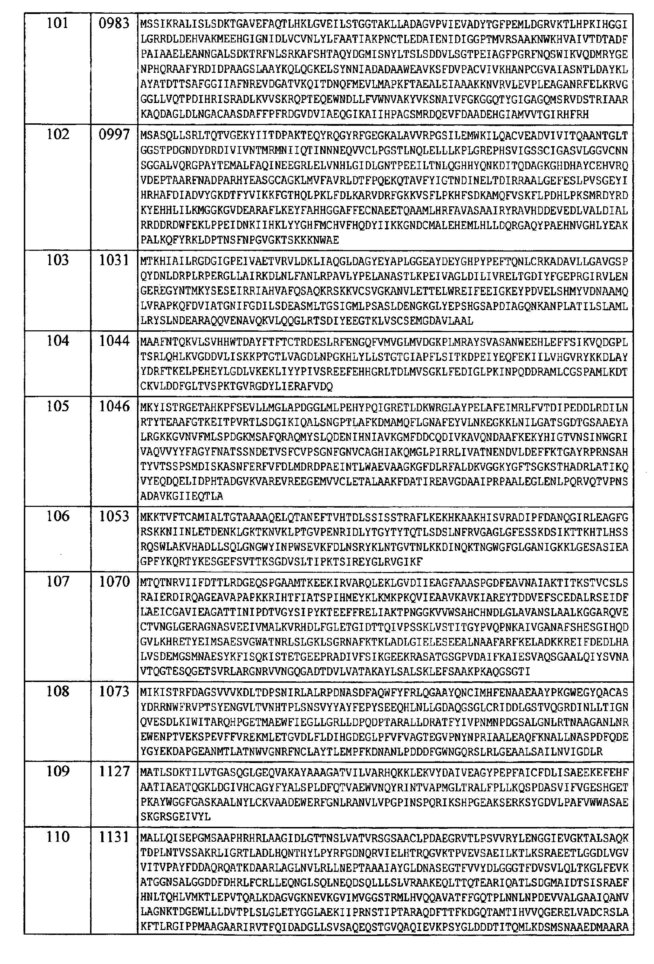

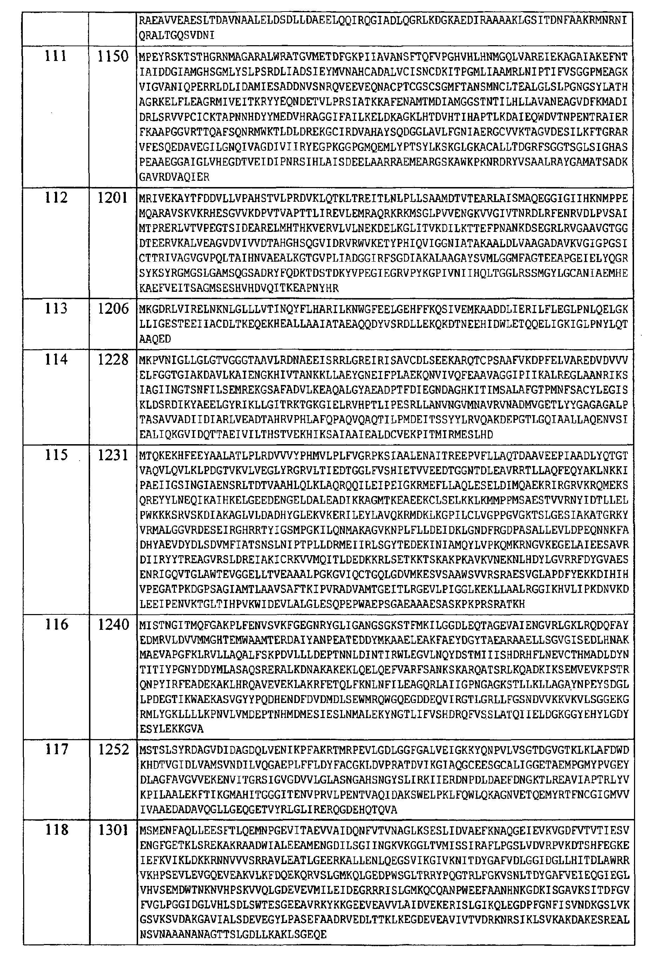

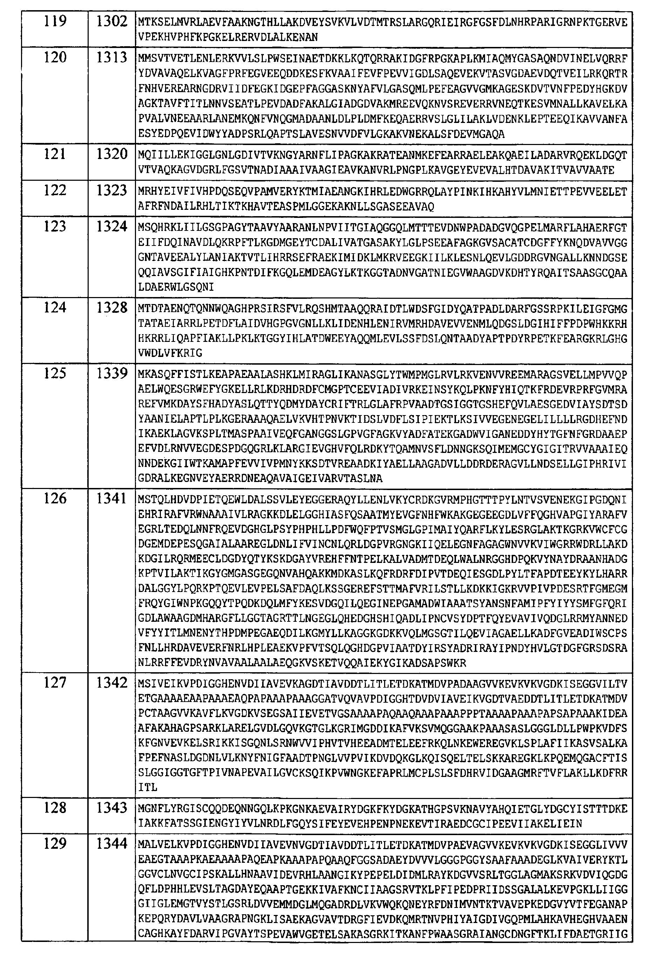

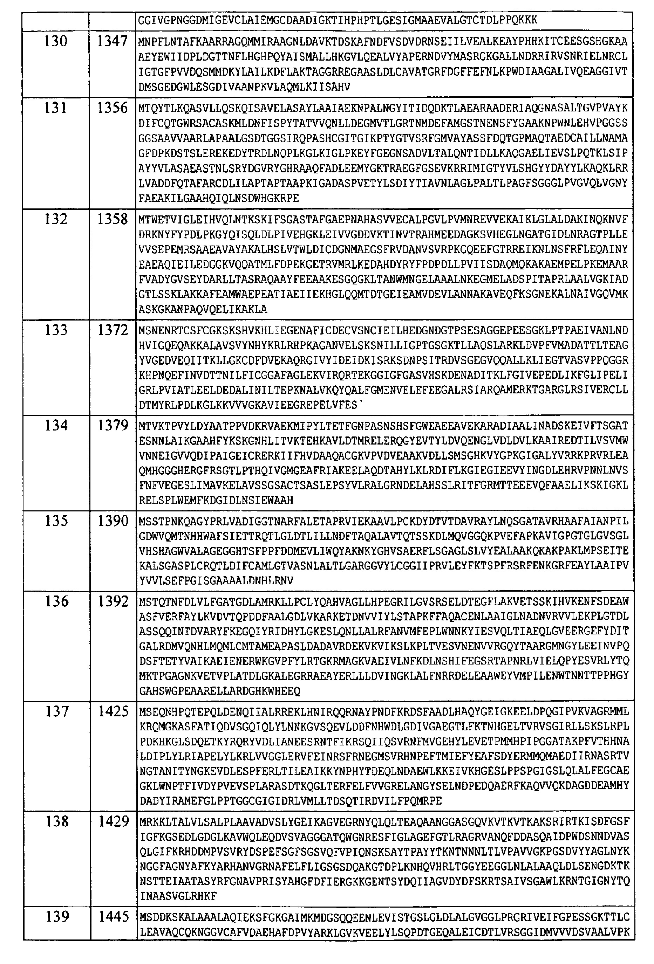

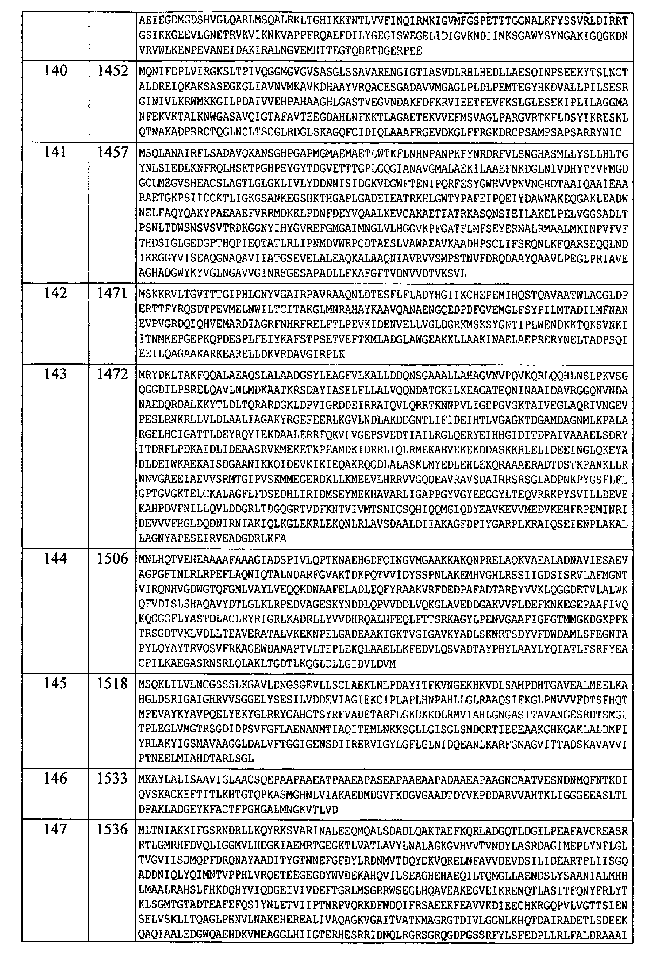

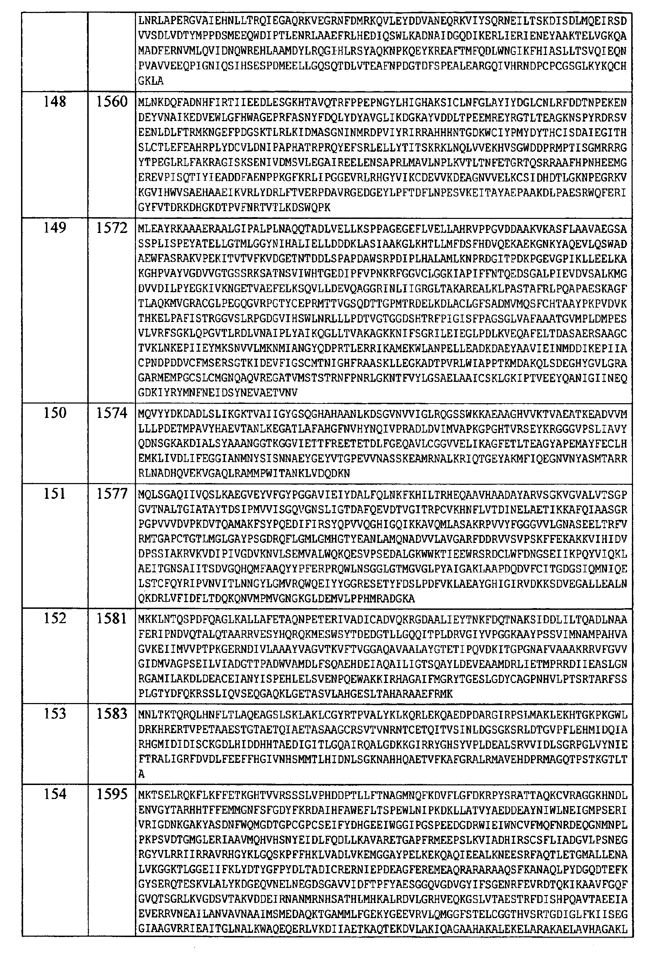

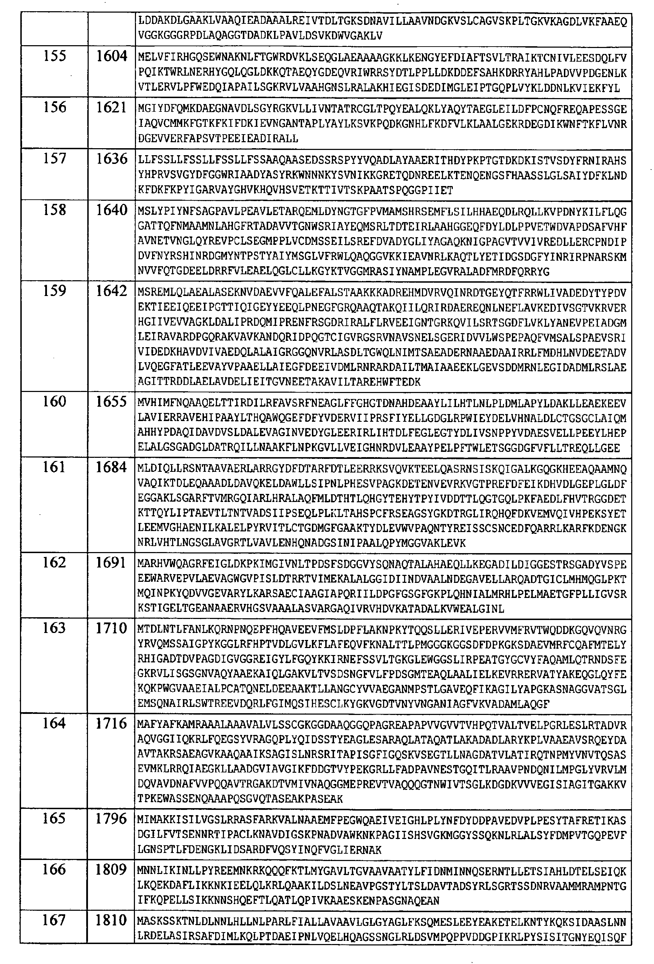

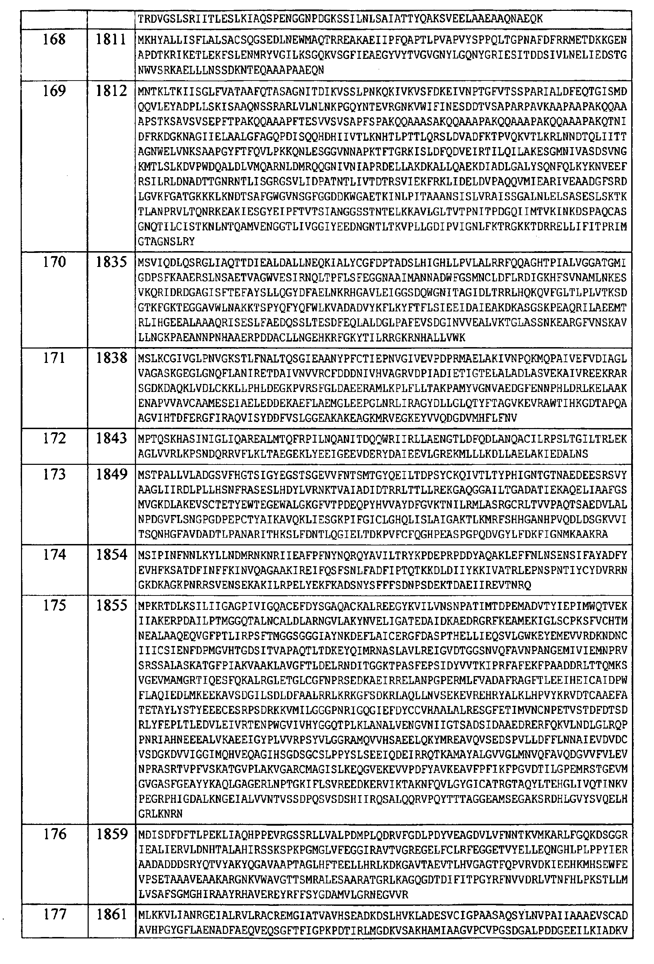

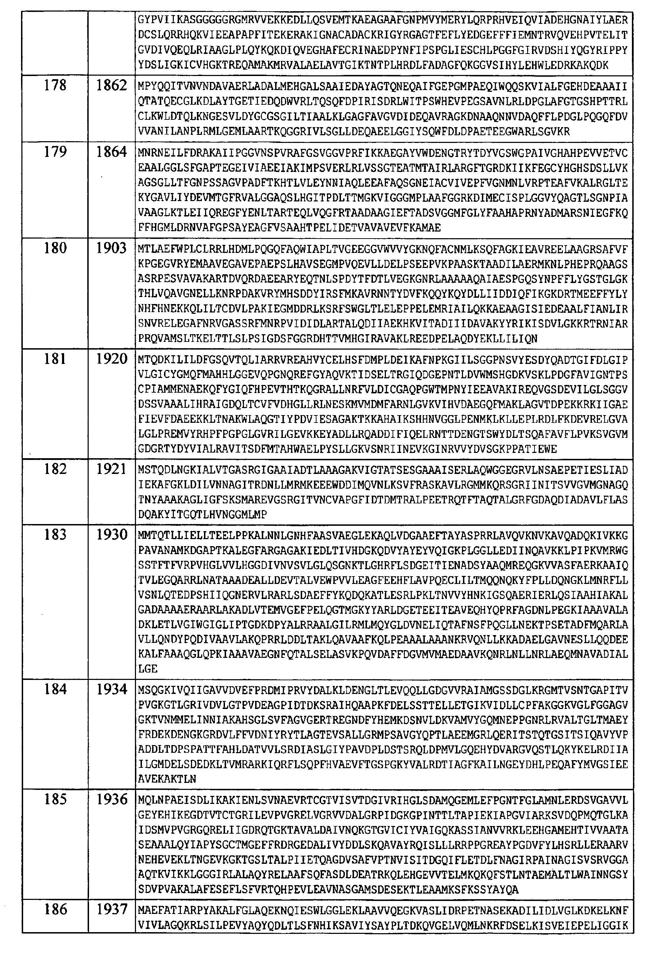

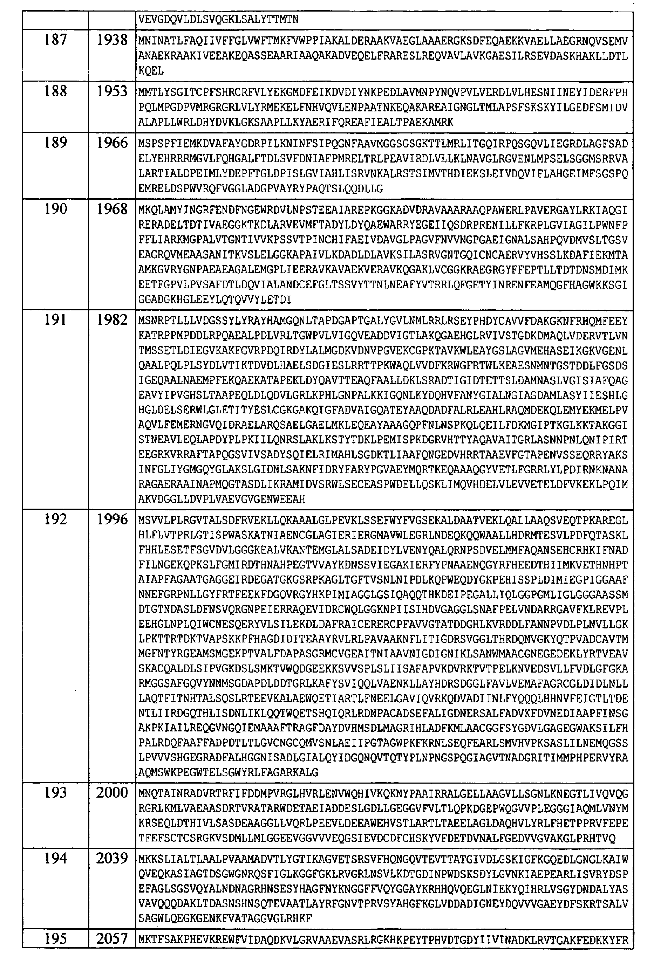

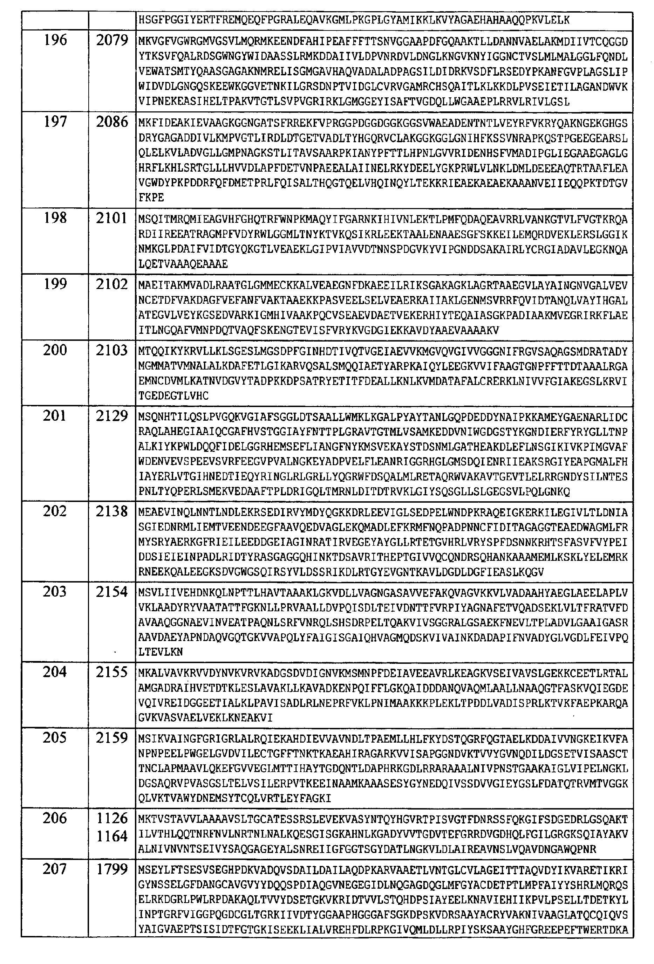

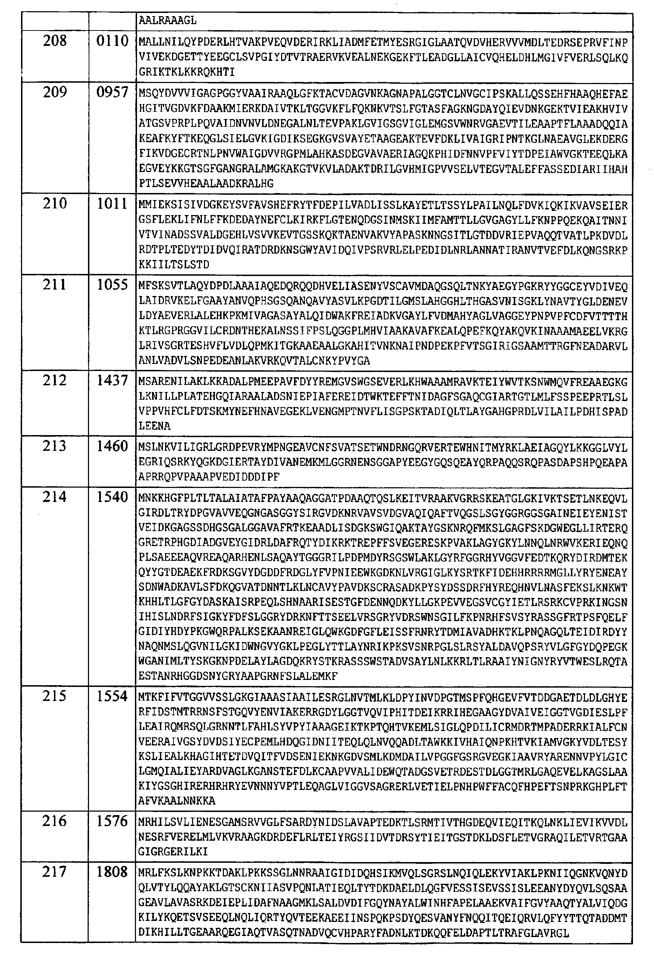

The 217 proteins are listed in Table 1 according to their 'NMB' numbering, which corresponds to the standardised nomenclature from reference 1. The NMB numbering can be used directly to query online databases *e.g.* a protein query of the NCBI Entrez system [12] using the term 'NMB1506' unequivocally identifies the Arginyl-tRNA synthetase of *N.meningitidis* (see Figure 5), leading to a single amino acid sequence. Details of the DNA sequences which encode these proteins are similarly accessible from the online databases. The original release of a sequence in a database of choice (*e.g.* GenBank) may be preferred.

Although the online databases are the most convenient source of information concerning the 217 proteins of the invention, and the most likely to be consulted by the skilled person, for formal reasons the amino acid sequences are also provided in a sequence listing (SEQ ID NOs: I to 217).

The invention provides a lipid bilayer which includes a protein comprising the amino acid sequence of SEQ ID NO: 165

It also provides a lipid bilayer which includes a protein comprising an amino acid sequence which shares at least 90% sequence identity with the amino acid sequence of SEQ ID NO: 165. Depending on the particular sequence, sequence identity is preferably 90% or more (*e.g.* 95%, 99% or more). These proteins include allelic variants, homologs, orthologs, paralogs, mutants *etc.* of SEQ ID NO: 165. Typically, 50% identity or more between two proteins is considered to be an indication of functional equivalence. Identity between proteins is preferably determined by the Smith-Waterman homology search algorithm as implemented in the MPSRCH program (Oxford Molecular), using an affine gap search with parameters *gap open penalty=12* and *gap extension penalty=1.*

It is preferred that one or more of the differences in these proteins compared to SEQ ID NO: 165 involves a conservative amino acid replacement *i.e.* replacement of one amino acid with another which has a related side chain. Genetically-encoded amino acids are generally divided into four families: (1) acidic *i.e.* aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e.* glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. Proteins may contain 1 or more (*e.g.* 2, 3, 4, 5, 6, 7, 8, 9, 10, 11, 12, 13, 14, 15, 16, 17, 18, 19, 20 or more) conservative mutations.

The invention further provides a lipid bilayer which includes a protein comprising a fragment of the amino acid sequence of SEQ ID NO: 165. The fragment should comprise at least $n$ consecutive amino acids where, $n$ is 7 or more (*e.g.* 8, 10, 12, 14, 16, 18, 20, 30, 40, 50, 75, 100, 150, 200 or more). Preferred fragments include : (a) fragments which comprise an epitope; (b) fragments common to two or more of SEQ IDs 1 to 217; (c) SEQ ID 165 with 1 or more (*e.g.* 1, 2, 3, 4, 5, 6, 7, 8, 9, 10, 11, 12 13, 14, 15, 16,17, 18, 19, 20, 21, 22, 23, 24, 25, *etc.*) N-terminal residues deleted; (d) SEQ ID 165 with 1 or more (*e.g.* 1, 2, 3, 4, 5, 6, 7, 8, 9, 10, 11, 12, 13, 14, 15, 16,17, 18, 19, 20, 21, 22, 23, 24, 25, *etc.*) C-terminal residues deleted; (e) SEQ ID 165 without the signal peptide; and (f) SEQ ID 165 with the N-terminal deleted up to and including a poly-glycine sequence (*i.e.* $Gly_g$, where $g \geq 3$ *e.g.* 4, 5, 6, 7, 8, 9 or more), referred to as '∆G' proteins. These preferred fragments are not mutually exclusive *e.g.* a fragment could fall into category (a) and (b), or category (c) and (d), *etc.*

Proteins can be prepared by various means *e.g.* by chemical synthesis (at least in part), by digesting longer polypeptides using proteases, by translation from RNA, by purification from cell culture (*e.g.* from recombinant expression or from *N.meningitidis* culture). *etc.* Heterologous expression in *E.coli* is a preferred preparative route.

Proteins can take various forms (*e.g.* native, fusions, glycosylated, non-glycosylated, lipidated, disulfide bridges, *etc.*).

Proteins are preferably prepared in substantially pure form (*i.e.* substantially free from other Neisserial or host cell proteins) or substantially isolated form.

Proteins are preferably meningococcal proteins.

Proteins may be attached to a solid support. They may comprise a detectable label (*e.g.* a radioactive or fluorescent label, or a biotin label).

The bilayer preferably does not include native membrane components such as porins (PorA in particular, class I outer membrane proteins (OMPs), class III OMPs, *etc.*), LOS, LPS, PilC, Omp85, opacity proteins (*e.g.* Opa & Opc), pilins (*e.g.* PilC, PilT, *etc.),* P64k, *etc.* Thus lipid bilayer immunogens can be provided which include useful immunogenic proteins of the invention, but which do not include undesired components.

Preferred fusion proteins follow the approach set out in references 13 to 15 in which two or more (*e.g.* 3, 4, 5, 6 or more) Neisserial proteins are joined such that they are translated as a single polypeptide chain. In general, such hybrid proteins can be represented by the formula:

$$NH_2\text{- A- [- X- L- ]}_n\text{- B- COOH}$$

wherein X is an amino acid sequence as defined above, L is an optional linker amino acid sequence, A is an optional N- terminal amino acid sequence, B is an optional C- terminal amino acid sequence, and $n$ is an integer greater than 1. The value of $n$ is between 2 and $x$, and the value of $x$ is typically 3, 4, 5, 6, 7, 8, 9 or 10. Preferably $n$ is 2, 3 or 4; it is more preferably 2 or 3; most preferably, $n$ = 2.

In some hybrid proteins, referred to as 'tandem' proteins, a -X- moiety has sequence identity to at least one of the other X moieties, as defined above *e.g.* $X_1$ is SEQ ID 1 and $X_2$ is a variant of $X_1$.

For X moieties other than $X_1$, it is preferred that the native leader peptide should be omitted. In one embodiment, the leader peptides will be deleted except for that of the -X- moiety located at the N-terminus of the hybrid protein *i.e.* the leader peptide of $X_1$ will be retained, but the leader peptides of $X_2$ ... $X_n$ will be omitted. This is equivalent to deleting all leader peptides and using the leader peptide of $X_1$ as moiety -A-.

For each $n$ instances of [- X- L- ], linker amino acid sequence- L- may be present or absent. For instance, when $n$=2 the hybrid may be $NH_2$- $X_1$- $L_1$- $X_2$- $L_2$- COOH, $NH_2$- $X_1$- $X_2$- COOH, $NH_2$- $X_1$- $L_1$- $X_2$- COOH, $NH_2$- $X_1$- $X_2$- $L_2$- COOH, *etc.* Linker amino acid sequence (s)- L- will typically be short (*e.g.* 20 or fewer amino acids *i.e.* 19, 18, 17, 16, 15, 14, 13, 12, 11, 10, 9, 8, 7, 6, 5, 4, 3, 2, 1) . Examples include short peptide sequences which facilitate cloning, poly-glycine linkers (*i.e.* Gly$_n$ where $n$ = 2, 3, 4, 5, 6, 7, 8, 9, 10 or more), and histidine tags (*i.e.* His$_n$ where $n$ = 3, 4, 5, 6, 7, 8, 9, 10 or more) . Other suitable linker amino acid sequences will be apparent to those skilled in the art. A useful linker is GSGGGG, with the Gly- Ser dipeptide being formed from a *Bam*HI restriction site, thus aiding cloning and manipulation, and the Gly$_4$ tetrapeptide being a typical poly- glycine linker.

- A- is an optional N-terminal amino acid sequence. This will typically be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification (*e.g.* histidine tags *i.e.* His$_n$ where $n$ = 3, 4, 5, 6, 7, 8, 9, 10 or more). Other suitable N-terminal amino acid sequences will be apparent to those skilled in the art. If X$_1$ lacks its own N-terminus methionine, -A-may be a methionine residue.

- B- is an optional C-terminal amino acid sequence. This will typically be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification (*e.g.* comprising histidine tags *i.e.* His$_n$ where $n$ = 3, 4, 5, 6, 7, 8, 9, 10 or more), or sequences which enhance protein stability. Other suitable C-terminal amino acid sequences will be apparent to those skilled in the art.

Within SEQ ID NO$^s$ I to 217, those marked with a '*' in Table 1 are preferred, as are SEQ IDs 206 to 217. In Table I, the 76 sequences marked '**' are particularly preferred.

The invention includes the use of a nucleic acid in a method of preparing a lipid bilayer as defined in the claims.

Nucleic acid can, of course, be prepared in many ways *e.g.* by chemical synthesis (at least in part), by digesting longer polynucleotides using restriction enzymes, from genomic or cDNA libraries, from the organism itself *etc.*

Nucleic acid can take various forms (*e.g.* single-stranded, double-stranded, linear, circular, vectors, primers, probes *etc.).*

Nucleic acids may be attached to a solid support (*e.g.* a bead, plate, filter, film, slide, resin, *etc.*). Nucleic acids may include a detectable label *(e.g.* a radioactive or fluorescent label, or a biotin label). This is particularly useful where the polynucleotide is to be used in nucleic acid detection techniques *e.g.* where the nucleic acid is a primer or as a probe for use in techniques such as PCR, LCR, TMA, NASBA, bDNA, *etc.*

Nucleic acids are preferably meningococcal nucleic acids.

The term "nucleic acid" includes DNA, RNA, DNA/RNA hybrids, and DNA or RNA analogs, such as those containing modified backbones or bases, and also peptide nucleic acids (PNA) *etc..*

Nucleic acids may be isolated and obtained in substantial purity, generally as other than an intact chromosome. Usually, the polynucleotides will be obtained substantially free of other naturally-occurring nucleic acid sequences, generally being at least about 50% (by weight) pure, usually at least about 90% pure.

Nucleic acids can be used, for example: to produce polypeptides; as probes for the detection of nucleic acid in biological samples; to generate additional copies of the polynucleotides; to generate ribozymes or antisense oligonucleotides; and as single-stranded DNA probes or as triple-strand forming oligonucleotides *etc.*

The invention can be used with vectors comprising nucleotide sequences of the invention (*e.g.* cloning or expression vectors) and host cells transformed therewith.

### *Outer membrane vesicles (OMVs)*

One of the various approaches to immunising against *N.meningitidis* infection is to use outer membrane vesicles (OMVs). An efficacious OMV vaccine against serogroup B has been produced by the Norwegian National Institute of Public Health [*e.g.* ref. 16] but, although this vaccine is safe and prevents meningococcal disease, its efficacy is limited to the strain used to make the vaccine.

To increase the efficacy of OMV vaccines, it has been proposed to supplement them with additional immunogens. Reference 17 discloses compositions comprising OMVs supplemented with transferrin binding proteins (*e.g.* TbpA and TbpB) and/or Cu,Zn-superoxide dismutase. Reference 18 discloses compositions comprising OMVs supplemented by various proteins.

The inventors have found that the portion of the proteome present in OMVs prepared from one strain of *N.meningitidis* can be very different from that of another strain. However, the proteins present in OMVs from any given strain must be compatible with the OMV environment, and so such proteins can be used to supplement OMVs from other strains without needing to be concerned that the proteins might lose immunogenicity, or that they might destabilise the OMVs, *etc.*

In general, therefore, the invention provides a composition comprising: (a) OMVs prepared from a first strain of *N.meningitidis*; and (b) one or more proteins which are present in OMVs prepared from a second strain of *N.meningitidis,* but which are not present in OMVs prepared from said first strain. Thus the invention allows the inter-strain reactivity of OMVs to be improved, without requiring the mixing of OMVs from different strains.

The protein (s) of component (b) can be included in the composition in various ways. For example: (i) they can be purified from the second strain and added to component (a) ; (ii) they can be expressed recombinantly, purified, and added to component (a) ; or (iii) the first strain can be engineered such that it expresses said protein (s), either from its chromosomal DNA or from extrachromosomal DNA *(e.g.* a plasmid), or such that existing expression of said protein (s) is up- regulated, or such that trafficking of said protein (s) already expressed by the first strain is altered to direct it/ them to a different cellular location, thereby causing it/ them to be present in OMVs. In situation (iii), the OMVs of component (a), prepared from the manipulated first strain, will already include the proteins of component (b), but these proteins are not present in OMVs prepared from the un- manipulated or wild- type first strain. Thus the invention provides a composition comprising OMVs prepared from a genetically modified first strain of *N.meningitidis,* wherein said OMVs include one or more proteins which are (a) not present in OMVs prepared from said first strain prior to its being genetically modified, but which are (b) present in OMVs prepared from a second strain of *N.meningitidis.*

The *N.meningitidis* strains are preferably from serogroup B of *N.meningitidis.*

Methods for preparing OMVs are well known [*e.g.* refs. 16 to 26]. also describe OMV preparations from meningococcus. A preferred method involves deoxycholate extraction.

The proteins present in component (b) will depend on the meningococcal strain used to prepare the OMVs of component (a). However, preferred proteins for inclusion in component (b) are: NMB0007, NMB0018, NMB0031, NMB0035, NMB0051, NMB0052, NMB0088, NMB0089, NMB0109, NMB0110, NMB0124, NMB0126, NMB0130, NMB132, NMB0138, NMB0139, NMB0143, NMB0154, NMB0157, NMB0168, NMB0171, NMB0182, NMB0204, NMB0214, NMB0219, NMB0280, NMB0313, NMB0336, NMB0359, NMB0375, NMB0382, NMB0387, NMB0410, NMB0423, NMB0426, NMB0427, NMB0461, NMB0462, NMB0477, NMB0546, NMB0554, NMB0586, NMB0595, NMB0604, NMB0610, NMB0617, NMB0618, NMB0623, NMB0626, NMB0631, NMB0634, NMB0638, NMB0652, NMB0663, NMB0703, NMB0757, NMB0758, NMB0763, NMB0787, NMB0854, NMB0875, NMB0876, NMB0889, NMB0920, NMB0943, NMB0944, NMB0946, NMB0954, NMB0955, NMB0957, NMB0959, NMB0983, NMB1011, NMB1046, NMB1053, NMB1055, NMB1124, NMB1126, NMB1127, NMB1131, NMB1153, NMB1162, NMB1164, NMB1165, NMB1191, NMB1199, NMB1201, NMB1228, NMB1240, NMB1252, NMB1285, NMB1301, NMB1313, NMB1323, NMB1332, NMB1339, NMB1341, NMB1342, NMB1358, NMB1392, NMB1429, NMB1437, NMB1445, NMB1457, NMB1460, NMB1497, NMB 1506, NMB 1518, NMB1533, NMB1540, NMB1554, NMB1567, NMB1572, NMB1574, NMB1576, NMB1577, NMB1594, NMB1612, NMB1642, NMB1668, NMB1684, NMB1710, NMB1714, NMB1762, NMB1796, NMB1799, NMB1808, NMB1812, NMB1849, NMB1854, NMB1855, NMB1861, NMB1869, NMB1874, NMB1903, NMB1921, NMB1934, NMB1936, NMB1946, NMB1949, NMB1953, NMB1969, NMB1972, NMB1988, NMB1998, NMB2039, NMB2069, NMB2086, NMB2096, NMB2101, NMB2102, NMB2103, NMB2129, NMB2134, NMB2138, NMB2154 and NMB2159. Particularly preferred are NMB0182, NMB0382, NMB0634, NMB0763, NMB1126, NMB1342, NMB1429, NMB1799 and NMB2039.

It will be appreciated that these references to NMB proteins are based on the genomic sequence of serogroup B strain MC58 [1], and that the corresponding proteins in any particular strain, and the genes encoding them, can readily be determined.

***Compositions***

According to a further aspect, the invention provides compositions comprising protein and/or nucleic acid and/or OMVs according to the invention. These compositions are preferably immunogenic compositions, such as vaccines, and are suitable for immunisation and vaccination purposes. Vaccines of the invention may be prophylactic or therapeutic, and will typically comprise an antigen which can induce antibodies which are (a) capable of binding to a meningococcal membrane component and/or (b) bactericidal against meningococcus.

Compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal; intranasal, ocular, aural, or pulmonary or other mucosal administration. Electrical methods may be used to for delivery *e.g.* delivery via electroporation.

The invention may be used to elicit systemic and/or mucosal immunity.

Dosage treatment can be a single dose schedule or a multiple dose schedule.

The immunogenic composition of the invention will generally include a pharmaceutically acceptable carrier, which can be any substance that does not itself induce the production of antibodies harmful to the patient receiving the composition, and which can be administered without undue toxicity. Suitable carriers can be large, slowly-metabolised macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers can include liquids such as water, saline, glycerol and ethanol. Auxiliary substances, such as wetting or emulsifying agents, pH buffering substances, and the like, can also be present in such vehicles. Liposomes are suitable carriers. A thorough discussion of pharmaceutical carriers is available in ref. 27.

Neisserial infections affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared. The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as drops.

The composition is preferably sterile. It is preferably pyrogen-free. It is preferably buffered *e.g.* at between pH 6 and pH 8, generally around pH 7.

*Further components of compositions*

Immunogenic compositions comprise an immunologically effective amount of immunogen, as well as any other of other specified components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.*), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g.* including booster doses). The composition may be administered in conjunction with other immunoregulatory agents.

The composition will generally comprise an adjuvant. Preferred adjuvants to enhance effectiveness of the composition include, but are not limited to: (A) MF59 (5% Squalene, 0.5% Tween 80, and 0.5% Span 85, formulated into submicron particles using a microfluidizer) [see Chapter 10 of ref. 28; see also ref. 29] ; (B) microparticles (*i.e.* a particle of ~100nm to ~150$\mu$m in diameter, more preferably ~200nm to ~30$\mu$m in diameter, and most preferably ~500nm to ~10$\mu$m in diameter) formed from materials that are biodegradable and non-toxic (*e.g.* a poly ($\alpha$-hydroxy acid), a polyhydroxybutyric acid, a polyorthoester, a polyanhydride, a polycaprolactone *etc.*), with poly (lactide-co-glycolide) being preferred ('PLG'), optionally being charged surface (*e.g.* by adding a cationic, anionic, or nonionic detergent such as SDS (negative) or CTAB (positive) [*e.g.* refs. 30 & 31] ) ; (C) liposomes [see Chapters 13 and 14 of ref. 28] ; (D) ISCOMs [see Chapter 23 of ref. 28], which may be devoid of additional detergent [32] ; (E) SAF, containing 10% Squalane, 0.4% Tween 80, 5% pluronic-block polymer L121, and thr-MDP, either microfluidized into a submicron emulsion or vortexed to generate a larger particle size emulsion [see Chapter 12 of ref. 28] ; (F) Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™) ; (G) saponin adjuvants, such as QuilA or QS21 [see Chapter 22 of ref. 28], also known as Stimulon™; (H) chitosan [*e.g.* 33] ; (I) complete Freund's adjuvant (CFA) and incomplete Freund's adjuvant (IFA) ; (J) cytokines, such as interleukins (*e.g.* IL-1, IL-2, IL-4, IL-5, IL-6, IL-7, IL-12, *etc.*), interferons (*e.g.* interferon-y), macrophage colony stimulating factor, tumor necrosis factor, *etc.* [see Chapters 27 & 28 of ref. 28], RC529; (K) a saponin (*e.g.* QS21) + 3dMPL + IL-12 (optionally + a sterol) [34] ; (L) monophosphoryl lipid A (MPL) or 3-O-deacylated MPL (3dMPL) [*e.g.* chapter 21 of ref. 28] ; (M) combinations of 3dMPL with, for example, QS21 and/or oil-in-water emulsions [35] ; (N) oligonucleotides comprising CpG motifs [36] *i.e.* containing at least one CG dinucleotide, with 5-methylcytosine optionally being used in place of cytosine; (O) a polyoxyethylene ether or a polyoxyethylene ester [37] ; (P) a polyoxyethylene sorbitan ester surfactant in combination with an octoxynol [38] or a polyoxyethylene alkyl ether or ester surfactant in combination with at least one additional non-ionic surfactant such as an octoxynol [39] ; (Q) an immunostimulatory oligonucleotide (*e.g.* a CpG oligonucleotide) and a saponin [40] ; (R) an immunostimulant and a particle of metal salt [41] ; (S) a saponin and an oil-in-water emulsion [42] ; (T) *E.coli* heat-labile enterotoxin ("LT"), or detoxified mutants thereof, such as the K63 or R72 mutants [*e.g.* Chapter 5 of ref. 43] ; (U) cholera toxin ("CT"), or detoxified mutants thereof [e.g. Chapter 5 of ref. 43] ; (V) double-stranded RNA; (W) aluminium salts, such as aluminium hydroxides (including oxyhydroxides), aluminium phosphates (including hydroxyphosphates), aluminium sulfate, *etc* [Chapters 8 & 9 in ref. 44] ; (X) monophosphoryl lipid A mimics, such as aminoalkyl glucosaminide phosphate derivatives e.g. RC-529 [45] ; and (Y) other substances that act as immunostimulating agents to enhance the effectiveness of the composition [*e.g.* see Chapter 7 of ref. 28], such as calcium phosphate. Aluminium salts (aluminium phosphates and particularly hydroxyphosphates, and/or hydroxides and particularly oxyhydroxide) are preferred adjuvants for parenteral immunisation. Toxin mutants are preferred mucosal adjuvants.

Muramyl peptides include N- acetyl- muramyl- L- threonyl- D- isoglutamine (thr- MDP), N- acetyl- normuramyl- L- alanyl- D- isoglutamine (nor- MDP), N- acetylmuramyl- L- alanyl- D- isoglutaminyl- L- alanine- 2- (1'- 2'- dipalmitoyl- *sn*- glycero- 3- hydroxyphosphoryloxy)- ethylamine MTP- PE), *etc.*

Compositions of the invention may comprise antigens (*e.g.* protective antigens against *N.meningitidis* or against other organisms) in addition to the proteins mentioned above *e.g.* DTP antigens, Hib antigen *etc.* The composition may comprise one or more of the following further antigens:

- antigens from *Helicobacter pylori* such as CagA [46 to 49], VacA [50, 51], NAP [52, 53, 54], HopX [*e.g.* 55], HopY [*e.g.* 55] and/or urease.

- a saccharide antigen from *N.meningitidis* serogroup A, C, W135 and/or Y, such as the oligosaccharide disclosed in ref. 56 from serogroup C [see also ref. 57] or the oligosaccharides of ref. 58.

- a saccharide antigen from *Streptococcus pneumoniae* [*e.g.* 59, 60, 61].

- an antigen from hepatitis A virus, such as inactivated virus [*e.g.* 62, 63].

- an antigen from hepatitis B virus, such as the surface and/or core antigens [*e.g.* 63, 64].

- an antigen from *Bordetella pertussis*, such as pertussis holotoxin (PT) and filamentous haemagglutinin (FHA) from *B.pertussis*, optionally also in combination with pertactin and/or agglutinogens 2 and 3 [*e.g.* refs. 65 & 66].

- a diphtheria antigen, such as a diphtheria toxoid [*e.g.* chapter 3 of ref. 67] e.g. the $CRM_{197}$ mutant [*e.g.* 68].

- a tetanus antigen, such as a tetanus toxoid [*e.g.* chapter 4 of ref. 87].

- a saccharide antigen from *Haemophilus influenzae* B [*e.g.* 57].

- an antigen from hepatitis C virus [*e.g.* 69].

- an antigen from *N.gonorrhoeae* [*e.g.* 70, 71, 72, 73].

- an antigen from *Chlamydia pneumoniae* [*e.g.* refs. 74 to 80].

- an antigen from *Chlamydia trachomatis* [*e.g.* 81].

- an antigen from *Porphyromonas gingivalis* [*e.g.* 82].

- polio antigen(s) [*e.g.* 83, 84] such as IPV.

- rabies antigen(s) [*e.g.* 85] such as lyophilised inactivated virus [*e.g.* 86, RabAvert™].

- measles, mumps and/or rubella antigens [*e.g.* chapters 9, 10 & 11 of ref. 87].

- influenza antigen(s) [*e.g.* chapter 19 of ref. 87], such as the haemagglutinin and/or neuraminidase surface proteins.

- an antigen from *Moraxella catarrhalis* [*e.g.* 88].

- an protein antigen from *Streptococcus agalactiae* (group B streptococcus) [*e.g.* 89, 90].

- a saccharide antigen from *Streptococcus agalactiae* (group B streptococcus).

- an antigen from *Streptococcus pyogenes* (group A streptococcus) [*e.g.* 90, 91, 92].

- an antigen from *Staphylococcus aureus* [*e.g.* 93].

- an antigen from *Bacillus anthracis* [*e.g.* 94, 95, 96].

- an antigen from a virus in the flaviviridae family (genus flavivirus), such as from yellow fever virus, Japanese encephalitis virus, four serotypes of Dengue viruses, tick-borne encephalitis virus, West Nile virus.

- a pestivirus antigen, such as from classical porcine fever virus, bovine viral diarrhoea virus, and/or border disease virus.

- a parvovirus antigen *e.g.* from parvovirus B 19.

- a prion protein (*e.g.* the CJD prion protein)

- an amyloid protein, such as a beta peptide [97]

- a cancer antigen, such as those listed in Table 1 of ref. 98 or in tables 3 & 4 of ref. 99.

The composition may comprise one or more of these further antigens.

Toxic protein antigens may be detoxified where necessary (*e.g.* detoxification of pertussis toxin by chemical and/or genetic means [66]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Saccharide antigens are preferably in the form of conjugates. Carrier proteins for the conjugates include the *N.meningitidis* outer membrane protein [100], synthetic peptides [101,102], heat shock proteins [103,104], pertussis proteins [105,106], protein D from *H.influenzae* [107], cytokines [108], lymphokines [108], streptococcal proteins, hormones [108], growth factors [108], toxin A or B from *C.difficile* [109], iron-uptake proteins [110], *etc.* A preferred carrier protein is the CRM197 diphtheria toxoid [111].

Antigens in the composition will typically be present at a concentration of at least 1 $\mu$g/ml each. In general, the concentration of any given antigen will be sufficient to elicit an immune response against that antigen.

Immunogenic compositions of the invention may be used therapeutically (*i.e.* to treat an existing infection) or prophylactically (*i.e.* to prevent future infection).

As an alternative to using proteins antigens in the immunogenic compositions of the invention, nucleic acid (preferably DNA *e.g.* in the form of a plasmid) encoding the antigen may be used.

### *Medical methods etc.*

The invention also provides nucleic acids or proteins or OMVs of the invention, or antibodies which bind to proteins of the invention, for use as medicaments (*e.g.* as vaccines). It also provides the use of nucleic acid or protein or OMVs according to the invention in the manufacture of a medicament (*e.g.* a vaccine or an immunogenic composition) for treating or preventing infection due to a *Neisseria.* This will generally be *N.meningitidis* but, due to inter-species cross-reactivity, it may also be *N.gonorrhoeae.*

The invention can be used with a method of treating (*e.g.* immunising) a patient (*e.g.* a human), comprising administering to the patient a therapeutically effective amount of nucleic acid and/or protein and/or OMVs according to the invention.

The invention can be used with a method of raising antibodies in an animal, comprising administering to the patient an immunologically effective amount of nucleic acid and/or protein and/or OMVs according to the invention.

The invention can be used with a method of detecting *N.meningitidis* bacteria in a sample, comprising the steps of: (a) contacting an antibody (*e.g.* monoclonal or polyclonal) which binds to a protein of the invention with a biological sample under conditions suitable for the formation of an antibody-antigen complexes; and (b) detecting said complexes. The method is particularly suitable for detecting intact bacteria because the proteins of the invention are present in the membrane. If the correct protein is used, it is also useful for distinguishing between *N.meningitidis* strains.

### *Techniques*

A summary of standard techniques and procedures which may be employed in order to perform the invention (*e.g.* to utilise the disclosed sequences for immunisation or diagnosis) follows. This summary is not a limitation on the invention but, rather, gives examples that may be used, but are not required.

*General*

The practice of the present invention will employ, unless otherwise indicated, conventional techniques of molecular biology, microbiology, recombinant DNA, and immunology, which are within the skill of the art. Such techniques are explained fully in the literature *e.g.* Sambrook Molecular Cloning; A Laboratory Manual, Second Edition (1989) and Third Edition (2001) ; DNA Cloning, Volumes I and ii (D.N Glover ed. 1985) ; Oligonucleotide Synthesis (M.J. Gait ed, 1984) ; Nucleic Acid Hybridization (B.D. Hames & S.J. Higgins eds. 1984) ; Transcription and Translation (B.D. Hames & S.J. Higgins eds. 1984) ; Animal Cell Culture (R.I. Freshney ed. 1986) ; Immobilized Cells and Enzymes (IRL Press, 1986) ; B. Perbal, A Practical Guide to Molecular Cloning (1984) ; the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory) ; Mayer and Walker, eds. (1987), Immunochemical Methods in Cell and Molecular Biology (Academic Press, London) ; Scopes, (1987) Protein Purification: Principles and Practice, Second Edition (Springer- Verlag, N.Y.), and Handbook of Experimental Immunology, Volumes I- IV (D.M. Weir and C. C. Blackwell eds 1986) .

Standard abbreviations for nucleotides and amino acids are used in this specification.

*Definitions*

A composition containing X is "substantially free of" Y when at least 85% by weight of the total X+Y in the composition is X. Preferably, X comprises at least about 90% by weight of the total of X+Y in the composition, more preferably at least about 95% or even 99% by weight.

The term "comprising" means "including" as well as "consisting" e.g. a composition "comprising" X may consist exclusively of X or may include something additional to X, such as X+Y.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a Neisserial sequence is heterologous to a mouse host cell. A further examples would be two epitopes from the same or different proteins which have been assembled in a single protein in an arrangement not found in nature.

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% (*e.g.* 60%, 70%, 80%, 90%, 95%, 99% or more, calculated using the Smith- Waterman algorithm as described above) . As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (*e.g.* see US patent 5, 753, 235) .

*Expression systems*

The Neisserial nucleotide sequences can be expressed in a variety of different expression systems; for example those used with mammalian cells, baculoviruses, plants, bacteria, and yeast.

i. Mammalian Systems

Mammalian expression systems are known in the art. A mammalian promoter is any DNA sequence capable of binding mammalian RNA polymerase and initiating the downstream (3') transcription of a coding sequence (*e.g.* structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.*].*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non- viral genes, such as the murine metallotheionein gene, also provide useful promoter sequences. Expression may be either constitutive or regulated (inducible), depending on the promoter can be induced with glucocorticoid in hormone- responsive cells.

The presence of an enhancer element (enhancer), combined with the promoter elements described above, will usually increase expression levels. An enhancer is a regulatory DNA sequence that can stimulate transcription up to 1000- fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236: 1237; Alberts et al. (1989) Molecular Biology of the Cell, 2nd ed.] . Enhancer elements derived from viruses may be particularly useful, because they usually have a broader host range. Examples include the SV40 early gene enhancer [Dijkema et al (1985) EMBO J. 4: 761] and the enhancer/ promoters derived from the long terminal repeat (LTR) of the Rous Sarcoma Virus [Gorman et al. (1982) PNAS USA 79: 6777] and from human cytomegalovirus [Boshart et al. (1985) Cell 41: 521] . Additionally, some enhancers are regulatable and become active only in the presence of an inducer, such as a hormone or metal ion [Sassone- Corsi and Borelli (1986) Trends Genet. 2: 215; Maniatis et al. (1987) Science 236: 1237] .

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site- specific post- transcriptional cleavage and polyadenylation [Birnstiel et al. (1985) Cell 41: 349; Proudfoot and Whitelaw (1988) "Termination and 3' end processing of eukaryotic RNA. In Transcription and splicing (ed. B.D. Hames and D.M. Glover) ; Proudfoot (1989) Trends Biochem. Sci. 14: 105] . These sequences direct the transcription of an mRNA which can be translated into the polypeptide encoded by the DNA. Examples of transcription terminater/ polyadenylation signals include those derived from SV40 [Sambrook et al (1989) "Expression of cloned genes in cultured mammalian cells." In Molecular Cloning: A Laboratory Manual] .

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (*e.g.* plasmids) capable of stable maintenance in a host, such as mammalian cells or bacteria. Mammalian replication systems include those derived from animal viruses, which require trans- acting factors to replicate. For example, plasmids containing the replication systems of papovaviruses, such as SV40 [Gluzman (1981) Cell 23: 175] or polyomavirus, replicate to extremely high copy number in the presence of the appropriate viral T antigen. Additional examples of mammalian replicons include those derived from bovine papillomavirus and Epstein- Barr virus. Additionally, the replicon may have two replicaton systems, thus allowing it to be maintained, for example, in mammalian cells for expression and in a prokaryotic host for cloning and amplification. Examples of such mammalian- bacteria shuttle vectors include pMT2 [Kaufman et al. (1989) Mol. Cell. Biol. 9: 946] and pHEBO [Shimizu et al. (1986) Mol. Cell. Biol. 6: 1074] .

The transformation procedure used depends upon the host to be transformed. Methods for introduction of heterologous polynucleotides into mammalian cells are known in the art and include dextran-mediated transfection, calcium phosphate precipitation, polybrene-mediated transfection, protoplast fusion, electroporation, encapsulation of polynucleotide(s) in liposomes, direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g. Hep G2), and a number of other cell lines.

ii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia,* Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (e.g. plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17: 31.

The plasmid usually also contains the polyhedrin polyadenylation signal (Miller et al. (1988) Ann. Rev. Microbiol., 42:177) and a prokaryotic ampicillin-resistance (*amp*) gene and origin of replication for selection and propagation in *E. coli.*

Baculovirus transfer vectors usually contain a baculovirus promoter. A baculovirus promoter is any DNA sequence capable of binding a baculovirus RNA polymerase and initiating the downstream (5' to 3') transcription of a coding sequence (*e.g.* structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human $\alpha$-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra*; Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91.The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between ~1% and ~5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to $15\mu$m in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers & Smith, *supra*; Miller et al. (1989).

Recombinant baculovirus expression vectors have been developed for infection into several insect cells. For example, recombinant baculoviruses have been developed for, *inter alia: Aedes aegypti, Autographa californica, Bombyx mori, Drosophila melanogaster, Spodoptera frugiperda,* and *Trichoplusia ni* (WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. *See, e.g.* Summers and Smith *supra*.

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g. HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g. proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iii. Plant Systems

There are many plant cell culture and whole plant genetic expression systems known in the art. Exemplary plant cellular genetic expression systems include those described in patents, such as: US 5, 693, 506; US 5, 659, 122; and US 5, 608, 143. Additional examples of genetic expression in plant cell culture has been described by Zenk, Phytochemistry 30: 3861- 3863 (1991) . Descriptions of plant protein signal peptides may be found in addition to the references described above in Vaulcombe et al., Mol. Gen. Genet. 209: 33- 40 (1987) ; Chandler et al., Plant Molecular Biology 3: 407- 418 (1984) ; Rogers, J. Biol. Chem. 260: 3731- 3738 (1985) ; Rothstein et al., Gene 55: 353- 356 (1987) ; Whittier et al., Nucleic Acids Research 15: 2515- 2535 (1987) ; Wirsel et al., Molecular Microbiology 3: 3- 14 (1989) ; Yu et al., Gene 122: 247- 253 (1992) . A description of the regulation of plant gene expression by the phytohormone, gibberellic acid and secreted enzymes induced by gibberellic acid can be found in R.L. Jones and J. MacMillin, Gibberellins: in:

Advanced Plant Physiology, . Malcolm B. Wilkins, ed., 1984 Pitman Publishing Limited, London, pp. 21- 52. References that describe other metabolically- regulated genes: Sheen, Plant Cell, 2: 1027- 1038 (1990) ; Maas et al., EMBO J. 9: 3447- 3452 (1990) ; Benkel and Hickey, Proc. Natl. Acad. Sci. 84: 1337- 1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/ plant vector construct will preferably provide a broad host range prokaryote replication origin; a prokaryote selectable marker; and, for Agrobacterium transformations, T DNA sequences for Agrobacterium- mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11 (2) : 165- 185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad. Sci. USA, 79, 1859-1863, 1982.

The vector may also be introduced into the plant cells by electroporation. (Fromm et al., Proc. Natl Acad. Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia,*

*Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein may be excreted or alternatively, the protein may be extracted from the whole plant. Where the desired protein is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

#### iv. Bacterial Systems

Bacterial expression techniques are known in the art. A bacterial promoter is any DNA sequence capable of binding bacterial RNA polymerase and initiating the downstream (3') transcription of a coding sequence (*e.g.* structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) [Chang et al. (1977) Nature 198: 1056], and maltose. Additional examples include promoter sequences derived from biosynthetic enzymes such as tryptophan (*trp*) [Goeddel et al. (1980) Nuc. Acids Res. 8: 4057; Yelverton et al. (1981) Nucl. Acids Res. 9: 731; US patent 4, 738, 921; EP- A- 0036776 and EP- A- 0121775] . The g- laotamase (*bla*) promoter system [Weissmann (1981) "The cloning of interferon and other mistakes." In Interferon 3 (ed. I. Gresser) ], bacteriophage lambda PL [Shimatake et al. (1981) Nature 292: 128] and T5 [US patent 4, 689, 406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4, 551, 433] . For example, the *tac* promoter is a hybrid *trp- lac* promoter comprised of both *trp* promoter and *lac* operon sequences that is regulated by the *lac* repressor [Amann et al. (1983) Gene 25: 167; de Boer et al. (1983) Proc. Natl. Acad. Sci. 80: 21] . Furthermore, a bacterial promoter can include naturally occurring promoters of non- bacterial origin that have the ability to bind bacterial RNA polymerase and initiate transcription. A naturally occurring promoter of non- bacterial origin can also be coupled with a compatible RNA polymerase to produce high levels of expression of some genes in prokaryotes. The bacteriophage T7 RNA polymerase/ promoter system is an example of a coupled promoter system [Studier et al. (1986) J. Mol. Biol. 189: 113; Tabor et al. (1985) Proc Natl. Acad Sci. 82: 1074] . In addition, a hybrid promoter can also be comprised of a bacteriophage promoter and an *E. coli* operator region (EPO- A- 0 267 851) .

In addition to a functioning promoter sequence, an efficient ribosome binding site is also useful for the expression of foreign genes in prokaryotes. In *E. coli,* the ribosome binding site is called the Shine-Dalgamo (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of *E. coli* 16S rRNA [Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual].

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from the *lacZ* [Jia et al. (1987) Gene 60:197], *trpE* [Allen et al. (1987) J. Biotechnol. 5:93; Makoff et al. (1989) J. Gen. Microbiol. 135:11], and Chey [EP-A-0 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (*e.g.* ubiquitin specific processing-protease) to cleave the ubiquitin from the foreign protein. Through this method, native foreign protein can be isolated [Miller et al. (1989) Bio/Technology 7:698].

Alternatively, foreign proteins can also be secreted from the cell by creating chimeric DNA molecules that encode a fusion protein comprised of a signal peptide sequence fragment that provides for secretion of the foreign protein in bacteria [US patent 4, 336, 336] . The signal sequence fragment usually encodes a signal peptide comprised of hydrophobic amino acids which direct the secretion of the protein from the cell. The protein is either secreted into the growth media (gram- positive bacteria) or into the periplasmic space, located between the inner and outer membrane of the cell (gram- negative bacteria) . Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene (*ompA*) [Masui et al. (1983), in: Experimental Manipulation of Gene Expression*;* Ghrayeb et al. (1984) EMBO J. 3:2437] and the *E. coli* alkaline phosphatase signal sequence (*phoA*) [Oka et al. (1985) Proc. Natl. Acad. Sci. 82:7212]. As an additional example, the signal sequence of the alpha-amylase gene from various Bacillus strains can be used to secrete heterologous proteins from *B. subtilis* [Palva et al. (1982) Proc. Natl. Acad. Sci. USA 79:5582; EP-A-0 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (*e.g.* plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP- A- 0 127 328) . Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32: 469] . Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra- chromosomal replicons or integrating vectors, have been developed for transformation into many bacteria. For example, expression vectors have been developed for, *inter alia,* the following bacteria: Bacillus subtilis [Palva et al. (1982) Proc. Natl. Acad. Sci. USA 79: 5582; EP- A- 0 036 259 and EP- A- 0 063 953; WO 84/04541], Escherichia coli [Shimatake et al. (1981) Nature 292: 128; Amann et al. (1985) Gene 40: 183; Studier et al. (1986) J. Mol. Biol. 189: 113; EP- A- 0 036 776, EP- A- 0 136 829 and EP- A- 0 136 907], Streptococcus cremoris [Powell et al. (1988) Appl. Environ. Microbiol. 54: 655] ; Streptococcus lividans [Powell et al. (1988) Appl. Environ. Microbiol. 54: 655], Streptomyces lividans [US patent 4, 745, 056] .

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. See *e.g.* [Masson et al. (1989) FEMS Microbiol. Lett. 60:273; Palva et al. (1982) Proc. Natl. Acad Sci. USA 79:5582; EP-A-0 036 259 and EP-A-0 063 953; WO 84/04541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69: 2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412, Streptococcus].

<u>v. Yeast Expression</u>

Yeast expression systems are also known to one of ordinary skill in the art. A yeast promoter is any DNA sequence capable of binding yeast RNA polymerase and initiating the downstream (3') transcription of a coding sequence (*e.g.* structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP- A- 0 284 044), enolase, glucokinase, glucose- 6- phosphate isomerase, glyceraldehyde- 3- phosphate- dehydrogenase (GAP or GAPDH), hexokinase, phosphofructokinase, 3- phosphoglycerate mutase, and pyruvate kinase (PyK) (EPO- A- 0 329 203) . The yeast *PHO5* gene, encoding acid phosphatase, also provides useful promoter sequences [Myanohara et al. (1983) Proc. Natl. Acad. Sci. USA 80: 1] .

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4, 876, 197 and 4, 880, 734) . Other examples of hybrid promoters include promoters which consist of the regulatory sequences of either the *ADH2, GAL4, GAL10,* OR *PHO5* genes, combined with the transcriptional activation region of a glycolytic enzyme gene such as GAP or PyK (EP- A- 0 164 556) . Furthermore, a yeast promoter can include naturally occurring promoters of non- yeast origin that have the ability to bind yeast RNA polymerase and initiate transcription. Examples of such promoters include, *inter alia,* [Cohen et al. (1980) Proc. Natl. Acad. Sci. USA 77: 1078; Henikoff et al. (1981) Nature 283: 835; Hollenberg et al. (1981) Curr. Topics Microbiol. Immunol. 96: 119; Hollenberg et al. (1979) "The Expression of Bacterial Antibiotic Resistance Genes in the Yeast Saccharomyces cerevisiae, " in: Plasmids of Medical, Environmental and Commercial Importance (eds. K.N. Timmis and A. Puhler) ; Mercerau- Puigalon et al. (1980) Gene 11: 163; Panthier et al. (1980) Curr. Genet. 2: 109;] .

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See *e.g.* EP-A-0 196 056. Another example is a ubiquitin fusion protein. Such a fusion protein is made with the ubiquitin region that preferably retains a site for a processing enzyme (*e.g.* ubiquitin-specific processing protease) to cleave the ubiquitin from the foreign protein. Through this method, therefore, native foreign protein can be isolated (*e.g.* WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the genes for invertase (EP- A- 0012873; JPO 62, 096, 086) and A- factor (US patent 4, 588, 684) . Alternatively, leaders of non- yeast origin exit, such as an interferon leader, that also provide for secretion in yeast (EP- A- 0060057) .

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha-factor leader fragment that provides for secretion include hybrid alpha-factor leaders made with a presequence of a first yeast, but a pro-region from a second yeast alphafactor. (*e.g.* see WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast- recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (*e.g.* plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast- bacteria shuttle vectors include YEp24 [Botstein et al. (1979) Gene 8: 17- 24], pCl/l [Brake et al. (1984) Proc. Natl. Acad. Sci USA 81: 4642- 4646], and YRp17 [Stinchcomb et al. (1982) J. Mol. Biol. 158: 157] . In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See *e.g.* Brake *et al., supra.*

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr- Weaver et al. (1983) Methods in Enzymol. 101: 228- 245] . An integrating vector may be directed to a specific locus in yeast by selecting the appropriate homologous sequence for inclusion in the vector. See Orr- Weaver *et al., supra.* One or more expression construct may integrate, possibly affecting levels of recombinant protein produced [Rine et al. (1983) Proc. Natl. Acad. Sci. USA 80: 6750] . The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LEU2, TRP1,* and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions [Butt et al. (1987) Microbiol, Rev. 51: 351] .

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, *inter alia,* the following yeasts: Candida albicans [Kurtz, et al. (1986) Mol. Cell. Biol. 6: 142], Candida maltosa [Kunze, et al. (1985) J. Basic Microbiol. 25: 141] . Hansenula polymorpha [Gleeson, et al. (1986) J. Gen. Microbiol. 132: 3459; Roggenkamp et al. (1986) Mol. Gen. Genet. 202: 302], Kluyveromyces fragilis [Das, et al. (1984) J. Bacteriol. 158: 1165], Kluyveromyces lactis [De Louvencourt et al. (1983) J. Bacteriol. 154: 737; Van den Berg et al. (1990) Bio/ Technology 8: 135], Pichia guillerimondii [Kunze et al. (1985) J. Basic Microbiol. 25: 141], Pichia pastoris [Cregg, et al. (1985) Mol. Cell. Biol. 5: 3376; US Patent Nos. 4, 837, 148 and 4, 929, 555], Saccharomyces cerevisiae [Hinnen et al. (1978) Proc. Natl. Acad. Sci. USA 75: 1929; Ito et al. (1983) J. Bacteriol. 153: 163], Schizosaccharomyces pombe [Beach and Nurse (1981) Nature 300: 706], and Yarrowia lipolytica [Davidow, et al. (1985) Curr. Genet. 10: 380471 Gaillardin, et al. (1985) Curr. Genet. 10: 49] .

Methods of introducing exogenous DNA into yeast hosts are well-known in the art, and usually include either the transformation of spheroplasts or of intact yeast cells treated with alkali cations. Transformation procedures usually vary with the yeast species to be transformed. See *e.g.* [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol 158:1165; De Louvencourt et al. (1983) J. Bacteriol. 154:1165; Van den Berg et al. (1990) Bio/Technology 8:135*; Kluyveromyces*]; [Cregg et al. (1985) Mol. Cell. Biol. 5: 3376; Kunze et al. (1985) J. Basic Microbiol. 25:141; US Patents 4,837,148 & 4,929,555; Pichia]; [Hinnen et al. (1978) Proc. Natl. Acad. Sci. USA 75;1929; Ito et al. (1983) J. Bacteriol. 153:163 Saccharomyces]; [Beach & Nurse (1981) Nature 300:706*; Schizosaccharomyces*]; [Davidow et al. (1985) Curr. Genet. 10:39; Gaillardin et al. (1985) Curr. Genet. 10:49; Yarrowia].

*Pharmaceutical Compositions*

Pharmaceutical compositions can comprise polypeptides and/or nucleic acid of the invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect.

The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgement of the clinician.

For purposes of the present invention, an effective dose will be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

*Delivery Methods*

Once formulated, the compositions of the invention can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*e.g.* see WO98/20734), needles, and gene guns or hyposprays. Dosage treatment may be a single dose schedule or a multiple dose schedule.

*Vaccines*

Vaccines according to the invention may either be prophylactic (*ie.* to prevent infection) or therapeutic (*ie.* to treat disease after infection).

Such vaccines comprise immunising antigen (s), immunogen (s), polypeptide (s), protein (s) or nucleic acid, usually in combination with "pharmaceutically acceptable carriers, " which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants") . Furthermore, the antigen or immunogen may be conjugated to a bacterial toxoid, such as a toxoid from diphtheria, tetanus, cholera, *H. pylori, etc.* pathogens.

Preferred adjuvants to enhance effectiveness of the composition include, but are not limited to: (1) aluminum salts (alum), such as aluminum hydroxide, aluminum phosphate, aluminum sulfate, etc; (2) oil- in- water emulsion formulations (with or without other specific immunostimulating agents such as muramyl peptides (see below) or bacterial cell wall components), such as for example (a) MF59™ (WO 90/14837; Chapter 10 in Vaccine design: the subunit and adjuvant approach, eds. Powell & Newman, Plenum Press 1995), containing 5% Squalene, 0.5% Tween 80, and 0.5% Span 85 (optionally containing various amounts of MTP- PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic- blocked polymer L 121, and thr- MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™) ; (3) saponin adjuvants, such as Stimulon™ (Cambridge Bioscience, Worcester, MA) may be used or particles generated therefrom such as ISCOMs (immunostimulating complexes) ; (4) Complete Freund's Adjuvant (CFA) and Incomplete Freund's Adjuvant (IFA) ; (5) cytokines, such as interleukins (*e.g.* IL- 1, IL- 2, IL- 4, IL- 5, IL- 6, IL- 7, IL- 12, *etc.*), interferons (*e.g.* gamma interferon), macrophage colony stimulating factor (M- CSF), tumor necrosis factor (TNF), etc; and (6) other substances that act as immunostimulating agents to enhance the effectiveness of the composition. Alum and MF59™ are preferred.

As mentioned above, muramyl peptides include, but are not limited to, N- acetyl- muramyl- L- threonyl- D- isoglutamine (thr- MDP), N- acetyl- normuramyl- L- alanyl- D- isoglutamine (nor- MDP), N- acetylmuramyl- L- alanyl- D- isoglutaminyl- L- alanine- 2- (1'- 2'- dipalmitoyl- sn- glycero- 3- hydroxyphosphoryloxy)- ethylamine (MTP- PE), *etc.*

The immunogenic compositions (*e.g.* the immunising antigen/immunogen/polypeptide/protein/ nucleic acid, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic or immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (*e.g.* nonhuman primate, primate, *etc.*), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, *e.g.* by injection, either subcutaneously, intramuscularly, or transdermally/transcutaneously (*e.g.* WO98/20734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

As an alternative to protein-based vaccines, DNA vaccination may be employed [e.g. Robinson & Torres (1997) Seminars in Immunology 9:271-283; Donnelly et al. (1997) Annu Rev Immunol 15:617-648; see later herein].

*Gene Delivery Vehicles*

Gene therapy vehicles for delivery of constructs including a coding sequence of a therapeutic of the invention, to be delivered to the mammal for expression in the mammal, can be administered either locally or systemically. These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The invention includes gene delivery vehicles capable of expressing the contemplated nucleic acid sequences. The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector. See generally, Jolly (1994) Cancer Gene Therapy 1:51-64; Kimura (1994) Human Gene Therapy 5:845-852; Connelly (1995) Human Gene Therapy 6:185-193; and Kaplitt (1994) Nature Genetics 6:148-153.

These recombinant retroviral vectors may be used to generate transduction competent retroviral vector particles by introducing them into appropriate packaging cell lines (see US patent 5, 591, 624) . Retrovirus vectors can be constructed for site- specific integration into host cell DNA by incorporation of a chimeric integrase enzyme into the retroviral particle (see WO96/37626) . It is preferable that the recombinant viral vector is a replication defective recombinant virus.

Packaging cell lines suitable for use with the above-described retrovirus vectors are well known in the art, are readily prepared (see WO95/30763 and WO92/05266), and can be used to create producer cell lines (also termed vector cell lines or "VCLs") for the production of recombinant vector particles. Preferably, the packaging cell lines are made from human parent cells (*e.g.* HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) J Virol 19:19-25), Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

The gene therapy vectors of the invention also include herpes vectors. Leading and preferred examples are herpes simplex virus vectors containing a sequence encoding a thymidine kinase polypeptide such as those disclosed in US 5, 288, 641 and EP0176170 (Roizman) . Additional exemplary herpes simplex virus vectors include HFEM/ICP6-LacZ disclosed in WO95/04139 (Wistar), pHSViac described in Geller (i988) Science 241: 1667- 1669 and in WO90/09441 & WO92/07945, HSV Us3:: pgC- lacZ described in Fink (1992) Human Gene Therapy 3: 11- 19 and HSV 7134, 2 RH 105 and GAL4 described in EP 0453242 (Breakefield), and those deposited with ATCC as accession numbers ATCC VR- 977 and ATCC VR- 260.

Also contemplated are alpha virus gene therapy vectors that can be employed in this invention. Preferred alpha virus vectors are Sindbis viruses vectors. Togaviruses, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in US Serial No. 08/405,627, filed March 15, 1995, WO94/21792, WO92/10578, WO95/07994, US 5,091,309 and US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see WO 1997/38087).

DNA vector systems such as eukaryotic layered expression systems are also useful for expressing the nucleic acids of the invention. See WO95/07994 for a detailed description of eukaryotic layered expression systems. Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature 339 (1989) 385 and Sabin (1973) J. Biol. Standardization 1:115; rhinovirus, for example ATCC VR-1110 and those described in Arnold (1990) J Cell Biochem L401; pox viruses such as canary pox virus or vaccinia virus, for example ATCC VR-111 and ATCC VR-2010 and those described in Fisher-Hoch (1989) Proc Natl Acad Sci 86:317; Flexner (1989) Ann NY Acad Sci 569:86, Flexner (1990) Vaccine 8:17; in US 4,603,112 and US 4,769,330 and WO89/01973; SV40 virus, for example ATCC VR-305 and those described in Mulligan (1979) Nature 277:108 and Madzak (1992) J Gen Virol 73:1533; influenza virus, for example ATCC VR-797 and recombinant influenza viruses made employing reverse genetics techniques as described in US 5,166,057 and in Enami (1990) Proc Natl Acad Sci 87:3802-3805; Enami & Palese (1991) J Virol 65:2711-2713 and Luytjes (1989) Cell 59:110, (see also McMichael (1983) NEJ Med 309:13, and Yap (1978) Nature 273:238 and Nature (1979) 277:108); human immunodeficiency virus as described in EP-0386882 and in Buchschacher (1992) J. Virol. 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; O'Nyong virus, Eastern encephalitis virus, for example ATCC VR-65 and ATCC VR-1242; Western encephalitis virus, for example ATCC VR-70, ATCC VR-1251, ATCC VR-622 and ATCC VR-1252; and coronavirus, for example ATCC VR-740 and those described in Hamre (1966) Proc Soc Exp Biol Med 121:190.

Delivery of the compositions of this invention into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) Hum Gene Ther 3:147-154 ligand linked DNA, for example see Wu (1989) J Biol Chem 264: 16985-16987, eucaryotic cell delivery vehicles cells, for example see US patent 6015686, deposition of photopolymerized hydrogel materials, hand-held gene transfer particle gun, as described in US Patent 5,149,655, ionizing radiation as described in US5,206,152 and in WO92/11033, nucleic charge neutralization or fusion with cell membranes. Additional approaches are described in Philip (1994) Mol Cell Biol 14:2411-2418 and in Woffendin (1994) Proc Natl Acad Sci 91: 1581-1585.

Particle mediated gene transfer may be employed, for example see WO 1998/06437. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) J. Biol. Chem. 262:4429-4432, insulin as described in Hucked (1990) Biochem Pharmacol 40:253-263, galactose as described in Plank (1992) Bioconjugate Chem 3:533-539, lactose or transferrin. Naked DNA may also be employed. Exemplary naked DNA introduction methods are described in WO90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in US 5, 422, 120, WO95/13796, WO94/23697, WO91/14445 and EP- 524, 968. As described in WO 1998/06437, on non- viral delivery, the nucleic acid sequences encoding a polypeptide can be inserted into conventional vectors that contain conventional control sequences for high level expression, and then be incubated with synthetic gene transfer molecules such as polymeric DNA- binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue- specific or ubiquitously- active promoters. Further non- viral delivery suitable for use includes mechanical delivery systems such as the approach described in Woffendin et al (1994) Proc. Natl. Acad. Sci. USA 91 (24) : 11581- 11585. Moreover, the coding sequence and the product of expression of such can be delivered through deposition of photopolymerized hydrogel materials. Other conventional methods for gene delivery that can be used for delivery of the coding sequence include, for example, use of hand- held gene transfer particle gun, as described in US 5, 149, 655; use of ionizing radiation for activating transferred gene, as described in US 5, 206, 152 and WO92/11033

Exemplary liposome and polycationic gene delivery vehicles are those described in US 5,422,120 and 4,762,915; in WO 95/13796; WO94/23697; and WO91/14445; in EP-0524968; and in Stryer, Biochemistry, pages 236-240 (1975) W.H. Freeman, San Francisco; Szoka (1980) Biochem Biophys Acta 600:1; Bayer (1979) Biochem Biophys Acta 550:464; Rivnay (1987) Meth Enzymol 149:119; Wang (1987) Proc Natl Acad Sci 84:7851; Plant (1989) Anal Biochem 176:420.

A polynucleotide composition can comprises therapeutically effective amount of a gene therapy vehicle, as the term is defined above. For purposes of the present invention, an effective dose will be from about 0.01 mg/ kg to 50 mg/kg or 0.05 mg/kg to about 10 mg/kg of the DNA constructs in the individual to which it is administered.

*Delivery Methods*

Once formulated, the polynucleotide compositions of the invention can be administered (1) directly to the subject; (2) delivered *ex vivo,* to cells derived from the subject; or (3) *in vitro* for recombinant protein expression. The subjects to be treated can be mammals or birds. Also, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*e.g.* see WO98/20734), needles, and gene guns or hyposprays. Dosage treatment may be a single dose schedule or a multiple dose schedule.

Methods for the *ex vivo* delivery and reimplantation of transformed cells into a subject are known in the art and described in *e.g.* WO93/14778. Examples of cells useful in ex vivo applications include, for example, stem cells, particularly hematopoetic, lymph cells, macrophages, dendritic cells, or tumor cells.

Generally, delivery of nucleic acids for both *ex vivo* and *in vitro* applications can be accomplished by the following procedures, for example, dextran- mediated transfection, calcium phosphate precipitation, polybrene mediated transfection, protoplast fusion, electroporation, encapsulation of the polynucleotide (s) in liposomes, and direct microinjection of the DNA into nuclei, all well known in the art.

*Polynucleotide and polypeptide pharmaceutical compositions*

In addition to the pharmaceutically acceptable carriers and salts described above, the following additional agents can be used with polynucleotide and/or polypeptide compositions.

A.Polypeptides

One example are polypeptides which include, without limitation: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

B.Hormones, Vitamins, *etc.*

Other groups that can be included are, for example: hormones, steroids, androgens, estrogens, thyroid hormone, or vitamins, folic acid.

C.Polyalkylenes, Polysaccharides, *etc.*

Also, polyalkylene glycol can be included with the desired polynucleotides/ polypeptides. In a preferred embodiment, the polyalkylene glycol is polyethlylene glycol. In addition, mono-, di-, or polysaccharides can be included. In a preferred embodiment of this aspect, the polysaccharide is dextran or DEAE- dextran. Also, chitosan and poly (lactide- co- glycolide)

D.Lipids, and Liposomes

The desired polynucleotide/polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present invention include cationic (positively charged), anionic (negatively charged) and neutral preparations. Cationic liposomes have been shown to mediate intracellular delivery of plasmid DNA (Felgner (1987) Proc. Natl. Acad. Sci. USA 84: 7413- 7416) ; mRNA (Malone (1989) Proc. Natl. Acad. Sci. USA 86: 6077- 6081) ; and purified transcription factors (Debs (1990) J. Biol. Chem. 265: 10189- 10192), in functional form.

Cationic liposomes are readily available. For example, N [1- 2, 3- dioleyloxy) propyl]- N, N, N- triethylammonium (DOTMA) liposomes are available under the trademark Lipofectin, from GIBCO BRL, Grand Island, NY. (See, also, Felgner *supra*) . Other commercially available liposomes include transfectace (DDAB/ DOPE) and DOTAP/ DOPE (Boerhinger) . Other cationic liposomes can be prepared from readily available materials using techniques well known in the art. See, *e.g.* Szoka (1978) Proc. Natl. Acad. Sci. USA 75: 4194- 4198; WO90/1109 for a description of the synthesis of DOTAP (1, 2- bis (oleoyloxy)- 3- (trimethylammonio) propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See *e.g.* Straubinger (1983) Meth. Immunol. 101:512-527; Szoka (1978) Proc. Natl. Acad. Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad. Sci. USA 76:145; Fraley (1980) J. Biol. Chem. (1980) 255:10431; Szoka & Papahadjopoulos (1978) Proc. Natl. Acad. Sci. USA 75:145; and Schaefer-Ridder (1982) Science 215:166.

E.Lipoproteins

In addition, lipoproteins can be included with the polynucleotide/polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.

Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.

A lipoprotein can comprise more than one apoprotein. For example, naturally occurring chylomicrons comprises of A, B, C, & E, over time these lipoproteins lose A and acquire C and E apoproteins. VLDL comprises A, B, C, & E apoproteins, LDL comprises apoprotein B; HDL comprises apoproteins A, C, & E.

The amino acid of these apoproteins are known and are described in, for example, Breslow (1985) Annu Rev. Biochem 54:699; Law (1986) Adv. Exp Med. Biol. 151:162; Chen (1986) J Biol Chem 261:12918; Kane (1980) Proc Natl Acad Sci USA 77:2465; and Utermann (1984) Hum Genet 65:232.

Lipoproteins contain a variety of lipids including, triglycerides, cholesterol (free and esters), and phospholipids. The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described in *Meth. Enzymol. (supra)*; Pitas (1980) J. Biochem. 255:5454-5460 and Mahey (1979) J Clin. Invest 64: 743-750. Lipoproteins can also be produced by *in vitro* or recombinant methods by expression of the apoprotein genes in a desired host cell. See, for example, Atkinson (1986) Annu Rev Biophys Chem 15:403 and Radding (1958) Biochim Biophys Acta 30: 443. Lipoproteins can also be purchased from commercial suppliers, such as Biomedical Technologies, Inc., Stoughton, Massachusetts, USA. Further description of lipoproteins can be found in Zuckermann et al. WO98/06437.

F.Polycationic Agents

Polycationic agents can be included, with or without lipoprotein, in a composition with the desired polynucleotide/polypeptide to be delivered.

Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, etc.

The following are examples of useful polypeptides as polycationic agents: polylysine, polyarginine, polyornithine, and protamine. Other examples include histones, protamines, human serum albumin, DNA binding proteins, non-histone chromosomal proteins, coat proteins from DNA viruses, such as (X174, transcriptional factors also contain domains that bind DNA and therefore may be useful as nucleic aid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that bind DNA sequences.

Organic polycationic agents include: spermine, spermidine, and purtrescine.

The dimensions and of the physical properties of a polycationic agent can be extrapolated from the list above, to construct other polypeptide polycationic agents or to produce synthetic polycationic agents.

Synthetic polycationic agents which are useful include, for example, DEAE-dextran, polybrene. Lipofectin™, and lipofectAMINE™ are monomers that form polycationic complexes when combined with polynucleotides/polypeptides.

*Nucleic Acid Hybridisation*

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al.* [*supra*] vol.2, chapt. 9, pp.9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in which samples of genomic DNA immobilized on filters are hybridized to the sequence of interest and then washed under conditions of different stringencies. See Sambrook *et al.* at page 9.50.

Variables to consider when performing, for example, a Southern blot are (1) the complexity of the DNA being blotted and (2) the homology between the probe and the sequences being detected. The total amount of the fragment (s) to be studied can vary a magnitude of 10, from 0.1 to 1$\mu$g for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only 1 hour starting with 1 $\mu$g of yeast DNA, blotting for two hours, and hybridizing for 4-8 hours with a probe of $10^8$ cpm/$\mu$g. For a single-copy mammalian gene a conservative approach would start with 10 $\mu$g of DNA, blot overnight, and hybridize overnight in the presence of 10% dextran sulfate using a probe of greater than $10^8$ cpm/$\mu$g, resulting in an exposure time of ~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation:

$$Tm = 81 + 16.6(\log_{10} Ci) + 0.4[\%(G + C)] - 0.6(\% formamide) - 600/n - 1.5(\% mismatch).$$

where Ci is the salt concentration (monovalent ions) and *n* is the length of the hybrid in base pairs (slightly modified from Meinkoth & Wahl (1984) Anal. Biochem. 138: 267-284).

In designing a hybridization experiment, some factors affecting nucleic acid hybridization can be conveniently altered. The temperature of the hybridization and washes and the salt concentration during the washes are the simplest to adjust. As the temperature of the hybridization increases (ie. stringency), it becomes less likely for hybridization to occur between strands that are nonhomologous, and as a result, background decreases. If the radiolabelled probe is not completely homologous with the immobilized fragment (as is frequently the case in gene family and interspecies hybridization experiments), the hybridization temperature must be reduced, and background will increase. The temperature of the washes affects the intensity of the hybridizing band and the degree of background in a similar manner. The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If non-specific bands or high background are observed after autoradiography, the filter can be washed at high stringency and reexposed. If the time required for exposure makes this approach impractical, several hybridization and/or washing stringencies should be tested in parallel.

*Nucleic Acid Probe Assays*

Methods such as PCR, branched DNA probe assays, or blotting techniques utilizing nucleic acid probes according to the invention can determine the presence of cDNA or mRNA. A probe is said to "hybridize" with a sequence of the invention if it can form a duplex or double stranded complex, which is stable enough to be detected.

The nucleic acid probes will hybridize to the Neisserial nucleotide sequences of the invention (including both sense and antisense strands). Though many different nucleotide sequences will encode the amino acid sequence, the native Neisserial sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the Neisserial sequence (or its complement) - some variation in the sequence and length can lead to increased assay sensitivity if the nucleic acid probe can form a duplex with target nucleotides, which can be detected. Also, the nucleic acid probe can include additional nucleotides to stabilize the formed duplex. Additional Neisserial sequence may also be helpful as a label to detect the formed duplex. For example, a non-complementary nucleotide sequence may be attached to the 5' end of the probe, with the remainder of the probe sequence being complementary to a Neisserial sequence. Alternatively, non-complementary bases or longer sequences can be interspersed into the probe, provided that the probe sequence has sufficient complementarity with the a Neisserial sequence in order to hybridize therewith and thereby form a duplex which can be detected.

The exact length and sequence of the probe will depend on the hybridization conditions, such as temperature, salt condition and the like. For example, for diagnostic applications, depending on the complexity of the analyte sequence, the nucleic acid probe typically contains at least 10-20 nucleotides, preferably 15-25, and more preferably ≥30 nucleotides, although it may be shorter than this. Short primers generally require cooler temperatures to form sufficiently stable hybrid complexes with the template.

Probes may be produced by synthetic procedures, such as the triester method of Matteucci et al. [J. Am. Chem. Soc. (1981) 103:3185], or according to Urdea et al. [Proc. Natl. Acad. Sci. USA (1983) 80: 7461], or using commercially available automated oligonucleotide synthesizers.

The chemical nature of the probe can be selected according to preference. For certain applications, DNA or RNA are appropriate. For other applications, modifications may be incorporated e.g. backbone modifications, such as phosphorothioates or methylphosphonates, can be used to increase *in vivo* half-life, alter RNA affinity, increase nuclease resistance *etc.* [*e.g.* see Agrawal & Iyer (1995) Curr Opin Biotechnol 6:12-19; Agrawal (1996) TIBTECH 14:376-387]; analogues such as peptide nucleic acids may also be used [*e.g.* see Corey (1997). TIBTECH 15:224-229; Buchardt et al. (1993) TIBTECH 11:384-386].

Alternatively, the polymerase chain reaction (PCR) is another well- known means for detecting small amounts of target nucleic acids. The assay is described in: Mullis et al. [Meth. Enzymol. (1987) 155: 335- 350] ; US patents 4, 683, 195 & 4, 683, 202. Two 'primers' hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired Neisserial sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labelled probe will hybridize to the Neisserial sequence (or its complement).

Also, mRNA or cDNA can be detected by traditional blotting techniques described in *Sambrook et al* [*supra*] . mRNA, or cDNA generated from mRNA using a polymerase enzyme, can be purified and separated using gel electrophoresis. The nucleic acids on the gel are then blotted onto a solid support, such as nitrocellulose. The solid support is exposed to a labelled probe and then washed to remove any unhybridized probe. Next, the duplexes containing the labelled probe are detected. Typically, the probe is labelled with a radioactive moiety.

**BRIEF DESCRIPTION OF THE DRAWINGS**

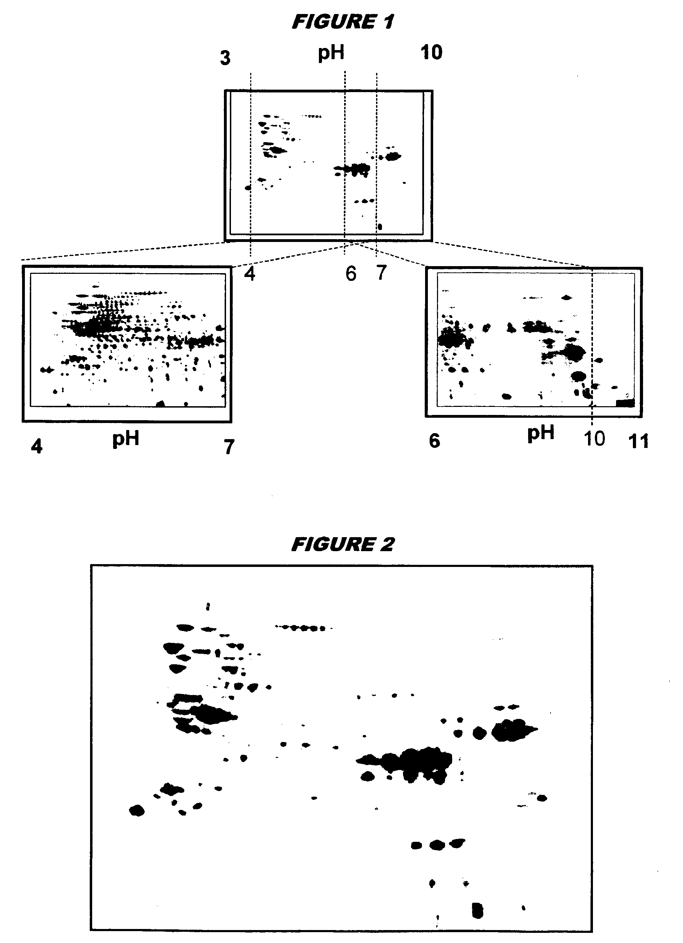

Figure 1 shows the overall strategy for 2D-PAGE. The individual gels are shown as Figures 2 to 4. Figure 5 shows the results of a protein query of the NCBI Entrez system using the term 'NMB1506'.

**MODES FOR CARRYING OUT THE INVENTION**

The overall strategy for identifying membrane proteins was to use two dimensional electrophoresis to separate the proteins in a *N.meningitidis* membrane preparation, followed by identification of proteins found in the electrophoretic gel. This experiment was not straightforward because, as is well known, preparing a membrane fraction which is not contaminated with cytoplasmic proteins is extremely difficult.

Some of the proteins identified by electrophoresis were expected to be there, but many were not. Furthermore, several proteins which were annotated as "hypothetical" have now been shown to exist *in vivo* - they are not artefacts. A further point to note is that proteins visible on a 2D gel must be expressed at least at a moderate level, thus making them useful targets for binding.

*Preparation of membrane fraction*

To prepare the membrane fraction, *N.meningitidis* strain MC58 was growth in liquid "base for the isolation of pathogenic Neisseria" (GC, Difco), supplemented with 4g/l glucose, 0.1 g/l glutamine, and 2.2 mg/l cocarboxylase, at 37°C in a humidified atmosphere containing 5% $CO_2$. Bacteria were harvested at $OD_{600nm}$=0.5 by centrifugation at 10,000 g for 20 minutes. The pellet was resuspended with 10 ml Tris-base containing the EDTA-free protease inhibitor cocktail used according to the manufacturer (Roche Diagnostic Mannhein, Germany). The bacteria were inactivated 45 minutes at 56°C, cooled in ice in presence of 1,000 U benzonase, and were disrupted with a French Press at 18000 psi (4 passages). The lysate was centrifuged at 70,000 g overnight at 5°C. The pellet was washed twice with 40mM Tris-base and resuspended in 10 ml of 5M urea, 2M thiourea, 2% (w/v) CHAPS, 2% (w/v) ASB 14, 0.5% (v/v) C.A., 2mM TBP and centrifuged at 100,000 g at 10°C for 3 hours. Supernatant containing solubilised membrane proteins was aliquoted and stored at -80°C.

This process is quick, easy to use, and results in a membrane preparation with little or no contamination from cytosolic proteins [*e.g.* refs. 112 to 115]. It was improved by using new techniques for the solubilisation of bacterial membrane proteins prior and during to isoelectric focusing, in order to optimise the separation of outer membrane proteins by two-dimensional electrophoresis (2DE). Thirourea was thus used with the urea as chaotropic agents [112], the zwitterionic detergent ASB-14 was used in association with CHAPS [116], and tributyl phosphine was added prior to 2DE [117].

*2D electrophoresis*

200μg of the membrane proteins were brought to a final volume of 125 μl with reswelling solution (7M urea, 2M thiourea, 2% (w/v) CHAPS, 2% (w/v) ASB 14, 2% (v/v) adequate ampholine (Amersham Pharmacia Biotech (Piscataway, NJ), 2 mM thiobutyl phosphine). The proteins were absorbed overnight onto an Immobiline DryStrip (7 cm; pH-gradient 3-10 non linear, pH-gradient, 4-7 or 6-11 linear) using the Immobiline Dry-Strip Reswelling Tray (Amersham Pharmacia Biotech (Piscataway, NJ). Proteins were then separated by 2D electrophoresis. The first dimension was run on an IPGphor Isoelectric Focusing Unit (Amersham Pharmacia Biotech, Piscataway, NJ), The proteins were separated applying sequentially 150 V for 35 min., 500 V for 35 min., 1000 V for 30 min, 2600 V for 10 min., 3500 V for 15 min., 4200 V for 15 min., and then 5000 V to reach 10kVh. For the second dimension, the strips were equilibrated as described in ref. 117 and the proteins were separated on a linear 9-16% acrylamide SDS-PAGE (1.5 mm thick, 6 cm high) using the Mini Proten II Cell from BioRad. The 2D gel was stained with colloidal Coomassie [118]. Gels were scanned with a Personal densitometer SI (Molecular Dynamics) at 12 bits and 50 μm per pixel.

The initial 2D IPG gradient gel is shown in Figure 2. 325 protein spots were visualised by colloidal Coomassie blue. In order to increase the spot number, 200μg of the same protein fraction was separated on 2D gels with a pH gradients of 4-7 (Figure 3) and 6-11 (Figure 4). 498 and 187 spots were visualised by colloidal Coomassie blue in these gels, respectively. Each gel was performed and analysed in duplicate, with no major differences observed between duplicates.

*Spot excision*

A total of 1,867 protein spots were excised from the three gels, plus the duplicated gels, in-gel digested with trypsin, and the generated peptides were analysed by MALD1-TOF mass spectrometry. Proteins were identified by interpreting their peptide mass fingerprint using the software Mascot.

The protocol used for in-gel digestion was a modification of reference 119. Protein spots were excised from the gel, washed sequentially with Milli-Q water and acetonitrile (1/1, v/v), and dried by a Speed Vac vacuum centrifuge apparatus (Savant, Holbrook, NY). The pieces of gel were re-hydrated by adding 7-10 μl of a solution of 50 mM ammonium bicarbonate, 5 mM $CaCl_2$ containing 12 mg/l trypsin sequencing grade (Roche Diagnostic Corporation, Indianapolis, IN). The tryptic digestion was allowed to run for 18 hours at 37°C. Following digestion, the peptides were extracted by sonication in a sonicator bath with 50 μl 50% acetonitrile, 5% TFA for 30 min. The extraction was repeated, extract solutions were pooled together and the volume was reduced to 10 μl in a Speed Vac vacuum centrifuge apparatus. The samples were automatically prepared for mass spectrometry using the MAP II (Bruker, Bremen, Germany). The instrument was programmed to perform a desalting of the sample with ZIP-TIP (C18, Millipore). The peptides were eluted from the ZIP TIP with a solution of 5 g/! of 2,5-dihydroxybenzoic acid in 50% acetonitrile/0,1% TFA and directly loaded onto the Anchorchip (400 μm, Bruker, Bremen, Germany), The samples were allowed to air dry at room temperature.

*Excised spot analysis*

MALDI- TOF spectra were acquired on a Bruker Biflex II MALDI- TOF (Bremen, Germany) equipped with the SCOUT 381 multiprobe ion source in a positive- ion reflector mode. The acceleration voltage was set to 19 kV and the reflector voltage was set to 20 kV. Typically about 100 laser shots were average per spectrum from a 337 nm N2 laser. Spectra were externally calibrated using a combination of angiotensin II (1, 046.54 Da), substance P (1, 347.74 Da), Bombensin (1, 619.82 Da) and Acts18- 39 (Clip human, 2, 465.20 Da) . Peptides were selected in the mass range of 700- 3000 Da. Resulting values for monoisotopic peaks were used for search using the software Mascot run on a database containing the genome information of *N.meningitidis* and *C.pneumoniae.* All the searches were performed using a window of 500 ppm as constraint, allowing one missed cleavage, and considering propiamide cysteines as fixed modification, and oxidized methionine as variable modification. Signals generated from keratin digestion or trypsin autolysis were not considered for the search in the database. Proteins were successfully identified when the MOWSE score was superior to 47. When proteins were identified with an inferior Mowse score, Post Source Decay (PSD) experiments were performed to confirm the identification. PSD spectra of the parent ions were built by pasting the spectra obtain with successive reflector voltage from 20 to 0.59 V. Within the Figure 2 gel, 261/325 spots were identified by peptide mass fingerprint. When the same protein was identified in two or more samples from a train of spots clearly arising from isoforms of the same protein, the identification was extended to cover all spots in this row. When needed, the identification of the proteins were confirmed by PSD experiments, mainly for protein with a Mr inferior to 15 kDa. The remaining 64/325 spots contained too little protein material to give an unambiguous identification. The identified 261 spots were encoded by 138 separate genes, of which 11 were confirmed by PSD experiments. An additional 18 proteins were identified from the duplicated gel.

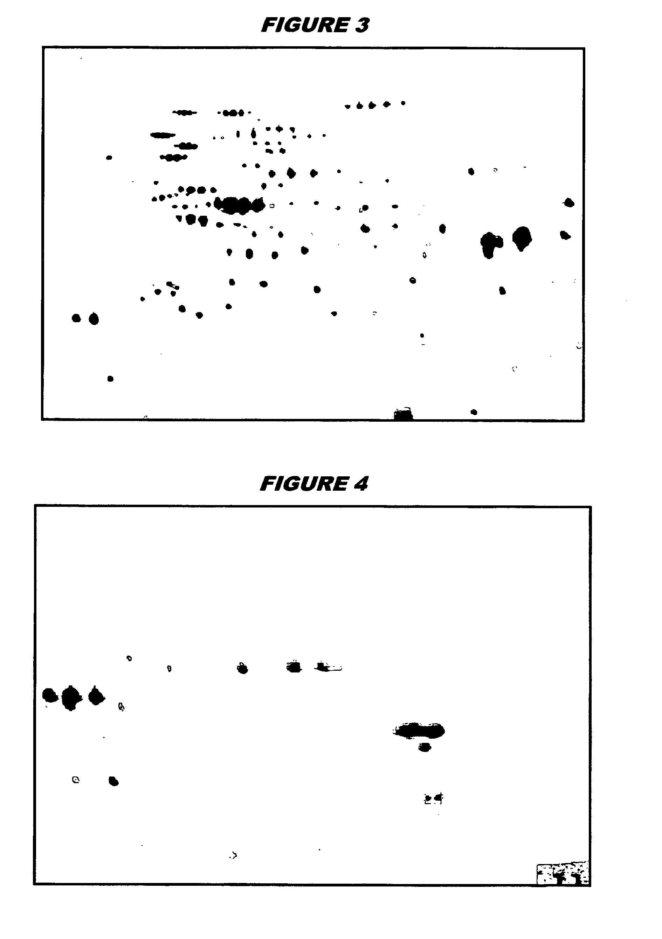

The Figure 3 gel allowed the identification of 194 unique proteins, of which 72 were not identified from the Figure 2 gel. Similarly, the Figure 4 gel allowed the identificaiton of 87 unique proteins of which 26 were not identified from the Figure 2 gel.

The identified proteins are listed in Table 1, with columns indicating which gel they were found in. In summary, among the 1,867 proteins spots processed, 1,433 proteins were identified. The identified spots represent 250 unique proteins. Only 8.8% of the proteins were identified from the pH 6-11 gels which confirms the difficulty of separating very basic proteins by 2DE. The identified proteins cover 11.58% of the predicted ORFs encoded by the genome [1].

Within the 250 proteins, 45 acted as positive controls, as they had previously been identified as useful vaccine/ diagnostic antigens in references 120 to 122. These 45 proteins are indicated with a '+' in Table 1. The remaining 205 proteins are listed as SEQ ID NOs I to 205.

Within the remaining 205 proteins, 138 completely elude the PSORT subcellular localisation algorithm [123, 124]. These 138 are marked with a '*' in Table 1, and it is particularly surprising to find these proteins in the membrane. Of these 138, 76 elude various algorithmic predictions, and this subset is marked '**'.

*OMV analysis*

In a separate series of experiments, outer membrane vesicles from serogroup B strains 394/98 (New Zealand) and H44/76 (Norway) were proteomically analysed. Sera from humans immunised with one of the two OMV preparations were analysed, and nine specific immunogenic proteins were identified: NMB 0182, 0382, 0634, 0763, 1126, 1342, 1429, 1799 & 2039. Of these 9 proteins: NMB 0382, 0634, 0763, 1342, 1429 & 2039 are in common with SEQ IDs 1 to 205; NMB0182 is a known immunogen [125]; NMB1126 (gi:7226362; SEQ ID 206) is annotated as 'hypothetical protein', having been identified only through GLIMMER2 prediction, and was not previously recognised as an immunogen; NMB1799 (gi:7227052; SEQ ID 207) is annotated as 'S-adenosylmethionine synthetase', which is a metabolic enzyme which would not normally be expected to be immunoaccessible. SEQ IDs 208-217 were not previously recognised as immunogens.

Proteins which were present in the 394/98 OMVs, but not in the H44/76 OMVs, were: 0007, 0018, 0031, 0051, 0052, 0089, 0110, 0130, 0132, 0168, 0214, 0336, 0375, 0423, 0427, 0462, 0554, 0586, 0604, 0610, 0623, 0626, 0634, 0652, 0758, 0854, 0920, 0944, 0954, 0955, 0959, 0983, 1046, 1055, 1127, 1153, 1191, 1199, 1201, 1228, 1240, 1285, 1313, 1332, 1339, 1358, 1392, 1437, 1457, 1460, 1506, 1518, 1533, 1554, 1572, 1577, 1594, 1612, 1642, 1668, 1684, 1710, 1762, 1796, 1812, 1849, 1854, 1855, 1861, 1874, 1903, 1921, 1949, 1953, 2086, 2102, 2129, 2134, 2138, 2159.

Proteins which were present in the H44/76 OMVs, but not in the 394/98 OMVs, were: 0143, 0204, 0280, 0313, 0410, 0477, 0546, 0638, 0889, 1011, 1124, 1162, 1342, 1540, 1714, 1799, 1808, 1969, 1998, 2069, 2103, 2154.

Proteins which were present in both OMV preparations were: 0035, 0088, 0109, 0124, 0126, 0138, 0139, 0154, 0157, 0171, 0182, 0219, 0359, 0382, 0387, 0426, 0461, 0595, 0617, 0618, 0631, 0663, 0703, 0757, 0763, 0787, 0875, 0876, 0943, 0946, 0957, 1053, 1126, 1131, 1252, 1301, 1323, 1341, 1429, 1445, 1497, 1567, 1574, 1576, 1869, 1934, 1936, 1946, 1972, 1988, 2039, 2096, 2101.

**TABLE 1**

| | NMB | ID (gi:722xxxx): name / MW / pI | Gel (Fig.) 2 | Gel (Fig.) 3 | Gel (Fig.) 4 | Note |
|---|---|---|---|---|---|---|
| 1 | 0010 | 5235: phosphoglycerate kinase / 41902 / 5.12 | | X | | |
| 2 | 0018 | 5244: pilin PilE / 15 246 / 9.21 | X | | X | |
| 3 | 0030 | 5249: methionyl-tRNA synthetase / 76 853 / 5.44 | | X | | |
| 4 | 0035 | 5255: conserved hypothetical protein / 40 21814.74 | X | X | | + |
| 5 | 0038 | 5258: UDP-N-acetylglucosamine pyrophosphorylase / 49 013/6.48 | X | | X | |
| 6 | 0045 | 5266: signal recognition particle protein / 45142 / 4.85 | | X | | * |
| 7 | 0051 | 5271: twitching motility protein / 45 675 / 6.51 | X | | X | * |
| 8 | 0052 | 5272: twitching motility protein PilT / 38 050 /6.61 | X | | X | ** |
| 9 | 0068 | 5288: polysialic acid capsule biosynthesis protein SiaC / 38821 / 5.35 | | X | | * |
| 10 | 0088 | 5306. outer membrane protein P1, putative / 45 902 / 9.35 | X | | X | + |
| 11 | 0089 | 5307: pyruvate kinase II / 52 435 / 5;27 | X | X | | * |
| 12 | 0109 | 5324: conserved hypothetical protein / 43 188 / 6.77 | X | | X | * |
| 13 | 0115 | 5331: nitrogen assimilation regulatory protein NtrX / 46373 / 4.98 | X | | | ** |
| 14 | 0124/0139 | 5341-5357: translation elongation factor TU / 42 909 - 42 925/ 5.07 | X | X | X | ** |
| 15 | 0126 | 5343: transcription antitermination protein NusG / 20 550 / 6.03 | X | X | | ** |
| 16 | 0128 | 5345: 50S ribosomal protein L1 / 24 102 / 9.59 | X | X | X | * |
| 17 | 0130 | 5347: 50S ribosomal protein L10 / 17 594 / 7.91 | X | X | X | ** |
| 18 | 0131 | 5348 : 50S ribosomal protein L7/L12 / 12623 / 4.60 | | X | | |
| 19 | 0132 | 5349: DNA-directed RNA polymerase, beta subunit / 155 945 / 5.34 | X | X | X | |
| 20 | 0133 | 5351: DNA-directed RNA polymerase, beta' subunit / 154660 / 6.86 | | | X | * |
| 21 | 0137 | 5355: 30S ribosomal protein S7 / 10646 / 10.33 | | | X | * |
| 22 | 0138 | 5356: elongation factor G (EF-G) / 77 244 / 5.08 | X | X | X | ** |
| 23 | 0140 | 5358: 30S ribosomal protein S10 / 11799/ 7.57 | | | X | ** |
| 24 | 0142 | 5360: 50S ribosomal protein L3 / 22664 / 10.03 | | | X | ** |
| 25 | 0143 | 5361: 50S ribosomal protein L4 / 23248 / 9.92 | | | X | ** |
| 26 | 0144 | 5362: 50S ribosomal protein L23 / 11268 / 9.85 | | | X | |
| 27 | 0154 | 5372: 50S ribosomal protein L5 / 20 323/ 9.49 | X | X | X | ** |
| 28 | 0156 | 5374: 30S ribosomal protein S8 / 1425/ 9.73 | | | X | * |
| 29 | 0157 | 5375: 50S ribosomal protein L6 / 18 892 / 9.63 | | X | X | * |

(continued)

| | NMB | ID (gi:722xxxx): name / MW / pI | Gel (Fig.) | | | Note |
|---|---|---|---|---|---|---|
| | | | 2 | 3 | 4 | |
| 30 | 0159 | 5377: 30s ribosomal protein S5 / 18235 / 10.04 | | | X | * |
| 31 | 0168 | 5386: DNA-directed RNA polymerase, alpha subunit / 36 076 / 4.94 | X | X | X | ** |
| 32 | 0171 | 5389: septum site-determining protein MinD / 29 559 / 5.70 | X | X | | ** |
| 33 | 0173 | 5391: transcriptional regulator, LysR family /34 071 /5.74 | X | X | | |
| 34 | 0181 | 5400: outer membrane protein OmpH, putative / 19261 / 9.23 | | X | | + |
| 35 | 0182 | 5401: outer membrane protein Omp85 / 86 254 / 8.37 | X | | X | + |
| 36 | 0191 | 5411. ParA family protein / 27150 / 5.53 | X | | | + |
| 37 | 0193 | 5413: glucose inhibited division protein A / 69 808 / 6.57 | X | | X | * |
| 38 | 0196 | 5417: ribonuclease E / 102087 / 5.97 | | X | X | * |
| 39 | 0212 | 5433: DNA gyrase subunit B / 88 188 / 5.46 | X | X | | * |
| 40 | 0214 | 5436: oligopeptidase A / 76 054 / 5.16 | X | X | | + |
| 41 | 0219 | 5441: 3-oxoacyl-(acyl-carrier-protein) synthase II / 43 221 / 5.36 | | X | | |
| 42 | 0246 | 5468: NADH dehydrogenase I, F subunit / 48007 / 5.66 | | X | | * |
| 43 | 0278 | 5503. thiol:disulfide interchange protein DsbA / 23 228 / 5.16 /18 | X | X | X | + |
| 44 | 0286 | 5510: conserved hypothetical protein / 22120 / 5.04 | | X | | * |
| 45 | 0292 | 5516: conserved hypothetical protein / 2208015.37 | | X | | + |
| 46 | 0294 | 5518: thiol:disulfide interchange protein DsbA 23 566 / 5.09 lipo | X | X | X | |
| 47 | 0329 | 5549: type IV pilus assembly protein / 61 965 / 5.42 | X | X | | * |
| 48 | 0335 | 5555: 2,3,4,5-tetrahydropyridine-2-carboxylate N-succinytransferase / 29 410 / 5.42 | X | X | | ** |
| 49 | 0336 | 5556: enoyl-(acyl-carrier-protein) reductase / 25 252 / 5.45 / 22 | | X | | |
| 50 | 0337 | 5557: branched-chain amino acid aminotransferase, putative / 36421 / 5.80 | | X | | |
| 51 | 0345 | 5566: cell-binding factor, putative / 31507 / 9.23 | | | X | + |
| 52 | 0351 | 5572: transaldolase / 37 578 / 5.09 | | X | | ** |
| 53 | 0359 | 5581: glutamate--ammonia ligase / 52137 / 5.20 | X | X | X | * |
| 54 | 0382 | 5603: outer membrane protein class 4 / 23 969 / 6.26 | X | X | X | |
| 55 | 0387 | 5608:ABC transporter, ATP-binding protein /62 073 / 5.23 | | X | X | + |
| 56 | 0390 | 5612: maltose phosphorylase / 85 490 / 5.36 | X | X | | * |
| 57 | 0391 | 5613: beta-phosphoglucomutase / 23728 / 5.08 | | X | | |

(continued)

| | NMB | ID (gi:722xxxx): name / MW / pI | Gel (Fig.) | | | Note |
|---|---|---|---|---|---|---|
| | | | 2 | 3 | 4 | |
| 58 | 0426 | 5649: cell division protein FtsA / 44 061 / 5,33 | X | X | | |
| 59 | 0427 | 5651: cell division protein FtsZ / 41 487 / 4.94 | X | | | |
| 60 | 0446 | 5671: chorismate mutase/prephenate dehydratase / 39778 / 5.65 | | X | | |
| 61 | 0462 | 5689: spermidine/putrescine ABC transporter, periplasmic ... / 50 556 / 6.92 | X | X | | |
| 62 | 0466 | 5693. aspartyl-tRNA synthetase / 68125 / 5.28 | X | X | X | ** |
| 63 | 0477 | 5705: conserved hypothetical protein / 19758 / 5.19 | | X | | ** |
| 64 | 0530 | 5755: glycosyl hydrolase, family 3 / 39554 / 5.39 | | X | | ** |
| 65 | 0537 | 5762: conserved hypothetical protein / 31 150 / 4.77 | | X | | + |
| 66 | 0540 | 5765: aspartate aminotransferase / 43 929 / 6.52 | X | X | X | ** |
| 67 | 0546 | 5772: alcohol dehydrogenase, propanol-preferring / 36 548 / 5.65 | X | X | | * |
| 68 | 0550 | 5777: thiol:disulfide interchange protein DsbC / 26 451 / 6.93 | X | | | + |
| 69 | 0554 | 5780: dnaK protein / 68 792 / 4.85 | X | X | X | + |
| 70 | 0564 | 5791: Na(+)-translocating NADH-quinone reductase, subunit F / 42 303 / 4.95 | X | X | | |
| 71 | 0569 | 5796: Na(+)-translocating NADH-quinone reductase, subunit A /48 636 /6.20 | X | X | X | + |
| 72 | 0589 | 5819: 50s ribosomal protein L19 / 13760 / 10.45 | | | X | ** |
| 73 | 0595 | 5825: DNA-binding response regulator / 24 780 / 5.44 | X | X | | ** |
| 74 | 0604 | 5834: alcohol dehydrogenase, zinc-containing / 38394 / 5.47 | | X | | |
| 75 | 0610 | 5840: spermidine/putrescine ABC transporter, ATP-binding protein / 47 425 / 6.03 | X | X | | * |
| 76 | 0617 | 5847: transcription termination factor Rho / 47 306 / 6.23 | X | | X | ** |
| 77 | 0618 | 5848:phosphoenolpyruvate synthase / 87 170 / 5.01 | X | X | X | ** |
| 78 | 0623 | 5854: spermidine/putrescine ABC transporter, periplasmic ... / 39 511 / 5.38 | X | X | | |
| 79 | 0626 | 5856: peptide chain release factor 3 / 59 589 / 5.43 | | X | | + |
| 80 | 0631 | 5861: phosphate acetyltransferase Pta, putative / 50306 / 4.83 | X | X | | |
| 81 | 0634 | 5862: iron(III) ABC transporter, periplasmic binding protein / 35806 / 9.60 | | | X | |
| 82 | 0637 | 5866: argininosuccinate lyase /51 245 / 5,22 | X | X | | ** |
| 83 | 0641 | 5870: inorganic pyrophosphatase / 19 812 / 4.71 | | X | | ** |
| 84 | 0663 | 5888: outer membrane protein NsgA / 18386 / 9.64 | | | X | |
| 85 | 0667 | 5893: hypothetical protein / 45 292 / 5,97 / 16 | X | X | | + |

(continued)

| | NMB | ID (gi:722xxxx): name / MW / pI | Gel (Fig.) | | | Note |
|---|---|---|---|---|---|---|
| | | | 2 | 3 | 4 | |
| 86 | 0697 | 5925: dimethyladenosine transferase / 2926917.11 | | | X | ** |
| 87 | 0703 | 5932 : competence lipoprotein ComL / 29 273 / 8.72 | X | | X | |
| 88 | 0711 | 5938: conserved hypothetical protein / 33516 / 6.11 | | X | | ** |
| 89 | 0720 | 5946: threonyl-tRNA synthetase / 72 692 / 5,86 | X | X | | * |
| 90 | 0724 | 5951: phenylalanyl-tRNA synthetase, alpha chain / 37 334 / 5.46 | X | X | X | ** |
| 91 | 0728 | 5955: phenylalanyl-tRNA synthetase, beta chain / 86 050 / 5.19 | X | X | | * |
| 92 | 0743 | 5972: ubiquinone/menaquinone biosynthesis methlytransferase UbiE / 27 366 / 7.14 | X | | X | ** |
| 93 | 0757 | 5987: phosphoribosylaminoimidazole-succinocarboxamide synthase / 32476 / 5.26 | | X | X | ** |
| 94 | 0758 | 5988: polyribonucleotide nucleotidyltransferase / 76 422 / 5.35 | X | X | X | |
| 95 | 0763 | 5993: cysteine synthase / 32 821 / 6.06 | X | X | | * |
| 96 | 0768 | 5999: twitching motility protein PilT / 41 508 / 7.64 | X | X | X | + |
| 97 | 0778 | 6009: uroporphyrin-III C-methyltransferase HemX, putative / 46 320/5,33 | X | X | | |
| 98 | 0787 | 6019: amino acid ABC transporter, periplasmic amino acid-binding protein / 26 995 / 5.42 | X | X | | + |
| 99 | 0791 | 6024 : peptidyl-prolyl cis-trans isomerase / 181853 / 5.05 | | X | | ** |
| 100 | 0798 | 6031: cell division protein FtsH / 70 058 / 5,07 /20 | X | | | + |
| 101 | 0801 | 6034: delta-aminolevulinic acid dehydratase / 36878 / 5.23 | | X | | + |
| 102 | 0804 | 6038: NAD(P)H nitroreductase, putative / 24687 / 6.17 | | X | | * |
| 103 | 0814 | 6048: histidyl-tRNA synthetase / 41746 / 5,45 | X | X | | ** |
| 104 | 0815 | 6050: adenylosuccinate synthetase / 46 250 / 5,77 | X | X | X | * |
| 105 | 0828 | 6063: ADP-L-glycero-D-mannoheptose-6-epimerase / 38 437 / 5,68 | X | X | | |
| 106 | 0853 | 6089: conserved hypothetical protein / 23136 / 5.67 | | X | | + |
| 107 | 0854 | 6090: histidyl-tRNA synthetase / 48 465 / 5.57 | X | X | | ** |
| 108 | 0875 | 6113: ribose-phosphatepyrophosphokinase / 35 598 /5.41 | X | X | | ** |
| 109 | 0876 | 6114: 50S ribosomal protein L25 / 20 956 / 6.60 | X | X | X | * |
| 110 | 0878 | 6116: threonine dehydratase / 55585 / 5.41 | | X | | |
| 111 | 0884 | 6122: superoxide dismutase / 21 893 / 5.78 | X | X | | * |
| 112 | 0885 | 6124: replicative DNA helicase / 52 089 / 5,04 | X | X | | ** |
| 113 | 0889 | 6127: hypothetical protein / 21970 / 6.40 | | X | | + |

(continued)

| | NMB | ID (gi:722xxxx): name / MW / pI | Gel (Fig.) | | | Note |
|---|---|---|---|---|---|---|
| | | | 2 | 3 | 4 | |
| 114 | 0894 | 6131: aminotransferase, class I / 44 013 / 5.79 | | X | | ** |
| 115 | 0920 | 6158 : isocitrate dehydrogenase / 80163 / 5.57 | X | X | X | * |
| 116 | 0928 | 6166: hypothetical protein /43 815 / 9.01 | X | | | + |
| 117 | 0944 | 6182: 5-methyltetrahydropteroyltriglutamate-homocysteine methyltransferase / 85 078 / 5.27 | X | X | | |
| 118 | 0946 | 6184: peroxiredoxin 2 family protein/glutaredoxin / 26 912 / 4.80 | X | X | | * |
| 119 | 0947 | 6186: lipoamide dehydrogenase, putative / 44527 / 5.66 | X | X | | |
| 120 | 0950 | 6189: succinate dehydrogenase, flavoprotein subunit / 66 787 / 5,84 | X | X | | + |
| 121 | 0955 | 6194: 2-oxoglutarate dehydrogenase, E1 component / 105 082 / 6.24 | X | X | X | |
| 122 | 0956 | 6195: 2-oxoglutarate dehydrogenase, E2, dihydrolipoamide succinytransferase / 41491 / 5.14 | | X | | |
| 123 | 0959 | 6199: succinyl-CoA synthetase, beta subunit / 41666 / 5.06 | | X | | |
| 124 | 0960 | 6200: succinyl-CoA synthetase, alpha subunit /30 548 / 6.00 | X | X | | |
| 125 | 0983 | 6223 : phosphoribosylaminoimidazolecarboxamide formyltransferase .../ 56 803 / 5.90 | | X | | |
| 126 | 0997 | 6237: D-lactate dehydrogenase / 64001 / 6.32 | | | X | |
| 127 | 1031 | 6270 : 3-isopropylmalate dehydrogenase / 39172 / 4.91 | | X | | ** |
| 128 | 1044 | 6284: ferredoxin-NADP reductase / 29 314 / 5.72 | X | X | | * |
| 129 | 1046 | 6286: threonine synthase / 51870 / 5.31 | | X | | * |
| 130 | 1053 | 6294: class 5 outer membrane protein / 28 009 / 9.68 / 20 | X | X | X | |
| 131 | 1057 | 6297: gamma-glutamyltranspeptidase /65 071 / 5.99 | X | X | | + |
| 132 | 1070 | 6309: 2-isopropylmalate synthase / 55 397 / 5.68 | | X | | ** |
| 133 | 1073 | 6313: conserved hypothetical protein / 42 032 / 4.60 | | X | | ** |
| 134 | 1127 | 6364: oxidoreductase, short chain dehydrogenase/reductase family / 25 917 / 5.99 | X | X | | ** |
| 135 | 1131 | 6368: chaperone protein HscA / 66166 / 5.18 | | X | | ** |
| 136 | 1150 | 6387: dihydroxy-acid dehydratase / 64 503 / 5,89 | X | X | | * |
| 137 | 1164/1126 | 6400-6362: hypothetical protein / 22 025 / 8.03 (duplicated gene) | X | | X | + |
| 138 | 1199 | 6435: GTP binding protein / 67 260 / 5.04 | X | X | | + |
| 139 | 1201 | 6438: IMP dehydrogenase / 53 383 / 6.72 | X | | | * |
| 140 | 1206 | 6443: bacterioferritin B / 18075 / 4.60 | | X | | ** |

(continued)

| NMB | ID (gi:722xxxx): name / MW / pI | Gel (Fig.) | | | Note |
| --- | --- | --- | --- | --- | --- |
| | | 2 | 3 | 4 | |
| 141 | 1228 | 6467: homoserine dehydrogenase / 44 737 / 5.254 / 20 | X | X | | |
| 142 | 1231 | 6470: ATP-dependent protease La / 90590 / 6.18 | | X | X | * |
| 143 | 1238 | 6478: peptidyl-prolyl cis-trans isomerase-related protein / 53213 / 5.68 | X | X | | + |
| 144 | 1240 | 6480: ABC transporter, ATP-binding protein / 60 779 / 5.09 | X | X | | ** |
| 145 | 1252 | 6492: phosphoribosylformylglycinamidine cyclo-ligase / 36 974 / 4.71 | X | | | ** |
| 146 | 1285 | 6525: enolase / 46134 / 4,78 | X | X | | + |
| 147 | 1301 | 6541: 30S ribosomal protein / 61 177 / 4.9 | X | X | X | * |
| 148 | 1302 | 6542: integration host factor, beta subunit / 11796 / 9.98 | | | X | ** |
| 149 | 1313 | 6554: trigger factor / 48 325 / 4.72 | X | X | | ** |
| 150 | 1320 | 6562: 50S ribosomal protein L9 / 15 747 / 6.62 | X | X | X | |
| 151 | 1323 | 6565: 30S ribosomal protein S6 / 13 949 / 6.37 | X | X | X | ** |
| 152 | 1324 | 6566: thioredoxin reductase / 33961 / 5.16 | | X | | |
| 153 | 1328 | 6571: conserved hypothetical protein / 27021 / 6.41 | | | X | * |
| 154 | 1339 | 6583:prolyl-tRNA synthetase / 62 992 / 5.09 | X | X | X | ** |
| 155 | 1341 | 6585:pyruvate dehydrogenase, E1 component / 99 562 / 5.5 | x | X | X | ** |
| 156 | 1342 | 6586 : pyruvate dehydrogenase, E2, dihydrolipoamide acetyltransferase / 55 223 / 5,29 | X | X | | * |
| 157 | 1343 | 6587: hypothetical protein / 16 339 / 4.99 | X | | | ** |
| 158 | 1344 | 6588: pyruvate dehydrogenase, E3 component, lipoamide dehydrogenase / 62358 / 5.07 | | X | | |
| 159 | 1345 | 6590: hypothetical protein / 57 143 / 5.13 | | X | | + |
| 160 | 1347 | 6591: extragenic suppressor protein SuhB / 28 469/ 5,82 | X | X | | * |
| 161 | 1356 | 6601: Glu-tRNA(Gln)amidotransferase, subunit A / 51 280 / 5,44 | X | | | * |
| 162 | 1358 | 6603: Glu-tRNA(Gln) amidotransferase, subunit B / 51912/ 5,05 | X | X | | ** |
| 163 | 1372 | 6618:ATP-dependent Clp protease, ATP-binding subunit ClpX / 45 100 / 5.18 | | X | | * |
| 164 | 1379 | 7413462: nifS protein / 44832 / 5.62 | | X | | ** |
| 165 | 1390 | 6628: glucokinase / 34 951 / 8.59 | X | | X | |
| 166 | 1392 | 6630: glucose-6-phosphate 1-dehydrogenase / 54124 / 5.30 | X | X | X | ** |
| 167 | 1425 | 6664: lysyl-tRNA synthetase, heat inducible / 57312 / 5.34 | X | X | | ** |

(continued)

| | NMB | ID (gi:722xxxx): name / MW / pI | Gel (Fig.) | | | Note |
|---|---|---|---|---|---|---|
| | | | 2 | 3 | 4 | |
| 168 | 1429 | 6669: outer membrane protein PorA / 40 129 / 8.73 | X | X | X | |
| 169 | 1445 | 6686: recA protein / 37 612 / 5.18 | | X | | * |
| 170 | 1452 | 6693: conserved hypothetical protein / 40 598/ 8.33 | X | | | * |
| 171 | 1457 | 6698: transketolase / 71 659 / 5.45 | X | X | X | * |
| 172 | 1471 | 6711: tryptophanyl-tRNA synthetase / 37 616 / 5.65 | X | X | | * |
| 173 | 1472 | 6713: clpB protein / 95 195 / 5.41 | | X | | ** |
| 174 | 1483 | 6723: lipoprotein NlpD, putative / 42991 / 9.55 | | | X | + |
| 175 | 1506 | 6749: arginyl-tRNA synthetase / 62 803 / 5.24 | | X | | |
| 176 | 1518 | 6762: acetate kinase / 42 410 / 5.76 | X | X | | |
| 177 | 1533 | 6778. H.8 outer membrane protein / 16 886 / 4.61 /17 | X | X | | |
| 178 | 1536 | 6781: preprotein translocase SecA subunit /103 295 / 5.05 | X | X | | * |
| 179 | 1560 | 6807: glutaminyl-tRNA synthetase / 64 650 / 57.74 | X | X | | ** |
| 180 | 1567 | 6815: macrophage infectivity potentiator / 26 875 / 5.50 | X | X | | + |
| 181 | 1572 | 6819: aconitate hydratase 2 / 92 716 7 5.42 | X | X | | |
| 182 | 1574 | 6822: ketol-acid reductoisomerase / 36 439 / 5.65 | X | X | | ** |
| 183 | 1577 | 6825: acetolactate synthase III, large subunit / 62 813 / 5.88 | X | X | | |
| 184 | 1581 | 6829: histidinol dehydrogenase / 46324 / 5.07 | X | X | | |
| 185 | 1583 | 6831: imidazoleglycerol-phosphate dehydratase / 34002 / 8.61 | | | X | ** |
| 186 | 1584 | 6833:3-hydroxyacid dehydrogenase / 30 378 / 5.33 | | X | | + |
| 187 | 1594 | 6843: spermidine/putrescine ABC transporter, periplasmic .../ 40 234 / 5.52 | X | X | | + |
| 188 | 1595 | 6845:alanyl-tRNA synthetase / 96 038/ 5.54 | X | | X | ** |
| 189 | 1604 | 6853: phosphoglycerate mutase / 25 959 / 5.59 | X | X | | ** |
| 190 | 1612 | 6862 : amino acid ABC transporter, periplasmic amino acid-binding protein / 27 68 / 4.87 | X | X | | + |
| 191 | 1613 | 6863: fumarate hydratase, class I / 54 951 / 5,12 | X | X | | + |
| 192 | 1621 | 6872: glutathione peroxidase / 20129 / 5.18 | | X | | ** |
| 193 | 1636 | gi 7443356 opacity protein, authentic frameshift / 27180 / 9.52 | | | X | |
| 194 | 1640 | 6890: phosphoserine aminotransferase / 41651 / 5.20 | | X | | * |
| 195 | 1642 | 6892: N utilization substance protein A / 55 752 I 4.54 | X | X | | ** |
| 196 | 1655 | 6907: adenine specific methylase, putative / 33 956 / 4,36 | X | | | * |
| 197 | 1684 | 6938: seryl-tRNA synthetase / 47 883 / 5.60 | X | | | ** |
| 198 | 1691 | 6945: dihydropteroate synthase / 30 313 / 5.30 | | X | | |

(continued)

| | NMB | ID (gi:722xxxx): name / MW / pI | Gel (Fig.) | | | Note |
|---|---|---|---|---|---|---|
| | | | 2 | 3 | 4 | |
| 199 | 1710 | 6965: glutamate dehydrogenase, NADP-specific / 48 490 / 5.98 | X | X | | ** |
| 200 | 1714 | 6969. multidrug efflux pump channel protein / 48 482 / 8.38 / lipo | X | | X | + |
| 201 | 1716 | 6971: membrane fusion protein / 40 209 / 8.60 | X | | X | |
| 202 | 1796 | 7050: conserved hypothetical protein/ 20 931 / 5.73 | X | X | | |
| 203 | 1809 | -----: putative; pilN protein,authentic frameshift / 22 258 / 9.00 | X | | | |
| 204 | 1810 | 7063: pilO protein / 19 870 / 4.74 | | X | | |
| 205 | 1811 | 7064: pilP protein / 20126 / 4.94 | | X | | |
| 206 | 1812 | ----. putative, pilQ protein, authentic frameshift / 83 151 / 10.75 / 25 | X | X | X | |
| 207 | 1835 | 7090: tyrosyl-tRNA synthetase / 47437 / 5.76 | | X | | * |
| 208 | 1838 | 7093: GTP-binding protein, putative / 39 376 / 4,80 | X | X | | ** |
| 209 | 1839 | 7094: formate-tetrahydrofolate ligase / 59063 / 5.85 | X | X | | + |
| 210 | 1843 | 7099: transcriptional regulator, MarR family / 16 583 / 5.15 | | X | | ** |
| 211 | 1849 | 7105: carbamoyl-phosphate synthase, small subunit / 40 587 / 5.54 | X | X | | |
| 212 | 1854 | 7110: hypothetical protein / 25131 / 8.95 | X | | X | ** |
| 213 | 1855 | 7111: carbamoyl-phosphate synthase, large subunit / 117 377 / 5.10 | X | X | | |
| 214 | 1856 | 7112: transcriptional regulator, LysR family / 33367 / 5.55 | | X | | + |
| 215 | 1859 | 7116: S-adenosylmethionine:tRNA ribosyltransferase-isomerase /38216/6.20 | | X | | * |
| 216 | 1861 | 7118: acetyl-CoA carboxylase, biotin carboxylase / 49 599 / 5,88 | X | X | X | ** |
| 217 | 1862 | 7119: ribosomal protein L11 methyltransferase / 32144 / 4.36 | | X | | |
| 218 | 1864 | 7121:glutamate-1-semialdehyde 2,1-aminomutase / 45 315 / 5.33 | | X | | * |
| 219 | 1869 | 7127: fructose-bisphosphate aldolase / 38 337 / 5.48 | X | X | X | + |
| 220 | 1870 | 7128: hypothetical protein / 26 964 / 7.23 | X | | X | + |
| 221 | 1903 | 7160: chromosomal replication initiator protein DnaA / 58 029 / 5.93 | | X | | ** |
| 222 | 1920 | 7178: GMP synthase / 57686 / 5.53 | X | X | | |
| 223 | 1921 | 7179: 3-oxoacyl-(acyl-carrier-protein) reductase / 26 068 / 5.95 | X | X | X | * |
| 224 | 1930 | 7187: glycyl-tRNA synthetase, beta chain / 74 574 / 5,86 | X | X | | |

(continued)

| | NMB | ID (gi:722xxxx): name / MW / pI | Gel (Fig.) | | | Note |
|---|---|---|---|---|---|---|
| | | | 2 | 3 | 4 | |
| 225 | 1934 | 7192: ATP synthase F1, beta subunit/ 50 391 / 5.01 | X | X | X | |
| 226 | 1936 | 7194: ATP synthase F1, alpha subunit / 55 291 / 5.43 | X | X | X | * |
| 227 | 1937 | 7195: ATP synthase F1, delta subunit /19 526 / 5.02 | | X | | ** |
| 228 | 1938 | 7196: ATP synthase F0, B subunit / 171281 / 5.64 | | X | | |
| 229 | 1946 | 7205: outer membrane lipoprotein / 29 258 / 5.01 | X | X | X | + |
| 230 | 1953 | 7212: stringent starvation protein A / 23165 / 6.23 | X | X | X | ** |
| 231 | 1966 | 7226 :ABC transporter, ATP-binding protein / 29 286 / 5.58 | | X | | * |
| 232 | 1968 | 7228: aldehyde dehydrogenase A / 52257 / 5.20 | X | X | | |
| 233 | 1972 | 7233: chaperonin, 60 kDa / 57 423 / 4.9 | X | X | X | + |
| 234 | 1982 | 7243: DNA polymerase I /103 184 / 5.21 | | X | | * |
| 235 | 1996 | 7259: phosphoribosylformylglycinamidine synthase / 143 854 / 5.31 | X | X | | * |
| 236 | 2000 | 7264: conserved hypothetical protein / 33468 4.77 | | X | | ** |
| 237 | 2039 | 7300 : major outer membrane protein PIB / 33 786 / 6.54 | X | X | X | |
| 238 | 2057 | 7319: 50S ribosomal protein L13 / 16212 / 9.72 | | | X | ** |
| 239 | 2079 | 7341: aspartate-semialdehyde dehydrogenase / 39 858 / 5.40 | | X | | * |
| 240 | 2086 | 7348: GTP-binding protein / 41 930 / 5.52 | | X | | * |
| 241 | 2091 | 7353: hemolysin, putative / 21804 / 9.81 | | | X | + |
| 242 | 2096 | 7359: malate:quinone oxidoreductase / 53 9687 / 5.51 | X | X | | + |
| 243 | 2101 | 7364: 30S ribosomal protein S2 / 26 899 / 9.04 | X | | | ** |
| 244 | 2102 | 7365. elongation factor TS (EF-TS) / 30 330 / 5.30 | X | X | | ** |
| 245 | 2103 | 7366: uridylate kinase / 25896 / 6.17 | | | X | * |
| 246 | 2129 | 7385: argininosuccinate synthase / 49 664 / 5.18 | X | X | | * |
| 247 | 2138 | 7395: peptide chain release factor 2/41 487/5.18 | X | | | ** |
| 248 | 2154 | 7412: electron transfer flavoprotein, alpha subunit / 32 579 / 4.99 | | X | | * |
| 249 | 2155 | 7413. electron transfer flavoprotein, beta subunit / 26 948 / 6.08 | X | X | X | ** |
| 250 | 2159 | 7417: glyceraldehyde 3-phosphate dehydrogenase / 35 845 / 5.40 | X | X | | * |
| | | TOTALS 7 | 156 | 194 | 87 | |

**REFERENCES**

[1] Tettelin et al. (2000) Science 287:1809-1815.

[2] Pizza et al. (2000) Science 287:1816-1820.

[3] Parkhill et al. (2000) Nature 404:502-506.

[4] Cole (2002) Eur Respir J Suppl. 36:78s-86s.

[5] McClelland et al. (2001) Nature 413:852-856.

[6] De Groot et al. (20001) Vaccine 19:4385-4395.

[7] Stephens (2000) J Infect Dis 181 Suppl 3:S521-523.

[8] Tettelin et al. (2001) Science 293:498-506.

[9] Weinstock et al. (2000) Res Microbiol 151:1551-158.

[10] Wizemann et al. (2001) Infect Immun 69:1593-1598.

[11] Grifantini et al. (2002) Nat Biotechnol. 20:914-921.

[12] *http://www.ncbi.nlm.nih.gov/entrez*

[13] WO01/64922.

[14] WO01/64920.

[15] WO03/020756.

[16] Bjune et al. (1991) Lancet 338 (8775) : 1093- 1096.

[17] International patent application WO00/25811.

[18] International patent application WO01/52885.

[19] de Kleijn et al. (2001) Vaccine 20:352-358.

[20] US patent 5597572.

[21] US patent 5747653.

[22] European patent application 0680512.

[23] International patent application WO01/09350.

[24] International patent application WO02/09746.

[25] European patent 0449958

[26] International patent application WO01/91788.

[27] Gennaro (2000) Remington: The Science and Practice of Pharmacy. 20th edition, ISBN: 0683306472.

[28]  Vaccine design: the subunit and adjuvant approach, eds. Powell & Newman, Plenum Press 1995 (ISBN 0-306- 44867- X) .

[29] WO90/14837.

[30] WO02/26212.

[31] WO98/33487.

[32] WO00/07621.

[33] WO99/27960.

[34] WO98/57659.

[35] European patent applications 0835318, 0735898 and 0761231.

[36] Krieg (2000) Vaccine 19:618-622; Krieg (2001) Curr opin Mol Ther 2001 3:15-24; WO96/02555, WO98/16247, WO98/18810, WO98/40100, WO98/55495, WO98/37919 and WO98/52581 *etc.*

[37] WO99/52549.

[38] WO01/21207.

[39] WO01/21152.

[40] WO00/62800.

[41] WO00/23105.

[42] WO99/11241.

[43] Del Giudice et al. (1998) Molecular Aspects of Medicine, vol. 19, number 1.

*[44]* Vaccine Design... (1995) eds. Powell & Newman. ISBN: 030644867X. Plenum.

[45] Johnson et al. (1999) Bioorg Med Chem Lett 9:2273-2278.

[46] Covacci & Rappuoli (2000) J. Exp. Med. 19:587-592.

[47] WO93/18150.

[48] Covacci et al. (1993) Proc. Natl. Acad. Sci. USA 90: 5791-5795.

[49] Tummuru et al. (1994) Infect. Immun. 61:1799-1809.

[50] Marchetti et al. (1998) Vaccine 16:33-37.

[51] Telford et al. (1994) J. Exp. Med 179:1653-1658.

[52] Evans et al. (1995) Gene 153:123-127.

[53] WO96/01272 & WO96/01273, especially SEQ ID NO:6.

[54] WO97/25429.

[55] WO98/04702.

[56] Costantino et al. (1992) Vaccine 10:691-698.

[57] Costantino et al. (1999) Vaccine 17:1251-1263.

[58] International patent application WO03/007985.

[59] Watson (2000) Pediatr Infect Dis J 19:331-332.

[60] Rubin (2000) Pediatr Clin North Am 47:269-285, v.

[61] Jedrzejas (2001) Microbiol Mol Biol Rev 65:187-207.

[62] Bell (2000) Pediatr Infect Dis J 19:1187-1188.

[63] Iwarson (1995) APMIS 103:321-326.

[64] Gerlich et al. (1990) Vaccine 8 Suppl:S63-68 & 79-80.

[65] Gustafsson et al. (1996) N. Engl. J. Med. 334:349-355.

[66] Rappuoli et al. (1991) TIBTECH 9:232-238.

[67]  Vaccines (1988) eds. Plotkin & Mortimer. ISBN 0- 7216- 1946- 0.

[68] Del Guidice et al. (1998) Molecular Aspects of Medicine 19:1-70.

[69] Hsu et al. (1999) Clin Liver Dis 3:901-915.

[70] International patent application WO99/24578.

[71] International patent application WO99/36544.

[72] International patent application WO99/57280.

[73] International patent application WO02/079243.

[74] International patent application WO02/02606.

[75] Kalman et al. (1999) Nature Genetics 21:385-389.

[76] Read et al. (2000) Nucleic Acids Res 28:1397-406.

[77] Shirai et al. (2000) J. Infect. Dis. 181(Suppl 3):S524-S527.

[78] International patent application WO99/27105.

[79] International patent application WO00/27994.

[80] International patent application WO00/37494.

[81] International patent application WO99/28475.

[82] Ross et al. (2001) Vaccine 19:4135-4142.

[83] Sutter et al. (2000) Pediatr Clin North Am 47:287-308.

[84] Zimmerman & Spann (1999) Am Fam Physician 59:113-118, 125-126.

[85] Dreesen (1997) Vaccine 15 Suppl:S2-6.

[86] MMWR Morb Mortal Wkly Rep 1998 Jan 16; 47 (1) : 12, 19.

[87] Vaccines (1988) eds. Plotkin & Mortimer. ISBN 0- 7216- 1946- 0.

[88] McMichael (2000) Vaccine 19 Suppl 1:S101-107.

[89] Schuchat (1999) Lancet 353 (9146) : 51- 6.

[90] International patent application WO02/34771.

[91] Dale (1999) Infect Dis Clin North Am 13:227-43, viii.

[92] Ferretti et al. (2001) PNAS USA 98: 4658-4663.

[93] Kuroda et al. (2001) Lancet 357 (9264) : 1225- 1240; see also pages 1218- 1219.

[94] J Toxicol Clin Toxicol (2001) 39:85-100.

[95] Demicheli et al. (1998) Vaccine 16:880-884.

[96] Stepanov et al. (1996) J Biotechnol 44:155-160.

[97] Ingram (2001) Trends Neurosci 24:305-307.

[98] Rosenberg (2001) Nature 411:380-384.

[99] Moingeon (2001) Vaccine 19:1305-1326.

[100] EP-A-0372501

[101] EP-A-0378881

[102] EP-A-0427347

[103] WO93/17712

[104] WO94/03208

[105] WO98/58668

[106] EP-A-0471177

[107] WO00/56360

[108] WO91/01146

[109] WO00/61761

[110] WO01/72337

[111] *Research Disclosure,* 453077 (Jan 2002)

[112] Molloy et al. (2000) Eur J Biochem 267 (10) : 2871- 81

[113] Molloy et al. (2001) Electrophoresis 22 (9) : 1686- 96

[114] Phadke et al. (2001) Proteomics 1 (5) : 705- 20.

[115] Nouwens et al. (2000) Electrophoresis 21 (17) : 3797- 809.

[116] Chevallet et al. (1998) Electrophoresis 19 (11) : 1901- 9.

[117] Herbert et al. (1998) Electrophoresis 19 (5) : 845- 51.

[118] Doherty et al. (1998) Electrophoresis 19 (2) : 355- 63.

[119] Wilm et al. (1996) Nature 379 (6564) : 466- 9.

[120] WO99/24578.

[121] WO99/36544.

[122] WO99/57280.

[123] Nakai & Kanehisa Proteins 11 (2):95-110

[124] Nakai (2000) Adv Protein Chem 54:277-344.

[125] WO01/38350

**Claims**

1. A lipid bilayer which includes a protein selected from: (i) a protein comprising the amino acid sequence of SEQ ID NO: 165; (ii) a protein comprising an amino acid sequence which shares at least 90% sequence identity with the amino acid sequence of SEQ ID NO: 165; (iii) a protein comprising a fragment of the amino acid sequence of SEQ ID NO: 165, wherein the fragment comprises at least 7 consecutive amino acids from the sequence; and/or (iv) a hybrid protein of formula: $NH_2$- A- [- X- L- $]_n$- B- COOH, wherein X is the amino acid sequence as defined in any one of (i) to (iii), L is an optional linker amino acid sequence, A is an optional N- terminal amino acid sequence, B is an optional C- terminal amino acid sequence, and n is an integer greater than 1, wherein the lipid bilayer does not include lipooligosaccharide.

2. The lipid bilayer of claim 1, in the form of a cell membrane, a liposome, a bacterial ghost, an outer membrane vesicle or a bleb.

3. The lipid bilayer of claim 1 or claim 2, which does not include at least one of the following native membrane components: a porin; lipopolysaccharide; PilC protein; Omp85 protein; an opacity proteins; a pilin; or P64k.

4. A composition comprising: (a) outer- membrane vesicles prepared from a first strain of *Neisseria meningitidis*; and (b) one or more proteins which are present in outer- membrane vesicles prepared from a second strain of *Neisseria meningitidis,* but which are not present in outer- membrane vesicles prepared from said first strain, wherein said one or more protein (s) includes a protein selected from: (i) the amino acid sequence of SEQ ID NO: 165; (ii) a protein comprising an amino acid sequence which shares at least 90% sequence identity with the amino acid sequence of SEQ ID NO: 165; (iii) a protein comprising a fragment of the amino acid sequence of SEQ ID NO: 165, wherein the fragment comprises at least 7 consecutive amino acids from the sequence; and/or (iv) a hybrid protein of formula: $NH_2$- A- [- X- L- $]_n$- B- COOH, wherein X is the amino acid sequence as defined in any one of (i) to (iii), L is an optional linker amino acid sequence, A is an optional N- terminal amino acid sequence, B is an optional C- terminal amino acid sequence, and n is an integer greater than 1.

5. The composition of claim 4, wherein the protein (s) of component (b) are: (i) purified from the second strain and added to component (a) ; (ii) expressed recombinantly, purified, and added to component (a) ; or (iii) expressed by said first strain following either engineering of the first strain such that it expresses said protein (s), either (1) from its chromosomal DNA or from extrachromosomal DNA, or (2) such that existing expression of said protein (s) is up-regulated, or (3) such that trafficking of said protein (s) already expressed by the first strain is altered to direct it/ them to a different cellular location, thereby causing it/ them to be present in the outer- membrane vesicles.

6. A composition according to claim 4, comprising outer-membrane vesicles (OMVs) prepared from a genetically-modified first strain of *Neisseria meningitidis,* wherein said OMVs include one or more proteins which are (a) not present in OMVs prepared from said first strain prior to its being genetically modified, but which are (b) present in OMVs prepared from a second strain of *Neisseria meningitidis.*

7. The use of a protein comprising the amino acid sequence of SEQ ID NO: 165 in a method of preparing a lipid bilayer according to any one of claims 1 to 3 and/or a composition according to any one of claims 4 to 6.

8. The use of a protein comprising an amino acid sequence which shares at least 90% sequence identity with the amino acid sequence of SEQ ID NO: 165 in a method of preparing a lipid bilayer according to any one of claims 1 to 3 and/or a composition according to any one of claims 4 to 6.

9. The use of a protein comprising a fragment of the amino acid sequence of SEQ ID NO: 165, wherein the fragment comprises at least 7 consecutive amino acids from the sequence in a method of preparing a lipid bilayer according to any one of claims 1 to 3 and/or a composition according to any one of claims 4 to 6.

10. The use of a hybrid protein of formula: $NH_2$- A- [- X- L- $]_n$- B- COOH, wherein X is the amino acid sequence as defined in any one of claims 7 to 9, L is an optional linker amino acid sequence, A is an optional N- terminal amino acid sequence, B is an optional C- terminal amino acid sequence, and n is an integer greater than 1 in a method of preparing a lipid bilayer according to any one of claims 1 to 3 and/or a composition according to any one of claims 4 to 6.

11. The use of a nucleic acid encoding the protein of any one of claims 7 to 10 in a method of preparing a lipid bilayer according to any one of claims 1 to 3 and/or a composition according to any one of claims 4 to 6.

12. A lipid bilayer according to any one of claims 1 to 3, and/or a composition according to any one of claims 4 to 6, for use in a method of treating or preventing infection due to *Neisseria.*

**Patentansprüche**

1. Lipid- Doppelschicht, die ein aus: (i) einem die Aminosäuresequenz der SEQ ID NO: 165 umfassenden Protein; (ii) einem eine Aminosäuresequenz, die wenigstens 90% Sequenzidentität mit der Aminosäuresequenz der SEQ ID NO: 165 teilt, umfassenden Protein; (iii) einem ein Fragment der Aminosäuresequenz der SEQ ID NO: 165 umfassenden Protein, wobei das Fragment wenigstens 7 aufeinander folgende Aminosäuren aus der Sequenz umfasst; und/ oder (iv) einem Hybridprotein der Formel: $NH_2$- A- [- X- L- $]_n$- B- COOH, wobei X für die Aminosäuresequenz mit der unter einem der Punkte (i) bis (iii) angegebenen Bedeutung, L für eine optionale Linker- Aminosäuresequenz, A für eine optionale N- terminale Aminosäuresequenz, B für eine optionale C- terminale Aminosäuresequenz und n für eine ganze Zahl größer 1 steht, ausgewähltes Protein enthält, wobei die Lipid- Doppelschicht kein Lipooligosaccharid enthält.

2. Lipid-Doppelschicht nach Anspruch 1, in Form einer Zellmembran, eines Liposoms, eines Bakterien-Ghost, eines Vesikels der äußeren Membran oder eines Bläschens.

3. Lipid-Doppelschicht nach Anspruch 1 oder Anspruch 2, die wenigstens eine der folgenden nativen Membrankomponenten nicht enthält: ein Porin; Lipopolysaccharid; PilC-Protein; Omp85-Protein; ein Opazität-Protein; ein Pilin; oder P64k.

4. Zusammensetzung, umfassend: (a) aus einem ersten Stamm von *Neisseria meningitidis* präparierte Vesikel der äußeren Membran; und (b) ein oder mehrere Proteine, die in aus einem zweiten Stamm von *Neisseria meningitidis* präparierten Vesikeln der äußeren Membran, jedoch nicht in aus dem ersten Stamm präparierten Vesikeln der äußeren Membran vorhanden sind, wobei das eine Protein bzw. die mehreren Proteine ein aus: (i) der Aminosäuresequenz der SEQ ID NO: 165; (ii) einem eine Aminosäuresequenz, die wenigstens 90% Sequenzidentität mit der Aminosäuresequenz der SEQ ID NO: 165 teilt, umfassenden Protein; (iii) einem ein Fragment der Aminosäuresequenz der SEQ ID NO: 165 umfassenden Protein, wobei das Fragment wenigstens 7 aufeinander folgende Aminosäuren aus der Sequenz umfasst; und/ oder (iv) einem Hybridprotein der Formel: $NH_2$- A- [- X- L- ]$_n$- B- COOH, wobei X für die Aminosäuresequenz mit der unter einem der Punkte (i) bis (iii) angegebenen Bedeutung, L für eine optionale Linker- Aminosäuresequenz, A für eine optionale N- terminale Aminosäuresequenz, B für eine optionale C- terminale Aminosäuresequenz und n für eine ganze Zahl größer 1 steht, ausgewähltes Protein enthält bzw. enthalten.

5. Zusammensetzung nach Anspruch 4, wobei das Protein bzw. die Proteine von Komponente (b): (i) aus dem zweiten Stamm aufgereinigt und zu Komponente (a) gegeben wird bzw. werden; (ii) rekombinant exprimiert, aufgereinigt und zu Komponente (a) gegeben wird bzw. werden; oder (iii) durch den ersten Stamm exprimiert wird bzw. werden, nachdem entweder der erste Stamm manipuliert wurde, so dass er das Protein bzw. die Proteine exprimiert, entweder (1) von seiner chromosomalen DNA oder von extrachromosomaler DNA, oder (2) so dass bereits vorliegende Expression des Proteins bzw. der Proteine heraufreguliert wird oder (3) so dass der Transport des bzw. der bereits durch den ersten Stamm exprimierten Proteins bzw. Proteine verändert wird, um es/sie zu einem anderen Zellort zu führen, was dazu führt, dass es/sie in den Vesikeln der äußeren Membran vorliegen.

6. Zusammensetzung nach Anspruch 4, umfassend aus einem gentechnisch veränderten ersten Stamm von *Neisseria meningitidis* präparierte Vesikel der äußeren Membran (OMV), wobei die OMV ein oder mehrere Proteine enthalten, die (a) nicht in aus dem ersten Stamm vor dessen gentechnischer Veränderung präparierten OMV, jedoch (b) in aus einem zweiten Stamm von *Neisseria meningitidis* präparierten OMV vorhanden sind.

7. Verwendung eines die Aminosäuresequenz der SEQ ID NO: 165 umfassenden Proteins bei einem Verfahren zur Herstellung einer Lipid-Doppelschicht gemäß einem der Ansprüche 1 bis 3 und/oder einer Zusammensetzung gemäß einem der Ansprüche 4 bis 6.

8. Verwendung eines eine Aminosäuresequenz, die wenigstens 90% Sequenzidentität mit der Aminosäuresequenz der SEQ ID NO: 165 teilt, umfassenden Proteins bei einem Verfahren zur Herstellung einer Lipid-Doppelschicht gemäß einem der Ansprüche 1 bis 3 und/oder einer Zusammensetzung gemäß einem der Ansprüche 4 bis 6.

9. Verwendung eines ein Fragment der Aminosäuresequenz der SEQ ID NO: 165 umfassenden Proteins, wobei das Fragment wenigstens 7 aufeinander folgende Aminosäuren aus der Sequenz umfasst, bei einem Verfahren zur Herstellung einer Lipid-Doppelschicht gemäß einem der Ansprüche 1 bis 3 und/oder einer Zusammensetzung gemäß einem der Ansprüche 4 bis 6.

10. Verwendung eines Hybridproteins der Formel: $NH_2$- A- [- X- L- ]$_n$- B- COOH, wobei X für die Aminosäuresequenz mit der in einem der Ansprüche 7 bis 9 angegebenen Bedeutung, L für eine optionale Linker- Aminosäuresequenz, A für eine optionale N- terminale Aminosäuresequenz, B für eine optionale C- terminale Aminosäuresequenz und n für eine ganze Zahl größer 1 steht, bei einem Verfahren zur Herstellung einer Lipid- Doppelschicht gemäß einem der Ansprüche 1 bis 3 und/ oder einer Zusammensetzung gemäß einem der Ansprüche 4 bis 6.

11. Verwendung einer für das Protein nach einem der Ansprüche 7 bis 10 codierenden Nukleinsäure bei einem Verfahren zur Herstellung einer Lipid-Doppelschicht gemäß einem der Ansprüche 1 bis 3 und/oder einer Zusammensetzung gemäß einem der Ansprüche 4 bis 6.

12. Lipid-Doppelschicht gemäß einem der Ansprüche 1 bis 3 und/oder Zusammensetzung gemäß einem der Ansprüche 4 bis 6 zur Verwendung bei einem Verfahren zur Behandlung oder Vorbeugung von Infektionen durch *Neisseria.*

**Revendications**

1. Bicouche lipidique qui comprend une protéine choisie parmi : (i) une protéine comprenant la séquence d'acides aminés de SEQ ID NO: 165 ; (ii) une protéine comprenant une séquence d'acides aminés qui partage au moins 90 % d'identité de séquence avec la séquence d'acides aminés de SEQ ID NO: 165 ; (iii) une protéine comprenant un fragment de la séquence d'acides aminés de SEQ ID NO: 165, le fragment comprenant au moins 7 acides aminés consécutifs de la séquence ; et/ou (iv) une protéine hybride de formule : $NH_2$- A- [- X- L- ]$_n$- B- COOH, dans laquelle X est la séquence d'acides aminés telle que définie dans l'un quelconque de (i) à (iii), L est une séquence d'acides aminés de lieur facultative, A est une séquence d'acides aminés N- terminale facultative, B est une séquence d'acides aminés C- terminale facultative, et n est un entier supérieur à 1, la bicouche lipidique ne comprenant pas de lipo- oligosaccharide.

2. Bicouche lipidique de la revendication 1, sous la forme d'une membrane cellulaire, un liposome, une bactérie vide, une vésicule de membrane externe ou une vésicule.

3. Bicouche lipidique de la revendication 1 ou la revendication 2, qui ne comprend pas au moins un des composants de membrane natifs : une porine ; un lipopolysaccharide ; la protéine PilC ; la protéine Omp85 ; des protéines d'opacité ; une piline ; ou P64k.

4. Composition comprenant : (a) des vésicules de membrane externe préparées à partir d'une première souche de *Neisseria meningitidis,* et (b) une ou plusieurs protéines qui sont présentes dans des vésicules de membrane externe préparées à partir d'une deuxième souche de *Neisseria meningitidis,* mais qui ne sont pas présentes dans des vésicules de membrane externe préparées à partir de ladite première souche, lesdites une ou plusieurs protéine (s) comprenant une protéine choisie parmi : (i) la séquence d'acides aminés de SEQ ID NO: 165 ; (ii) une protéine comprenant une séquence d'acides aminés qui partage au moins 90 % d'identité de séquence avec la séquence d'acides aminés de SEQ ID NO: 165 ; (iii) une protéine comprenant un fragment de la séquence d'acides aminés de SEQ ID NO: 165, le fragment comprenant au moins 7 acides aminés consécutifs de la séquence ; et/ou (iv) une protéine hybride de formule : $NH_2$- A- [- X- L- ]$_n$- B- COOH, dans laquelle X est la séquence d'acides aminés telle que définie dans l'un quelconque de (i) à (iii), L est un séquence d'acides aminés de lieur facultative, A est une séquence d'acides aminés N- terminale facultative, B est une séquence d'acides aminés C- terminale facultative, et n est un entier supérieur à 1.

5. Composition de la revendication 4, dans laquelle la/les protéine (s) de composant (b) sont : (i) purifiées à partir de la deuxième souche et ajoutées au composant (a) ; (ii) exprimées de façon recombinante, purifiées, et ajoutées au composant (a) ; ou (iii) exprimées par ladite première souche après manipulation de la première souche de sorte qu'elle exprime ladite/ lesdites protéine (s), soit (1) à partir de son ADN chromosomique ou à partir d'ADN extrachromosomique, ou (2) de sorte que l'expression existante de ladite/ lesdites protéine (s) soit régulée à la hausse, ou (3) de sorte que le trafic de ladite/ lesdites protéine (s) déjà exprimées par la première souche soit modifiée de manière à la/les diriger vers un emplacement cellulaire différent, de manière à la/les amener à être présente (s) dans les vésicules de membrane externe.

6. Composition selon la revendication 4, comprenant des vésicules de membrane externe (OMV) préparées à partir d'une première souche génétiquement modifiée de *Neisseria meningitidis,* lesdites OMV comprenant une ou plusieurs protéines qui sont (a) non présentes dans des OMV préparées à partir de ladite première souche avant d'être génétiquement modifiée, mais qui sont (b) présentes dans des OMV préparées à partir d'une deuxième souche de *Neisseria meningitidis.*

7. Utilisation d'une protéine comprenant la séquence d'acides aminés de SEQ ID NO: 165 dans un procédé de préparation d'une bicouche lipidique selon l'une quelconque des revendications 1 à 3 et/ou une composition selon l'une quelconque des revendications 4 à 6.

8. Utilisation d'une protéine comprenant une séquence d'acides aminés qui partage au moins 90 % d'identité de séquence avec la séquence d'acides aminés de SEQ ID NO: 165 dans un procédé de préparation d'une bicouche lipidique selon l'une quelconque des revendications 1 à 3 et/ou une composition selon l'une quelconque des revendications 4 à 6.

9. Utilisation d'une protéine comprenant un fragment de la séquence d'acides aminés de SEQ ID NO: 165, le fragment comprenant au moins 7 acides aminés consécutifs de la séquence dans un procédé de préparation d'une bicouche lipidique selon l'une quelconque des revendications 1 à 3 et/ou une composition selon l'une quelconque des revendications 4 à 6.

10. Utilisation d'une protéine hybride de formule : $NH_2$- A- [- X- L- $]_n$- B- COOH, dans laquelle X est la séquence d'acides aminés telle que définie dans l'une quelconque des revendications 7 à 9, L est une séquence d'acides aminés de lieur facultative, A est une séquence d'acides aminés N- terminale facultative, B est une séquence d'acides aminés C- terminale facultative, et n est un entier supérieur à 1 dans un procédé de préparation d'une bicouche lipidique selon l'une quelconque des revendications 1 à 3 et/ou une composition selon l'une quelconque des revendications 4 à 6.

11. Utilisation d'un acide nucléique codant pour la protéine de l'une quelconque des revendications 7 à 10 dans un procédé de préparation d'une bicouche lipidique selon l'une quelconque des revendications 1 à 3 et/ou d'une composition selon l'une quelconque des revendications 4 à 6.

12. Bicouche lipidique selon l'une quelconque des revendications 1 à 3, et/ou une composition selon l'une quelconque des revendications 4 à 6, pour utilisation dans un procédé de traitement ou prévention d'une infection due à *Neisseria.*

## FIGURE 1

## FIGURE 2

## *FIGURE 3*

## *FIGURE 4*

## FIGURE 5

## SEQUENCE LISTING

| SEQ ID | NMB | Sequence |
|---|---|---|
| 1 | 0010 | MAFLKLTEQNVQGKTVLIRADMNVPFKDGKISDDTRIRASLASIKYCVDNGASVIVMTHLGRPTEGEFHPEDDVAP VAAHLGSLLGKDVKVLNDWRENKPALNAGDVVMLQNVRINKGEKKNDLELGKAYASLCDVFVNDAFGTAHRAQAST EAVAQAAPVACAGVLMAGELDALGKALKQPARPMVAIVAGSKVSTKLTILESLADKVDQLIVGGGIANTFLLAEGK AIGKSLAEHDLVEESKKIMAKMAAKGGSVPLPTDVVVAKAFAADAEAVVKDIAADAEAVVKDIADVAEDEMILDIG PKSAAALADLLKAAGTVVWNGPVGVFEFDQFAGGTKALAEAIAQSKAFSIAGGGDTLAAIAKFGVTEQIGYISTGG GAFLEFLEGKELPAVAALEKRGA |
| 2 | 0018 | MNTLQKGFTLIELMIVIAIVGILAAVALPAYQDYTARAQVSEAILLAEGQKSAVTEYYLNHGEWPGNNTSAGVATS SEIKGKYVKSVEVKNGVVTAQMASSNVNNEIKGKKLSLWAKRQNGSVKWFCGQPVTRDKAKAANDDVTAAAAANGK KIDTKHLPSTCRDASDAS |
| 3 | 0030 | MTRKILVTSALPYANGSIHLGHMVEHIQTDVWVRFQKLRGHACHYCCADDTHGTPVMLAAQKQGIAPEDMIAKVRE EHLADFTGFFIGYDNYYSTHSPENKQFSQDIYRALKANGKIESRVIEQLFDPEKQMFLPDRFVKGECPKCHAQDQY GDNCEVCGTTYSPTELINPYSAVSGTKPELRESEHFFFKLGECADFLKAWTSGNNPHDGKPHLQAEALNKMKEWLG EGEETTLSDWDISRDAPYFGFEIPDAPGKYFYVWLDAPVGYMASFKNLCDRIGVDFDEYFKADSQTEMYHFIGKDI LYFHALFWPAMLHFSGHRAPTGVYAHGFLTVDGQKMSKSRGTFITAKSYLEQGLNPEWMRYYIAAKLNSKIEDIDL NLQDFISRVNSDLVGKYVNIAARASGFIAKRFEGRLKDVADSELLAKLTAQSEAIAECYESREYAKALRDIMALAD IVNEYVDANKPWELAKQEGQDERLHEVCSELINAFTMLTAYLAPVLPQTAANAAKFLNLEAITWANTRDTLGKHAI NKYEHLMQRVEQKQVDDLIEANKQSIAAAAAPAAEEGKYEKVAEQASFDDFMKIDMRVAKVLNCEAVEGSTKLLKF DLDFGFEKRIIFSGIAASYPNPAELNGRMVIAVANFAPRKMAKFGVSEGMILSAATADGKLKLLDVDTGAQPGDKV G |
| 4 | 0038 | MPQNTLNIVILAAGKGTRMYSKMPKVLHRIGGKPMVGRVIDTAAALNPQNICVVIGHGKEQVLDTVKRDVVWVEQT EQLGTGHAVKTALPHLSAEGRTLVLYGDVPLIDVETLETLLEAAGNEVGLLTDVPNDPTGLGRIIRDSNGSVTAIV EEKDADAVQKAVKEINTGILVLPNAKLENWLNSLSSNNAQGEYYLTDLIAKAVADGIKVHPVQVRASHLAAGVNNK LQLTELERIFQTEQAQELLKAGVTLRDPARFDLRGRLKHGQDVVIDVNCIFEGDIELGDNVEIGANCVIKNAKIGA NSKIAPFSHLESCEVGENNRIGPYARLRPQARLADDVHVGNFVEIKNAAIGKGTKANHLTYIGDAEVGCKTNFGAG TIIANYDGVHKHKTVIGDEVRIGSNCVLVAPVTLGNKVTTGAGSTITRNVEDNKLALARARQTVIEGWVRPEKDKQ |
| 5 | 0045 | MFSFFRRKKKQETPALEEAQIQETAAKAESELAQIVENIKEDAESLAESVKGQVESAVETVSGAVEQVKETVAEML SEAEEAAEKAAEQVEAAKEAVAETVGEAVGQVQEAVATTEEHKLGWAARLKQGLTKSRDKMAKSLAGVFGGGQIDE DLYEELETVLITSDMGMEATEYLMKDVRDRVSLKGLKDGNELRGALKEALYDLIKPLEKPLVLPETKEPFVIMLAG INGAGKTTSIGKLAKYFQAQGKSVLLAAGDTFRAAAREQLQAWGERNNVTVISQTTGDSAAVCFDAVQAAKARGID IVLADTAGRLPTQLHLMEEIKKVKRVLQKAMPDAPHEIIVVLDANIGQNAVNQVKAFDDALGLTGLIVTKLDGTAK GGILAALASDRPVPVRYIGVGEGIDDLRPFDARAFVDALLD |
| 6 | 0051 | MNTDNLHDILDEMVQVYSQKKQSRSETPAEIGAHFHPLLDRLCETAEAQNASDILISKGFPPSLKINSALTPQPQK ALTGEETAAIAASTMNAEQSEIFRRDGEINYSVQSRSGTRYRANAYHSQGSAGLVLRRINHVIPQMQELGLPEKLK DLAVAPRGLLIIVGPTGSGKSTTMATMLEHRNKTLPSHIVTIEDPIEFIYKPRRCIFTQREIGVDTINWQTAVQNA MRQSPDVVCIGEVRSRESMEYAMQLAQTGHLCIFTLHANTAPQSLERILNFYPKEQHNQILIDIALNLTGIICQRL ALKQDKTGRTAVVDLLINTPAIQDFILKGDLMNISKIMETAKTDGMQTMDQNLFELYRHGIISYEEALRQSVSANN LRLHIQLHKEGKTPELLYDRVNGLNLIS |
| 7 | 0052 | MQITDLLAFGAKNKASDLHLSSGISPMIRVHGDMRRINLPEMSAEEVGNMVTSVMNDHQRKIYQQNLEVDFSFELP NVARFRVNAFNIGRGPAAVFRTIPSTVLSLEELKAPSIFQKIAESPRGMVLVTGPTGSGKSTTLAAMINYINETQP AHILTIEDPIEFVHQSKKSLINQRELHQHTLSFANALRSALREDPDVILVGEMRDPETIGLALTAAETGHLVFGTL HTTGAAKTVDRIVDVFPAGEKEMVRSMLSESLTAVISQNLLKTHDGNGRVASHEILIANPAVRNLIRENKITQINS VLQTGQASGMQTMDQSLQSLVRQGLIAPEVARRRAQNSESMSF |
| 8 | 0068 | MQNNNEFKIGNRSVGYNHEPLIICEIGINHEGSLKTAFEMVDAAYNAGAEVVKHQTHIVEDEMSDEAKQVIPGNAD VSIYEIMERCALNEEDEIKLKEYVESKGMIFISTPFSRAAALRLQRMDIPAYKIGSGECNNYPLIKLVASFGKPII LSTGMNSIESIKKSVEIIREAGVPYALLHCTNIYPTPYEDVRLGGMNDLSEAFPDAIIGLSDHTLDNYACLGAVAL GGSILERHFTDRMDRPGPDIVCSMNPDTFKELKQGAHALKLARGGKKDTIIAGEKPTKDFAFASVVADKDIKKGEL LSGDNLWVKRPGNGDFSVNEYETLFGKVAACNIRKGAQIKKTDIE |
| 9 | 0089 | MNQTSRDLTRISHNTKIVATLGPGSNNVELLEDMIRVGGLNVVRFNFSHGTPEFHQENALIVREAAKRAGQEIAII ADLQGPKIRVGKIAGGGIELNKGETLVLDAALEGEGTREAVGLDYRDLPDDVAAGDVLWLDDGLLTLTVESVEGSR IITRVENSHVLKSNKGINKRGGGLSAGALTEKDFRDLKTAIAIGCDYLAISFVKSAEDLHIARAKVEEEMKGSTAV RPGLVSKIERVEAIENLDEIILAGDGIMVARGDLAVEVGHAAVPALQKRMIRRARELRRFSITATQMMESMITNPV PTRAEVSDVANAVLDGTDAVMCSAETAVGAYPFETVSQMAIICAAAEKEQDSLNGVAEQVEYPEAVSTNLAVAGGA VSVARAVHAKAIVALTESGSTAFEISRHNITLPIFALTPSVSAQRRMAMYRGVRPLILATSTDHDTALNEVETMLV EHNILHSGDQYIITSGSQMRESGSTNTLEVLRVK |
| 10 | 0109 | MLKCGTFFITRHIPRGCRRFFQPNQARQTEIYQIRGTVMQRRIITLLCAAGMAFSTQTLAANLEVRPNAPERYTVK |

| | | |
|---|---|---|
| | | QGDTLWGISGKYLYSPWQWGRLWDANRDQIHNPDLIYPDQVLVLRHVDGEPRLGLEQTDGIPVVKMSPDKEVSGYG IPAIDVNFYRIFMRHPQIVSRKETAAAPRLLSGPEGRLLYTKGTRVYTKGLKEPGRYLTYRINKNITDPDTGKFLG QEVAFSGIVRSLDYTDSVLEQRSKQAGERPKDNEYHTRTHPLITPLRTPSIQPLVVETAISEIQQGDYLMKMPEDT DRFNMMPHEPSRPVQAKIVSVFEGTRIAGQFQTITIDKGEADGLDKGTVLSLYKRKKTMQVDLSNNFKSRDTVELI STPAEEVGLAMVYRTSEHLSSAIILENISDISVGDTAANPGRDLDNIPDQGRSRVKFGFNRSE |
| 11 | 0115 | MRSSDILIVDDEIGIRDLLSEILQDEGYSVALAENAEEARKLRHQARPAMVLLDIWMPDCDGITLLKEWAKNGQLN MPVVMMSGHASIDTAVEATKIGAIDFLEKPISLQKLLSAVENALKYGAAQTETGPVFDKLGNSAAIQEMNREVGAA VKCASPVLLTGEAGSPFETVARYFHKNGTPWVSPARVEYLIDMPMELLQKAEGGVLYVGDIAQYSRNIQAGIAFIV GKAEHRRVRVVASGSRAAGSDGIACEEKLAELLSESVVRIPPLRMQHEDIPFLIQGIACNVAESQKIAPASFSEEA LAALTRYDWPGNFDQLQSVVATLLLEADGQEIGAGAVSSLLGQNVPAEGAEDMVGGFNFNLPLRELREEVERRYFE YHIAQEGQNMSQVAQKVGLERTHLYRKLKQLGIGVSRRAGEKTEE |
| 12 | 0124 | MAKEKFERSKPHVNVGTIGHVDHGKTTLTAALTTILAKKFGGAAKAYDQIDNAPEEKARGITINTSHVEYETETRH YAHVDCPGHADYVKNMITGAAQMDGAILVCSAADGPMPQTREHILLARQVGVPYIIVFMNKCDMVDDAELLELVEM EIRDLLSSYDFPGDDCPIVQGSALKALEGDAAYEEKIFELAAALDSYIPTPERAVDKPFLLPIEDVFSISGRGTVV TGRVERGIIHVGDEIEIVGLKETQKTTCTGVEMFRKLLDEGQAGDNVGVLLRGTKREDVERGQVLAKPGTITPHTK FKAEVYVLSKEEGGRHTPFFANYRPQFYFRTTDVTGAVTLEEGVEMVMPGENVTITVELIAPIAMEEGLRFAIREG GRTVGAGVVSSVIA |
| 13 | 0126 | MSKKWYVVQAYSGFEKNVQRILEERIAREEMGDYFGQILVPVEKVVDIRNGRKTISERKSYPGYVLVEMEMTDDSW HLVKSTPRVSGFIGGRANRPTPISQREAEIILQQVQTGIEKPKPKVEFEVGQQVRVNEGPFADFNGVVEEVNYERN KLRVSVQIFGRETPVELEFSQVEKIN |
| 14 | 0128 | MAKVSKRLKALRSSVEANKLYAIDEAIALVKKAATAKFDESVDVSFNLGVDPRKSDQVIRGSVVLPKGTGKITRVA VFTQGANAEAAKEAGADIVGFEDLAAEIKAGNLNFDVVIASPDAMRIVGQLGTILGPRGLMPNPKVGTVTPNVAEA VKNAKAGQVQYRTDKAGIVHATIGRASFAEADLKENFDALLDAIVKAKPAAAKGQYLKKVAVSSTMGLGIRVDTSS VNN |
| 15 | 0130 | MSLNIETKKVAVEEISAAIANAQTLVVAEYRGISVSSMTELRANARKEGVYLRVLKNTLARRAVQGTSFAELADQM VGPLVYAASEDAVAAAKVLHQFAKKDDKIVVKAGSYNGEVMNAAQVAELASIPSREELLSKLLFVMQAPVSGFARG LAALAEKKAGEEAA |
| 16 | 0131 | MAITKEDILEAVGSLTVMELNDLVKAFEEKFGVSAAAVAVAGPAGAGAADAEEKTEFDVVLASAGDQKVGVIKVVR AITGLGLKEAKDIVDGAPKTIKEGVSKAEAEDIQKQLEEAGAKVEIK |
| 17 | 0132 | MCMNYSFTEKKRIRKSFAKRENVLEVPFLLATQIDSYAKFLQLENAFDKRTDDGLQAAFNSIFPIVSHNGYARLEF VHYTLGEPLFDIPECQLRGITYAAPLRARIRLVILDKEASKPTVKEVRENEVYMGEIPLMTPSGSFVINGTERVIV SQLHRSPGVFFEHDKGKTHSSGKLLFSARIIPYRGSWLDFEFDPKDLLYFRIDRRRKMPVTILLKALGYNNEQILD IFYDKETFYLSSNGVQTDLVADRLKGETAKVDILDKEGNVLVAKGKRITAKNIRDITNAGLTRLDVEPESLLGKAL AADLIDSETGEVLASANDEITEELLAKFDINGVKEITTLYINELDQGAYISNTLRTDETAGRQAARVAIYRMMRPG EPPTEEAVEQLFNRLFFSEDSYDLSRVGRMKFNTRTYEQKLSEAQQNSWYGRLLNETFAGAADKGGYVLSVEDIVA SIATLVELRNGHGEVDDIDHLGNRRVRSVGELTENQFRSGLARVERAVKERLNQAESENLMPHDLINAKPVSAAIK EFFGSSQLSQFMDQTNPLSEVTHKRRVSALGPGGLTRERAGFEVRDVHPTHYGRVCPIETPEGPNIGLINSLSVYA RTNDYGFLETPYRRVIDGKVTEEIDYLSAIEEGRYVIAQANADLDSDGNLIGDLVTCREKGETIMATPDRVQYMDV ATGQVVSVAASLIPFLEHDDANRALMGANMQRQAVPCLRPEKPMVGTGIERSVAVDSATAIVARRGGVVEYVDANR VVIRVHDDEATAGEVGVDIYNLVKFTRSNQSTNINQRPAVKAGDVLQRGDLVADGASTDFGELALGQNMTIAFMPW NGYNYEDSILISEKVAADDRYTSIHIEELNVVARDTKLGAEDITRDIPNLSERMQNRLDESGIVYIGAEVEAGDVL VGKVTPKGETQLTPEEKLLRAIFGEKASDVKDTSLRMPTGMSGTVIDVQVFTREGIQRDKRAQSIIDSELKRYRLD LNDQLRIFDNDAFDRIERMIVGQKANGGPMKLAKGSEITTEYLAGLPSRHDWFDIRLTDEDLAKQLELIKVSLQQK REEADELYEIKKKKLTQGDELQPGVQKMVKVFIAIKRRLQAGDKMAGRHGNKGVVSRILPVEDMPYMADGRPVDIV LNPLGVPSRMNIGQILEVHLGWAAKGIGERIDRMLKEQRKAGELREFLNRLYNGSGKKEDLDALTDEEIIELASNL RKGASFASPVFDGAKESEIREMLNLAYPSDDPEVEKLGFNDSKTQITLYDGRSGEAFDRKVTVGVMHYLKLHHLVD EKMHARSTGPYSLVTQQPLGGKAQFGGQRFGEMEVWALEAYGAAYTLQEMLTVKSDDVNGRTKMYENIVKGEHKID AGMPESFNVLVKEIRSLGLDIDLERY |
| 18 | 0133 | MNLLNLFNPLQTAGMEEEFDAIKIGIASPETIRSWSYGEVKKPETINYRTFKPERDGLFCAKIFGPVKDYECLCGK YKRLKFKGVTCEKCGVEVTLSKVRRERMGHIELAAPVAHIWFLKSLPSRLGMVLDMTLRDIERVLYFEAFVVTDPG MTPLQRRQLLTEDDYYNKLDEYGDDFDAKMGAEGIRELLRTLNVAGEIEILRQELESTGSDTKIKKIAKRLKVLEA FHRSGMKLEWMIMDVLPVLPPDLRPLVPLDGGRFATSDLNDLYRRVINRNNRLKRLLELHAPDIIVRNEKRMLQEA VDSLLDNGRRGKAMTGANKRPLKSLADMIKGKGGRFRQNLLGKRVDYSGRSVITVGPYLRLHQCGLPKKMALELFK PFIFHKLEKQGLASTVKAAKKLVEQEVPEVWDILEEVIREHPIMLNRAPTLHRLGIQAFEPILIEGKAIQLHPLVC AAFNADFDGDQMAVHVPLSLEAQMEARTLMLASNNVLSPANGEPIIVPSQDIVLGLYYMTRDRINAKGEGSLFADV KEVHRAYHTKQVELGTKITVRLREWVKNEAGEFEPVVNRYETTVGRALLSEILPKGLPFEYVNKALKKKEISKLIN ASFRLCGLRDTVIFADHLMYTGFGFAAKGGISIAVDDMEIPKEKAALLAEANAEVKEIEDQYRQGLVTNGERYNKV VDIWGRAGDKIAKAMMDNLSKQKVIDRAGNEVDQESFNSIYMMADSGARGSAAQIKQLSGMRGLMAKPDGSIIETP ITSNFREGLTVLQYFIATHGARKGLADTALKTANSGYLTRRLVDVTQDLVVVEDDCGTSDGFVMKAVVQGGDVIEA |

| | | |
|---|---|---|
| | | LRDRILGRVTASDVVDPSSGETLVEAGTLLTEKLVDMIDQSGVDEVKVRTPITCKTRHGLCAHCYGRDLARGKLVN AGEAVGVIAAQSIGEPGTQLTMRTFHIGGAASRAAAASQVEAKSNGTARFSSQMRYVANNKGELVVIGRSCEVVIH DDIGRERERHKVPYGAILLVQDGMAIKAGQTLATWDPHTRPMITEHAGMVKFENVEEGVTVAKQTDDVTGLSTLVV IDGKRRSSSASKLLRPTVKLLDENGVEICIPGTSTPVSMAFPVGAVITVREGQEIGKGDVLARIPQASSKTRDITG GLPRVAELFEARVPKDAGMLAEITGTVSFGKETKGKQRLIVTDVDGVAYETLISKEKQILVHDGQVVNRGETIVDG AVDPHDILRLQGIEALARYIVQEVQEVYRLQGVKISDKHIEVIIRQMLRRVNIADAGETGFITGEQVERGDVMAAN EKALEEGKEPARYENVLLGITKASLSTDSFISAASFQETTRVLTEAAIMGKQDELRGLKENVIVGRLIPAGTGLTY HRSRHQQWQEVEQETAETQVTDE |
| 19 | 0137 | MPRRREVPKRDVLPDPKFGSVELTKFMNVLMIDGKKSVAERIVYGALEQIEKKTGKVAIEVFNEAIANAKPIVEVK SRRVGGANYQVPVEVRPSRRLALAMRWVRDAARKRGEKSMDLRLAGELIDASEGRGGALKKREEVHRMAEANKAFS HFRF |
| 20 | 0138 | MARKTPISLYRNIGISAHIDAGKTTTTERILFYTGLTHKLGEVHDGAATTDYMEQEQERGITITSAAVTSYWSGMA KQFPEHRFNIIDTPGHVDFTVEVERSMRVLDGAVMVYCAVGGVQPQSETVWRQANKYQVPRLAFVNKMDRQGANFF RVVEQMKTRLRANPVPIVIPVGAEDNFSGVVDLLKMKSIIWNEVDKGTTFTYGDIPAELVETAEEWRQNMIEAAAE ASEELMDKYLGGDELTEEEIVGALRQRTLAGEIQPMLCGSAFKNKGVQRMLDAVVELLPAPTDIPPVQGVNPNTEE ADSRQASDEEKFSALAFKMLNDKYVGQLTFIRVYSGVVKSGDTVLNSVKGTRERIGRLVQMTAADRTEIEEVRAGD IAAAIGLKDVTTGETLCAESAPIILERMEFPEPVIHIAVEPKTKADQEKMGIALNRLAKEDPSFRVRTDEESGQTI ISGMGELHLEIIVDRMKREFGVEANIGAPQVAYRETIRKAVKAEYKHAKQSGGKGQYGHVVIEMEPMEPGGEGYEF IDEIKGGVIPREFIPSVDKGIRDTLPNGIVAGYPVVDVRIRLVFGSYHDVDSSQLAFELAASQAFKEGMRQASPAL LEPIMAVEVETPEEYMGDVMGDLNRRRGVVLGMDDDGIGGKKVRAEVPLAEMFGYSTDLRSATQGRATYSMEFKKY SEAPAHIAAAVTEARKG |
| 21 | 0140 | MANQKIRIRLKAYDYALIDRSAQEIVETAKRTGAVVKGPIPLPTKIERFNILRSPHVNKTSREQLEIRTHLRLMDI VDWTDKTTDALMKLDLPAGVDVEIKVQ |
| 22 | 0142 | MTLGLVGRKVGMTRVFDEQGVSVPVTVLDMSANRVTQVKSKDTDGYTAVQVTFGQKKANRVNKAEAGHFAKAGVEA GRGLIEFALTEEKLAELKAGDEITVSMFEVGQLVDVTGTSKGKGFSGTIKRHNFGAQRTSHGNSRSHRVPGSIGMA QDPGRVFPGKRMAGQYGNTKATVQKLEVVRVDAERQLLLVKGAVPGAVNSDVVVRPSVKVGA |
| 23 | 0143 | MELKVIDAKGQVSGSLSVSDALFAREYNEALVHQLVNAYLANARSGNRAQKTRAEVKHSTKKPWRQKGTGRARSGM TSSPLWRKGGRAFPNKPDENFTQKVNRKMYRAGMATILSQLTRDERLFAIEALTAETPKTKVFAEQVKNLGLEQVL FVTKQLDENVYLASRNLPNVLVLEAQQVDPYSLLRYKKVIITKDAVAQLEEQWV |
| 24 | 0144 | MNQQRLTQVILAPIVSEKSNVLAEKRNQMTFKVLANATKPEIKAAVELLFGVQVADVTTVTIKGKVKRFGRTLGRR SDVKKAYVSLAAGQELDLEAAAAAADKE |
| 25 | 0154 | MARLREFYKETVVPELVKQFGYKSVMEVPRIEKITLNMGVGEAVADKKVMEHAVSDLEKIAGQKPVVTVARKSIAG FKIRDNYPVGCKVTLRRDQMFEFLDRLITIALPRVRDFRGVSGKSFDGRGNYNMGVREQIIFPEIEYDKIDALRGL NITITTTAKTDEEAKALLSLFKFPFKG |
| 26 | 0156 | MSMHDPISDMLTRIRNAQRANKAAVAMPSSKLKCAIAKVLKEEGYIEDFAVSSDVKSILEIQLKYYAGRPVIEQIK RVSRPGLRIYKASSEIPSVMNGLGIAIVSTSKGVMTDRKARSQGVGGELLCIVA |
| 27 | 0157 | MSRVAKNPVTVPAGVEVKFGAEALVIKGKNGELSFPLHSDVAIEFNDGKLTFVANNSSKQANAMSGTARALVSNMV KGVSEGFEKRLQLIGVGYRAQAQGKILNLSLGFSHPIVYEMPEGVSVQTPSQTEIVLTGSDKQVVGQVAAEIRAFR APEPYKGKGVRYVGEVVVMKEAKKK |
| 28 | 0159 | MAKHEIEERGDGLIEKMVAVNRVTKVVKGGRIMAFSALTVVGDGDGRIGMGKGKSKEVPVAVQKAMDQARRSMIKV PLKNGTIHHEVIGRHGATKVFMQPAKEGSGVKAGGPMRLVFDAMGIHNISAKVHGSTNPYNIVRATLDGLSKLHTP ADIAAKRGLTVEDILGVNHG |
| 29 | 0168 | MQNSTTEFLKPRQIDVNTFSATRAKVSMQPFERGFGHTLGNALRRILLSSMNGFAPTEVAIAGVLHEYSTVDGIQE DVVDILLNIKGIVFKLHGRSQVQLVLKKSGSGVVSAGDIELPHDVEILNPGHVICHLADNGQIEMEIKVEQGRGYQ SVSGRQVVRDENRQIGAIQLDASFSPISRVSFEVEPARVEQRTDLDKLVLDIETDGSIDPEEAVRSAARILIDQMS IFADLQGTPVEEVEEKAPPIDPVLLRPVDDLELTVRSANCLKAEDIYYIGDLIQRTETELLKTPNLGRKSLNEIKE VLASKGLTLGSKLEAWPPVGLEKP |
| 30 | 0171 | MAKIIVVTSGKGGVGKTTTSASIATGLALRGYKTAVIDFDVGLRNLDLIMGCERRVVYDLINVIQGEATLNQALIK DKNCENLFILPASQTRDKDALTREGVEKVMQELSGKKMGFEYIICDSPAGIEQGALMALYFADEAIVTTNPEVSSV RDSDRILGILQSKSHKAEQGGSVKEHLLITRYSPERVAKGEMLSVQDICDILHIPLLGVIPESQNVLQASNSGEPV IHQDSVAASEAYKDVIARLLGENREMRFLEAEKKSFFKRLFGG |
| 31 | 0173 | MTLTELRYIVAVAQERHFGRAARRCFVSQPTLSIAIKKLEEELAVSLFDRSSNDIITTEAGERIVAQARKVLEEAE LIRHLANEEQNELEGAFKLGLIFTVAPYLLPKLIVSLRRTAPKMPLMLEENYTHTLTESLKRGDVDAIIVAEPFQE PGIVTEPLYDEPFFVIVPKGHSFEELDAVSPRMLGEEQVLLLTEGNCMRDQVLSSCSELAAKQRIQGLTNTLQGSS INTIRHMVASGLAISVLPATALTENDHMLFSIIPFEGTPPSRRVVLAYRRNFVRPKALSAMKAAIMQSQLHGVSFI CD |
| 32 | 0193 | MTHMIYPKTYDVIVVGGGHAGTEAALAAARMGAQTLLLLSHNIETLGQMSCNPSIGGIGKGHLVRELDALGGAMALA |

431

| | | |
|---|---|---|
| | | TDKSGIQFRRLNASKGAAVRATRAQADRILYKAAIREMLENQENLDLFQQAVEDVTLDGERISGVITAMGVEFKAR AVVLTAGTFLSGKIHIGLENYEGGRAGDPAAKSLGGRLRELKLPQGRLKTGTPPRIDGRTIDFSQLTEQPGDTPVP VMSVRGNADMHPRQVSCWITHTNTQTHDIIRSGFDRSPMFTGKIEGVGPRYCPSIEDKINRFADKDSHQIFLEPEG LTTHEYYPNGISTSLPFDIQIALVRSMKGLENAHILRPGYAIEYDYFDPRNLKASLETKTIAGLFFAGQINGTTGY EEAAAQGLLAGANAVQYVRGQDPLLLRREQAYLGVLVDDLITKGVNEPYRMFTSRAEYRLQLREDNADMRLTEDGY KIGLVSEAQWRMFNEKREAVEREIQRLKTTWYTPQKLAEGEQIRVFGQKLSREANLHDLLRRPNLDYAALMTLEGA MPSENLSAEVIEQVEIQVKYQGYIDRQNEEIDSRRDIETLKLPDGIDYGKVKGLSAEVQQKLNQHKPETVGQASRI SGVTPAAVALLMVHLKRGFKDAK |
| 33 | 0196 | MKRMLFNATQAEELRVAIVDGQNLLDLDIETLGKEQRKGNIYKGIITRIEPSLEACFVDYGTDRHGFLPFKEVSRS YFQDYEGGRARIQDVLKEGMEVIVQVEKDERGNKGAALTTFISLAGRYLVLMPNNPRGGGVSRRIEGEERQELKAA MAELDIPNGMSIIARTAGIGRSAEELEWDLNYLKQLWQAIEEAGKAHHDPYLLFMESSLLIRAIRDYFRPDIGEIL VDNQEVYDQVAEFMSYVMPGNIGRLKLYEDHTPLFSRFQIEHQIESAFSRSVSLPSGGAIVIDHTEALVSIDVNSA RATRGADIEDTAFKTNMEAAEEVARQMRLRDLGGLVVIDFIDMENPKHQRDVENVLRDALKKDRARVQMGKLSRFG LLELSRQRLKPALGESSHVACPRCAGTGVIRGIESTALHVLRIIQEEAMKDNTGEVRAQVPVDVATFLLNEKRAEL FAMEERLDVNVVLIPNIHLENPHYEINRIRTDDVEEDGEPSYKRVAEPEEDESAKPFGGEKAKAARPEPAVKGVRH TSPAPTAAPEKKTSWWDSFKAWLKRIFGGSETQAAPAAETSEKRSTANRSGSRANNRRQNPRRSKREGSKVEVREV AGKTAGQEARADKAETRNNGNRRRNERGDRAAERANEAEIQSRNVQPAATVADAAPSETEVQTGKRRRNGSRSERG QTAPETATVAETTVQTAENTPSEPHTAEDKGSKPKSERNRRERDSRDAKERRERNNQRDRRQNGKKRNIPSAAKIE QYLNIHDTADKVRSAAAHVFGETDANAPITVSIADPVAERDLPTASPAVSNGDAPVYDAAEKIRRATAAILPEGAT PKAEAQEMPSETATFTAAAEQARETAQTGGLVLIETDPAALKAWAAQPEVQAGRGLRRSEQPKPSEVATVPAEEMI QVETRQG |
| 34 | 0212 | MTEQKHEEYGADSIQVLEGLEAVRKRPGMYIGDTQDGSGLHHMVFEVLDNAIDEALAGHCDKITVTIHADHSVSVA DNGRGMPTGIHPKEGRSAAEVIMTVLHAGGKFDNNSYKISGGLHGVGVSVVNALSDWVTLTIYRDGKEHFVRFVRG ETEEPLKIVGDSDKKGTTVRFLASTETFGNVEYSFDILAKRIRELSFLNNGVDIELTDERDGKHESFALSGGVAGF VQYMNRKKTPLHEKIFYAFGEKDGMSVECAMQWNDSYQESVQCFTNNIPQRDGGTHLTALRQVMTRTINNYIEANE VAKKAKVETAGDDMREGLTCVLSVKLPDPKFSSQTKDKLVSGEIGPVVNEVISQALTDFLEENPNEAKIITGKIVD AARAREAARKAREITRRKGVMDGLGLPGKLADCQEKDPALSELYLVEGDSAGGSAMQGRDRKFQAILPLKGKILNV EKARFEKMLASQEVATLITALGAGIGKEEFNAEKLRYHRIIIMTDADVDGAHIRTLLLTFFYRQMPELVERGYIYI AQPPLYKAKYGKQERYLKDELEKDQWLLGLALEKAKIISDGRTIEGAELADTAKQFLLAKTVIEQESRFVDELVLR AMLHASPIDLTSSENADKAVAELSGLLDEKEVALERIEGHEGHRFIKITRKLHGNVMVSYIEPKFLNSKAYQTLTQ TAAALKGMVGEGAKLYKGENGYDADSFETALDILMSVAQKGMSIQRYKGLGEMNPEQLWETTMDPAVRRLLKVRIE DAIAADEVFVTLMGDEVEPRRAFIENNALIAQNIDA |
| 35 | 0219 | MSQRRVVITGLGQVSPVGNTVAEAWDTLLTGKSGIGAITRFDTSDINSRVAGEVRGFDIGQYISAKEARRMDVFIH YGIAAALQAIADSGLDDVENLDKDRIGVNIGSGIGGLPGIEVTGKAVIEGGARKINPFFIPGSLINLISGHVTILK GYRGPSYGMVSACTTGAHAIGDSLRMIKYGDADIMVAGGAEGAISTLGVGGFAAMKALSTRNDDPATASRPWDKGR DGFVIGEGAGILVLEELEHAKKRGAKIYAEIVGFGMSSDAYHITAPNEEGPALAVTRALKDAGINPEDVDYVNAHG TSTPLGDANETKALKRAFGEHAYKTVVSSTKSMTGHLLGAAGGVEAVYSILAIHDGKIPPTINIFEQDVEAGCDLD YCANEARDAEIDVAISNSFGFGGTNGTLVFKRFKG |
| 36 | 0246 | MAIYQSGVIFDQVDTANPDCWTLDEYVKRGGYTALRKILSENISQTDVIDEVKTSGLRGRGGAGFPTGLKWSFMPR SFPGEKYVVCNTDEGEPGTFKDRDIIMFNPHALIEGMIIAGYAMGAKAGYNYIHGEIFEGYQRFEAALEQARAAGF LGKNILGSDFEFELFAHHGYGAYICGEETALLESLEGKKGQPRFKPPFPASFGLYGKPTTINNTETFSSVPFIIRD GGQAFADKGIPNAGGTKLFCISGHVERPGNYEVPLGTPFAEVLKMAGGMRGGKKLKAVIPGGSSAPVLPADIMMQT NMDYDSISKAGSMLGSGAIIVMDEDVCMVKALERLSYFYYDESCGQCTPCREGTGWLYRIVHRIVEGKGKMEDLDL LDSVGNQMAGRTICALADAAVFPVRSFTKHFRDEFVHYIEHGGPMKEHKWGGW |
| 37 | 0286 | MNFTRLLNQVLSTVQKKGNTFSDSPLNSFGGGALVAGVASMLLNGKNRKTITKIGSTAALGYLAYRGYQMWQQNKG RATVTQSDFQPAGETEETYSRTVLRTMIAAAASDGMIDEAERRTIEQESGTDPETAAWLAAEYRLPASIEDIAAAV GNDEALAAEAYLAARLVCADLSRKETVFLARLSQALKLDDNLVESLERQLGF |
| 38 | 0294 | MKLKTLALTSLTLLALAACSKQAETSVPADSAQSSSSAPAAPAELNEGVNYTVLSTPIPQQQAGKIEVLEFFGYFC PHCAHLEPVLSEHIKTFKDDTYMRREHVVWGDEMKPLARLAAAVEMAGESDKANSHIFDAMVNQKINLADTDTLKK WLSEQTAFDGKKVLAAFEAPESQARAAQMEELTNKFQISGTPTVIVGGKYQVEFKDWQSGMTTIDQLVDKVREEQK KPQ |
| 39 | 0329 | MSVGLLRILVQNQVVTVEQAEHYYNESQAGKEVLPMLFSDGVISPKSLAALIARVFSYSILDLRHYPRHRVLMGVL TEEQMVEFHCVPVFRRGDKVFFAVSDPTQMPQIQKTVSAAGIEVELVIVEDDQLAGLLDWVGSRSTSLLQELGEGQ EEEESHTLYIDNEEAEDGPVPRFIHKTLSDALRSGASDIHFEFYEHNARIRFRVDGQLREVVQPPIAVRGQLASRI KVMSRLDISEKRIPQDGRMQLTFQKGGKPVDFRVSTLPTLFGEKVVMRILNSDAASLNIDQLGFEPFQKKLLLEAI HRPYGMVLVTGPTGSGKTVSLYTCLNILNTESVNIATAEDPAEINLPGINQVNVNDKQGLTFAAALKSFLRQDPDI IMVGEIRDLETADIAIKAAQTGHMVFSTLHTNNAPATLSRMLNMGVAPFNIASSVSLIMAQRLLRRLCSSCKQEVE RPSASALKEVGFTDEDLAKDWKLYRAVGCDRCRGQGYKGRAGVYEVMPISEEMQRVIMNNGTEVDILDVAYKEGMV DLRRAGILKVMQGITSLEEVTANTND |

| | | |
|---|---|---|
| 40 | 0335 | MSLQNIIETAFENRADITPTTVTPEVKEAVLETIRQLDSGKLRVAERLGVGEWKVNEWAKKAVLLSFRIQDNEVLN DGVNKYFDKVPTKFADWSEDEFKNAGFRAVPGAVARRGSFVAKNVVLMPSYVNIGAYVDEGAMVDTWATVGSCAQI GKNVHLSGGVGIGGVLEPLQAAPTIIEDNCFIGARSEIVEGVIVEEGSVISMGVFIGQSTKIFDRTTGEIYQGRVP AGSVVVSGSMPSKDGSHSLYCAVIVKRVDAQTRAKTSVNELLRGI |
| 41 | 0336 | MGFLQGKKILITGMISERSIAYGIAKACREQGAELAFTYVVDKLEERVRKMAAELDSELVFRCDVASDDEINQVFA DLGKHWDGLDGLVHSIGFAPKEALSGDFLDSISREAFNTAHEISAYSLPALAKAARPMMRGRNSAIVALSYLGAVR AIPNYNVMGMAKASLEAGIRFTAACLGKEGIRCNGISAGPIKTLAASGIADFGKLLGHVAAHNPLRRNVTIEEVGN TAAFLLSDLSSGITGEITYVDGGYSINALSTEG |
| 42 | 0337 | MSRPVPAVFGSVFHSQMPVLAYREGKWQPTEWQSSQDLSLAPGAHALHYGSECFEGLKAFRQADGKIVLFRPTANI ARMRQSADILHLPRPETEAYLDALIKLVKRAADEIPDAPAALYLRPTLIGTDPVIGKAGSPSETALLYILASPVGD YFKVGSPVKILVETEHIRCAPHMGRVKCGGNYASAMHWVLKAKAEYGANQVLFCPNGDVQETGASNFILINGDEII TKPLTDEFLHGVTRDSVLTVAKDLGYTVSERNFTVDELKAAVENGAEAILTGTAAVISPVTSFVIGGKEIEVKSQE RGYAIRKAITDIQYGLAEDKYGWLVEVC |
| 43 | 0351 | MTILSDVKALGQQIWLDNLSRSLVQSGELAQMLKQGVCGVTSNPAIFQKAFAGDALYADEIAALKQQNLSPKQRYE TMAVADVRAACDVCLAEHESTGGKTGFVSLEVSPELSKDAQGTVEEARRLYAAIGCKNAMIKVPATDAGIDALETL VSDGISVNLTLLFSRAQTLKAYAAYARGIAKRLAAGQSVAHIQVVASFFISRVDGALDTTLPDHLKGKIAIALAKA AYQDWAQYFGSPEFAALETKGANRVQLLWASTGVKNPAYPDTLYVDSLIGAHTVNTVPDATLKAFIDHGTAKATLT EGVEEAQAQLAETAALGIDVETLATRLQEDGLKQFEEAFEKLLAPLA |
| 44 | 0359 | MSIKNAVKLIEESEARFVDLRFTDTKGKQHHFTVPARIVLEDPEEWFENGQAFDGSSIGGWKGIQASDMQLRPDAS TAFVDPFYDDATVVLTCDVIDPADGQGYDRDPRSIARRAEAYLKSSGIGETAYFGPEPEFFVFDGIEFETDMHKTR YEITSESGAWASGLHMDGQNTGHRPTVKGGYAPVAPIDCGQDLRSAMVNILEELGIEVEVHHSEVGTGSQMEIGTR FATLVKRADQTQDMKYVIQNVAHNFGKTATFMPKPIMGDNGSGMHVHQSIWKDGQNLFAGDGYAGLSDTALYYIGG IIKHAKALNAITNPSTNSYKRLVPHFEAPTKLAYSAKNRSASIRIPSVNSSKARRIEARFPDPTANPYLAFAALLM AGLDGIQNKIHPGDPADKNLYDLPPEEDALVPTVCASLEEALAALKADHEFLLRGGVFSKDWIDSYIAFKEEDVRR IRMAPHPLEFEMYYSL |
| 45 | 0382 | MTKQLKLSALFVALLASGTAVAGEASVQGYTVSGQSNEIVRNNYGECWKNAYFDKASQGRVECGDAVAAPEPEPEP EPAPAPVVVVEQAPQYVDETISLSAKTLFGFDKDSLRAEAQDNLKVLAQRLSRTNVQSVRVEGHTDFMGSDKYNQA LSERRAYVVANNLVSNGVPVSRISAVGLGESQAQMTQVCEAEVAKLGAKVSKAKKREALIACIEPDRRVDVKIRSI VTRQVVPAHNHHQH |
| 46 | 0390 | MYTRIMEISPWTLRSAKLEKEHKRLQESLTSLGNGYMGMRGSFEETYSADSHLGTYIAGVWFPDKTRVGWWKNGYP KYFGKAINAFNFSKVKIFVDGQEVDLAKNDVAGFSVELDMQHGVLRRSFTVFGVRFNVCKFLSVAQKELAVIRWEA VSVDGKTHQVRIDSIIDADVKNEDSNYEEKFWQVLDKGVSDSLSYIAAQTVANPFGVEQFIVNAEQTFAGSFKALG GSQTDWQVSNSFESEVGSTPETFEKRVIVTTSRDYQSLEAVKAAGRALSEKIAGVAFETLLDAHKAGWLHRWEIAD VVIEGSDEAQQGIRFNLFQLFSTYYGEDARLNIGPKGFTGEKYGGATYWDTEAYAVPLYLALAEPEVTRNLLQYRR NQLPQAQHNAREQGLAGALYPMVTFTGIECHNEWEITFEEIHRNGAIPYAIYNYTNYTGDEGYLAKEGLEVLVEVS RFWADRVHFSKRNGKYMIHGVTGPNEYENNINNNWYTNTLAAWVLDYTREALAKYPRPDLNVRADELEKWADISAN MYRPHDEELGVFVQHDGFLDKDIRPVSALSPDDLPLNQKWSWDKILRSPFIKQADVLQGIYFFSDRFNIDEKRRNF DFYEPMTVHESSLSPCIHSILAAELGKEEKAVEMYQRTARLDLDNYNNDTEDGLHITSMTGSWLAIVQGFAQMKTW GGKLSFAPFLPSAWTGYAFHINYRGRLIKVAVGKENVVFTLLKGESLDLQVYGKDITLDGSHTVALEK |
| 47 | 0391 | MTFTAVLFDLDGVITDTAEYHYRAWKKLAEELGISIDRKFNEQLKGVSRDDSLKRILAHGGKTVSEAEFAELTRRK NDNYVEMIQAVKPEDVYPGILPLLEALRANGKKIALASASKNGPFLLERMGLTHFFDAIADPAAVAHSKPAPDIFL AAAEGVDADIRQCIGIEDAAAGVAAIKAAGALPIGVGKAEDLGSDIALVSGTAELTYAYLQSVWEQSGR |
| 48 | 0426 | MEQQQRYISVLDIGTSKVLALIGEVQDDDKINIVGLGQAPSRGLRAGMVTNIDATVQAIRQAVNDAELMADTKITH VTTGIAGNHIRSLNSQGVVKIKDGEVTQADIDRAIETAKAINIPPDQKILDAVVQDYIIDTQLGVREPIGMSGVRL DTRVHIITGASTAVQNVQKCIERCGLKSDQIMLQPLASGQAVLTEDEKDLGVCVIDIGGGTTDIAVYMNGAIRHTS VIPAGGNLITKDLSKSLRTPLDAAEYIKIHYGVASCDTEGLGEMIEVPGVGDRTSRQVSSKVLAAIISARIQEIFG VVLGELQKSGFPKEVLNAGIVLTGGVSMMTGIVEFAEKIFDLPVRTGAPQEMGGLSDRVRTPRFSTAIGLLHAACK LEGNLPQPENGAVQEREGGGGLLARLKRWIENSF |
| 49 | 0427 | MEFVYDVAESAVSPAVIKVIGLGGGGCNAINNMVANNVRGVEFISANTDAQSLAKNHAAKRIQLGTNLTRGLGAGA NPDIGRAAAQEDREAIEEAIRGANMLFITTGMGGGTGTGSAPVVAEIAKSLGILTVAVVTRPFAYEGKRVHVAQAG LEQLKEHVDSLIIIPNDKLMTALGEDVTMREAFRAADNVLRDAVAGISEVVTCPSEIINLDFADVKTVMSNRGIAM MGSGYAQGIDRARMATDQAISSPLLDDVTLDGARGVLVNITTAPGCLKMSELSEVMKIVNQSAHPDLECKFGAAED ETMSEDAIRITIIATGLKEKGAVDFVPAREVEAVAPSKQEQSHNVEGMIRTNRGIRTMNLTAADFDNQSVLDDFEI PAILRRQHNSDK |
| 50 | 0446 | MSQTIDELLLPHRNAIDTIDAEILRLLNERAQHAHAIGELKGTGAVYRPEREVAVLRRIQDLNKGPLPDESVARLF REVMSECLAVERPLTIAYLGPQGTFTQQAAIKHFGHAAHTMACPTIDDCFKQVETRQADYLVAPVENSTEGSVGRT LDLLAVTALQACGEIVLRIHHNLLRKNNGSTEGIAKVFSHAQALAQCNDWLGRHLPNAERIAVSSNAEAARLVAES DDGTVAAIAGRTAAEIYGLDMVAECIEDEPNNTTRFLVMGHHETGASGSDKTSLAVSAPNRAGAVASLLQPLTESG |

| | | |
|---|---|---|
| | | ISMTKFESRPSKSVLWEYLFFIDIEGHRRDAQIQTALERLGERASFVKVIGSYPTAVL |
| 51 | 0462 | MQAFSLYPPVGHPDSAKKRQNRADVFLPFWKQDNFLGKSVQYRFEFAQIYFTMPKPCAGVTMGRGDFFFSNLFHQE SARMKKSVLAVLAALSLAACGGSEKNAVQPQADAASAANAEAAATDTLNIYNWSNYVDESTVEDFKKANNLKLTYD LYENNETLEAKMLTGKSGYDLVVPGIAFLPRQIEAGAYQKVNKDLIPNYKNIDPELLKMLETADPGNQYAVPYFSG VNTIAITAKGKELLGGKLPENGWDLLFKPEYTHKLKSCGIALWDTPSEMFPILLNYLGKDPKGSNPEDLKAAAEVL KSIRPDVKRFSPSIIDELARGDICLAAGNGGDLNLAKARSEEVKNNVGIEVLTPKGMGFWIESWLIPADAKNVANA HKYINYTLDPEIAAKNGIAVTFAPASKPAREKMPAELVNTRSIFPNEQDMKDGFVMPQMSTDAKKLSVSLWQKIKV GTN |
| 52 | 0466 | MRTNYCGLISEQYLDQTVTVKGWVHRRRDHGGVIFIDLRDREGIVQVVIDPDTPEAFAAADSSRNEYVLSITGRVR NRPEGTTNDKMISGKIEILAKEIEVLNAAATPPFQIDDENISENVRLTNRVIDLRRPVMQRNLRLRYQVAMGVRRY LDAQGFIDIETPMLTRSTPEGARDYLVPSRVHPGEFFALPQSPQLFKQLLMVAGFDRYYQITKCFRDEDLRADRQP EFTQIDLETSFLNEDEIMDITEGMAKQVFKDALNVDLGDFPRMPYSEAMFYYGSDKPDMRINLKFTELTDLMKTEE FKVFRGAADMKGGRVVALRVPNGAEFSRKEIDEYTKFVGIYGAKGLAYIKVNDVSNLSNGEDSGLQSPIVKYLSEN ALKEIIARTGAQNGDIIFFGADKAKVVNEAIGALRIKVGLEHGKDNGYFTDEWKPLWVVDFPMFEYDEEADRYVAV HHPFTAPKEGHEDLMVSDPANCLARAYDMVLNGWEIGGGSIRIHRADVQEKVFAALKISPEEQQEKFGFLLDNLKF GAPPHGGLAFGLDRLVTLMTGAESIRDVIAFPKTQRAQCLLTNAPNSVDDKQLRELSLRLRQKATETKEV |
| 53 | 0477 | MRENKIIFTGPVGVGKTTAIAAISDEALVQTDASASDMTLDRKRNTTVAMDYGAISLDEDTKVHLYGTPGQERFNF MWEILSQGSMGLVLLLDNARTNPLKDLEFFLHSFRGLLEKAPVVVGITKMDIRSQPGIDVYHKYLAKHNLNVPVFE IDARKEDDVKQLVSAMLFSIDPGLEV |
| 54 | 0530 | MTVPHIPRGPVMADIAAFRLTEEEKQRLLDPAVGGIILFRRNFQNIEQLKTLTAEIKALRTPELIIAVDHEGGRVQ RFIEGFTRLPAMSTLGEIWDKDGASAAETAAGQVGRVLATELSACGIDLSFTPVLDLDWGNCPVIGNRSFHRNPEA VARLALALQKGLTKGGMKSCGKHFPGHGFVEGDSHLVLPEDWRSLSELETADLAPFRIMSREGMAAVMPAHVVYPQ VDTKPAGFSEIWLKQILRRDIGFKGVIFSDDLTMEGACGAGGIKERARISFEAGCDIVLVCNRPDLVDELREDFRI PDNPTLAQRWQYMANTLGSAAAQAVMQTADFQAAQAFVAGLASPQDTAGGVKVGEAF |
| 55 | 0540 | MFFKHIEAAPADPILGLGEAFKAETRPEKVNLGIGVYKDASGATPLVKAVKEAEKRLLESETTKNYLTIDGVADYN AQTQILLFGKDHEIIASRRAKTAQSLGGTGALRIAAEFAKRQLNAQTIWISNPTWPNHNAIAKAVGIQDKPYRYYD AAKHGLDWDGMIEDLSQAQKGDIVLLHGCCHNPTGIDPTPEQWETLAKLSAEKGWLPLFDFAYQGFGNGLEEDAYG LRVFLKHNTELLIASSYSKNFGMYNERVGAFTLVAEDEETAARAHSQVKTIIRTLYSNPASHGANTIALVLKNDDL KAQWIAELDEMRGRIKAMRQKFVGLLKAKGASQNFDFIIKQNGMFSFSGLTPEQVDRLKNEFAIYAVRSGRINVAG ITDNNIDYLCESIVKVL |
| 56 | 0546 | MKMQAVVVNKNVAGDVEVIEREVRPLEYGEALVEVEYCGVCHTDLHVAAGDYGEKPGRVLGHEGIGLVKEVADGVK NLKVGDRVSIAWLFQSCGSCEYCNTGRETLCRSVLNAGYTADGGMATHCIVSADYAVKVPEGLDPAQASSITCAGV TTYKAIKVSGVRPGQWIAIYGAGGLGNLGVQYAKKVFGAHVVAIDINDDKLAFAKETGADLVVNAAKEDAAKVIQE KTGGAHAAVVTAVSAAAFNSAVNCVRAGGRVVAIGLPPESMDLSIPRLVLDGIEVVGSLVGTRKDLEEAFQFGAEG LVVPKVQLRALDEAPAIFQEMREGKITGRMVIDMKKECGCGHHH |
| 57 | 0564 | MEIILGIVMFTVIVLVLALMILFAKSKLVSEGDITIKVNGEKELTMPAGGKLLGALANEGIFIPSACGGGGSCGQC RVVVKSGGGDILPTELSHISKREAREGCRLSCQVNVKTDMDIEVPEEVFGVKKWECTVISNDNKATFIKELKLAIP EGEEVPFRAGGYIQIEAPPHTVAYKDFDIPKEYHEDWDKYNLWQYVSKVDEPILRAYSMASYPEEKGIIMLNVRIA TPPPRVPDAPPGQMSSYIWSLKPGDKVTISGPFGEFFAKDTDAEMVFIGGGAGMAPMRSHIFDQLKRLNSKRKITF WYGARSKREMFYVEDFDQLAAEFPNFTWHVALSDPLPEDNWDGYTGFIHNVVYENHLKNHEAPEDCEFYMCGPPIM NQSVIKMLKDLGVEDENILLDDFGG |
| 58 | 0589 | MNLIQQLEQEEIARLNKEIPEFAPGDTVVVSVRVVEGTRSRLQAYEGVVIARRNRGLNSNFIVRKISSGEGVERTF QLYSPTVEKIEVKRRGDVRRAKLYYLRGLTGKAARIKEKLPARKG |
| 59 | 0595 | MSRVLLVDDDALLTELLTEYLSAEGLNVRSVPDGEAGVQEILSGQYDVVVLDSMMPKMNGLDVLKNVRARSTVPII MLTAKGDDIDRIIGLEMGADDYVPKPCTPRELLARINAILRRAQHSGEQNNAPNSISVSDVVLYPAKRQASVKDMP LELTSTEFNLLEVLMRHAGQVVSKETLSVEALDRKLAKFDRSIDVHISSIRHKLGDASLIQTVRGLGYLFVKN |
| 60 | 0604 | MKAARFYDKGDIRIEDIPEPTVAPGTVGINVAWCGICGTDLHEFMEGPIFIPPCGHPHPISGESAPVTMGHEFSGV VYAVGEGVDDIKVGQHVVVEPYIIRDDVPTGEGSNYHLSKDMNFIGLGGCGGGLSEKIAVKRRWVHPISDKIPLDQ AALIEPLSVGHHAYVRSGAKEGDVALVGGAGPIGLLLAAVLKAKGIKVIITELSKARKDKARESGVADYILDPSEV DVVAEVKKLTNGEGVDVAFECTSVNKVLDTLVEACKPAANLVIVSIWSHPATINVHSVVMKELDVRGTIAYCNDHA ETIKLVEEGKINLEPFITQRIKLDELVSKGFERLIHNNESAVKIIVSPNL |
| 61 | 0610 | MLPVRFTRVSDGIKRLTPASNQTAFYHPFENPFCRYSSFYWSIAIMTATTASSAKPYLKIQGLVKKFGDNYAVDNI DLDIYQHEIFALLGSSGSGKSTLLRMLAGMESPNQGKIILDGQDITKLAPYDRPINMMFQSYALFPHMTVEQNIAF GLKQDKMPKGEIAARVEEMLRLVQMTKFAKRKPHQLSGGQQQRIALARSLAKRPKILLLDEPLGALDKKLRQQTQL ELVNTLEQVGVTCIMVTHDQEEAMTMATRIAIMSDGQLQQVGTPSDVYDYPNSRFTAEFIGETNIFDGVVIEDHAD YAVIECEGLENHVRIDHGLGGPSEQDLWVSIRPEDIDLYKEKPEYLGDYNWAKGTVKEIAYLGSFAIYHIKLGNGR VVKSQVPAPYWYVRNITPPTWDETVYISWPENQPTPLFR |

| 62 | 0617 | MHVSELQTLHISKLLELAEEHGIENANRFRKQDLVFAIVRQMMKKGEGFTCSGTLEILPDGFGFLRSADTSYLAGP DDIYVSPTQIRRFNLHTGDTIEGSVRVPKDNERYFALVRLDTINGDHPEVCRHKILFENLTPLFPTEQLKLERDLK SEENLTGRAIDLISPIGKGQRALLVAPPKSGKTVMLQNIAHAVTANYPEVELIVLLIDERPEEVTEMSRSVRGEVV SSTFDEPAQRHVQVAEMVLEKAKRMVEHKKDVVILLDSITRLARAYNTVVPTSGKILTGGVDANALHRPKRFFGAA RNVEEGGSLTIIATALVETGSRMDDVIYEEFKGTGNMELHLDRRMAEKRLFPAININKSGTRREELLVPNDQLQRM WLLRKFLHPMDEIEAAEFLIGKIKASKNNDDFFELMRGK |
| 63 | 0618 | MADNYVIWFENLRMTDVERVGGKNASLGEMISQLTEKGVRVPGGFATTAEAYRAFLAHNGLSERISAALAKLDVED VAELARVGKEIRQWILDTPFPEQLDAEIEAAWNKMVADAGGADISVAVRSSATAEDLPDASFAGQQETFLNINGLD NVKEAMHHVFASLYNDRAISYRVHKGFEHDIVALSAGVQRMVRSDSGASGVMFTLDTESGYDQVVFVTSSYGLGEN VVQGAVNPDEFYVFKPTLKAGKPAILRKTMGSKHIKMIFTDKAEAGKSVTNVDVPEEDRNRFSITDEEITELAHYA LTIEKHYGRPMDIEWGRDGLDGKLYILQARPETVKSQEEGNRNLRRFAINGDKTVLCEGRAIGQKVGQGKVRLIKD ASEMDSVEAGDVLVTDMTDPDWEPVMKRASAIVTNRGGRTCHAAIIARELGIPAVVGCGNATELLKNGQEVTVSCA EGDTGFIYAGLLDVQITDVALDNMPKAPVKVMMNVGNPELAFSFANLPSEGIGLARMEFIINRQIGIHPKALLEFD KQDDELKAEITRRIAGYASPVDFYVDKIAEGVATLAASVYPRKTIVRMSDFKSNEYANLVGGNVYEPHEENPMLGF RGAARYVADNFKDCFALECKALKRVRDEMGLTNVEIMIPFVRTLGEAEAVVKALKENGLERGKNGLRLIMMCELPS NAVLAEQFLQYFDGFSIGSNDMTQLTLGLDRDSGLVSESFDERNPAVKVMLHLAISACRKQNKYVGICGQGPSDHP DFAKWLVEEGIESVSLNPDTVIETWLYLANELNK |
| 64 | 0623 | MKKTLVAAAILSLALTACGGGSDTAAQTPSAKPEAEQSGKLNIYNWSDYVDPETVAAFEKETGIKTRSDYYDSNET LEAKVLTGKSGYDLTAPSIANVGRQIKAGAYQKIDKAQIPHYGNIDKDLLKMMEAVDPGNEYAVPYFWGINTLAIN TQQVKKALGTDKLPENEWDLVFKPEYTAKLKSCGISYFDSAIEQIPLALHYLGKDPNSENPEDIKAAVDMMKAVRG DVKRFSSSGYIDDMAAGNLCAAIGYGGDLNIAKTRAEEAANGVEIKVLTPKTGVGVWVDSFMIPRDAQNVANAHRY IDYTLRPEVAAKNGSFVTYAPASRPARELMDEKYTSDASIFPNKELMEKSFIVSPKSAESVKLGVKLWQGLKAGK |
| 65 | 0631 | MAKVLIVPVSAGLDASAAAQAFAKALDAQIFQAVDATAETLLAQGKSDDWFDALVGKVAALDAANLVIEGIAPDAD KIYLAGKNVELALSLDAAAVFAVRSDNADADELANRVNLAKQFFAAAPGVLEGFVVDGAAASVAEAAAEKTGLTFF GSSDALKDVSVLAGREAKRLSPAQFRYNLIDFARQADKRIVLPEGAEPRTVQAAAICHEKGIARCVLLAKREEVEA VAKERGISLPDSLEIIDPASLVEQYVEPMCELRKSKGLTPEDARKQLQDTVVLGTMMMAQNDVDGLVSGAVHTTAN TIRPALQLIKTAPGASLVSSVFFMLLPNQVLVFGDCAVNPNPTAQQLADIAIQSADSAKAFGIDPKVAMISYSTVN SGSGPDVDTVIEATKLAREKRPDLAIDGPLQYDAATVPGVGKSKAPGSPVAGQATVLVFPDLNTGNCTYKAVQRNA NVLSVGPLLQGLRKPVNDLSRGALVEDIVFTIALTAVQAKQMEG |
| 66 | 0634 | MKTSIRYALLAAALTAATPALADITVYNGQHKEAAQAVADAFTRATGIKVKLNSAKGDQLAGQIKEEGSRSPADVF YSEQIPALATLSAANLLEPLPASTINETRGKGVPVAAKKDWVALSGRSRVVVYDTRKLSEKDLEKSVLNYATPKWK NRIGYAPTSGAFLEQVVAIVKLKGEAAALKWLKGLKEYGKPYAKNSVALQAVENGEIDAALINNYYWHAFAREKGV QNVHTRLNFVRHRDPGALVTYSGAAVLKSSQNKDEAKKFVAFLASKEGQRALTAVRAEYPLNPHVVSTFNLEPIAK LEAPQVSATTVSEKEHATRLLEQAGMK |
| 67 | 0637 | MHDKTWSGRFNEPVSELVKQYTASIGFDRRLAEWDIQGSLAHAQMLKETGVLDEGDLADIRRGMAEILEEIRSGKI EWSSDLEDVHMNIERRLTDKIGDAGKRLHTGRSRNDQVATDIRLWLRDQITVIQSLIQSLQTALLDLAEQNAETVM PGFTHLQVAQPVSFGHHMLAYVEMLGRDNERMADCRCRVNRMPLGAAALAGTTYPIQREITAELLGFEQICQNSLD AVSDRDFAIEFTAAASLVMVHLSRLSEELILWMSPRFGFIDIADRFCTGSSIMPQKKNPDVPELVRGKSGRVIGHL IGLITLMKSQPLAYNKDNQEDKEPLFDTADTLIDTLRIYADMMRGVTVKPDNMRAAVMQGFATATDLADYLVKKGM PFRDAHEVVAQAVRHADQAGVDLSELPLEVLQGFSDLIADDVYGVLTPEGSLNARNHLGGTAPEQVRFQVKRWREM LA |
| 68 | 0641 | MADFNQILTPGDVDGGIINVVNEIPAGSNHKIEWNRKLAAFQLDRVEPAIFAKPTNYGFIPQTLDEDGDELDVLLV TEQPLATGVFLEARVIGVMKFVDDGEVDDKIVCVPADDRNNGNAYKTLSDLPQQLIKQIEFHFNHYKDLKKAGTTK VESWGDAEEAKKVIKESIERWNKQA |
| 69 | 0663 | MKKALATLIALALPAAALAEGASGFYVQADAAHAKASSSLGSAKGFSPRISAGYRINDLRFAVDYTRYKNYKAPST DFKLYSIGASAIYDFDTQSPVKPYLGARLSLNRASVDLGGSDSFSQTSIGLGVLTGVSYAVTPNVDLDAGYRYNYI GKVNTVKNVRSGELSAGVRVKF |
| 70 | 0697 | MKEHKARKRFGQNFLQDTRIISDIVNAVRPQADDVVIEIGPGLAAITEPLAKKLNRLHVVEIDRDIVCRLKTLPFA DKLVIHEGDVLQFDFNGIAGKKKIVGNLPYNISTPLLFKLAEVADDVVDMHFMLQKEVVERMVAAPKSNDYGRLGV MLQYFFDMEMLIDVPPESFDPAPKVDSAVVRMIPVKHRIGKADDFEHFAKLVKLAFHQRRKTIRNNLKELAGDDDL QAVGINPQDRAEHIAPEKYVALSNYLAGKVV |
| 71 | 0703 | MKKILLTVSLGLALSACATQGTVDKDAQITQDWSVEKLYAEAQDELNSSNYTRAVKLYEILESRFPTSRHAQQSQL DTAYAYYKDDEKDKALAAIDRFRRLHPQHPNMDYALYLRGLVLFNEDQSFLNKLASQDWSDRDPKANREAYQAFAE LVQRFPNSKYAADATARMVKLVDALGGNEMSVARYYMKRGAYIAAANRAQKIIGSYQNTRYVEESLAILELAYKKL DKPRLAADTRRVLETNFPKSPFLKQPWRSDDMPWWRYWH |
| 72 | 0711 | MSSGNIHIHSMTGFANAAAECGSKRINLDLRAVNHRFLDIQIRMPDDLRYLESGIREKISSHIARGKVECKIQIQD AENGSQSLELNRDLVGQLAEINKDLRKHHDLAKLGVADILRFPGVLASQRENTEELAKSITELTEKALKDFTAARK REGKKLGEHLLQRLEAMEEIIDALSELFPTLLETHKEKIRARLVEAVGSIDNDRLQQEFALFIQKSDIDEEFSRLR |

| | | THIAEVRRIVTEHKGSSGKRLDFLMQELNREANTLGSKSIAAECTQASVELKVLIEQMREQVQNIE |
|---|---|---|
| 73 | 0720 | MLNITLPDGSVRQYESPVTVAQIAASIGAGLAKATVAGRVNGKLVDACDPIVEDSAVQIITPKDQEGIEIIRHSCA HLVGHAVKQLYPNAKMVIGPVIEEGFYYDIATEKPFTPEDVAAIEARMKELIAQDYDVVKIMTPRAEAIKIFQERG EEYKLRLIDDMPEVEAMGMYHHQEYVDMCRGPHVPNTRFLKNFKLTKLAGAYWRGDSNNEMLQRIYGTAWATKDEL KAYIQRIEEAEKRDHRKLGKQLDLFHLQDEAPGMVFWHPKGWALWQVIEQHMRKELNAAGYKEVKTPQIMDKTFWE KSGHWDNYKDNMFVTSSEKREYAVKPMNCPGHVQIFNNGLRSYRDLPMRLAEFGSCHRNEPSGALHGLMRVRGFVQ DDAHIFCTEDQIVSEARAFNELLIRIYKQFGFHDVSVKLSLRPEKRAGSDDVWDKAEQGLREALTACGVEWGELPG EGAFYGPKIEYHVRDALGRSWQCGTLQLDFVLPERLNAEYVTENNDRARPVMLHRAILGSLERFIGILIENHAGSF PLWLAPVQLVIMNITENQADYCREVAAKLQAAGFRAELDLRNEKIGYKIRDNSQYRFPYQIVVGDKEKQENKVAVR RKAEDLGSLDLDDFIAQLQQEITDALVNH |
| 74 | 0724 | MENVNRIVAEGIAAVEAAQDFNALEQIKARYLGKTGELTGLLKTLGQMSPEERKTIGAHINECKNRFQTAFNAKRE ALNEVKLQARLAAEALDITLPGRAQEGGSLHPVTLTLQRVVELFHGMGFEVADGPEIEDDFHNFQALNIPANHPAR AMQDTFYVENGDVLRTHTSPIQIRYMLDKKEPPIRIIAPGRVYRVDSDATHSPMFHQAEGLWVEEGVTFADLKAVF TDFIRRFFERDDLQVRFRPSFFPFTEPSAEIDIMGENGKWLEVGGCGMVHPNVLKNVNIDPEKYTGFAFGIGLDRF AMLRYNVNDLRLFFDNDLNFLKQFAK |
| 75 | 0728 | MQFSYSWLKTQADTELSSDKLEHLLTMSGLEVEEAETAAPAFAGVVIAEVKSVEKHPDADRLNVTRVDAGTGGLVQ IVCGAPNVKAGIKVPCSLPGAVLPGNFKIKPTKMRGEVSDGMLCSTDELGLPDDGVNGLHILPEDAPVGTNIREYL DLDDTLFTLKITPNRADCLSIKGIAREVSALTGCAFRQPEIHTAPITGSRKQPVQINAPADCGRFISRVIENVNAR ATTPDWMKQRLERSGIRSISALVDIGNYVMLEIGQPMHVFDADKLSGSLHIRRAREGETLECLNEKTVSLSENTLV VADEKGVLSLAGLMGGAASAVSDGTQNIVLEAAWFAPEIIAGKSRQYGFGSDSSFRFERGVDYRLQADAIERATEL VLQICGGAAGEMVEAQGELPEAKQVGLRLDRLKTVLGVDIPAEQVETILQHLGLQPEKTAEGFRVTAPSFRFDIEI EADLIEEIGRVYGYENIPDDYTSGRLKMLELPETRRPRFAVYNEMAARGYREVVSYAFVDEQWEQDFAANADPIRL QNPLAAQYAVMRSTLIGGLVEILQNNLNRKQNRVCVFEIARVFSKGSDGQFVQNERIGGLWYGAVMPEQWGGKTRN ADFYDIKADVENLLKNKAVEFVKTGHPALHPGRAANIVSDGKVIGFVGELHPKWLQKYDLPQAPLVFEIDMAAVLE CGKTRYRVVSKFQPVRRDLAFVMPEAMSHDDLLLVLKGAANKLVQEISVFDVYRGTGLPEGMKSVAVKVILQDMEN TLTDEAVEPLIGKLIGAATAAGARLRS |
| 76 | 0743 | MGGQKTHFGFSTVNEDEKAGKVAEVFHSVAKNYDIMNDVMSAGLHRVWKHFTINTAHLKKGDKVLDIAGGTGDLSR GWAKRVGKEGEVWLTDINSSMLTVGRDRLLNEGMILPVSLADAEKLPFPDNYFNLVSVAFGLRNMTHKDAALKEMY RVLKPGGTLLVLEFSKIYKPLEGAYDFYSFKLLPVMGRLIAKDAESYQYLAESIRMHPDQETLKQMMLDAGFDSVD YHNMSAGIVALHKGVKF |
| 77 | 0757 | MSEIGLVKIYFGKVRDLYEIDDKRMLMVASDRLSAFDVILDDPIPSKGEILTQISNFWFKKLAHIMPNHFTGQTVY DVLPENEAKALEKRAVVAKKLTPVKVEAIVRGYLAGSGWKDYQKTGSVCGIQLPEGMQEAQQLPEVIFTPSTKAAV GDHDENISFEECGRIIGKELAEEVRAKAVRLYTEAAEYAKSRGIIICDTKFEFGLDENGTLTLMDEVLTPDSSRFW PADQYKVGTNPPSFDKQFVRDWLEQSGWNKKAPAPKVPADVIQKTVEKYREALTLLTQD |
| 78 | 0758 | MMFDKHVKTFQYGNQTVTLETGEIARQAAAAVKVSMGDTVVLVAVTTNKEVKEGQDFFPLTVDYLERTYAAGKIPG GFFKREGKQSEKEILTSRLIDRPIRPLFPEGFYHDIQIVAMVVSVDPEIDSDIPAMLGASAALVLSGVPFAGPIGA ARVGYVNGVYVLNPTKAELAKSQLDLVVAGTSKAVLMVESEAKILPEDVMLGAVVYGHDQMQVAINAINEFADEVN PELWDWKAPETNEELVAKVRGIAGETIKEAFKIRQKQARSAKLDEAWSAVKEALITEETDTLAANEIKGIFKHLEA DVVRSQILDGQPRIDGRDTRTVRPLNIQTSVLPRTHGSALFTRGETQALAVATLGTSRDEQIIDALSGEYTDRFML HYNFPPYSTGEVGRMGAPKRREIGHGRLAKRALLAVLPKPEDFSYTMRVVSEITESNGSSSMASVCGGCLSLLSAG VPLKAHVAGIAMGLILEGNKFAVLTDILGDEDHLGDMDFKVAGTTEGVTALQMDIKIQGITKEIMQIALAQAKEAR LHILDQMKAAVAGPQELSAHAPRLFTMKINQDKIREVIGKGGETIRSITAETGTEINIAEDGTITIAATTQEAGDA AKKRIEQITAEVEVGKVYEGTVVKILDNNVGAIVSVMPGKDGLVHISQIAHERVRNVGDYLQVGQVVNVKALEVDD RGRVRLSIKALLDAPAREENAAE |
| 79 | 0763 | MKIANSITELIGNTPLVKLNRLTEGLKAEVAVKLEFFNPGSSVKDRIAEAMIEGAEKAGKINKNTVIVEATSGNTG VGLAMVCAARGYKLAITMPESMSKERKMLLRAFGAELILTPAAEGMAGAIAKAKSLVDAHPDTYFMPRQFDNEANP EVHRKTTAEEIWRDTDGKVDVFVAGVGTGGTITGVGEVLKKYKPEVKVVAVEPEASPVLSGGEKGPHPIQGIGAGF IPTVLNTKIYDSITKVSNEAAFETARAIAEKEGILVGISSGAAVWSALQLAKQPENEGKLIVVLLPSYGERYLSTP LFADLA |
| 80 | 0778 | MPSETSSPRKENETEVHIPAAPFIVKQSGSNALAVCALVLAALGLGTSGFLFVQGQNVLKNQELAFNQKIDKAALG ESENAALLKDNLNRQAAIQSELDRLDGNVKANGEQILEMQKSYRELTKGRADWLVDETETILNLAAQQLVLTGNIQ TAVGVLEHIDSRLSRFNQAELLPIKQAVSSDLAELKNRPYVDISGTALRLDRLETAVSGLPLMLDGVLKPGVQVKN EAASASWWQNVWEKSLGTLKGLVEIRRLENNDAMLISPEQAYFVRENLRLRLLDARTALMQRNSEVYQGDLNNAEA AVRQYFDAKSPATQSWLKELAELKALDVRMTADDGLKNSLNAVRAYRDGTRMTAAENQEAEQAASEPANEKTASEP AAASDVKTIEAPSLPSERKPEQPAKKQTVPEKAGRSPSAKGERA |
| 81 | 0791 | MIILHTNKGDIKIELDFDKAPVTAKNFEQYVKDGFYDGVIFHRVIKGFMIQGGGMDENMNEKETRDPIQNEASNGL PNDKYTIAMARTSDPHSASAQFFINTADNAFLNFRSKELYGKTVVQDWGYAVFGKVVDGFDVVDAIEGVSTKRHGY HDDVPSEPVVIIKAEAV |

| 82 | 0804 | MTVLSKEQVLSAFKNRKSCRHYDAARKISAEDFQFILELGRLSPSSVGSEPWQFIVVQNPEIRQAIKPFSWGMADA<br>LDTASHLVVFLAKKNARSDSPFMLESLKRRGVTEPDAVAKSLARYQAFQADDIKILDDSRALFDWCCRQTYIALAN<br>MMTGAAMAGIDSCPVEGFNYAEMERILSGQFGLFDAAEWGVSVAATFGYRVQEIATKARRPLEETVIWA |
|---|---|---|
| 83 | 0814 | MQTWQLPEHIADVLPTNARQLESAREQLLALFRVHGYELVQPPLMEYAHSLLTHIDAGLSLKTILVTDRLSGRQLG<br>IRADITPQVARIDAHLLSANQGINRLCYAGPVLHAQPDGLLNMREPLQAGAEMYGFADIRGDIELIDLMLKSMKIA<br>DMGKVLLSLGHIGIFRALSDAAHLDAGQSATLLALMQDKDTGAVEAQVKAWKLDGMWAKAFSLLPRLYGGREVLSD<br>ARGRLPDLSAVGGALGELQAVCDAFPDCEIHIDLSELRVDNYHTGLLYAAYAADFHDAVARGGRYDGLGGYFGRAR<br>PATGFSFDLRSFIGRLPAIERQPAVLVDAEDAEAAHEAVEALREQGQCVVIDYGIGHNVSEELAGRLKKTDGVWQV<br>VKR |
| 84 | 0815 | MAMAKNVVVIGAQWGDEGKGKIVDWLAEEAGGVVRFQGGHNAGHTLVVGGKKTILRLIPSGILHESLDCFIGSGVV<br>VSPEALLGEIDELNAAGVKNVEGRLKIAPTCPLILPYHIALDQAREASRGKGKIGTTGRGIGPAYEDKVARRAIRA<br>ADLLHPEKLREKLDAVLAYYNVQLQHLHNAEPVKAEDVMAVIEKVAPRIAPMITDVSRVLNEKNKNGEKLLFEGAQ<br>GALLDIDYGTYPFVTSSNCLAGAASAGAGVGPQMLDYVLGIVKAYTTRVGSGPFPTELFDEVGVGLAERGHEFGSV<br>TGRARRCGWFDAAALKRSIQINGISGMCITKLDVMDGVETINICVGYELPDGGKTDILPCGSDAVETCKPIYETMP<br>GWRESTFGVKDYGALPENAKAYLKRIEEVCGAPVAIVSTGPDREETIVLHHPFA |
| 85 | 0828 | MTIIVTGAAGFIGSNIVKALNQRGITDIVAVDNLSKGEKFKNLAECEIAHYLDKHEFIRQVREHILPYQNIEAVFH<br>QGACSDTMNHDGLYMMDNNYQYTLDLLDWCQDERIPFLYASSAAVYGKGEIFREERELEKPLNVYGYSKFLFDQVL<br>RRRMKEGLTAQVVGFRYFNVYGQHEQHKGRMASVAFHHFHQYREHGYVNLFGSNDGYGNGEQTRDFVSVEDVAKVN<br>LYFFDHPELSGIYNLGTGRSQQFNELAAATVNACRAAEGKPEMSLKELVEEELIRYIPFPDALKGKYQSFTQADIT<br>KLREAGYKEEFFDVKSGVDRYVKWMLENLA |
| 86 | 0854 | MAQKIQSVKGMNDLLPVKQKDFKLTAAFWQAFEDTVGRWTRAYGYQQIRTPIVEQTGLFVRSIGEETDVVGKEMYT<br>FSDSNDSLSLSLRPEGTASCLRAVVEHNLLYNSPQKLWYMGPMFRRERPQKGRYRQFHQVGIEALGFEGPDIDAEI<br>IAMSADLWEKLGIREYLTLEINSLGNREERAAHRAALVEYLTRYEDKLDEDSKRRLKTNPLRVLDTKNPDLQEICN<br>AAPRLVDYLGEDSQNHYVRFKAMLDGLGIQYIENPRLVRGLDYYNQTVFEWTTDKLGAQATVCGGGRYDGLIEELG<br>GKPAPSIGFAMGIERLLLLVSEYGSLEVNAAPDVYAMHQGEGADLQVMKYAQALRAQGFNVMQHSGYQSLKAQMKK<br>ADNSGARFALIVAQDELANGTVTLKDMNGAHGQQTVAAEDLTPTLQQWKNA |
| 87 | 0875 | MAAYDSLMVFTGNANPELAQRVVRHLDISLGNASVSKFSDGEVAVELLENVRGRDVFILQPTCAPTNDNLMEILTM<br>ADALKRASAGRITTAIPYFGYARQDRRPRSVRVPISAKLVANMLYSAGIDRVLTVDLHADQIQGFFDIPVDNIYAT<br>PILLNDIKQQRIENLTVVSPDIGGVVRARAVAKSLNADLAIIDKRRPKANVAEVMNIIGDIQGRTCLIVDDMIDTA<br>NTLCKAAVALKERGAERVLAYASHAVFSGEAVSRIASSEIDQVVVTDTIPLSEAAKNCDRIRQVTIAGLLAETVRR<br>ISNEESVSYLFNEEVMTGSMLLP |
| 88 | 0876 | MTYEIQASVREAQGTGASRRLRREGQIPGILYGEGQEPVAIAVDHKTVFYALEKESFHTALIKLSLNGETKDVIVR<br>DFQMHPFRREVQHIDFQAVKADQLVRIRVPLHIVNAENSQAVKLQGGRVSLLNTSVEVVALPANIPAFLDLDCAEV<br>VAGDILHLSDIKLPEGVESVSLKRNENLAVATVTGKKR |
| 89 | 0878 | MNTPLPYSDYLIRILTASVYDVAVETPLEPARSLSVRLKNNILLKREDLQPVFSFKIRGAYNKMSKLPKDALACGV<br>IAASAGNHAQGVALSAQRLGCRAVIVMPETTPKIKVDAVKSHGGEVVLRGVSYNDAYDYAMELAEKEGLTYIAPFD<br>DPDVIAGQGTVGMEIVSQHPDPIRAVFVPIGGGGLAAGVAAFIKQVRPEIKVIGVQTNDSCCMKQSVEAGEIVHLK<br>DVGLFSDGTAVKVVGNETFRLCKELLDEIITVDTDAVCGAVKDIFDDTRSITEPAGALALAGLKAYIAREGAENQT<br>LIAVTSGANMNFHRLRHVSERSELGEGNEGIFAVTIPEERGSFLKFVNILGNRNITEFNYRYGDDEKAHIFVGLQA<br>AGPQDLAVIGSRLDEAGLPNVDLTNNEIAKIHIRYMVGGRTDKVENERLVSFEFPERPGALARFLNHMQGGWNITL<br>FHYRNHGADYGRILVGIDVPPHDAAAFDGFLESLGYSYHEETQNAAYKLFLA |
| 90 | 0884 | MEHKLPQLPYELDALSPHLSKETLEFHYGKHHQTYITNLNNQIKGTEFENLPLEEIVKKSSGGVFNNAAQTWNHTF<br>YWLGFTSKGQGKPAGELAAAIDAKWGSFEKFQEAFNACAAGTFGSGWAWLVKTPAGGLDLVSTSNAATPLTTENTP<br>LLTCDVWEHAYYIDYRNSRPNYLKGFWEIVNWDEVAKRFAALS |
| 91 | 0885 | MNDYAAMPSEDREVGALSLPPHSMEAEQSVLGGLMLENPAWDRIADVVSGEDFYRHEHRLIFRSIAKLINESRPAD<br>VITVQEDLQRNEELEAAGGFEYLITLAQNTPSAANIRRYAEIVRERSIMRQLAEVGTEIARSAYNPQGRDAGQLLD<br>EAENKVFQIAESTAKSKQGFLEMPDLLKEVVQRIDMLYSRDNPDEVTGVPTGFIDLDKKTSGLQPGDLIIVAGRPS<br>MGKTAFSINIAEHVAVEGRLPVAVFSMEMGGAQLVMRMLGSVGRLDQSVLKTGRLEDEHWGRLNEAVVKLSDAPVY<br>IDETPGLTALELRARARRLARQFNNKLGLIVIDYLQLMAGSGRSDNRASELGEISRSLKALAKELQVPIIALSQLS<br>RTVESRTDKRPMMSDLRESGAIEQDADLIMFMYRDEYYNQDSPMKGLAECIIGKHRNGPVGKIFLTWTGQFTKFDN<br>AAYIPEEAKIED |
| 92 | 0894 | MNTLLNQLKPYPFARLREAMQGISAPEGLEAVPLHIGEPKHPTPKVITDALTASLHELEKYPLTAGLPELRQACAN<br>WLKRRYDGLTVDADNEILPVLGSREALFSFVQTVLNPVSDGIKPAIVSPNPFYQIYEGATLLGGGEIHFANCPAPS<br>FNPDWRSISEEVWKRTKLVFVCSPNNPSGSVLDLDGWKEVFDLQDKYGFIIASDECYSEIYFDGNKPLGCLQAAAQ<br>LGRSRQKLLMFTSLSKRSNVPGLRSGFVAGDAELLKNFLLYRTYHGSAMSIPVQRASIAAWDDEQHVIDNRRMYQE<br>KFERVIPILQQVFDVKLPDASFYIWLKVPDGDDLALARNLWQKAAIQVLPGRFLARDTEQGNPGEGYVRIALVADV<br>ATCVKAAETIVSLYR |
| 93 | 0920 | MTQKSTIVYTHTDEAPALATQSLLPIVQAFARHADIDVKTSDISLSGRILAAFPEYLTEAQRVPDALAELGELVKQ |

| | | |
|---|---|---|
| | | PDANVIKLPNISASVPQLTAAIKELQSKGFAVPDYPADPQTDEEKAVRERYDRIKGSAVNPVLREGNSDRRAPKAV KNFAKKNPHSMGAWTKDSKTHVATMQSGDFFHNEQSVIVPEATSVSIVFTDKQGNKKELREPVALKAGEIIDATVM SKKALLAFLAEQVKDAKAKGVLFSLHMKATMMKVSDPIIFGHAVKVFFAPVFEKFGDKLAAAGVNVNNGFGNLLAN LDKLDADTRTAVEAEIAAVYAANPDLAMVDSDKGITNLHVPSDVIVDASMPAMIRNSGRMWDKNGKAQDTKAVIPD SSYAGVYQATIDFCREHGAFDPTTMGTVPNVGLMAQAAEEYGSHNKTFEIEADGQVQVIDAAGKVLMQHDVEAGDI WRMCQTKDAPVKDWVQLAVNRARLSNTPAVFWLDENRPHDKSLLAKVKAYLAELDTNGLDIRVLAPEEAAKFSLGR LKNGEDTISVTGNVLRDYLTDLFPILELGTSAKMLSIVPLMNGGGMFETGAGGSAPKHVQQFLEENHLRWDSLGEF LALAVSFEHLAQKTGNAKAQVLADTLDAATEKLLLNDKSPKRKAGELDNRGSHFYLTLYWAQELAAQDKDAELKAA FTPLAAALTADEAKIVAELSAVQGKAADIGGYYAANPEKAAQVMRPSVTFNQALNAL |
| 94 | 0944 | MTTLHFSGFPRVGAFRELKFAQEKYWRKEISEQELLAVAKDLREKNWKHQVAANADFVAVGDFTFYDHILDLQVAT GAIPARFGFDSQNLSLEQFFQLARGNKDQFAIEMTKWFDTNYHYLVPEFHADTEFKANAKHYVQQLQEAQALGLKA KPTVVGPLTFLWVGKEKGAVEFDRLSLLPKLLPVYVEILTALVEAGAEWIQIDEPALAVDLPKEWVEAYKDVYATL SKVSAKILLSTYFGSVAEHAALLKALPVDGLHIDLVRAPEQLDAFADYDKVLSAGVIDGRNIWRANLNKVLETVEP LQAKLGDRLWISSSCSLLHTPFDLSVEEKLKANKPDLYSWLAFTLQKTQELRVLKAALNEGRDSVAEELAASQAAA DSRANSSEIHRADVAKRLADLPANADQRKSPFADRIKAQQAWLNLPLLPTTNIGSFPQTTEIRQARSAFKKGELSA ADYEAAMKKEIALVVEEQEKLDLDVLVHGEAERNDMVEYFGELLSGFAFTQYGWVQSYGSRCVKPPIIFGDVSRPE AMTVAWSTYAQSLTKRPMKGMLTGPVTILQWSFVRNDIPRSTVCKQIALALNDEVLDLEKAGIKVIQIDEPAIREG LPLKRADWDAYLNWAGESFRLSSAGCEDSTQIHTHMCYSEFNDILPAIAAMDADVITIETSRSDMELLTAFGEFQY PNDIGPGVYDIHSPRVPTEAEVEHLLRKAIEVVPVERLWVNPDCGLKTRGWKETLEQLQVMMNVTRKLRAELAK |
| 95 | 0946 | MALQDRTGQKVPSVVFRTRVGDTWKDVSTDDLFKGKKVVVFSLPGAFTPTCSSSHLPRYNELFGAFKENGVDAIYC VSVNDTFVMNAWAAEEESDNIYMIPDGNGEFTEGMGMLVGKEDLGFGKRSWRYSMLVNDGVVEKMFIEPEEPGDPF KVSDADTMLQFVAPDWKAQESVAIFTKPGCQFCAKAKQALQDKGLSYEEIVLGKDATVTSVRAITGKMTAPQVFIG GKYIGGSEDLEAYLAKN |
| 96 | 0947 | MKKIQADVVVIGGGTAGMGAFRNARLHSDNVYLIENNVFGTTCARVGCMPSKLLIAAAEARHHALHTDPFGVHLDK DSIVVNGEEVMQRVKSERDRFVGFVVADVEEWPADKRIMGSAKFIDEHTVQIDEHTQITAKSFVIATGSRPVILPQ WQSLGNRLIINDDVFSWDTLPKRVAVFGPGVIGLELGQALHRLGVKVEIFGLGGIIGGISDPVVSDEANAVFGEEL KLHLDAKTEVKLDADGNVEVHWEQDGEKGVFVAEYMLAAVGRRPNVDNIGLENINIEKDARGVPVADPLTMQTSIP HIFIAGDASNQLPLLHEAADQGKIAGDNAGRYPNIGGGLRRSTIGVVFTSPQIGFVGLKYAQVAAQYQADEFVIGE VSFKNQGRSRVMLVNKGHMRLYAEKATGRFIGAEIVGPAAEHLAHLLAWAHQMKMTVPQMLDMPFYHPVIEEGLRT ALRDADAKLKA |
| 97 | 0955 | MMDEKLNFSYLFGSNAPYIEELYEAFLENPDAVDEKWKQYFTDLSKQPGTVAVDVAHTPIRESFVTLAKKKIASAV AGGADEAMLKKQVSVLRLISAYRIQGVGAAQLDPLKRIPPRDIEALDPKFHGLSDADMALQFNMGEGDFANRGKLP LSQIISNLKQTYCGHIALEYIYIPNTEERRWVRNYFESVLSTPHYNADQKRRILKEMTAAETLERYLHTKYVGQKR FGVEGGESAIAGLNYLIQNAGKDGVEEVIIGMAHRGRLNVLVNILGKKPGDLFAEFEGRAEIKLPSGDVKYHMGFS SDIATPHGPMHVSLAFNPSHLEIVNPVVEGSARAKQKRLGENGRDKVLPVLIHGDSAFIGLGVNQATFNLSKTRGY TTGGTVHIVINNQIGFTTSDIRDTRSTVHCTDIAKMVSAPVIHVNGDDPERVCFAIQAALDYRKKFHKDIVIDVVC YRKWGHNEGDDPTLTQPMMYKKVSQHPGARALYTEQLIAEGVVTQAEADGYIQAYRDALDKGEHVEQTTLSNFQRT QIDWSKYQGKDWREHIETGLPAADIERLTEKFTAVPEGFALHPTAKRVIEARKAMASGKQAIDWGMAETLAYASLV TKGHGVRISGEDSGRGTFSHRHAVLHDQKREKWDDGTYVPLRHMGEGMGEFLVIDSILNEEAVMAFEYGFACSAPD KLTIWEAQFGDFANGAQVTIDQFLSSGETKWGRLCGLTTILPHGYDGQGPEHSSARVERWLQLCSENNMQVIMPSE ASQMFHLLQRQVLGSYRKPLVIFMSKRLLRFKGAMSPLENFTEGSTFRPVIGDTAERASNDSVKRVVLCAGQVYYD LEAGRAERKLEDDVAIVRVEQLYPFPYDEVKAELAKYPNAKSVVWAQEEPKNQGAFYQIRHRIEDVISEEQKLSYA GRPSSASPAVGYSSKHIAQLKQLVEDALAL |
| 98 | 0956 | MIIDVKVPMLSESVSEGTLLEWKKKVGEAVARDEILIDIETDKVVLEVPSPQAGVLVEIVAQDGETVVADQVLARV DTAATAAAEAPAAAPAEAAPAAAPAATQNNAAMPAAAKLAAETGVDVNALQGSGRDGRVLKEDVQNAAAKPAGAAA PAVALPAGARPEERVPMSRLRARVAERLLASQQENAILTTFNEVNMKPIMDLRAKYKEKFEKEHGVKLGFMSFFVK AAVAALKKYPVVNASVDGKDIVYHGYFDIGIAIGSPRGLVVPILRDADQMSIADIEQAIVDYAKKAKDGKIAIEDL TGGTFSITNGGTFGSMMSTPIINPPQSAILGMHATKERAVVENGQVVVRPMMYLALSYDHRIIDGREAVLTLVAIK DALEDPARLLLDL |
| 99 | 0959 | MNLHEYQAKELLASYGLPVQGGILAHNGEEAAAAYDKLGGKFAVVKAQVHAGGRGKAGGVKVVKSREEAKEVAESL IGTNLVTYQTDANGQPVNSVLVCEDMYPVQTELYLGAVVDRSTRRITFMASTEGGVEIEKVAAETPEKIFKVTVDP LVGLQPCQAREVAFQLGLKDKQINEFVKLMTGAYKAFVENDFALFEVNPLAVRENGALACVDGKIGIDSNALYRLP KIAELRDKSQENERELKASEFDLNYVALEGNIGCMVNGAGLAMATMDIIKLKGGQPANFLDVGGGATKDRVVEAFK LILEDKSVQGVLINIFGGIVRCDMIAEAIVAAVKEINVNVPVVVRLEGNNAELGAKILNESGLKLTSADGLNDAAE KIVAAVNA |
| 100 | 0960 | MSVLINKDTKVLVQGFTGKNGTFHSEQALAYGTKVVGGVTPGKGGQTHLNLPVFNTMKEAVKETGADASVIYVPAP FVLDSIVEAVDSGVGLVVVITEGVPTLDMLKAKRYLETNGNGTRLVGPNCPGVITPGECKIGIMPGHIHTPGRIGI ISRSGTLTYEAVAQTTKLGLGQSTCIGIGGDPIPGMNQIDALKLFQEDPDTDAIIMIGEIGGTAEEEAAEYIQSNV SKPVVGYIAGVTAPKGKRMGHAGAIISGGKGTAEEKFAAFEKAGIAYTRSPAELGTTMLEVLKAKGLA |

| 101 | 0983 | MSSIKRALISLSDKTGAVEFAQTLHKLGVEILSTGGTAKLLADAGVPVIEVADYTGFPEMLDGRVKTLHPKIHGGI LGRRDLDEHVAKMEEHGIGNIDLVCVNLYLFAATIAKPNCTLEDAIENIDIGGPTMVRSAAKNWKHVAIVTDTADF PAIAAELEANNGALSDKTRFNLSRKAFSHTAQYDGMISNYLTSLSDDVLSGTPEIAGFPGRFNQSWIKVQDMRYGE NPHQRAAFYRDIDPAAGSLAAYKQLQGKELSYNNIADADAAWEAVKSFDVPACVIVKHANPCGVAIASNTLDAYKL AYATDTTSAFGGIIAFNREVDGATVKQITDNQFMEVLMAPKFTAEALEIAAAKKNVRVLEVPLEAGANRFELKRVG GGLLVQTPDIHRISRADLKVVSKRQPTEQEWNDLLFVWNVAKYVKSNAIVFGKGGQTYGIGAGQMSRVDSTRIAAR KAQDAGLDLNGACAASDAFFPFRDGVDVIAEQGIKAIIHPAGSMRDQEVFDAADEHGIAMVVTGIRHFRH |
| 102 | 0997 | MSASQLLSRLTQTVGEKYIITDPAKTEQYRQGYRFGEGKALAVVRPGSILEMWKILQACVEADVIVITQAANTGLT GGSTPDGNDYDRDIVIVNTMRMNIIQTINNNEQVVCLPGSTLNQLELLLKPLGREPHSVIGSSCIGASVLGGVCNN SGGALVQRGPAYTEMALFAQINEEGRLELVNHLGIDLGNTPEEILTNLQGHHYQNKDITQDAGKGHDHAYCEHVRQ VDEPTAARFNADPARHYEASGCAGKLMVFAVRLDTFPQEKQTAVFYIGTNDINELTDIRRAALGEFESLPVSGEYI HRHAFDIADVYGKDTFYVIKKFGTHQLPKLFDLKARVDRFGKKVSFLPKHFSDKAMQFVSKFLPDHLPKSMRDYRD KYEHHLILKMGGKGVDEARAFLKEYFAHHGGAFFECNAEETQAAMLHRFAVASAAIRYRAVHDDEVEDLVALDIAL RRDDRDWFEKLPPEIDNKIIHKLYYGHFMCHVFHQDYIIKKGNDCMALEHEMLHLLDQRGAQYPAEHNVGHLYEAK PALKQFYRKLDPTNSFNPGVGKTSKKKNWAE |
| 103 | 1031 | MTKHIAILRGDGIGPEIVAETVRVLDKLIAQGLDAGYEYAPLGGEAYDEYGHPYPEFTQNLCRKADAVLLGAVGSP QYDNLDRPLRPERGLLAIRKDLNLFANLRPAVLYPELANASTLKPEIVAGLDILIVRELTGDIYFGEPRGIRVLEN GEREGYNTMKYSESEIRRIAHVAFQSAQKRSKKVCSVGKANVLETTELWREIFEEIGKEYPDVELSHMYVDNAAMQ LVRAPKQFDVIATGNIFGDILSDEASMLTGSIGMLPSASLDENGKGLYEPSHGSAPDIAGQNKANPLATILSLAML LRYSLNDEARAQQVENAVQKVLQQGLRTSDIYEEGTKLVSCSEMGDAVLAAL |
| 104 | 1044 | MAAFNTQKVLSVHHWTDAYFTFTCTRDESLRFENGQFVMVGLMVDGKPLMRAYSVASANWEEHLEFFSIKVQDGPL TSRLQHLKVGDDVLISKKPTGTLVAGDLNPGKHLYLLSTGTGIAPFLSITKDPEIYEQFEKIILVHGVRYKKDLAY YDRFTKELPEHEYLGDLVKEKLIYYPIVSREEFEHHGRLTDLMVSGKLFEDIGLPKINPQDDRAMLCGSPAMLKDT CKVLDDFGLTVSPKTGVRGDYLIERAFVDQ |
| 105 | 1046 | MKYISTRGETAHKPFSEVLLMGLAPDGGLMLPEHYPQIGRETLDKWRGLAYPELAFEIMRLFVTDIPEDDLRDILN RTYTEAAFGTKEITPVRTLSDGIKIQALSNGPTLAFKDMAMQFLGNAFEYVLNKEGKKLNILGATSGDTGSAAEYA LRGKKGVNVFMLSPDGKMSAFQRAQMYSLQDENIHNIAVKGMFDDCQDIVKAVQNDAAFKEKYHIGTVNSINWGRI VAQVVYFAGYFNATSSNDETVSFCVPSGNFGNVCAGHIAKQMGLPIRRLIVATNENDVLDEFFKTGAYRPRNSAH TYVTSSPSMDISKASNFERFVFDLMDRDPAEINTLWAEVAAGKGFDLRFALDKVGGKYGFTSGKSTHADRLATIKQ VYEQDQELIDPHTADGVKVAREVREEGEMVVCLETALAAKFDATIREAVGDAAIPRPAALEGLENLPQRVQTVPNS ADAVKGIIEQTLA |
| 106 | 1053 | MKKTVFTCAMIALTGTAAAAQELQTANEFTVHTDLSSISSTRAFLKEKHKAAKHISVRADIPFDANQGIRLEAGFG RSKKNIINLETDENKLGKTKNVKLPTGVPENRIDLYTGYTYTQTLSDSLNFRVGAGLGFESSKDSIKTTKHTLHSS RQSWLAKVHADLLSQLGNGWYINPWSEVKFDLNSRYKLNTGVTNLKKDINQKTNGWGFGLGANIGKKLGESASIEA GPFYKQRTYKESGEFSVTTKSGDVSLTIPKTSIREYGLRVGIKF |
| 107 | 1070 | MTQTNRVIIFDTTLRDGEQSPGAAMTKEEKIRVARQLEKLGVDIIEAGFAAASPGDFEAVNAIAKTITKSTVCSLS RAIERDIRQAGEAVAPAPKKRIHTFIATSPIHMEYKLKMKPKQVIEAAVKAVKIAREYTDDVEFSCEDALRSEIDF LAEICGAVIEAGATTINIPDTVGYSIPYKTEEFFRELIAKTPNGGKVVWSAHCHNDLGLAVANSLAALKGGARQVE CTVNGLGERAGNASVEEIVMALKVRHDLFGLETGIDTTQIVPSSKLVSTITGYPVQPNKAIVGANAFSHESGIHQD GVLKHRETYEIMSAESVGWATNRLSLGKLSGRNAFKTKLADLGIELESEEALNAAFARFKELADKKREIFDEDLHA LVSDEMGSMNAESYKFISQKISTETGEEPRADIVFSIKGEEKRASATGSGPVDAIFKAIESVAQSGAALQIYSVNA VTQGTESQGETSVRLARGNRVVNGQGADTDVLVATAKAYLSALSKLEFSAAKPKAQGSGTI |
| 108 | 1073 | MIKISTRFDAGSVVVKDLTDPSNIRLALRPDNASDFAQWFYFRLQGAAYQNCIMHFENAAEAAYPKGWEGYQACAS YDRRNWFRVPTSYENGVLTVNHTPLSNSVYYAYFEPYSEEQHLNLLGDAQGSGLCRIDDLGSTVQGRDINLLTIGN QVESDLKIWITARQHPGETMAEWFIEGLLGRLLDPQDPTARALLDRATFYIVPNMNPDGSALGNLRTNAAGANLNR EWENPTVEKSPEVFFVREKMLETGVDLFLDIHGDEGLPFVFVAGTEGVPNYNPRIAALEAQFKNALLNASPDFQDE YGYEKDAPGEANMTLATNWVGNRFNCLAYTLEMPFKDNANLPDDDFGWNGQRSLRLGEAALSAILNVIGDLR |
| 109 | 1127 | MATLSDKTILVTGASQGLGEQVAKAYAAAGATVILVARHQKKLEKVYDAIVEAGYPEPFAICFDLISAEEKEFEHF AATIAEATQGKLDGIVHCAGYFYALSPLDFQTVAEWVNQYRINTVAPMGLTRALFPLLKQSPDASVIFVGESHGET PKAYWGGFGASKAALNYLCKVAADEWERFGNLRANVLVPGPINSPQRIKSHPGEAKSERKSYGDVLPAFVWWASAE SKGRSGEIVYL |
| 110 | 1131 | MALLQISEPGMSAAPHRHRLAAGIDLGTTNSLVATVRSGSAACLPDAEGRVTLPSVVRYLENGGIEVGKTALSAQK TDPLNTVSSAKRLIGRTLADLHQNTHYLPYRFGDNQRVIELHTRQGVKTPVEVSAEILKTLKSRAEETLGGDLVGV VITVPAYFDDAQRQATKDAARLAGLNVLRLLNEPTAAAIAYGLDNASEGTFVVYDLGGGTFDVSVLQLTKGLFEVK ATGGNSALGGDDFDHRLFCRLLEQNGLSQLNEQDSQLLLSLVRAAKEQLTTQTEARIQATLSDGMAIDTSISRAEF HNLTQHLVMKTLEPVTQALKDAGVGKNEVKGVIMVGGSTRMLHVQQAVATFFGQTPLNNLNPDEVVALGAAIQANV LAGNKTDGEWLLLDVTPLSLGLETYGGLAEKIIPRNSTIPTARAQDFTTFKDGQTAMTIHVVQGERELVADCRSLA KFTLRGIPPMAAGAARIRVTFQIDADGLLSVSAQEQSTGVQAQIEVKPSYGLDDDTITQMLKDSMSNAAEDMAARA |

| | | |
|---|---|---|
| | | RAEAVVEAESLTDAVNAALELDSDLLDAEELQQIRQGIADLQGRLKDGKAEDIRAAAAKLGSITDNFAAKRMNRNI QRALTGQSVDNI |
| 111 | 1150 | MPEYRSKTSTHGRNMAGARALWRATGVMETDFGKPIIAVANSFTQFVPGHVHLHNMGQLVAREIEKAGAIAKEFNT IAIDDGIAMGHSGMLYSLPSRDLIADSIEYMVNAHCADALVCISNCDKITPGMLIAAMRLNIPTIFVSGGPMEAGK VIGVANIQPERRLDLIDAMIESADDNVSNRQVEEVEQNACPTCGSCSGMFTANSMNCLTEALGLSLPGNGSYLATH AGRKELFLEAGRMIVEITKRYYEQNDETVLPRSIATKKAFENAMTMDIAMGGSTNTILHLLAVANEAGVDFKMADI DRLSRVVPCICKTAPNNHDYYMEDVHRAGGIFAILKELDKAGKLHTDVHTIHAPTLKDAIEQWDVTNPENTRAIER FKAAPGGVRTTQAFSQNRMWKTLDLDREKGCIRDVAHAYSQDGGLAVLFGNIAERGCVVKTAGVDESILKFTGRAR VFESQEDAVEGILGNQIVAGDIVIIRYEGPKGGPGMQEMLYPTSYLKSKGLGKACALLTDGRFSGGTSGLSIGHAS PEAAEGGAIGLVHEGDTVEIDIPNRSIHLAISDEELAARRAEMEARGSKAWKPKNRDRYVSAALRAYGAMATSADK GAVRDVAQIER |
| 112 | 1201 | MRIVEKAYTFDDVLLVPAHSTVLPRDVKLQTKLTREITLNLPLLSAAMDTVTEARLAISMAQEGGIGIIHKNMPPE MQARAVSKVKRHESGVVKDPVTVAPTTLIREVLEMRAQRKRKMSGLPVVENGKVVGIVTNRDLRFENRVDLPVSAI MTPRERLVTVPEGTSIDEARELMHTHKVERVLVLNEKDELKGLITVKDILKTTEFPNANKDSEGRLRVGAAVGTGG DTEERVKALVEAGVDVIVVDTAHGHSQGVIDRVRWVKETYPHIQVIGGNIATAKAALDLVAAGADAVKVGIGPGSI CTTRIVAGVGVPQLTAIHNVAEALKGTGVPLIADGGIRFSGDIAKALAAGAYSVMLGGMFAGTEEAPGEIELYQGR SYKSYRGMGSLGAMSQGSADRYFQDKTDSTDKYVPEGIEGRVPYKGPIVNIIHQLTGGLRSSMGYLGCANIAEMHE KAEFVEITSAGMSESHVHDVQITKEAPNYHR |
| 113 | 1206 | MKGDRLVIRELNKNLGLLLVTINQYFLHARILKNWGFEELGEHFFKQSIVEMKAADDLIERILFLEGLPNLQELGK LLIGESTEEIIACDLTKEQEKHEALLAAIATAEAQQDYVSRDLLEKQKDTNEEHIDWLETQQELIGKIGLPNYLQT AAQED |
| 114 | 1228 | MKPVNIGLLGLGTVGGGTAAVLRDNAEEISRRLGREIRISAVCDLSEEKARQTCPSAAFVKDPFELVAREDVDVVV ELFGGTGIAKDAVLKAIENGKHIVTANKKLLAEYGNEIFPLAEKQNVIVQFEAAVAGGIPIIKALREGLAANRIKS IAGIINGTSNFILSEMREKGSAFADVLKEAQALGYAEADPTFDIEGNDAGHKITIMSALAFGTPMNFSACYLEGIS KLDSRDIKYAEELGYRIKLLGITRKTGKGIELRVHPTLIPESRLLANVNGVMNAVRVNADMVGETLYYGAGAGALP TASAVVADIIDIARLVEADTAHRVPHLAFQPAQVQAQTILPMDEITSSYYLRVQAKDEPGTLGQIAALLAQENVSI EALIQKGVIDQTTAEIVILTHSTVEKHIKSAIAAIEALDCVEKPITMIRMESLHD |
| 115 | 1231 | MTQKEKHFEEYAALATLPLRDVVVYPHMVLPLFVGRPKSIAALENAITREEPVFLLAQTDAAVEEPIAADLYQTGT VAQVLQVLKLPDGTVKVLVEGLYRGRVLTIEDTGGLFVSHIETVVEEDTGGNTDLEAVRRTLLAQFEQYAKLNKKI PAEIIGSINGIAENSRLTDTVAAHLQLKLAQRQQILEIPEIGKRMEFLLAQLESELDIMQAEKRIRGRVKRQMEKS QREYYLNEQIKAIHKELGEEDENGELDALEADIKKAGMTKEAEEKCLSELKKLKMMPPMSAESTVVRNYIDTLLEL PWKKKSRVSKDIAKAGLVLDADHYGLEKVKERILEYLAVQKRMDKLKGPILCLVGPPGVGKTSLGESIAKATGRKY VRMALGGVRDESEIRGHRRTYIGSMPGKILQNMAKAGVKNPLFLLDEIDKLGNDFRGDPASALLEVLDPEQNNKFA DHYAEVDYDLSDVMFIATSNSLNIPTPLLDRMEIIRLSGYTEDEKINIAMQYLVPKQMKRNGVKEGELAIEESAVR DIIRYYTREAGVRSLDREIAKICRKVVMQITLDEDKKRLSETKKTSKAKPKAVKVNEKNLHDYLGVRRFDYGVAES ENRIGQVTGLAWTEVGGELLTVEAAALPGKGVIQCTGQLGDVMKESVSAAWSVVRSRAESVGLAPDFYEKKDIHIH VPEGATPKDGPSAGIAMTLAAVSAFTKIPVRADVAMTGEITLRGEVLPIGGLKEKLLAALRGGIKHVLIPKDNVKD LEEIPENVKTGLTIHPVKWIDEVLALGLESQPEPWAEPSGAEAAAESASKPKPRSRATKH |
| 116 | 1240 | MISTNGITMQFGAKPLFENVSVKFGEGNRYGLIGANGSGKSTFMKILGGDLEQTAGEVAIENGVRLGKLRQDQFAY EDMRVLDVVMMGHTEMWAAMTERDAIYANPEATEDDYMKAAELEAKFAEYDGYTAEARAAELLSGVGISEDLHNAK MAEVAPGFKLRVLLAQALFSKPDVLLLDEPTNNLDINTIRWLEGVLNQYDSTMIIISHDRHFLNEVCTHMADLDYN TITIYPGNYDDYMLASAQSRERALKDNAKAKEKLQELQEFVARFSANKSKARQATSRLKQADKIKSEMVEVKPSTR QNPYIRFEADEKAKLHRQAVEVEKLAKRFETQLFKNLNFILEAGQRLAIIGPNGAGKSTLLKLLAGAYNPEYSDGL LPDEGTIKWAEKASVGYYPQDHENDFDVDMDLSEWMRQWGQEGDDEQVIRGTLGRLLFGSNDVVKKVKVLSGGEKG RMLYGKLLLLKPNVLVMDEPTNHMDMESIESLNMALEKYNGTLIFVSHDRQFVSSLATQIIELDGKGGYEHYLGDY ESYLEKKGVA |
| 117 | 1252 | MSTSLSYRDAGVDIDAGDQLVENIKPFAKRTMRPEVLGDLGGFGALVEIGKKYQNPVLVSGTDGVGTKLKLAFDWD KHDTVGIDLVAMSVNDILVQGAEPLFFLDYFACGKLDVPRATDVIKGIAQGCEESGCALIGGETAEMPGMYPVGEY DLAGFAVGVVEKENVITGRSIGVGDVVLGLASNGAHSNGYSLIRKIIERDNPDLDAEFDNGKTLREAVIAPTRLYV KPILAALEKFTIKGMAHITGGGITENVPRVLPENTVAQIDAKSWELPKLFQWLQKAGNVETQEMYRTFNCGIGMVV IVAAEDADAVQGLLGEQGETVYRLGLIRERQGDEHQTQVA |
| 118 | 1301 | MSMENFAQLLEESFTLQEMNPGEVITAEVVAIDQNFVTVNAGLKSESLIDVAEFKNAQGEIEVKVGDFVTVTIESV ENGFGETKLSREKAKRAADWIALEEAMENGDILSGIINGKVKGGLTVMISSIRAFLPGSLVDVRPVKDTSHFEGKE IEFKVIKLDKKRNNVVVSRRAVLEATLGEERKALLENLQEGSVIKGIVKNITDYGAFVDLGGIDGLLHITDLAWRR VKHPSEVLEVGQEVEAKVLKFDQEKQRVSLGMKQLGEDPWSGLTRRYPQGTRLFGKVSNLTDYGAFVEIEQGIEGL VHVSEMDWTNKNVHPSKVVQLGDEVEVMILEIDEGRRRISLGMKQCQANPWEEFAANHNKGDKISGAVKSITDFGV FVGLPGGIDGLVHLSDLSWTESGEEAVRKYKKGEEVEAVVLAIDVEKERISLGIKQLEGDPFGNFISVNDKGSLVK GSVKSVDAKGAVIALSDEVEGYLPASEFAADRVEDLTTKLKEGDEVEAVIVTVDRKNRSIKLSVKAKDAKESREAL NSVNAAANANAGTTSLGDLLKAKLSGEQE |

| 119 | 1302 | MTKSELMVRLAEVFAAKNGTHLLAKDVEYSVKVLVDTMTRSLARGQRIEIRGFGSFDLNHRPARIGRNPKTGERVE VPEKHVPHFKPGKELRERVDLALKENAN |
|---|---|---|
| 120 | 1313 | MMSVTVETLENLERKVVLSLPWSEINAETDKKLKQTQRRAKIDGFRPGKAPLKMIAQMYGASAQNDVINELVQRRF YDVAVAQELKVAGFPRFEGVEEQDDKESFKVAAIFEVFPEVVIGDLSAQEVEKVTASVGDAEVDQTVEILRKQRTR FNHVEREARNGDRVIIDFEGKIDGEPFAGGASKNYAFVLGASQMLPEFEAGVVGMKAGESKDVTVNFPEDYHGKDV AGKTAVFTITLNNVSEATLPEVDADFAKALGIADGDVAKMREEVQKNVSREVERRVNEQTKESVMNALLKAVELKA PVALVNEEAARLANEMKQNFVNQGMADAANLDLPLDMFKEQAERRVSLGLILAKLVDENKLEPTEEQIKAVVANFA ESYEDPQEVIDWYYADPSRLQAPTSLAVESNVVDFVLGKAKVNEKALSFDEVMGAQA |
| 121 | 1320 | MQIILLEKIGGLGNLGDIVTVKNGYARNFLIPAGKAKRATEANMKEFEARRAELEAKQAEILADARVRQEKLDGQT VTVAQKAGVDGRLFGSVTNADIAAAIVAAGIEAVKANVRLPNGPLKAVGEYEVEVALHTDAVAKITVAVVAATE |
| 122 | 1323 | MRHYEIVFIVHPDQSEQVPAMVERYKTMIAEANGKIHRLEDWGRRQLAYPINKIHKAHYVLMNIETTPEVVEELET AFRFNDAILRHLTIKTKHAVTEASPMLGGEKAKNLLSGASEEAVAQ |
| 123 | 1324 | MSQHRKLIILGSGPAGYTAAVYAARANLNPVIITGIAQGGQLMTTTEVDNWPADADGVQGPELMARFLAHAERFGT EIIFDQINAVDLQKRPFTLKGDMGEYTCDALIVATGASAKYLGLPSEEAFAGKGVSACATCDGFFYKNQDVAVVGG GNTAVEEALYLANIAKTVTLIHRRSEFRAEKIMIDKLMKRVEEGKIILKLESNLQEVLGDDRGVNGALLKNNDGSE QQIAVSGIFIAIGHKPNTDIFKGQLEMDEAGYLKTKGGTADNVGATNIEGVWAAGDVKDHTYRQAITSAASGCQAA LDAERWLGSQNI |
| 124 | 1328 | MTDTAENQTQNNWQAGHPRSIRSFVLRQSHMTAAQQRAIDTLWDSFGIDYQATPADLDARFGSSRPKILEIGFGMG TATAEIARRLPETDFLAIDVHGPGVGNLLKLIDENHLENIRVMRHDAVEVVENMLQDGSLDGIHIFFPDPWHKKRH HKRRLIQAPFIAKLLPKLKTGGYIHLATDWEEYAQQMLEVLSSFDSLQNTAADYAPTPDYRPETKFEARGKRLGHG VWDLVFKRIG |
| 125 | 1339 | MKASQFFISTLKEAPAEAALASHKLMIRAGLIKANASGLYTWMPMGLRVLRKVENVVREEMARAGSVELLMPVVQP AELWQESGRWEFYGKELLRLKDRHDRDFCMGPTCEEVIADIVRKEINSYKQLPKNFYHIQTKFRDEVRPRFGVMRA REFVMKDAYSFHADYASLQTTYQDMYDAYCRIFTRLGLAFRPVAADTGSIGGTGSHEFQVLAESGEDVIAYSDTSD YAANIELAPTLPLKGERAAAQAELVKVHTPNVKTIDSLVDFLSIPIEKTLKSIVVEGENEGELILLLLRGDHEFND IKAEKLAGVKSPLTMASPAAIVEQFGANGGSLGPVGFAGKVYADFATEKGADWVIGANEDDYHYTGFNFGRDAAEP EFVDLRNVVEGDESPDGQGRLKLARGIEVGHVFQLRDKYTQAMNVSFLDNNGKSQIMEMGCYGIGITRVVAAAIEQ NNDEKGIIWTKAMAPFEVVIVPMNYKKSDTVREAADKIYAELLAAGADVLLDDRDERAGVLLNDSELLGIPHRIVI GDRALKEGNVEYAERRDNEAQAVAIGEIVARVTASLNA |
| 126 | 1341 | MSTQLHDVDPIETQEWLDALSSVLEYEGGERAQYLLENLVKYCRDKGVRMPHGTTTPYLNTVSVENEKGIPGDQNI EHRIRAFVRWNAAAIVLRAGKKDLELGGHIASFQSAATMYEVGFNHFWKAKGEGEEGDLVFFQGHVAPGIYARAFV EGRLTEDQLNNFRQEVDGHGLPSYPHPHLLPDFWQFPTVSMGLGPIMAIYQARFLKYLESRGLAKTKGRKVWCFCG DGEMDEPESQGAIALAAREGLDNLIFVINCNLQRLDGPVRGNGKIIQELEGNFAGAGWNVVKVIWGRRWDRLLAKD KDGILRQRMEECLDGDYQTYKSKDGAYVREHFFNTPELKALVADMTDEQLWALNRGGHDPQKVYNAYDRAANHADG KPTVILAKTIKGYGMGASGEGQNVAHQAKKMDKASLKQFRDRFDIPVTDEQIESGDLPYLTFAPDTEEYKYLHARR DALGGYLPQRKPTQEVLEVPELSAFDAQLKSSGEREFSTTMAFVRILSTLLKDKKIGKRVVPIVPDESRTFGMEGM FRQYGIWNPKGQQYTPQDKDQLMFYKESVDGQILQEGINEPGAMADWIAAATSYANSNFAMIPFYIYYSMFGFQRI GDLAWAAGDMHARGFLLGGTAGRTTLNGEGLQHEDGHSHIQADLIPNCVSYDPTFQYEVAVIVQDGLRRMYANNED VFYYITLMNENYTHPDMPEGAEQDILKGMYLLKAGGKGDKKVQLMGSGTILQEVIAGAELLKADFGVEADIWSCPS FNLLHRDAVEVERFNRLHPLEAEKVPFVTSQLQGHDGPVIAATDYIRSYADRIRAYIPNDYHVLGTDGFGRSDSRA NLRRFFEVDRYNVAVAALAALAEQGKVSKETVQQAIEKYGIKADSAPSWKR |
| 127 | 1342 | MSIVEIKVPDIGGHENVDIIAVEVKAGDTIAVDDTLITLETDKATMDVPADAAGVVKEVKVKVGDKISEGGVILTV ETGAAAAEAAPAAAEAQPAPAAAPAAAGGATVQVAVPDIGGHTDVDVIAVEIKVGDTVAEDDTLITLETDKATMDV PCTAAGVVKAVFLKVGDKVSEGSAIIEVETVGSAAAAPAQAAQAAAPAAAPPPTAAAAPAAAPAPSAPAAAKIDEA AFAKAHAGPSARKLARELGVDLGQVKGTGLKGRIMGDDIKAFVKSVMQGGAAKPAAASASLGGGLDLLPWPKVDFS KFGNVEVKELSRIKKISGQNLSRNWVVIPHVTVHEEADMTELEEFRKQLNKEWEREGVKLSPLAFIIKASVSALKA FPEFNASLDGDNLVLKNYFNIGFAADTPNGLVVPVIKDVDQKGLKQISQELTELSKKAREGKLKPQEMQGACFTIS SLGGIGGTGFTPIVNAPEVAILGVCKSQIKPVWNGKEFAPRLMCPLSLSFDHRVIDGAAGMRFTVFLAKLLKDFRR ITL |
| 128 | 1343 | MGNFLYRGISCQQDEQNNGQLKPKGNKAEVAIRYDGKFKYDGKATHGPSVKNAVYAHQIETGLYDGCYISTTTDKE IAKKFATSSGIENGYIYVLNRDLFGQYSIFEYEVEHPENPNEKEVTIRAEDCGCIPEEVIAKELIEIN |
| 129 | 1344 | MALVELKVPDIGGHENVDIIAVEVNVGDTIAVDDTLITLETDKATMDVPAEVAGVVKEVKVKVGDKISEGGLIVVV EAEGTAAAPKAEAAAAPAQEAPKAAAPAPQAAQFGGSADAEYDVVVLGGGPGGYSAAFAAAADEGLKVAIVERYKTL GGVCLNVGCIPSKALLHNAAVIDEVRHLAANGIKYPEPELDIDMLRAYKDGVVSRLTGGLAGMAKSRKVDVIQGDG QFLDPHHLEVSLTAGDAYEQAAPTGEKKIVAFKNCIIAAGSRVTKLPFIPEDPRIIDSSGALALKEVPGKLLIIGG GIIGLEMGTVYSTLGSRLDVVEMMDGLMQGADRDLVKVWQKQNEYRFDNIMVNTKTVAVEPKEDGVYVTFEGANAP KEPQRYDAVLVAAGRAPNGKLISAEKAGVAVTDRGFIEVDKQMRTNVPHIYAIGDIVGQPMLAHKAVHEGHVAAEN CAGHKAYFDARVIPGVAYTSPEVAWVGETELSAKASGRKITKANFPWAASGRAIANGCDNGFTKLIFDAETGRIIG |

| | | |
|---|---|---|
| | | GGIVGPNGGDMIGEVCLAIEMGCDAADIGKTIHPHPTLGESIGMAAEVALGTCTDLPPQKKK |
| 130 | 1347 | MNPFLNTAFKAARRAGQMMIRAAGNLDAVKTDSKAFNDFVSDVDRNSEIILVEALKEAYPHHKITCEESGSHGKAA<br>AEYEWIIDPLDGTTNFLHGHPQYAISMALLHKGVLQEALVYAPERNDVYMASRGKGALLNDRRIRVSNRIELNRCL<br>IGTGFPVVDQSMMDKYLAILKDFLAKTAGGRREGAASLDLCAVATGRFDGFFEFNLKPWDIAAGALIVQEAGGIVT<br>DMSGEDGWLESGDIVAANPKVLAQMLKIISAHV |
| 131 | 1356 | MTQYTLKQASVLLQSKQISAVELASAYLAAIAEKNPALNGYITIDQDKTLAEARAADERIAQGNASALTGVPVAYK<br>DIFCQTGWRSACASKMLDNFISPYTATVVQNLLDEGMVTLGRTNMDEFAMGSTNENSFYGAAKNPWNLEHVPGGSS<br>GGSAAVVAARLAPAALGSDTGGSIRQPASHCGITGIKPTYGTVSRFGMVAYASSFDQTGPMAQTAEDCAILLNAMA<br>GFDPKDSTSLEREKEDYTRDLNQPLKGLKIGLPKEYFGEGNSADVLTALQNTIDLLKAQGAELIEVSLPQTKLSIP<br>AYYVLASAEASTNLSRYDGVRYGHRAAQFADLEEMYGKTRAEGFGSEVKRRIMIGTYVLSHGYYDAYYLKAQKLRR<br>LVADDFQTAFARCDLILAPTAPTAAPKIGADASPVETYLSDIYTIAVNLAGLPALTLPAGFSGGGLPVGVQLVGNY<br>FAEAKILGAAHQIQLNSDWHGKRPE |
| 132 | 1358 | MTWETVIGLEIHVQLNTKSKIFSGASTAFGAEPNAHASVVECALPGVLPVMNREVVEKAIKLGLALDAKINQKNVF<br>DRKNYFYPDLPKGYQISQLDLPIVEHGKLEIVVGDDVKTINVTRAHMEEDAGKSVHEGLNGATGIDLNRAGTPLLE<br>VVSEPEMRSAAEAVAYAKALHSLVTWLDICDGNMAEGSFRVDANVSVRPKGQEEFGTRREIKNLNSFRFLEQAINY<br>EAEAQIEILEDGGKVQQATMLFDPEKGETRVMRLKEDAHDYRYFPDPDLLPVIISDAQMQKAKAEMPELPKEMAAR<br>FVADYGVSEYDARLLTASRAQAAYFEEAAKESGQGKLTANWMNGELAAALNKEGMELADSPITAPRLAALVGKIAD<br>GTLSSKLAKKAFEAMWAEPEATIAEIIEKHGLQQMTDTGEIEAMVDEVLANNAKAVEQFKSGNEKALNAIVGQVMK<br>ASKGKANPAQVQELIKAKLA |
| 133 | 1372 | MSNENRTCSFCGKSKSHVKHLIEGENAFICDECVSNCIEILHEDGNDGTPSESAGGEPEESGKLPTPAEIVANLND<br>HVIGQEQAKKALAVSVYNHYKRLRHPKAGANVELSKSNILLIGPTGSGKTLLAQSLARKLDVPFVMADATTLTEAG<br>YVGEDVEQIITKLLGKCDFDVEKAQRGIVYIDEIDKISRKSDNPSITRDVSGEGVQQALLKLIEGTVASVPPQGGR<br>KHPNQEFINVDTTNILFICGGAFAGLEKVIRQRTEKGGIGFGASVHSKDENADITKLFGIVEPEDLIKFGLIPELI<br>GRLPVIATLEELDEDALINILTEPKNALVKQYQALFGMENVELEFEEGALRSIARQAMERKTGARGLRSIVERCLL<br>DTMYRLPDLKGLKKVVVGKAVIEEGREPELVFES · |
| 134 | 1379 | MTVKTPVYLDYAATPPVDKRVAEKMIPYLTETFGNPASNSHSFGWEAEEAVEKARADIAALINADSKEIVFTSGAT<br>ESNNLAIKGAAHFYKSKGCNHLITVKTEHKAVLDTMRELERQGYEVTYLDVQENGLVDLDVLKAAIREDTILVSVMW<br>VNNEIGVVQDIPAIGEICRERKIIFHVDAAQACGKVPVDVEAAKVDLLSMSGHKVYGPKGIGALYVRRKPRVRLEA<br>QMHGGGHERGFRSGTLPTHQIVGMGEAFRIAKEELAQDTAHYLKLRDIFLKGIEGIEEVYINGDLEHRVPNNLNVS<br>FNFVEGESLIMAVKELAVSSGSACTSASLEPSYVLRALGRNDELAHSSLRITFGRMTTEEEVQFAAELIKSKIGKL<br>RELSPLWEMFKDGIDLNSIEWAAH |
| 135 | 1390 | MSSTPNKQAGYPRLVADIGGTNARFALETAPRVIEKAAVLPCKDYDTVTDAVRAYLNQSGATAVRHAAFAIANPIL<br>GDWVQMTNHHWAFSIETTRQTLGLDTLILLNDFTAQALAVTQTSSKDLMQVGGQKPVEFAPKAVIGPGTGLGVSGL<br>VHSHAGWVALAGEGGHTSFPPFDDMEVLIWQYAKNKYGHVSAERFLSGAGLSLVYEALAAKQKAKPAKLMPSEITE<br>KALSGASPLCRQTLDIFCAMLGTVASNLALTLGARGGVYLCGGIIPRVLEYFKTSPFRSRFENKGRFEAYLAAIPV<br>YVVLSEFPGISGAAAALDNHLRNV |
| 136 | 1392 | MSTQTNFDLVLFGATGDLAMRKLLPCLYQAHVAGLLHPEGRILGVSRSELDTEGFLAKVETSSKIHVKENFSDEAW<br>ASFVERFAYLKVDVTQPDDFAALGDLVKARKETDNVVIYLSTAPKFFAQACENLAAIGLNADNVRVVLEKPLGTDL<br>ASSQQINTDVARYFKEGQIYRIDHYLGKESLQNLLALRFANVMFEPLWNNKYIESVQLTIAEQLGVEERGEFYDIT<br>GALRDMVQNHLMQMLCMTAMEAPASLDADAVRDEKVKVIKSLKPLTVESVNENVVRGQYTAARGMNGYLEEINVPQ<br>DSFTETYVAIKAEIENERWKGVPFYLRTGKRMAGKVAEIVLNFKDLNSHIFEGSRTAPNRLVIELQPYESVRLYTQ<br>MKTPGAGNKVETVPLATDLGKALEGRRAEAYERLLLDVINGKLALFNRRDELEAAWEYVMPILENWTNNTTPPHGY<br>GAHSWGPEAARELLARDGHKWHEEQ |
| 137 | 1425 | MSEQNHPQTEPQLDENQIIALRREKLHNIRQQRNAYPNDFKRDSFAADLHAQYGEIGKEELDPQGIPVKVAGRMML<br>KRQMGKASFATIQDVSGQIQLYLNNKGVSQEVLDDFNHWDLGDIVGAEGTLFKTNHGELTVRVSGIRLLSKSLRPL<br>PDKHHKGLSDQETKYRQRYVDLIANEESRNTFIKRSQIIQSVRNFMVGEHYLEVETPMMHPIPGGATAKPFVTHHNA<br>LDIPLYLRIAPELYLKRLVVGGLERVFEINRSFRNEGMSVRHNPEFTMIEFYEAFSDYERMMQMAEDIIRNASRTV<br>NGTANITYNGKEVDLESPFERLTILEAIKKYNPHYTDEQLNDAEWLKKEIVKHGESLPPSPGIGSLQLALFEGCAE<br>GKLWNPTFIVDYPVEVSPLARASDTKQGLTERFELFVVGRELANGYSELNDPEDQAERFKAQVVQKDAGDDEAMHY<br>DADYIRAMEFGLPPTGGCGIGIDRLVMLLTDSQTIRDVILFPQMRPE |
| 138 | 1429 | MRKKLTALVLSALPLAAVADVSLYGEIKAGVEGRNYQLQLTEAQAANGGASGQVKVTKVTKAKSRIRTKISDFGSF<br>IGFKGSEDLGDGLKAVWQLEQDVSVAGGGATQWGNRESFIGLAGEFGTLRAGRVANQFDDASQAIDPWDSNNDVAS<br>QLGIFKRHDDMPVSVRYDSPEFSGFSGSVQFVPIQNSKSAYTPAYYTKNTNNNLTLVPAVVGKPGSDVYYAGLNYK<br>NGGFAGNYAFKYARHANVGRNAFELFLIGSGSDQAKGTDPLKNHQVHRLTGGYEEGGLNLALAAQLDLSENGDKTK<br>NSTTEIAATASYRFGNAVPRISYAHGFDFIERGKKGENTSYDQIIAGVDYDFSKRTSAIVSGAWLKRNTGIGNYTQ<br>INAASVGLRHKF |
| 139 | 1445 | MSDDKSKALAAALAQIEKSFGKGAIMKMDGSQQEENLEVISTGSLGLDLALGVGGLPRGRIVEIFGPESSGKTTLC<br>LEAVAQCQKNGGVCAFVDAEHAFDPVYARKLGVKVEELYLSQPDTGEQALEICDTLVRSGGIDMVVVDSVAALVPK |

| | | |
|---|---|---|
| | | AEIEGDMGDSHVGLQARLMSQALRKLTGHIKKTNTLVVFINQIRMKIGVMFGSPETTTGGNALKFYSSVRLDIRRT GSIKKGEEVLGNETRVKVIKNKVAPPFRQAEFDILYGEGISWEGELIDIGVKNDIINKSGAWYSYNGAKIGQGKDN VRVWLKENPEVANEIDAKIRALNGVEMHITEGTQDETDGERPEE |
| 140 | 1452 | MQNIFDPLVIRGKSLTPIVQGGMGVGVSASGLSSAVARENGIGTIASVDLRHLHEDLLAESQINPSEEKYTSLNCT ALDREIQKAKSASEGKGLIAVNVMKAVKDHAAYVRQACESGADAVVMGAGLPLDLPEMTEGYHKDVALLPILSESR GINIVLKRWMKKGILPDAIVVEHPAHAAGHLGASTVEGVNDAKFDFKRVIEETFEVFKSLGLESEKIPLILAGGMA NFEKVKTALKNWGASAVQIGTAFAVTEEGDAHLNFKKTLAGAETEKVVEFMSVAGLPARGVRTKFLDSYIKRESKL QTNAKADPRRCTQGLNCLTSCGLRDGLSKAGQFCIDIQLAAAFRGEVDKGLFFRGKDRCPSAMPSAPSARRYNIC |
| 141 | 1457 | MSQLANAIRFLSADAVQKANSGHPGAPMGMAEMAETLWTKFLNHNPANPKFYNRDRFVLSNGHASMLLYSLLHLTG YNLSIEDLKNFRQLHSKTPGHPEYGYTDGVETTTGPLGQGIANAVGMALAEKILAAEFNKDGLNIVDHYTYVFMGD GCLMEGVSHEACSLAGTLGLGKLIVLYDDNNISIDGKVDGWFTENIPQRFESYGWHVVPNVNGHDTAAIQAAIEAA RAETGKPSIICCKTLIGKGSANKEGSHKTHGAPLGADEIEATRKHLGWTYPAFEIPQEIYDAWNAKEQGAKLEADW NELFAQYQAKYPAEAAEFVRRMDKKLPDNFDEYVQAALKEVCAKAETIATRKASQNSIEILAKELPELVGGSADLT PSNLTDWSNSVSVTRDKGGNYIHYGVREFGMGAIMNGLVLHGGVKPFGATFLMFSEYERNALRMAALMKINPVFVF THDSIGLGEDGPTHQPIEQTATLRLIPNMDVWRPCDTAESLVAWAEAVKAADHPSCLIFSRQNLKFQARSEQQLND IKRGGYVISEAQGNAQAVIIATGSEVELALEAQKALAAQNIAVRVVSMPSTNVFDRQDAAYQAAVLPEGLPRIAVE AGHADGWYKYVGLNGAVVGINRFGESAPADLLFKAFGFTVDNVVDTVKSVL |
| 142 | 1471 | MSKKRVLTGVTTTGIPHLGNYVGAIRPAVRAAQNLDTESFLFLADYHGIIKCHEPEMIHQSTQAVAATWLACGLDP ERTTFYRQSDTPEVMELNWILTCITAKGLMNRAHAYKAAVQANAENGQEDPDFGVEMGLFSYPILMTADILMFNAN EVPVGRDQIQHVEMARDIAGRFNHRFRELFTLPEVKIDENVELLVGLDGRKMSKSYGNTIPLWENDKKTQKSVNKI ITNMKEPGEPKQPDESPLFEIYKAFSTPSETVEFTKMLADGLAWGEAKKLLAAKINAELAEPRERYNELTADPSQI EEILQAGAAKARKEARELLDKVRDAVGIRPLK |
| 143 | 1472 | MRYDKLTAKFQQALAEAQSLALAADGSYLEAGFVLKALLDDQNSGAAALLAHAGVNVPQVKQRLQQHLNSLPKVSG QGGDILPSRELQAVLNLMDKAATKRSDAYIASELFLLALVQQNDATGKILKEAGATEQNINAAIDAVRGGQNVNDA NAEDQRDALKKYTLDLTQRARDGKLDPVIGRDDEIRRAIQVLQRRTKNNPVLIGEPGVGKTAIVEGLAQRIVNGEV PESLRNKRLLVLDLAALIAGAKYRGEFEERLKGVLNDLAKDDGNTLIFIDEIHTLVGAGKTDGAMDAGNMLKPALA RGELHCIGATTLDEYRQYIEKDAALERRFQKVLVGEPSVEDTIAILRGLQERYEIHHGIDITDPAIVAAAELSDRY ITDRFLPDKAIDLIDEAASRVKMEKETKPEAMDKIDRRLIQLRMEKAHVEKEKDDASKKRLELIDEEINGLQKEYA DLDEIWKAEKAISDGAANIKKQIDEVKIKIEQAKRQGDLALASKLMYEDLEHLEKQRAAAERADTDSTKPANKLLR NNVGAEEIAEVVSRMTGIPVSKMMEGERDKLLKMEEVLHRRVVGQDEAVRAVSDAIRRSRSGLADPNKPYGSFLFL GPTGVGKTELCKALAGFLFDSEDHLIRIDMSEYMEKHAVARLIGAPPGYVGYEEGGYLTEQVRRKPYSVILLDEVE KAHPDVFNILLQVLDDGRLTDGQGRTVDFKNTVIVMTSNIGSQHIQQMGIQDYEAVKEVVMEDVKEHFRPEMINRI DEVVVFHGLDQDNIRNIAKIQLKGLEKRLEKQNLRLAVSDAALDIIAKAGFDPIYGARPLKRAIQSEIENPLAKAL LAGNYAPESEIRVEADGDRLKFA |
| 144 | 1506 | MNLHQTVEHEAAAAFAAAGIADSPIVLQPTKNAEHGDFQINGVMGAAKKAKQNPRELAQKVAEALADNAVIESAEV AGPGFINLRLRPEFLAQNIQTALNDARFGVAKTDKPQTVVIDYSSPNLAKEMHVGHLRSSIIGDSISRVLAFMGNT VIRQNHVGDWGTQFGMLVAYLVEQQKDNAAFELADLEQFYRAAKVRFDEDPAFADTAREYVVKLQGGDETVLALWK QFVDISLSHAQAVYDTLGLKLRPEDVAGESKYNDDLQPVVDDLVQKGLAVEDDGAKVVFLDEFKNKEGEPAAFIVQ KQGGGFLYASTDLACLRYRIGRLKADRLLYVVDHRQALHFEQLFTTSRKAGYLPENVGAAFIGFGTMMGKDGKPFK TRSGDTVKLVDLLTEAVERATALVKEKNPELGADEAAKIGKTVGIGAVKYADLSKNRTSDYVFDWDAMLSFEGNTA PYLQYAYTRVQSVFRKAGEWDANAPTVLTEPLEKQLAAELLKFEDVLQSVADTAYPHYLAAYLYQIATLFSRFYEA CPILKAEGASRNSRLQLAKLTGDTLKQGLDLLGIDVLDVM |
| 145 | 1518 | MSQKLILVLNCGSSSLKGAVLDNGSGEVLLSCLAEKLNLPDAYITFKVNGEKHKVDLSAHPDHTGAVEALMEELKA HGLDSRIGAIGHRVVSGGELYSESILVDDEVIAGIEKCIPLAPLHNPAHLLGLRAAQSIFKGLPNVVVFDTSFHQT MPEVAYKYAVPQELYEKYGLRRYGAHGTSYRFVADETARFLGKDKKDLRMVIAHLGNGASITAVANGESRDTSMGL TPLEGLVMGTRSGDIDPSVFGFLAENANMTIAQITEMLNKKSGLLGISGLSNDCRTIEEEAAKGHKGAKLALDMFI YRLAKYIGSMAVAAGGLDALVFTGGIGENSDIIRERVIGYLGFLGLNIDQEANLKARFGNAGVITTADSKAVAVVI PTNEELMIAHDTARLSGL |
| 146 | 1533 | MKAYLALISAAVIGLAACSQEPAAPAAEATPAAEAPASEAPAAEAAPADAAEAPAAGNCAATVESNDNMQFNTKDI QVSKACKEFTITLKHTGTQPKASMGHNLVIAKAEDMDGVFKDGVGAADTDYVKPDDARVVAHTKLIGGGEEASLTL DPAKLADGEYKFACTFPGHGALMNGKVTLVD |
| 147 | 1536 | MLTNIAKKIFGSRNDRLLKQYRKSVARINALEEQMQALSDADLQAKTAEFKQRLADGQTLDGILPEAFAVCREASR RTLGMRHFDVQLIGGMVLHDGKIAEMRTGEGKTLVATLAVYLNALAGKGVHVVTVNDYLASRDAGIMEPLYNFLGL TVGVIISDMQPFDRQNAYAADITYGTNNEFGFDYLRDNMVTDQYDKVQRELNFAVVDEVDSILIDEARTPLIISGQ ADDNIQLYQIMNTVPPHLVRQETEEGEGDYWVDEKAHQVILSEAGHEHAEQILTQMGLLAENDSLYSAANIALMHH LMAALRAHSLFHKDQHYVIQDGEIVIVDEFTGRLMSGRRWSEGLHQAVEAKEGVEIKRENQTLASITFQNYFRLYT KLSGMTGTADTEAFEFQSIYNLETVIIPTNRPVQRKDFNDQIFRSAEEKFEAVVKDIEECHKRGQPVLVGTTSIEN SELVSKLLTQAGLPHNVLNAKEHEREALIVAQAGKVGAITVATNMAGRGTDIVLGGNLKHQTDAIRADETLSDEEK QAQIAALEDGWQAEHDKVMEAGGLHIIGTERHESRRIDNQLRGRSGRQGDPGSSRFYLSFEDPLLRLFALDRAAAI |

| | | |
|---|---|---|
| | | LNRLAPERGVAIEHNLLTRQIEGAQRKVEGRNFDMRKQVLEYDDVANEQRKVIYSQRNEILTSKDISDLMQEIRSD<br>VVSDLVDTYMPPDSMEEQWDIPTLENRLAAEFRLHEDIQSWLKADNAIDGQDIKERLIERIENEYAAKTELVGKQA<br>MADFERNVMLQVIDNQWREHLAAMDYLRQGIHLRSYAQKNPKQEYKREAFTMFQDLWNGIKFHIASLLTSVQIEQN<br>PVAVVEEQPIGNIQSIHSESPDMEELLGQSQTDLVTEAFNPDGTDFSPEALEARGQIVHRNDPCPCGSGLKYKQCH<br>GKLA |
| 148 | 1560 | MLNKDQFADNHFIRTIIEEDLESGKHTAVQTRFPPEPNGYLHIGHAKSICLNFGLAYIYDGLCNLRFDDTNPEKEN<br>DEYVNAIKEDVEWLGFHWAGEPRFASNYFDQLYDYAVGLIKDGKAYVDDLTPEEMREYRGTLTEAGKNSPYRDRSV<br>EENLDLFTRMKNGEFPDGSKTLRLKIDMASGNINMRDPVIYRIRRAHHHNTGDKWCIYPMYDYTHCISDAIEGITH<br>SLCTLEFEAHRPLYDCVLDNIPAPHATRPRQYEFSRLELLYTITSKRKLNQLVVEKHVSGWDDPRMPTISGMRRRG<br>YTPEGLRLFAKRAGISKSENIVDMSVLEGAIREELENSAPRLMAVLNPLKVTLTNFETGRTQSRRAAFHPNHEEMG<br>EREVPISQTIYIEADDFAENPPKGFKRLIPGGEVRLRHGYVIKCDEVVKDEAGNVVELKCSIDHDTLGKNPEGRKV<br>KGVIHWVSAEHAAEIKVRLYDRLFTVERPDAVRGEDGEYLPFTDFLNPESVKEITAYAEPAAKDLPAESRWQFERI<br>GYFVTDRKDHGKDTPVFNRTVTLKDSWQPK |
| 149 | 1572 | MLEAYRKAAAERAALGIPALPLNAQQTADLVELLKSPPAGEGEFLVELLAHRVPPGVDDAAKVKASFLAAVAEGSA<br>SSPLISPEYATELLGTMLGGYNIHALIELLDDDKLASIAAKGLKHTLLMFDSFHDVQEKAEKGNKYAQEVLQSWAD<br>AEWFASRAKVPEKITVTVFKVDGETNTDDLSPAPDAWSRPDIPLHALAMLKNPRDGITPDKPGEVGPIKLLEELKA<br>KGHPVAYVGDVVGTGSSRKSATNSVIWHTGEDIPFVPNKRFGGVCLGGKIAPIFFNTQEDSGALPIEVDVSALKMG<br>DVVDILPYEGKIVKNGETVAEFELKSQVLLDEVQAGGRINLIIGRGLTAKAREALKLPASTAFRLPQAPAESKAGF<br>TLAQKMVGRACGLPEGQGVRPGTYCEPRMTTVGSQDTTGPMTRDELKDLACLGFSADMVMQSFCHTAAYPKPVDVK<br>THKELPAFISTRGGVSLRPGDGVIHSWLNRLLLPDTVGTGGDSHTRFPIGISFPAGSGLVAFAAATGVMPLDMPES<br>VLVRFSGKLQPGVTLRDLVNAIPLYAIKQGLLTVAKAGKKNIFSGRILEIEGLPDLKVEQAFELTDASAERSAAGC<br>TVKLNKEPIIEYMKSNVVLMKNMIANGYQDPRTLERRIKAMEKWLANPELLEADKDAEYAAVIEINMDDIKEPIIA<br>CPNDPDDVCFMSERSGTKIDEVFIGSCMTNIGHFRAASKLLEGKADTPVRLWIAPPTKMDAKQLSDEGHYGVLGRA<br>GARMEMPGCSLCMGNQAQVREGATVMSTSTRNFPNRLGKNTFVYLGSAELAAICSKLGKIPTVEEYQANIGIINEQ<br>GDKIYRYMNFNEIDSYNEVAETVNV |
| 150 | 1574 | MQVYYDKDADLSLIKGKTVAIIGYGSQGHAHAANLKDSGVNVVIGLRQGSSWKKAEAAGHVVKTVAEATKEADVVM<br>LLLLPDETMPAVYHAEVTANLKEGATLAFAHGFNVHYNQIVPRADLDVIMVAPKGPGHTVRSEYKRGGGVPSLIAVY<br>QDNSGKAKDIALSYAAANGGTKGGVIETTFREETETDLFGEQAVLCGGVVELIKAGFETLTEAGYAPEMAYFECLH<br>EMKLIVDLIFEGGIANMNYSISNNAEYGEYVTGPEVVNASSKEAMRNALKRIQTGEYAKMFIQEGNVNYASMTARR<br>RLNADHQVEKVGAQLRAMMPWITANKLVDQDKN |
| 151 | 1577 | MQLSGAQIIVQSLKAEGVEYVFGYPGGAVIEIYDALFQLNKFKHILTRHEQAAVHAADAYARVSGKVGVALVTSGP<br>GVTNALTGIATAYTDSIPMVVISGQVGNSLIGTDAFQEVDTVGITRPCVKHNFLVTDINELAETIKKAFQIAASGR<br>PGPVVVDVPKDVTQAMAKFSYPQEDIFIRSYQPVVQGHIGQIKKAVQMLASAKRPVVYFGGGVVLGNASEELTRFV<br>RMTGAPCTGTLMGLGAYPSGDRQFLGMLGMHGTYEANLAMQNADVVLAVGARFDDRVVSVPSKFFEKAKKVIHIDV<br>DPSSIAKRVKVDIPIVGDVKNVLSEMVALWQKQESVPSEDALGKWWKTIEEWRSRDCLWFDNGSEIIKPQYVIQKL<br>AEITGNSAIITSDVGQHQMFAAQYYPFERPRQWLNSGGLGTMGVGLPYAIGAKLAAPDQDVFCITGDGSIQMNIQE<br>LSTCFQYRIPVNVITLNNGYLGMVRQWQEIYYGGRESETYFDSLPDFVKLAEAYGHIGIRVDKKSDVEGALLEALN<br>QKDRLVFIDFLTDQKQNVMPMVGNGKGLDEMVLPPHMRADGKA |
| 152 | 1581 | MKKLNTQSPDFQAGLKALLAFETAQNPETERIVADICADVQKRGDAALIEYTNKFDQTNAKSIDDLILTQADLNAA<br>FERIPNDVQTALQTAARRVESYHQRQKMESWSYTDEDGTLLGQQITPLDRVGIYVPGGKAAYPSSVIMNAMPAHVA<br>GVKEIIMVVPTPKGERNDIVLAAAYVAGVTKVFTVGGAQAVAALAYGTETIPQVDKITGPGNAFVAAAKRRVFGVV<br>GIDMVAGPSEILVIADGTTPADWVAMDLFSQAEHDEIAQAILIGTSQAYLDEVEAAMDRLIETMPRRDIIEASLGN<br>RGAMILAKDLDEACEIANYISPEHLELSVENPQEWAKKIRHAGAIFMGRYTGESLGDYCAGPNHVLPTSRTARFSS<br>PLGTYDFQKRSSLIQVSEQGAQKLGETASVLAHGESLTAHARAAEFRMK |
| 153 | 1583 | MNLTKTQRQLHNFLTLAQEAGSLSKLAKLCGYRTPVALYKLKQRLEKQAEDPDARGIRPSLMAKLEKHTGKPKGWL<br>DRKHRERTVPETAAESTGTAETQIAETASAAGCRSVTVNRNTCETQITVSINLDGSGKSRLDTGVPFLEHMIDQIA<br>RHGMIDIDISCKGDLHIDDHHTAEDIGITLGQAIRQALGDKKGIRRYGHSYVPLDEALSRVVIDLSGRPGLVYNIE<br>FTRALIGRFDVDLFEEFFHGIVNHSMMTLHIDNLSGKNAHHQAETVFKAFGRALRMAVEHDPRMAGQTPSTKGTLT<br>A |
| 154 | 1595 | MKTSELRQKFLKFFETKGHTVVRSSSLVPHDDPTLLFTNAGMNQFKDVFLGFDKRPYSRATTAQKCVRAGGKHNDL<br>ENVGYTARHHTFFEMMGNFSFGDYFKRDAIHFAWEFLTSPEWLNIPKDKLLATVYAEDDEAYNIWLNEIGMPSERI<br>VRIGDNKGAKYASDNFWQMGDTGPCGPCSEIFYDHGEEIWGGIPGSPEEDGDRWIEIWNCVFMQFNRDEQGNMNPL<br>PKPSVDTGMGLERIAAVMQHVHSNYEIDLFQDLLKAVARETGAPFRMEEPSLKVIADHIRSCSFLIADGVLPSNEG<br>RGYVLRRIIRRAVRHGYKLGQSKPFFHKLVADLVKEMGGAYPELKEKQAQIEEALKNEESRFAQTLETGMALLENA<br>LVKGGKTLGGEIIFKLYDTYGFPYDLTADICRERNIEPDEAGFEREMEAQRARARAAQSFKANAQLPYDGQDTEFK<br>GYSERQTESKVLALYKDGEQVNELNEGDSGAVVIDFTPFYAESGGQVGDVGYIFSGENRFEVRDTQKIKAAVFGQF<br>GVQTSGRLKVGDSVTAKVDDEIRNANMRNHSATHLMHKALRDVLGRHVEQKGSLVTAESTRFDISHPQAVTAEEIA<br>EVERRRVNEAILANVAVNAAIMSMEDAQKTGAMMLFGEKYGEEVRVLQMGGFSTELCGGTHVSRTGDIGLFKIISEG<br>GIAAGVRRIEAITGLNALKWAQEQERLVKDIIAETKAQTEKDVLAKIQAGAAHAKALEKELARAKAELAVHAGAKL |

| | | |
|---|---|---|
| | | LDDAKDLGAAKLVAAQIEADAAALREIVTDLTGKSDNAVILLAAVNDGKVSLCAGVSKPLTGKVKAGDLVKFAAEQ VGGKGGGRPDLAQAGGTDADKLPAVLDSVKDWVGAKLV |
| 155 | 1604 | MELVFIRHGQSEWNAKNLFTGWRDVKLSEQGLAEAAAAGKKLKENGYEFDIAFTSVLTRAIKTCNIVLEESDQLFV PQIKTWRLNERHYGQLQGLDKKQTAEQYGDEQVRIWRRSYDTLPPLLDKDDEFSAHKDRRYAHLPADVVPDGENLK VTLERVLPFWEDQIAPAILSGKRVLVAAHGNSLRALAKHIEGISDEDIMGLEIPTGQPLVYKLDDNLKVIEKFYL |
| 156 | 1621 | MGIYDFQMKDAEGNAVDLSGYRGKVLLIVNTATRCGLTPQYEALQKLYAQYTAEGLEILDFPCNQFREQAPESSGE IAQVCMMKFGTKFKIFDKIEVNGANTAPLYAYLKSVKPQDKGNHLFKDFVLKLAALGEKRDEGDIKWNFTKFLVNR DGEVVERFAPSVTPEEIEADIRALL |
| 157 | 1636 | LLFSSLLFSSLLFSSLLFSSAAQAASEDSSRSPYYVQADLAYAAERITHDYPKPTGTDKDKISTVSDYFRNIRAHS YHPRVSVGYDFGGWRIAADYASYRKWNNNKYSVNIKKGRETQDNREELKTENQENGSFHAASSLGLSAIYDFKLND KFDKFKPYIGARVAYGHVKHQVHSVETKTTIVTSKPAATSPQGGPIIET |
| 158 | 1640 | MSLYPIYNFSAGPAVLPEAVLETARQEMLDYNGTGFPVMAMSHRSEMFLSILHHAEQDLRQLLKVPDNYKILFLQG GATTQFNMAAMNLAHGFRTADAVVTGNWSRIAYEQMSRLTDTEIRLAAHGGEQFDYLDLPPVETWDVAPDSAFVHF AVNETVNGLQYREVPCLSEGMPPLVCDMSSEILSREFDVADYGLIYAGAQKNIGPAGVTVVIVREDLLERCPNDIP DVFNYRSHINRDGMYNTPSTYAIYMSGLVFRWLQAQGGVKKIEAVNRLKAQTLYETIDGSDGFYINRIRPNARSKM NVVFQTGDEELDRRFVLEAELQGLCLLKGYKTVGGMRASIYNAMPLEGVRALADFMRDFQRRYG |
| 159 | 1642 | MSREMLQLAEALASEKNVDAEVVFQALEFALSTAAKKKADREHMDVRVQINRDTGEYQTFRRWLIVADEDYTYPDV EKTIEEIQEEIPGTTIQIGEYYEEQLPNEGFGRQAAQTAKQIILQRIRDAEREQNLNEFLAVKEDIVSGTVKRVER HGIIVEVVAGKLDALIPRDQMIPRENFRSGDRIRALFLRVEEIGNTGRKQVILSRTSGDFLVKLYANEVPEIADGM LEIRAVARDPGQRAKVAVKANDQRIDPQGTCIGVRGSRVNAVSNELSGERIDVVLWSPEPAQFVMSALSPAEVSRI VIDEDKHAVDVIVAEDQLALAIGRGGQNVRLASDLTGWQLNIMTSAEADERNAAEDAAIRRLFMDHLNVDEETADV LVQEGFATLEEVAYVPAAELLAIEGFDEEIVDMLRNRARDAILTMAIAAEEKLGEVSDDMRNLEGIDADMLRSLAE AGITTRDDLAELAVDELIEITGVNEETAKAVILTAREHWFTEDK |
| 160 | 1655 | MVHIMFNQAAQELTTIRDILRFAVSRFNEAGLFFGHGTDNAHDEAAYLILHTLNLPLDMLAPYLDAKLLEAEKEEV LAVIERRAVEHIPAAYLTHQAWQGEFDFYVDERVIIPRSFIYELLGDGLRPWIEYDELVHNALDLCTGSGCLAIQM AHHYPDAQIDAVDVSLDALEVAGINVEDYGLEERIRLIHTDLFEGLEGTYDLIVSNPPYVDAESVELLPEEYLHEP ELALGSGADGLDATRQILLNAAKFLNPKGVLLVEIGHNRDVLEAAYPELPFTWLETSGGDGFVFLLTREQLLGEE |
| 161 | 1684 | MLDIQLLRSNTAAVAERLARRGYDFDTARFDTLEERRKSVQVKTEELQASRNSISKQIGALKGQGKHEEAQAAMNQ VAQIKTDLEQAAADLDAVQKELDAWLLSIPNLPHESVPAGKDETENVEVRKVGTPREFDFEIKDHVDLGEPLGLDF EGGAKLSGARFTVMRGQIARLHRALAQFMLDTHTLQHGYTEHYTPYIVDDTTLQGTGQLPKFAEDLFHVTRGGDET KTTQYLIPTAEVTLTNTVADSIIPSEQLPLKLTAHSPCFRSEAGSYGKDTRGLIRQHQFDKVEMVQIVHPEKSYET LEEMVGHAENILKALELPYRVITLCTGDMGFGAAKTYDLEVWVPAQNTYREISSCSNCEDFQARRLKARFKDENGK NRLVHTLNGSGLAVGRTLVAVLENHQNADGSINIPAALQPYMGGVAKLEVK |
| 162 | 1691 | MARHVWQAGRFEIGLDKPKIMGIVNLTPDSFSDGGVYSQNAQTALAHAEQLLKEGADILDIGGESTRSGADYVSPE EEWARVEPVLAEVAGWGVPISLDTRRTVIMEKALALGGIDIINDVAALNDEGAVELLARQADTGICLMHMQGLPKT MQINPKYQDVVGEVARYLKARSAECIAAGIAPQRIILDPGFGSSGFGKPLQHNIALMRHLPELMAETGFPLLIGVSR KSTIGELTGEANAAERVHGSVAAALASVARGAQIVRVHDVKATADALKVWEALGINL |
| 163 | 1710 | MTDLNTLFANLKQRNPNQEPFHQAVEEVFMSLDPFLAKNPKYTQQSLLERIVEPERVVMFRVTWQDDKGQVQVNRG YRVQMSSAIGPYKGGLRFHPTVDLGVLKFLAFEQVFKNALTTLPMGGGKGGSDFDPKGKSDAEVMRFCQAFMTELY RHIGADTDVPAGDIGVGGREIGYLFGQYKKIRNEFSSVLTGKGLEWGGSLIRPEATGYGCVYFAQAMLQTRNDSFE GKRVLISGSGNVAQYAAEKAIQLGAKVLTVSDSNGFVLFPDSGMTEAQLAALIELKEVRRERVATYAKEQGLQYFE KQKPWGVAAEIALPCATQNELDEEAAKTLLANGCYVVAEGANMPSTLGAVEQFIKAGILYAPGKASNAGGVATSGL EMSQNAIRLSWTREEVDQRLFGIMQSIHESCLKYGKVGDTVNYVNGANIAGFVKVADAMLAQGF |
| 164 | 1716 | MAFYAFKAMRAAALAAAVALVLSSCGKGGDAAQGGQPAGREAPAPVVGVVTVHPQTVALTVELPGRLESLRTADVR AQVGGIIQKRLFQEGSYVRAGQPLYQIDSSTYEAGLESARAQLATAQATLAKADADLARYKPLVAAEAVSRQEYDA AVTAKRSAEAGVKAAQAAIKSAGISLNRSRITAPISGFIGQSKVSEGTLLNAGDATVLATIRQTNPMYVNVTQSAS EVMKLRRQIAEGKLLAADGVIAVGIKFDDGTVYPEKGRLLFADPAVNESTGQITLRAAVPNDQNILMPGLYVRVLM DQVAVDNAFVVPQQAVTRGAKDTVMIVNAQGGMEPREVTVAQQQGTNWIVTSGLKDGDKVVVEGISIAGITGAKKV TPKEWASSENQAAAPQSGVQTASEAKPASEAK |
| 165 | 1796 | MIMAKKISILVGSLRRASFARKVALNAAEMFPEGWQAEIVEIGHLPLYNFDYDDPAVEDVPLPESYTAFRETIKAS DGILFVTSENNRTIPACLKNAVDIGSKPNADVAWKNKPAGIISHSVGKMGGYSSQKNLRLALSYFDMPVTGQPEVF LGNSPTLFDENGKLIDSARDFVQSYINQFVGLIERNAK |
| 166 | 1809 | MNNLIKINLLPYREEMNKRKQQQFKTLMYGAVLTGVAAVAATYLFIDNMINNQSERNTLLETSIAHLDTELSEIQK LKQEKDAFLIKKNKIEELQLKRLQAAKILDSLNEAVPGSTYLTSLDAVTADSYRLSGRTSSDNRVAAMMRAMPNTG IFKQPELLSIKKNNSHQEFTLQATLQPIVKAAESKENPASGNAQEAN |
| 167 | 1810 | MASKSSKTNLDLNNLHLLNLPARLFIALLAVAAVLGLGYAGLFKSQMESLEEYEAKETELKNTYKQKSIDAASLNN LRDELASIRSAFDIMLKQLPTDAEIPNLVQELHQAGSSNGLRLDSVMPQPPVDDGPIKRLPYSISITGNYEQISQF |

| | | |
|---|---|---|
| | | TRDVGSLSRIITLESLKIAQSPENGGNPDGKSSILNLSAIATTYQAKSVEELAAEAAQNAEQK |
| 168 | 1811 | MKHYALLISFLALSACSQGSEDLNEWMAQTRREAKAEIIPFQAPTLPVAPVYSPPQLTGPNAFDFRRMETDKKGEN APDTKRIKETLEKFSLENMRYVGILKSGQKVSGFIEAEGYVYTVGVGNYLGQNYGRIESITDDSIVLNELIEDSTG NWVSRKAELLLNSSDKNTEQAAAPAAEQN |
| 169 | 1812 | MNTKLTKIISGLFVATAAFQTASAGNITDIKVSSLPNKQKIVKVSFDKEIVNPTGFVTSSPARIALDFEQTGISMD QQVLEYADPLLSKISAAQNSSRARLVLNLNKPGQYNTEVRGNKVWIFINESDDTVSAPARPAVKAAPAAPAKQQAA APSTKSAVSVSEPFTPAKQQAAAPFTESVVSVSAPFSPAKQQAAASAKQQAAAPAKQQAAAPAKQQAAAPAKQTNI DFRKDGKNAGIIELAALGFAGQPDISQQHDHIIVTLKNHTLPTTLQRSLDVADFKTPVQKVTLKRLNNDTQLIITT AGNWELVNKSAAPGYFTFQVLPKKQNLESGGVNNAPKTFTGRKISLDFQDVEIRTILQILAKESGMNIVASDSVNG KMTLSLKDVPWDQALDLVMQARNLDMRQQGNIVNIAPRDELLAKDKALLQAEKDIADLGALYSQNFQLKYKNVEEF RSILRLDNADTTGNRNTLISGRGSVLIDPATNTLIVTDTRSVIEKFRKLIDELDVPAQQVMIEARIVEAADGFSRD LGVKFGATGKKKLKNDTSAFGWGVNSGFGGDDKWGAETKINLPITAAANSISLVRAISSGALNLELSASESLSKTK TLANPRVLTQNRKEAKIESGYEIPFTVTSIANGGSSTNTELKKAVLGLTVTPNITPDGQIIMTVKINKDSPAQCAS GNQTILCISTKNLNTQAMVENGGTLIVGGIYEEDNGNTLTKVPLLGDIPVIGNLFKTRGKKTDRRELLIFITPRIM GTAGNSLRY |
| 170 | 1835 | MSVIQDLQSRGLIAQTTDIEALDALLNEQKIALYCGFDPTADSLHIGHLLPVLALRRFQQAGHTPIALVGGATGMI GDPSFKAAERSLNSAETVAGWVESIRNQLTPFLSFEGGNAAIMANNADWFGSMNCLDFLRDIGKHFSVNAMLNKES VKQRIDRDGAGISFTEFAYSLLQGYDFAELNKRHGAVLEIGGSDQWGNITAGIDLTRRLHQKQVFGLTLPLVTKSD GTKFGKTEGGAVWLNAKKTSPYQFYQFWLKVADADVYKFLKYFTFLSIEEIDAIEAKDKASGSKPEAQRILAEEMT RLIHGEEALAAAQRISESLFAEDQSSLTESDFEQLALDGLPAFEVSDGINVVEALVKTGLASSNKEARGFVNSKAV LLNGKPAEANNPNHAAERPDDACLLNGEHKRFGKYTILRRGKRNHALLVWK |
| 171 | 1838 | MSLKCGIVGLPNVGKSTLFNALTQSGIEAANYPFCTIEPNVGIVEVPDPRMAELAKIVNPQKMQPAIVEFVDIAGL VAGASKGEGLGNQFLANIRETDAIVNVVRCFDDDNIVHVAGRVDPIADIETIGTELALADLASVEKAIVREEKRAR SGDKDAQKLVDLCKKLLPHLDEGKPVRSFGLDAEERAMLKPLFLLTAKPAMYVGNVAEDGFENNPHLDRLKELAAK ENAPVVAVCAAMESEIAELEDDEKAEFLAEMGLEEPGLNRLIRAGYDLLGLQTYFTAGVKEVRAWTIHKGDTAPQA AGVIHTDFERGFIRAQVISYDDFVSLGGEAKAKEAGKMRVEGKEYVVQDGDVMHFLFNV |
| 172 | 1843 | MPTQSKHASINIGLIQAREALMTQFRPILNQANITDQQWRIIRLLAENGTLDFQDLANQACILRPSLTGILTRLEK AGLVVRLKPSNDQRRVFLKLTAEGEKLYEEIGEEVDERYDAIEEVLGREKMLLLKDLLAELAKIEDALNS |
| 173 | 1849 | MSTPALLVLADGSVFHGTSIGYEGSTSGEVVFNTSMTGYQEILTDPSYCKQIVTLTYPHIGNTGTNAEDEESRSVY AAGLIIRDLPLLHSNFRASESLHDYLVRNKTVAIADIDTRRLTTLLREKGAQGGAILTGADATIEKAQELIAAFGS MVGKDLAKEVSCTETYEWTEGEWALGKGFVTPDEQPYHVVAYDFGVKTNILRMLASRGCRLTVVPAQTSAEDVLAL NPDGVFLSNGPGDPEPCTYAIKAVQKLIESGKPIFGICLGHQLISLAIGAKTLKMRFSHHGANHPVQDLDSGKVVI TSQNHGFAVDADTLPANARITHKSLFDNTLQGIELTDKPVFCFQGHPEASPGPQDVGYLFDKFIGNMKAAKRA |
| 174 | 1854 | MSIPINFNNLKYLLNDMRNKNRIIEAFPFNYNQRQYAVILTRYKPDEPRPDDYAQAKLEFFNLNSENSIFAYADFY EVHFKSATDFINFFKINVQAGAAKIREIFQSFSNLFADFIPTQTKKDLDIIYKKIVATRLEPNSPNTIYCYDVRRN GKDKAGKPNRRSVENSEKAKILRPELYEKFKADSNYSFFFSDNPSDEKTDAEIIREVTNRQ |
| 175 | 1855 | MPKRTDLKSILIIGAGPIVIGQACEFDYSGAQACKALREEGYKVILVNSNPATIMTDPEMADVTYIEPIMWQTVEK IIAKERPDAILPTMGGQTALNCALDLARNGVLAKYNVELIGATEDAIDKAEDRGRFKEAMEKIGLSCPKSFVCHTM NEALAAQEQVGFPTLIRPSFTMGGSGGGIAYNKDEFLAICERGFDASPTHELLIEQSVLGWKEYEMEVVRDKNDNC IIICSIENFDPMGVHTGDSITVAPAQTLTDKEYQIMRNASLAVLREIGVDTGGSNVQFAVNPANGEMIVIEMNPRV SRSSALASKATGFPIAKVAAKLAVGFTLDELRNDITGGKTPASFEPSIDYVVTKIPRFAFEKFPAADDRLTTQMKS VGEVMAMGRTIQESFQKALRGLETGLCGFNPRSEDKAEIRRELANPGPERMLFVADAFRAGFTLEEIHEICAIDPW FLAQIEDLMKEEKAVSDGILSDLDFAALRRLKRKGFSDKRLAQLLNVSEKEVREHRYALKLHPVYKRVDTCAAEFA TETAYLYSTYEEECESRPSDRKKVMILGGGPNRIGQGIEFDYCCVHAALALRESGFETIMVNCNPETVSTDFDTSD RLYFEPLTLEDVLEIVRTENPWGVIVHYGGQTPLKLANALVENGVNIIGTSADSIDAAEDRERFQKVLNDLGLRQP PNRIAHNEEEALVKAEEIGYPLVVRPSYVLGGRAMQVVHSAEELQKYMREAVQVSEDSPVLLDFFLNNAIEVDVDC VSDGKDVVIGGIMQHVEQAGIHSGDSGCSLPPYSLSEEIQDEIRRQTKAMAYALGVVGLMNVQFAVQDGVVFVLEV NPRASRTVPFVSKATGVPLAKVGARCMAGISLKEQGVEKEVVPDFYAVKEAVFPFIKFPGVDTILGPEMRSTGEVM GVGASFGEAYYKAQLGAGERLNPTGKIFLSVREEDKERVIKTAKNFQVLGYGICATRGTAQYLTEHGLIVQTINKV PEGRPHIGDALKNGEIALVVNTVSSDPQSVSDSHIIRQSALQQRVPQYTTTAGGEAMSEGAKSRDHLGVYSVQELH GRLKNRN |
| 176 | 1859 | MDISDFDFTLPEKLIAQHPPEVRGSSRLLVALPDMPLQDRVFGDLPDYVEAGDVLVFNNTKVMKARLFGQKDSGGR IEALIERVLDNHTALAHIRSSKSPKPGMGLVFEGGIRAVTVGREGELFCLRFEGGETVYELLEQNGHLPLPPYIER AADADDDSRYQTVYAKYQGAVAAPTAGLHFTEELLHRLKDKGAVTAEVTLHVGAGTFQPVRVDKIEEHKMHSEWFE VPSETAAAVEAAKARGNKVWAVGTTSMRALESAARATGRLKAGQGDTDIFITPGYRFNVVDRLVTNFHLPKSTLLM LVSAFSGMGHIRAAYRHAVEREYRFFSYGDAMVLGRNEGVVR |
| 177 | 1861 | MLKKVLIANRGEIALRVLRACREMGIATVAVHSEADKDSLHVKLADESVCIGPAASAQSYLNVPAIIAAAEVSCAD AVHPGYGFLAENADFAEQVEQSGFTFIGPKPDTIRLMGDKVSAKHAMIAAGVPCVPGSDGALPDDGEEILKIADKV |

| | | |
|---|---|---|
| | | GYPVIIKASGGGGGRGMRVVEKKEDLLQSVEMTKAEAGAAFGNPMVYMERYLQRPRHVEIQVIADEHGNAIYLAER<br>DCSLQRRHQKVIEEAPAPFITEKERAKIGNACADACKRIGYRGAGTFEFLYEDGEFFFIEMNTRVQVEHPVTELIT<br>GVDIVQEQLRIAAGLPLQYKQKDIQVEGHAFECRINAEDPYNFIPSPGLIESCHLPGGFGIRVDSHIYQGYRIPPY<br>YDSLIGKICVHGKTREQAMAKMRVALAELAVTGIKTNTPLHRDLFADAGFQKGGVSIHYLEHWLEDRKAKQDK |
| 178 | 1862 | MPYQQITVNVNDAVAERLADALMEHGALSAAIEDAYAGTQNEQAIFGEPGMPAEQIWQQSKVIALFGEHDEAAAII<br>QTATQECGLKDLAYTGETIEDQDWVRLTQSQFDPIRISDRLWITPSWHEVPEGSAVNLRLDPGLAFGTGSHPTTRL<br>CLKWLDTQLKNGESVLDYGCGSGILTIAALKLGAGFAVGVDIDEQAVRAGKDNAAQNNVDAQFFLPDGLPQGQFDV<br>VVANILANPLRMLGEMLAARTKQGGRIVLSGLLDEQAEELGGIYSQWFDLDPAETEEGWARLSGVKR |
| 179 | 1864 | MNRNEILFDRAKAIIPGGVNSPVRAFGSVGGVPRFIKKAEGAYVWDENGTRYTDYVGSWGPAIVGHAHPEVVETVC<br>EAALGGLSFGAPTEGEIVIAEEIAKIMPSVERLRLVSSGTEATMTAIRLARGFTGRDKIIKFEGCYHGHSDSLLVK<br>AGSGLLTFGNPSSAGVPADFTKHTLVLEYNNIAQLEEEAFAQSGNEIACVIVEPFVGNMNLVRPTEAFVKALRGLTE<br>KYGAVLIYDEVMTGFRVALGGAQSLHGITPDLTTMGKVIGGGMPLAAFGGRKDIMECISPLGGVYQAGTLSGNPIA<br>VAAGLKTLEIIQREGFYENLTARTEQLVQGFRTAADAAGIEFTADSVGGMFGLYFAAHAPRNYADMARSNIEGFKQ<br>FFHGMLDRNVAFGPSAYEAGFVSAAHTPELIDETVAVAVEVFKAMAE |
| 180 | 1903 | MTLAEFWPLCLRRLHDMLPQGQFAQWIAPLTVGEEGGVWVVYGKNQFACNMLKSQFAGKIEAVREELAAGRSAFVF<br>KPGEGVRYEMAAVEGAVEPAEPSLHAVSEGMPVQEVLLDELPSEEPVKPAASKTAADILAERMKNLPHEPRQAAGS<br>ASRPESVAVAKARTDVQRDAEEEARYEQTNLSPDYTFDTLVEGKGNRLAAAAAQAIAESPGQSYNPFFLYGSTGLGK<br>THLVQAVGNELLKNRPDAKVRYMHSDDYIRSFMKAVRNNTYDVFKQQYKQYDLLIIDDIQFIKGKDRTMEEFFYLY<br>NHFHNEKKQLILTCDVLPAKIEGMDDRLKSRFSWGLTLELEPPELEMRIAILQKKAEAAGISIEDEAALFIANLIR<br>SNVRELEGAFNRVGASSRFMNRPVIDIDLARTALQDIIAEKHKVITADIIIDAVAKYYRIKISDVLGKKRTRNIAR<br>PRQVAMSLTKELTTLSLPSIGDSFGGRDHTTVMHGIRAVAKLREEDPELAQDYEKLLILIQN |
| 181 | 1920 | MTQDKILILDFGSQVTQLIARRVREAHVYCELHSFDMPLDEIKAFNPKGIILSGGPNSVYESDYQADTGIFDLGIP<br>VLGICYGMQFMAHHLGGEVQPGNQREFGYAQVKTIDSELTRGIQDGEPNTLDVWMSHGDKVSKLPDGFAVIGNTPS<br>CPIAMMENAEKQFYGIQFHPEVTHTKQGRALLNRFVLDICGAQPGWTMPNYIEEAVAKIREQVGSDEVILGLSGGV<br>DSSVAAALIHRAIGDQLTCVFVDHGLLRLNESKMVMDMFARNLGVKVIHVDAEGQFMAKLAGVTDPEKKRKIIGAE<br>FIEVFDAEEKKLTNAKWLAQGTIYPDVIESAGAKTKKAHAIKSHHNVGGLPENMKLKLLEPLRDLFKDEVRELGVA<br>LGLPREMVYRHPFPGPGLGVRILGEVKKEYADLLRQADDIFIQELRNTTDENGTSWYDLTSQAFAVFLPVKSVGVM<br>GDGRTYDYVIALRAVITSDFMTAHWAELPYSLLGKVSNRIINEVKGINRVVYDVSGKPPATIEWE |
| 182 | 1921 | MSTQDLNGKIALVTGASRGIGAAIADTLAAAGAKVIGTATSESGAAAISERLAQWGGEGRVLNSAEPETIESLIAD<br>IEKAFGKLDILVNNAGITRDNLLMRMKEEEWDDIMQVNLKSVFRASKAVLRGMMKQRSGRIINITSVVGVMGNAGQ<br>TNYAAAKAGLIGFSKSMAREVGSRGITVNCVAPGFIDTDMTRALPEETRQTFTAQTALGRFGDAQDIADAVLFLAS<br>DQAKYITGQTLHVNGGMLMP |
| 183 | 1930 | MMTQTLLIELLTEELPPKALNNLGNHFAASVAEGLEKAQLVDGAAEFTAYASPRRLAVQVKNVKAVQADQKIVKKG<br>PAVANAMKDGAPTKALEGFARGAGAKIEDLTIVHDGKQDVYAYEYVQIGKPLGGLLEDIINQAVKKLPIPKVMRWG<br>SSTFTFVRPVHGLVVLHGGDIVNVSVLGLQSGNKTLGHRFLSDGEITIENADSYAAQMREQGKVVASFAERKAAIQ<br>TVLEGQARRLNATAAADEALLDEVTALVEWPVVLEAGFEEHFLAVPQECLILTMQQNQKYFPLLDQNGKLMNRFLL<br>VSNLQTEDPSHIIQGNERVLRARLSDAEFFYKQDQKATLESRLPKLTNVVYHNKIGSQAERIERLQSIAAHIAKAL<br>GADAAAAERAARLAKADLVTEMVGEFPELQGTMGKYYARLDGETEEITEAVEQHYQPRFAGDNLPEGKIAAAVALA<br>DKLETLVGIWGIGLIPTGDKDPYALRRAALGILRMLMQYGLDVNELIQTAFNSFPQGLLNEKTPSETADFMQARLA<br>VLLQNDYPQDIVAAVLAKQPRRLDDLTAKLQAVAAFKQLPEAAALAAANKRVQNLLKKADAELGAVNESLLQQDEE<br>KALFAAAQGLQPKIAAAVAEGNFQTALSELASVKPQVDAFFDGVMVMAEDAAVKQNRLNLLNRLAEQMNAVADIAL<br>LGE |
| 184 | 1934 | MSQGKIVQIIGAVVDVEFPRDMIPRVYDALKLDENGLTLEVQQLLGDGVVRAIAMGSSDGLKRGMTVSNTGAPITV<br>PVGKGTLGRIVDVLGTPVDEAGPIDTDKSRAIHQAAPKFDELSSTTELLETGIKVIDLLCPFAKGGKVGLFGGAGV<br>GKTVNMMELINNIAKAHSGLSVFAGVGERTREGNDFYHEMKDSNVLDKVAMVYGQMNEPPGNRLRVALTGLTMAEY<br>FRDEKDENGKGRDVLFFVDNIYRYTLAGTEVSALLGRMPSAVGYQPTLAEEMGRLQERITSTQTGSITSIQAVYVP<br>ADDLTDPSPATTFAHLDATVVLSRDIASLGIYPAVDPLDSTSRQLDPMVLGQEHYDVARGVQSTLQKYKELRDIIA<br>ILGMDELSDEDKLTVMRARKIQRFLSQPFHVAEVFTGSPGKYVALRDTIAGFKAILNGEYDHLPEQAFYMVGSIEE<br>AVEKAKTLN |
| 185 | 1936 | MQLNPAEISDLIKAKIENLSVNAEVRTCGTVISVTDGIVRIHGLSDAMQGEMLEFPGNTFGLAMNLERDSVGAVVL<br>GEYEHIKEGDTVTCTGRILEVPVGRELVGRVVDALGRPIDGKGPINTTLTAPIEKIAPGVIARKSVDQPMQTGLKA<br>IDSMVPVGRGQRELIIGDRQTGKTAVALDAIVNQKGTGVICIYVAIGQKASSIANVVRKLEEHGAMEHTIVVAATA<br>SEAAALQYIAPYSGCTMGEFFRDRGEDALIVYDDLSKQAVAYRQISLLLRRPPGREAYPGDVFYLHSRLLERAARV<br>NEHEVEKLTNGEVKGKTGSLTALPIIETQAGDVSAFVPTNVISITDGQIFLETDLFNAGIRPAINAGISVSRVGGA<br>AQTKVIKKLGGGIRLALAQYRELAAFSQFASDLDEATRKQLEHGEVVTELMKQKQFSTLNTAEMALTLWAINNGSY<br>SDVPVAKALAFESEFLSFVRTQHPEVLEAVNASGAMSDESEKTLEAAMKSFKSSYAYQA |
| 186 | 1937 | MAEFATIARPYAKALFGLAQEKNQIESWLGGLEKLAAVVQEGKVASLIDRPETNASEKADILIDLVGLKDKELKNF<br>VIVLAGQKRLSILPEVYAQYQDLTLSFNHIKSAVIYSAYPLTDKQVGELVQMLNKRFDSELKISVEIEPELIGGIK |

| | | |
|---|---|---|
| | | VEVGDQVLDLSVQGKLSALYTTMTN |
| 187 | 1938 | MNINATLFAQIIVFFGLVWFTMKFVWPPIAKALDERAAKVAEGLAAAERGKSDFEQAEKKVAELLAEGRNQVSEMV ANAEKRAAKIVEEAKEQASSEAARIAAQAKADVEQELFRARESLREQVAVLAVKGAESILRSEVDASKHAKLLDTL KQEL |
| 188 | 1953 | MMTLYSGITCPFSHRCRFVLYEKGMDFEIKDVDIYNKPEDLAVMNPYNQVPVLVERDLVLHESNIINEYIDERFPH PQLMPGDPVMRGRGRLVLYRMEKELFNHVQVLENPAATNKEQAKAREAIGNGLTMLAPSFSKSKYILGEDFSMIDV ALAPLLWRLDHYDVKLGKSAAPLLKYAERIFQREAFIEALTPAEKAMRK |
| 189 | 1966 | MSPSPFIEMKDVAFAYGDRPILKNINFSIPQGNFAAVMGGSGSGKTTLMRLITGQIRPQSGQVLIEGRDLAGFSAD ELYEHRRRMGVLFQHGALFTDLSVFDNIAFPMRELTRLPEAVIRDLVLLKLNAVGLRGVENLMPSELSGGMSRRVA LARTIALDPEIMLYDEPFTGLDPISLGVIAHLISRVNKALRSTSIMVTHDIEKSLEIVDQVIFLAHGEIMFSGSPQ EMRELDSPWVRQFVGGLADGPVAYRYPAQTSLQQDLLG |
| 190 | 1968 | MKQLAMYINGRFENDFNGEWRDVLNPSTEEAIAREPKGGKADVDRAVAAARAAQPAWERLPAVERGAYLRKIAQGI RERADELTDTIVAEGGKTKDLARVEVMFTADYLDYQAEWARRYEGEIIQSDRPRENILLFKRPLGVIAGILPWNFP FFLIARKMGPALVTGNTIVVKPSSVTPINCHIFAEIVDAVGLPAGVFNVVNGPGAEIGNALSAHPQVDMVSLTGSV EAGRQVMEAASANITKVSLELGGKAPAIVLKDADLDLAVKSILASRVGNTGQICNCAERVYVHSSLKDAFIEKMTA AMKGVRYGNPAEAEAGALEMGPLIEERAVKAVAEKVERAVKQGAKLVCGGKRAEGRGYFFEPTLLTDTDNSMDIMK EETFGPVLPVSAFDTLDQVIALANDCEFGLTSSVYTTNLNEAFYVTRRLQFGETYINRENFEAMQGFHAGWKKSGI GGADGKHGLEEYLQTQVVYLETDI |
| 191 | 1982 | MSNRPTLLLVDGSSYLYRAYHAMGQNLTAPDGAPTGALYGVLNMLRRLRSEYPHDYCAVVFDAKGKNFRHQMFEEY KATRPPMPDDLRPQAEALPDLVRLTGWPVLVIGQVEADDVIGTLAKQGAEHGLRVIVSTGDKDMAQLVDERVTLVN TMSSETLDIEGVKAKFGVRPDQIRDYLALMGDKVDNVPGVEKCGPKTAVKWLEAYGSLAGVMEHASEIKGKVGENL QAALPQLPLSYDLVTIKTDVDLHAELSDGIESLRRTTPKWAQLVVDFKRWGFRTWLKEAESNMNTGSTDDLFGSDS IGEQAALNAEMPFEKQAEKATAPEKLDYQAVTTEAQFAALLDKLSRADTIGIDTETTSLDAMNASLVGISIAFQAG EAVYIPVGHSLTAAPEQLDLQDVLGRLKPHLGNPALKKIGQNLKYDQHVFANYGIALNGIAGDAMLASYIIESHLG HGLDELSERWLGLETITYESLCGKGAKQIGFADVAIGQATEYAAQDADFALRLEAHLRAQMDEKQLEMYEKMELPV AQVLFEMERNGVQIDRAELARQSAELGAELMKLEQEAYAAAGQPFNLNSPKQLQEILFDKMGIPTKGLKKTAKGGI STNEAVLEQLAPDYPLPKIILQNRSLAKLKSTYTDKLPEMISPKDGRVHTTYAQAVAITGRLASNNPNLQNIPIRT EEGRKVRRAFTAPQGSVIVSADYSQIELRIMAHLSGDKTLIAAFQNGEDVHRRTAAEVFGTAPENVSSEQRRYAKS INFGLIYGMGQYGLAKSLGIDNLSAKNFIDRYFARYPGVAEYMQRTKEQAAAQGYVETLFGRRLYLPDIRNKNANA RAGAERAAINAPMQGTASDLIKRAMIDVSRWLSECEASPWDELLQSKLIMQVHDELVLEVVETELDFVKEKLPQIM AKVDGGLLDVPLVAEVGVGENWEEAH |
| 192 | 1996 | MSVVLPLRGVTALSDFRVEKLLQKAAALGLPEVKLSSEFWYFVGSEKALDAATVEKLQALLAAQSVEQTPKAREGL HLFLVTPRLGTISPWASKATNIAENCGLAGIERIERGMAVWLEGRLNDEQKQQWAALLHDRMTESVLPDFQTASKL FHHLESETFSGVDVLGGGKEALVKANTEMGLALSADEIDYLVENYQALQRNPSDVELMMFAQANSEHCRHKIFNAD FILNGEKQPKSLFGMIRDTHNAHPEGTVVAYKDNSSVIEGAKIERFYPNAAENQGYRFHEEDTHIIMKVETHNHPT AIAPFAGAATGAGGEIRDEGATGKGSRPKAGLTGFTVSNLNIPDLKQPWEQDYGKPEHISSPLDIMIEGPIGGAAF NNEFGRPNLLGYFRTFEEKFDGQVRGYHKPIMIAGGLGSIQAQQTHKDEIPEGALLIQLGGPGMLIGLGGGAASSM DTGTNDASLDFNSVQRGNPEIERRAQEVIDRCWQLGGKNPIISIHDVGAGGLSNAFPELVNDARRGAVFKLREVPL EEHGLNPLQIWCNESQERYVLSILEKDLDAFRAICERERCPFAVVGTATDDGHLKVRDDLFANNPVDLPLNVLLGK LPKTTRTDKTVAPSKKPFHAGDIDITEAAYRVLRLPAVAAKNFLITIGDRSVGGLTHRDQMVGKYQTPVADCAVTM MGFNTYRGEAMSMGEKPTVALFDAPASGRMCVGEAITNIAAVNIGDIGNIKLSANWMAACGNEGEDEKLYRTVEAV SKACQALDLSIPVGKDSLSMKTVWQDGEEKKSVVSPLSLIISAFAPVKDVRKTVTPELKNVEDSVLLFVDLGFGKA RMGGSAFGQVYNNMSGDAPDLDDTGRLKAFYSVIQQLVAENKLLAYHDRSDGGLFAVLVEMAFAGRCGLDIDLNLL LAQTFITNHTALSQSLRTEEVKALAEWQETIARTLFNEELGAVIQVRKQDVADIINLFYQQQLHHNVFEIGTLTDE NTLIIRDGQTHLISDNLIKLQQTWQETSHQIQRLRDNPACADSEFALIGDNERSALFADVKFDVNEDIAAPFINSG AKPKIAILREQGVNGQIEMAAAFTRAGFDAYDVHMSDLMAGRIHLADFKMLAACGGFSYGDVLGAGEGWAKSILFH PALRDQFAAFFADPDTLTLGVCNGCQMVSNLAEIIPGTAGWPKFKRNLSEQFEARLSMVHVPKSASLILNEMQGSS LPVVVSHGEGRADFALHGGNISADLGIALQYIDGQNQVTQTYPLNPNGSPQGIAGVTNADGRITIMMPHPERVYRA AQMSWKPEGWTELSGWYRLFAGARKALG |
| 193 | 2000 | MNQTAINRADVRTRFIFDDMPVRGLHVRLENVWQHIVKQKNYPAAIRRALGELLAAGVLLSGNLKNEGTLIVQVQG RGRLKMLVAEAASDRTVRATARWDETAEIADDESLGDLLGEGGVFVLTLQPKDGEPWQGVVPLEGGGIAQMLVNYM KRSEQLDTHIVLSASDEAAGGLLVQRLPEEVLDEEAWEHVSTLARTLTAEELAGLDAQHVLYRLFHETPPRVFEPE TFEFSCTCSRGKVSDMLLMLGGEEVGGVVVEQGSIEVDCDFCHSKYVFDETDVNALFGEDVVGVAKGLPRHTVQ |
| 194 | 2039 | MKKSLIALTLAALPVAAMADVTLYGTIKAGVETSRSVFHQNGQVTEVTTATGIVDLGSKIGFKGQEDLGNGLKAIW QVEQKASIAGTDSGWGNRQSFIGLKGGFGKLRVGRLNSVLKDTGDINPWDSKSDYLGVNKIAEPEARLISVRYDSP EFAGLSGSVQYALNDNAGRHNSESYHAGFNYKNGGFFVQYGGAYKRHHQVQEGLNIEKYQIHRLVSGYDNDALYAS VAVQQQDAKLTDASNSHNSQTEVAATLAYRFGNVTPRVSYAHGFKGLVDDADIGNEYDQVVVGAEYDFSKRTSALV SAGWLQEGKGENKFVATAGGVGLRHKF |
| 195 | 2057 | MKTFSAKPHEVKREWFVIDAQDKVLGRVAAEVASRLRGKHKPEYTPHVDTGDYIIVINADKLRVTGAKFEDKKYFR |

| | | |
|---|---|---|
| | | HSGFPGGIYERTFREMQEQFPGRALEQAVKGMLPKGPLGYAMIKKLKVYAGAEHAHAAQQPKVLELK |
| 196 | 2079 | MKVGFVGWRGMVGSVLMQRMKEENDFAHIPEAFFFTTSNVGGAAPDFGQAAKTLLDANNVAELAKMDIIVTCQGGD YTKSVFQALRDSGWNGYWIDAASSLRMKDDAIIVLDPVNRDVLDNGLKNGVKNYIGGNCTVSLMLMALGGLFQNDL VEWATSMTYQAASGAGAKNMRELISGMGAVHAQVADALADPAGSILDIDRKVSDFLRSEDYPKANFGVPLAGSLIP WIDVDLGNGQSKEEWKGGVETNKILGRSDNPTVIDGLCVRVGAMRCHSQAITLKLKKDLPVSEIETILAGANDWVK VIPNEKEASIHELTPAKVTGTLSVPVGRIRKLGMGGEYISAFTVGDQLLWGAAEPLRRVLRIVLGSL |
| 197 | 2086 | MKFIDEAKIEVAAGKGGNGATSFRREKFVPRGGPDGGDGGKGGSVWAEADENTNTLVEYRFVKRYQAKNGEKGHGS DRYGAGADDIVLKMPVGTLIRDLDTGETVADLTYHGQRVCLAKGGKGGLGNIHFKSSVNRAPKQSTPGEEGEARSL QLELKVLADVGLLGMPNAGKSTLITAVSAARPKIANYPFTTLHPNLGVVRIDENHSFVMADIPGLIEGAAEGAGLG HRFLKHLSRTGLLLHVVDLAPFDETVNPAEEALAIINELRKYDEELYGKPRWLVLNKLDMLDEEEAQTRTAAFLEA VGWDYPKPDDRFQFDMETPRLFQISALTHQGTQELVHQINQYLTEKKRIEAEKAEAEKAAANVEIIEQQPKTDTGV FKPE |
| 198 | 2101 | MSQITMRQMIEAGVHFGHQTRFWNPKMAQYIFGARNKIHIVNLEKTLPMFQDAQEAVRRLVANKGTVLFVGTKRQA RDIIREEATRAGMPFVDYRWLGGMLTNYKTVKQSIKRLEEKTAALENAAESGFSKKEILEMQRDVEKLERSLGGIK NMKGLPDAIFVIDTGYQKGTLVEAEKLGIPVIAVVDTNNSPDGVKYVIPGNDDSAKAIRLYCRGIADAVLEGKNQA LQETVAAAQEAAAE |
| 199 | 2102 | MAEITAKMVADLRAATGLGMMECKKALVEAEGNFDKAEEILRIKSGAKAGKLAGRTAAEGVLAYAINGNVGALVEV NCETDFVAKDAGFVEFANFVAKTAAEKKPASVEELSELVEAERKAIIAKLGENMSVRRFQVIDTANQLVAYIHGAL ATEGVLVEYKGSEDVARKIGMHIVAAKPQCVSEAEVDAETVEKERHIYTEQAIASGKPADIAAKMVEGRIRKFLAE ITLNGQAFVMNPDQTVAQFSKENGTEVISFVRYKVGDGIEKKAVDYAAEVAAAAKV |
| 200 | 2103 | MTQQIKYKRVLLKLSGESLMGSDPFGINHDTIVQTVGEIAEVVKMGVQVGIVVGGGNIFRGVSAQAGSMDRATADY MGMMATVMNALALKDAFETLGIKARVQSALSMQQIAETYARPKAIQYLEEGKVVIFAAGTGNPFFTTDTAAALRGA EMNCDVMLKATNVDGVYTADPKKDPSATRYETITFDEALLKNLKVMDATAFALCRERKLNIVVFGIAKEGSLKRVI TGEDEGTLVHC |
| 201 | 2129 | MSQNHTILQSLPVGQKVGIAFSGGGLDTSAALLWMKLKGALPYAYTANLGQPDEDDYNAIPKKAMEYGAENARLIDC RAQLAHEGIAAIQCGAFHVSTGGIAYFNTTPLGRAVTGTMLVSAMKEDDVNIWGDGSTYKGNDIERFYRYGLLTNP ALKIYKPWLDQQFIDELGGRHEMSEFLIANGFNYKMSVEKAYSTDSNMLGATHEAKDLEFLNSGIKIVKPIMGVAF WDENVEVSPEEVSVRFEEGVPVALNGKEYADPVELFLEANRIGGRHGLGMSDQIENRIIEAKSRGIYEAPGMALFH IAYERLVTGIHNEDTIEQYRINGLRLGRLLYQGRWFDSQALMLRETAQRWVAKAVTGEVTLELRRGNDYSILNTES PNLTYQPERLSMEKVEDAAFTPLDRIGQLTMRNLDITDTRVKLGIYSQSGLLSLGEGSVLPQLGNKQ |
| 202 | 2138 | MEAEVINQLNNTLNDLEKRSEDIRVYMDYQGKKDRLEEVIGLSEDPELWNDPKRAQEIGKERKILEGIVLTLDNIA SGIEDNRMLIEMTVEENDEEGFAAVQEDVAGLEKQMADLEFKRMFNQPADPNNCFIDITAGAGGTEAEDWAGMLFR MYSRYAERKGFRIEILEEDDGEIAGINRATIRVEGEYAYGLLRTETGVHRLVRYSPFDSNNKRHTSFASVFVYPEI DDSIEIEINPADLRIDTYRASGAGGQHINKTDSAVRITHEPTGIVVQCQNDRSQHANKAAAMEMLKSKLYELEMRK RNEEKQALEEGKSDVGWGSQIRSYVLDSSRIKDLRTGYEVGNTKAVLDGDLDGFIEASLKQGV |
| 203 | 2154 | MSVLIIVEHDNKQLNPTTLHAVTAAAKLGKVDLLVAGNGASAVVEFAKQVAGVKKVLVADAAHYAEGLAEELAPLV VKLAADYRYVAATATTFGKNLLPRVAALLDVPQISDLTEIVDNTTFVRPIYAGNAFETVQADSEKLVLTFRATVFD AVAAQGGNAEVINVEATPAQNLSRFVNRQLSHSDRPELTQAKVIVSGGRALGSAEKFNEVLTPLADVLGAAIGASR AAVDAEYAPNDAQVGQTGKVVAPQLYFAIGISGAIQHVAGMQDSKVIVAINKDADAPIFNVADYGLVGDLFEIVPQ LTEVLKN |
| 204 | 2155 | MKALVAVKRVVDYNVKVRVKADGSDVDIGNVKMSMNPFDEIAVEEAVRLKEAGKVSEIVAVSLGEKKCEETLRTAL AMGADRAIHVETDTKLESLAVAKLLKAVADKENPQIFFLGKQAIDDDANQVAQMLAALLNAAQGTFASKVQIEGDE VQIVREIDGGEETIALKLPAVISADLRLNEPRFVKLPNIMAAKKKPLEKLTPDDLVADISPRLKTVKFAEPKARQA GVKVASVAELVEKLKNEAKVI |
| 205 | 2159 | MSIKVAINGFGRIGRLALRQIEKAHDIEVVAVNDLTPAEMLLHLFKYDSTQGRFQGTAELKDDAIVVNGKEIKVFA NPNPEELPWGELGVDVILECTGFFTNKTKAEAHIRAGARKVVISAPGGNDVKTVVYGVNQDILDGSETVISAASCT TNCLAPMAAVLQKEFGVVEGLMTTIHAYTGDQNTLDAPHRKGDLRRARAAALNIVPNSTGAAKAIGLVIPELNGKL DGSAQRVPVASGSLTELVSILERPVTKEEINAAMKAAASESYGYNEDQIVSSDVVGIEYGSLFDATQTRVMTVGGK QLVKTVAWYDNEMSYTCQLVRTLEYFAGKI |
| 206 | 1126 1164 | MKTVSTAVVLAAAAVSLTGCATESSRSLEVEKVASYNTQYHGVRTPISVGTFDNRSSFQKGIFSDGEDRLGSQAKT ILVTHLQQTNRFNVLNRTNLNALKQESGISGKAHNLKGADYVVTGDVTEFGRRDVGDHQLFGILGRGKSQIAYAKV ALNIVNVNTSEIVYSAQGAGEYALSNREIIGFGGTSGYDATLNGKVLDLAIREAVNSLVQAVDNGAWQPNR |
| 207 | 1799 | MSEYLFTSESVSEGHPDKVADQVSDAILDAILAQDPKARVAAETLVNTGLCVLAGEITTTAQVDYIKVARETIKRI GYNSSELGFDANGCAVGVYYDQQSPDIAQGVNEGEGIDLNQGAGDQGLMFGYACDETPTLMPFAIYYSHRLMQRQS ELRKDGRLPWLRPDAKAQLTVVYDSETGKVKRIDTVVLSTQHDPSIAYEELKNAVIEHIIKPVLPSELLTDETKYL INPTGRFVIGGPQGDCGLTGRKIIVDTYGGAAPHGGGAFSGKDPSKVDRSAAYACRYVAKNIVAAGLATQCQIQVS YAIGVAEPTSISIDTFGTGKISEEKLIALVREHFDLRPKGIVQMLDLLRPIYSKSAAYGHFGREEPEFTWERTDKA |

| | | AALRAAAGL |
|---|---|---|
| 208 | 0110 | MALLNILQYPDERLHTVAKPVEQVDERIRKLIADMFETMYESRGIGLAATQVDVHERVVVMDLTEDRSEPRVFINP VIVEKDGETTYEEGCLSVPGIYDTVTRAERVKVEALNEKGEKFTLEADGLLAICVQHELDHLMGIVFVERLSQLKQ GRIKTKLKKRQKHTI |
| 209 | 0957 | MSQYDVVVIGAGPGGYVAAIRAAQLGFKTACVDAGVNKAGNAPALGGTCLNVGCIPSKALLQSSEHFHAAQHEFAE HGITVGDVKFDAAKMIERKDAIVTKLTGGVKFLFQKNKVTSLFGTASFAGKNGDAYQIEVDNKGEKTVIEAKHVIV ATGSVPRPLPQVAIDNVNVLDNEGALNLTEVPAKLGVIGSGVIGLEMGSVWNRVGAEVTILEAAPTFLAAADQQIA KEAFKYFTKEQGLSIELGVKIGDIKSEGKGVSVAYETAAGEAKTEVFDKLIVAIGRIPNTKGLNAEAVGLEKDERG FIKVDGECRTNLPNVWAIGDVVRGPMLAHKASDEGVAVAERIAGQKPHIDFNNVPFVIYTDPEIAWVGKTEEQLKA EGVEYKKGTSGFGANGRALAMGKAKGTVKVLADAKTDRILGVHMIGPVVSELVTEGVTALEFFASSEDIARIIHAH PTLSEVVHEAALAADKRALHG |
| 210 | 1011 | MMIEKSISIVDGKEYSVFAVSHEFRYTFDEPILVADLISSLKAYETLTSSYLPAILNQLFDVKIQKIKVAVSEIER GSFLEKLIFNLFFKDEDAYNEFCLKIRKFLGTENQDGSINMSKIIMFAMTTLLGVGAGYLLFKNPPQEKQAITNNI VTVINADSSVALDGEHLVSVVKEVTGSSKQKTAENVAKVYAPASKNNGSITLGTDDVRIEPVAQQTVATLPKDVDL RDTPLTEDYTDIDVQIRATDRDKNSGWYAVIDQIVPSRVRLELPEDIDLNRLANNATIRANVTVEFDLKQNGSRKP KKIILTSLSTD |
| 211 | 1055 | MFSKSVTLAQYDPDLAAAIAQEDQRQQDHVELIASENYVSCAVMDAQGSQLTNKYAEGYPGKRYYGGCEYVDIVEQ LAIDRVKELFGAAYANVQPHSGSQANQAVYASVLKPGDTILGMSLAHGGHLTHGASVNISGKLYNAVTYGLDENEV LDYAEVERLALEHKPKMIVAGASAYALQIDWAKFREIADKVGAYLFVDMAHYAGLVAGGEYPNPVPFCDFVTTTH KTLRGPRGGVILCRDNTHEKALNSSIFPSLQGGPLMHVIAAKAVAFKEALQPEFKQYAKQVKINAAAMAEELVKRG LRIVSGRTESHVFLVDLQPMKITGKAAEAALGKAHITVNKNAIPNDPEKPFVTSGIRIGSAAMTTRGFNEADARVL ANLVADVLSNPEDEANLAKVRKQVTALCNKYPVYGA |
| 212 | 1437 | MSARENILAKLKKADALPMEEPAVFDYYREMGVSWGSEVERLKHWAAAMRAVKTEIYWVTKSNWMQVFREAAEGKG LKNILLPLATEHGQIARAALADSNIEPIAFEREIDTWKTEFFTNIDAGFSGAQCGIARTGTLMLFSSPEEPRTLSL VPPVHFCLFDTSKMYNEFHNAVEGEKLVENGMPTNVFLISGPSKTADIQLTLAYGAHGPRDLVILAILPDHISPAD LEENA |
| 213 | 1460 | MSLNKVILIGRLGRDPEVRYMPNGEAVCNFSVATSETWNDRNGQRVERTEWHNITMYRKLAEIAGQYLKKGGLVYL EGRIQSRKYQGKDGIERTAYDIVANEMKMLGGRNENSGGAPYEEGYGQSQEAYQRPAQQSRQPASDAPSHPQEAPA APRRQPVPAAAPVEDIDDDIPF |
| 214 | 1540 | MNKKHGFPLTLTALAIATAFPAYAAQAGGATPDAAQTQSLKEITVRAAKVGRRSKEATGLGKIVKTSETLNKEQVL GIRDLTRYDPGVAVVEQGNGASGGYSIRGVDKNRVAVSVDGVAQIQAFTVQGSLSGYGGRGGSGAINEIEYENIST VEIDKGAGSSDHGSGALGGAVAFRTKEAADLISDGKSWGIQAKTAYGSKNRQFMKSLGAGFSKDGWEGLLIRTERQ GRETRPHGDIADGVEYGIDRLDAFRQTYDIKRKTREPPFFSVEGERESKPVAKLAGYGKYLNNQLNRWVKERIEQNQ PLSAEEEAQVREAQARHENLSAQAYTGGGRILPDPMDYRSGSWLAKLGYRFGGRHYVGGVFEDTKQRYDIRDMTEK QYYGTDEAEKFRDKSGVYDGDDFRDGLYFVPNIEEWKGDKNLVRGIGLKYSRTKFIDEHHRRRRMGLLYRYENEAY SDNWADKAVLSFDKQGVATDNNTLKLNCAVYPAVDKSCRASADKPYSYDSSDRFHYREQHNVLNASFEKSLKNKWT KHHLTLGFGYDASKAISRPEQLSHNAARISESTGFDENNQDKYLLGKPEVVEGSVCGYIETLRSRKCVPRKINGSN IHISLNDRFSIGKYFDFSLGGRYDRKNFTTSEELVRSGRYVDRSWNSGILFKPNRHFSVSYRASSGFRTPSFQELF GIDIYHDYPKGWQRPALKSEKAANREIGLQWKGDFGFLEISSFRNRYTDMIAVADHKTKLPNQAGQLTEIDIRDYY NAQNMSLQGVNILGKIDWNGVYGKLPEGLYTTLAYNRIKPKSVSNRPGLSLRSYALDAVQPSRYVLGFGYDQPEGK WGANIMLTYSKGKNPDELAYLAGDQKRYSTKRASSSWSTADVSAYLNLKKRLTLRAAIYNIGNYRYVTWESLRQTA ESTANRHGGDSNYGRYAAPGRNFSLALEMKF |
| 215 | 1554 | MTKFIFVTGGVVSSLGKGIAAASIAAILESRGLNVTMLKLDPYINVDPGTMSPFQHGEVFVTDDGAETDLDLGHYE RFIDSTMTRRNSFSTGQVYENVIAKERRGDYLGGTVQVIPHITDEIKRRIHEGAAGYDVAIVEIGGTVGDIESLPF LEAIRQMRSQLGRNNTLFAHLSYVPYIAAAGEIKTKPTQHTVKEMLSIGLQPDILICRMDRTMPADERRKIALFCN VEERAIVGSYDVDSIYECPEMLHDQGIDNIITEQLQLNVQQADLTAWKKIVHAIQNPKHTVKIAMVGKYVDLTESY KSLIEALKHAGIHTETDVQITFVDSENIEKNKGDVSMLKDMDAILVPGGFGSRGVEGKIAAVRYARENNVPYLGIC LGMQIALIEYARDVAGLKGANSTEFDLKCAAPVVALIDEWQTADGSVETRDESTDLGGTMRLGAQEVELKAGSLAA KIYGSGHIRERHRHRYEVNNNYVPTLEQAGLVIGGVSAGRERLVETIELPNHPWFFACQFHPEFTSNPRKGHPLFT AFVKAALNNKKA |
| 216 | 1576 | MRHILSVLIENESGAMSRVVGLFSARDYNIDSLAVAPTEDKTLSRMTIVTHGDEQVIEQITKQLNKLIEVIKVVDL NESRFVERELMLVKVRAAGKDRDEFLRLTEIYRGSIIDVTDRSYTIEITGSTDKLDSFLETVGRAQILETVRTGAA GIGRGERILKI |
| 217 | 1808 | MRLFKSLKNPKKTDAKLPKKSSGLNNRAAIGIDIDQHSIKMVQLSGRSLNQIQLEKYVIAKLPKNIIQGNKVQNYD QLVTYLQQAYAKLGTSCKNIIASVPQNLATIEQLTYTDKDAELDLQGFVESSISEVSSISLEEANYDYQVLSQSAA GEAVLAVASRKDEIEPLIDAFNAAGMKLSALDVDIFGQYNAYALWINHFAPELAAEKVAIFGVYAAQTYALVIQDG KILYKQETSVSEEQLNQLIQRTYQVTEEKAEEIINSPQKPSDYQESVANYFNQQITQEIQRVLQFYYTTQTADDMT DIKHILLTGEAARQEGIAQTVASQTNADVQCVHPARYFADNLKTDKQQFELDAPTLTRAFGLAVRGL |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0071725 A
- US 5753235 A
- EP 127839 A
- EP 155476 A
- WO 89046699 A
- US 5693506 A
- US 5659122 A
- US 5608143 A
- US 4738921 A
- EP 0036776 A
- EP 0121775 A
- US 4689406 A
- US 4551433 A
- EP 0267851 A
- EP 0219237 A
- EP 0324647 A, Chey
- US 4336336 A
- EP 0244042 A
- EP 0127328 A
- EP 0036259 A
- EP 0063953 A
- WO 8404541 A
- EP 0136829 A
- EP 0136907 A
- US 4745056 A
- EP 0284044 A
- EP 0329203 A
- US 4876197 A
- US 4880734 A
- EP 0164556 A
- EP 0196056 A
- WO 88024066 A
- EP 0012873 A
- JP O62096086 B
- US 4588684 A
- EP 0060057 A
- US 4546083 A
- US 4870008 A
- EP 0324274 A
- WO 8902463 A
- US 4837148 A
- US 4929555 A
- WO 9820734 A
- WO 9014837 A
- US 5591624 A
- WO 9637626 A
- WO 9530763 A
- WO 9205266 A
- US 5288641 A

- EP 0176170 A, Roizman
- WO 9504139 A, Wistar
- WO 9009441 A
- WO 9207945 A
- EP 0453242 A
- US 5091309 A
- US 5217879 A
- WO 9210578 A
- US 08405627 B
- WO 9421792 A
- WO 9507994 A
- WO 199738087 A
- US 4603112 A
- US 4769330 A
- WO 8901973 A
- US 5166057 A
- EP 0386882 A
- EP 0440219 A
- US 08366787 B
- US 6015686 A
- US 5149655 A
- US 5206152 A
- WO 9211033 A
- WO 199806437 A
- WO 9011092 A
- US 5580859 A
- US 5422120 A
- WO 9513796 A
- WO 9423697 A
- WO 9114445 A
- EP 524968 A
- US 4762915 A
- EP 0524968 A
- WO 9314778 A
- WO 901109 A
- WO 9806437 A, Zuckermann
- US 4683195 A
- US 4683202 A
- WO 0164922 A
- WO 0164920 A
- WO 03020756 A
- WO 0025811 A
- WO 0152885 A
- US 5597572 A
- US 5747653 A
- EP 0680512 A
- WO 0109350 A
- WO 0209746 A
- EP 0449958 A

- WO 0191788 A
- WO 0226212 A
- WO 9833487 A
- WO 0007621 A
- WO 9927960 A
- WO 9857659 A
- EP 08353180735898AND0761231 A
- WO 9602555 A
- WO 9816247 A
- WO 9818810 A
- WO 9840100 A
- WO 9855495 A
- WO 9837919 A
- WO 9852581 A
- WO 9952549 A
- WO 0121207 A
- WO 0121152 A
- WO 0062800 A
- WO 0023105 A
- WO 9911241 A
- WO 9318150 A
- WO 9601272 A
- WO 9601273 A
- WO 9725429 A
- WO 9804702 A
- WO 03007985 A

- WO 9924578 A
- WO 9936544 A
- WO 9957280 A
- WO 02079243 A
- WO 0202606 A
- WO 9927105 A
- WO 0027994 A
- WO 0037494 A
- WO 9928475 A
- WO 0234771 A
- EP 0372501 A
- EP 0378881 A
- EP 0427347 A
- WO 9317712 A
- WO 9403208 A
- WO 9858668 A
- EP 0471177 A
- WO 0056360 A
- WO 9101146 A
- WO 0061761 A
- WO 0172337 A
- WO 0138350 A
- WO IB0306281 W
- GB 0226734 A
- GB 0307131 A

**Non-patent literature cited in the description**

- **CLAASSEN et al.** *Vaccine,* 1996, vol. 14 (10), 1001-1008
- **NORAIS et al.** *Proteomics,* 2001, vol. 1, 1378-1389
- **WRIGHT et al.** *Infections and Immunity,* 2002, vol. 70 (8), 4028-4034
- **JOLLEY et al.** *Infection and Immunity,* 2001, vol. 69 (6), 3809-3816
- **SAMBROOK.** Molecular Cloning; A Laboratory Manual. 1989
- MOLECULAR CLONING; A LABORATORY MANUAL. 2001
- DNA Cloning. 1985
- Oligonucleotide Synthesis. 1984
- Nucleic Acid Hybridization
- Transcription and Translation. 1984
- Animal Cell Culture. 1986
- Immobilized Cells and Enzymes. IRL Press, 1986
- **B. PERBAL.** A Practical Guide to Molecular Cloning. 1984
- Methods in Enzymology. Academic Press, Inc, vol. 154-155
- Gene Transfer Vectors for Mammalian Cells. Cold Spring Harbor Laboratory, 1987
- Immunochemical Methods in Cell and Molecular Biology. Academic Press, 1987

- **SCOPES.** Protein Purification: Principles and Practice. Springer-Verlag, 1987
- Handbook of Experimental Immunology. 1986, vol. I-IV
- Expression of Cloned Genes in Mammalian Cells. **SAMBROOK et al.** Molecular Cloning: A Laboratory Manual. 1989
- **MANIATIS et al.** *Science,* 1987, vol. 236, 1237
- **ALBERTS et al.** Molecular Biology of the Cell. 1989
- **DIJKEMA et al.** *EMBO J.,* 1985, vol. 4, 761
- **GORMAN et al.** *PNAS USA,* 1982, vol. 79, 6777
- **BOSHART et al.** *Cell,* 1985, vol. 41, 521
- **SASSONE-CORSI ; BORELLI.** *Trends Genet.,* 1986, vol. 2, 215
- **BIRNSTIEL et al.** *Cell,* 1985, vol. 41, 349
- Termination and 3' end processing of eukaryotic RNA. **PROUDFOOT ; WHITELAW.** Transcription and splicing. 1988
- **PROUDFOOT.** *Trends Biochem. Sci.,* 1989, vol. 14, 105
- Expression of cloned genes in cultured mammalian cells. **SAMBROOK et al.** Molecular Cloning: A Laboratory Manual. 1989
- **GLUZMAN.** *Cell,* 1981, vol. 23, 175

- **KAUFMAN et al.** *Mol. Cell. Biol.,* 1989, vol. 9, 946
- **SHIMIZU et al.** *Mol. Cell. Biol.,* 1986, vol. 6, 1074
- **SUMMERS ; SMITH ; SUMMERS ; SMITH.** *Texas Agricultural Experiment Station Bulletin No. 1555,* 1987
- **LUCKOW ; SUMMERS.** *Virology,* 1989, vol. 17, 31
- **MILLER et al.** *Ann. Rev. Microbiol.,* 1988, vol. 42, 177
- The Regulation of Baculovirus Gene Expression. **FRIESEN et al.** The Molecular Biology of Baculoviruses. 1986
- **VLAK et al.** *J. Gen. Virol.,* 1988, vol. 69, 765
- **CARBONELL et al.** *Gene,* 1988, vol. 73, 409
- **MAEDA et al.** *Nature,* 1985, vol. 315, 592
- **LEBACQ-VERHEYDEN et al.** *Molec. Cell. Biol.,* 1988, vol. 8, 3129
- **SMITH et al.** *Proc. Nat'l Acad. Sci. USA,* 1985, vol. 82, 8404
- **MIYAJIMA et al.** *Gene,* 1987, vol. 58, 273
- **MARTIN et al.** *DNA,* 1988, vol. 7, 99
- **SMITH et al.** *Mol. Cell. Biol.,* 1983, vol. 3, 2156
- **MILLER et al.** *Bioessays,* 1989, vol. 4, 91
- Current Protocols in Microbiology. 1990, vol. 2
- **CARBONELL et al.** *J. Virol.,* 1985, vol. 56, 153
- **WRIGHT.** *Nature,* 1986, vol. 321, 718
- **FRASER et al.** *In Vitro Cell. Dev. Biol.,* 1989, vol. 25, 225
- **ZENK.** *Phytochemistry,* 1991, vol. 30, 3861-3863
- **VAULCOMBE et al.** *Mol. Gen. Genet.,* 1987, vol. 209, 33-40
- **CHANDLER et al.** *Plant Molecular Biology,* 1984, vol. 3, 407-418
- **ROGERS.** *J. Biol. Chem.,* 1985, vol. 260, 3731-3738
- **ROTHSTEIN et al.** *Gene,* 1987, vol. 55, 353-356
- **WHITTIER et al.** *Nucleic Acids Research,* 1987, vol. 15, 2515-2535
- **WIRSEL et al.** *Molecular Microbiology,* 1989, vol. 3, 3-14
- **YU et al.** *Gene,* 1992, vol. 122, 247-253
- **R.L. JONES ; J. MACMILLIN.** Gibberellins: in: Advanced Plant Physiology. Pitman Publishing Limited, 1984, 21-52
- **SHEEN.** *Plant Cell,* 1990, vol. 2, 1027-1038
- **MAAS et al.** *EMBO J.,* 1990, vol. 9, 3447-3452
- **BENKEL ; HICKEY.** *Proc. Natl. Acad. Sci.,* 1987, vol. 84, 1337-1339
- **WILMINK ; DONS.** *Plant Mol. Biol. Reptr,* 1993, vol. 11 (2), 165-185
- **REED ; MANIATIS.** *Cell,* 1985, vol. 41, 95-105
- **CROSSWAY.** *Mol. Gen. Genet,* 1985, vol. 202, 179-185
- **KRENS et al.** *Nature,* 1982, vol. 296, 72-74
- **KLEIN et al.** *Nature,* 1987, vol. 327, 70-73
- **KNUDSEN ; MULLER.** *Planta,* 1991, vol. 185, 330-336
- **FRALEY et al.** *Proc. Natl. Acad. Sci. USA,* 1982, vol. 79, 1859-1863
- **FROMM et al.** *Proc. Natl Acad. Sci. USA,* 1985, vol. 82, 5824
- **RAIBAUD et al.** *Annu. Rev. Genet.,* 1984, vol. 18, 173
- **CHANG et al.** *Nature,* 1977, vol. 198, 1056
- **GOEDDEL et al.** *Nuc. Acids Res.,* 1980, vol. 8, 4057
- **YELVERTON et al.** *Nucl. Acids Res.,* 1981, vol. 9, 731
- The cloning of interferon and other mistakes. **WEISSMANN.** Interferon. 1981
- **SHIMATAKE et al.** *Nature,* 1981, vol. 292, 128
- **AMANN et al.** *Gene,* 1983, vol. 25, 167
- **DE BOER et al.** *Proc. Natl. Acad. Sci.,* 1983, vol. 80, 21
- **STUDIER et al.** *J. Mol. Biol.,* 1986, vol. 189, 113
- **TABOR et al.** *Proc Natl. Acad Sci.,* 1985, vol. 82, 1074
- **SHINE et al.** *Nature,* 1975, vol. 254, 34
- Genetic signals and nucleotide sequences in messenger RNA. **STEITZ et al.** Biological Regulation and Development: Gene Expression. 1979
- Expression of cloned genes in Escherichia coli. **SAMBROOK et al.** Molecular Cloning: A Laboratory Manual
- **NAGAI et al.** *Nature,* 1984, vol. 309, 810
- **JIA et al.** *Gene,* 1987, vol. 60, 197
- **ALLEN et al.** *J. Biotechnol.,* 1987, vol. 5, 93
- **MAKOFF et al.** *J. Gen. Microbiol.,* 1989, vol. 135, 11
- **MILLER et al.** *Bio/Technology,* 1989, vol. 7, 698
- **MASUI et al.** *Experimental Manipulation of Gene Expression,* 1983
- **GHRAYEB et al.** *EMBO J.,* 1984, vol. 3, 2437
- **OKA et al.** *Proc. Natl. Acad. Sci.,* 1985, vol. 82, 7212
- **PALVA et al.** *Proc. Natl. Acad. Sci. USA,* 1982, vol. 79, 5582
- **DAVIES et al.** *Annu. Rev. Microbiol.,* 1978, vol. 32, 469
- **AMANN et al.** *Gene,* 1985, vol. 40, 183
- **POWELL et al.** *Appl. Environ. Microbiol.,* 1988, vol. 54, 655
- **MASSON et al.** *FEMS Microbiol. Lett.,* 1989, vol. 60, 273
- **PALVA et al.** *Proc. Natl. Acad Sci USA,* 1982, vol. 79, 5582

- **MILLER et al.** *Proc. Natl. Acad. Sci.,* 1998, vol. 85, 856
- **WANG et al.** *J. Bacteriol.,* 1990, vol. 172, 949
- **COHEN et al.** *Proc. Natl. Acad. Sci.,* 1973, vol. 69, 2110
- **DOWER et al.** *Nucleic Acids Res.,* 1988, vol. 16, 6127
- An improved method for transformation of Escherichia coli with ColE1-derived plasmids. **KUSHNER.** Genetic Engineering: Proceedings of the International Symposium on Genetic Engineering. 1978
- **MANDEL et al.** *J. Mol. Biol.,* 1970, vol. 53, 159
- **TAKETO.** *Biochim. Biophys. Acta,* 1988, vol. 949, 318
- **CHASSY et al.** *FEMS Microbiol. Lett.,* 1987, vol. 44, 173
- **FIEDLER et al.** *Anal. Biochem,* 1988, vol. 170, 38
- **AUGUSTIN et al.** *FEMS Microbiol. Lett.,* 1990, vol. 66, 203
- **BARANY et al.** *J. Bacteriol.,* 1980, vol. 144, 698
- Transformation of Streptococcus lactis by electroporation. **HARLANDER.** Streptococcal Genetics. 1981
- **PERRY et al.** *Infect. Immun.,* vol. 32, 1295
- **SOMKUTI et al.** *Proc. 4th Evr. Cong. Biotechnology,* 1987, vol. 1, 412
- **MYANOHARA et al.** *Proc. Natl. Acad. Sci. USA,* 1983, vol. 80, 1
- **COHEN et al.** *Proc. Natl. Acad. Sci. USA,* 1980, vol. 77, 1078
- **HENIKOFF et al.** *Nature,* 1981, vol. 283, 835
- **HOLLENBERG et al.** *Curr. Topics Microbiol. Immunol.,* 1981, vol. 96, 119
- The Expression of Bacterial Antibiotic Resistance Genes in the Yeast Saccharomyces cerevisiae. **HOLLENBERG et al.** Plasmids of Medical, Environmental and Commercial Importance. 1979
- **MERCERAU-PUIGALON et al.** *Gene,* 1980, vol. 11, 163
- **PANTHIER et al.** *Curr. Genet.,* 1980, vol. 2, 109
- **BOTSTEIN et al.** *Gene,* 1979, vol. 8, 17-24
- **BRAKE et al.** *Proc. Natl. Acad. Sci USA,* 1984, vol. 81, 4642-4646
- **STINCHCOMB et al.** *J. Mol. Biol.,* 1982, vol. 158, 157
- **ORR-WEAVER et al.** *Methods in Enzymol.,* 1983, vol. 101, 228-245
- **RINE et al.** *Proc. Natl. Acad. Sci. USA,* 1983, vol. 80, 6750
- **BUTT et al.** *Microbiol, Rev.,* 1987, vol. 51, 351
- **KURTZ et al.** *Mol. Cell. Biol.,* 1986, vol. 6, 142
- **KUNZE et al.** *J. Basic Microbiol.,* 1985, vol. 25, 141
- **GLEESON et al.** *J. Gen. Microbiol.,* 1986, vol. 132, 3459
- **ROGGENKAMP et al.** *Mol. Gen. Genet.,* 1986, vol. 202, 302
- **DAS et al.** *J. Bacteriol.,* 1984, vol. 158, 1165
- **DE LOUVENCOURT et al.** *J. Bacteriol.,* 1983, vol. 154, 737
- **VAN DEN BERG et al.** *Bio/Technology,* 1990, vol. 8, 135
- **CREGG et al.** *Mol. Cell. Biol.,* 1985, vol. 5, 3376
- **HINNEN et al.** *Proc. Natl. Acad. Sci. USA,* 1978, vol. 75, 1929
- **ITO et al.** *J. Bacteriol.,* 1983, vol. 153, 163
- **BEACH ; NURSE.** *Nature,* 1981, vol. 300, 706
- **DAVIDOW et al.** *Curr. Genet.,* 1985, vol. 10, 380-471
- **GAILLARDIN et al.** *Curr. Genet.,* 1985, vol. 10, 49
- **DAS et al.** *J. Bacteriol,* 1984, vol. 158, 1165
- **DE LOUVENCOURT et al.** *J. Bacteriol.,* 1983, vol. 154, 1165
- **DAVIDOW et al.** *Curr. Genet.,* 1985, vol. 10, 39
- Remington's Pharmaceutical Sciences. Mack Pub. Co, 1991
- Vaccine design: the subunit and adjuvant approach. Plenum Press, 1995
- **ROBINSON ; TORRES.** *Seminars in Immunology,* 1997, vol. 9, 271-283
- **DONNELLY et al.** *Annu Rev Immunol,* 1997, vol. 15, 617-648
- **JOLLY.** *Cancer Gene Therapy,* 1994, vol. 1, 51-64
- **KIMURA.** *Human Gene Therapy,* 1994, vol. 5, 845-852
- **CONNELLY.** *Human Gene Therapy,* 1995, vol. 6, 185-193
- **KAPLITT.** *Nature Genetics,* 1994, vol. 6, 148-153
- **HARTLEY ; ROWE.** *J Virol,* 1976, vol. 19, 19-25
- **GELLER.** *Science,* 1998, vol. 241, 1667-1669
- **FINK.** *Human Gene Therapy,* 1992, vol. 3, 11-19
- **EVANS.** *Nature,* 1989, vol. 339, 385
- **SABIN.** *J. Biol. Standardization,* 1973, vol. 1, 115
- **ARNOLD.** *J Cell Biochem,* 1990, L401
- **FISHER-HOCH.** *Proc Natl Acad Sci,* 1989, vol. 86, 317
- **FLEXNER.** *Ann NY Acad Sci,* 1989, vol. 569, 86
- **FLEXNER.** *Vaccine,* 1990, vol. 8, 17
- **MULLIGAN.** *Nature,* 1979, vol. 277, 108
- **MADZAK.** *J Gen Virol,* 1992, vol. 73, 1533

- **ENAMI.** *Proc Natl Acad Sci,* 1990, vol. 87, 3802-3805
- **ENAMI ; PALESE.** *J Virol,* 1991, vol. 65, 2711-2713
- **LUYTJES.** *Cell,* 1989, vol. 59, 110
- **MCMICHAEL.** *NEJ Med,* 1983, vol. 309, 13
- **YAP.** *Nature,* 1978, vol. 273, 238
- *Nature,* 1979, vol. 277, 108
- **BUCHSCHACHER.** *J. Virol.,* 1992, vol. 66, 2731
- **HAMRE.** *Proc Soc Exp Biol Med,* 1966, vol. 121, 190
- **CURIEL.** *Hum Gene Ther,* 1992, vol. 3, 147-154
- **WU.** *J Biol Chem,* 1989, vol. 264, 16985-16987
- **PHILIP.** *Mol Cell Biol,* 1994, vol. 14, 2411-2418
- **WOFFENDIN.** *Proc Natl Acad Sci,* 1994, vol. 91, 1581-1585
- **WU ; WU.** *J. Biol. Chem.,* 1987, vol. 262, 4429-4432
- **HUCKED.** *Biochem Pharmacol,* 1990, vol. 40, 253-263
- **PLANK.** *Bioconjugate Chem,* 1992, vol. 3, 533-539
- **WOFFENDIN et al.** *Proc. Natl. Acad. Sci. USA,* 1994, vol. 91 (24), 11581-11585
- **STRYER.** Biochemistry. W.H. Freeman, 1975, 236-240
- **SZOKA.** *Biochem Biophys Acta,* 1980, vol. 600, 1
- **BAYER.** *Biochem Biophys Acta,* 1979, vol. 550, 464
- **RIVNAY.** *Meth Enzymol,* 1987, vol. 149, 119
- **WANG.** *Proc Natl Acad Sci,* 1987, vol. 84, 7851
- **PLANT.** *Anal Biochem,* 1989, vol. 176, 420
- **HUG ; SLEIGHT.** *Biochim. Biophys. Acta,* 1991, vol. 1097, 1-17
- **STRAUBINGER.** *Meth. Enzymol.,* 1983, vol. 101, 512-527
- **FELGNER.** *Proc. Natl. Acad. Sci. USA,* 1987, vol. 84, 7413-7416
- **MALONE.** *Proc. Natl. Acad. Sci. USA,* 1989, vol. 86, 6077-6081
- **DEBS.** *J. Biol. Chem.,* 1990, vol. 265, 10189-10192
- **SZOKA.** *Proc. Natl. Acad. Sci. USA,* 1978, vol. 75, 4194-4198
- **STRAUBINGER.** *Meth. Immunol.,* 1983, vol. 101, 512-527
- **PAPAHADJOPOULOS.** *Biochim. Biophys. Acta,* 1975, vol. 394, 483
- **WILSON.** *Cell,* 1979, vol. 17, 77
- **DEAMER ; BANGHAM.** *Biochim. Biophys. Acta,* 1976, vol. 443, 629
- **OSTRO.** *Biochem. Biophys. Res. Commun.,* 1977, vol. 76, 836
- **FRALEY.** *Proc. Natl. Acad. Sci. USA,* 1979, vol. 76, 3348
- **ENOCH ; STRITTMATTER.** *Proc. Natl. Acad. Sci. USA,* 1979, vol. 76, 145
- **FRALEY.** *J. Biol. Chem.,* 1980, vol. 255, 10431
- **SZOKA ; PAPAHADJOPOULOS.** *Proc. Natl. Acad. Sci. USA,* 1978, vol. 75, 145
- **SCHAEFER-RIDDER.** *Science,* 1982, vol. 215, 166
- **BRESLOW.** *Annu Rev. Biochem,* 1985, vol. 54, 699
- **LAW.** *Adv. Exp Med. Biol.,* 1986, vol. 151, 162
- **CHEN.** *J Biol Chem,* 1986, vol. 261, 12918
- **KANE.** *Proc Natl Acad Sci USA,* 1980, vol. 77, 2465
- **UTERMANN.** *Hum Genet,* 1984, vol. 65, 232
- *Meth. Enzymol.,* 1986, 128
- **PITAS.** *J. Biochem.,* 1980, vol. 255, 5454-5460
- **MAHEY.** *J Clin. Invest,* 1979, vol. 64, 743-750
- **ATKINSON.** *Annu Rev Biophys Chem,* 1986, vol. 15, 403
- **RADDING.** *Biochim Biophys Acta,* 1958, vol. 30, 443
- **MEINKOTH ; WAHL.** *Anal. Biochem.,* 1984, vol. 138, 267-284
- **MATTEUCCI et al.** *J. Am. Chem. Soc.,* 1981, vol. 103, 3185
- **URDEA et al.** *Proc. Natl. Acad. Sci. USA,* 1983, vol. 80, 7461
- **AGRAWAL ; IYER.** *Curr Opin Biotechnol,* 1995, vol. 6, 12-19
- **AGRAWAL.** *TIBTECH,* 1996, vol. 14, 376-387
- **COREY.** *TIBTECH,* 1997, vol. 15, 224-229
- **BUCHARDT et al.** *TIBTECH,* 1993, vol. 11, 384-386
- **MULLIS et al.** *Meth. Enzymol.,* 1987, vol. 155, 335-350
- **TETTELIN et al.** *Science,* 2000, vol. 287, 1809-1815
- **PIZZA et al.** *Science,* 2000, vol. 287, 1816-1820
- **PARKHILL et al.** *Nature,* 2000, vol. 404, 502-506
- **COLE.** *Eur Respir J,* 2002, vol. 36, 78s-86s
- **MCCLELLAND et al.** *Nature,* 2001, vol. 413, 852-856
- **DE GROOT et al.** *Vaccine,* 2001, vol. 19, 4385-4395
- **STEPHENS.** *J Infect Dis,* 2000, vol. 181 (3), 521-523
- **TETTELIN et al.** *Science,* 2001, vol. 293, 498-506

- **WEINSTOCK et al.** *Res Microbiol,* 2000, vol. 151, 1551-158
- **WIZEMANN et al.** *Infect Immun,* 2001, vol. 69, 1593-1598
- **GRIFANTINI et al.** *Nat Biotechnol.,* 2002, vol. 20, 914-921
- **BJUNE et al.** *Lancet,* 1991, vol. 338 (8775), 1093-1096
- **DE KLEIJN et al.** *Vaccine,* 2001, vol. 20, 352-358
- **GENNARO.** Remington: The Science and Practice of Pharmacy. 2000
- **KRIEG.** *Vaccine,* 2000, vol. 19, 618-622
- **KRIEG.** *Curr opin Mol Ther,* 2001, vol. 3, 15-24
- **DEL GIUDICE et al.** *Molecular Aspects of Medicine,* 1998, vol. 19 (1
- Vaccine Design... Plenum, 1995
- **JOHNSON et al.** *Bioorg Med Chem Lett,* 1999, vol. 9, 2273-2278
- **COVACCI ; RAPPUOLI.** *J. Exp. Med.,* 2000, vol. 19, 587-592
- **COVACCI et al.** *Proc. Natl. Acad. Sci. USA,* 1993, vol. 90, 5791-5795
- **TUMMURU et al.** *Infect. Immun.,* 1994, vol. 61, 1799-1809
- **MARCHETTI et al.** *Vaccine,* 1998, vol. 16, 33-37
- **TELFORD et al.** *J. Exp. Med,* 1994, vol. 179, 1653-1658
- **EVANS et al.** *Gene,* 1995, vol. 153, 123-127
- **COSTANTINO et al.** *Vaccine,* 1992, vol. 10, 691-698
- **COSTANTINO et al.** *Vaccine,* 1999, vol. 17, 1251-1263
- **WATSON.** *Pediatr Infect Dis J,* 2000, vol. 19, 331-332
- **RUBIN.** *Pediatr Clin North Am,* 2000, vol. 47, 269-285v
- **JEDRZEJAS.** *Microbiol Mol Biol,* 2001, vol. 65, 187-207
- **BELL.** *Pediatr Infect Dis J,* 2000, vol. 19, 1187-1188
- **IWARSON.** *APMIS,* 1995, vol. 103, 321-326
- **GERLICH et al.** *Vaccine,* 1990, vol. 8, S63-6879-80
- **GUSTAFSSON et al.** *N. Engl. J. Med.,* 1996, vol. 334, 349-355
- **RAPPUOLI et al.** *TIBTECH,* 1991, vol. 9, 232-238
- Vaccines. 1988
- **DEL GUIDICE et al.** *Molecular Aspects of Medicine,* 1998, vol. 19, 1-70
- **HSU et al.** *Clin Liver Dis,* 1999, vol. 3, 901-915
- **KALMAN et al.** *Nature Genetics,* 1999, vol. 21, 385-389
- **READ et al.** *Nucleic Acids Res,* 2000, vol. 28, 1397-406
- **SHIRAI et al.** *J. Infect. Dis.,* 2000, vol. 181 (3), S524-S527
- **ROSS et al.** *Vaccine,* 2001, vol. 19, 4135-4142
- **SUTTER et al.** *Pediatr Clin North Am,* 2000, vol. 47, 287-308
- **ZIMMERMAN ; SPANN.** *Am Fam Physician,* 1999, vol. 59, 113-118125-126
- **DREESEN.** *Vaccine,* 1997, vol. 15, 2-6
- *MMWR Morb Mortal Wkly Rep,* 16 January 1998, vol. 47 (1), 12, 19
- **MCMICHAEL.** *Vaccine,* 2000, vol. 1, 101-107
- **SCHUCHAT.** *Lancet,* 1999, vol. 353 (9146), 51-6
- **DALE.** *Infect Dis Clin North Am,* 1999, vol. 13, 227-43viii
- **FERRETTI et al.** *PNAS USA,* 2001, vol. 98, 4658-4663
- **KURODA et al.** *Lancet,* 2001, vol. 357 (9264), 1225-1240
- *LANCET,* 1218-1219
- *J Toxicol Clin Toxicol,* 2001, vol. 39, 85-100
- **DEMICHELI et al.** *Vaccine,* 1998, vol. 16, 880-884
- **STEPANOV et al.** *J Biotechnol,* 1996, vol. 44, 155-160
- **INGRAM.** *Trends Neurosci,* 2001, vol. 24, 305-307
- **ROSENBERG.** *Nature,* 2001, vol. 411, 380-384
- **MOINGEON.** *Vaccine,* 2001, vol. 19, 1305-1326
- **MOLLOY et al.** *Eur J Biochem,* 2000, vol. 267 (10), 2871-81
- **MOLLOY et al.** *Electrophoresis,* 2001, vol. 22 (9), 1686-96
- **PHADKE et al.** *Proteomics,* 2001, vol. 1 (5), 705-20
- **NOUWENS et al.** *Electrophoresis,* 2000, vol. 21 (17), 3797-809
- **CHEVALLET et al.** *Electrophoresis,* 1998, vol. 19 (11), 1901-9
- **HERBERT et al.** *Electrophoresis,* 1998, vol. 19 (5), 845-51
- **DOHERTY et al.** *Electrophoresis,* 1998, vol. 19 (2), 355-63
- **WILM et al.** *Nature,* 1996, vol. 379 (6564), 466-9
- **NAKAI ; KANEHISA.** *Proteins,* vol. 11 (2), 95-110
- **NAKAI.** *Adv Protein Chem,* 2000, vol. 54, 277-344

SEQUENCE LISTING

<110> Novartis Vaccines and Diagnostics S.r.1.

<120> unexpected Meningococcus surface Proteins

<130> P054628EP

<150> PCT/IB03/06281
<151> 2003-11-17

<150> GB0226734.2
<151> 2002-11-15

<150> GB0307131.3
<151> 2003-03-27

<160> 217

<170> SeqWin99, version 1.02

<210> 1
<211> 403
<212> PRT
<213> Neisseria meningitidis

<400> 1

```
Met Ala Phe Leu Lys Leu Thr Glu Gln Asn Val Gln Gly Lys Thr Val
1               5               10              15

Leu Ile Arg Ala Asp Met Asn Val Pro Phe Lys Asp Gly Lys Ile Ser
            20              25              30

Asp Asp Thr Arg Ile Arg Ala Ser Leu Ala Ser Ile Lys Tyr Cys Val
        35              40              45

Asp Asn Gly Ala Ser Val Ile Val Met Thr His Leu Gly Arg Pro Thr
    50              55              60

Glu Gly Glu Phe His Pro Glu Asp Asp Val Ala Pro Val Ala Ala His
65              70              75              80

Leu Gly Ser Leu Leu Gly Lys Asp Val Lys Val Leu Asn Asp Trp Arg
            85              90              95

Glu Asn Lys Pro Ala Leu Asn Ala Gly Asp Val Val Met Leu Gln Asn
        100             105             110

Val Arg Ile Asn Lys Gly Glu Lys Lys Asn Asp Leu Glu Leu Gly Lys
        115             120             125

Ala Tyr Ala Ser Leu Cys Asp Val Phe Val Asn Asp Ala Phe Gly Thr
    130             135             140

Ala His Arg Ala Gln Ala Ser Thr Glu Ala Val Ala Gln Ala Ala Pro
145             150             155             160

Val Ala Cys Ala Gly Val Leu Met Ala Gly Glu Leu Asp Ala Leu Gly
            165             170             175

Lys Ala Leu Lys Gln Pro Ala Arg Pro Met Val Ala Ile Val Ala Gly
            180             185             190

Ser Lys Val Ser Thr Lys Leu Thr Ile Leu Glu Ser Leu Ala Asp Lys
        195             200             205

Val Asp Gln Leu Ile Val Gly Gly Gly Ile Ala Asn Thr Phe Leu Leu
    210             215             220

Ala Glu Gly Lys Ala Ile Gly Lys Ser Leu Ala Glu His Asp Leu Val
```

```
         225                    230                   235                    240
    Glu Glu Ser Lys Lys Ile Met Ala Lys Met Ala Ala Lys Gly Gly Ser
                    245                250                255

    Val Pro Leu Pro Thr Asp Val Val Val Ala Lys Ala Phe Ala Ala Asp
                260                265                270

    Ala Glu Ala Val Val Lys Asp Ile Ala Ala Asp Ala Glu Ala Val Val
                275                280                285

    Lys Asp Ile Ala Asp Val Ala Glu Asp Glu Met Ile Leu Asp Ile Gly
        290                295                300

    Pro Lys Ser Ala Ala Ala Leu Ala Asp Leu Leu Lys Ala Ala Gly Thr
    305                310                315                320

    Val Val Trp Asn Gly Pro Val Gly Val Phe Glu Phe Asp Gln Phe Ala
                325                330                335

    Gly Gly Thr Lys Ala Leu Ala Glu Ala Ile Ala Gln Ser Lys Ala Phe
                340                345                350

    Ser Ile Ala Gly Gly Gly Asp Thr Leu Ala Ala Ile Ala Lys Phe Gly
                355                360                365

    Val Thr Glu Gln Ile Gly Tyr Ile Ser Thr Gly Gly Gly Ala Phe Leu
        370                375                380

    Glu Phe Leu Glu Gly Lys Glu Leu Pro Ala Val Ala Ala Leu Glu Lys
    385                390                395                400

    Arg Gly Ala
```

<210> 2
<211> 170
<212> PRT
<213> Neisseria meningitidis

<400> 2

```
Met Asn Thr Leu Gln Lys Gly Phe Thr Leu Ile Glu Leu Met Ile Val
1               5               10              15

Ile Ala Ile Val Gly Ile Leu Ala Ala Val Ala Leu Pro Ala Tyr Gln
            20              25              30

Asp Tyr Thr Ala Arg Ala Gln Val Ser Glu Ala Ile Leu Leu Ala Glu
        35              40              45

Gly Gln Lys Ser Ala Val Thr Glu Tyr Tyr Leu Asn His Gly Glu Trp
    50              55              60

Pro Gly Asn Asn Thr Ser Ala Gly Val Ala Thr Ser Ser Glu Ile Lys
65              70              75              80

Gly Lys Tyr Val Lys Ser Val Glu Val Lys Asn Gly Val Val Thr Ala
            85              90              95

Gln Met Ala Ser Ser Asn Val Asn Asn Glu Ile Lys Gly Lys Lys Leu
        100             105             110

Ser Leu Trp Ala Lys Arg Gln Asn Gly Ser Val Lys Trp Phe Cys Gly
        115             120             125

Gln Pro Val Thr Arg Asp Lys Ala Lys Ala Ala Asn Asp Asp Val Thr
    130             135             140

Ala Ala Ala Ala Ala Asn Gly Lys Lys Ile Asp Thr Lys His Leu Pro
145             150             155             160

        Ser Thr Cys Arg Asp Ala Ser Asp Ala Ser
                    165             170
```

<210> 3
<211> 685
<212> PRT
<213> Neisseria meningitidis

<400> 3

```
Met Thr Arg Lys Ile Leu Val Thr Ser Ala Leu Pro Tyr Ala Asn Gly
1                5                10              15

Ser Ile His Leu Gly His Met Val Glu His Ile Gln Thr Asp Val Trp
            20              25              30

Val Arg Phe Gln Lys Leu Arg Gly His Ala Cys His Tyr Cys Cys Ala
        35              40              45

Asp Asp Thr His Gly Thr Pro Val Met Leu Ala Ala Gln Lys Gln Gly
    50              55              60

Ile Ala Pro Glu Asp Met Ile Ala Lys Val Arg Glu Glu His Leu Ala
65              70              75              80

Asp Phe Thr Gly Phe Phe Ile Gly Tyr Asp Asn Tyr Tyr Ser Thr His
            85              90              95

Ser Pro Glu Asn Lys Gln Phe Ser Gln Asp Ile Tyr Arg Ala Leu Lys
            100             105             110

Ala Asn Gly Lys Ile Glu Ser Arg Val Ile Glu Gln Leu Phe Asp Pro
        115             120             125

Glu Lys Gln Met Phe Leu Pro Asp Arg Phe Val Lys Gly Glu Cys Pro
    130             135             140

Lys Cys His Ala Gln Asp Gln Tyr Gly Asp Asn Cys Glu Val Cys Gly
145             150             155             160

Thr Thr Tyr Ser Pro Thr Glu Leu Ile Asn Pro Tyr Ser Ala Val Ser
            165             170             175

Gly Thr Lys Pro Glu Leu Arg Glu Ser Glu His Phe Phe Phe Lys Leu
        180             185             190

Gly Glu Cys Ala Asp Phe Leu Lys Ala Trp Thr Ser Gly Asn Asn Pro
        195             200             205

His Asp Gly Lys Pro His Leu Gln Ala Glu Ala Leu Asn Lys Met Lys
    210             215             220

Glu Trp Leu Gly Glu Gly Glu Glu Thr Thr Leu Ser Asp Trp Asp Ile
225             230             235             240

Ser Arg Asp Ala Pro Tyr Phe Gly Phe Glu Ile Pro Asp Ala Pro Gly
            245             250             255

Lys Tyr Phe Tyr Val Trp Leu Asp Ala Pro Val Gly Tyr Met Ala Ser
            260             265             270

Phe Lys Asn Leu Cys Asp Arg Ile Gly Val Asp Phe Asp Glu Tyr Phe
        275             280             285

Lys Ala Asp Ser Gln Thr Glu Met Tyr His Phe Ile Gly Lys Asp Ile
    290             295             300

Leu Tyr Phe His Ala Leu Phe Trp Pro Ala Met Leu His Phe Ser Gly
305             310             315             320
```

His Arg Ala Pro Thr Gly Val Tyr Ala His Gly Phe Leu Thr Val Asp
325                     330                     335

Gly Gln Lys Met Ser Lys Ser Arg Gly Thr Phe Ile Thr Ala Lys Ser
340                     345                     350

Tyr Leu Glu Gln Gly Leu Asn Pro Glu Trp Met Arg Tyr Tyr Ile Ala
355                     360                     365

Ala Lys Leu Asn Ser Lys Ile Glu Asp Ile Asp Leu Asn Leu Gln Asp
370                     375                     380

Phe Ile Ser Arg Val Asn Ser Asp Leu Val Gly Lys Tyr Val Asn Ile
385                     390                     395                     400

Ala Ala Arg Ala Ser Gly Phe Ile Ala Lys Arg Phe Glu Gly Arg Leu
405                     410                     415

Lys Asp Val Ala Asp Ser Glu Leu Leu Ala Lys Leu Thr Ala Gln Ser
420                     425                     430

Glu Ala Ile Ala Glu Cys Tyr Glu Ser Arg Glu Tyr Ala Lys Ala Leu
435                     440                     445

Arg Asp Ile Met Ala Leu Ala Asp Ile Val Asn Glu Tyr Val Asp Ala
450                     455                     460

Asn Lys Pro Trp Glu Leu Ala Lys Gln Glu Gly Gln Asp Glu Arg Leu
465                     470                     475                     480

His Glu Val Cys Ser Glu Leu Ile Asn Ala Phe Thr Met Leu Thr Ala
485                     490                     495

Tyr Leu Ala Pro Val Leu Pro Gln Thr Ala Ala Asn Ala Ala Lys Phe
500                     505                     510

Leu Asn Leu Glu Ala Ile Thr Trp Ala Asn Thr Arg Asp Thr Leu Gly
515                     520                     525

Lys His Ala Ile Asn Lys Tyr Glu His Leu Met Gln Arg Val Glu Gln
530                     535                     540

Lys Gln Val Asp Asp Leu Ile Glu Ala Asn Lys Gln Ser Ile Ala Ala
545                     550                     555                     560

Ala Ala Ala Pro Ala Ala Glu Glu Gly Lys Tyr Glu Lys Val Ala Glu
565                     570                     575

Gln Ala Ser Phe Asp Asp Phe Met Lys Ile Asp Met Arg Val Ala Lys
580                     585                     590

Val Leu Asn Cys Glu Ala Val Glu Gly Ser Thr Lys Leu Leu Lys Phe
595                     600                     605

Asp Leu Asp Phe Gly Phe Glu Lys Arg Ile Ile Phe Ser Gly Ile Ala
610                     615                     620

Ala Ser Tyr Pro Asn Pro Ala Glu Leu Asn Gly Arg Met Val Ile Ala
625                     630                     635                     640

Val Ala Asn Phe Ala Pro Arg Lys Met Ala Lys Phe Gly Val Ser Glu
645                     650                     655

Gly Met Ile Leu Ser Ala Ala Thr Ala Asp Gly Lys Leu Lys Leu Leu
660                     665                     670

Asp Val Asp Thr Gly Ala Gln Pro Gly Asp Lys Val Gly
675                     680                     685

45

<210> 4
<211> 456
<212> PRT
<213> Neisseria meningitidis

<400> 4

```
Met Pro Gln Asn Thr Leu Asn Ile Val Ile Leu Ala Ala Gly Lys Gly
1               5               10                      15

Thr Arg Met Tyr Ser Lys Met Pro Lys Val Leu His Arg Ile Gly Gly
            20              25                      30

Lys Pro Met Val Gly Arg Val Ile Asp Thr Ala Ala Ala Leu Asn Pro
        35                  40              45

Gln Asn Ile Cys Val Val Ile Gly His Gly Lys Glu Gln Val Leu Asp
    50                  55              60

Thr Val Lys Arg Asp Val Val Trp Val Glu Gln Thr Glu Gln Leu Gly
65              70              75                      80

Thr Gly His Ala Val Lys Thr Ala Leu Pro His Leu Ser Ala Glu Gly
                85              90                      95

Arg Thr Leu Val Leu Tyr Gly Asp Val Pro Leu Ile Asp Val Glu Thr
        100             105                 110

Leu Glu Thr Leu Leu Glu Ala Ala Gly Asn Glu Val Gly Leu Leu Thr
        115             120                 125

Asp Val Pro Asn Asp Pro Thr Gly Leu Gly Arg Ile Ile Arg Asp Ser
    130             135             140

Asn Gly Ser Val Thr Ala Ile Val Glu Glu Lys Asp Ala Asp Ala Val
145             150             155                 160

Gln Lys Ala Val Lys Glu Ile Asn Thr Gly Ile Leu Val Leu Pro Asn
            165             170                 175

Ala Lys Leu Glu Asn Trp Leu Asn Ser Leu Ser Ser Asn Asn Ala Gln
        180             185             190

Gly Glu Tyr Tyr Leu Thr Asp Leu Ile Ala Lys Ala Val Ala Asp Gly
        195             200             205

Ile Lys Val His Pro Val Gln Val Arg Ala Ser His Leu Ala Ala Gly
    210             215             220

Val Asn Asn Lys Leu Gln Leu Thr Glu Leu Glu Arg Ile Phe Gln Thr
225             230             235                 240

Glu Gln Ala Gln Glu Leu Leu Lys Ala Gly Val Thr Leu Arg Asp Pro
            245             250                 255

Ala Arg Phe Asp Leu Arg Gly Arg Leu Lys His Gly Gln Asp Val Val
        260             265                 270

Ile Asp Val Asn Cys Ile Phe Glu Gly Asp Ile Glu Leu Gly Asp Asn
    275             280                 285

Val Glu Ile Gly Ala Asn Cys Val Ile Lys Asn Ala Lys Ile Gly Ala
    290             295             300

Asn Ser Lys Ile Ala Pro Phe Ser His Leu Glu Ser Cys Glu Val Gly
305             310             315                 320

Glu Asn Asn Arg Ile Gly Pro Tyr Ala Arg Leu Arg Pro Gln Ala Arg
            325             330                 335

Leu Ala Asp Asp Val His Val Gly Asn Phe Val Glu Ile Lys Asn Ala
        340             345                 350
```

```
Ala Ile Gly Lys Gly Thr Lys Ala Asn His Leu Thr Tyr Ile Gly Asp
        355             360                 365

Ala Glu Val Gly Cys Lys Thr Asn Phe Gly Ala Gly Thr Ile Ile Ala
        370             375                 380

Asn Tyr Asp Gly Val His Lys His Lys Thr Val Ile Gly Asp Glu Val
385                 390                 395                 400

Arg Ile Gly Ser Asn Cys Val Leu Val Ala Pro Val Thr Leu Gly Asn
                405                 410                 415

Lys Val Thr Thr Gly Ala Gly Ser Thr Ile Thr Arg Asn Val Glu Asp
            420                 425                 430

Asn Lys Leu Ala Leu Ala Arg Ala Arg Gln Thr Val Ile Glu Gly Trp
        435                 440                 445

Val Arg Pro Glu Lys Asp Lys Gln
    450                 455
```

<210> 5
<211> 421
<212> PRT
<213> Neisseria meningitidis

<400> 5

```
Met Phe Ser Phe Phe Arg Arg Lys Lys Lys Gln Glu Thr Pro Ala Leu
1               5                   10              15

Glu Glu Ala Gln Ile Gln Glu Thr Ala Ala Lys Ala Glu Ser Glu Leu
            20                  25              30

Ala Gln Ile Val Glu Asn Ile Lys Glu Asp Ala Glu Ser Leu Ala Glu
        35                  40              45

Ser Val Lys Gly Gln Val Glu Ser Ala Val Glu Thr Val Ser Gly Ala
    50              55              60

Val Glu Gln Val Lys Glu Thr Val Ala Glu Met Leu Ser Glu Ala Glu
65              70              75              80

Glu Ala Ala Glu Lys Ala Ala Glu Gln Val Glu Ala Ala Lys Glu Ala
            85                  90                  95

Val Ala Glu Thr Val Gly Glu Ala Val Gly Gln Val Gln Glu Ala Val
            100             105             110

Ala Thr Thr Glu Glu His Lys Leu Gly Trp Ala Ala Arg Leu Lys Gln
        115             120             125

Gly Leu Thr Lys Ser Arg Asp Lys Met Ala Lys Ser Leu Ala Gly Val
    130             135             140

Phe Gly Gly Gly Gln Ile Asp Glu Asp Leu Tyr Glu Glu Leu Glu Thr
145             150             155             160

Val Leu Ile Thr Ser Asp Met Gly Met Glu Ala Thr Glu Tyr Leu Met
            165             170             175

Lys Asp Val Arg Asp Arg Val Ser Leu Lys Gly Leu Lys Asp Gly Asn
            180             185             190

Glu Leu Arg Gly Ala Leu Lys Glu Ala Leu Tyr Asp Leu Ile Lys Pro
        195             200             205

Leu Glu Lys Pro Leu Val Leu Pro Glu Thr Lys Glu Pro Phe Val Ile
    210             215             220
```

```
Met Leu Ala Gly Ile Asn Gly Ala Gly Lys Thr Thr Ser Ile Gly Lys
225                 230             235                 240

Leu Ala Lys Tyr Phe Gln Ala Gln Gly Lys Ser Val Leu Leu Ala Ala
            245             250                 255

Gly Asp Thr Phe Arg Ala Ala Ala Arg Glu Gln Leu Gln Ala Trp Gly
            260             265             270

Glu Arg Asn Asn Val Thr Val Ile Ser Gln Thr Thr Gly Asp Ser Ala
        275             280             285

Ala Val Cys Phe Asp Ala Val Gln Ala Ala Lys Ala Arg Gly Ile Asp
    290             295             300

Ile Val Leu Ala Asp Thr Ala Gly Arg Leu Pro Thr Gln Leu His Leu
305             310             315             320

Met Glu Glu Ile Lys Lys Val Lys Arg Val Leu Gln Lys Ala Met Pro
            325             330             335

Asp Ala Pro His Glu Ile Ile Val Val Leu Asp Ala Asn Ile Gly Gln
            340             345             350

Asn Ala Val Asn Gln Val Lys Ala Phe Asp Asp Ala Leu Gly Leu Thr
    355             360             365

Gly Leu Ile Val Thr Lys Leu Asp Gly Thr Ala Lys Gly Gly Ile Leu
370             375             380

Ala Ala Leu Ala Ser Asp Arg Pro Val Pro Val Arg Tyr Ile Gly Val
385             390             395             400

Gly Glu Gly Ile Asp Asp Leu Arg Pro Phe Asp Ala Arg Ala Phe Val
            405             410             415

Asp Ala Leu Leu Asp
            420
```

<210> 6
<211> 408
<212> PRT
<213> Neisseria meningitidis

<400> 6

```
Met Asn Thr Asp Asn Leu His Asp Ile Leu Asp Glu Met Val Gln Val
1               5                  10              .       15

Tyr Ser Gln Lys Lys Gln Ser Arg Ser Glu Thr Pro Ala Glu Ile Gly
            20              25                  30

Ala His Phe His Pro Leu Leu Asp Arg Leu Cys Glu Thr Ala Glu Ala
        35                  40                  45

Gln Asn Ala Ser Asp Ile Leu Ile Ser Lys Gly Phe Pro Pro Ser Leu
    50                  55                  60

Lys Ile Asn Ser Ala Leu Thr Pro Gln Pro Gln Lys Ala Leu Thr Gly
65                  70                  75                  80

Glu Glu Thr Ala Ala Ile Ala Ala Ser Thr Met Asn Ala Glu Gln Ser
            85                  90                  95

Glu Ile Phe Arg Arg Asp Gly Glu Ile Asn Tyr Ser Val Gln Ser Arg
        100                 105                 110

Ser Gly Thr Arg Tyr Arg Ala Asn Ala Tyr His Ser Gln Gly Ser Ala
        115                 120                 125

Gly Leu Val Leu Arg Arg Ile Asn His Val Ile Pro Gln Met Gln Glu
```

```
                130                     135                        140

      Leu Gly Leu Pro Glu Lys Leu Lys Asp Leu Ala Val Ala Pro Arg Gly
      145             150             155                     160

      Leu Leu Ile Ile Val Gly Pro Thr Gly Ser Gly Lys Ser Thr Thr Met
                  165             170                     175

      Ala Thr Met Leu Glu His Arg Asn Lys Thr Leu Pro Ser His Ile Val
                  180             185             190

      Thr Ile Glu Asp Pro Ile Glu Phe Ile Tyr Lys Pro Arg Arg Cys Ile
              195             200             205

      Phe Thr Gln Arg Glu Ile Gly Val Asp Thr Ile Asn Trp Gln Thr Ala
          210             215             220

      Val Gln Asn Ala Met Arg Gln Ser Pro Asp Val Val Cys Ile Gly Glu
      225             230             235             240

      Val Arg Ser Arg Glu Ser Met Glu Tyr Ala Met Gln Leu Ala Gln Thr
                  245             250             255

      Gly His Leu Cys Ile Phe Thr Leu His Ala Asn Thr Ala Pro Gln Ser
                  260             265             270

      Leu Glu Arg Ile Leu Asn Phe Tyr Pro Lys Glu Gln His Asn Gln Ile
              275             280             285

      Leu Ile Asp Ile Ala Leu Asn Leu Thr Gly Ile Ile Cys Gln Arg Leu
          290             295             300

      Ala Leu Lys Gln Asp Lys Thr Gly Arg Thr Ala Val Val Asp Leu Leu
      305             310             315             320

      Ile Asn Thr Pro Ala Ile Gln Asp Phe Ile Leu Lys Gly Asp Leu Met
                  325             330             335

      Asn Ile Ser Lys Ile Met Glu Thr Ala Lys Thr Asp Gly Met Gln Thr
              340             345             350

      Met Asp Gln Asn Leu Phe Glu Leu Tyr Arg His Gly Ile Ile Ser Tyr
              355             360             365

      Glu Glu Ala Leu Arg Gln Ser Val Ser Ala Asn Asn Leu Arg Leu His
          370             375             380

      Ile Gln Leu His Lys Glu Gly Lys Thr Pro Glu Leu Leu Tyr Asp Arg
      385             390             395             400

      Val Asn Gly Leu Asn Leu Ile Ser
                  405
```

<210> 7
<211> 347
<212> PRT
<213> Neisseria meningitidis

<400> 7

```
Met Gln Ile Thr Asp Leu Leu Ala Phe Gly Ala Lys Asn Lys Ala Ser
1               5               10              15

Asp Leu His Leu Ser Ser Gly Ile Ser Pro Met Ile Arg Val His Gly
            20              25              30

Asp Met Arg Arg Ile Asn Leu Pro Glu Met Ser Ala Glu Glu Val Gly
        35              40              45

Asn Met Val Thr Ser Val Met Asn Asp His Gln Arg Lys Ile Tyr Gln
        50              55              60

Gln Asn Leu Glu Val Asp Phe Ser Phe Glu Leu Pro Asn Val Ala Arg
65              70              75              80

Phe Arg Val Asn Ala Phe Asn Ile Gly Arg Gly Pro Ala Ala Val Phe
            85              90              95

Arg Thr Ile Pro Ser Thr Val Leu Ser Leu Glu Glu Leu Lys Ala Pro
        100             105             110

Ser Ile Phe Gln Lys Ile Ala Glu Ser Pro Arg Gly Met Val Leu Val
        115             120             125

Thr Gly Pro Thr Gly Ser Gly Lys Ser Thr Thr Leu Ala Ala Met Ile
    130             135             140

Asn Tyr Ile Asn Glu Thr Gln Pro Ala His Ile Leu Thr Ile Glu Asp
145             150             155             160

Pro Ile Glu Phe Val His Gln Ser Lys Lys Ser Leu Ile Asn Gln Arg
            165             170             175

Glu Leu His Gln His Thr Leu Ser Phe Ala Asn Ala Leu Arg Ser Ala
        180             185             190

Leu Arg Glu Asp Pro Asp Val Ile Leu Val Gly Glu Met Arg Asp Pro
        195             200             205

Glu Thr Ile Gly Leu Ala Leu Thr Ala Ala Glu Thr Gly His Leu Val
    210             215             220

Phe Gly Thr Leu His Thr Thr Gly Ala Ala Lys Thr Val Asp Arg Ile
225             230             235             240

Val Asp Val Phe Pro Ala Gly Glu Lys Glu Met Val Arg Ser Met Leu
            245             250             255

Ser Glu Ser Leu Thr Ala Val Ile Ser Gln Asn Leu Leu Lys Thr His
        260             265             270

Asp Gly Asn Gly Arg Val Ala Ser His Glu Ile Leu Ile Ala Asn Pro
        275             280             285

Ala Val Arg Asn Leu Ile Arg Glu Asn Lys Ile Thr Gln Ile Asn Ser
    290             295             300

Val Leu Gln Thr Gly Gln Ala Ser Gly Met Gln Thr Met Asp Gln Ser
305             310             315             320

Leu Gln Ser Leu Val Arg Gln Gly Leu Ile Ala Pro Glu Val Ala Arg
            325             330             335

Arg Arg Ala Gln Asn Ser Glu Ser Met Ser Phe
        340             345
```

<210> 8

<211> 349
<212> PRT
<213> Neisseria meningitidis

<400> 8

```
Met Gln Asn Asn Asn Glu Phe Lys Ile Gly Asn Arg Ser Val Gly Tyr
1                   5                   10                  15

Asn His Glu Pro Leu Ile Ile Cys Glu Ile Gly Ile Asn His Glu Gly
            20                  25                  30

Ser Leu Lys Thr Ala Phe Glu Met Val Asp Ala Ala Tyr Asn Ala Gly
            35                  40                  45
```

```
Ala Glu Val Val Lys His Gln Thr His Ile Val Glu Asp Glu Met Ser
    50              55              60
Asp Glu Ala Lys Gln Val Ile Pro Gly Asn Ala Asp Val Ser Ile Tyr
65              70              75              80
Glu Ile Met Glu Arg Cys Ala Leu Asn Glu Glu Asp Glu Ile Lys Leu
            85              90              95
Lys Glu Tyr Val Glu Ser Lys Gly Met Ile Phe Ile Ser Thr Pro Phe
            100             105             110
Ser Arg Ala Ala Ala Leu Arg Leu Gln Arg Met Asp Ile Pro Ala Tyr
        115             120             125
Lys Ile Gly Ser Gly Glu Cys Asn Asn Tyr Pro Leu Ile Lys Leu Val
    130             135             140
Ala Ser Phe Gly Lys Pro Ile Ile Leu Ser Thr Gly Met Asn Ser Ile
145             150             155             160
Glu Ser Ile Lys Lys Ser Val Glu Ile Ile Arg Glu Ala Gly Val Pro
            165             170             175
Tyr Ala Leu Leu His Cys Thr Asn Ile Tyr Pro Thr Pro Tyr Glu Asp
        180             185             190
Val Arg Leu Gly Gly Met Asn Asp Leu Ser Glu Ala Phe Pro Asp Ala
    195             200             205
Ile Ile Gly Leu Ser Asp His Thr Leu Asp Asn Tyr Ala Cys Leu Gly
    210             215             220
Ala Val Ala Leu Gly Gly Ser Ile Leu Glu Arg His Phe Thr Asp Arg
225             230             235             240
Met Asp Arg Pro Gly Pro Asp Ile Val Cys Ser Met Asn Pro Asp Thr
            245             250             255
Phe Lys Glu Leu Lys Gln Gly Ala His Ala Leu Lys Leu Ala Arg Gly
            260             265             270
Gly Lys Lys Asp Thr Ile Ile Ala Gly Glu Lys Pro Thr Lys Asp Phe
        275             280             285
Ala Phe Ala Ser Val Val Ala Asp Lys Asp Ile Lys Lys Gly Glu Leu
    290             295             300
Leu Ser Gly Asp Asn Leu Trp Val Lys Arg Pro Gly Asn Gly Asp Phe
305             310             315             320
Ser Val Asn Glu Tyr Glu Thr Leu Phe Gly Lys Val Ala Ala Cys Asn
            325             330             335
Ile Arg Lys Gly Ala Gln Ile Lys Lys Thr Asp Ile Glu
            340             345
```

<210> 9
<211> 490
<212> PRT
<213> Neisseria meningitidis

<400> 9

55

```
Met Asn Gln Thr Ser Arg Asp Leu Thr Arg Ile Ser His Asn Thr Lys
1               5                   10                  15

Ile Val Ala Thr Leu Gly Pro Gly Ser Asn Asn Val Glu Leu Leu Glu
              20                  25                  30

Asp Met Ile Arg Val Gly Gly Leu Asn Val Val Arg Phe Asn Phe Ser
```

```
                 35                      40                      45

      His Gly Thr Pro Glu Phe His Gln Glu Asn Ala Leu Ile Val Arg Glu
          50              55              60

      Ala Ala Lys Arg Ala Gly Gln Glu Ile Ala Ile Ile Ala Asp Leu Gln
      65              70              75              80

      Gly Pro Lys Ile Arg Val Gly Lys Ile Ala Gly Gly Gly Ile Glu Leu
                  85              90              95

      Asn Lys Gly Glu Thr Leu Val Leu Asp Ala Ala Leu Glu Gly Glu Gly
              100             105             110

      Thr Arg Glu Ala Val Gly Leu Asp Tyr Arg Asp Leu Pro Asp Asp Val
              115             120             125

      Ala Ala Gly Asp Val Leu Trp Leu Asp Asp Gly Leu Leu Thr Leu Thr
          130             135             140

      Val Glu Ser Val Glu Gly Ser Arg Ile Ile Thr Arg Val Glu Asn Ser
      145             150             155             160

      His Val Leu Lys Ser Asn Lys Gly Ile Asn Lys Arg Gly Gly Gly Leu
                  165             170             175

      Ser Ala Gly Ala Leu Thr Glu Lys Asp Phe Arg Asp Leu Lys Thr Ala
              180             185             190

      Ile Ala Ile Gly Cys Asp Tyr Leu Ala Ile Ser Phe Val Lys Ser Ala
              195             200             205

      Glu Asp Leu His Ile Ala Arg Ala Lys Val Glu Glu Glu Met Lys Gly
          210             215             220

      Ser Thr Ala Val Arg Pro Gly Leu Val Ser Lys Ile Glu Arg Val Glu
      225             230             235             240

      Ala Ile Glu Asn Leu Asp Glu Ile Ile Leu Ala Gly Asp Gly Ile Met
                  245             250             255

      Val Ala Arg Gly Asp Leu Ala Val Glu Val Gly His Ala Ala Val Pro
              260             265             270

      Ala Leu Gln Lys Arg Met Ile Arg Arg Ala Arg Glu Leu Arg Arg Phe
              275             280             285

      Ser Ile Thr Ala Thr Gln Met Met Glu Ser Met Ile Thr Asn Pro Val
          290             295             300

      Pro Thr Arg Ala Glu Val Ser Asp Val Ala Asn Ala Val Leu Asp Gly
      305             310             315             320

      Thr Asp Ala Val Met Cys Ser Ala Glu Thr Ala Val Gly Ala Tyr Pro
                  325             330             335

      Phe Glu Thr Val Ser Gln Met Ala Ile Ile Cys Ala Ala Ala Glu Lys
              340             345             350

      Glu Gln Asp Ser Leu Asn Gly Val Ala Glu Gln Val Glu Tyr Pro Glu
              355             360             365

      Ala Val Ser Thr Asn Leu Ala Val Ala Gly Gly Ala Val Ser Val Ala
          370             375             380

      Arg Ala Val His Ala Lys Ala Ile Val Ala Leu Thr Glu Ser Gly Ser
      385             390             395             400

      Thr Ala Phe Glu Ile Ser Arg His Asn Ile Thr Leu Pro Ile Phe Ala
                  405             410             415
```

```
Leu Thr Pro Ser Val Ser Ala Gln Arg Arg Met Ala Met Tyr Arg Gly
            420             425             430

Val Arg Pro Leu Ile Leu Ala Thr Ser Thr Asp His Asp Thr Ala Leu
            435             440             445

Asn Glu Val Glu Thr Met Leu Val Glu His Asn Ile Leu His Ser Gly
    450             455             460

Asp Gln Tyr Ile Ile Thr Ser Gly Ser Gln Met Arg Glu Ser Gly Ser
465             470             475             480

Thr Asn Thr Leu Glu Val Leu Arg Val Lys
            485             490
```

<210> 10
<211> 443
<212> PRT
<213> Neisseria meningitidis

<400> 10

```
Met Leu Lys Cys Gly Thr Phe Phe Ile Thr Arg His Ile Pro Arg Gly
1             5                 10               15

Cys Arg Arg Phe Phe Gln Pro Asn Gln Ala Arg Gln Thr Glu Ile Tyr
            20              25              30

Gln Ile Arg Gly Thr Val Met Gln Arg Arg Ile Ile Thr Leu Leu Cys
        35              40              45

Ala Ala Gly Met Ala Phe Ser Thr Gln Thr Leu Ala Ala Asn Leu Glu
    50              55              60

Val Arg Pro Asn Ala Pro Glu Arg Tyr Thr Val Lys Gln Gly Asp Thr
65              70              75              80

Leu Trp Gly Ile Ser Gly Lys Tyr Leu Tyr Ser Pro Trp Gln Trp Gly
                85              90              95

Arg Leu Trp Asp Ala Asn Arg Asp Gln Ile His Asn Pro Asp Leu Ile
            100             105             110

Tyr Pro Asp Gln Val Leu Val Leu Arg His Val Asp Gly Glu Pro Arg
        115             120             125

Leu Gly Leu Glu Gln Thr Asp Gly Ile Pro Val Val Lys Met Ser Pro
    130             135             140

Asp Lys Glu Val Ser Gly Tyr Gly Ile Pro Ala Ile Asp Val Asn Phe
145             150             155             160

Tyr Arg Ile Phe Met Arg His Pro Gln Ile Val Ser Arg Lys Glu Thr
            165             170             175

Ala Ala Ala Pro Arg Leu Leu Ser Gly Pro Glu Gly Arg Leu Leu Tyr
        180             185             190

Thr Lys Gly Thr Arg Val Tyr Thr Lys Gly Leu Lys Glu Pro Gly Arg
        195             200             205

Tyr Leu Thr Tyr Arg Ile Asn Lys Asn Ile Thr Asp Pro Asp Thr Gly
    210             215             220

Lys Phe Leu Gly Gln Glu Val Ala Phe Ser Gly Ile Val Arg Ser Leu
225             230             235             240

Asp Tyr Thr Asp Ser Val Leu Glu Gln Arg Ser Lys Gln Ala Gly Glu
            245             250             255
```

59

```
Arg Pro Lys Asp Asn Glu Tyr His Thr Arg Thr His Pro Leu Ile Thr
            260                 265             270

Pro Leu Arg Thr Pro Ser Ile Gln Pro Leu Val Val Glu Thr Ala Ile
            275                 280             285

Ser Glu Ile Gln Gln Gly Asp Tyr Leu Met Lys Met Pro Glu Asp Thr
    290                 295             300

Asp Arg Phe Asn Met Met Pro His Glu Pro Ser Arg Pro Val Gln Ala
305                 310             315                 320

Lys Ile Val Ser Val Phe Glu Gly Thr Arg Ile Ala Gly Gln Phe Gln
            325                 330                 335

Thr Ile Thr Ile Asp Lys Gly Glu Ala Asp Gly Leu Asp Lys Gly Thr
            340                 345             350

Val Leu Ser Leu Tyr Lys Arg Lys Lys Thr Met Gln Val Asp Leu Ser
            355                 360             365

Asn Asn Phe Lys Ser Arg Asp Thr Val Glu Leu Ile Ser Thr Pro Ala
    370                 375             380

Glu Glu Val Gly Leu Ala Met Val Tyr Arg Thr Ser Glu His Leu Ser
385                 390             395                 400

Ser Ala Ile Ile Leu Glu Asn Ile Ser Asp Ile Ser Val Gly Asp Thr
            405                 410                 415

Ala Ala Asn Pro Gly Arg Asp Leu Asp Asn Ile Pro Asp Gln Gly Arg
            420                 425             430

Ser Arg Val Lys Phe Gly Phe Asn Arg Ser Glu
            435                 440
```

<210> 11
<211> 425
<212> PRT
<213> Neisseria meningitidis

<400> 11

```
Met Arg Ser Ser Asp Ile Leu Ile Val Asp Asp Glu Ile Gly Ile Arg
1               5               10              15

Asp Leu Leu Ser Glu Ile Leu Gln Asp Glu Gly Tyr Ser Val Ala Leu
            20              25              30

Ala Glu Asn Ala Glu Glu Ala Arg Lys Leu Arg His Gln Ala Arg Pro
        35              40              45

Ala Met Val Leu Leu Asp Ile Trp Met Pro Asp Cys Asp Gly Ile Thr
    50              55              60

Leu Leu Lys Glu Trp Ala Lys Asn Gly Gln Leu Asn Met Pro Val Val
65              70              75              80

Met Met Ser Gly His Ala Ser Ile Asp Thr Ala Val Glu Ala Thr Lys
            85              90              95

Ile Gly Ala Ile Asp Phe Leu Glu Lys Pro Ile Ser Leu Gln Lys Leu
            100             105             110

Leu Ser Ala Val Glu Asn Ala Leu Lys Tyr Gly Ala Ala Gln Thr Glu
            115             120             125

Thr Gly Pro Val Phe Asp Lys Leu Gly Asn Ser Ala Ala Ile Gln Glu
    130             135             140

Met Asn Arg Glu Val Gly Ala Ala Val Lys Cys Ala Ser Pro Val Leu
```

```
        145                      150                      155                      160
    Leu Thr Gly Glu Ala Gly Ser Pro Phe Glu Thr Val Ala Arg Tyr Phe
                    165             170                 175

    His Lys Asn Gly Thr Pro Trp Val Ser Pro Ala Arg Val Glu Tyr Leu
                180             185                 190

    Ile Asp Met Pro Met Glu Leu Leu Gln Lys Ala Glu Gly Gly Val Leu
            195             200                 205

    Tyr Val Gly Asp Ile Ala Gln Tyr Ser Arg Asn Ile Gln Ala Gly Ile
        210             215                 220

    Ala Phe Ile Val Gly Lys Ala Glu His Arg Arg Val Arg Val Val Ala
    225             230                 235                 240

    Ser Gly Ser Arg Ala Ala Gly Ser Asp Gly Ile Ala Cys Glu Glu Lys
                245             250                 255

    Leu Ala Glu Leu Leu Ser Glu Ser Val Val Arg Ile Pro Pro Leu Arg
                260             265                 270

    Met Gln His Glu Asp Ile Pro Phe Leu Ile Gln Gly Ile Ala Cys Asn
            275             280                 285

    Val Ala Glu Ser Gln Lys Ile Ala Pro Ala Ser Phe Ser Glu Glu Ala
            290             295                 300

    Leu Ala Ala Leu Thr Arg Tyr Asp Trp Pro Gly Asn Phe Asp Gln Leu
    305             310                 315                 320

    Gln Ser Val Val Ala Thr Leu Leu Leu Glu Ala Asp Gly Gln Glu Ile
                325             330                 335

    Gly Ala Gly Ala Val Ser Ser Leu Leu Gly Gln Asn Val Pro Ala Glu
                340             345                 350

    Gly Ala Glu Asp Met Val Gly Gly Phe Asn Phe Asn Leu Pro Leu Arg
                355             360                 365

    Glu Leu Arg Glu Glu Val Glu Arg Arg Tyr Phe Glu Tyr His Ile Ala
            370             375                 380

    Gln Glu Gly Gln Asn Met Ser Gln Val Ala Gln Lys Val Gly Leu Glu
    385             390                 395                 400

    Arg Thr His Leu Tyr Arg Lys Leu Lys Gln Leu Gly Ile Gly Val Ser
                405             410                 415

    Arg Arg Ala Gly Glu Lys Thr Glu Glu
                420             425
```

<210> 12
<211> 394
<212> PRT
<213> Neisseria meningitidis

<400> 12

```
Met Ala Lys Glu Lys Phe Glu Arg Ser Lys Pro His Val Asn Val Gly
1               5                   10                  15

Thr Ile Gly His Val Asp His Gly Lys Thr Thr Leu Thr Ala Ala Leu
                20                  25                  30

Thr Thr Ile Leu Ala Lys Lys Phe Gly Gly Ala Ala Lys Ala Tyr Asp
            35                  40                  45

Gln Ile Asp Asn Ala Pro Glu Glu Lys Ala Arg Gly Ile Thr Ile Asn
            50                  55                  60
```

```
Thr Ser His Val Glu Tyr Glu Thr Glu Thr Arg His Tyr Ala His Val
65              70              75                      80

Asp Cys Pro Gly His Ala Asp Tyr Val Lys Asn Met Ile Thr Gly Ala
            85              90                      95

Ala Gln Met Asp Gly Ala Ile Leu Val Cys Ser Ala Ala Asp Gly Pro
            100             105                     110

Met Pro Gln Thr Arg Glu His Ile Leu Leu Ala Arg Gln Val Gly Val
            115             120                     125

Pro Tyr Ile Ile Val Phe Met Asn Lys Cys Asp Met Val Asp Asp Ala
    130             135                 140

Glu Leu Leu Glu Leu Val Glu Met Glu Ile Arg Asp Leu Leu Ser Ser
145             150                 155                     160

Tyr Asp Phe Pro Gly Asp Asp Cys Pro Ile Val Gln Gly Ser Ala Leu
            165             170                     175

Lys Ala Leu Glu Gly Asp Ala Ala Tyr Glu Glu Lys Ile Phe Glu Leu
            180             185                 190

Ala Ala Ala Leu Asp Ser Tyr Ile Pro Thr Pro Glu Arg Ala Val Asp
        195             200             205

Lys Pro Phe Leu Leu Pro Ile Glu Asp Val Phe Ser Ile Ser Gly Arg
    210             215             220

Gly Thr Val Val Thr Gly Arg Val Glu Arg Gly Ile Ile His Val Gly
225             230             235                     240

Asp Glu Ile Glu Ile Val Gly Leu Lys Glu Thr Gln Lys Thr Thr Cys
            245             250                     255

Thr Gly Val Glu Met Phe Arg Lys Leu Leu Asp Glu Gly Gln Ala Gly
            260             265                 270

Asp Asn Val Gly Val Leu Leu Arg Gly Thr Lys Arg Glu Asp Val Glu
        275             280             285

Arg Gly Gln Val Leu Ala Lys Pro Gly Thr Ile Thr Pro His Thr Lys
    290             295             300

Phe Lys Ala Glu Val Tyr Val Leu Ser Lys Glu Glu Gly Gly Arg His
305             310             315                     320

Thr Pro Phe Phe Ala Asn Tyr Arg Pro Gln Phe Tyr Phe Arg Thr Thr
            325             330                     335

Asp Val Thr Gly Ala Val Thr Leu Glu Glu Gly Val Glu Met Val Met
            340             345                 350

Pro Gly Glu Asn Val Thr Ile Thr Val Glu Leu Ile Ala Pro Ile Ala
        355             360             365

Met Glu Glu Gly Leu Arg Phe Ala Ile Arg Glu Gly Gly Arg Thr Val
    370             375             380

Gly Ala Gly Val Val Ser Ser Val Ile Ala
385             390
```

<210> 13

<211> 178

<212> PRT

<213> Neisseria meningitidis

<400> 13

```
Met Ser Lys Lys Trp Tyr Val Val Gln Ala Tyr Ser Gly Phe Glu Lys
1               5               10                      15

Asn Val Gln Arg Ile Leu Glu Glu Arg Ile Ala Arg Glu Glu Met Gly
            20              25                      30

Asp Tyr Phe Gly Gln Ile Leu Val Pro Val Glu Lys Val Val Asp Ile
        35              40                  45

Arg Asn Gly Arg Lys Thr Ile Ser Glu Arg Lys Ser Tyr Pro Gly Tyr
    50                  55                  60

Val Leu Val Glu Met Glu Met Thr Asp Asp Ser Trp His Leu Val Lys
65              70                  75          .           80

Ser Thr Pro Arg Val Ser Gly Phe Ile Gly Gly Arg Ala Asn Arg Pro
                85              90                  95

Thr Pro Ile Ser Gln Arg Glu Ala Glu Ile Ile Leu Gln Gln Val Gln
            100             105                 110

Thr Gly Ile Glu Lys Pro Lys Pro Lys Val Glu Phe Glu Val Gly Gln
            115             120                 125

Gln Val Arg Val Asn Glu Gly Pro Phe Ala Asp Phe Asn Gly Val Val
    130             135                 140

Glu Glu Val Asn Tyr Glu Arg Asn Lys Leu Arg Val Ser Val Gln Ile
145             150                 155                 160

Phe Gly Arg Glu Thr Pro Val Glu Leu Glu Phe Ser Gln Val Glu Lys
                165             170                 175

Ile Asn
```

<210> 14
<211> 231
<212> PRT
<213> Neisseria meningitidis

<400> 14

```
Met Ala Lys Val Ser Lys Arg Leu Lys Ala Leu Arg Ser Ser Val Glu
1               5                   10                  15

Ala Asn Lys Leu Tyr Ala Ile Asp Glu Ala Ile Ala Leu Val Lys Lys
            20                  25                  30

Ala Ala Thr Ala Lys Phe Asp Glu Ser Val Asp Val Ser Phe Asn Leu
        35                  40                  45

Gly Val Asp Pro Arg Lys Ser Asp Gln Val Ile Arg Gly Ser Val Val
    50                  55                  60

Leu Pro Lys Gly Thr Gly Lys Ile Thr Arg Val Ala Val Phe Thr Gln
65              70                  75                      80

Gly Ala Asn Ala Glu Ala Ala Lys Glu Ala Gly Ala Asp Ile Val Gly
            85                  90                  95

Phe Glu Asp Leu Ala Ala Glu Ile Lys Ala Gly Asn Leu Asn Phe Asp
            100                 105                 110

Val Val Ile Ala Ser Pro Asp Ala Met Arg Ile Val Gly Gln Leu Gly
        115                 120                 125

Thr Ile Leu Gly Pro Arg Gly Leu Met Pro Asn Pro Lys Val Gly Thr
    130                 135                 140

Val Thr Pro Asn Val Ala Glu Ala Val Lys Asn Ala Lys Ala Gly Gln

145                 150                 155                 160

Val Gln Tyr Arg Thr Asp Lys Ala Gly Ile Val His Ala Thr Ile Gly
            165                 170                 175

Arg Ala Ser Phe Ala Glu Ala Asp Leu Lys Glu Asn Phe Asp Ala Leu
            180                 185                 190

Leu Asp Ala Ile Val Lys Ala Lys Pro Ala Ala Ala Lys Gly Gln Tyr
        195                 200                 205

Leu Lys Lys Val Ala Val Ser Ser Thr Met Gly Leu Gly Ile Arg Val
    210                 215                 220

Asp Thr Ser Ser Val Asn Asn
225                 230
```

<210> 15
<211> 166
<212> PRT
<213> Neisseria meningitidis

<400> 15

```
Met Ser Leu Asn Ile Glu Thr Lys Lys Val Ala Val Glu Glu Ile Ser
1               5                   10                  15

Ala Ala Ile Ala Asn Ala Gln Thr Leu Val Val Ala Glu Tyr Arg Gly
            20              25                  30

Ile Ser Val Ser Ser Met Thr Glu Leu Arg Ala Asn Ala Arg Lys Glu
        35              40                  45

Gly Val Tyr Leu Arg Val Leu Lys Asn Thr Leu Ala Arg Arg Ala Val
    50              55                  60

Gln Gly Thr Ser Phe Ala Glu Leu Ala Asp Gln Met Val Gly Pro Leu
65              70                  75                  80

Val Tyr Ala Ala Ser Glu Asp Ala Val Ala Ala Ala Lys Val Leu His
            85                  90                  95

Gln Phe Ala Lys Lys Asp Asp Lys Ile Val Val Lys Ala Gly Ser Tyr
            100             105                 110

Asn Gly Glu Val Met Asn Ala Ala Gln Val Ala Glu Leu Ala Ser Ile
            115                 120                 125

Pro Ser Arg Glu Glu Leu Leu Ser Lys Leu Leu Phe Val Met Gln Ala
    130             135                 140

Pro Val Ser Gly Phe Ala Arg Gly Leu Ala Ala Leu Ala Glu Lys Lys
145             150                 155                 160

Ala Gly Glu Glu Ala Ala
                165
```

<210> 16
<211> 123
<212> PRT
<213> Neisseria meningitidis

<400> 16

```
Met Ala Ile Thr Lys Glu Asp Ile Leu Glu Ala Val Gly Ser Leu Thr
1               5                   10                  15

Val Met Glu Leu Asn Asp Leu Val Lys Ala Phe Glu Glu Lys Phe Gly
            20              25                  30

Val Ser Ala Ala Ala Val Ala Val Ala Gly Pro Ala Gly Ala Gly Ala
        35              40                  45


Ala Asp Ala Glu Glu Lys Thr Glu Phe Asp Val Val Leu Ala Ser Ala
    50              55                  60

Gly Asp Gln Lys Val Gly Val Ile Lys Val Val Arg Ala Ile Thr Gly
65              70                  75                  80

Leu Gly Leu Lys Glu Ala Lys Asp Ile Val Asp Gly Ala Pro Lys Thr
            85              90                  95

Ile Lys Glu Gly Val Ser Lys Ala Glu Ala Glu Asp Ile Gln Lys Gln
            100             105                 110

Leu Glu Glu Ala Gly Ala Lys Val Glu Ile Lys
            115                 120
```

<210> 17
<211> 1394
<212> PRT
<213> Neisseria meningitidis

<400> 17

```
Met Cys Met Asn Tyr Ser Phe Thr Glu Lys Lys Arg Ile Arg Lys Ser
1               5                   10                  15

Phe Ala Lys Arg Glu Asn Val Leu Glu Val Pro Phe Leu Leu Ala Thr
            20                  25                  30

Gln Ile Asp Ser Tyr Ala Lys Phe Leu Gln Leu Glu Asn Ala Phe Asp
        35                  40                  45

Lys Arg Thr Asp Asp Gly Leu Gln Ala Ala Phe Asn Ser Ile Phe Pro
    50                  55                  60

Ile Val Ser His Asn Gly Tyr Ala Arg Leu Glu Phe Val His Tyr Thr
65                  70                  75                  80

Leu Gly Glu Pro Leu Phe Asp Ile Pro Glu Cys Gln Leu Arg Gly Ile
            85                  90                  95

Thr Tyr Ala Ala Pro Leu Arg Ala Arg Ile Arg Leu Val Ile Leu Asp
            100                 105                 110

Lys Glu Ala Ser Lys Pro Thr Val Lys Glu Val Arg Glu Asn Glu Val
        115                 120                 125

Tyr Met Gly Glu Ile Pro Leu Met Thr Pro Ser Gly Ser Phe Val Ile
    130                 135                 140

Asn Gly Thr Glu Arg Val Ile Val Ser Gln Leu His Arg Ser Pro Gly
145                 150                 155                 160

Val Phe Phe Glu His Asp Lys Gly Lys Thr His Ser Ser Gly Lys Leu
            165                 170                 175

Leu Phe Ser Ala Arg Ile Ile Pro Tyr Arg Gly Ser Trp Leu Asp Phe
            180                 185                 190

Glu Phe Asp Pro Lys Asp Leu Leu Tyr Phe Arg Ile Asp Arg Arg Arg
        195                 200                 205

Lys Met Pro Val Thr Ile Leu Leu Lys Ala Leu Gly Tyr Asn Asn Glu
    210                 215                 220

Gln Ile Leu Asp Ile Phe Tyr Asp Lys Glu Thr Phe Tyr Leu Ser Ser
225                 230                 235                 240

Asn Gly Val Gln Thr Asp Leu Val Ala Asp Arg Leu Lys Gly Glu Thr
            245                 250                 255
```

```
Ala Lys Val Asp Ile Leu Asp Lys Glu Gly Asn Val Leu Val Ala Lys
            260             265             270

Gly Lys Arg Ile Thr Ala Lys Asn Ile Arg Asp Ile Thr Asn Ala Gly
        275             280             285

Leu Thr Arg Leu Asp Val Glu Pro Glu Ser Leu Leu Gly Lys Ala Leu
    290             295              300

Ala Ala Asp Leu Ile Asp Ser Glu Thr Gly Glu Val Leu Ala Ser Ala
305             310             315             320

Asn Asp Glu Ile Thr Glu Glu Leu Leu Ala Lys Phe Asp Ile Asn Gly
            325             330             335

Val Lys Glu Ile Thr Thr Leu Tyr Ile Asn Glu Leu Asp Gln Gly Ala
            340             345             350

Tyr Ile Ser Asn Thr Leu Arg Thr Asp Glu Thr Ala Gly Arg Gln Ala
        355             360             365

Ala Arg Val Ala Ile Tyr Arg Met Met Arg Pro Gly Glu Pro Pro Thr
    370             375             380

Glu Glu Ala Val Glu Gln Leu Phe Asn Arg Leu Phe Phe Ser Glu Asp
385             390             395             400

Ser Tyr Asp Leu Ser Arg Val Gly Arg Met Lys Phe Asn Thr Arg Thr
            405             410             415

Tyr Glu Gln Lys Leu Ser Glu Ala Gln Gln Asn Ser Trp Tyr Gly Arg
            420             425             430

Leu Leu Asn Glu Thr Phe Ala Gly Ala Ala Asp Lys Gly Gly Tyr Val
        435             440             445

Leu Ser Val Glu Asp Ile Val Ala Ser Ile Ala Thr Leu Val Glu Leu
    450             455             460

Arg Asn Gly His Gly Glu Val Asp Asp Ile Asp His Leu Gly Asn Arg
465             470             475             480

Arg Val Arg Ser Val Gly Glu Leu Thr Glu Asn Gln Phe Arg Ser Gly
            485             490             495

Leu Ala Arg Val Glu Arg Ala Val Lys Glu Arg Leu Asn Gln Ala Glu
        500             505             510

Ser Glu Asn Leu Met Pro His Asp Leu Ile Asn Ala Lys Pro Val Ser
        515             520             525

Ala Ala Ile Lys Glu Phe Phe Gly Ser Ser Gln Leu Ser Gln Phe Met
    530             535             540

Asp Gln Thr Asn Pro Leu Ser Glu Val Thr His Lys Arg Arg Val Ser
545             550             555             560

Ala Leu Gly Pro Gly Gly Leu Thr Arg Glu Arg Ala Gly Phe Glu Val
            565             570             575

Arg Asp Val His Pro Thr His Tyr Gly Arg Val Cys Pro Ile Glu Thr
            580             585             590

Pro Glu Gly Pro Asn Ile Gly Leu Ile Asn Ser Leu Ser Val Tyr Ala
        595             600             605

Arg Thr Asn Asp Tyr Gly Phe Leu Glu Thr Pro Tyr Arg Arg Val Ile
    610             615             620

Asp Gly Lys Val Thr Glu Glu Ile Asp Tyr Leu Ser Ala Ile Glu Glu
```

<pre>
      625                    630                   635                   640
      Gly Arg Tyr Val Ile Ala Gln Ala Asn Ala Asp Leu Asp Ser Asp Gly
                      645                   650                   655

      Asn Leu Ile Gly Asp Leu Val Thr Cys Arg Glu Lys Gly Glu Thr Ile
                      660                   665                   670

      Met Ala Thr Pro Asp Arg Val Gln Tyr Met Asp Val Ala Thr Gly Gln
              675                   680                   685

      Val Val Ser Val Ala Ala Ser Leu Ile Pro Phe Leu Glu His Asp Asp
          690                   695                   700

      Ala Asn Arg Ala Leu Met Gly Ala Asn Met Gln Arg Gln Ala Val Pro
      705                   710                   715                   720

      Cys Leu Arg Pro Glu Lys Pro Met Val Gly Thr Gly Ile Glu Arg Ser
                      725                   730                   735

      Val Ala Val Asp Ser Ala Thr Ala Ile Val Ala Arg Arg Gly Gly Val
                      740                   745                   750

      Val Glu Tyr Val Asp Ala Asn Arg Val Val Ile Arg Val His Asp Asp
                  755                   760                   765

      Glu Ala Thr Ala Gly Glu Val Gly Val Asp Ile Tyr Asn Leu Val Lys
          770                   775                   780

      Phe Thr Arg Ser Asn Gln Ser Thr Asn Ile Asn Gln Arg Pro Ala Val
      785                   790                   795                   800

      Lys Ala Gly Asp Val Leu Gln Arg Gly Asp Leu Val Ala Asp Gly Ala
                      805                   810                   815

      Ser Thr Asp Phe Gly Glu Leu Ala Leu Gly Gln Asn Met Thr Ile Ala
                  820                   825                   830

      Phe Met Pro Trp Asn Gly Tyr Asn Tyr Glu Asp Ser Ile Leu Ile Ser
                  835                   840                   845

      Glu Lys Val Ala Ala Asp Asp Arg Tyr Thr Ser Ile His Ile Glu Glu
          850                   855                   860

      Leu Asn Val Val Ala Arg Asp Thr Lys Leu Gly Ala Glu Asp Ile Thr
      865                   870                   875                   880

      Arg Asp Ile Pro Asn Leu Ser Glu Arg Met Gln Asn Arg Leu Asp Glu
                      885                   890                   895

      Ser Gly Ile Val Tyr Ile Gly Ala Glu Val Glu Ala Gly Asp Val Leu
                  900                   905                   910

      Val Gly Lys Val Thr Pro Lys Gly Glu Thr Gln Leu Thr Pro Glu Glu
                  915                   920                   925

      Lys Leu Leu Arg Ala Ile Phe Gly Glu Lys Ala Ser Asp Val Lys Asp
          930                   935                   940

      Thr Ser Leu Arg Met Pro Thr Gly Met Ser Gly Thr Val Ile Asp Val
      945                   950                   955                   960

      Gln Val Phe Thr Arg Glu Gly Ile Gln Arg Asp Lys Arg Ala Gln Ser
                  965                   970                   975

      Ile Ile Asp Ser Glu Leu Lys Arg Tyr Arg Leu Asp Leu Asn Asp Gln
                  980                   985                   990

      Leu Arg Ile Phe Asp Asn Asp Ala Phe Asp Arg Ile Glu Arg Met Ile
          995                  1000                  1005
</pre>

```
Val Gly Gln Lys Ala Asn Gly Gly Pro Met Lys Leu Ala Lys Gly Ser
    1010                1015                1020

Glu Ile Thr Thr Glu Tyr Leu Ala Gly Leu Pro Ser Arg His Asp Trp
1025                1030                1035                1040

Phe Asp Ile Arg Leu Thr Asp Glu Asp Leu Ala Lys Gln Leu Glu Leu
                1045                1050                1055

Ile Lys Val Ser Leu Gln Gln Lys Arg Glu Glu Ala Asp Glu Leu Tyr
            1060                1065                1070

Glu Ile Lys Lys Lys Lys Leu Thr Gln Gly Asp Glu Leu Gln Pro Gly
        1075                1080                1085

Val Gln Lys Met Val Lys Val Phe Ile Ala Ile Lys Arg Arg Leu Gln
    1090                1095                1100

Ala Gly Asp Lys Met Ala Gly Arg His Gly Asn Lys Gly Val Val Ser
1105                1110                1115                1120

Arg Ile Leu Pro Val Glu Asp Met Pro Tyr Met Ala Asp Gly Arg Pro
                1125                1130                1135

Val Asp Ile Val Leu Asn Pro Leu Gly Val Pro Ser Arg Met Asn Ile
            1140                1145                1150

Gly Gln Ile Leu Glu Val His Leu Gly Trp Ala Ala Lys Gly Ile Gly
        1155                1160                1165

Glu Arg Ile Asp Arg Met Leu Lys Glu Gln Arg Lys Ala Gly Glu Leu
    1170                1175                1180

Arg Glu Phe Leu Asn Arg Leu Tyr Asn Gly Ser Gly Lys Lys Glu Asp
1185                1190                1195                1200

Leu Asp Ala Leu Thr Asp Glu Glu Ile Ile Glu Leu Ala Ser Asn Leu
                1205                1210                1215

Arg Lys Gly Ala Ser Phe Ala Ser Pro Val Phe Asp Gly Ala Lys Glu
                1220                1225                1230

Ser Glu Ile Arg Glu Met Leu Asn Leu Ala Tyr Pro Ser Asp Asp Pro
        1235                1240                1245

Glu Val Glu Lys Leu Gly Phe Asn Asp Ser Lys Thr Gln Ile Thr Leu
    1250                1255                1260

Tyr Asp Gly Arg Ser Gly Glu Ala Phe Asp Arg Lys Val Thr Val Gly
1265                1270                1275                1280

Val Met His Tyr Leu Lys Leu His His Leu Val Asp Glu Lys Met His
                1285                1290                1295

Ala Arg Ser Thr Gly Pro Tyr Ser Leu Val Thr Gln Gln Pro Leu Gly
            1300                1305                1310

Gly Lys Ala Gln Phe Gly Gly Gln Arg Phe Gly Glu Met Glu Val Trp
        1315                1320                1325

Ala Leu Glu Ala Tyr Gly Ala Ala Tyr Thr Leu Gln Glu Met Leu Thr
    1330                1335                1340

Val Lys Ser Asp Asp Val Asn Gly Arg Thr Lys Met Tyr Glu Asn Ile
1345                1350                1355                1360

Val Lys Gly Glu His Lys Ile Asp Ala Gly Met Pro Glu Ser Phe Asn
            1365                1370                1375
```

```
Val Leu Val Lys Glu Ile Arg Ser Leu Gly Leu Asp Ile Asp Leu Glu
            1380                1385                1390

    Arg Tyr
```

<210> 18
<211> 1391
<212> PRT
<213> Neisseria meningitidis

<400> 18

```
Met Asn Leu Leu Asn Leu Phe Asn Pro Leu Gln Thr Ala Gly Met Glu
1               5               10              15

Glu Glu Phe Asp Ala Ile Lys Ile Gly Ile Ala Ser Pro Glu Thr Ile
            20              25              30

Arg Ser Trp Ser Tyr Gly Glu Val Lys Lys Pro Glu Thr Ile Asn Tyr
        35              40              45

Arg Thr Phe Lys Pro Glu Arg Asp Gly Leu Phe Cys Ala Lys Ile Phe
    50              55              60

Gly Pro Val Lys Asp Tyr Glu Cys Leu Cys Gly Lys Tyr Lys Arg Leu
65              70              75              80

Lys Phe Lys Gly Val Thr Cys Glu Lys Cys Gly Val Glu Val Thr Leu
            85              90              95

Ser Lys Val Arg Arg Glu Arg Met Gly His Ile Glu Leu Ala Ala Pro
            100             105             110

Val Ala His Ile Trp Phe Leu Lys Ser Leu Pro Ser Arg Leu Gly Met
        115             120             125

Val Leu Asp Met Thr Leu Arg Asp Ile Glu Arg Val Leu Tyr Phe Glu
    130             135             140

Ala Phe Val Val Thr Asp Pro Gly Met Thr Pro Leu Gln Arg Arg Gln
145             150             155             160

Leu Leu Thr Glu Asp Asp Tyr Tyr Asn Lys Leu Asp Glu Tyr Gly Asp
            165             170             175

Asp Phe Asp Ala Lys Met Gly Ala Glu Gly Ile Arg Glu Leu Leu Arg
        180             185             190

Thr Leu Asn Val Ala Gly Glu Ile Glu Ile Leu Arg Gln Glu Leu Glu
        195             200             205

Ser Thr Gly Ser Asp Thr Lys Ile Lys Lys Ile Ala Lys Arg Leu Lys
    210             215             220

Val Leu Glu Ala Phe His Arg Ser Gly Met Lys Leu Glu Trp Met Ile
225             230             235             240

Met Asp Val Leu Pro Val Leu Pro Pro Asp Leu Arg Pro Leu Val Pro
            245             250             255

Leu Asp Gly Gly Arg Phe Ala Thr Ser Asp Leu Asn Asp Leu Tyr Arg
            260             265             270

Arg Val Ile Asn Arg Asn Asn Arg Leu Lys Arg Leu Leu Glu Leu His
        275             280             285

Ala Pro Asp Ile Ile Val Arg Asn Glu Lys Arg Met Leu Gln Glu Ala
    290             295             300

Val Asp Ser Leu Leu Asp Asn Gly Arg Arg Gly Lys Ala Met Thr Gly
```

305 310 315 320

Ala Asn Lys Arg Pro Leu Lys Ser Leu Ala Asp Met Ile Lys Gly Lys
325 330 335

Gly Gly Arg Phe Arg Gln Asn Leu Leu Gly Lys Arg Val Asp Tyr Ser
340 345 350

Gly Arg Ser Val Ile Thr Val Gly Pro Tyr Leu Arg Leu His Gln Cys
355 360 365

Gly Leu Pro Lys Lys Met Ala Leu Glu Leu Phe Lys Pro Phe Ile Phe
370 375 380

His Lys Leu Glu Lys Gln Gly Leu Ala Ser Thr Val Lys Ala Ala Lys
385 390 395 400

Lys Leu Val Glu Gln Glu Val Pro Glu Val Trp Asp Ile Leu Glu Glu
405 410 415

Val Ile Arg Glu His Pro Ile Met Leu Asn Arg Ala Pro Thr Leu His
420 425 430

Arg Leu Gly Ile Gln Ala Phe Glu Pro Ile Leu Ile Glu Gly Lys Ala
435 440 445

Ile Gln Leu His Pro Leu Val Cys Ala Ala Phe Asn Ala Asp Phe Asp
450 455 460

Gly Asp Gln Met Ala Val His Val Pro Leu Ser Leu Glu Ala Gln Met
465 470 475 480

Glu Ala Arg Thr Leu Met Leu Ala Ser Asn Asn Val Leu Ser Pro Ala
485 490 495

Asn Gly Glu Pro Ile Ile Val Pro Ser Gln Asp Ile Val Leu Gly Leu
500 505 510

Tyr Tyr Met Thr Arg Asp Arg Ile Asn Ala Lys Gly Glu Gly Ser Leu
515 520 525

Phe Ala Asp Val Lys Glu Val His Arg Ala Tyr His Thr Lys Gln Val
530 535 540

Glu Leu Gly Thr Lys Ile Thr Val Arg Leu Arg Glu Trp Val Lys Asn
545 550 555 560

Glu Ala Gly Glu Phe Glu Pro Val Val Asn Arg Tyr Glu Thr Thr Val
565 570 575

Gly Arg Ala Leu Leu Ser Glu Ile Leu Pro Lys Gly Leu Pro Phe Glu
580 585 590

Tyr Val Asn Lys Ala Leu Lys Lys Lys Glu Ile Ser Lys Leu Ile Asn
595 600 605

Ala Ser Phe Arg Leu Cys Gly Leu Arg Asp Thr Val Ile Phe Ala Asp
610 615 620

His Leu Met Tyr Thr Gly Phe Gly Phe Ala Ala Lys Gly Gly Ile Ser
625 630 635 640

Ile Ala Val Asp Asp Met Glu Ile Pro Lys Glu Lys Ala Ala Leu Leu
645 650 655

Ala Glu Ala Asn Ala Glu Val Lys Glu Ile Glu Asp Gln Tyr Arg Gln
660 665 670

Gly Leu Val Thr Asn Gly Glu Arg Tyr Asn Lys Val Val Asp Ile Trp
675 680 685

74

Gly Arg Ala Gly Asp Lys Ile Ala Lys Ala Met Met Asp Asn Leu Ser
690                 695                 700

Lys Gln Lys Val Ile Asp Arg Ala Gly Asn Glu Val Asp Gln Glu Ser
705                 710                 715                 720

Phe Asn Ser Ile Tyr Met Met Ala Asp Ser Gly Ala Arg Gly Ser Ala
            725                 730                 735

Ala Gln Ile Lys Gln Leu Ser Gly Met Arg Gly Leu Met Ala Lys Pro
            740                 745                 750

Asp Gly Ser Ile Ile Glu Thr Pro Ile Thr Ser Asn Phe Arg Glu Gly
            755                 760                 765

Leu Thr Val Leu Gln Tyr Phe Ile Ala Thr His Gly Ala Arg Lys Gly
770                 775                 780

Leu Ala Asp Thr Ala Leu Lys Thr Ala Asn Ser Gly Tyr Leu Thr Arg
785                 790                 795                 800

Arg Leu Val Asp Val Thr Gln Asp Leu Val Val Val Glu Asp Asp Cys
            805                 810                 815

Gly Thr Ser Asp Gly Phe Val Met Lys Ala Val Val Gln Gly Gly Asp
            820                 825                 830

Val Ile Glu Ala Leu Arg Asp Arg Ile Leu Gly Arg Val Thr Ala Ser
            835                 840                 845

Asp Val Val Asp Pro Ser Ser Gly Glu Thr Leu Val Glu Ala Gly Thr
850                 855                 860

Leu Leu Thr Glu Lys Leu Val Asp Met Ile Asp Gln Ser Gly Val Asp
865                 870                 875                 880

Glu Val Lys Val Arg Thr Pro Ile Thr Cys Lys Thr Arg His Gly Leu
            885                 890                 895

Cys Ala His Cys Tyr Gly Arg Asp Leu Ala Arg Gly Lys Leu Val Asn
            900                 905                 910

Ala Gly Glu Ala Val Gly Val Ile Ala Ala Gln Ser Ile Gly Glu Pro
            915                 920                 925

Gly Thr Gln Leu Thr Met Arg Thr Phe His Ile Gly Gly Ala Ala Ser
            930                 935                 940

Arg Ala Ala Ala Ala Ser Gln Val Glu Ala Lys Ser Asn Gly Thr Ala
945                 950                 955                 960

Arg Phe Ser Ser Gln Met Arg Tyr Val Ala Asn Asn Lys Gly Glu Leu
            965                 970                 975

Val Val Ile Gly Arg Ser Cys Glu Val Val Ile His Asp Asp Ile Gly
            980                 985                 990

Arg Glu Arg Glu Arg His Lys Val Pro Tyr Gly Ala Ile Leu Leu Val
            995                 1000                1005

Gln Asp Gly Met Ala Ile Lys Ala Gly Gln Thr Leu Ala Thr Trp Asp
1010                1015                1020

Pro His Thr Arg Pro Met Ile Thr Glu His Ala Gly Met Val Lys Phe
1025                1030                1035                1040

Glu Asn Val Glu Glu Gly Val Thr Val Ala Lys Gln Thr Asp Asp Val
            1045                1050                1055

```
Thr Gly Leu Ser Thr Leu Val Val Ile Asp Gly Lys Arg Arg Ser Ser
            1060                1065            1070

Ser Ala Ser Lys Leu Leu Arg Pro Thr Val Lys Leu Leu Asp Glu Asn
            1075                1080                1085

Gly Val Glu Ile Cys Ile Pro Gly Thr Ser Thr Pro Val Ser Met Ala
    1090                1095                1100

Phe Pro Val Gly Ala Val Ile Thr Val Arg Glu Gly Gln Glu Ile Gly
1105                1110                1115                1120

Lys Gly Asp Val Leu Ala Arg Ile Pro Gln Ala Ser Ser Lys Thr Arg
                1125                1130                1135

Asp Ile Thr Gly Gly Leu Pro Arg Val Ala Glu Leu Phe Glu Ala Arg
            1140                1145                1150

Val Pro Lys Asp Ala Gly Met Leu Ala Glu Ile Thr Gly Thr Val Ser
            1155                1160                1165

Phe Gly Lys Glu Thr Lys Gly Lys Gln Arg Leu Ile Val Thr Asp Val
    1170         .      1175                1180

Asp Gly Val Ala Tyr Glu Thr Leu Ile Ser Lys Glu Lys Gln Ile Leu
1185                1190                1195                1200

Val His Asp Gly Gln Val Val Asn Arg Gly Glu Thr Ile Val Asp Gly
                1205                1210                1215

Ala Val Asp Pro His Asp Ile Leu Arg Leu Gln Gly Ile Glu Ala Leu
            1220                1225                1230

Ala Arg Tyr Ile Val Gln Glu Val Gln Glu Val Tyr Arg Leu Gln Gly
            1235                1240                1245

Val Lys Ile Ser Asp Lys His Ile Glu Val Ile Ile Arg Gln Met Leu
    1250                1255                1260

Arg Arg Val Asn Ile Ala Asp Ala Gly Glu Thr Gly Phe Ile Thr Gly
1265                1270                1275                1280

Glu Gln Val Glu Arg Gly Asp Val Met Ala Ala Asn Glu Lys Ala Leu
                1285                1290                1295

Glu Glu Gly Lys Glu Pro Ala Arg Tyr Glu Asn Val Leu Leu Gly Ile
                1300                1305                1310

Thr Lys Ala Ser Leu Ser Thr Asp Ser Phe Ile Ser Ala Ala Ser Phe
    1315                1320                1325

Gln Glu Thr Thr Arg Val Leu Thr Glu Ala Ala Ile Met Gly Lys Gln
    1330                1335                1340

Asp Glu Leu Arg Gly Leu Lys Glu Asn Val Ile Val Gly Arg Leu Ile
1345                1350                1355                1360

Pro Ala Gly Thr Gly Leu Thr Tyr His Arg Ser Arg His Gln Gln Trp
                1365                1370                1375

Gln Glu Val Glu Gln Glu Thr Ala Glu Thr Gln Val Thr Asp Glu
                1380                1385                1390
```

<210> 19
<211> 156
<212> PRT
<213> Neisseria meningitidis

<400> 19

```
Met Pro Arg Arg Arg Glu Val Pro Lys Arg Asp Val Leu Pro Asp Pro

1               5               10              15

Lys Phe Gly Ser Val Glu Leu Thr Lys Phe Met Asn Val Leu Met Ile
            20              25              30

Asp Gly Lys Lys Ser Val Ala Glu Arg Ile Val Tyr Gly Ala Leu Glu
            35              40              45

Gln Ile Glu Lys Lys Thr Gly Lys Val Ala Ile Glu Val Phe Asn Glu
        50              55              60

Ala Ile Ala Asn Ala Lys Pro Ile Val Glu Val Lys Ser Arg Arg Val
65              70              75              80

Gly Gly Ala Asn Tyr Gln Val Pro Val Glu Val Arg Pro Ser Arg Arg
                85              90              95

Leu Ala Leu Ala Met Arg Trp Val Arg Asp Ala Ala Arg Lys Arg Gly
            100             105             110

Glu Lys Ser Met Asp Leu Arg Leu Ala Gly Glu Leu Ile Asp Ala Ser
            115             120             125

Glu Gly Arg Gly Gly Ala Leu Lys Lys Arg Glu Glu Val His Arg Met
        130             135             140

Ala Glu Ala Asn Lys Ala Phe Ser His Phe Arg Phe
145             150             155
```

<210> 20
<211> 701
<212> PRT
<213> Neisseria meningitidis

<400> 20

```
Met Ala Arg Lys Thr Pro Ile Ser Leu Tyr Arg Asn Ile Gly Ile Ser
1               5               10              15

Ala His Ile Asp Ala Gly Lys Thr Thr Thr Thr Glu Arg Ile Leu Phe
            20              25              30

Tyr Thr Gly Leu Thr His Lys Leu Gly Glu Val His Asp Gly Ala Ala
        35              40              45

Thr Thr Asp Tyr Met Glu Gln Glu Gln Glu Arg Gly Ile Thr Ile Thr
    50              55              60

Ser Ala Ala Val Thr Ser Tyr Trp Ser Gly Met Ala Lys Gln Phe Pro
65              70              75              80

Glu His Arg Phe Asn Ile Ile Asp Thr Pro Gly His Val Asp Phe Thr
            85              90              95

Val Glu Val Glu Arg Ser Met Arg Val Leu Asp Gly Ala Val Met Val
        100             105             110

Tyr Cys Ala Val Gly Gly Val Gln Pro Gln Ser Glu Thr Val Trp Arg
    115             120             125

Gln Ala Asn Lys Tyr Gln Val Pro Arg Leu Ala Phe Val Asn Lys Met
    130             135             140

Asp Arg Gln Gly Ala Asn Phe Phe Arg Val Val Glu Gln Met Lys Thr
145             150             155             160

Arg Leu Arg Ala Asn Pro Val Pro Ile Val Ile Pro Val Gly Ala Glu
            165             170             175

Asp Asn Phe Ser Gly Val Val Asp Leu Leu Lys Met Lys Ser Ile Ile
        180             185             190
```

```
Trp Asn Glu Val Asp Lys Gly Thr Thr Phe Thr Tyr Gly Asp Ile Pro
        195                 200                 205

Ala Glu Leu Val Glu Thr Ala Glu Glu Trp Arg Gln Asn Met Ile Glu
    210                 215                 220

Ala Ala Ala Glu Ala Ser Glu Glu Leu Met Asp Lys Tyr Leu Gly Gly
225                 230                 235                 240

Asp Glu Leu Thr Glu Glu Glu Ile Val Gly Ala Leu Arg Gln Arg Thr
                245                 250                 255

Leu Ala Gly Glu Ile Gln Pro Met Leu Cys Gly Ser Ala Phe Lys Asn
                260                 265                 270

Lys Gly Val Gln Arg Met Leu Asp Ala Val Val Glu Leu Leu Pro Ala
            275                 280                 285

Pro Thr Asp Ile Pro Pro Val Gln Gly Val Asn Pro Asn Thr Glu Glu
    290                 295                 300

Ala Asp Ser Arg Gln Ala Ser Asp Glu Glu Lys Phe Ser Ala Leu Ala
305                 310                 315                 320

Phe Lys Met Leu Asn Asp Lys Tyr Val Gly Gln Leu Thr Phe Ile Arg
                325                 330                 335

Val Tyr Ser Gly Val Val Lys Ser Gly Asp Thr Val Leu Asn Ser Val
            340                 345                 350

Lys Gly Thr Arg Glu Arg Ile Gly Arg Leu Val Gln Met Thr Ala Ala
            355                 360                 365

Asp Arg Thr Glu Ile Glu Glu Val Arg Ala Gly Asp Ile Ala Ala Ala
    370                 375                 380

Ile Gly Leu Lys Asp Val Thr Thr Gly Glu Thr Leu Cys Ala Glu Ser
385                 390                 395                 400

Ala Pro Ile Ile Leu Glu Arg Met Glu Phe Pro Glu Pro Val Ile His
                405                 410                 415

Ile Ala Val Glu Pro Lys Thr Lys Ala Asp Gln Glu Lys Met Gly Ile
            420                 425                 430

Ala Leu Asn Arg Leu Ala Lys Glu Asp Pro Ser Phe Arg Val Arg Thr
            435                 440                 445

Asp Glu Glu Ser Gly Gln Thr Ile Ile Ser Gly Met Gly Glu Leu His
    450                 455                 460

Leu Glu Ile Ile Val Asp Arg Met Lys Arg Glu Phe Gly Val Glu Ala
465                 470                 475                 480

Asn Ile Gly Ala Pro Gln Val Ala Tyr Arg Glu Thr Ile Arg Lys Ala
                485                 490                 495

Val Lys Ala Glu Tyr Lys His Ala Lys Gln Ser Gly Gly Lys Gly Gln
            500                 505                 510

Tyr Gly His Val Val Ile Glu Met Glu Pro Met Glu Pro Gly Gly Glu
        515                 520                 525

Gly Tyr Glu Phe Ile Asp Glu Ile Lys Gly Gly Val Ile Pro Arg Glu
    530                 535                 540

Phe Ile Pro Ser Val Asp Lys Gly Ile Arg Asp Thr Leu Pro Asn Gly
545                 550                 555                 560
```

```
Ile Val Ala Gly Tyr Pro Val Val Asp Val Arg Ile Arg Leu Val Phe
             565              570                    575

Gly Ser Tyr His Asp Val Asp Ser Ser Gln Leu Ala Phe Glu Leu Ala
             580              585              590

Ala Ser Gln Ala Phe Lys Glu Gly Met Arg Gln Ala Ser Pro Ala Leu
         595              600              605

Leu Glu Pro Ile Met Ala Val Glu Val Glu Thr Pro Glu Glu Tyr Met
         610              615              620

Gly Asp Val Met Gly Asp Leu Asn Arg Arg Arg Gly Val Val Leu Gly
625              630              635              640

Met Asp Asp Asp Gly Ile Gly Gly Lys Lys Val Arg Ala Glu Val Pro
             645              650              655

Leu Ala Glu Met Phe Gly Tyr Ser Thr Asp Leu Arg Ser Ala Thr Gln
         660              665              670

Gly Arg Ala Thr Tyr Ser Met Glu Phe Lys Lys Tyr Ser Glu Ala Pro
         675              680              685

Ala His Ile Ala Ala Ala Val Thr Glu Ala Arg Lys Gly
    690              695              700
```

<210> 21
<211> 103
<212> PRT
<213> Neisseria meningitidis

<400> 21

```
Met Ala Asn Gln Lys Ile Arg Ile Arg Leu Lys Ala Tyr Asp Tyr Ala
1              5              10                   15

Leu Ile Asp Arg Ser Ala Gln Glu Ile Val Glu Thr Ala Lys Arg Thr
         20              25              30

Gly Ala Val Val Lys Gly Pro Ile Pro Leu Pro Thr Lys Ile Glu Arg
         35              40              45

Phe Asn Ile Leu Arg Ser Pro His Val Asn Lys Thr Ser Arg Glu Gln
    50              55              60

Leu Glu Ile Arg Thr His Leu Arg Leu Met Asp Ile Val Asp Trp Thr
65              70              75              80

Asp Lys Thr Thr Asp Ala Leu Met Lys Leu Asp Leu Pro Ala Gly Val
             85              90              95

Asp Val Glu Ile Lys Val Gln
             100
```

<210> 22
<211> 214
<212> PRT
<213> Neisseria meningitidis

<400> 22

```
Met Thr Leu Gly Leu Val Gly Arg Lys Val Gly Met Thr Arg Val Phe
1               5               10              15

Asp Glu Gln Gly Val Ser Val Pro Val Thr Val Leu Asp Met Ser Ala
            20              25              30

Asn Arg Val Thr Gln Val Lys Ser Lys Asp Thr Asp Gly Tyr Thr Ala
        35              40              45

Val Gln Val Thr Phe Gly Gln Lys Lys Ala Asn Arg Val Asn Lys Ala

    50                      55                      60

Glu Ala Gly His Phe Ala Lys Ala Gly Val Glu Ala Gly Arg Gly Leu
65              70              75              80

Ile Glu Phe Ala Leu Thr Glu Glu Lys Leu Ala Glu Leu Lys Ala Gly
            85              90              95

Asp Glu Ile Thr Val Ser Met Phe Glu Val Gly Gln Leu Val Asp Val
            100             105             110

Thr Gly Thr Ser Lys Gly Lys Gly Phe Ser Gly Thr Ile Lys Arg His
        115             120             125

Asn Phe Gly Ala Gln Arg Thr Ser His Gly Asn Ser Arg Ser His Arg
    130             135             140

Val Pro Gly Ser Ile Gly Met Ala Gln Asp Pro Gly Arg Val Phe Pro
145             150             155             160

Gly Lys Arg Met Ala Gly Gln Tyr Gly Asn Thr Lys Ala Thr Val Gln
            165             170             175

Lys Leu Glu Val Val Arg Val Asp Ala Glu Arg Gln Leu Leu Leu Val
        180             185             190

Lys Gly Ala Val Pro Gly Ala Val Asn Ser Asp Val Val Val Arg Pro
        195             200             205

Ser Val Lys Val Gly Ala
210
```

<210> 23
<211> 206
<212> PRT
<213> Neisseria meningitidis

<400> 23

```
Met Glu Leu Lys Val Ile Asp Ala Lys Gly Gln Val Ser Gly Ser Leu
1                5                    10                15

Ser Val Ser Asp Ala Leu Phe Ala Arg Glu Tyr Asn Glu Ala Leu Val
            20                25                30

His Gln Leu Val Asn Ala Tyr Leu Ala Asn Ala Arg Ser Gly Asn Arg
            35                40                45

Ala Gln Lys Thr Arg Ala Glu Val Lys His Ser Thr Lys Lys Pro Trp
    50                55                60

Arg Gln Lys Gly Thr Gly Arg Ala Arg Ser Gly Met Thr Ser Ser Pro
65                70                75                80

Leu Trp Arg Lys Gly Gly Arg Ala Phe Pro Asn Lys Pro Asp Glu Asn
            85                90                95

Phe Thr Gln Lys Val Asn Arg Lys Met Tyr Arg Ala Gly Met Ala Thr
            100               105               110

Ile Leu Ser Gln Leu Thr Arg Asp Glu Arg Leu Phe Ala Ile Glu Ala
            115               120               125

Leu Thr Ala Glu Thr Pro Lys Thr Lys Val Phe Ala Glu Gln Val Lys
    130               135               140

Asn Leu Gly Leu Glu Gln Val Leu Phe Val Thr Lys Gln Leu Asp Glu
145               150               155               160

Asn Val Tyr Leu Ala Ser Arg Asn Leu Pro Asn Val Leu Val Leu Glu
            165               170               175

Ala Gln Gln Val Asp Pro Tyr Ser Leu Leu Arg Tyr Lys Lys Val Ile
            180               185               190

Ile Thr Lys Asp Ala Val Ala Gln Leu Glu Glu Gln Trp Val
    195               200               205
```

<210> 24
<211> 104
<212> PRT
<213> Neisseria meningitidis

<400> 24

```
Met Asn Gln Gln Arg Leu Thr Gln Val Ile Leu Ala Pro Ile Val Ser
1                5                    10                15

Glu Lys Ser Asn Val Leu Ala Glu Lys Arg Asn Gln Met Thr Phe Lys
            20                25                30

Val Leu Ala Asn Ala Thr Lys Pro Glu Ile Lys Ala Ala Val Glu Leu
            35                40                45

Leu Phe Gly Val Gln Val Ala Asp Val Thr Thr Val Thr Ile Lys Gly
    50                55                60

Lys Val Lys Arg Phe Gly Arg Thr Leu Gly Arg Arg Ser Asp Val Lys
65                70                75                80

Lys Ala Tyr Val Ser Leu Ala Ala Gly Gln Glu Leu Asp Leu Glu Ala
            85                90                95

Ala Ala Ala Ala Ala Asp Lys Glu
            100
```

<210> 25
<211> 179
<212> PRT
<213> Neisseria meningitidis

<400> 25

```
Met Ala Arg Leu Arg Glu Phe Tyr Lys Glu Thr Val Val Pro Glu Leu
1               5                   10                  15

Val Lys Gln Phe Gly Tyr Lys Ser Val Met Glu Val Pro Arg Ile Glu
            20                  25                  30

Lys Ile Thr Leu Asn Met Gly Val Gly Glu Ala Val Ala Asp Lys Lys
        35                  40                  45

Val Met Glu His Ala Val Ser Asp Leu Glu Lys Ile Ala Gly Gln Lys
    50                  55                  60

Pro Val Val Thr Val Ala Arg Lys Ser Ile Ala Gly Phe Lys Ile Arg
65                  70                  75                  80

Asp Asn Tyr Pro Val Gly Cys Lys Val Thr Leu Arg Arg Asp Gln Met
            85                  90                  95

Phe Glu Phe Leu Asp Arg Leu Ile Thr Ile Ala Leu Pro Arg Val Arg
            100                 105                 110

Asp Phe Arg Gly Val Ser Gly Lys Ser Phe Asp Gly Arg Gly Asn Tyr
        115                 120                 125

Asn Met Gly Val Arg Glu Gln Ile Ile Phe Pro Glu Ile Glu Tyr Asp
    130                 135                 140

Lys Ile Asp Ala Leu Arg Gly Leu Asn Ile Thr Ile Thr Thr Thr Ala
145                 150                 155                 160

Lys Thr Asp Glu Glu Ala Lys Ala Leu Leu Ser Leu Phe Lys Phe Pro
                165                 170                 175

Phe Lys Gly
```

<210> 26
<211> 130
<212> PRT
<213> Neisseria meningitidis

<400> 26

```
Met Ser Met His Asp Pro Ile Ser Asp Met Leu Thr Arg Ile Arg Asn
1               5               10                  15

Ala Gln Arg Ala Asn Lys Ala Ala Val Ala Met Pro Ser Ser Lys Leu
            20              25              30

Lys Cys Ala Ile Ala Lys Val Leu Lys Glu Glu Gly Tyr Ile Glu Asp
        35              40              45

Phe Ala Val Ser Ser Asp Val Lys Ser Ile Leu Glu Ile Gln Leu Lys
    50              55              60

Tyr Tyr Ala Gly Arg Pro Val Ile Glu Gln Ile Lys Arg Val Ser Arg
65              70              75              80

Pro Gly Leu Arg Ile Tyr Lys Ala Ser Ser Glu Ile Pro Ser Val Met
            85              90              95

Asn Gly Leu Gly Ile Ala Ile Val Ser Thr Ser Lys Gly Val Met Thr
            100             105             110

Asp Arg Lys Ala Arg Ser Gln Gly Val Gly Gly Glu Leu Leu Cys Ile
        115             120             125

Val Ala
130
```

<210> 27
<211> 177
<212> PRT
<213> Neisseria meningitidis

<400> 27

```
Met Ser Arg Val Ala Lys Asn Pro Val Thr Val Pro Ala Gly Val Glu
1               5               10                  15

Val Lys Phe Gly Ala Glu Ala Leu Val Ile Lys Gly Lys Asn Gly Glu
            20              25              30

Leu Ser Phe Pro Leu His Ser Asp Val Ala Ile Glu Phe Asn Asp Gly
        35              40              45

Lys Leu Thr Phe Val Ala Asn Asn Ser Ser Lys Gln Ala Asn Ala Met
    50              55              60

Ser Gly Thr Ala Arg Ala Leu Val Ser Asn Met Val Lys Gly Val Ser
65              70              75              80

Glu Gly Phe Glu Lys Arg Leu Gln Leu Ile Gly Val Gly Tyr Arg Ala
            85              90              95

Gln Ala Gln Gly Lys Ile Leu Asn Leu Ser Leu Gly Phe Ser His Pro
            100             105             110

Ile Val Tyr Glu Met Pro Glu Gly Val Ser Val Gln Thr Pro Ser Gln
        115             120             125

Thr Glu Ile Val Leu Thr Gly Ser Asp Lys Gln Val Val Gly Gln Val
```

```
                130                    135    .              140
        Ala Ala Glu Ile Arg Ala Phe Arg Ala Pro Glu Pro Tyr Lys Gly Lys
        145                 150                 155                 160

        Gly Val Arg Tyr Val Gly Glu Val Val Val Met Lys Glu Ala Lys Lys
                        165                 170                 175

        Lys
```

<210> 28
<211> 172
<212> PRT
<213> Neisseria meningitidis

<400> 28

```
        Met Ala Lys His Glu Ile Glu Glu Arg Gly Asp Gly Leu Ile Glu Lys
        1               5                   10                  15

        Met Val Ala Val Asn Arg Val Thr Lys Val Val Lys Gly Gly Arg Ile
                        20                  25                  30

        Met Ala Phe Ser Ala Leu Thr Val Val Gly Asp Gly Asp Gly Arg Ile
                    35                  40                  45

        Gly Met Gly Lys Gly Lys Ser Lys Glu Val Pro Val Ala Val Gln Lys
            50                  55                  60

        Ala Met Asp Gln Ala Arg Arg Ser Met Ile Lys Val Pro Leu Lys Asn
        65                  70                  75                  80

        Gly Thr Ile His His Glu Val Ile Gly Arg His Gly Ala Thr Lys Val
                        85                  90                  95

        Phe Met Gln Pro Ala Lys Glu Gly Ser Gly Val Lys Ala Gly Gly Pro
                    100                 105                 110

        Met Arg Leu Val Phe Asp Ala Met Gly Ile His Asn Ile Ser Ala Lys
                    115                 120                 125

        Val His Gly Ser Thr Asn Pro Tyr Asn Ile Val Arg Ala Thr Leu Asp
            130                 135                 140

        Gly Leu Ser Lys Leu His Thr Pro Ala Asp Ile Ala Ala Lys Arg Gly
        145                 150                 155                 160

        Leu Thr Val Glu Asp Ile Leu Gly Val Asn His Gly
                        165                 170
```

<210> 29
<211> 328
<212> PRT
<213> Neisseria meningitidis

<400> 29

```
Met Gln Asn Ser Thr Thr Glu Phe Leu Lys Pro Arg Gln Ile Asp Val
1               5                   10                  15

Asn Thr Phe Ser Ala Thr Arg Ala Lys Val Ser Met Gln Pro Phe Glu
            20                  25                  30

Arg Gly Phe Gly His Thr Leu Gly Asn Ala Leu Arg Arg Ile Leu Leu
            35                  40                  45

Ser Ser Met Asn Gly Phe Ala Pro Thr Glu Val Ala Ile Ala Gly Val
        50                  55                  60

Leu His Glu Tyr Ser Thr Val Asp Gly Ile Gln Glu Asp Val Val Asp
65                  70                  75                  80

Ile Leu Leu Asn Ile Lys Gly Ile Val Phe Lys Leu His Gly Arg Ser
                85                  90                  95

Gln Val Gln Leu Val Leu Lys Lys Ser Gly Ser Gly Val Val Ser Ala
            100                 105                 110

Gly Asp Ile Glu Leu Pro His Asp Val Glu Ile Leu Asn Pro Gly His
            115                 120                 125

Val Ile Cys His Leu Ala Asp Asn Gly Gln Ile Glu Met Glu Ile Lys
    130                 135                 140

Val Glu Gln Gly Arg Gly Tyr Gln Ser Val Ser Gly Arg Gln Val Val
145                 150                 155                 160

Arg Asp Glu Asn Arg Gln Ile Gly Ala Ile Gln Leu Asp Ala Ser Phe
                165                 170                 175

Ser Pro Ile Ser Arg Val Ser Phe Glu Val Glu Pro Ala Arg Val Glu
            180                 185                 190

Gln Arg Thr Asp Leu Asp Lys Leu Val Leu Asp Ile Glu Thr Asp Gly
            195                 200                 205

Ser Ile Asp Pro Glu Glu Ala Val Arg Ser Ala Ala Arg Ile Leu Ile
    210                 215                 220

Asp Gln Met Ser Ile Phe Ala Asp Leu Gln Gly Thr Pro Val Glu Glu
225                 230                 235                 240

Val Glu Glu Lys Ala Pro Pro Ile Asp Pro Val Leu Leu Arg Pro Val
            245                 250                 255

Asp Asp Leu Glu Leu Thr Val Arg Ser Ala Asn Cys Leu Lys Ala Glu
            260                 265                 270

Asp Ile Tyr Tyr Ile Gly Asp Leu Ile Gln Arg Thr Glu Thr Glu Leu
            275                 280                 285

Leu Lys Thr Pro Asn Leu Gly Arg Lys Ser Leu Asn Glu Ile Lys Glu
            290                 295                 300

Val Leu Ala Ser Lys Gly Leu Thr Leu Gly Ser Lys Leu Glu Ala Trp
305                 310                 315                 320

Pro Pro Val Gly Leu Glu Lys Pro
                325
```

<210> 30
<211> 271
<212> PRT
<213> Neisseria meningitidis

<400> 30

```
Met Ala Lys Ile Ile Val Val Thr Ser Gly Lys Gly Gly Val Gly Lys
1               5               10                      15

Thr Thr Thr Ser Ala Ser Ile Ala Thr Gly Leu Ala Leu Arg Gly Tyr
        20              25                      30

Lys Thr Ala Val Ile Asp Phe Asp Val Gly Leu Arg Asn Leu Asp Leu
        35              40                      45

Ile Met Gly Cys Glu Arg Arg Val Val Tyr Asp Leu Ile Asn Val Ile
    50              55                      60

Gln Gly Glu Ala Thr Leu Asn Gln Ala Leu Ile Lys Asp Lys Asn Cys
65                      70              75                      80


Glu Asn Leu Phe Ile Leu Pro Ala Ser Gln Thr Arg Asp Lys Asp Ala
                85              90                      95

Leu Thr Arg Glu Gly Val Glu Lys Val Met Gln Glu Leu Ser Gly Lys
            100             105                     110

Lys Met Gly Phe Glu Tyr Ile Ile Cys Asp Ser Pro Ala Gly Ile Glu
        115             120                     125

Gln Gly Ala Leu Met Ala Leu Tyr Phe Ala Asp Glu Ala Ile Val Thr
    130             135                 140

Thr Asn Pro Glu Val Ser Ser Val Arg Asp Ser Asp Arg Ile Leu Gly
145                 150                 155                 160

Ile Leu Gln Ser Lys Ser His Lys Ala Glu Gln Gly Gly Ser Val Lys
                165             170                     175

Glu His Leu Leu Ile Thr Arg Tyr Ser Pro Glu Arg Val Ala Lys Gly
            180                 185                 190

Glu Met Leu Ser Val Gln Asp Ile Cys Asp Ile Leu His Ile Pro Leu
        195             200                 205

Leu Gly Val Ile Pro Glu Ser Gln Asn Val Leu Gln Ala Ser Asn Ser
    210             215                 220

Gly Glu Pro Val Ile His Gln Asp Ser Val Ala Ala Ser Glu Ala Tyr
225             230                 235                     240

Lys Asp Val Ile Ala Arg Leu Leu Gly Glu Asn Arg Glu Met Arg Phe
            245                 250                     255

Leu Glu Ala Glu Lys Lys Ser Phe Phe Lys Arg Leu Phe Gly Gly
            260             265                 270
```

<210> 31
<211> 306
<212> PRT
<213> Neisseria meningitidis

<400> 31

```
Met Thr Leu Thr Glu Leu Arg Tyr Ile Val Ala Val Ala Gln Glu Arg
1               5                   10              15

His Phe Gly Arg Ala Ala Arg Arg Cys Phe Val Ser Gln Pro Thr Leu
            20              25                  30

Ser Ile Ala Ile Lys Lys Leu Glu Glu Glu Leu Ala Val Ser Leu Phe
            35              40                  45

Asp Arg Ser Ser Asn Asp Ile Ile Thr Thr Glu Ala Gly Glu Arg Ile
        50              55              60

Val Ala Gln Ala Arg Lys Val Leu Glu Glu Ala Glu Leu Ile Arg His
65              70              75                  80

Leu Ala Asn Glu Glu Gln Asn Glu Leu Glu Gly Ala Phe Lys Leu Gly
                85              90                  95

Leu Ile Phe Thr Val Ala Pro Tyr Leu Leu Pro Lys Leu Ile Val Ser
            100             105                 110

Leu Arg Arg Thr Ala Pro Lys Met Pro Leu Met Leu Glu Glu Asn Tyr
        115             120                 125

Thr His Thr Leu Thr Glu Ser Leu Lys Arg Gly Asp Val Asp Ala Ile
    130             135             140

Ile Val Ala Glu Pro Phe Gln Glu Pro Gly Ile Val Thr Glu Pro Leu

145                 150                 155                 160

Tyr Asp Glu Pro Phe Phe Val Ile Val Pro Lys Gly His Ser Phe Glu
                165                 170                 175

Glu Leu Asp Ala Val Ser Pro Arg Met Leu Gly Glu Glu Gln Val Leu
            180                 185                 190

Leu Leu Thr Glu Gly Asn Cys Met Arg Asp Gln Val Leu Ser Ser Cys
            195             200                 205

Ser Glu Leu Ala Ala Lys Gln Arg Ile Gln Gly Leu Thr Asn Thr Leu
    210             215                 220

Gln Gly Ser Ser Ile Asn Thr Ile Arg His Met Val Ala Ser Gly Leu
225             230             235                 240

Ala Ile Ser Val Leu Pro Ala Thr Ala Leu Thr Glu Asn Asp His Met
            245             250                 255

Leu Phe Ser Ile Ile Pro Phe Glu Gly Thr Pro Pro Ser Arg Arg Val
            260             265                 270

Val Leu Ala Tyr Arg Arg Asn Phe Val Arg Pro Lys Ala Leu Ser Ala
            275             280                 285

Met Lys Ala Ala Ile Met Gln Ser Gln Leu His Gly Val Ser Phe Ile
    290             295                 300

Cys Asp
305
```

<210> 32
<211> 631
<212> PRT
<213> Neisseria meningitidis

<400> 32

```
Met Thr His Met Ile Tyr Pro Lys Thr Tyr Asp Val Ile Val Val Gly
1               5               10              15

Gly Gly His Ala Gly Thr Glu Ala Ala Leu Ala Ala Ala Arg Met Gly
        20              25              30

Ala Gln Thr Leu Leu Leu Ser His Asn Ile Glu Thr Leu Gly Gln Met
        35              40              45

Ser Cys Asn Pro Ser Ile Gly Gly Ile Gly Lys Gly His Leu Val Arg
        50              55              60

Glu Leu Asp Ala Leu Gly Gly Ala Met Ala Leu Ala Thr Asp Lys Ser
65              70              75              80

Gly Ile Gln Phe Arg Arg Leu Asn Ala Ser Lys Gly Ala Ala Val Arg
                85              90              95

Ala Thr Arg Ala Gln Ala Asp Arg Ile Leu Tyr Lys Ala Ala Ile Arg
            100             105             110

Glu Met Leu Glu Asn Gln Glu Asn Leu Asp Leu Phe Gln Gln Ala Val
        115             120             125

Glu Asp Val Thr Leu Asp Gly Glu Arg Ile Ser Gly Val Ile Thr Ala
        130             135             140

Met Gly Val Glu Phe Lys Ala Arg Ala Val Val Leu Thr Ala Gly Thr
145             150             155             160

Phe Leu Ser Gly Lys Ile His Ile Gly Leu Glu Asn Tyr Glu Gly Gly
                165             170             175
```

```
Arg Ala Gly Asp Pro Ala Ala Lys Ser Leu Gly Gly Arg Leu Arg Glu
            180                 185                 190

Leu Lys Leu Pro Gln Gly Arg Leu Lys Thr Gly Thr Pro Pro Arg Ile
        195                 200                 205

Asp Gly Arg Thr Ile Asp Phe Ser Gln Leu Thr Glu Gln Pro Gly Asp
    210                 215                 220

Thr Pro Val Pro Val Met Ser Val Arg Gly Asn Ala Asp Met His Pro
225                 230                 235                 240

Arg Gln Val Ser Cys Trp Ile Thr His Thr Asn Thr Gln Thr His Asp
                245                 250                 255

Ile Ile Arg Ser Gly Phe Asp Arg Ser Pro Met Phe Thr Gly Lys Ile
            260                 265                 270

Glu Gly Val Gly Pro Arg Tyr Cys Pro Ser Ile Glu Asp Lys Ile Asn
            275                 280                 285

Arg Phe Ala Asp Lys Asp Ser His Gln Ile Phe Leu Glu Pro Glu Gly
    290                 295                 300

Leu Thr Thr His Glu Tyr Tyr Pro Asn Gly Ile Ser Thr Ser Leu Pro
305                 310                 315                 320

Phe Asp Ile Gln Ile Ala Leu Val Arg Ser Met Lys Gly Leu Glu Asn
            325                 330                 335

Ala His Ile Leu Arg Pro Gly Tyr Ala Ile Glu Tyr Asp Tyr Phe Asp
            340                 345                 350

Pro Arg Asn Leu Lys Ala Ser Leu Glu Thr Lys Thr Ile Ala Gly Leu
        355                 360                 365

Phe Phe Ala Gly Gln Ile Asn Gly Thr Thr Gly Tyr Glu Glu Ala Ala
    370                 375                 380

Ala Gln Gly Leu Leu Ala Gly Ala Asn Ala Val Gln Tyr Val Arg Gly
385                 390                 395                 400

Gln Asp Pro Leu Leu Leu Arg Arg Glu Gln Ala Tyr Leu Gly Val Leu
            405                 410                 415

Val Asp Asp Leu Ile Thr Lys Gly Val Asn Glu Pro Tyr Arg Met Phe
            420                 425                 430

Thr Ser Arg Ala Glu Tyr Arg Leu Gln Leu Arg Glu Asp Asn Ala Asp
        435                 440                 445

Met Arg Leu Thr Glu Asp Gly Tyr Lys Ile Gly Leu Val Ser Glu Ala
    450                 455                 460

Gln Trp Arg Met Phe Asn Glu Lys Arg Glu Ala Val Glu Arg Glu Ile
465                 470                 475                 480

Gln Arg Leu Lys Thr Thr Trp Tyr Thr Pro Gln Lys Leu Ala Glu Gly
            485                 490                 495

Glu Gln Ile Arg Val Phe Gly Gln Lys Leu Ser Arg Glu Ala Asn Leu
            500                 505                 510

His Asp Leu Leu Arg Arg Pro Asn Leu Asp Tyr Ala Ala Leu Met Thr
            515                 520                 525

Leu Glu Gly Ala Met Pro Ser Glu Asn Leu Ser Ala Glu Val Ile Glu
    530                 535                 540
```

```
Gln Val Glu Ile Gln Val Lys Tyr Gln Gly Tyr Ile Asp Arg Gln Asn
545             550             555                         560

Glu Glu Ile Asp Ser Arg Arg Asp Ile Glu Thr Leu Lys Leu Pro Asp
                565             570                 575

Gly Ile Asp Tyr Gly Lys Val Lys Gly Leu Ser Ala Glu Val Gln Gln
            580             585                 590

Lys Leu Asn Gln His Lys Pro Glu Thr Val Gly Gln Ala Ser Arg Ile
            595             600             605

Ser Gly Val Thr Pro Ala Ala Val Ala Leu Leu Met Val His Leu Lys
        610             615             620

Arg Gly Phe Lys Asp Ala Lys
625             630
```

<210> 33
<211> 919
<212> PRT
<213> Neisseria meningitidis

<400> 33

Met Lys Arg Met Leu Phe Asn Ala Thr Gln Ala Glu Glu Leu Arg Val
1               5                   10                  15

Ala Ile Val Asp Gly Gln Asn Leu Leu Asp Leu Asp Ile Glu Thr Leu
            20                  25                  30

Gly Lys Glu Gln Arg Lys Gly Asn Ile Tyr Lys Gly Ile Ile Thr Arg
        35                  40                  45

Ile Glu Pro Ser Leu Glu Ala Cys Phe Val Asp Tyr Gly Thr Asp Arg
    50                  55                  60

His Gly Phe Leu Pro Phe Lys Glu Val Ser Arg Ser Tyr Phe Gln Asp
65                  70                  75                  80

Tyr Glu Gly Gly Arg Ala Arg Ile Gln Asp Val Leu Lys Glu Gly Met
                85                  90                  95

Glu Val Ile Val Gln Val Glu Lys Asp Glu Arg Gly Asn Lys Gly Ala
            100                 105                 110

Ala Leu Thr Thr Phe Ile Ser Leu Ala Gly Arg Tyr Leu Val Leu Met
        115                 120                 125

Pro Asn Asn Pro Arg Gly Gly Gly Val Ser Arg Arg Ile Glu Gly Glu
    130                 135                 140

Glu Arg Gln Glu Leu Lys Ala Ala Met Ala Glu Leu Asp Ile Pro Asn
145                 150                 155                 160

Gly Met Ser Ile Ile Ala Arg Thr Ala Gly Ile Gly Arg Ser Ala Glu
                165                 170                 175

Glu Leu Glu Trp Asp Leu Asn Tyr Leu Lys Gln Leu Trp Gln Ala Ile
            180                 185                 190

Glu Glu Ala Gly Lys Ala His His Asp Pro Tyr Leu Leu Phe Met Glu
        195                 200                 205

Ser Ser Leu Leu Ile Arg Ala Ile Arg Asp Tyr Phe Arg Pro Asp Ile
    210                 215                 220

Gly Glu Ile Leu Val Asp Asn Gln Glu Val Tyr Asp Gln Val Ala Glu
225                 230                 235                 240

Phe Met Ser Tyr Val Met Pro Gly Asn Ile Gly Arg Leu Lys Leu Tyr

|     | 245 |     |     |     | 250 |     |     |     | 255 |     |
| --- | --- | --- | --- | --- | --- | --- | --- | --- | --- | --- |

Glu Asp His Thr Pro Leu Phe Ser Arg Phe Gln Ile Glu His Gln Ile
260 265 270

Glu Ser Ala Phe Ser Arg Ser Val Ser Leu Pro Ser Gly Gly Ala Ile
275 280 285

Val Ile Asp His Thr Glu Ala Leu Val Ser Ile Asp Val Asn Ser Ala
290 295 300

Arg Ala Thr Arg Gly Ala Asp Ile Glu Asp Thr Ala Phe Lys Thr Asn
305 310 315 320

Met Glu Ala Ala Glu Glu Val Ala Arg Gln Met Arg Leu Arg Asp Leu
325 330 335

Gly Gly Leu Val Val Ile Asp Phe Ile Asp Met Glu Asn Pro Lys His
340 345 350

Gln Arg Asp Val Glu Asn Val Leu Arg Asp Ala Leu Lys Lys Asp Arg
355 360 365

Ala Arg Val Gln Met Gly Lys Leu Ser Arg Phe Gly Leu Leu Glu Leu
370 375 380

Ser Arg Gln Arg Leu Lys Pro Ala Leu Gly Glu Ser Ser His Val Ala
385 390 395 400

Cys Pro Arg Cys Ala Gly Thr Gly Val Ile Arg Gly Ile Glu Ser Thr
405 410 415

Ala Leu His Val Leu Arg Ile Ile Gln Glu Glu Ala Met Lys Asp Asn
420 425 430

Thr Gly Glu Val Arg Ala Gln Val Pro Val Asp Val Ala Thr Phe Leu
435 440 445

Leu Asn Glu Lys Arg Ala Glu Leu Phe Ala Met Glu Glu Arg Leu Asp
450 455 460

Val Asn Val Val Leu Ile Pro Asn Ile His Leu Glu Asn Pro His Tyr
465 470 475 480

Glu Ile Asn Arg Ile Arg Thr Asp Asp Val Glu Glu Asp Gly Glu Pro
485 490 495

Ser Tyr Lys Arg Val Ala Glu Pro Glu Glu Asp Glu Ser Ala Lys Pro
500 505 510

Phe Gly Gly Glu Lys Ala Lys Ala Ala Arg Pro Glu Pro Ala Val Lys
515 520 525

Gly Val Arg His Thr Ser Pro Ala Pro Thr Ala Ala Pro Glu Lys Lys
530 535 540

Thr Ser Trp Trp Asp Ser Phe Lys Ala Trp Leu Lys Arg Ile Phe Gly
545 550 555 560

Gly Ser Glu Thr Gln Ala Ala Pro Ala Ala Glu Thr Ser Glu Lys Arg
565 570 575

Ser Thr Ala Asn Arg Ser Gly Ser Arg Ala Asn Asn Arg Arg Gln Asn
580 585 590

Pro Arg Arg Ser Lys Arg Glu Gly Ser Lys Val Glu Val Arg Glu Val
595 600 605

Ala Gly Lys Thr Ala Gly Gln Glu Ala Arg Ala Asp Lys Ala Glu Thr
610 615 620

```
Arg Asn Asn Gly Asn Arg Arg Arg Asn Glu Arg Gly Asp Arg Ala Ala
625             630             635             640

Glu Arg Ala Asn Glu Ala Glu Ile Gln Ser Arg Asn Val Gln Pro Ala
            645             650             655

Ala Thr Val Ala Asp Ala Ala Pro Ser Glu Thr Glu Val Gln Thr Gly
            660             665             670

Lys Arg Arg Arg Asn Gly Ser Arg Ser Glu Arg Gly Gln Thr Ala Pro
            675             680             685

Glu Thr Ala Thr Val Ala Glu Thr Thr Val Gln Thr Ala Glu Asn Thr
    690             695             700

Pro Ser Glu Pro His Thr Ala Glu Asp Lys Gly Ser Lys Pro Lys Ser
705             710             715             720

Glu Arg Asn Arg Arg Glu Arg Asp Ser Arg Asp Ala Lys Glu Arg Arg
            725             730             735

Glu Arg Asn Asn Gln Arg Asp Arg Arg Gln Asn Gly Lys Lys Arg Asn
            740             745             750

Ile Pro Ser Ala Ala Lys Ile Glu Gln Tyr Leu Asn Ile His Asp Thr
            755             760             765

Ala Asp Lys Val Arg Ser Ala Ala Ala His Val Phe Gly Glu Thr Asp
    770             775             780

Ala Asn Ala Pro Ile Thr Val Ser Ile Ala Asp Pro Val Ala Glu Arg
785             790             795             800

Asp Leu Pro Thr Ala Ser Pro Ala Val Ser Asn Gly Asp Ala Pro Val
            805             810             815

Tyr Asp Ala Ala Glu Lys Ile Arg Arg Ala Thr Ala Ala Ile Leu Pro
            820             825             830

Glu Gly Ala Thr Pro Lys Ala Glu Ala Gln Glu Met Pro Ser Glu Thr
            835             840             845

Ala Thr Phe Thr Ala Ala Ala Glu Gln Ala Arg Glu Thr Ala Gln Thr
    850             855             860

Gly Gly Leu Val Leu Ile Glu Thr Asp Pro Ala Ala Leu Lys Ala Trp
865             870             875             880

Ala Ala Gln Pro Glu Val Gln Ala Gly Arg Gly Leu Arg Arg Ser Glu
            885             890             895

Gln Pro Lys Pro Ser Glu Val Ala Thr Val Pro Ala Glu Glu Met Ile
            900             905             910

Gln Val Glu Thr Arg Gln Gly
            915
```

<210> 34
<211> 796
<212> PRT
<213> Neisseria meningitidis

<400> 34

Met Thr Glu Gln Lys His Glu Glu Tyr Gly Ala Asp Ser Ile Gln Val
1                   5                   10                  15

Leu Glu Gly Leu Glu Ala Val Arg Lys Arg Pro Gly Met Tyr Ile Gly
                20                  25                  30

Asp Thr Gln Asp Gly Ser Gly Leu His His Met Val Phe Glu Val Leu
35                    40                    45

Asp Asn Ala Ile Asp Glu Ala Leu Ala Gly His Cys Asp Lys Ile Thr
50                    55                    60

Val Thr Ile His Ala Asp His Ser Val Ser Val Ala Asp Asn Gly Arg
65                    70                    75                    80

Gly Met Pro Thr Gly Ile His Pro Lys Glu Gly Arg Ser Ala Ala Glu
85                    90                    95

Val Ile Met Thr Val Leu His Ala Gly Gly Lys Phe Asp Asn Asn Ser
100                   105                   110

Tyr Lys Ile Ser Gly Gly Leu His Gly Val Gly Val Ser Val Val Asn
115                   120                   125

Ala Leu Ser Asp Trp Val Thr Leu Thr Ile Tyr Arg Asp Gly Lys Glu
130                   135                   140

His Phe Val Arg Phe Val Arg Gly Glu Thr Glu Glu Pro Leu Lys Ile
145                   150                   155                   160

Val Gly Asp Ser Asp Lys Lys Gly Thr Thr Val Arg Phe Leu Ala Ser
165                   170                   175

Thr Glu Thr Phe Gly Asn Val Glu Tyr Ser Phe Asp Ile Leu Ala Lys
180                   185                   190

Arg Ile Arg Glu Leu Ser Phe Leu Asn Asn Gly Val Asp Ile Glu Leu
195                   200                   205

Thr Asp Glu Arg Asp Gly Lys His Glu Ser Phe Ala Leu Ser Gly Gly
210                   215                   220

Val Ala Gly Phe Val Gln Tyr Met Asn Arg Lys Lys Thr Pro Leu His
225                   230                   235                   240

Glu Lys Ile Phe Tyr Ala Phe Gly Glu Lys Asp Gly Met Ser Val Glu
245                   250                   255

Cys Ala Met Gln Trp Asn Asp Ser Tyr Gln Glu Ser Val Gln Cys Phe
260                   265                   270

Thr Asn Asn Ile Pro Gln Arg Asp Gly Gly Thr His Leu Thr Ala Leu
275                   280                   285

Arg Gln Val Met Thr Arg Thr Ile Asn Asn Tyr Ile Glu Ala Asn Glu
290                   295                   300

Val Ala Lys Lys Ala Lys Val Glu Thr Ala Gly Asp Asp Met Arg Glu
305                   310                   315                   320

Gly Leu Thr Cys Val Leu Ser Val Lys Leu Pro Asp Pro Lys Phe Ser
325                   330                   335

Ser Gln Thr Lys Asp Lys Leu Val Ser Gly Glu Ile Gly Pro Val Val
340                   345                   350

Asn Glu Val Ile Ser Gln Ala Leu Thr Asp Phe Leu Glu Glu Asn Pro
355                   360                   365

Asn Glu Ala Lys Ile Ile Thr Gly Lys Ile Val Asp Ala Ala Arg Ala
370                   375                   380

Arg Glu Ala Ala Arg Lys Ala Arg Glu Ile Thr Arg Arg Lys Gly Val
385                   390                   395                   400

Met Asp Gly Leu Gly Leu Pro Gly Lys Leu Ala Asp Cys Gln Glu Lys

```
                    405                     410                     415
        Asp Pro Ala Leu Ser Glu Leu Tyr Leu Val Glu Gly Asp Ser Ala Gly
                    420                 425                 430

        Gly Ser Ala Met Gln Gly Arg Asp Arg Lys Phe Gln Ala Ile Leu Pro
                    435             440                 445

        Leu Lys Gly Lys Ile Leu Asn Val Glu Lys Ala Arg Phe Glu Lys Met
                450                 455                 460

        Leu Ala Ser Gln Glu Val Ala Thr Leu Ile Thr Ala Leu Gly Ala Gly
        465                 470                 475                 480

        Ile Gly Lys Glu Glu Phe Asn Ala Glu Lys Leu Arg Tyr His Arg Ile
                        485                 490                 495

        Ile Ile Met Thr Asp Ala Asp Val Asp Gly Ala His Ile Arg Thr Leu
                    500                 505                 510

        Leu Leu Thr Phe Phe Tyr Arg Gln Met Pro Glu Leu Val Glu Arg Gly
                    515                 520                 525

        Tyr Ile Tyr Ile Ala Gln Pro Pro Leu Tyr Lys Ala Lys Tyr Gly Lys
                530                 535                 540

        Gln Glu Arg Tyr Leu Lys Asp Glu Leu Glu Lys Asp Gln Trp Leu Leu
        545                 550                 555                 560

        Gly Leu Ala Leu Glu Lys Ala Lys Ile Ile Ser Asp Gly Arg Thr Ile
                        565                 570                 575

        Glu Gly Ala Glu Leu Ala Asp Thr Ala Lys Gln Phe Leu Leu Ala Lys
                    580                 585                 590

        Thr Val Ile Glu Gln Glu Ser Arg Phe Val Asp Glu Leu Val Leu Arg
                    595                 600                 605

        Ala Met Leu His Ala Ser Pro Ile Asp Leu Thr Ser Ser Glu Asn Ala
            610                 615                 620

        Asp Lys Ala Val Ala Glu Leu Ser Gly Leu Leu Asp Glu Lys Glu Val
        625                 630                 635                 640

        Ala Leu Glu Arg Ile Glu Gly His Glu Gly His Arg Phe Ile Lys Ile
                        645                 650                 655

        Thr Arg Lys Leu His Gly Asn Val Met Val Ser Tyr Ile Glu Pro Lys
                    660                 665                 670

        Phe Leu Asn Ser Lys Ala Tyr Gln Thr Leu Thr Gln Thr Ala Ala Ala
                    675                 680                 685

        Leu Lys Gly Met Val Gly Glu Gly Ala Lys Leu Tyr Lys Gly Glu Asn
                690                 695                 700

        Gly Tyr Asp Ala Asp Ser Phe Glu Thr Ala Leu Asp Ile Leu Met Ser
        705                 710                 715                 720

        Val Ala Gln Lys Gly Met Ser Ile Gln Arg Tyr Lys Gly Leu Gly Glu
                        725                 730                 735

        Met Asn Pro Glu Gln Leu Trp Glu Thr Thr Met Asp Pro Ala Val Arg
                    740                 745                 750

        Arg Leu Leu Lys Val Arg Ile Glu Asp Ala Ile Ala Ala Asp Glu Val
                    755                 760                 765

        Phe Val Thr Leu Met Gly Asp Glu Val Glu Pro Arg Arg Ala Phe Ile
                770                 775                 780
```

97

```
Glu Asn Asn Ala Leu Ile Ala Gln Asn Ile Asp Ala
    785                 790                 795
```

<210> 35
<211> 415
<212> PRT
<213> Neisseria meningitidis

<400> 35

```
Met Ser Gln Arg Arg Val Val Ile Thr Gly Leu Gly Gln Val Ser Pro
1           5               10              15

Val Gly Asn Thr Val Ala Glu Ala Trp Asp Thr Leu Leu Thr Gly Lys
            20              25              30

Ser Gly Ile Gly Ala Ile Thr Arg Phe Asp Thr Ser Asp Ile Asn Ser
        35              40              45

Arg Val Ala Gly Glu Val Arg Gly Phe Asp Ile Gly Gln Tyr Ile Ser
    50              55              60

Ala Lys Glu Ala Arg Arg Met Asp Val Phe Ile His Tyr Gly Ile Ala
65              70              75              80

Ala Ala Leu Gln Ala Ile Ala Asp Ser Gly Leu Asp Asp Val Glu Asn
            85              90              95

Leu Asp Lys Asp Arg Ile Gly Val Asn Ile Gly Ser Gly Ile Gly Gly
        100             105             110

Leu Pro Gly Ile Glu Val Thr Gly Lys Ala Val Ile Glu Gly Gly Ala
        115             120             125

Arg Lys Ile Asn Pro Phe Phe Ile Pro Gly Ser Leu Ile Asn Leu Ile
    130             135             140

Ser Gly His Val Thr Ile Leu Lys Gly Tyr Arg Gly Pro Ser Tyr Gly
145             150             155             160

Met Val Ser Ala Cys Thr Thr Gly Ala His Ala Ile Gly Asp Ser Leu
            165             170             175

Arg Met Ile Lys Tyr Gly Asp Ala Asp Ile Met Val Ala Gly Gly Ala
        180             185             190

Glu Gly Ala Ile Ser Thr Leu Gly Val Gly Gly Phe Ala Ala Met Lys
    195             200             205

Ala Leu Ser Thr Arg Asn Asp Asp Pro Ala Thr Ala Ser Arg Pro Trp
    210             215             220

Asp Lys Gly Arg Asp Gly Phe Val Ile Gly Glu Gly Ala Gly Ile Leu
225             230             235             240

Val Leu Glu Glu Leu Glu His Ala Lys Lys Arg Gly Ala Lys Ile Tyr
        245             250             255

Ala Glu Ile Val Gly Phe Gly Met Ser Ser Asp Ala Tyr His Ile Thr
        260             265             270

Ala Pro Asn Glu Glu Gly Pro Ala Leu Ala Val Thr Arg Ala Leu Lys
        275             280             285

Asp Ala Gly Ile Asn Pro Glu Asp Val Asp Tyr Val Asn Ala His Gly
    290             295             300

Thr Ser Thr Pro Leu Gly Asp Ala Asn Glu Thr Lys Ala Leu Lys Arg
305             310             315             320
```

```
Ala Phe Gly Glu His Ala Tyr Lys Thr Val Val Ser Ser Thr Lys Ser
                325                 330                 335

Met Thr Gly His Leu Leu Gly Ala Ala Gly Gly Val Glu Ala Val Tyr
            340             345                 350

Ser Ile Leu Ala Ile His Asp Gly Lys Ile Pro Pro Thr Ile Asn Ile
        355             360                 365

Phe Glu Gln Asp Val Glu Ala Gly Cys Asp Leu Asp Tyr Cys Ala Asn
    370                 375             380

Glu Ala Arg Asp Ala Glu Ile Asp Val Ala Ile Ser Asn Ser Phe Gly
385                 390                 395                 400

Phe Gly Gly Thr Asn Gly Thr Leu Val Phe Lys Arg Phe Lys Gly
            405             410                 415
```

<210> 36
<211> 433
<212> PRT
<213> Neisseria meningitidis

<400> 36

**100**

```
Met Ala Ile Tyr Gln Ser Gly Val Ile Phe Asp Gln Val Asp Thr Ala
1               5               10              15

Asn Pro Asp Cys Trp Thr Leu Asp Glu Tyr Val Lys Arg Gly Gly Tyr
            20              25              30

Thr Ala Leu Arg Lys Ile Leu Ser Glu Asn Ile Ser Gln Thr Asp Val
        35              40              45

Ile Asp Glu Val Lys Thr Ser Gly Leu Arg Gly Arg Gly Gly Ala Gly
    50              55              60

Phe Pro Thr Gly Leu Lys Trp Ser Phe Met Pro Arg Ser Phe Pro Gly
65              70              75              80

Glu Lys Tyr Val Val Cys Asn Thr Asp Glu Gly Glu Pro Gly Thr Phe
            85              90              95

Lys Asp Arg Asp Ile Ile Met Phe Asn Pro His Ala Leu Ile Glu Gly
        100             105             110

Met Ile Ile Ala Gly Tyr Ala Met Gly Ala Lys Ala Gly Tyr Asn Tyr
        115             120             125

Ile His Gly Glu Ile Phe Glu Gly Tyr Gln Arg Phe Glu Ala Ala Leu
    130             135             140

Glu Gln Ala Arg Ala Ala Gly Phe Leu Gly Lys Asn Ile Leu Gly Ser
145             150             155             160

Asp Phe Glu Phe Glu Leu Phe Ala His His Gly Tyr Gly Ala Tyr Ile
            165             170             175

Cys Gly Glu Glu Thr Ala Leu Leu Glu Ser Leu Glu Gly Lys Lys Gly
        180             185             190

Gln Pro Arg Phe Lys Pro Pro Phe Pro Ala Ser Phe Gly Leu Tyr Gly
        195             200             205

Lys Pro Thr Thr Ile Asn Asn Thr Glu Thr Phe Ser Ser Val Pro Phe
    210             215             220

Ile Ile Arg Asp Gly Gly Gln Ala Phe Ala Asp Lys Gly Ile Pro Asn
225             230             235             240

Ala Gly Gly Thr Lys Leu Phe Cys Ile Ser Gly His Val Glu Arg Pro
```

```
                    245                 250                 255
Gly Asn Tyr Glu Val Pro Leu Gly Thr Pro Phe Ala Glu Val Leu Lys
            260             265             270

Met Ala Gly Gly Met Arg Gly Gly Lys Lys Leu Lys Ala Val Ile Pro
        275             280             285

Gly Gly Ser Ser Ala Pro Val Leu Pro Ala Asp Ile Met Met Gln Thr
    290             295             300

Asn Met Asp Tyr Asp Ser Ile Ser Lys Ala Gly Ser Met Leu Gly Ser
305             310             315             320

Gly Ala Ile Ile Val Met Asp Glu Asp Val Cys Met Val Lys Ala Leu
            325             330             335

Glu Arg Leu Ser Tyr Phe Tyr Tyr Asp Glu Ser Cys Gly Gln Cys Thr
        340             345             350

Pro Cys Arg Glu Gly Thr Gly Trp Leu Tyr Arg Ile Val His Arg Ile
        355             360             365

Val Glu Gly Lys Gly Lys Met Glu Asp Leu Asp Leu Leu Asp Ser Val
    370             375             380

Gly Asn Gln Met Ala Gly Arg Thr Ile Cys Ala Leu Ala Asp Ala Ala
385             390             395             400

Val Phe Pro Val Arg Ser Phe Thr Lys His Phe Arg Asp Glu Phe Val
            405             410             415

His Tyr Ile Glu His Gly Gly Pro Met Lys Glu His Lys Trp Gly Gly
            420             425             430

Trp
```

<210> 37
<211> 204
<212> PRT
<213> Neisseria meningitidis

<400> 37

```
Met Asn Phe Thr Arg Leu Leu Asn Gln Val Leu Ser Thr Val Gln Lys
1               5               10              15

Lys Gly Asn Thr Phe Ser Asp Ser Pro Leu Asn Ser Phe Gly Gly Gly
            20              25              30

Ala Leu Val Ala Gly Val Ala Ser Met Leu Leu Asn Gly Lys Asn Arg
            35              40              45

Lys Thr Ile Thr Lys Ile Gly Ser Thr Ala Ala Leu Gly Tyr Leu Ala
    50              55              60

Tyr Arg Gly Tyr Gln Met Trp Gln Gln Asn Lys Gly Arg Ala Thr Val
65              70              75              80

Thr Gln Ser Asp Phe Gln Pro Ala Gly Glu Thr Glu Glu Thr Tyr Ser
            85              90              95

Arg Thr Val Leu Arg Thr Met Ile Ala Ala Ala Ala Ser Asp Gly Met
            100             105             110

Ile Asp Glu Ala Glu Arg Arg Thr Ile Glu Gln Glu Ser Gly Thr Asp
            115             120             125

Pro Glu Thr Ala Ala Trp Leu Ala Ala Glu Tyr Arg Leu Pro Ala Ser
    130             135             140

Ile Glu Asp Ile Ala Ala Ala Val Gly Asn Asp Glu Ala Leu Ala Ala
145             150             155             160

Glu Ala Tyr Leu Ala Ala Arg Leu Val Cys Ala Asp Leu Ser Arg Lys
            165             170             175

Glu Thr Val Phe Leu Ala Arg Leu Ser Gln Ala Leu Lys Leu Asp Asp
            180             185             190

Asn Leu Val Glu Ser Leu Glu Arg Gln Leu Gly Phe
            195             200
```

<210> 38
<211> 231
<212> PRT
<213> Neisseria meningitidis

<400> 38

```
Met Lys Leu Lys Thr Leu Ala Leu Thr Ser Leu Thr Leu Leu Ala Leu
1               5                   10                  15
Ala Ala Cys Ser Lys Gln Ala Glu Thr Ser Val Pro Ala Asp Ser Ala
            20                  25                  30
Gln Ser Ser Ser Ser Ala Pro Ala Ala Pro Ala Glu Leu Asn Glu Gly
        35                  40                  45
Val Asn Tyr Thr Val Leu Ser Thr Pro Ile Pro Gln Gln Gln Ala Gly
        50              55                  60
Lys Ile Glu Val Leu Glu Phe Phe Gly Tyr Phe Cys Pro His Cys Ala
65              70                  75                      80
His Leu Glu Pro Val Leu Ser Glu His Ile Lys Thr Phe Lys Asp Asp
                85                  90                  95
Thr Tyr Met Arg Arg Glu His Val Val Trp Gly Asp Glu Met Lys Pro
            100                 105                 110
Leu Ala Arg Leu Ala Ala Ala Val Glu Met Ala Gly Glu Ser Asp Lys
            115                 120                 125
Ala Asn Ser His Ile Phe Asp Ala Met Val Asn Gln Lys Ile Asn Leu
    130             135                 140
Ala Asp Thr Asp Thr Leu Lys Lys Trp Leu Ser Glu Gln Thr Ala Phe
145                 150                 155                 160
Asp Gly Lys Lys Val Leu Ala Ala Phe Glu Ala Pro Glu Ser Gln Ala
                165                 170                 175
Arg Ala Ala Gln Met Glu Glu Leu Thr Asn Lys Phe Gln Ile Ser Gly
            180                 185                 190
Thr Pro Thr Val Ile Val Gly Gly Lys Tyr Gln Val Glu Phe Lys Asp
        195                 200                 205
Trp Gln Ser Gly Met Thr Thr Ile Asp Gln Leu Val Asp Lys Val Arg
    210                 215                 220
Glu Glu Gln Lys Lys Pro Gln
225                 230
```

<210> 39
<211> 558
<212> PRT
<213> Neisseria meningitidis

<400> 39

```
Met Ser Val Gly Leu Leu Arg Ile Leu Val Gln Asn Gln Val Val Thr
1               5                   10              15

Val Glu Gln Ala Glu His Tyr Tyr Asn Glu Ser Gln Ala Gly Lys Glu
            20              25              30

Val Leu Pro Met Leu Phe Ser Asp Gly Val Ile Ser Pro Lys Ser Leu
        35              40              45

Ala Ala Leu Ile Ala Arg Val Phe Ser Tyr Ser Ile Leu Asp Leu Arg
    50              55              60

His Tyr Pro Arg His Arg Val Leu Met Gly Val Leu Thr Glu Glu Gln
65              70              75              80

Met Val Glu Phe His Cys Val Pro Val Phe Arg Arg Gly Asp Lys Val
            85              90              95

Phe Phe Ala Val Ser Asp Pro Thr Gln Met Pro Gln Ile Gln Lys Thr
        100             105             110

Val Ser Ala Ala Gly Ile Glu Val Glu Leu Val Ile Val Glu Asp Asp
        115             120             125

Gln Leu Ala Gly Leu Leu Asp Trp Val Gly Ser Arg Ser Thr Ser Leu
    130             135             140

Leu Gln Glu Leu Gly Glu Gly Gln Glu Glu Glu Ser His Thr Leu
145             150             155             160

Tyr Ile Asp Asn Glu Glu Ala Glu Asp Gly Pro Val Pro Arg Phe Ile
            165             170             175

His Lys Thr Leu Ser Asp Ala Leu Arg Ser Gly Ala Ser Asp Ile His
        180             185             190

Phe Glu Phe Tyr Glu His Asn Ala Arg Ile Arg Phe Arg Val Asp Gly
    195             200             205

Gln Leu Arg Glu Val Val Gln Pro Pro Ile Ala Val Arg Gly Gln Leu
    210             215             220

Ala Ser Arg Ile Lys Val Met Ser Arg Leu Asp Ile Ser Glu Lys Arg
225             230             235             240

Ile Pro Gln Asp Gly Arg Met Gln Leu Thr Phe Gln Lys Gly Gly Lys
            245             250             255

Pro Val Asp Phe Arg Val Ser Thr Leu Pro Thr Leu Phe Gly Glu Lys
        260             265             270

Val Val Met Arg Ile Leu Asn Ser Asp Ala Ala Ser Leu Asn Ile Asp
        275             280             285

Gln Leu Gly Phe Glu Pro Phe Gln Lys Lys Leu Leu Leu Glu Ala Ile
    290             295             300

His Arg Pro Tyr Gly Met Val Leu Val Thr Gly Pro Thr Gly Ser Gly
305             310             315             320

Lys Thr Val Ser Leu Tyr Thr Cys Leu Asn Ile Leu Asn Thr Glu Ser
            325             330             335

Val Asn Ile Ala Thr Ala Glu Asp Pro Ala Glu Ile Asn Leu Pro Gly
        340             345             350

Ile Asn Gln Val Asn Val Asn Asp Lys Gln Gly Leu Thr Phe Ala Ala
    355             360             365

Ala Leu Lys Ser Phe Leu Arg Gln Asp Pro Asp Ile Ile Met Val Gly
```

```
            370                 375                 380
Glu Ile Arg Asp Leu Glu Thr Ala Asp Ile Ala Ile Lys Ala Ala Gln
385                 390                 395                 400
Thr Gly His Met Val Phe Ser Thr Leu His Thr Asn Asn Ala Pro Ala
                405                 410                 415
Thr Leu Ser Arg Met Leu Asn Met Gly Val Ala Pro Phe Asn Ile Ala
            420                 425                 430
Ser Ser Val Ser Leu Ile Met Ala Gln Arg Leu Leu Arg Arg Leu Cys
        435                 440                 445
Ser Ser Cys Lys Gln Glu Val Glu Arg Pro Ser Ala Ser Ala Leu Lys
    450                 455                 460
Glu Val Gly Phe Thr Asp Glu Asp Leu Ala Lys Asp Trp Lys Leu Tyr
465                 470                 475                 480
Arg Ala Val Gly Cys Asp Arg Cys Arg Gly Gln Gly Tyr Lys Gly Arg
            485                 490                 495
Ala Gly Val Tyr Glu Val Met Pro Ile Ser Glu Glu Met Gln Arg Val
        500                 505                 510
Ile Met Asn Asn Gly Thr Glu Val Asp Ile Leu Asp Val Ala Tyr Lys
        515                 520                 525
Glu Gly Met Val Asp Leu Arg Arg Ala Gly Ile Leu Lys Val Met Gln
    530                 535                 540
Gly Ile Thr Ser Leu Glu Glu Val Thr Ala Asn Thr Asn Asp
545                 550                 555
```

<210> 40
<211> 273
<212> PRT
<213> Neisseria meningitidis

<400> 40

```
Met Ser Leu Gln Asn Ile Ile Glu Thr Ala Phe Glu Asn Arg Ala Asp
1               5               10              15

Ile Thr Pro Thr Thr Val Thr Pro Glu Val Lys Glu Ala Val Leu Glu
        20              25              30

Thr Ile Arg Gln Leu Asp Ser Gly Lys Leu Arg Val Ala Glu Arg Leu
        35              40              45

Gly Val Gly Glu Trp Lys Val Asn Glu Trp Ala Lys Lys Ala Val Leu
    50              55              60

Leu Ser Phe Arg Ile Gln Asp Asn Glu Val Leu Asn Asp Gly Val Asn
65              70              75              80

Lys Tyr Phe Asp Lys Val Pro Thr Lys Phe Ala Asp Trp Ser Glu Asp
            85              90              95

Glu Phe Lys Asn Ala Gly Phe Arg Ala Val Pro Gly Ala Val Ala Arg
        100             105             110

Arg Gly Ser Phe Val Ala Lys Asn Val Val Leu Met Pro Ser Tyr Val
        115             120             125

Asn Ile Gly Ala Tyr Val Asp Glu Gly Ala Met Val Asp Thr Trp Ala
    130             135             140

Thr Val Gly Ser Cys Ala Gln Ile Gly Lys Asn Val His Leu Ser Gly
145             150             155             160

Gly Val Gly Ile Gly Gly Val Leu Glu Pro Leu Gln Ala Ala Pro Thr
            165             170             175

Ile Ile Glu Asp Asn Cys Phe Ile Gly Ala Arg Ser Glu Ile Val Glu
        180             185             190

Gly Val Ile Val Glu Glu Gly Ser Val Ile Ser Met Gly Val Phe Ile
        195             200             205

Gly Gln Ser Thr Lys Ile Phe Asp Arg Thr Thr Gly Glu Ile Tyr Gln
    210             215             220

Gly Arg Val Pro Ala Gly Ser Val Val Val Ser Gly Ser Met Pro Ser
225             230             235             240

Lys Asp Gly Ser His Ser Leu Tyr Cys Ala Val Ile Val Lys Arg Val
            245             250             255

Asp Ala Gln Thr Arg Ala Lys Thr Ser Val Asn Glu Leu Leu Arg Gly
        260             265             270

Ile
```

<210> 41
<211> 261
<212> PRT
<213> Neisseria meningitidis

<400> 41

```
Met Gly Phe Leu Gln Gly Lys Lys Ile Leu Ile Thr Gly Met Ile Ser
1               5                   10                  15

Glu Arg Ser Ile Ala Tyr Gly Ile Ala Lys Ala Cys Arg Glu Gln Gly
            20              25                  30

Ala Glu Leu Ala Phe Thr Tyr Val Val Asp Lys Leu Glu Glu Arg Val
        35                  40                  45

Arg Lys Met Ala Ala Glu Leu Asp Ser Glu Leu Val Phe Arg Cys Asp
    50                  55                  60

Val Ala Ser Asp Asp Glu Ile Asn Gln Val Phe Ala Asp Leu Gly Lys
65                  70                  75                  80

His Trp Asp Gly Leu Asp Gly Leu Val His Ser Ile Gly Phe Ala Pro
            85                  90                  95

Lys Glu Ala Leu Ser Gly Asp Phe Leu Asp Ser Ile Ser Arg Glu Ala
            100                 105                 110

Phe Asn Thr Ala His Glu Ile Ser Ala Tyr Ser Leu Pro Ala Leu Ala
        115                 120                 125

Lys Ala Ala Arg Pro Met Met Arg Gly Arg Asn Ser Ala Ile Val Ala
    130             135                 140

Leu Ser Tyr Leu Gly Ala Val Arg Ala Ile Pro Asn Tyr Asn Val Met
145             150                 155                 160

Gly Met Ala Lys Ala Ser Leu Glu Ala Gly Ile Arg Phe Thr Ala Ala
            165                 170                 175

Cys Leu Gly Lys Glu Gly Ile Arg Cys Asn Gly Ile Ser Ala Gly Pro
        180                 185                 190

Ile Lys Thr Leu Ala Ala Ser Gly Ile Ala Asp Phe Gly Lys Leu Leu
    195                 200                 205

Gly His Val Ala Ala His Asn Pro Leu Arg Arg Asn Val Thr Ile Glu
    210                 215                 220

Glu Val Gly Asn Thr Ala Ala Phe Leu Leu Ser Asp Leu Ser Ser Gly
225                 230                 235                 240

Ile Thr Gly Glu Ile Thr Tyr Val Asp Gly Gly Tyr Ser Ile Asn Ala
            245                 250                 255

Leu Ser Thr Glu Gly
            260
```

<210> 42
<211> 332
<212> PRT
<213> Neisseria meningitidis

<400> 42

```
Met Ser Arg Pro Val Pro Ala Val Phe Gly Ser Val Phe His Ser Gln
1               5               10              15

Met Pro Val Leu Ala Tyr Arg Glu Gly Lys Trp Gln Pro Thr Glu Trp
            20              25              30

Gln Ser Ser Gln Asp Leu Ser Leu Ala Pro Gly Ala His Ala Leu His
        35              40              45

Tyr Gly Ser Glu Cys Phe Glu Gly Leu Lys Ala Phe Arg Gln Ala Asp
    50              55              60

Gly Lys Ile Val Leu Phe Arg Pro Thr Ala Asn Ile Ala Arg Met Arg
65              70              75              80

Gln Ser Ala Asp Ile Leu His Leu Pro Arg Pro Glu Thr Glu Ala Tyr
            85              90              95

Leu Asp Ala Leu Ile Lys Leu Val Lys Arg Ala Ala Asp Glu Ile Pro
            100             105             110

Asp Ala Pro Ala Ala Leu Tyr Leu Arg Pro Thr Leu Ile Gly Thr Asp
        115             120             125

Pro Val Ile Gly Lys Ala Gly Ser Pro Ser Glu Thr Ala Leu Leu Tyr
    130             135             140

Ile Leu Ala Ser Pro Val Gly Asp Tyr Phe Lys Val Gly Ser Pro Val
145             150             155             160

Lys Ile Leu Val Glu Thr Glu His Ile Arg Cys Ala Pro His Met Gly
            165             170             175

Arg Val Lys Cys Gly Gly Asn Tyr Ala Ser Ala Met His Trp Val Leu
        180             185             190

Lys Ala Lys Ala Glu Tyr Gly Ala Asn Gln Val Leu Phe Cys Pro Asn
        195             200             205

Gly Asp Val Gln Glu Thr Gly Ala Ser Asn Phe Ile Leu Ile Asn Gly
    210             215             220

Asp Glu Ile Ile Thr Lys Pro Leu Thr Asp Glu Phe Leu His Gly Val
225             230             235             240

Thr Arg Asp Ser Val Leu Thr Val Ala Lys Asp Leu Gly Tyr Thr Val
            245             250             255

Ser Glu Arg Asn Phe Thr Val Asp Glu Leu Lys Ala Ala Val Glu Asn
        260             265             270

Gly Ala Glu Ala Ile Leu Thr Gly Thr Ala Ala Val Ile Ser Pro Val

            275             280             285

Thr Ser Phe Val Ile Gly Gly Lys Glu Ile Glu Val Lys Ser Gln Glu
    290             295             300

Arg Gly Tyr Ala Ile Arg Lys Ala Ile Thr Asp Ile Gln Tyr Gly Leu
305             310             315             320

Ala Glu Asp Lys Tyr Gly Trp Leu Val Glu Val Cys
            325             330
```

<210> 43
<211> 351
<212> PRT

<213> Neisseria meningitidis

<400> 43

```
Met Thr Ile Leu Ser Asp Val Lys Ala Leu Gly Gln Gln Ile Trp Leu
1               5                   10                  15

Asp Asn Leu Ser Arg Ser Leu Val Gln Ser Gly Glu Leu Ala Gln Met
            20                  25                  30

Leu Lys Gln Gly Val Cys Gly Val Thr Ser Asn Pro Ala Ile Phe Gln
        35                  40                  45

Lys Ala Phe Ala Gly Asp Ala Leu Tyr Ala Asp Glu Ile Ala Ala Leu
    50                  55                  60

Lys Gln Gln Asn Leu Ser Pro Lys Gln Arg Tyr Glu Thr Met Ala Val
65                  70                  75                  80

Ala Asp Val Arg Ala Ala Cys Asp Val Cys Leu Ala Glu His Glu Ser
                85                  90                  95

Thr Gly Gly Lys Thr Gly Phe Val Ser Leu Glu Val Ser Pro Glu Leu
            100                 105                 110

Ser Lys Asp Ala Gln Gly Thr Val Glu Glu Ala Arg Arg Leu Tyr Ala
        115                 120                 125

Ala Ile Gly Cys Lys Asn Ala Met Ile Lys Val Pro Ala Thr Asp Ala
    130                 135                 140

Gly Ile Asp Ala Leu Glu Thr Leu Val Ser Asp Gly Ile Ser Val Asn
145                 150                 155                 160

Leu Thr Leu Leu Phe Ser Arg Ala Gln Thr Leu Lys Ala Tyr Ala Ala
                165                 170                 175

Tyr Ala Arg Gly Ile Ala Lys Arg Leu Ala Ala Gly Gln Ser Val Ala
            180                 185                 190

His Ile Gln Val Val Ala Ser Phe Phe Ile Ser Arg Val Asp Gly Ala
        195                 200                 205

Leu Asp Thr Thr Leu Pro Asp His Leu Lys Gly Lys Ile Ala Ile Ala
    210                 215                 220

Leu Ala Lys Ala Ala Tyr Gln Asp Trp Ala Gln Tyr Phe Gly Ser Pro
225                 230                 235                 240

Glu Phe Ala Ala Leu Glu Thr Lys Gly Ala Asn Arg Val Gln Leu Leu
                245                 250                 255

Trp Ala Ser Thr Gly Val Lys Asn Pro Ala Tyr Pro Asp Thr Leu Tyr
            260                 265                 270

Val Asp Ser Leu Ile Gly Ala His Thr Val Asn Thr Val Pro Asp Ala
            275                 280                 285
```

```
Thr Leu Lys Ala Phe Ile Asp His Gly Thr Ala Lys Ala Thr Leu Thr
    290             295             300

Glu Gly Val Glu Glu Ala Gln Ala Gln Leu Ala Glu Thr Ala Ala Leu
305             310             315             320

Gly Ile Asp Val Glu Thr Leu Ala Thr Arg Leu Gln Glu Asp Gly Leu
                325             330             335

Lys Gln Phe Glu Glu Ala Phe Glu Lys Leu Leu Ala Pro Leu Ala
            340             345             350
```

<210> 44
<211> 472
<212> PRT
<213> Neisseria meningitidis

<400> 44

```
Met Ser Ile Lys Asn Ala Val Lys Leu Ile Glu Glu Ser Glu Ala Arg
1               5                   10                  15

Phe Val Asp Leu Arg Phe Thr Asp Thr Lys Gly Lys Gln His His Phe
            20                  25                  30

Thr Val Pro Ala Arg Ile Val Leu Glu Asp Pro Glu Glu Trp Phe Glu
        35                  40                  45

Asn Gly Gln Ala Phe Asp Gly Ser Ser Ile Gly Gly Trp Lys Gly Ile
    50                  55                  60

Gln Ala Ser Asp Met Gln Leu Arg Pro Asp Ala Ser Thr Ala Phe Val
65                  70                  75                  80

Asp Pro Phe Tyr Asp Asp Ala Thr Val Val Leu Thr Cys Asp Val Ile
            85                  90                  95

Asp Pro Ala Asp Gly Gln Gly Tyr Asp Arg Asp Pro Arg Ser Ile Ala
            100                 105                 110

Arg Arg Ala Glu Ala Tyr Leu Lys Ser Ser Gly Ile Gly Glu Thr Ala
        115                 120                 125

Tyr Phe Gly Pro Glu Pro Glu Phe Phe Val Phe Asp Gly Ile Glu Phe
    130                 135                 140

Glu Thr Asp Met His Lys Thr Arg Tyr Glu Ile Thr Ser Glu Ser Gly
145                 150                 155                 160

Ala Trp Ala Ser Gly Leu His Met Asp Gly Gln Asn Thr Gly His Arg
            165                 170                 175

Pro Thr Val Lys Gly Gly Tyr Ala Pro Val Ala Pro Ile Asp Cys Gly
            180                 185                 190

Gln Asp Leu Arg Ser Ala Met Val Asn Ile Leu Glu Glu Leu Gly Ile
    195                 200                 205

Glu Val Glu Val His His Ser Glu Val Gly Thr Gly Ser Gln Met Glu
    210                 215                 220

Ile Gly Thr Arg Phe Ala Thr Leu Val Lys Arg Ala Asp Gln Thr Gln
225                 230                 235                 240

Asp Met Lys Tyr Val Ile Gln Asn Val Ala His Asn Phe Gly Lys Thr
            245                 250                 255

Ala Thr Phe Met Pro Lys Pro Ile Met Gly Asp Asn Gly Ser Gly Met
            260                 265                 270
```

```
His Val His Gln Ser Ile Trp Lys Asp Gly Gln Asn Leu Phe Ala Gly
        275                 280                 285

Asp Gly Tyr Ala Gly Leu Ser Asp Thr Ala Leu Tyr Tyr Ile Gly Gly
        290                 295                 300

Ile Ile Lys His Ala Lys Ala Leu Asn Ala Ile Thr Asn Pro Ser Thr
305                 310                 315                 320

Asn Ser Tyr Lys Arg Leu Val Pro His Phe Glu Ala Pro Thr Lys Leu
                325                 330                 335

Ala Tyr Ser Ala Lys Asn Arg Ser Ala Ser Ile Arg Ile Pro Ser Val
            340                 345                 350

Asn Ser Ser Lys Ala Arg Arg Ile Glu Ala Arg Phe Pro Asp Pro Thr
            355                 360                 365

Ala Asn Pro Tyr Leu Ala Phe Ala Ala Leu Leu Met Ala Gly Leu Asp
    370                 375                 380

Gly Ile Gln Asn Lys Ile His Pro Gly Asp Pro Ala Asp Lys Asn Leu
385                 390                 395                 400

Tyr Asp Leu Pro Pro Glu Glu Asp Ala Leu Val Pro Thr Val Cys Ala
            405                 410                 415

Ser Leu Glu Glu Ala Leu Ala Ala Leu Lys Ala Asp His Glu Phe Leu
            420                 425                 430

Leu Arg Gly Gly Val Phe Ser Lys Asp Trp Ile Asp Ser Tyr Ile Ala
        435                 440                 445

Phe Lys Glu Glu Asp Val Arg Arg Ile Arg Met Ala Pro His Pro Leu
    450                 455                 460

Glu Phe Glu Met Tyr Tyr Ser Leu
465                 470
```

<210> 45
<211> 242
<212> PRT
<213> Neisseria meningitidis

<400> 45

```
Met Thr Lys Gln Leu Lys Leu Ser Ala Leu Phe Val Ala Leu Leu Ala
1                5                    10                   15

Ser Gly Thr Ala Val Ala Gly Glu Ala Ser Val Gln Gly Tyr Thr Val
            20                  25                   30

Ser Gly Gln Ser Asn Glu Ile Val Arg Asn Asn Tyr Gly Glu Cys Trp
        35                   40                   45

Lys Asn Ala Tyr Phe Asp Lys Ala Ser Gln Gly Arg Val Glu Cys Gly
    50                  55                   60

Asp Ala Val Ala Ala Pro Glu Pro Glu Pro Glu Pro Glu Pro Ala Pro
65                  70                   75                   80

Ala Pro Val Val Val Val Glu Gln Ala Pro Gln Tyr Val Asp Glu Thr
            85                   90                   95

Ile Ser Leu Ser Ala Lys Thr Leu Phe Gly Phe Asp Lys Asp Ser Leu
        100                 105                  110

Arg Ala Glu Ala Gln Asp Asn Leu Lys Val Leu Ala Gln Arg Leu Ser
        115                 120                  125

Arg Thr Asn Val Gln Ser Val Arg Val Glu Gly His Thr Asp Phe Met
    130                 135                  140

Gly Ser Asp Lys Tyr Asn Gln Ala Leu Ser Glu Arg Arg Ala Tyr Val
145                 150                  155                  160

Val Ala Asn Asn Leu Val Ser Asn Gly Val Pro Val Ser Arg Ile Ser
            165                 170                  175

Ala Val Gly Leu Gly Glu Ser Gln Ala Gln Met Thr Gln Val Cys Glu
        180                 185                  190

Ala Glu Val Ala Lys Leu Gly Ala Lys Val Ser Lys Ala Lys Lys Arg
        195                 200                  205

Glu Ala Leu Ile Ala Cys Ile Glu Pro Asp Arg Arg Val Asp Val Lys
    210                 215                  220

Ile Arg Ser Ile Val Thr Arg Gln Val Val Pro Ala His Asn His His
225                 230                  235                  240

Gln His
```

<210> 46
<211> 752
<212> PRT
<213> Neisseria meningitidis

<400> 46

```
Met Tyr Thr Arg Ile Met Glu Ile Ser Pro Trp Thr Leu Arg Ser Ala
1               5               10              15

Lys Leu Glu Lys Glu His Lys Arg Leu Gln Glu Ser Leu Thr Ser Leu
            20              25              30

Gly Asn Gly Tyr Met Gly Met Arg Gly Ser Phe Glu Glu Thr Tyr Ser
        35              40              45

Ala Asp Ser His Leu Gly Thr Tyr Ile Ala Gly Val Trp Phe Pro Asp
    50              55              60

Lys Thr Arg Val Gly Trp Trp Lys Asn Gly Tyr Pro Lys Tyr Phe Gly
65              70              75              80

Lys Ala Ile Asn Ala Phe Asn Phe Ser Lys Val Lys Ile Phe Val Asp
            85              90              95

Gly Gln Glu Val Asp Leu Ala Lys Asn Asp Val Ala Gly Phe Ser Val
        100             105             110

Glu Leu Asp Met Gln His Gly Val Leu Arg Arg Ser Phe Thr Val Phe
        115             120             125

Gly Val Arg Phe Asn Val Cys Lys Phe Leu Ser Val Ala Gln Lys Glu
    130             135             140

Leu Ala Val Ile Arg Trp Glu Ala Val Ser Val Asp Gly Lys Thr His
145             150             155             160

Gln Val Arg Ile Asp Ser Ile Ile Asp Ala Asp Val Lys Asn Glu Asp
            165             170             175

Ser Asn Tyr Glu Glu Lys Phe Trp Gln Val Leu Asp Lys Gly Val Ser
        180             185             190

Asp Ser Leu Ser Tyr Ile Ala Ala Gln Thr Val Ala Asn Pro Phe Gly
        195             200             205

Val Glu Gln Phe Ile Val Asn Ala Glu Gln Thr Phe Ala Gly Ser Phe
    210             215             220
```

```
Lys Ala Leu Gly Gly Ser Gln Thr Asp Trp Gln Val Ser Asn Ser Phe
225                 230                 235                 240

Glu Ser Glu Val Gly Ser Thr Pro Glu Thr Phe Glu Lys Arg Val Ile
                245                 250                 255

Val Thr Thr Ser Arg Asp Tyr Gln Ser Leu Glu Ala Val Lys Ala Ala
            260                 265                 270

Gly Arg Ala Leu Ser Glu Lys Ile Ala Gly Val Ala Phe Glu Thr Leu
        275                 280                 285

Leu Asp Ala His Lys Ala Gly Trp Leu His Arg Trp Glu Ile Ala Asp
    290                 295                 300

Val Val Ile Glu Gly Ser Asp Glu Ala Gln Gln Gly Ile Arg Phe Asn
305                 310                 315                 320

Leu Phe Gln Leu Phe Ser Thr Tyr Tyr Gly Glu Asp Ala Arg Leu Asn
                325                 330                 335

Ile Gly Pro Lys Gly Phe Thr Gly Glu Lys Tyr Gly Gly Ala Thr Tyr
            340                 345                 350

Trp Asp Thr Glu Ala Tyr Ala Val Pro Leu Tyr Leu Ala Leu Ala Glu
            355                 360                 365

Pro Glu Val Thr Arg Asn Leu Leu Gln Tyr Arg Arg Asn Gln Leu Pro
    370                 375                 380

Gln Ala Gln His Asn Ala Arg Glu Gln Gly Leu Ala Gly Ala Leu Tyr
385                 390                 395                 400

Pro Met Val Thr Phe Thr Gly Ile Glu Cys His Asn Glu Trp Glu Ile
            405                 410                 415

Thr Phe Glu Glu Ile His Arg Asn Gly Ala Ile Pro Tyr Ala Ile Tyr
            420                 425                 430

Asn Tyr Thr Asn Tyr Thr Gly Asp Glu Gly Tyr Leu Ala Lys Glu Gly
            435                 440                 445

Leu Glu Val Leu Val Glu Val Ser Arg Phe Trp Ala Asp Arg Val His
    450                 455                 460

Phe Ser Lys Arg Asn Gly Lys Tyr Met Ile His Gly Val Thr Gly Pro
465                 470                 475                 480

Asn Glu Tyr Glu Asn Asn Ile Asn Asn Asn Trp Tyr Thr Asn Thr Leu
            485                 490                 495

Ala Ala Trp Val Leu Asp Tyr Thr Arg Glu Ala Leu Ala Lys Tyr Pro
        500                 505                 510

Arg Pro Asp Leu Asn Val Arg Ala Asp Glu Leu Glu Lys Trp Ala Asp
        515                 520                 525

Ile Ser Ala Asn Met Tyr Arg Pro His Asp Glu Glu Leu Gly Val Phe
    530                 535                 540

Val Gln His Asp Gly Phe Leu Asp Lys Asp Ile Arg Pro Val Ser Ala
545                 550                 555                 560

Leu Ser Pro Asp Asp Leu Pro Leu Asn Gln Lys Trp Ser Trp Asp Lys
                565                 570                 575

Ile Leu Arg Ser Pro Phe Ile Lys Gln Ala Asp Val Leu Gln Gly Ile
            580                 585                 590
```

```
Tyr Phe Phe Ser Asp Arg Phe Asn Ile Asp Glu Lys Arg Arg Asn Phe
        595             600             605

Asp Phe Tyr Glu Pro Met Thr Val His Glu Ser Ser Leu Ser Pro Cys
        610             615             620

Ile His Ser Ile Leu Ala Ala Glu Leu Gly Lys Glu Glu Lys Ala Val
625             630             635             640

Glu Met Tyr Gln Arg Thr Ala Arg Leu Asp Leu Asp Asn Tyr Asn Asn
            645             650             655

Asp Thr Glu Asp Gly Leu His Ile Thr Ser Met Thr Gly Ser Trp Leu
            660             665             670

Ala Ile Val Gln Gly Phe Ala Gln Met Lys Thr Trp Gly Gly Lys Leu
        675             680             685

Ser Phe Ala Pro Phe Leu Pro Ser Ala Trp Thr Gly Tyr Ala Phe His
    690             695             700

Ile Asn Tyr Arg Gly Arg Leu Ile Lys Val Ala Val Gly Lys Glu Asn
705             710             715             720

Val Val Phe Thr Leu Leu Lys Gly Glu Ser Leu Asp Leu Gln Val Tyr
            725             730             735

Gly Lys Asp Ile Thr Leu Asp Gly Ser His Thr Val Ala Leu Glu Lys
            740             745             750
```

<210> 47
<211> 221
<212> PRT
<213> Neisseria meningitidis

<400> 47

```
Met Thr Phe Thr Ala Val Leu Phe Asp Leu Asp Gly Val Ile Thr Asp
1               5                   10              15
Thr Ala Glu Tyr His Tyr Arg Ala Trp Lys Lys Leu Ala Glu Glu Leu
            20                  25              30
Gly Ile Ser Ile Asp Arg Lys Phe Asn Glu Gln Leu Lys Gly Val Ser
            35                  40              45
Arg Asp Asp Ser Leu Lys Arg Ile Leu Ala His Gly Gly Lys Thr Val
    50                  55              60
Ser Glu Ala Glu Phe Ala Glu Leu Thr Arg Arg Lys Asn Asp Asn Tyr
65                  70                  75              80
Val Glu Met Ile Gln Ala Val Lys Pro Glu Asp Val Tyr Pro Gly Ile
                85                  90                  95
Leu Pro Leu Leu Glu Ala Leu Arg Ala Asn Gly Lys Lys Ile Ala Leu
            100                 105             110
Ala Ser Ala Ser Lys Asn Gly Pro Phe Leu Leu Glu Arg Met Gly Leu
        115                 120                 125
Thr His Phe Phe Asp Ala Ile Ala Asp Pro Ala Ala Val Ala His Ser
    130                 135                 140
Lys Pro Ala Pro Asp Ile Phe Leu Ala Ala Ala Glu Gly Val Asp Ala
145                 150                 155             160
Asp Ile Arg Gln Cys Ile Gly Ile Glu Asp Ala Ala Ala Gly Val Ala
                165                 170                 175
Ala Ile Lys Ala Ala Gly Ala Leu Pro Ile Gly Val Gly Lys Ala Glu
            180                 185             190
Asp Leu Gly Ser Asp Ile Ala Leu Val Ser Gly Thr Ala Glu Leu Thr
        195                 200             205
Tyr Ala Tyr Leu Gln Ser Val Trp Glu Gln Ser Gly Arg
    210                 215             220
```

<210> 48
<211> 414
<212> PRT
<213> Neisseria meningitidis

<400> 48

```
Met Glu Gln Gln Gln Arg Tyr Ile Ser Val Leu Asp Ile Gly Thr Ser
1               5               10              15

Lys Val Leu Ala Leu Ile Gly Glu Val Gln Asp Asp Asp Lys Ile Asn
            20              25              30

Ile Val Gly Leu Gly Gln Ala Pro Ser Arg Gly Leu Arg Ala Gly Met
            35              40              45

Val Thr Asn Ile Asp Ala Thr Val Gln Ala Ile Arg Gln Ala Val Asn
    50              55              60

Asp Ala Glu Leu Met Ala Asp Thr Lys Ile Thr His Val Thr Thr Gly
65              70              75              80

Ile Ala Gly Asn His Ile Arg Ser Leu Asn Ser Gln Gly Val Val Lys
            85              90              95

Ile Lys Asp Gly Glu Val Thr Gln Ala Asp Ile Asp Arg Ala Ile Glu
            100             105             110

Thr Ala Lys Ala Ile Asn Ile Pro Pro Asp Gln Lys Ile Leu Asp Ala
            115             120             125

Val Val Gln Asp Tyr Ile Ile Asp Thr Gln Leu Gly Val Arg Glu Pro
    130             135             140

Ile Gly Met Ser Gly Val Arg Leu Asp Thr Arg Val His Ile Ile Thr
145             150             155             160

Gly Ala Ser Thr Ala Val Gln Asn Val Gln Lys Cys Ile Glu Arg Cys
            165             170             175

Gly Leu Lys Ser Asp Gln Ile Met Leu Gln Pro Leu Ala Ser Gly Gln
            180             185             190

Ala Val Leu Thr Glu Asp Glu Lys Asp Leu Gly Val Cys Val Ile Asp
            195             200             205

Ile Gly Gly Gly Thr Thr Asp Ile Ala Val Tyr Met Asn Gly Ala Ile
    210             215             220

Arg His Thr Ser Val Ile Pro Ala Gly Gly Asn Leu Ile Thr Lys Asp
225             230             235             240

Leu Ser Lys Ser Leu Arg Thr Pro Leu Asp Ala Ala Glu Tyr Ile Lys
            245             250             255

Ile His Tyr Gly Val Ala Ser Cys Asp Thr Glu Gly Leu Gly Glu Met
            260             265             270

Ile Glu Val Pro Gly Val Gly Asp Arg Thr Ser Arg Gln Val Ser Ser
    275             280             285

Lys Val Leu Ala Ala Ile Ile Ser Ala Arg Ile Gln Glu Ile Phe Gly
```

290 295 300

Val Val Leu Gly Glu Leu Gln Lys Ser Gly Phe Pro Lys Glu Val Leu
305 310 315 320

Asn Ala Gly Ile Val Leu Thr Gly Gly Val Ser Met Met Thr Gly Ile
325 330 335

Val Glu Phe Ala Glu Lys Ile Phe Asp Leu Pro Val Arg Thr Gly Ala
340 345 350

Pro Gln Glu Met Gly Gly Leu Ser Asp Arg Val Arg Thr Pro Arg Phe
355 360 365

Ser Thr Ala Ile Gly Leu Leu His Ala Ala Cys Lys Leu Glu Gly Asn
370 375 380

Leu Pro Gln Pro Glu Asn Gly Ala Val Gln Glu Arg Glu Gly Gly Gly
385 390 395 400

Gly Leu Leu Ala Arg Leu Lys Arg Trp Ile Glu Asn Ser Phe
405 410 .

<210> 49
<211> 392
<212> PRT
<213> Neisseria meningitidis

<400> 49

```
Met Glu Phe Val Tyr Asp Val Ala Glu Ser Ala Val Ser Pro Ala Val
1               5               10              15

Ile Lys Val Ile Gly Leu Gly Gly Gly Gly Cys Asn Ala Ile Asn Asn
            20              25              30

Met Val Ala Asn Asn Val Arg Gly Val Glu Phe Ile Ser Ala Asn Thr
        35              40              45

Asp Ala Gln Ser Leu Ala Lys Asn His Ala Ala Lys Arg Ile Gln Leu
    50              55              60

Gly Thr Asn Leu Thr Arg Gly Leu Gly Ala Gly Ala Asn Pro Asp Ile
65              70              75              80

Gly Arg Ala Ala Ala Gln Glu Asp Arg Glu Ala Ile Glu Glu Ala Ile
            85              90              95

Arg Gly Ala Asn Met Leu Phe Ile Thr Thr Gly Met Gly Gly Gly Thr
            100             105             110

Gly Thr Gly Ser Ala Pro Val Val Ala Glu Ile Ala Lys Ser Leu Gly
        115             120             125

Ile Leu Thr Val Ala Val Val Thr Arg Pro Phe Ala Tyr Glu Gly Lys
    130             135             140

Arg Val His Val Ala Gln Ala Gly Leu Glu Gln Leu Lys Glu His Val
145             150             155             160

Asp Ser Leu Ile Ile Ile Pro Asn Asp Lys Leu Met Thr Ala Leu Gly
            165             170             175

Glu Asp Val Thr Met Arg Glu Ala Phe Arg Ala Ala Asp Asn Val Leu
        180             185             190

Arg Asp Ala Val Ala Gly Ile Ser Glu Val Val Thr Cys Pro Ser Glu
    195             200             205

Ile Ile Asn Leu Asp Phe Ala Asp Val Lys Thr Val Met Ser Asn Arg
    210             215             220
```

```
Gly Ile Ala Met Met Gly Ser Gly Tyr Ala Gln Gly Ile Asp Arg Ala
225                 230                 235                 240

Arg Met Ala Thr Asp Gln Ala Ile Ser Ser Pro Leu Leu Asp Asp Val
            245                 250                 255

Thr Leu Asp Gly Ala Arg Gly Val Leu Val Asn Ile Thr Thr Ala Pro
        260                 265                 270

Gly Cys Leu Lys Met Ser Glu Leu Ser Glu Val Met Lys Ile Val Asn
        275                 280                 285

Gln Ser Ala His Pro Asp Leu Glu Cys Lys Phe Gly Ala Ala Glu Asp
    290                 295                 300

Glu Thr Met Ser Glu Asp Ala Ile Arg Ile Thr Ile Ile Ala Thr Gly
305                 310                 315                 320

Leu Lys Glu Lys Gly Ala Val Asp Phe Val Pro Ala Arg Glu Val Glu
                325                 330                 335

Ala Val Ala Pro Ser Lys Gln Glu Gln Ser His Asn Val Glu Gly Met
            340                 345                 350

Ile Arg Thr Asn Arg Gly Ile Arg Thr Met Asn Leu Thr Ala Ala Asp
        355                 360                 365

Phe Asp Asn Gln Ser Val Leu Asp Asp Phe Glu Ile Pro Ala Ile Leu
    370                 375                 380

Arg Arg Gln His Asn Ser Asp Lys
385                 390
```

<210> 50
<211> 362
<212> PRT
<213> Neisseria meningitidis

<400> 50

```
Met Ser Gln Thr Ile Asp Glu Leu Leu Leu Pro His Arg Asn Ala Ile
1               5                   10              15

Asp Thr Ile Asp Ala Glu Ile Leu Arg Leu Leu Asn Glu Arg Ala Gln
            20              25              30

His Ala His Ala Ile Gly Glu Leu Lys Gly Thr Gly Ala Val Tyr Arg
        35              40              45

Pro Glu Arg Glu Val Ala Val Leu Arg Arg Ile Gln Asp Leu Asn Lys
    50              55              60

Gly Pro Leu Pro Asp Glu Ser Val Ala Arg Leu Phe Arg Glu Val Met
65              70              75              80

Ser Glu Cys Leu Ala Val Glu Arg Pro Leu Thr Ile Ala Tyr Leu Gly
            85              90              95

Pro Gln Gly Thr Phe Thr Gln Gln Ala Ala Ile Lys His Phe Gly His
        100             105             110

Ala Ala His Thr Met Ala Cys Pro Thr Ile Asp Asp Cys Phe Lys Gln
        115             120             125

Val Glu Thr Arg Gln Ala Asp Tyr Leu Val Ala Pro Val Glu Asn Ser
    130             135             140

Thr Glu Gly Ser Val Gly Arg Thr Leu Asp Leu Leu Ala Val Thr Ala
145             150             155             160
```

```
Leu Gln Ala Cys Gly Glu Ile Val Leu Arg Ile His His Asn Leu Leu
                165             170              175

Arg Lys Asn Asn Gly Ser Thr Glu Gly Ile Ala Lys Val Phe Ser His
            180             185              190

Ala Gln Ala Leu Ala Gln Cys Asn Asp Trp Leu Gly Arg His Leu Pro
        195             200              205

Asn Ala Glu Arg Ile Ala Val Ser Ser Asn Ala Glu Ala Ala Arg Leu
        210             215              220

Val Ala Glu Ser Asp Asp Gly Thr Val Ala Ala Ile Ala Gly Arg Thr
225             230              235              240

Ala Ala Glu Ile Tyr Gly Leu Asp Met Val Ala Glu Cys Ile Glu Asp
            245             250              255

Glu Pro Asn Asn Thr Thr Arg Phe Leu Val Met Gly His His Glu Thr
            260             265              270

Gly Ala Ser Gly Ser Asp Lys Thr Ser Leu Ala Val Ser Ala Pro Asn
        275             280              285

Arg Ala Gly Ala Val Ala Ser Leu Leu Gln Pro Leu Thr Glu Ser Gly
        290             295              300

Ile Ser Met Thr Lys Phe Glu Ser Arg Pro Ser Lys Ser Val Leu Trp
305             310              315              320

Glu Tyr Leu Phe Phe Ile Asp Ile Glu Gly His Arg Arg Asp Ala Gln
            325             330              335

Ile Gln Thr Ala Leu Glu Arg Leu Gly Glu Arg Ala Ser Phe Val Lys
        340             345              350

Val Ile Gly Ser Tyr Pro Thr Ala Val Leu
        355             360
```

<210> 51
<211> 459
<212> PRT
<213> Neisseria meningitidis

<400> 51

```
Met Gln Ala Phe Ser Leu Tyr Pro Pro Val Gly His Pro Asp Ser Ala
1               5                   10              15

Lys Lys Arg Gln Asn Arg Ala Asp Val Phe Leu Pro Phe Trp Lys Gln
            20              25                  30

Asp Asn Phe Leu Gly Lys Ser Val Gln Tyr Arg Phe Glu Phe Ala Gln
        35              40                  45

Ile Tyr Phe Thr Met Pro Lys Pro Cys Ala Gly Val Thr Met Gly Arg
    50              55                  60

Gly Asp Phe Phe Phe Ser Asn Leu Phe His Gln Glu Ser Ala Arg Met
65                  70              75                      80

Lys Lys Ser Val Leu Ala Val Leu Ala Ala Leu Ser Leu Ala Ala Cys
            85              90                  95

Gly Gly Ser Glu Lys Asn Ala Val Gln Pro Gln Ala Asp Ala Ala Ser
        100             105                 110

Ala Ala Asn Ala Glu Ala Ala Ala Thr Asp Thr Leu Asn Ile Tyr Asn
        115                 120                 125

Trp Ser Asn Tyr Val Asp Glu Ser Thr Val Glu Asp Phe Lys Lys Ala
```

```
              130                    135                    140
      Asn Asn Leu Lys Leu Thr Tyr Asp Leu Tyr Glu Asn Asn Glu Thr Leu
      145             150                 155                 160

      Glu Ala Lys Met Leu Thr Gly Lys Ser Gly Tyr Asp Leu Val Val Pro
                  165                 170                 175

      Gly Ile Ala Phe Leu Pro Arg Gln Ile Glu Ala Gly Ala Tyr Gln Lys
                  180                 185                 190

      Val Asn Lys Asp Leu Ile Pro Asn Tyr Lys Asn Ile Asp Pro Glu Leu
                  195                 200                 205

      Leu Lys Met Leu Glu Thr Ala Asp Pro Gly Asn Gln Tyr Ala Val Pro
          210                 215                 220

      Tyr Phe Ser Gly Val Asn Thr Ile Ala Ile Thr Ala Lys Gly Lys Glu
      225             230                 235                 240

      Leu Leu Gly Gly Lys Leu Pro Glu Asn Gly Trp Asp Leu Leu Phe Lys
                  245                 250                 255

      Pro Glu Tyr Thr His Lys Leu Lys Ser Cys Gly Ile Ala Leu Trp Asp
                  260                 265                 270

      Thr Pro Ser Glu Met Phe Pro Ile Leu Leu Asn Tyr Leu Gly Lys Asp
                  275                 280                 285

      Pro Lys Gly Ser Asn Pro Glu Asp Leu Lys Ala Ala Ala Glu Val Leu
          290                 295                 300

      Lys Ser Ile Arg Pro Asp Val Lys Arg Phe Ser Pro Ser Ile Ile Asp
      305                 310                 315                 320

      Glu Leu Ala Arg Gly Asp Ile Cys Leu Ala Ala Gly Asn Gly Gly Asp
                  325                 330                 335

      Leu Asn Leu Ala Lys Ala Arg Ser Glu Glu Val Lys Asn Asn Val Gly
                  340                 345                 350

      Ile Glu Val Leu Thr Pro Lys Gly Met Gly Phe Trp Ile Glu Ser Trp
                  355                 360                 365

      Leu Ile Pro Ala Asp Ala Lys Asn Val Ala Asn Ala His Lys Tyr Ile
          370                 375                 380

      Asn Tyr Thr Leu Asp Pro Glu Ile Ala Ala Lys Asn Gly Ile Ala Val
      385                 390                 395                 400

      Thr Phe Ala Pro Ala Ser Lys Pro Ala Arg Glu Lys Met Pro Ala Glu
                  405                 410                 415

      Leu Val Asn Thr Arg Ser Ile Phe Pro Asn Glu Gln Asp Met Lys Asp
                  420                 425                 430

      Gly Phe Val Met Pro Gln Met Ser Thr Asp Ala Lys Lys Leu Ser Val
                  435                 440                 445

      Ser Leu Trp Gln Lys Ile Lys Val Gly Thr Asn
      450                 455
```

<210> 52

<211> 602

<212> PRT

<213> Neisseria meningitidis

126

<400> 52

```
Met Arg Thr Asn Tyr Cys Gly Leu Ile Ser Glu Gln Tyr Leu Asp Gln
1               5                   10                  15
```

```
Thr Val Thr Val Lys Gly Trp Val His Arg Arg Arg Asp His Gly Gly
            20              25                  30
Val Ile Phe Ile Asp Leu Arg Asp Arg Glu Gly Ile Val Gln Val Val
        35              40                  45
Ile Asp Pro Asp Thr Pro Glu Ala Phe Ala Ala Ala Asp Ser Ser Arg
    50              55                  60
Asn Glu Tyr Val Leu Ser Ile Thr Gly Arg Val Arg Asn Arg Pro Glu
65              70              75                  80
Gly Thr Thr Asn Asp Lys Met Ile Ser Gly Lys Ile Glu Ile Leu Ala
            85              90                  95
Lys Glu Ile Glu Val Leu Asn Ala Ala Ala Thr Pro Pro Phe Gln Ile
            100             105                 110
Asp Asp Glu Asn Ile Ser Glu Asn Val Arg Leu Thr Asn Arg Val Ile
        115             120                 125
Asp Leu Arg Arg Pro Val Met Gln Arg Asn Leu Arg Leu Arg Tyr Gln
    130             135             140
Val Ala Met Gly Val Arg Arg Tyr Leu Asp Ala Gln Gly Phe Ile Asp
145             150             155                 160
Ile Glu Thr Pro Met Leu Thr Arg Ser Thr Pro Glu Gly Ala Arg Asp
            165             170                 175
Tyr Leu Val Pro Ser Arg Val His Pro Gly Glu Phe Phe Ala Leu Pro
            180             185                 190
Gln Ser Pro Gln Leu Phe Lys Gln Leu Leu Met Val Ala Gly Phe Asp
    195             200             205
Arg Tyr Tyr Gln Ile Thr Lys Cys Phe Arg Asp Glu Asp Leu Arg Ala
    210             215             220
Asp Arg Gln Pro Glu Phe Thr Gln Ile Asp Leu Glu Thr Ser Phe Leu
225             230             235                 240
Asn Glu Asp Glu Ile Met Asp Ile Thr Glu Gly Met Ala Lys Gln Val
            245             250                 255
Phe Lys Asp Ala Leu Asn Val Asp Leu Gly Asp Phe Pro Arg Met Pro
            260             265                 270
Tyr Ser Glu Ala Met Phe Tyr Tyr Gly Ser Asp Lys Pro Asp Met Arg
    275             280             285
Ile Asn Leu Lys Phe Thr Glu Leu Thr Asp Leu Met Lys Thr Glu Glu
    290             295             300
Phe Lys Val Phe Arg Gly Ala Ala Asp Met Lys Gly Gly Arg Val Val
305             310             315                 320
Ala Leu Arg Val Pro Asn Gly Ala Glu Phe Ser Arg Lys Glu Ile Asp
            325             330                 335
Glu Tyr Thr Lys Phe Val Gly Ile Tyr Gly Ala Lys Gly Leu Ala Tyr
        340             345             350
Ile Lys Val Asn Asp Val Ser Asn Leu Ser Asn Gly Glu Asp Ser Gly
        355             360             365
Leu Gln Ser Pro Ile Val Lys Tyr Leu Ser Glu Asn Ala Leu Lys Glu
    370             375             380
```

```
Ile Ile Ala Arg Thr Gly Ala Gln Asn Gly Asp Ile Ile Phe Phe Gly
385             390             395             400

Ala Asp Lys Ala Lys Val Val Asn Glu Ala Ile Gly Ala Leu Arg Ile
                405             410             415

Lys Val Gly Leu Glu His Gly Lys Asp Asn Gly Tyr Phe Thr Asp Glu
            420             425             430

Trp Lys Pro Leu Trp Val Val Asp Phe Pro Met Phe Glu Tyr Asp Glu
        435             440             445

Glu Ala Asp Arg Tyr Val Ala Val His His Pro Phe Thr Ala Pro Lys
    450             455             460

Glu Gly His Glu Asp Leu Met Val Ser Asp Pro Ala Asn Cys Leu Ala
465             470             475             480

Arg Ala Tyr Asp Met Val Leu Asn Gly Trp Glu Ile Gly Gly Gly Ser
                485             490             495

Ile Arg Ile His Arg Ala Asp Val Gln Glu Lys Val Phe Ala Ala Leu
            500             505             510

Lys Ile Ser Pro Glu Glu Gln Gln Glu Lys Phe Gly Phe Leu Leu Asp
        515             520             525

Asn Leu Lys Phe Gly Ala Pro Pro His Gly Gly Leu Ala Phe Gly Leu
    530             535             540

Asp Arg Leu Val Thr Leu Met Thr Gly Ala Glu Ser Ile Arg Asp Val
545             550             555             560

Ile Ala Phe Pro Lys Thr Gln Arg Ala Gln Cys Leu Leu Thr Asn Ala
                565             570             575

Pro Asn Ser Val Asp Asp Lys Gln Leu Arg Glu Leu Ser Leu Arg Leu
            580             585             590

Arg Gln Lys Ala Thr Glu Thr Lys Glu Val
        595             600
```

<210> 53
<211> 178
<212> PRT
<213> Neisseria meningitidis

<400> 53

```
Met Arg Glu Asn Lys Ile Ile Phe Thr Gly Pro Val Gly Val Gly Lys
1               5                   10                  15

Thr Thr Ala Ile Ala Ala Ile Ser Asp Glu Ala Leu Val Gln Thr Asp
            20                  25                  30

Ala Ser Ala Ser Asp Met Thr Leu Asp Arg Lys Arg Asn Thr Thr Val
        35                  40                  45

Ala Met Asp Tyr Gly Ala Ile Ser Leu Asp Glu Asp Thr Lys Val His
    50                  55                  60

Leu Tyr Gly Thr Pro Gly Gln Glu Arg Phe Asn Phe Met Trp Glu Ile
65                  70                  75                  80

Leu Ser Gln Gly Ser Met Gly Leu Val Leu Leu Leu Asp Asn Ala Arg
            85                  90                  95

Thr Asn Pro Leu Lys Asp Leu Glu Phe Phe Leu His Ser Phe Arg Gly
            100                 105                 110

Leu Leu Glu Lys Ala Pro Val Val Val Gly Ile Thr Lys Met Asp Ile


            115                 120                 125

Arg Ser Gln Pro Gly Ile Asp Val Tyr His Lys Tyr Leu Ala Lys His
    130                 135                 140

Asn Leu Asn Val Pro Val Phe Glu Ile Asp Ala Arg Lys Glu Asp Asp
145                 150                 155                 160

Val Lys Gln Leu Val Ser Ala Met Leu Phe Ser Ile Asp Pro Gly Leu
                165                 170                 175

Glu Val
```

<210> 54
<211> 361
<212> PRT
<213> Neisseria meningitidis

<400> 54

```
Met Thr Val Pro His Ile Pro Arg Gly Pro Val Met Ala Asp Ile Ala
1               5                   10              15

Ala Phe Arg Leu Thr Glu Glu Glu Lys Gln Arg Leu Leu Asp Pro Ala
            20                  25                  30

Val Gly Gly Ile Ile Leu Phe Arg Arg Asn Phe Gln Asn Ile Glu Gln
        35                  40                  45

Leu Lys Thr Leu Thr Ala Glu Ile Lys Ala Leu Arg Thr Pro Glu Leu
    50                  55                  60

Ile Ile Ala Val Asp His Glu Gly Gly Arg Val Gln Arg Phe Ile Glu
65                  70                  75                  80

Gly Phe Thr Arg Leu Pro Ala Met Ser Thr Leu Gly Glu Ile Trp Asp
            85                  90                  95

Lys Asp Gly Ala Ser Ala Ala Glu Thr Ala Ala Gly Gln Val Gly Arg
            100                 105                 110

Val Leu Ala Thr Glu Leu Ser Ala Cys Gly Ile Asp Leu Ser Phe Thr
        115                 120                 125

Pro Val Leu Asp Leu Asp Trp Gly Asn Cys Pro Val Ile Gly Asn Arg
    130                 135                 140

Ser Phe His Arg Asn Pro Glu Ala Val Ala Arg Leu Ala Leu Ala Leu
145                 150                 155                 160

Gln Lys Gly Leu Thr Lys Gly Gly Met Lys Ser Cys Gly Lys His Phe
            165                 170                 175

Pro Gly His Gly Phe Val Glu Gly Asp Ser His Leu Val Leu Pro Glu
            180                 185                 190

Asp Trp Arg Ser Leu Ser Glu Leu Glu Thr Ala Asp Leu Ala Pro Phe
        195                 200                 205

Arg Ile Met Ser Arg Glu Gly Met Ala Ala Val Met Pro Ala His Val
    210                 215                 220

Val Tyr Pro Gln Val Asp Thr Lys Pro Ala Gly Phe Ser Glu Ile Trp
225                 230                 235                 240

Leu Lys Gln Ile Leu Arg Arg Asp Ile Gly Phe Lys Gly Val Ile Phe
            245                 250                 255

Ser Asp Asp Leu Thr Met Glu Gly Ala Cys Gly Ala Gly Gly Ile Lys
            260                 265                 270

Glu Arg Ala Arg Ile Ser Phe Glu Ala Gly Cys Asp Ile Val Leu Val
        275                 280                 285

Cys Asn Arg Pro Asp Leu Val Asp Glu Leu Arg Glu Asp Phe Arg Ile
    290                 295                 300

Pro Asp Asn Pro Thr Leu Ala Gln Arg Trp Gln Tyr Met Ala Asn Thr
305                 310                 315                 320

Leu Gly Ser Ala Ala Ala Gln Ala Val Met Gln Thr Ala Asp Phe Gln
            325                 330                 335

Ala Ala Gln Ala Phe Val Ala Gly Leu Ala Ser Pro Gln Asp Thr Ala
            340                 345                 350

Gly Gly Val Lys Val Gly Glu Ala Phe
        355                 360
```

<210> 55
<211> 397
<212> PRT
<213> Neisseria meningitidis

<400> 55

```
Met Phe Phe Lys His Ile Glu Ala Ala Pro Ala Asp Pro Ile Leu Gly
1               5                   10                  15

Leu Gly Glu Ala Phe Lys Ala Glu Thr Arg Pro Glu Lys Val Asn Leu
            20                  25                  30

Gly Ile Gly Val Tyr Lys Asp Ala Ser Gly Ala Thr Pro Leu Val Lys
            35                  40                  45

Ala Val Lys Glu Ala Glu Lys Arg Leu Leu Glu Ser Glu Thr Thr Lys
        50                  55                  60

Asn Tyr Leu Thr Ile Asp Gly Val Ala Asp Tyr Asn Ala Gln Thr Gln
65                  70                  75                  80

Ile Leu Leu Phe Gly Lys Asp His Glu Ile Ile Ala Ser Arg Arg Ala
                85                  90                  95

Lys Thr Ala Gln Ser Leu Gly Gly Thr Gly Ala Leu Arg Ile Ala Ala
            100                 105                 110

Glu Phe Ala Lys Arg Gln Leu Asn Ala Gln Thr Ile Trp Ile Ser Asn
        115                 120                 125

Pro Thr Trp Pro Asn His Asn Ala Ile Ala Lys Ala Val Gly Ile Gln
        130                 135                 140

Asp Lys Pro Tyr Arg Tyr Tyr Asp Ala Ala Lys His Gly Leu Asp Trp
145                 150                 155                 160

Asp Gly Met Ile Glu Asp Leu Ser Gln Ala Gln Lys Gly Asp Ile Val
                165                 170                 175

Leu Leu His Gly Cys Cys His Asn Pro Thr Gly Ile Asp Pro Thr Pro
                180                 185                 190

Glu Gln Trp Glu Thr Leu Ala Lys Leu Ser Ala Glu Lys Gly Trp Leu
            195                 200                 205

Pro Leu Phe Asp Phe Ala Tyr Gln Gly Phe Gly Asn Gly Leu Glu Glu
        210                 215                 220

Asp Ala Tyr Gly Leu Arg Val Phe Leu Lys His Asn Thr Glu Leu Leu
225                 230                 235                 240
```

```
Ile Ala Ser Ser Tyr Ser Lys Asn Phe Gly Met Tyr Asn Glu Arg Val
                245                 250                 255

Gly Ala Phe Thr Leu Val Ala Glu Asp Glu Glu Thr Ala Ala Arg Ala
            260                 265                 270

His Ser Gln Val Lys Thr Ile Ile Arg Thr Leu Tyr Ser Asn Pro Ala
        275                 280                 285

Ser His Gly Ala Asn Thr Ile Ala Leu Val Leu Lys Asn Asp Asp Leu
        290                 295                 300

Lys Ala Gln Trp Ile Ala Glu Leu Asp Glu Met Arg Gly Arg Ile Lys
305                 310                 315                 320

Ala Met Arg Gln Lys Phe Val Gly Leu Leu Lys Ala Lys Gly Ala Ser
                325                 330                 335

Gln Asn Phe Asp Phe Ile Ile Lys Gln Asn Gly Met Phe Ser Phe Ser
            340                 345                 350

Gly Leu Thr Pro Glu Gln Val Asp Arg Leu Lys Asn Glu Phe Ala Ile
            355                 360                 365

Tyr Ala Val Arg Ser Gly Arg Ile Asn Val Ala Gly Ile Thr Asp Asn
    370                 375                 380

Asn Ile Asp Tyr Leu Cys Glu Ser Ile Val Lys Val Leu
385                 390                 395
```

<210> 56
<211> 348
<212> PRT
<213> Neisseria meningitidis

<400> 56

```
Met Lys Met Gln Ala Val Val Val Asn Lys Asn Val Ala Gly Asp Val
1             5                 10                15

Glu Val Ile Glu Arg Glu Val Arg Pro Leu Glu Tyr Gly Glu Ala Leu
            20                25                30

Val Glu Val Glu Tyr Cys Gly Val Cys His Thr Asp Leu His Val Ala
        35                40                45

Ala Gly Asp Tyr Gly Glu Lys Pro Gly Arg Val Leu Gly His Glu Gly
    50                55                60

Ile Gly Leu Val Lys Glu Val Ala Asp Gly Val Lys Asn Leu Lys Val
65                70                75                80

Gly Asp Arg Val Ser Ile Ala Trp Leu Phe Gln Ser Cys Gly Ser Cys
                85                90                95

Glu Tyr Cys Asn Thr Gly Arg Glu Thr Leu Cys Arg Ser Val Leu Asn
            100               105               110

Ala Gly Tyr Thr Ala Asp Gly Gly Met Ala Thr His Cys Ile Val Ser
            115               120               125

Ala Asp Tyr Ala Val Lys Val Pro Glu Gly Leu Asp Pro Ala Gln Ala
    130               135               140

Ser Ser Ile Thr Cys Ala Gly Val Thr Thr Tyr Lys Ala Ile Lys Val
145               150               155               160

Ser Gly Val Arg Pro Gly Gln Trp Ile Ala Ile Tyr Gly Ala Gly Gly
                165               170               175

Leu Gly Asn Leu Gly Val Gln Tyr Ala Lys Lys Val Phe Gly Ala His

            180               185               190

Val Val Ala Ile Asp Ile Asn Asp Asp Lys Leu Ala Phe Ala Lys Glu
    195               200               205

Thr Gly Ala Asp Leu Val Val Asn Ala Ala Lys Glu Asp Ala Ala Lys
    210               215               220

Val Ile Gln Glu Lys Thr Gly Gly Ala His Ala Ala Val Val Thr Ala
225               230               235               240

Val Ser Ala Ala Ala Phe Asn Ser Ala Val Asn Cys Val Arg Ala Gly
            245               250               255

Gly Arg Val Val Ala Ile Gly Leu Pro Pro Glu Ser Met Asp Leu Ser
            260               265               270

Ile Pro Arg Leu Val Leu Asp Gly Ile Glu Val Val Gly Ser Leu Val
        275               280               285

Gly Thr Arg Lys Asp Leu Glu Glu Ala Phe Gln Phe Gly Ala Glu Gly
    290               295               300

Leu Val Val Pro Lys Val Gln Leu Arg Ala Leu Asp Glu Ala Pro Ala
305               310               315               320

Ile Phe Gln Glu Met Arg Glu Gly Lys Ile Thr Gly Arg Met Val Ile
            325               330               335

Asp Met Lys Lys Glu Cys Gly Cys Gly His His His
        340               345
```

134

<210> 57
<211> 405
<212> PRT
<213> Neisseria meningitidis

<400> 57

```
Met Glu Ile Ile Leu Gly Ile Val Met Phe Thr Val Ile Val Leu Val
1               5                   10                  15

Leu Ala Leu Met Ile Leu Phe Ala Lys Ser Lys Leu Val Ser Glu Gly
            20                  25                  30

Asp Ile Thr Ile Lys Val Asn Gly Glu Lys Glu Leu Thr Met Pro Ala
        35                  40                  45

Gly Gly Lys Leu Leu Gly Ala Leu Ala Asn Glu Gly Ile Phe Ile Pro
    50                  55                  60

Ser Ala Cys Gly Gly Gly Gly Ser Cys Gly Gln Cys Arg Val Val Val
65                  70                  75                  80

Lys Ser Gly Gly Gly Asp Ile Leu Pro Thr Glu Leu Ser His Ile Ser
                85                  90                  95

Lys Arg Glu Ala Arg Glu Gly Cys Arg Leu Ser Cys Gln Val Asn Val
            100                 105                 110

Lys Thr Asp Met Asp Ile Glu Val Pro Glu Glu Val Phe Gly Val Lys
        115                 120                 125

Lys Trp Glu Cys Thr Val Ile Ser Asn Asp Asn Lys Ala Thr Phe Ile
    130                 135                 140

Lys Glu Leu Lys Leu Ala Ile Pro Glu Gly Glu Glu Val Pro Phe Arg
145                 150                 155                 160

Ala Gly Gly Tyr Ile Gln Ile Glu Ala Pro Pro His Thr Val Ala Tyr
                165                 170                 175
```

```
Lys Asp Phe Asp Ile Pro Lys Glu Tyr His Glu Asp Trp Asp Lys Tyr
            180                 185                 190

Asn Leu Trp Gln Tyr Val Ser Lys Val Asp Glu Pro Ile Leu Arg Ala
            195                 200                 205

Tyr Ser Met Ala Ser Tyr Pro Glu Glu Lys Gly Ile Ile Met Leu Asn
        210                 215                 220

Val Arg Ile Ala Thr Pro Pro Pro Arg Val Pro Asp Ala Pro Pro Gly
225                 230                 235                 240

Gln Met Ser Ser Tyr Ile Trp Ser Leu Lys Pro Gly Asp Lys Val Thr
                245                 250                 255

Ile Ser Gly Pro Phe Gly Glu Phe Phe Ala Lys Asp Thr Asp Ala Glu
            260                 265                 270

Met Val Phe Ile Gly Gly Gly Ala Gly Met Ala Pro Met Arg Ser His
            275                 280                 285

Ile Phe Asp Gln Leu Lys Arg Leu Asn Ser Lys Arg Lys Ile Thr Phe
            290                 295                 300

Trp Tyr Gly Ala Arg Ser Lys Arg Glu Met Phe Tyr Val Glu Asp Phe
305                 310                 315                 320

Asp Gln Leu Ala Ala Glu Phe Pro Asn Phe Thr Trp His Val Ala Leu
                325                 330                 335

Ser Asp Pro Leu Pro Glu Asp Asn Trp Asp Gly Tyr Thr Gly Phe Ile
            340                 345                 350

His Asn Val Val Tyr Glu Asn His Leu Lys Asn His Glu Ala Pro Glu
            355                 360                 365

Asp Cys Glu Phe Tyr Met Cys Gly Pro Pro Ile Met Asn Gln Ser Val
    370                 375                 380

Ile Lys Met Leu Lys Asp Leu Gly Val Glu Asp Glu Asn Ile Leu Leu
385                 390                 395                 400

Asp Asp Phe Gly Gly
                405
```

<210> 58

<211> 121

<212> PRT

<213> Neisseria meningitidis

<400> 58

```
Met Asn Leu Ile Gln Gln Leu Glu Gln Glu Glu Ile Ala Arg Leu Asn
1               5               10              15
Lys Glu Ile Pro Glu Phe Ala Pro Gly Asp Thr Val Val Val Ser Val
            20              25              30
Arg Val Val Glu Gly Thr Arg Ser Arg Leu Gln Ala Tyr Glu Gly Val
        35              40              45
Val Ile Ala Arg Arg Asn Arg Gly Leu Asn Ser Asn Phe Ile Val Arg
    50              55              60
Lys Ile Ser Ser Gly Glu Gly Val Glu Arg Thr Phe Gln Leu Tyr Ser
65              70              75              80
Pro Thr Val Glu Lys Ile Glu Val Lys Arg Arg Gly Asp Val Arg Arg
            85              90              95
Ala Lys Leu Tyr Tyr Leu Arg Gly Leu Thr Gly Lys Ala Ala Arg Ile
        100             105             110
Lys Glu Lys Leu Pro Ala Arg Lys Gly
        115             120
```

<210> 59
<211> 225
<212> PRT
<213> Neisseria meningitidis

<400> 59

137

```
Met Ser Arg Val Leu Leu Val Asp Asp Asp Ala Leu Leu Thr Glu Leu
1               5                   10                  15

Leu Thr Glu Tyr Leu Ser Ala Glu Gly Leu Asn Val Arg Ser Val Pro
            20                  25                  30

Asp Gly Glu Ala Gly Val Gln Glu Ile Leu Ser Gly Gln Tyr Asp Val
        35                  40                  45

Val Val Leu Asp Ser Met Met Pro Lys Met Asn Gly Leu Asp Val Leu
    50                  55                  60

Lys Asn Val Arg Ala Arg Ser Thr Val Pro Ile Ile Met Leu Thr Ala
65                  70                  75                  80

Lys Gly Asp Asp Ile Asp Arg Ile Ile Gly Leu Glu Met Gly Ala Asp
            85                  90                  95

Asp Tyr Val Pro Lys Pro Cys Thr Pro Arg Glu Leu Leu Ala Arg Ile
            100                 105                 110

Asn Ala Ile Leu Arg Arg Ala Gln His Ser Gly Glu Gln Asn Asn Ala
            115                 120                 125

Pro Asn Ser Ile Ser Val Ser Asp Val Val Leu Tyr Pro Ala Lys Arg
    130                 135                 140

Gln Ala Ser Val Lys Asp Met Pro Leu Glu Leu Thr Ser Thr Glu Phe
145                 150                 155                 160

Asn Leu Leu Glu Val Leu Met Arg His Ala Gly Gln Val Val Ser Lys
            165                 170                 175

Glu Thr Leu Ser Val Glu Ala Leu Asp Arg Lys Leu Ala Lys Phe Asp
            180                 185                 190

Arg Ser Ile Asp Val His Ile Ser Ser Ile Arg His Lys Leu Gly Asp
        195                 200                 205

Ala Ser Leu Ile Gln Thr Val Arg Gly Leu Gly Tyr Leu Phe Val Lys
    210                 215                 220

Asn
225
```

<210> 60
<211> 354
<212> PRT
<213> Neisseria meningitidis

<400> 60

```
Met Lys Ala Ala Arg Phe Tyr Asp Lys Gly Asp Ile Arg Ile Glu Asp
1               5                   10                  15

Ile Pro Glu Pro Thr Val Ala Pro Gly Thr Val Gly Ile Asn Val Ala
            20                  25                  30

Trp Cys Gly Ile Cys Gly Thr Asp Leu His Glu Phe Met Glu Gly Pro
```

138

```
                35                      40                      45
   Ile Phe Ile Pro Pro Cys Gly His Pro His Pro Ile Ser Gly Glu Ser
       50                  55                  60
   Ala Pro Val Thr Met Gly His Glu Phe Ser Gly Val Val Tyr Ala Val
   65                  70                  75                  80
   Gly Glu Gly Val Asp Asp Ile Lys Val Gly Gln His Val Val Val Glu
                   85                  90                  95
   Pro Tyr Ile Ile Arg Asp Asp Val Pro Thr Gly Glu Gly Ser Asn Tyr
               100                 105                 110
   His Leu Ser Lys Asp Met Asn Phe Ile Gly Leu Gly Gly Cys Gly Gly
           115                 120                 125
   Gly Leu Ser Glu Lys Ile Ala Val Lys Arg Arg Trp Val His Pro Ile
       130                 135                 140
   Ser Asp Lys Ile Pro Leu Asp Gln Ala Ala Leu Ile Glu Pro Leu Ser
   145                 150                 155                 160
   Val Gly His His Ala Tyr Val Arg Ser Gly Ala Lys Glu Gly Asp Val
                   165                 170                 175
   Ala Leu Val Gly Gly Ala Gly Pro Ile Gly Leu Leu Leu Ala Ala Val
               180                 185                 190
   Leu Lys Ala Lys Gly Ile Lys Val Ile Ile Thr Glu Leu Ser Lys Ala
           195                 200                 205
   Arg Lys Asp Lys Ala Arg Glu Ser Gly Val Ala Asp Tyr Ile Leu Asp
       210                 215                 220
   Pro Ser Glu Val Asp Val Val Ala Glu Val Lys Lys Leu Thr Asn Gly
   225                 230                 235                 240
   Glu Gly Val Asp Val Ala Phe Glu Cys Thr Ser Val Asn Lys Val Leu
                   245                 250                 255
   Asp Thr Leu Val Glu Ala Cys Lys Pro Ala Ala Asn Leu Val Ile Val
               260                 265                 270
   Ser Ile Trp Ser His Pro Ala Thr Ile Asn Val His Ser Val Val Met
           275                 280                 285
   Lys Glu Leu Asp Val Arg Gly Thr Ile Ala Tyr Cys Asn Asp His Ala
       290                 295                 300
   Glu Thr Ile Lys Leu Val Glu Glu Gly Lys Ile Asn Leu Glu Pro Phe
   305                 310                 315                 320
   Ile Thr Gln Arg Ile Lys Leu Asp Glu Leu Val Ser Lys Gly Phe Glu
               325                 330                 335
   Arg Leu Ile His Asn Asn Glu Ser Ala Val Lys Ile Ile Val Ser Pro
           340                 345                 350
   Asn Leu
```

<210> 61
<211> 419
<212> PRT
<213> Neisseria meningitidis

<400> 61

Met Leu Pro Val Arg Phe Thr Arg Val Ser Asp Gly Ile Lys Arg Leu
1               5               10              15

```
Thr Pro Ala Ser Asn Gln Thr Ala Phe Tyr His Pro Phe Glu Asn Pro
            20                  25                  30

Phe Cys Arg Tyr Ser Ser Phe Tyr Trp Ser Ile Ala Ile Met Thr Ala
        35                  40                  45

Thr Thr Ala Ser Ser Ala Lys Pro Tyr Leu Lys Ile Gln Gly Leu Val
    50                  55                  60

Lys Lys Phe Gly Asp Asn Tyr Ala Val Asp Asn Ile Asp Leu Asp Ile
65                  70                  75                  80

Tyr Gln His Glu Ile Phe Ala Leu Leu Gly Ser Ser Gly Ser Gly Lys
                85                  90                  95

Ser Thr Leu Leu Arg Met Leu Ala Gly Met Glu Ser Pro Asn Gln Gly
            100                 105                 110

Lys Ile Ile Leu Asp Gly Gln Asp Ile Thr Lys Leu Ala Pro Tyr Asp
        115                 120                 125

Arg Pro Ile Asn Met Met Phe Gln Ser Tyr Ala Leu Phe Pro His Met
    130                 135                 140

Thr Val Glu Gln Asn Ile Ala Phe Gly Leu Lys Gln Asp Lys Met Pro
145                 150                 155                 160

Lys Gly Glu Ile Ala Ala Arg Val Glu Glu Met Leu Arg Leu Val Gln
            165                 170                 175

Met Thr Lys Phe Ala Lys Arg Lys Pro His Gln Leu Ser Gly Gly Gln
            180                 185                 190

Gln Gln Arg Ile Ala Leu Ala Arg Ser Leu Ala Lys Arg Pro Lys Ile
        195                 200                 205

Leu Leu Leu Asp Glu Pro Leu Gly Ala Leu Asp Lys Lys Leu Arg Gln
    210                 215                 220

Gln Thr Gln Leu Glu Leu Val Asn Thr Leu Glu Gln Val Gly Val Thr
225                 230                 235                 240

Cys Ile Met Val Thr His Asp Gln Glu Glu Ala Met Thr Met Ala Thr
            245                 250                 255

Arg Ile Ala Ile Met Ser Asp Gly Gln Leu Gln Gln Val Gly Thr Pro
        260                 265                 270

Ser Asp Val Tyr Asp Tyr Pro Asn Ser Arg Phe Thr Ala Glu Phe Ile
    275                 280                 285

Gly Glu Thr Asn Ile Phe Asp Gly Val Val Ile Glu Asp His Ala Asp
    290                 295                 300

Tyr Ala Val Ile Glu Cys Glu Gly Leu Glu Asn His Val Arg Ile Asp
305                 310                 315                 320

His Gly Leu Gly Gly Pro Ser Glu Gln Asp Leu Trp Val Ser Ile Arg
            325                 330                 335

Pro Glu Asp Ile Asp Leu Tyr Lys Glu Lys Pro Glu Tyr Leu Gly Asp
        340                 345                 350

Tyr Asn Trp Ala Lys Gly Thr Val Lys Glu Ile Ala Tyr Leu Gly Ser
        355                 360                 365

Phe Ala Ile Tyr His Ile Lys Leu Gly Asn Gly Arg Val Val Lys Ser
    370                 375                 380
```

```
Gln Val Pro Ala Pro Tyr Trp Tyr Val Arg‾ Asn Ile Thr Pro Pro Thr
385                 390             395                     400

Trp Asp Glu Thr Val Tyr Ile Ser Trp Pro Glu Asn Gln Pro Thr Pro
                405             410                     415

Leu Phe Arg
```

<210> 62
<211> 419
<212> PRT
<213> Neisseria meningitidis

<400> 62

```
Met His Val Ser Glu Leu Gln Thr Leu His Ile Ser Lys Leu Leu Glu
1               5               10              15

Leu Ala Glu Glu His Gly Ile Glu Asn Ala Asn Arg Phe Arg Lys Gln
            20              25              30

Asp Leu Val Phe Ala Ile Val Arg Gln Met Met Lys Lys Gly Glu Gly
        35              40              45

Phe Thr Cys Ser Gly Thr Leu Glu Ile Leu Pro Asp Gly Phe Gly Phe
    50              55              60

Leu Arg Ser Ala Asp Thr Ser Tyr Leu Ala Gly Pro Asp Asp Ile Tyr
65              70              75              80

Val Ser Pro Thr Gln Ile Arg Arg Phe Asn Leu His Thr Gly Asp Thr
            85              90              95

Ile Glu Gly Ser Val Arg Val Pro Lys Asp Asn Glu Arg Tyr Phe Ala
            100             105             110

Leu Val Arg Leu Asp Thr Ile Asn Gly Asp His Pro Glu Val Cys Arg
            115             120             125

His Lys Ile Leu Phe Glu Asn Leu Thr Pro Leu Phe Pro Thr Glu Gln
    130             135             140

Leu Lys Leu Glu Arg Asp Leu Lys Ser Glu Glu Asn Leu Thr Gly Arg
145             150             155             160

Ala Ile Asp Leu Ile Ser Pro Ile Gly Lys Gly Gln Arg Ala Leu Leu
            165             170             175

Val Ala Pro Pro Lys Ser Gly Lys Thr Val Met Leu Gln Asn Ile Ala
            180             185             190

His Ala Val Thr Ala Asn Tyr Pro Glu Val Glu Leu Ile Val Leu Leu
    195             200             205

Ile Asp Glu Arg Pro Glu Glu Val Thr Glu Met Ser Arg Ser Val Arg
    210             215             220

Gly Glu Val Val Ser Ser Thr Phe Asp Glu Pro Ala Gln Arg His Val
225             230             235             240

Gln Val Ala Glu Met Val Leu Glu Lys Ala Lys Arg Met Val Glu His
            245             250             255

Lys Lys Asp Val Val Ile Leu Leu Asp Ser Ile Thr Arg Leu Ala Arg
            260             265             270

Ala Tyr Asn Thr Val Val Pro Thr Ser Gly Lys Ile Leu Thr Gly Gly
            275             280             285

Val Asp Ala Asn Ala Leu His Arg Pro Lys Arg Phe Phe Gly Ala Ala
```

```
            290                      295                      300
        Arg Asn Val Glu Glu Gly Gly Ser Leu Thr Ile Ile Ala Thr Ala Leu
        305             310             315             320

        Val Glu Thr Gly Ser Arg Met Asp Asp Val Ile Tyr Glu Glu Phe Lys
                        325             330             335

        Gly Thr Gly Asn Met Glu Leu His Leu Asp Arg Arg Met Ala Glu Lys
                    340             345             350

        Arg Leu Phe Pro Ala Ile Asn Ile Asn Lys Ser Gly Thr Arg Arg Glu
                    355             360             365

        Glu Leu Leu Val Pro Asn Asp Gln Leu Gln Arg Met Trp Leu Leu Arg
            370             375             380

        Lys Phe Leu His Pro Met Asp Glu Ile Glu Ala Ala Glu Phe Leu Ile
        385             390             395             400

        Gly Lys Ile Lys Ala Ser Lys Asn Asn Asp Asp Phe Phe Glu Leu Met
                        405             410             415

        Arg Gly Lys
```

<210> 63
<211> 794
<212> PRT
<213> Neisseria meningitidis

<400> 63

```
Met Ala Asp Asn Tyr Val Ile Trp Phe Glu Asn Leu Arg Met Thr Asp
1               5               10              15
Val Glu Arg Val Gly Gly Lys Asn Ala Ser Leu Gly Glu Met Ile Ser
            20              25              30
Gln Leu Thr Glu Lys Gly Val Arg Val Pro Gly Gly Phe Ala Thr Thr
        35              40              45
Ala Glu Ala Tyr Arg Ala Phe Leu Ala His Asn Gly Leu Ser Glu Arg
    50              55              60
Ile Ser Ala Ala Leu Ala Lys Leu Asp Val Glu Asp Val Ala Glu Leu
65              70              75              80
Ala Arg Val Gly Lys Glu Ile Arg Gln Trp Ile Leu Asp Thr Pro Phe
            85              90              95
Pro Glu Gln Leu Asp Ala Glu Ile Glu Ala Ala Trp Asn Lys Met Val
        100             105             110
Ala Asp Ala Gly Gly Ala Asp Ile Ser Val Ala Val Arg Ser Ser Ala
        115             120             125
Thr Ala Glu Asp Leu Pro Asp Ala Ser Phe Ala Gly Gln Gln Glu Thr
    130             135             140
Phe Leu Asn Ile Asn Gly Leu Asp Asn Val Lys Glu Ala Met His His
145             150             155             160
Val Phe Ala Ser Leu Tyr Asn Asp Arg Ala Ile Ser Tyr Arg Val His
            165             170             175
Lys Gly Phe Glu His Asp Ile Val Ala Leu Ser Ala Gly Val Gln Arg
        180             185             190
Met Val Arg Ser Asp Ser Gly Ala Ser Gly Val Met Phe Thr Leu Asp
        195             200             205
```

Thr Glu Ser Gly Tyr Asp Gln Val Val Phe Val Thr Ser Ser Tyr Gly
210                     215                 220

Leu Gly Glu Asn Val Val Gln Gly Ala Val Asn Pro Asp Glu Phe Tyr
225                 230                 235                     240

Val Phe Lys Pro Thr Leu Lys Ala Gly Lys Pro Ala Ile Leu Arg Lys
                245                 250                 255

Thr Met Gly Ser Lys His Ile Lys Met Ile Phe Thr Asp Lys Ala Glu
            260                 265                 270

Ala Gly Lys Ser Val Thr Asn Val Asp Val Pro Glu Glu Asp Arg Asn
        275                 280                 285

Arg Phe Ser Ile Thr Asp Glu Glu Ile Thr Glu Leu Ala His Tyr Ala
    290                 295                 300

Leu Thr Ile Glu Lys His Tyr Gly Arg Pro Met Asp Ile Glu Trp Gly
305                 310                 315                 320

Arg Asp Gly Leu Asp Gly Lys Leu Tyr Ile Leu Gln Ala Arg Pro Glu
                325                 330                 335

Thr Val Lys Ser Gln Glu Glu Gly Asn Arg Asn Leu Arg Arg Phe Ala
            340                 345                 350

Ile Asn Gly Asp Lys Thr Val Leu Cys Glu Gly Arg Ala Ile Gly Gln
            355                 360                 365

Lys Val Gly Gln Gly Lys Val Arg Leu Ile Lys Asp Ala Ser Glu Met
370                 375                 380

Asp Ser Val Glu Ala Gly Asp Val Leu Val Thr Asp Met Thr Asp Pro
385                 390                 395                 400

Asp Trp Glu Pro Val Met Lys Arg Ala Ser Ala Ile Val Thr Asn Arg
                405                 410                 415

Gly Gly Arg Thr Cys His Ala Ala Ile Ile Ala Arg Glu Leu Gly Ile
            420                 425                 430

Pro Ala Val Val Gly Cys Gly Asn Ala Thr Glu Leu Leu Lys Asn Gly
        435                 440                 445

Gln Glu Val Thr Val Ser Cys Ala Glu Gly Asp Thr Gly Phe Ile Tyr
    450                 455                 460

Ala Gly Leu Leu Asp Val Gln Ile Thr Asp Val Ala Leu Asp Asn Met
465                 470                 475                 480

Pro Lys Ala Pro Val Lys Val Met Met Asn Val Gly Asn Pro Glu Leu
            485                 490                 495

Ala Phe Ser Phe Ala Asn Leu Pro Ser Glu Gly Ile Gly Leu Ala Arg
            500                 505                 510

Met Glu Phe Ile Ile Asn Arg Gln Ile Gly Ile His Pro Lys Ala Leu
        515                 520                 525

Leu Glu Phe Asp Lys Gln Asp Asp Glu Leu Lys Ala Glu Ile Thr Arg
    530                 535                 540

Arg Ile Ala Gly Tyr Ala Ser Pro Val Asp Phe Tyr Val Asp Lys Ile
545                 550                 555                 560

Ala Glu Gly Val Ala Thr Leu Ala Ala Ser Val Tyr Pro Arg Lys Thr
                565                 570                 575

146

```
Ile Val Arg Met Ser Asp Phe Lys Ser Asn Glu Tyr Ala Asn Leu Val
            580                 585                 590

Gly Gly Asn Val Tyr Glu Pro His Glu Glu Asn Pro Met Leu Gly Phe
        595                 600                 605

Arg Gly Ala Ala Arg Tyr Val Ala Asp Asn Phe Lys Asp Cys Phe Ala
    610                 615                 620

Leu Glu Cys Lys Ala Leu Lys Arg Val Arg Asp Glu Met Gly Leu Thr
625                 630                 635                 640

Asn Val Glu Ile Met Ile Pro Phe Val Arg Thr Leu Gly Glu Ala Glu
                645                 650                 655

Ala Val Val Lys Ala Leu Lys Glu Asn Gly Leu Glu Arg Gly Lys Asn
            660                 665                 670

Gly Leu Arg Leu Ile Met Met Cys Glu Leu Pro Ser Asn Ala Val Leu
        675                 680                 685

Ala Glu Gln Phe Leu Gln Tyr Phe Asp Gly Phe Ser Ile Gly Ser Asn
    690                 695                 700

Asp Met Thr Gln Leu Thr Leu Gly Leu Asp Arg Asp Ser Gly Leu Val
705                 710                 715                 720

Ser Glu Ser Phe Asp Glu Arg Asn Pro Ala Val Lys Val Met Leu His
                725                 730                 735

Leu Ala Ile Ser Ala Cys Arg Lys Gln Asn Lys Tyr Val Gly Ile Cys
            740                 745                 750

Gly Gln Gly Pro Ser Asp His Pro Asp Phe Ala Lys Trp Leu Val Glu
        755                 760                 765

Glu Gly Ile Glu Ser Val Ser Leu Asn Pro Asp Thr Val Ile Glu Thr
770                 775                 780

Trp Leu Tyr Leu Ala Asn Glu Leu Asn Lys
785                 790
```

<210> 64
<211> 379
<212> PRT
<213> Neisseria meningitidis

<400> 64

```
Met Lys Lys Thr Leu Val Ala Ala Ala Ile Leu Ser Leu Ala Leu Thr
1               5               10              15

Ala Cys Gly Gly Gly Ser Asp Thr Ala Ala Gln Thr Pro Ser Ala Lys
        20              25              30

Pro Glu Ala Glu Gln Ser Gly Lys Leu Asn Ile Tyr Asn Trp Ser Asp
        35              40              45

Tyr Val Asp Pro Glu Thr Val Ala Ala Phe Glu Lys Glu Thr Gly Ile
    50              55              60

Lys Thr Arg Ser Asp Tyr Tyr Asp Ser Asn Glu Thr Leu Glu Ala Lys
65              70              75              80

Val Leu Thr Gly Lys Ser Gly Tyr Asp Leu Thr Ala Pro Ser Ile Ala
            85              90              95

Asn Val Gly Arg Gln Ile Lys Ala Gly Ala Tyr Gln Lys Ile Asp Lys
        100             105             110

Ala Gln Ile Pro His Tyr Gly Asn Ile Asp Lys Asp Leu Leu Lys Met
```

```
                115                    120                    125

      Met Glu Ala Val Asp Pro Gly Asn Glu Tyr Ala Val Pro Tyr Phe Trp
          130                 135             140

      Gly Ile Asn Thr Leu Ala Ile Asn Thr Gln Gln Val Lys Lys Ala Leu
      145                 150                 155                 160

      Gly Thr Asp Lys Leu Pro Glu Asn Glu Trp Asp Leu Val Phe Lys Pro
                  165                 170                 175

      Glu Tyr Thr Ala Lys Leu Lys Ser Cys Gly Ile Ser Tyr Phe Asp Ser
                  180                 185                 190

      Ala Ile Glu Gln Ile Pro Leu Ala Leu His Tyr Leu Gly Lys Asp Pro
              195                 200                 205

      Asn Ser Glu Asn Pro Glu Asp Ile Lys Ala Ala Val Asp Met Met Lys
          210                 215                 220

      Ala Val Arg Gly Asp Val Lys Arg Phe Ser Ser Ser Gly Tyr Ile Asp
      225                 230                 235                 240

      Asp Met Ala Ala Gly Asn Leu Cys Ala Ala Ile Gly Tyr Gly Gly Asp
                  245                 250                 255

      Leu Asn Ile Ala Lys Thr Arg Ala Glu Glu Ala Ala Asn Gly Val Glu
                  260                 265                 270

      Ile Lys Val Leu Thr Pro Lys Thr Gly Val Gly Val Trp Val Asp Ser
                  275                 280                 285

      Phe Met Ile Pro Arg Asp Ala Gln Asn Val Ala Asn Ala His Arg Tyr
          290                 295                 300

      Ile Asp Tyr Thr Leu Arg Pro Glu Val Ala Ala Lys Asn Gly Ser Phe
      305                 310                 315                 320

      Val Thr Tyr Ala Pro Ala Ser Arg Pro Ala Arg Glu Leu Met Asp Glu
                  325                 330                 335

      Lys Tyr Thr Ser Asp Ala Ser Ile Phe Pro Asn Lys Glu Leu Met Glu
                  340                 345                 350

      Lys Ser Phe Ile Val Ser Pro Lys Ser Ala Glu Ser Val Lys Leu Gly
                  355                 360                 365

      Val Lys Leu Trp Gln Gly Leu Lys Ala Gly Lys
          370                 375
```

<210> 65
<211> 500
<212> PRT
<213> Neisseria meningitidis

<400> 65

Met Ala Lys Val Leu Ile Val Pro Val Ser Ala Gly Leu Asp Ala Ser
1               5                   10              15

Ala Ala Ala Gln Ala Phe Ala Lys Ala Leu Asp Ala Gln Ile Phe Gln
            20              25                  30

Ala Val Asp Ala Thr Ala Glu Thr Leu Leu Ala Gln Gly Lys Ser Asp
            35              40                  45

Asp Trp Phe Asp Ala Leu Val Gly Lys Val Ala Ala Leu Asp Ala Ala
        50              55                  60

Asn Leu Val Ile Glu Gly Ile Ala Pro Asp Ala Asp Lys Ile Tyr Leu
65              70                  75                  80

Ala Gly Lys Asn Val Glu Leu Ala Leu Ser Leu Asp Ala Ala Ala Val
85 90 95

Phe Ala Val Arg Ser Asp Asn Ala Asp Ala Asp Glu Leu Ala Asn Arg
100 105 110

Val Asn Leu Ala Lys Gln Phe Phe Ala Ala Ala Pro Gly Val Leu Glu
115 120 125

Gly Phe Val Val Asp Gly Ala Ala Ala Ser Val Ala Glu Ala Ala Ala
130 135 140

Glu Lys Thr Gly Leu Thr Phe Phe Gly Ser Ser Asp Ala Leu Lys Asp
145 150 155 160

Val Ser Val Leu Ala Gly Arg Glu Ala Lys Arg Leu Ser Pro Ala Gln
165 170 175

Phe Arg Tyr Asn Leu Ile Asp Phe Ala Arg Gln Ala Asp Lys Arg Ile
180 185 190

Val Leu Pro Glu Gly Ala Glu Pro Arg Thr Val Gln Ala Ala Ala Ile
195 200 205

Cys His Glu Lys Gly Ile Ala Arg Cys Val Leu Leu Ala Lys Arg Glu
210 215 220

Glu Val Glu Ala Val Ala Lys Glu Arg Gly Ile Ser Leu Pro Asp Ser
225 230 235 240

Leu Glu Ile Ile Asp Pro Ala Ser Leu Val Glu Gln Tyr Val Glu Pro
245 250 255

Met Cys Glu Leu Arg Lys Ser Lys Gly Leu Thr Pro Glu Asp Ala Arg
260 265 270

Lys Gln Leu Gln Asp Thr Val Val Leu Gly Thr Met Met Met Ala Gln
275 280 285

Asn Asp Val Asp Gly Leu Val Ser Gly Ala Val His Thr Thr Ala Asn
290 295 300

Thr Ile Arg Pro Ala Leu Gln Leu Ile Lys Thr Ala Pro Gly Ala Ser
305 310 315 320

Leu Val Ser Ser Val Phe Phe Met Leu Leu Pro Asn Gln Val Leu Val
325 330 335

Phe Gly Asp Cys Ala Val Asn Pro Asn Pro Thr Ala Gln Gln Leu Ala
340 345 350

Asp Ile Ala Ile Gln Ser Ala Asp Ser Ala Lys Ala Phe Gly Ile Asp
355 360 365

Pro Lys Val Ala Met Ile Ser Tyr Ser Thr Val Asn Ser Gly Ser Gly
370 375 380

Pro Asp Val Asp Thr Val Ile Glu Ala Thr Lys Leu Ala Arg Glu Lys
385 390 395 400

Arg Pro Asp Leu Ala Ile Asp Gly Pro Leu Gln Tyr Asp Ala Ala Thr
405 410 415

Val Pro Gly Val Gly Lys Ser Lys Ala Pro Gly Ser Pro Val Ala Gly
420 425 430

Gln Ala Thr Val Leu Val Phe Pro Asp Leu Asn Thr Gly Asn Cys Thr
435 440 445

```
Tyr Lys Ala Val Gln Arg Asn Ala Asn Val Leu Ser Val Gly Pro Leu
    450                 455             460

Leu Gln Gly Leu Arg Lys Pro Val Asn Asp Leu Ser Arg Gly Ala Leu
465             470             475                 480

Val Glu Asp Ile Val Phe Thr Ile Ala Leu Thr Ala Val Gln Ala Lys
                485                 490                 495

Gln Met Glu Gly
            500
```

<210> 66
<211> 331
<212> PRT
<213> Neisseria meningitidis

<400> 66

```
Met Lys Thr Ser Ile Arg Tyr Ala Leu Leu Ala Ala Ala Leu Thr Ala
1               5              10                  15

Ala Thr Pro Ala Leu Ala Asp Ile Thr Val Tyr Asn Gly Gln His Lys
            20              25                  30

Glu Ala Ala Gln Ala Val Ala Asp Ala Phe Thr Arg Ala Thr Gly Ile
        35              40              45

Lys Val Lys Leu Asn Ser Ala Lys Gly Asp Gln Leu Ala Gly Gln Ile
    50              55              60

Lys Glu Glu Gly Ser Arg Ser Pro Ala Asp Val Phe Tyr Ser Glu Gln
65              70              75              80

Ile Pro Ala Leu Ala Thr Leu Ser Ala Ala Asn Leu Leu Glu Pro Leu
            85              90                  95

Pro Ala Ser Thr Ile Asn Glu Thr Arg Gly Lys Gly Val Pro Val Ala
            100             105                 110

Ala Lys Lys Asp Trp Val Ala Leu Ser Gly Arg Ser Arg Val Val Val
        115             120                 125

Tyr Asp Thr Arg Lys Leu Ser Glu Lys Asp Leu Glu Lys Ser Val Leu
    130             135             140

Asn Tyr Ala Thr Pro Lys Trp Lys Asn Arg Ile Gly Tyr Ala Pro Thr
145             150             155             160

Ser Gly Ala Phe Leu Glu Gln Val Val Ala Ile Val Lys Leu Lys Gly
            165             170                 175

Glu Ala Ala Ala Leu Lys Trp Leu Lys Gly Leu Lys Glu Tyr Gly Lys
            180             185                 190

Pro Tyr Ala Lys Asn Ser Val Ala Leu Gln Ala Val Glu Asn Gly Glu
    195             200                 205

Ile Asp Ala Ala Leu Ile Asn Asn Tyr Tyr Trp His Ala Phe Ala Arg
    210             215             220

Glu Lys Gly Val Gln Asn Val His Thr Arg Leu Asn Phe Val Arg His
225             230             235             240

Arg Asp Pro Gly Ala Leu Val Thr Tyr Ser Gly Ala Ala Val Leu Lys
            245             250                 255

Ser Ser Gln Asn Lys Asp Glu Ala Lys Lys Phe Val Ala Phe Leu Ala
            260             265                 270

Ser Lys Glu Gly Gln Arg Ala Leu Thr Ala Val Arg Ala Glu Tyr Pro
275                     280                     285

Leu Asn Pro His Val Val Ser Thr Phe Asn Leu Glu Pro Ile Ala Lys
    290             295             300

Leu Glu Ala Pro Gln Val Ser Ala Thr Thr Val Ser Glu Lys Glu His
305             310             315             320

Ala Thr Arg Leu Leu Glu Gln Ala Gly Met Lys
            325             330
```

<210> 67
<211> 458
<212> PRT

153

<213> Neisseria meningitidis

<400> 67

```
Met His Asp Lys Thr Trp Ser Gly Arg Phe Asn Glu Pro Val Ser Glu
1               5                  10                 15

Leu Val Lys Gln Tyr Thr Ala Ser Ile Gly Phe Asp Arg Arg Leu Ala
            20                  25              30

Glu Trp Asp Ile Gln Gly Ser Leu Ala His Ala Gln Met Leu Lys Glu
        35              40                  45

Thr Gly Val Leu Asp Glu Gly Asp Leu Ala Asp Ile Arg Arg Gly Met
    50              55                  60

Ala Glu Ile Leu Glu Glu Ile Arg Ser Gly Lys Ile Glu Trp Ser Ser
65              70                  75                  80

Asp Leu Glu Asp Val His Met Asn Ile Glu Arg Arg Leu Thr Asp Lys
                85              90                  95

Ile Gly Asp Ala Gly Lys Arg Leu His Thr Gly Arg Ser Arg Asn Asp
            100             105             110

Gln Val Ala Thr Asp Ile Arg Leu Trp Leu Arg Asp Gln Ile Thr Val
        115             120             125

Ile Gln Ser Leu Ile Gln Ser Leu Gln Thr Ala Leu Leu Asp Leu Ala
    130             135             140

Glu Gln Asn Ala Glu Thr Val Met Pro Gly Phe Thr His Leu Gln Val
145             150             155             160

Ala Gln Pro Val Ser Phe Gly His His Met Leu Ala Tyr Val Glu Met
                165             170             175

Leu Gly Arg Asp Asn Glu Arg Met Ala Asp Cys Arg Cys Arg Val Asn
            180             185             190

Arg Met Pro Leu Gly Ala Ala Ala Leu Ala Gly Thr Thr Tyr Pro Ile
        195             200             205

Gln Arg Glu Ile Thr Ala Glu Leu Leu Gly Phe Glu Gln Ile Cys Gln
    210             215             220

Asn Ser Leu Asp Ala Val Ser Asp Arg Asp Phe Ala Ile Glu Phe Thr
225             230             235             240

Ala Ala Ala Ser Leu Val Met Val His Leu Ser Arg Leu Ser Glu Glu
            245             250             255

Leu Ile Leu Trp Met Ser Pro Arg Phe Gly Phe Ile Asp Ile Ala Asp
        260             265             270

Arg Phe Cys Thr Gly Ser Ser Ile Met Pro Gln Lys Lys Asn Pro Asp
        275             280             285
```

```
Val Pro Glu Leu Val Arg Gly Lys Ser Gly Arg Val Ile Gly His Leu
    290                 295             300

Ile Gly Leu Ile Thr Leu Met Lys Ser Gln Pro Leu Ala Tyr Asn Lys
305             310                 315                 320

Asp Asn Gln Glu Asp Lys Glu Pro Leu Phe Asp Thr Ala Asp Thr Leu
            325             330                 335

Ile Asp Thr Leu Arg Ile Tyr Ala Asp Met Met Arg Gly Val Thr Val
            340             345                 350

Lys Pro Asp Asn Met Arg Ala Ala Val Met Gln Gly Phe Ala Thr Ala
        355             360             365

Thr Asp Leu Ala Asp Tyr Leu Val Lys Lys Gly Met Pro Phe Arg Asp
    370             375             380

Ala His Glu Val Val Ala Gln Ala Val Arg His Ala Asp Gln Ala Gly
385             390             395                 400

Val Asp Leu Ser Glu Leu Pro Leu Glu Val Leu Gln Gly Phe Ser Asp
            405             410                 415

Leu Ile Ala Asp Asp Val Tyr Gly Val Leu Thr Pro Glu Gly Ser Leu
            420             425             430

Asn Ala Arg Asn His Leu Gly Gly Thr Ala Pro Glu Gln Val Arg Phe
        435             440             445

Gln Val Lys Arg Trp Arg Glu Met Leu Ala
    450             455
```

<210> 68
<211> 177
<212> PRT
<213> Neisseria meningitidis

<400> 68

```
Met Ala Asp Phe Asn Gln Ile Leu Thr Pro Gly Asp Val Asp Gly Gly
1               5               10              15
Ile Ile Asn Val Val Asn Glu Ile Pro Ala Gly Ser Asn His Lys Ile
            20              25              30
Glu Trp Asn Arg Lys Leu Ala Ala Phe Gln Leu Asp Arg Val Glu Pro
        35              40              45
Ala Ile Phe Ala Lys Pro Thr Asn Tyr Gly Phe Ile Pro Gln Thr Leu
    50              55              60
Asp Glu Asp Gly Asp Glu Leu Asp Val Leu Leu Val Thr Glu Gln Pro
65              70              75              80
Leu Ala Thr Gly Val Phe Leu Glu Ala Arg Val Ile Gly Val Met Lys
            85              90              95
Phe Val Asp Asp Gly Glu Val Asp Asp Lys Ile Val Cys Val Pro Ala
        100             105             110
Asp Asp Arg Asn Asn Gly Asn Ala Tyr Lys Thr Leu Ser Asp Leu Pro
        115             120             125
Gln Gln Leu Ile Lys Gln Ile Glu Phe His Phe Asn His Tyr Lys Asp
    130             135             140
Leu Lys Lys Ala Gly Thr Thr Lys Val Glu Ser Trp Gly Asp Ala Glu
145             150             155             160

Glu Ala Lys Lys Val Ile Lys Glu Ser Ile Glu Arg Trp Asn Lys Gln
                165             170             175
Ala
```

<210> 69
<211> 174
<212> PRT
<213> Neisseria meningitidis

<400> 69

Met Lys Lys Ala Leu Ala Thr Leu Ile Ala Leu Ala Leu Pro Ala Ala
1               5                   10                  15

Ala Leu Ala Glu Gly Ala Ser Gly Phe Tyr Val Gln Ala Asp Ala Ala
            20                  25                  30

His Ala Lys Ala Ser Ser Ser Leu Gly Ser Ala Lys Gly Phe Ser Pro
        35                  40                  45

Arg Ile Ser Ala Gly Tyr Arg Ile Asn Asp Leu Arg Phe Ala Val Asp
    50                  55                  60

Tyr Thr Arg Tyr Lys Asn Tyr Lys Ala Pro Ser Thr Asp Phe Lys Leu
65                  70                  75                  80

Tyr Ser Ile Gly Ala Ser Ala Ile Tyr Asp Phe Asp Thr Gln Ser Pro
            85                  90                  95

Val Lys Pro Tyr Leu Gly Ala Arg Leu Ser Leu Asn Arg Ala Ser Val
            100                 105                 110

Asp Leu Gly Gly Ser Asp Ser Phe Ser Gln Thr Ser Ile Gly Leu Gly
        115                 120                 125

Val Leu Thr Gly Val Ser Tyr Ala Val Thr Pro Asn Val Asp Leu Asp
    130                 135                 140

Ala Gly Tyr Arg Tyr Asn Tyr Ile Gly Lys Val Asn Thr Val Lys Asn
145                 150                 155                 160

Val Arg Ser Gly Glu Leu Ser Ala Gly Val Arg Val Lys Phe
                165                 170

<210> 70
<211> 259
<212> PRT
<213> Neisseria meningitidis

<400> 70

Met Lys Glu His Lys Ala Arg Lys Arg Phe Gly Gln Asn Phe Leu Gln
1               5                   10                  15

Asp Thr Arg Ile Ile Ser Asp Ile Val Asn Ala Val Arg Pro Gln Ala
            20                  25                  30

Asp Asp Val Val Ile Glu Ile Gly Pro Gly Leu Ala Ala Ile Thr Glu
        35                  40                  45

Pro Leu Ala Lys Lys Leu Asn Arg Leu His Val Val Glu Ile Asp Arg
    50                  55                  60

Asp Ile Val Cys Arg Leu Lys Thr Leu Pro Phe Ala Asp Lys Leu Val
65                  70                  75                  80

Ile His Glu Gly Asp Val Leu Gln Phe Asp Phe Asn Gly Ile Ala Gly
            85                  90                  95

Lys Lys Lys Ile Val Gly Asn Leu Pro Tyr Asn Ile Ser Thr Pro Leu

```
                    100                        105                        110

     Leu Phe Lys Leu Ala Glu Val Ala Asp Asp Val Val Asp Met His Phe
             115                 120                 125

     Met Leu Gln Lys Glu Val Val Glu Arg Met Val Ala Ala Pro Lys Ser
             130                 135                 140

     Asn Asp Tyr Gly Arg Leu Gly Val Met Leu Gln Tyr Phe Phe Asp Met
     145                 150                 155                 160

     Glu Met Leu Ile Asp Val Pro Pro Glu Ser Phe Asp Pro Ala Pro Lys
                     165                 170                 175

     Val Asp Ser Ala Val Val Arg Met Ile Pro Val Lys His Arg Ile Gly
                 180                 185                 190

     Lys Ala Asp Asp Phe Glu His Phe Ala Lys Leu Val Lys Leu Ala Phe
                 195                 200                 205

     His Gln Arg Arg Lys Thr Ile Arg Asn Asn Leu Lys Glu Leu Ala Gly
             210                 215                 220

     Asp Asp Asp Leu Gln Ala Val Gly Ile Asn Pro Gln Asp Arg Ala Glu
     225                 230                 235                 240

     His Ile Ala Pro Glu Lys Tyr Val Ala Leu Ser Asn Tyr Leu Ala Gly
                     245                 250                 255

     Lys Val Val
```

<210> 71
<211> 267
<212> PRT
<213> Neisseria meningitidis

<400> 71

EP 2 279 746 B1

```
Met Lys Lys Ile Leu Leu Thr Val Ser Leu Gly Leu Ala Leu Ser Ala
1               5               10              15

Cys Ala Thr Gln Gly Thr Val Asp Lys Asp Ala Gln Ile Thr Gln Asp
            20              25              30

Trp Ser Val Glu Lys Leu Tyr Ala Glu Ala Gln Asp Glu Leu Asn Ser
        35              40              45

Ser Asn Tyr Thr Arg Ala Val Lys Leu Tyr Glu Ile Leu Glu Ser Arg
        50              55              60

Phe Pro Thr Ser Arg His Ala Gln Gln Ser Gln Leu Asp Thr Ala Tyr
65              70              75              80

Ala Tyr Tyr Lys Asp Asp Glu Lys Asp Lys Ala Leu Ala Ala Ile Asp
            85              90              95

Arg Phe Arg Arg Leu His Pro Gln His Pro Asn Met Asp Tyr Ala Leu
        100             105             110

Tyr Leu Arg Gly Leu Val Leu Phe Asn Glu Asp Gln Ser Phe Leu Asn
        115             120             125

Lys Leu Ala Ser Gln Asp Trp Ser Asp Arg Asp Pro Lys Ala Asn Arg
    130             135             140

Glu Ala Tyr Gln Ala Phe Ala Glu Leu Val Gln Arg Phe Pro Asn Ser
145             150             155             160

Lys Tyr Ala Ala Asp Ala Thr Ala Arg Met Val Lys Leu Val Asp Ala
            165             170             175

Leu Gly Gly Asn Glu Met Ser Val Ala Arg Tyr Tyr Met Lys Arg Gly
            180             185             190

Ala Tyr Ile Ala Ala Ala Asn Arg Ala Gln Lys Ile Ile Gly Ser Tyr
        195             200             205

Gln Asn Thr Arg Tyr Val Glu Glu Ser Leu Ala Ile Leu Glu Leu Ala
    210             215             220

Tyr Lys Lys Leu Asp Lys Pro Arg Leu Ala Ala Asp Thr Arg Arg Val
225             230             235             240

Leu Glu Thr Asn Phe Pro Lys Ser Pro Phe Leu Lys Gln Pro Trp Arg
            245             250             255

Ser Asp Asp Met Pro Trp Trp Arg Tyr Trp His
            260             265
```

<210> 72
<211> 294
<212> PRT
<213> Neisseria meningitidis

<400> 72

```
Met Ser Ser Gly Asn Ile His Ile His Ser Met Thr Gly Phe Ala Asn
1               5               10                  15

Ala Ala Ala Glu Cys Gly Ser Lys Arg Ile Asn Leu Asp Leu Arg Ala
            20              25              30

Val Asn His Arg Phe Leu Asp Ile Gln Ile Arg Met Pro Asp Asp Leu
        35              40              45

Arg Tyr Leu Glu Ser Gly Ile Arg Glu Lys Ile Ser Ser His Ile Ala
    50              55              60

Arg Gly Lys Val Glu Cys Lys Ile Gln Ile Gln Asp Ala Glu Asn Gly
65              70              75                  80

Ser Gln Ser Leu Glu Leu Asn Arg Asp Leu Val Gly Gln Leu Ala Glu
            85              90              95

Ile Asn Lys Asp Leu Arg Lys His His Asp Leu Ala Lys Leu Gly Val
            100             105             110

Ala Asp Ile Leu Arg Phe Pro Gly Val Leu Ala Ser Gln Arg Glu Asn
        115             120             125

Thr Glu Glu Leu Ala Lys Ser Ile Thr Glu Leu Thr Glu Lys Ala Leu
    130             135             140

Lys Asp Phe Thr Ala Ala Arg Lys Arg Glu Gly Lys Lys Leu Gly Glu
145             150             155             160

His Leu Leu Gln Arg Leu Glu Ala Met Glu Glu Ile Ile Asp Ala Leu
            165             170             175

Ser Glu Leu Phe Pro Thr Leu Leu Glu Thr His Lys Glu Lys Ile Arg
            180             185             190

Ala Arg Leu Val Glu Ala Val Gly Ser Ile Asp Asn Asp Arg Leu Gln
        195             200             205

Gln Glu Phe Ala Leu Phe Ile Gln Lys Ser Asp Ile Asp Glu Glu Phe
    210             215             220

Ser Arg Leu Arg Thr His Ile Ala Glu Val Arg Arg Ile Val Thr Glu
225             230             235             240

His Lys Gly Ser Ser Gly Lys Arg Leu Asp Phe Leu Met Gln Glu Leu
            245             250             255

Asn Arg Glu Ala Asn Thr Leu Gly Ser Lys Ser Ile Ala Ala Glu Cys
        260             265             270

Thr Gln Ala Ser Val Glu Leu Lys Val Leu Ile Glu Gln Met Arg Glu
    275             280             285

Gln Val Gln Asn Ile Glu
    290
```

<210> 73
<211> 637
<212> PRT
<213> Neisseria meningitidis

<400> 73

```
Met Leu Asn Ile Thr Leu Pro Asp Gly Ser Val Arg Gln Tyr Glu Ser
1               5                   10                  15

Pro Val Thr Val Ala Gln Ile Ala Ala Ser Ile Gly Ala Gly Leu Ala
            20                  25                  30

Lys Ala Thr Val Ala Gly Arg Val Asn Gly Lys Leu Val Asp Ala Cys
        35                  40                  45

Asp Pro Ile Val Glu Asp Ser Ala Val Gln Ile Ile Thr Pro Lys Asp
        50                  55                  60

Gln Glu Gly Ile Glu Ile Ile Arg His Ser Cys Ala His Leu Val Gly
65                  70                  75                  80

His Ala Val Lys Gln Leu Tyr Pro Asn Ala Lys Met Val Ile Gly Pro
                85                  90                  95

Val Ile Glu Glu Gly Phe Tyr Tyr Asp Ile Ala Thr Glu Lys Pro Phe
            100                 105                 110

Thr Pro Glu Asp Val Ala Ala Ile Glu Ala Arg Met Lys Glu Leu Ile
        115                 120                 125

Ala Gln Asp Tyr Asp Val Val Lys Ile Met Thr Pro Arg Ala Glu Ala
    130                 135                 140

Ile Lys Ile Phe Gln Glu Arg Gly Glu Glu Tyr Lys Leu Arg Leu Ile
145                 150                 155                 160

Asp Asp Met Pro Glu Val Glu Ala Met Gly Met Tyr His His Gln Glu
            165                 170                 175

Tyr Val Asp Met Cys Arg Gly Pro His Val Pro Asn Thr Arg Phe Leu
            180                 185                 190

Lys Asn Phe Lys Leu Thr Lys Leu Ala Gly Ala Tyr Trp Arg Gly Asp
        195                 200                 205

Ser Asn Asn Glu Met Leu Gln Arg Ile Tyr Gly Thr Ala Trp Ala Thr
        210                 215                 220

Lys Asp Glu Leu Lys Ala Tyr Ile Gln Arg Ile Glu Glu Ala Glu Lys
225                 230                 235                 240

Arg Asp His Arg Lys Leu Gly Lys Gln Leu Asp Leu Phe His Leu Gln
            245                 250                 255

Asp Glu Ala Pro Gly Met Val Phe Trp His Pro Lys Gly Trp Ala Leu
            260                 265                 270

Trp Gln Val Ile Glu Gln His Met Arg Lys Glu Leu Asn Ala Ala Gly
```

```
              275                   280                   285
    Tyr Lys Glu Val Lys Thr Pro Gln Ile Met Asp Lys Thr Phe Trp Glu
        290                   295                   300

    Lys Ser Gly His Trp Asp Asn Tyr Lys Asp Asn Met Phe Val Thr Ser
    305                   310                   315                   320

    Ser Glu Lys Arg Glu Tyr Ala Val Lys Pro Met Asn Cys Pro Gly His
                    325                   330                   335

    Val Gln Ile Phe Asn Asn Gly Leu Arg Ser Tyr Arg Asp Leu Pro Met
                    340                   345                   350

    Arg Leu Ala Glu Phe Gly Ser Cys His Arg Asn Glu Pro Ser Gly Ala
                    355                   360                   365

    Leu His Gly Leu Met Arg Val Arg Gly Phe Val Gln Asp Asp Ala His
        370                   375                   380

    Ile Phe Cys Thr Glu Asp Gln Ile Val Ser Glu Ala Arg Ala Phe Asn
    385                   390                   395                   400

    Glu Leu Leu Ile Arg Ile Tyr Lys Gln Phe Gly Phe His Asp Val Ser
                    405                   410                   415

    Val Lys Leu Ser Leu Arg Pro Glu Lys Arg Ala Gly Ser Asp Asp Val
                    420                   425                   430

    Trp Asp Lys Ala Glu Gln Gly Leu Arg Glu Ala Leu Thr Ala Cys Gly
                    435                   440                   445

    Val Glu Trp Gly Glu Leu Pro Gly Glu Gly Ala Phe Tyr Gly Pro Lys
        450                   455                   460

    Ile Glu Tyr His Val Arg Asp Ala Leu Gly Arg Ser Trp Gln Cys Gly
    465                   470                   475                   480

    Thr Leu Gln Leu Asp Phe Val Leu Pro Glu Arg Leu Asn Ala Glu Tyr
                    485                   490                   495

    Val Thr Glu Asn Asn Asp Arg Ala Arg Pro Val Met Leu His Arg Ala
                    500                   505                   510

    Ile Leu Gly Ser Leu Glu Arg Phe Ile Gly Ile Leu Ile Glu Asn His
                    515                   520                   525

    Ala Gly Ser Phe Pro Leu Trp Leu Ala Pro Val Gln Leu Val Ile Met
        530                   535                   540

    Asn Ile Thr Glu Asn Gln Ala Asp Tyr Cys Arg Glu Val Ala Ala Lys
    545                   550                   555                   560

    Leu Gln Ala Ala Gly Phe Arg Ala Glu Leu Asp Leu Arg Asn Glu Lys
                    565                   570                   575

    Ile Gly Tyr Lys Ile Arg Asp Asn Ser Gln Tyr Arg Phe Pro Tyr Gln
                    580                   585                   590

    Ile Val Val Gly Asp Lys Glu Lys Gln Glu Asn Lys Val Ala Val Arg
                    595                   600                   605

    Arg Lys Ala Glu Asp Leu Gly Ser Leu Asp Leu Asp Asp Phe Ile Ala
        610                   615                   620

    Gln Leu Gln Gln Glu Ile Thr Asp Ala Leu Val Asn His
    625                   630                   635
```

162

<210> 74
<211> 330
<212> PRT
<213> Neisseria meningitidis

<400> 74

```
Met Glu Asn Val Asn Arg Ile Val Ala Glu Gly Ile Ala Ala Val Glu
1               5               10              15

Ala Ala Gln Asp Phe Asn Ala Leu Glu Gln Ile Lys Ala Arg Tyr Leu
            20              25              30

Gly Lys Thr Gly Glu Leu Thr Gly Leu Leu Lys Thr Leu Gly Gln Met
        35              40              45

Ser Pro Glu Glu Arg Lys Thr Ile Gly Ala His Ile Asn Glu Cys Lys
    50              55              60

Asn Arg Phe Gln Thr Ala Phe Asn Ala Lys Arg Glu Ala Leu Asn Glu
65              70              75              80

Val Lys Leu Gln Ala Arg Leu Ala Ala Glu Ala Leu Asp Ile Thr Leu
            85              90              95

Pro Gly Arg Ala Gln Glu Gly Gly Ser Leu His Pro Val Thr Leu Thr
            100             105             110

Leu Gln Arg Val Val Glu Leu Phe His Gly Met Gly Phe Glu Val Ala
            115             120             125

Asp Gly Pro Glu Ile Glu Asp Asp Phe His Asn Phe Gln Ala Leu Asn
    130             135             140

Ile Pro Ala Asn His Pro Ala Arg Ala Met Gln Asp Thr Phe Tyr Val
145             150             155             160

Glu Asn Gly Asp Val Leu Arg Thr His Thr Ser Pro Ile Gln Ile Arg
            165             170             175

Tyr Met Leu Asp Lys Lys Glu Pro Pro Ile Arg Ile Ile Ala Pro Gly
            180             185             190

Arg Val Tyr Arg Val Asp Ser Asp Ala Thr His Ser Pro Met Phe His
            195             200             205

Gln Ala Glu Gly Leu Trp Val Glu Glu Gly Val Thr Phe Ala Asp Leu
    210             215             220

Lys Ala Val Phe Thr Asp Phe Ile Arg Arg Phe Phe Glu Arg Asp Asp
225             230             235             240

Leu Gln Val Arg Phe Arg Pro Ser Phe Phe Pro Phe Thr Glu Pro Ser
            245             250             255

Ala Glu Ile Asp Ile Met Gly Glu Asn Gly Lys Trp Leu Glu Val Gly
            260             265             270

Gly Cys Gly Met Val His Pro Asn Val Leu Lys Asn Val Asn Ile Asp
        275             280             285

Pro Glu Lys Tyr Thr Gly Phe Ala Phe Gly Ile Gly Leu Asp Arg Phe
    290             295             300

Ala Met Leu Arg Tyr Asn Val Asn Asp Leu Arg Leu Phe Phe Asp Asn
305             310             315             320

Asp Leu Asn Phe Leu Lys Gln Phe Ala Lys
            325             330
```

<210> 75

<211> 787

<212> PRT

<213> Neisseria meningitidis

<400> 75

```
Met Gln Phe Ser Tyr Ser Trp Leu Lys Thr Gln Ala Asp Thr Glu Leu
1               5                   10                  15

Ser Ser Asp Lys Leu Glu His Leu Leu Thr Met Ser Gly Leu Glu Val
            20                  25                  30

Glu Glu Ala Glu Thr Ala Ala Pro Ala Phe Ala Gly Val Val Ile Ala
        35                  40                  45

Glu Val Lys Ser Val Glu Lys His Pro Asp Ala Asp Arg Leu Asn Val
        50                  55                  60

Thr Arg Val Asp Ala Gly Thr Gly Gly Leu Val Gln Ile Val Cys Gly
65                  70                  75                  80

Ala Pro Asn Val Lys Ala Gly Ile Lys Val Pro Cys Ser Leu Pro Gly
                85                  90                  95

Ala Val Leu Pro Gly Asn Phe Lys Ile Lys Pro Thr Lys Met Arg Gly
            100                 105                 110

Glu Val Ser Asp Gly Met Leu Cys Ser Thr Asp Glu Leu Gly Leu Pro
            115                 120                 125

Asp Asp Gly Val Asn Gly Leu His Ile Leu Pro Glu Asp Ala Pro Val
    130                 135                 140

Gly Thr Asn Ile Arg Glu Tyr Leu Asp Leu Asp Asp Thr Leu Phe Thr
145                 150                 155                 160

Leu Lys Ile Thr Pro Asn Arg Ala Asp Cys Leu Ser Ile Lys Gly Ile
                165                 170                 175

Ala Arg Glu Val Ser Ala Leu Thr Gly Cys Ala Phe Arg Gln Pro Glu
            180                 185                 190

Ile His Thr Ala Pro Ile Thr Gly Ser Arg Lys Gln Pro Val Gln Ile
        195                 200                 205

Asn Ala Pro Ala Asp Cys Gly Arg Phe Ile Ser Arg Val Ile Glu Asn
    210                 215                 220

Val Asn Ala Arg Ala Thr Thr Pro Asp Trp Met Lys Gln Arg Leu Glu
225                 230                 235                 240

Arg Ser Gly Ile Arg Ser Ile Ser Ala Leu Val Asp Ile Gly Asn Tyr
                245                 250                 255

Val Met Leu Glu Ile Gly Gln Pro Met His Val Phe Asp Ala Asp Lys
            260                 265                 270

Leu Ser Gly Ser Leu His Ile Arg Arg Ala Arg Glu Gly Glu Thr Leu
            275                 280                 285

Glu Cys Leu Asn Glu Lys Thr Val Ser Leu Ser Glu Asn Thr Leu Val
    290                 295                 300

Val Ala Asp Glu Lys Gly Val Leu Ser Leu Ala Gly Leu Met Gly Gly
305                 310                 315                 320

Ala Ala Ser Ala Val Ser Asp Gly Thr Gln Asn Ile Val Leu Glu Ala
            325                 330                 335

Ala Trp Phe Ala Pro Glu Ile Ile Ala Gly Lys Ser Arg Gln Tyr Gly
            340                 345                 350

Phe Gly Ser Asp Ser Ser Phe Arg Phe Glu Arg Gly Val Asp Tyr Arg
```

|  |  | 355 |  |  |  |  | 360 |  |  |  |  | 365 |  |  |
|---|---|---|---|---|---|---|---|---|---|---|---|---|---|---|

Leu Gln Ala Asp Ala Ile Glu Arg Ala Thr Glu Leu Val Leu Gln Ile
     370                375                380

Cys Gly Gly Ala Ala Gly Glu Met Val Glu Ala Gln Gly Glu Leu Pro
385                390                395                400

Glu Ala Lys Gln Val Gly Leu Arg Leu Asp Arg Leu Lys Thr Val Leu
               405                410                415

Gly Val Asp Ile Pro Ala Glu Gln Val Glu Thr Ile Leu Gln His Leu
          420                425                430

Gly Leu Gln Pro Glu Lys Thr Ala Glu Gly Phe Arg Val Thr Ala Pro
          435                440                445

Ser Phe Arg Phe Asp Ile Glu Ile Glu Ala Asp Leu Ile Glu Glu Ile
     450                455                460

Gly Arg Val Tyr Gly Tyr Glu Asn Ile Pro Asp Asp Tyr Thr Ser Gly
465                470                475                480

Arg Leu Lys Met Leu Glu Leu Pro Glu Thr Arg Arg Pro Arg Phe Ala
               485                490                495

Val Tyr Asn Glu Met Ala Ala Arg Gly Tyr Arg Glu Val Val Ser Tyr
          500                505                510

Ala Phe Val Asp Glu Gln Trp Glu Gln Asp Phe Ala Ala Asn Ala Asp
     515                520                525

Pro Ile Arg Leu Gln Asn Pro Leu Ala Ala Gln Tyr Ala Val Met Arg
     530                535                540

Ser Thr Leu Ile Gly Gly Leu Val Glu Ile Leu Gln Asn Asn Leu Asn
545                550                555                560

Arg Lys Gln Asn Arg Val Cys Val Phe Glu Ile Ala Arg Val Phe Ser
               565                570                575

Lys Gly Ser Asp Gly Gln Phe Val Gln Asn Glu Arg Ile Gly Gly Leu
          580                585                590

Trp Tyr Gly Ala Val Met Pro Glu Gln Trp Gly Gly Lys Thr Arg Asn
     595                600                605

Ala Asp Phe Tyr Asp Ile Lys Ala Asp Val Glu Asn Leu Leu Lys Asn
     610                615                620

Lys Ala Val Glu Phe Val Lys Thr Gly His Pro Ala Leu His Pro Gly
625                630                635                640

Arg Ala Ala Asn Ile Val Ser Asp Gly Lys Val Ile Gly Phe Val Gly
               645                650                655

Glu Leu His Pro Lys Trp Leu Gln Lys Tyr Asp Leu Pro Gln Ala Pro
          660                665                670

Leu Val Phe Glu Ile Asp Met Ala Ala Val Leu Glu Cys Gly Lys Thr
          675                680                685

Arg Tyr Arg Val Val Ser Lys Phe Gln Pro Val Arg Arg Asp Leu Ala
     690                695                700

Phe Val Met Pro Glu Ala Met Ser His Asp Asp Leu Leu Leu Val Leu
705                710                715                720

Lys Gly Ala Ala Asn Lys Leu Val Gln Glu Ile Ser Val Phe Asp Val
               725                730                735

Tyr Arg Gly Thr Gly Leu Pro Glu Gly Met Lys Ser Val Ala Val Lys
              740                     745                 750

Val Ile Leu Gln Asp Met Glu Asn Thr Leu Thr Asp Glu Ala Val Glu
          755                 760                 765

Pro Leu Ile Gly Lys Leu Ile Gly Ala Ala Thr Ala Ala Gly Ala Arg
    770                 775                 780

Leu Arg Ser
785

<210> 76
<211> 245
<212> PRT
<213> Neisseria meningitidis

<400> 76

Met Gly Gly Gln Lys Thr His Phe Gly Phe Ser Thr Val Asn Glu Asp
1               5                   10                  15

Glu Lys Ala Gly Lys Val Ala Glu Val Phe His Ser Val Ala Lys Asn
          20                  25                  30

Tyr Asp Ile Met Asn Asp Val Met Ser Ala Gly Leu His Arg Val Trp
          35                  40                  45

Lys His Phe Thr Ile Asn Thr Ala His Leu Lys Lys Gly Asp Lys Val
    50                  55                  60

Leu Asp Ile Ala Gly Gly Thr Gly Asp Leu Ser Arg Gly Trp Ala Lys
65                  70                  75                  80

Arg Val Gly Lys Glu Gly Glu Val Trp Leu Thr Asp Ile Asn Ser Ser
              85                  90                  95

Met Leu Thr Val Gly Arg Asp Arg Leu Leu Asn Glu Gly Met Ile Leu
          100                 105                 110

Pro Val Ser Leu Ala Asp Ala Glu Lys Leu Pro Phe Pro Asp Asn Tyr
          115                 120                 125

Phe Asn Leu Val Ser Val Ala Phe Gly Leu Arg Asn Met Thr His Lys
    130                 135                 140

Asp Ala Ala Leu Lys Glu Met Tyr Arg Val Leu Lys Pro Gly Gly Thr
145                 150                 155                 160

Leu Leu Val Leu Glu Phe Ser Lys Ile Tyr Lys Pro Leu Glu Gly Ala
              165                 170                 175

Tyr Asp Phe Tyr Ser Phe Lys Leu Leu Pro Val Met Gly Arg Leu Ile
          180                 185                 190

Ala Lys Asp Ala Glu Ser Tyr Gln Tyr Leu Ala Glu Ser Ile Arg Met
          195                 200                 205

His Pro Asp Gln Glu Thr Leu Lys Gln Met Met Leu Asp Ala Gly Phe
    210                 215                 220

Asp Ser Val Asp Tyr His Asn Met Ser Ala Gly Ile Val Ala Leu His
225                 230                 235                 240

Lys Gly Val Lys Phe
              245

168

EP 2 279 746 B1

<210> 77
<211> 287
<212> PRT
<213> Neisseria meningitidis

<400> 77

```
Met Ser Glu Ile Gly Leu Val Lys Ile Tyr Phe Gly Lys Val Arg Asp
1               5                   10                  15

Leu Tyr Glu Ile Asp Asp Lys Arg Met Leu Met Val Ala Ser Asp Arg
            20                  25                  30

Leu Ser Ala Phe Asp Val Ile Leu Asp Asp Pro Ile Pro Ser Lys Gly
            35                  40                  45

Glu Ile Leu Thr Gln Ile Ser Asn Phe Trp Phe Lys Lys Leu Ala His
    50                  55                  60

Ile Met Pro Asn His Phe Thr Gly Gln Thr Val Tyr Asp Val Leu Pro
65                  70                  75                  80

Glu Asn Glu Ala Lys Ala Leu Glu Lys Arg Ala Val Val Ala Lys Lys
                85                  90                  95

Leu Thr Pro Val Lys Val Glu Ala Ile Val Arg Gly Tyr Leu Ala Gly
            100                 105                 110

Ser Gly Trp Lys Asp Tyr Gln Lys Thr Gly Ser Val Cys Gly Ile Gln
        115                 120                 125

Leu Pro Glu Gly Met Gln Glu Ala Gln Gln Leu Pro Glu Val Ile Phe
        130                 135                 140

Thr Pro Ser Thr Lys Ala Ala Val Gly Asp His Asp Glu Asn Ile Ser
145                 150                 155                 160

Phe Glu Glu Cys Gly Arg Ile Ile Gly Lys Glu Leu Ala Glu Glu Val
                165                 170                 175

Arg Ala Lys Ala Val Arg Leu Tyr Thr Glu Ala Ala Glu Tyr Ala Lys
            180                 185                 190

Ser Arg Gly Ile Ile Ile Cys Asp Thr Lys Phe Glu Phe Gly Leu Asp
        195                 200                 205

Glu Asn Gly Thr Leu Thr Leu Met Asp Glu Val Leu Thr Pro Asp Ser
    210                 215                 220

Ser Arg Phe Trp Pro Ala Asp Gln Tyr Lys Val Gly Thr Asn Pro Pro
225                 230                 235                 240

Ser Phe Asp Lys Gln Phe Val Arg Asp Trp Leu Glu Gln Ser Gly Trp
            245                 250                 255

Asn Lys Lys Ala Pro Ala Pro Lys Val Pro Ala Asp Val Ile Gln Lys
            260                 265                 270

Thr Val Glu Lys Tyr Arg Glu Ala Leu Thr Leu Leu Thr Gln Asp
        275                 280                 285
```

<210> 78
<211> 707
<212> PRT
<213> Neisseria meningitidis

169

<400> 78

```
Met Met Phe Asp Lys His Val Lys Thr Phe Gln Tyr Gly Asn Gln Thr
1               5               10              15

Val Thr Leu Glu Thr Gly Glu Ile Ala Arg Gln Ala Ala Ala Ala Val
            20              25              30

Lys Val Ser Met Gly Asp Thr Val Val Leu Val Ala Val Thr Thr Asn
```

```
                35                    40                    45
        Lys Glu Val Lys Glu Gly Gln Asp Phe Phe Pro Leu Thr Val Asp Tyr
            50              55              60
        Leu Glu Arg Thr Tyr Ala Ala Gly Lys Ile Pro Gly Gly Phe Phe Lys
        65              70              75              80
        Arg Glu Gly Lys Gln Ser Glu Lys Glu Ile Leu Thr Ser Arg Leu Ile
                    85              90                  95
        Asp Arg Pro Ile Arg Pro Leu Phe Pro Glu Gly Phe Tyr His Asp Ile
                    100             105             110
        Gln Ile Val Ala Met Val Val Ser Val Asp Pro Glu Ile Asp Ser Asp
                    115             120             125
        Ile Pro Ala Met Leu Gly Ala Ser Ala Ala Leu Val Leu Ser Gly Val
            130             135             140
        Pro Phe Ala Gly Pro Ile Gly Ala Ala Arg Val Gly Tyr Val Asn Gly
        145             150             155             160
        Val Tyr Val Leu Asn Pro Thr Lys Ala Glu Leu Ala Lys Ser Gln Leu
                    165             170             175
        Asp Leu Val Val Ala Gly Thr Ser Lys Ala Val Leu Met Val Glu Ser
                    180             185             190
        Glu Ala Lys Ile Leu Pro Glu Asp Val Met Leu Gly Ala Val Val Tyr
                    195             200             205
        Gly His Asp Gln Met Gln Val Ala Ile Asn Ala Ile Asn Glu Phe Ala
            210             215             220
        Asp Glu Val Asn Pro Glu Leu Trp Asp Trp Lys Ala Pro Glu Thr Asn
        225             230             235             240
        Glu Glu Leu Val Ala Lys Val Arg Gly Ile Ala Gly Glu Thr Ile Lys
                    245             250             255
        Glu Ala Phe Lys Ile Arg Gln Lys Gln Ala Arg Ser Ala Lys Leu Asp
                    260             265             270
        Glu Ala Trp Ser Ala Val Lys Glu Ala Leu Ile Thr Glu Glu Thr Asp
                    275             280             285
        Thr Leu Ala Ala Asn Glu Ile Lys Gly Ile Phe Lys His Leu Glu Ala
            290             295             300
        Asp Val Val Arg Ser Gln Ile Leu Asp Gly Gln Pro Arg Ile Asp Gly
        305             310             315             320
        Arg Asp Thr Arg Thr Val Arg Pro Leu Asn Ile Gln Thr Ser Val Leu
                    325             330             335
        Pro Arg Thr His Gly Ser Ala Leu Phe Thr Arg Gly Glu Thr Gln Ala
                    340             345             350
        Leu Ala Val Ala Thr Leu Gly Thr Ser Arg Asp Glu Gln Ile Ile Asp
                    355             360             365
        Ala Leu Ser Gly Glu Tyr Thr Asp Arg Phe Met Leu His Tyr Asn Phe
            370             375             380
        Pro Pro Tyr Ser Thr Gly Glu Val Gly Arg Met Gly Ala Pro Lys Arg
        385             390             395             400
        Arg Glu Ile Gly His Gly Arg Leu Ala Lys Arg Ala Leu Leu Ala Val
                    405             410             415
```

```
Leu Pro Lys Pro Glu Asp Phe Ser Tyr Thr Met Arg Val Val Ser Glu
            420             425             430

Ile Thr Glu Ser Asn Gly Ser Ser Ser Met Ala Ser Val Cys Gly Gly
            435             440             445

Cys Leu Ser Leu Leu Ser Ala Gly Val Pro Leu Lys Ala His Val Ala
        450             455             460

Gly Ile Ala Met Gly Leu Ile Leu Glu Gly Asn Lys Phe Ala Val Leu
465             470             475             480

Thr Asp Ile Leu Gly Asp Glu Asp His Leu Gly Asp Met Asp Phe Lys
            485             490             495

Val Ala Gly Thr Thr Glu Gly Val Thr Ala Leu Gln Met Asp Ile Lys
            500             505             510

Ile Gln Gly Ile Thr Lys Glu Ile Met Gln Ile Ala Leu Ala Gln Ala
            515             520             525

Lys Glu Ala Arg Leu His Ile Leu Asp Gln Met Lys Ala Ala Val Ala
    530             535             540

Gly Pro Gln Glu Leu Ser Ala His Ala Pro Arg Leu Phe Thr Met Lys
545             550             555             560

Ile Asn Gln Asp Lys Ile Arg Glu Val Ile Gly Lys Gly Gly Glu Thr
            565             570             575

Ile Arg Ser Ile Thr Ala Glu Thr Gly Thr Glu Ile Asn Ile Ala Glu
            580             585             590

Asp Gly Thr Ile Thr Ile Ala Ala Thr Thr Gln Glu Ala Gly Asp Ala
        595             600             605

Ala Lys Lys Arg Ile Glu Gln Ile Thr Ala Glu Val Glu Val Gly Lys
    610             615             620

Val Tyr Glu Gly Thr Val Val Lys Ile Leu Asp Asn Asn Val Gly Ala
625             630             635             640

Ile Val Ser Val Met Pro Gly Lys Asp Gly Leu Val His Ile Ser Gln
            645             650             655

Ile Ala His Glu Arg Val Arg Asn Val Gly Asp Tyr Leu Gln Val Gly
            660             665             670

Gln Val Val Asn Val Lys Ala Leu Glu Val Asp Asp Arg Gly Arg Val
        675             680             685

Arg Leu Ser Ile Lys Ala Leu Leu Asp Ala Pro Ala Arg Glu Glu Asn
    690             695             700

Ala Ala Glu
705
```

<210> 79
<211> 310
<212> PRT
<213> Neisseria meningitidis

<400> 79

```
Met Lys Ile Ala Asn Ser Ile Thr Glu Leu Ile Gly Asn Thr Pro Leu
1               5                   10              15

Val Lys Leu Asn Arg Leu Thr Glu Gly Leu Lys Ala Glu Val Ala Val
            20              25              30

Lys Leu Glu Phe Phe Asn Pro Gly Ser Ser Val Lys Asp Arg Ile Ala
            35              40              45

Glu Ala Met Ile Glu Gly Ala Glu Lys Ala Gly Lys Ile Asn Lys Asn
        50              55              60

Thr Val Ile Val Glu Ala Thr Ser Gly Asn Thr Gly Val Gly Leu Ala
65              70              75              80

Met Val Cys Ala Ala Arg Gly Tyr Lys Leu Ala Ile Thr Met Pro Glu
                85              90              95

Ser Met Ser Lys Glu Arg Lys Met Leu Leu Arg Ala Phe Gly Ala Glu
            100             105             110

Leu Ile Leu Thr Pro Ala Ala Glu Gly Met Ala Gly Ala Ile Ala Lys
            115             120             125

Ala Lys Ser Leu Val Asp Ala His Pro Asp Thr Tyr Phe Met Pro Arg
    130             135             140

Gln Phe Asp Asn Glu Ala Asn Pro Glu Val His Arg Lys Thr Thr Ala
145             150             155             160

Glu Glu Ile Trp Arg Asp Thr Asp Gly Lys Val Asp Val Phe Val Ala
                165             170             175

Gly Val Gly Thr Gly Gly Thr Ile Thr Gly Val Gly Glu Val Leu Lys
            180             185             190

Lys Tyr Lys Pro Glu Val Lys Val Val Ala Val Glu Pro Glu Ala Ser
        195             200             205

Pro Val Leu Ser Gly Gly Glu Lys Gly Pro His Pro Ile Gln Gly Ile
    210             215             220

Gly Ala Gly Phe Ile Pro Thr Val Leu Asn Thr Lys Ile Tyr Asp Ser
225             230             235             240

Ile Thr Lys Val Ser Asn Glu Ala Ala Phe Glu Thr Ala Arg Ala Ile
                245             250             255

Ala Glu Lys Glu Gly Ile Leu Val Gly Ile Ser Ser Gly Ala Ala Val
            260             265             270

Trp Ser Ala Leu Gln Leu Ala Lys Gln Pro Glu Asn Glu Gly Lys Leu
        275             280             285

Ile Val Val Leu Leu Pro Ser Tyr Gly Glu Arg Tyr Leu Ser Thr Pro
    290             295             300

Leu Phe Ala Asp Leu Ala
305             310
```

<210> 80
<211> 424
<212> PRT
<213> Neisseria meningitidis

<400> 80

Met Pro Ser Glu Thr Ser Ser Pro Arg Lys Glu Asn Glu Thr Glu Val
1               5                   10                  15

His Ile Pro Ala Ala Pro Phe Ile Val Lys Gln Ser Gly Ser Asn Ala
            20                  25                  30

Leu Ala Val Cys Ala Leu Val Leu Ala Ala Leu Gly Leu Gly Thr Ser
        35                  40                  45

Gly Phe Leu Phe Val Gln Gly Gln Asn Val Leu Lys Asn Gln Glu Leu

```
              50                    55                    60
     Ala Phe Asn Gln Lys Ile Asp Lys Ala Ala Leu Gly Glu Ser Glu Asn
     65                  70                  75                  80

     Ala Ala Leu Leu Lys Asp Asn Leu Asn Arg Gln Ala Ala Ile Gln Ser
                     85                  90                  95

     Glu Leu Asp Arg Leu Asp Gly Asn Val Lys Ala Asn Gly Glu Gln Ile
                     100                 105                 110

     Leu Glu Met Gln Lys Ser Tyr Arg Glu Leu Thr Lys Gly Arg Ala Asp
                 115                 120                 125

     Trp Leu Val Asp Glu Thr Glu Thr Ile Leu Asn Leu Ala Ala Gln Gln
             130                 135                 140

     Leu Val Leu Thr Gly Asn Ile Gln Thr Ala Val Gly Val Leu Glu His
     145                 150                 155                 160

     Ile Asp Ser Arg Leu Ser Arg Phe Asn Gln Ala Glu Leu Leu Pro Ile
                     165                 170                 175

     Lys Gln Ala Val Ser Ser Asp Leu Ala Glu Leu Lys Asn Arg Pro Tyr
                 180                 185                 190

     Val Asp Ile Ser Gly Thr Ala Leu Arg Leu Asp Arg Leu Glu Thr Ala
                 195                 200                 205

     Val Ser Gly Leu Pro Leu Met Leu Asp Gly Val Leu Lys Pro Gly Val
     210                 215                 220

     Gln Val Lys Asn Glu Ala Ala Ser Ala Ser Trp Trp Gln Asn Val Trp
     225                 230                 235                 240

     Glu Lys Ser Leu Gly Thr Leu Lys Gly Leu Val Glu Ile Arg Arg Leu
                 245                 250                 255

     Glu Asn Asn Asp Ala Met Leu Ile Ser Pro Glu Gln Ala Tyr Phe Val
                 260                 265                 270

     Arg Glu Asn Leu Arg Leu Arg Leu Leu Asp Ala Arg Thr Ala Leu Met
                 275                 280                 285

     Gln Arg Asn Ser Glu Val Tyr Gln Gly Asp Leu Asn Asn Ala Glu Ala
         290                 295                 300

     Ala Val Arg Gln Tyr Phe Asp Ala Lys Ser Pro Ala Thr Gln Ser Trp
     305                 310                 315                 320

     Leu Lys Glu Leu Ala Glu Leu Lys Ala Leu Asp Val Arg Met Thr Ala
                 325                 330                 335

     Asp Asp Gly Leu Lys Asn Ser Leu Asn Ala Val Arg Ala Tyr Arg Asp
                 340                 345                 350

     Gly Thr Arg Met Thr Ala Ala Glu Asn Gln Glu Ala Glu Gln Ala Ala
             355                 360                 365

     Ser Glu Pro Ala Asn Glu Lys Thr Ala Ser Glu Pro Ala Ala Ala Ser
         370                 375                 380

     Asp Val Lys Thr Ile Glu Ala Pro Ser Leu Pro Ser Glu Arg Lys Pro
     385                 390                 395                 400

     Glu Gln Pro Ala Lys Lys Gln Thr Val Pro Glu Lys Ala Gly Arg Ser
                 405                 410                 415

     Pro Ser Ala Lys Gly Glu Arg Ala
                 420
```

175

<210> 81
<211> 169
<212> PRT
<213> Neisseria meningitidis

<400> 81

```
Met Ile Ile Leu His Thr Asn Lys Gly Asp Ile Lys Ile Glu Leu Asp
1               5                   10                  15

Phe Asp Lys Ala Pro Val Thr Ala Lys Asn Phe Glu Gln Tyr Val Lys
            20                  25                  30

Asp Gly Phe Tyr Asp Gly Val Ile Phe His Arg Val Ile Lys Gly Phe
        35                  40                  45

Met Ile Gln Gly Gly Gly Met Asp Glu Asn Met Asn Glu Lys Glu Thr
    50                  55                  60

Arg Asp Pro Ile Gln Asn Glu Ala Ser Asn Gly Leu Pro Asn Asp Lys
65                  70                  75                  80

Tyr Thr Ile Ala Met Ala Arg Thr Ser Asp Pro His Ser Ala Ser Ala
            85                  90                  95

Gln Phe Phe Ile Asn Thr Ala Asp Asn Ala Phe Leu Asn Phe Arg Ser
            100                 105                 110

Lys Glu Leu Tyr Gly Lys Thr Val Val Gln Asp Trp Gly Tyr Ala Val
        115                 120                 125

Phe Gly Lys Val Val Asp Gly Phe Asp Val Val Asp Ala Ile Glu Gly
    130                 135                 140

Val Ser Thr Lys Arg His Gly Tyr His Asp Asp Val Pro Ser Glu Pro
145                 150                 155                 160

Val Val Ile Ile Lys Ala Glu Ala Val
                165
```

<210> 82
<211> 221
<212> PRT
<213> Neisseria meningitidis

<400> 82

```
Met Thr Val Leu Ser Lys Glu Gln Val Leu Ser Ala Phe Lys Asn Arg
1               5               10              15

Lys Ser Cys Arg His Tyr Asp Ala Ala Arg Lys Ile Ser Ala Glu Asp
            20          25              30

Phe Gln Phe Ile Leu Glu Leu Gly Arg Leu Ser Pro Ser Ser Val Gly
        35              40              45

Ser Glu Pro Trp Gln Phe Ile Val Val Gln Asn Pro Glu Ile Arg Gln
    50              55              60

Ala Ile Lys Pro Phe Ser Trp Gly Met Ala Asp Ala Leu Asp Thr Ala
65              70              75              80

Ser His Leu Val Val Phe Leu Ala Lys Lys Asn Ala Arg Ser Asp Ser
            85              90              95

Pro Phe Met Leu Glu Ser Leu Lys Arg Arg Gly Val Thr Glu Pro Asp
        100             105             110

Ala Val Ala Lys Ser Leu Ala Arg Tyr Gln Ala Phe Gln Ala Asp Asp
        115             120             125


Ile Lys Ile Leu Asp Asp Ser Arg Ala Leu Phe Asp Trp Cys Cys Arg
    130             135             140

Gln Thr Tyr Ile Ala Leu Ala Asn Met Met Thr Gly Ala Ala Met Ala
145             150             155             160

Gly Ile Asp Ser Cys Pro Val Glu Gly Phe Asn Tyr Ala Glu Met Glu
            165             170             175

Arg Ile Leu Ser Gly Gln Phe Gly Leu Phe Asp Ala Ala Glu Trp Gly
        180             185             190

Val Ser Val Ala Ala Thr Phe Gly Tyr Arg Val Gln Glu Ile Ala Thr
        195             200             205

Lys Ala Arg Arg Pro Leu Glu Glu Thr Val Ile Trp Ala
210             215             220
```

<210> 83
<211> 383
<212> PRT
<213> Neisseria meningitidis

<400> 83

```
Met Gln Thr Trp Gln Leu Pro Glu His Ile Ala Asp Val Leu Pro Thr
1               5                   10                  15

Asn Ala Arg Gln Leu Glu Ser Ala Arg Glu Gln Leu Leu Ala Leu Phe
            20                  25                  30

Arg Val His Gly Tyr Glu Leu Val Gln Pro Pro Leu Met Glu Tyr Ala
        35                  40                  45

His Ser Leu Leu Thr His Ile Asp Ala Gly Leu Ser Leu Lys Thr Ile
    50                  55                  60

Leu Val Thr Asp Arg Leu Ser Gly Arg Gln Leu Gly Ile Arg Ala Asp
65                  70                  75                  80

Ile Thr Pro Gln Val Ala Arg Ile Asp Ala His Leu Leu Ser Ala Asn
            85                  90                  95

Gln Gly Ile Asn Arg Leu Cys Tyr Ala Gly Pro Val Leu His Ala Gln
            100                 105                 110

Pro Asp Gly Leu Leu Asn Met Arg Glu Pro Leu Gln Ala Gly Ala Glu
        115                 120                 125

Met Tyr Gly Phe Ala Asp Ile Arg Gly Asp Ile Glu Leu Ile Asp Leu
    130                 135                 140

Met Leu Lys Ser Met Lys Ile Ala Asp Met Gly Lys Val Leu Leu Ser
145                 150                 155                 160

Leu Gly His Ile Gly Ile Phe Arg Ala Leu Ser Asp Ala Ala His Leu
            165                 170                 175

Asp Ala Gly Gln Ser Ala Thr Leu Leu Ala Leu Met Gln Asp Lys Asp
        180                 185                 190

Thr Gly Ala Val Glu Ala Gln Val Lys Ala Trp Lys Leu Asp Gly Met
        195                 200                 205

Trp Ala Lys Ala Phe Ser Leu Leu Pro Arg Leu Tyr Gly Gly Arg Glu
    210                 215                 220

Val Leu Ser Asp Ala Arg Gly Arg Leu Pro Asp Leu Ser Ala Val Gly
225                 230                 235                 240

Gly Ala Leu Gly Glu Leu Gln Ala Val Cys Asp Ala Phe Pro Asp Cys
```

```
                          245                      250                      255
    Glu Ile His Ile Asp Leu Ser Glu Leu Arg Val Asp Asn Tyr His Thr
                260                     265                 270

    Gly Leu Leu Tyr Ala Ala Tyr Ala Ala Asp Phe His Asp Ala Val Ala
                275                     280                 285

    Arg Gly Gly Arg Tyr Asp Gly Leu Gly Gly Tyr Phe Gly Arg Ala Arg
            290                     295                 300

    Pro Ala Thr Gly Phe Ser Phe Asp Leu Arg Ser Phe Ile Gly Arg Leu
    305                     310                     315                 320

    Pro Ala Ile Glu Arg Gln Pro Ala Val Leu Val Asp Ala Glu Asp Ala
                    325                     330                     335

    Glu Ala Ala His Glu Ala Val Glu Ala Leu Arg Glu Gln Gly Gln Cys
                340                     345                 350

    Val Val Ile Asp Tyr Gly Ile Gly His Asn Val Ser Glu Glu Leu Ala
                355                     360                     365

    Gly Arg Leu Lys Lys Thr Asp Gly Val Trp Gln Val Val Lys Arg
    370                     375                     380
```

<210> 84
<211> 434
<212> PRT
<213> Neisseria meningitidis

<400> 84

```
Met Ala Met Ala Lys Asn Val Val Val Ile Gly Ala Gln Trp Gly Asp
1               5                   10                  15

Glu Gly Lys Gly Lys Ile Val Asp Trp Leu Ala Glu Glu Ala Gly Gly
            20                  25                  30

Val Val Arg Phe Gln Gly Gly His Asn Ala Gly His Thr Leu Val Val
        35                  40                  45

Gly Gly Lys Lys Thr Ile Leu Arg Leu Ile Pro Ser Gly Ile Leu His
        50                  55                  60

Glu Ser Leu Asp Cys Phe Ile Gly Ser Gly Val Val Val Ser Pro Glu
65                  70                  75                  80

Ala Leu Leu Gly Glu Ile Asp Glu Leu Asn Ala Ala Gly Val Lys Asn
                85                  90                  95

Val Glu Gly Arg Leu Lys Ile Ala Pro Thr Cys Pro Leu Ile Leu Pro
            100                 105                 110

Tyr His Ile Ala Leu Asp Gln Ala Arg Glu Ala Ser Arg Gly Lys Gly
        115                 120                 125

Lys Ile Gly Thr Thr Gly Arg Gly Ile Gly Pro Ala Tyr Glu Asp Lys
    130                 135                 140

Val Ala Arg Arg Ala Ile Arg Ala Ala Asp Leu Leu His Pro Glu Lys
145                 150                 155                 160

Leu Arg Glu Lys Leu Asp Ala Val Leu Ala Tyr Tyr Asn Val Gln Leu
            165                 170                 175

Gln His Leu His Asn Ala Glu Pro Val Lys Ala Glu Asp Val Met Ala
        180                 185                 190

Val Ile Glu Lys Val Ala Pro Arg Ile Ala Pro Met Ile Thr Asp Val
        195                 200                 205
```

```
Ser Arg Val Leu Asn Glu Lys Asn Lys Asn Gly Glu Lys Leu Leu Phe
    210             215             220

Glu Gly Ala Gln Gly Ala Leu Leu Asp Ile Asp Tyr Gly Thr Tyr Pro
225             230             235             240

Phe Val Thr Ser Ser Asn Cys Leu Ala Gly Ala Ala Ser Ala Gly Ala
            245             250             255

Gly Val Gly Pro Gln Met Leu Asp Tyr Val Leu Gly Ile Val Lys Ala
            260             265             270

Tyr Thr Thr Arg Val Gly Ser Gly Pro Phe Pro Thr Glu Leu Phe Asp
    275             280             285

Glu Val Gly Val Gly Leu Ala Glu Arg Gly His Glu Phe Gly Ser Val
    290             295             300

Thr Gly Arg Ala Arg Arg Cys Gly Trp Phe Asp Ala Ala Ala Leu Lys
305             310             315             320

Arg Ser Ile Gln Ile Asn Gly Ile Ser Gly Met Cys Ile Thr Lys Leu
            325             330             335

Asp Val Met Asp Gly Val Glu Thr Ile Asn Ile Cys Val Gly Tyr Glu
            340             345             350

Leu Pro Asp Gly Gly Lys Thr Asp Ile Leu Pro Cys Gly Ser Asp Ala
            355             360             365

Val Glu Thr Cys Lys Pro Ile Tyr Glu Thr Met Pro Gly Trp Arg Glu
    370             375             380

Ser Thr Phe Gly Val Lys Asp Tyr Gly Ala Leu Pro Glu Asn Ala Lys
385             390             395             400

Ala Tyr Leu Lys Arg Ile Glu Glu Val Cys Gly Ala Pro Val Ala Ile
            405             410             415

Val Ser Thr Gly Pro Asp Arg Glu Glu Thr Ile Val Leu His His Pro
            420             425             430

Phe Ala
```

<210> 85
<211> 334
<212> PRT
<213> Neisseria meningitidis

<400> 85

```
Met Thr Ile Ile Val Thr Gly Ala Ala Gly Phe Ile Gly Ser Asn Ile
1               5               10              15

Val Lys Ala Leu Asn Gln Arg Gly Ile Thr Asp Ile Val Ala Val Asp
            20              25              30

Asn Leu Ser Lys Gly Glu Lys Phe Lys Asn Leu Ala Glu Cys Glu Ile
        35              40              45

Ala His Tyr Leu Asp Lys His Glu Phe Ile Arg Gln Val Arg Glu His
    50              55              60

Ile Leu Pro Tyr Gln Asn Ile Glu Ala Val Phe His Gln Gly Ala Cys
65              70              75              80

Ser Asp Thr Met Asn His Asp Gly Leu Tyr Met Met Asp Asn Asn Tyr
            85              90              95


Gln Tyr Thr Leu Asp Leu Leu Asp Trp Cys Gln Asp Glu Arg Ile Pro
        100             105             110

Phe Leu Tyr Ala Ser Ser Ala Ala Val Tyr Gly Lys Gly Glu Ile Phe
        115             120             125

Arg Glu Glu Arg Glu Leu Glu Lys Pro Leu Asn Val Tyr Gly Tyr Ser
    130             135             140

Lys Phe Leu Phe Asp Gln Val Leu Arg Arg Arg Met Lys Glu Gly Leu
145             150             155             160

Thr Ala Gln Val Val Gly Phe Arg Tyr Phe Asn Val Tyr Gly Gln His
            165             170             175

Glu Gln His Lys Gly Arg Met Ala Ser Val Ala Phe His His Phe His
            180             185             190

Gln Tyr Arg Glu His Gly Tyr Val Asn Leu Phe Gly Ser Asn Asp Gly
        195             200             205

Tyr Gly Asn Gly Glu Gln Thr Arg Asp Phe Val Ser Val Glu Asp Val
    210             215             220

Ala Lys Val Asn Leu Tyr Phe Phe Asp His Pro Glu Leu Ser Gly Ile
225             230             235             240

Tyr Asn Leu Gly Thr Gly Arg Ser Gln Gln Phe Asn Glu Leu Ala Ala
            245             250             255

Ala Thr Val Asn Ala Cys Arg Ala Ala Glu Gly Lys Pro Glu Met Ser
            260             265             270

Leu Lys Glu Leu Val Glu Glu Leu Ile Arg Tyr Ile Pro Phe Pro
        275             280             285

Asp Ala Leu Lys Gly Lys Tyr Gln Ser Phe Thr Gln Ala Asp Ile Thr
    290             295             300

Lys Leu Arg Glu Ala Gly Tyr Lys Glu Glu Phe Phe Asp Val Lys Ser
305             310             315             320

Gly Val Asp Arg Tyr Val Lys Trp Met Leu Glu Asn Leu Ala
            325             330
```

<210> 86
<211> 431
<212> PRT

<213> Neisseria meningitidis

<400> 86

```
Met Ala Gln Lys Ile Gln Ser Val Lys Gly Met Asn Asp Leu Leu Pro
1               5                   10                  15

Val Lys Gln Lys Asp Phe Lys Leu Thr Ala Ala Phe Trp Gln Ala Phe
            20                  25                  30

Glu Asp Thr Val Gly Arg Trp Thr Arg Ala Tyr Gly Tyr Gln Gln Ile
            35                  40                  45

Arg Thr Pro Ile Val Glu Gln Thr Gly Leu Phe Val Arg Ser Ile Gly
        50                  55                  60

Glu Glu Thr Asp Val Val Gly Lys Glu Met Tyr Thr Phe Ser Asp Ser
65                  70                  75                  80

Asn Asp Ser Leu Ser Leu Ser Leu Arg Pro Glu Gly Thr Ala Ser Cys
                85                  90                  95

Leu Arg Ala Val Val Glu His Asn Leu Leu Tyr Asn Ser Pro Gln Lys
```

```
                       100                      105                      110
       Leu Trp Tyr Met Gly Pro Met Phe Arg Arg Glu Arg Pro Gln Lys Gly
               115                      120                      125

       Arg Tyr Arg Gln Phe His Gln Val Gly Ile Glu Ala Leu Gly Phe Glu
               130                      135                      140

       Gly Pro Asp Ile Asp Ala Glu Ile Ile Ala Met Ser Ala Asp Leu Trp
       145                      150                      155                      160

       Glu Lys Leu Gly Ile Arg Glu Tyr Leu Thr Leu Glu Ile Asn Ser Leu
                           165                      170                      175

       Gly Asn Arg Glu Glu Arg Ala Ala His Arg Ala Ala Leu Val Glu Tyr
                   180                      185                      190

       Leu Thr Arg Tyr Glu Asp Lys Leu Asp Glu Asp Ser Lys Arg Arg Leu
               195                      200                      205

       Lys Thr Asn Pro Leu Arg Val Leu Asp Thr Lys Asn Pro Asp Leu Gln
               210                      215                      220

       Glu Ile Cys Asn Ala Ala Pro Arg Leu Val Asp Tyr Leu Gly Glu Asp
       225                      230                      235                      240

       Ser Gln Asn His Tyr Val Arg Phe Lys Ala Met Leu Asp Gly Leu Gly
                           245                      250                      255

       Ile Gln Tyr Ile Glu Asn Pro Arg Leu Val Arg Gly Leu Asp Tyr Tyr
                   260                      265                      270

       Asn Gln Thr Val Phe Glu Trp Thr Thr Asp Lys Leu Gly Ala Gln Ala
                   275                      280                      285

       Thr Val Cys Gly Gly Gly Arg Tyr Asp Gly Leu Ile Glu Glu Leu Gly
           290                      295                      300

       Gly Lys Pro Ala Pro Ser Ile Gly Phe Ala Met Gly Ile Glu Arg Leu
       305                      310                      315                      320

       Leu Leu Leu Val Ser Glu Tyr Gly Ser Leu Glu Val Asn Ala Ala Pro
                       325                      330                      335

       Asp Val Tyr Ala Met His Gln Gly Glu Gly Ala Asp Leu Gln Val Met
                   340                      345                      350

       Lys Tyr Ala Gln Ala Leu Arg Ala Gln Gly Phe Asn Val Met Gln His
               355                      360                      365

       Ser Gly Tyr Gln Ser Leu Lys Ala Gln Met Lys Lys Ala Asp Asn Ser
       370                      375                      380

       Gly Ala Arg Phe Ala Leu Ile Val Ala Gln Asp Glu Leu Ala Asn Gly
       385                      390                      395                      400

       Thr Val Thr Leu Lys Asp Met Asn Gly Ala His Gly Gln Gln Thr Val
                       405                      410                      415

       Ala Ala Glu Asp Leu Thr Pro Thr Leu Gln Gln Trp Lys Asn Ala
                   420                      425                      430
```

<210> 87
<211> 327
<212> PRT
<213> Neisseria meningitidis

<400> 87

```
Met Ala Ala Tyr Asp Ser Leu Met Val Phe Thr Gly Asn Ala Asn Pro
1               5                   10                  15

Glu Leu Ala Gln Arg Val Val Arg His Leu Asp Ile Ser Leu Gly Asn
            20                  25                  30

Ala Ser Val Ser Lys Phe Ser Asp Gly Glu Val Ala Val Glu Leu Leu
        35                  40                  45

Glu Asn Val Arg Gly Arg Asp Val Phe Ile Leu Gln Pro Thr Cys Ala
        50                  55                  60

Pro Thr Asn Asp Asn Leu Met Glu Ile Leu Thr Met Ala Asp Ala Leu
65                  70                  75                  80

Lys Arg Ala Ser Ala Gly Arg Ile Thr Thr Ala Ile Pro Tyr Phe Gly
                85                  90                  95

Tyr Ala Arg Gln Asp Arg Arg Pro Arg Ser Val Arg Val Pro Ile Ser
            100                 105                 110

Ala Lys Leu Val Ala Asn Met Leu Tyr Ser Ala Gly Ile Asp Arg Val
        115                 120                 125

Leu Thr Val Asp Leu His Ala Asp Gln Ile Gln Gly Phe Phe Asp Ile
    130                 135                 140

Pro Val Asp Asn Ile Tyr Ala Thr Pro Ile Leu Leu Asn Asp Ile Lys
145                 150                 155                 160

Gln Gln Arg Ile Glu Asn Leu Thr Val Val Ser Pro Asp Ile Gly Gly
            165                 170                 175

Val Val Arg Ala Arg Ala Val Ala Lys Ser Leu Asn Ala Asp Leu Ala
        180                 185                 190

Ile Ile Asp Lys Arg Arg Pro Lys Ala Asn Val Ala Glu Val Met Asn
        195                 200                 205

Ile Ile Gly Asp Ile Gln Gly Arg Thr Cys Leu Ile Val Asp Asp Met
    210                 215                 220

Ile Asp Thr Ala Asn Thr Leu Cys Lys Ala Ala Val Ala Leu Lys Glu
225                 230                 235                 240

Arg Gly Ala Glu Arg Val Leu Ala Tyr Ala Ser His Ala Val Phe Ser
            245                 250                 255

Gly Glu Ala Val Ser Arg Ile Ala Ser Ser Glu Ile Asp Gln Val Val
            260                 265                 270

Val Thr Asp Thr Ile Pro Leu Ser Glu Ala Ala Lys Asn Cys Asp Arg
        275                 280                 285

Ile Arg Gln Val Thr Ile Ala Gly Leu Leu Ala Glu Thr Val Arg Arg
        290                 295                 300

Ile Ser Asn Glu Glu Ser Val Ser Tyr Leu Phe Asn Glu Glu Val Met
305                 310                 315                 320

Thr Gly Ser Met Leu Leu Pro
                325
```

<210> 88
<211> 190

<212> PRT
<213> Neisseria meningitidis

<400> 88

```
Met Thr Tyr Glu Ile Gln Ala Ser Val Arg Glu Ala Gln Gly Thr Gly
1               5               10              15

Ala Ser Arg Arg Leu Arg Arg Glu Gly Gln Ile Pro Gly Ile Leu Tyr
        20              25              30

Gly Glu Gly Gln Glu Pro Val Ala Ile Ala Val Asp His Lys Thr Val
        35              40              45

Phe Tyr Ala Leu Glu Lys Glu Ser Phe His Thr Ala Leu Ile Lys Leu
    50              55              60

Ser Leu Asn Gly Glu Thr Lys Asp Val Ile Val Arg Asp Phe Gln Met
65              70              75              80

His Pro Phe Arg Arg Glu Val Gln His Ile Asp Phe Gln Ala Val Lys
        85              90              95

Ala Asp Gln Leu Val Arg Ile Arg Val Pro Leu His Ile Val Asn Ala
        100             105             110

Glu Asn Ser Gln Ala Val Lys Leu Gln Gly Gly Arg Val Ser Leu Leu
        115             120             125

Asn Thr Ser Val Glu Val Val Ala Leu Pro Ala Asn Ile Pro Ala Phe
    130             135             140

Leu Asp Leu Asp Cys Ala Glu Val Val Ala Gly Asp Ile Leu His Leu
145             150             155             160

Ser Asp Ile Lys Leu Pro Glu Gly Val Glu Ser Val Ser Leu Lys Arg
        165             170             175

Asn Glu Asn Leu Ala Val Ala Thr Val Thr Gly Lys Lys Arg
    180             185             190
```

<210> 89
<211> 508
<212> PRT
<213> Neisseria meningitidis

<400> 89

```
Met Asn Thr Pro Leu Pro Tyr Ser Asp Tyr Leu Ile Arg Ile Leu Thr
1               5                   10              15

Ala Ser Val Tyr Asp Val Ala Val Glu Thr Pro Leu Glu Pro Ala Arg
            20                  25              30

Ser Leu Ser Val Arg Leu Lys Asn Asn Ile Leu Leu Lys Arg Glu Asp
        35                  40              45

Leu Gln Pro Val Phe Ser Phe Lys Ile Arg Gly Ala Tyr Asn Lys Met
    50              55                  60

Ser Lys Leu Pro Lys Asp Ala Leu Ala Cys Gly Val Ile Ala Ala Ser
65              70                  75                  80

Ala Gly Asn His Ala Gln Gly Val Ala Leu Ser Ala Gln Arg Leu Gly
            85                  90                  95

Cys Arg Ala Val Ile Val Met Pro Glu Thr Thr Pro Lys Ile Lys Val
            100             105             110

Asp Ala Val Lys Ser His Gly Gly Glu Val Val Leu Arg Gly Val Ser
        115             120             125

Tyr Asn Asp Ala Tyr Asp Tyr Ala Met Glu Leu Ala Glu Lys Glu Gly
    130             135             140

Leu Thr Tyr Ile Ala Pro Phe Asp Asp Pro Asp Val Ile Ala Gly Gln
145             150             155             160

Gly Thr Val Gly Met Glu Ile Val Ser Gln His Pro Asp Pro Ile Arg
```

```
                    165                    170                    175
          Ala Val Phe Val Pro Ile Gly Gly Gly Gly Leu Ala Ala Gly Val Ala
                      180                    185                    190

          Ala Phe Ile Lys Gln Val Arg Pro Glu Ile Lys Val Ile Gly Val Gln
                      195                    200                    205

          Thr Asn Asp Ser Cys Cys Met Lys Gln Ser Val Glu Ala Gly Glu Ile
                      210                    215                    220

          Val His Leu Lys Asp Val Gly Leu Phe Ser Asp Gly Thr Ala Val Lys
          225                    230                    235                    240

          Val Val Gly Asn Glu Thr Phe Arg Leu Cys Lys Glu Leu Leu Asp Glu
                          245                    250                    255

          Ile Ile Thr Val Asp Thr Asp Ala Val Cys Gly Ala Val Lys Asp Ile
                      260                    265                    270

          Phe Asp Asp Thr Arg Ser Ile Thr Glu Pro Ala Gly Ala Leu Ala Leu
                      275                    280                    285

          Ala Gly Leu Lys Ala Tyr Ile Ala Arg Glu Gly Ala Glu Asn Gln Thr
              290                    295                    300

          Leu Ile Ala Val Thr Ser Gly Ala Asn Met Asn Phe His Arg Leu Arg
          305                    310                    315                    320

          His Val Ser Glu Arg Ser Glu Leu Gly Glu Gly Asn Glu Gly Ile Phe
                          325                    330                    335

          Ala Val Thr Ile Pro Glu Glu Arg Gly Ser Phe Leu Lys Phe Val Asn
                      340                    345                    350

          Ile Leu Gly Asn Arg Asn Ile Thr Glu Phe Asn Tyr Arg Tyr Gly Asp
                      355                    360                    365

          Asp Glu Lys Ala His Ile Phe Val Gly Leu Gln Ala Ala Gly Pro Gln
              370                    375                    380

          Asp Leu Ala Val Ile Gly Ser Arg Leu Asp Glu Ala Gly Leu Pro Asn
          385                    390                    395                    400

          Val Asp Leu Thr Asn Asn Glu Ile Ala Lys Ile His Ile Arg Tyr Met
                      405                    410                    415

          Val Gly Gly Arg Thr Asp Lys Val Glu Asn Glu Arg Leu Val Ser Phe
                      420                    425                    430

          Glu Phe Pro Glu Arg Pro Gly Ala Leu Ala Arg Phe Leu Asn His Met
                      435                    440                    445

          Gln Gly Gly Trp Asn Ile Thr Leu Phe His Tyr Arg Asn His Gly Ala
              450                    455                    460

          Asp Tyr Gly Arg Ile Leu Val Gly Ile Asp Val Pro Pro His Asp Ala
          465                    470                    475                    480

          Ala Ala Phe Asp Gly Phe Leu Glu Ser Leu Gly Tyr Ser Tyr His Glu
                      485                    490                    495

          Glu Thr Gln Asn Ala Ala Tyr Lys Leu Phe Leu Ala
                      500                    505
```

<210> 90
<211> 195
<212> PRT

<213> Neisseria meningitidis

<400> 90

```
        Met Glu His Lys Leu Pro Gln Leu Pro Tyr Glu Leu Asp Ala Leu Ser
        1               5               10              15

        Pro His Leu Ser Lys Glu Thr Leu Glu Phe His Tyr Gly Lys His His
                    20              25              30

        Gln Thr Tyr Ile Thr Asn Leu Asn Asn Gln Ile Lys Gly Thr Glu Phe
                    35              40              45

        Glu Asn Leu Pro Leu Glu Glu Ile Val Lys Lys Ser Ser Gly Gly Val
                50              55              60

        Phe Asn Asn Ala Ala Gln Thr Trp Asn His Thr Phe Tyr Trp Leu Gly
        65              70              75              80

        Phe Thr Ser Lys Gly Gln Gly Lys Pro Ala Gly Glu Leu Ala Ala Ala
                    85              90              95

        Ile Asp Ala Lys Trp Gly Ser Phe Glu Lys Phe Gln Glu Ala Phe Asn
                    100             105             110

        Ala Cys Ala Ala Gly Thr Phe Gly Ser Gly Trp Ala Trp Leu Val Lys
                    115             120             125

        Thr Pro Ala Gly Gly Leu Asp Leu Val Ser Thr Ser Asn Ala Ala Thr
                130             135             140

        Pro Leu Thr Thr Glu Asn Thr Pro Leu Leu Thr Cys Asp Val Trp Glu
        145             150             155             160

        His Ala Tyr Tyr Ile Asp Tyr Arg Asn Ser Arg Pro Asn Tyr Leu Lys
                    165             170             175

        Gly Phe Trp Glu Ile Val Asn Trp Asp Glu Val Ala Lys Arg Phe Ala
                    180             185             190

        Ala Leu Ser
                195
```

<210> 91
<211> 468
<212> PRT
<213> Neisseria meningitidis

<400> 91

```
Met Asn Asp Tyr Ala Ala Met Pro Ser Glu Asp Arg Glu Val Gly Ala
1               5               10              15

Leu Ser Leu Pro Pro His Ser Met Glu Ala Glu Gln Ser Val Leu Gly
            20              25              30

Gly Leu Met Leu Glu Asn Pro Ala Trp Asp Arg Ile Ala Asp Val Val
        35              40              45

Ser Gly Glu Asp Phe Tyr Arg His Glu His Arg Leu Ile Phe Arg Ser
    50              55              60

Ile Ala Lys Leu Ile Asn Glu Ser Arg Pro Ala Asp Val Ile Thr Val
65              70              75              80

Gln Glu Asp Leu Gln Arg Asn Glu Glu Leu Glu Ala Ala Gly Gly Phe
            85              90              95

Glu Tyr Leu Ile Thr Leu Ala Gln Asn Thr Pro Ser Ala Ala Asn Ile
        100             105             110

Arg Arg Tyr Ala Glu Ile Val Arg Glu Arg Ser Ile Met Arg Gln Leu
        115             120             125
```

```
Ala Glu Val Gly Thr Glu Ile Ala Arg Ser Ala Tyr Asn Pro Gln Gly
    130             135             140

Arg Asp Ala Gly Gln Leu Leu Asp Glu Ala Glu Asn Lys Val Phe Gln
145             150             155             160

Ile Ala Glu Ser Thr Ala Lys Ser Lys Gln Gly Phe Leu Glu Met Pro
            165             170             175

Asp Leu Leu Lys Glu Val Val Gln Arg Ile Asp Met Leu Tyr Ser Arg
            180             185             190

Asp Asn Pro Asp Glu Val Thr Gly Val Pro Thr Gly Phe Ile Asp Leu
            195             200             205

Asp Lys Lys Thr Ser Gly Leu Gln Pro Gly Asp Leu Ile Ile Val Ala
    210             215             220

Gly Arg Pro Ser Met Gly Lys Thr Ala Phe Ser Ile Asn Ile Ala Glu
225             230             235             240

His Val Ala Val Glu Gly Arg Leu Pro Val Ala Val Phe Ser Met Glu
            245             250             255

Met Gly Gly Ala Gln Leu Val Met Arg Met Leu Gly Ser Val Gly Arg
        260             265             270

Leu Asp Gln Ser Val Leu Lys Thr Gly Arg Leu Glu Asp Glu His Trp
        275             280             285

Gly Arg Leu Asn Glu Ala Val Val Lys Leu Ser Asp Ala Pro Val Tyr
    290             295             300

Ile Asp Glu Thr Pro Gly Leu Thr Ala Leu Glu Leu Arg Ala Arg Ala
305             310             315             320

Arg Arg Leu Ala Arg Gln Phe Asn Asn Lys Leu Gly Leu Ile Val Ile
            325             330             335

Asp Tyr Leu Gln Leu Met Ala Gly Ser Gly Arg Ser Asp Asn Arg Ala
        340             345             350

Ser Glu Leu Gly Glu Ile Ser Arg Ser Leu Lys Ala Leu Ala Lys Glu
        355             360             365

Leu Gln Val Pro Ile Ile Ala Leu Ser Gln Leu Ser Arg Thr Val Glu
    370             375             380

Ser Arg Thr Asp Lys Arg Pro Met Met Ser Asp Leu Arg Glu Ser Gly
385             390             395             400

Ala Ile Glu Gln Asp Ala Asp Leu Ile Met Phe Met Tyr Arg Asp Glu
            405             410             415

Tyr Tyr Asn Gln Asp Ser Pro Met Lys Gly Leu Ala Glu Cys Ile Ile
            420             425             430

Gly Lys His Arg Asn Gly Pro Val Gly Lys Ile Phe Leu Thr Trp Thr
            435             440             445

Gly Gln Phe Thr Lys Phe Asp Asn Ala Ala Tyr Ile Pro Glu Glu Ala
    450             455             460

Lys Ile Glu Asp
465
```

<210> 92
<211> 395

191

<212> PRT
<213> Neisseria meningitidis

<400> 92

```
Met Asn Thr Leu Leu Asn Gln Leu Lys Pro Tyr Pro Phe Ala Arg Leu
1               5               10              15

Arg Glu Ala Met Gln Gly Ile Ser Ala Pro Glu Gly Leu Glu Ala Val
            20              25              30

Pro Leu His Ile Gly Glu Pro Lys His Pro Thr Pro Lys Val Ile Thr
        35              40              45

Asp Ala Leu Thr Ala Ser Leu His Glu Leu Glu Lys Tyr Pro Leu Thr
    50              55              60

Ala Gly Leu Pro Glu Leu Arg Gln Ala Cys Ala Asn Trp Leu Lys Arg
65              70              75              80

Arg Tyr Asp Gly Leu Thr Val Asp Ala Asp Asn Glu Ile Leu Pro Val
            85              90              95

Leu Gly Ser Arg Glu Ala Leu Phe Ser Phe Val Gln Thr Val Leu Asn
            100             105             110

Pro Val Ser Asp Gly Ile Lys Pro Ala Ile Val Ser Pro Asn Pro Phe
        115             120             125

Tyr Gln Ile Tyr Glu Gly Ala Thr Leu Leu Gly Gly Gly Glu Ile His
    130             135             140

Phe Ala Asn Cys Pro Ala Pro Ser Phe Asn Pro Asp Trp Arg Ser Ile
145             150             155             160

Ser Glu Glu Val Trp Lys Arg Thr Lys Leu Val Phe Val Cys Ser Pro
            165             170             175

Asn Asn Pro Ser Gly Ser Val Leu Asp Leu Asp Gly Trp Lys Glu Val
            180             185             190

Phe Asp Leu Gln Asp Lys Tyr Gly Phe Ile Ile Ala Ser Asp Glu Cys
        195             200             205

Tyr Ser Glu Ile Tyr Phe Asp Gly Asn Lys Pro Leu Gly Cys Leu Gln
    210             215             220

Ala Ala Ala Gln Leu Gly Arg Ser Arg Gln Lys Leu Leu Met Phe Thr
225             230             235             240

Ser Leu Ser Lys Arg Ser Asn Val Pro Gly Leu Arg Ser Gly Phe Val
            245             250             255

Ala Gly Asp Ala Glu Leu Leu Lys Asn Phe Leu Leu Tyr Arg Thr Tyr
        260             265             270

His Gly Ser Ala Met Ser Ile Pro Val Gln Arg Ala Ser Ile Ala Ala
    275             280             285

Trp Asp Asp Glu Gln His Val Ile Asp Asn Arg Arg Met Tyr Gln Glu
290             295             300

Lys Phe Glu Arg Val Ile Pro Ile Leu Gln Gln Val Phe Asp Val Lys
305             310             315             320

Leu Pro Asp Ala Ser Phe Tyr Ile Trp Leu Lys Val Pro Asp Gly Asp
            325             330             335

Asp Leu Ala Leu Ala Arg Asn Leu Trp Gln Lys Ala Ala Ile Gln Val
        340             345             350

Leu Pro Gly Arg Phe Leu Ala Arg Asp Thr Glu Gln Gly Asn Pro Gly
        355             360             365
```

```
Glu Gly Tyr Val Arg Ile Ala Leu Val Ala Asp Val Ala Thr Cys Val
    370             375             380

Lys Ala Ala Glu Thr Ile Val Ser Leu Tyr Arg
385             390             395
```

<210> 93
<211> 741
<212> PRT
<213> Neisseria meningitidis

<400> 93

```
Met Thr Gln Lys Ser Thr Ile Val Tyr Thr His Thr Asp Glu Ala Pro
1               5                   10              15

Ala Leu Ala Thr Gln Ser Leu Leu Pro Ile Val Gln Ala Phe Ala Arg
            20              25              30

His Ala Asp Ile Asp Val Lys Thr Ser Asp Ile Ser Leu Ser Gly Arg
        35              40              45

Ile Leu Ala Ala Phe Pro Glu Tyr Leu Thr Glu Ala Gln Arg Val Pro
    50              55              60

Asp Ala Leu Ala Glu Leu Gly Glu Leu Val Lys Gln Pro Asp Ala Asn
65              70              75              80

Val Ile Lys Leu Pro Asn Ile Ser Ala Ser Val Pro Gln Leu Thr Ala
                85              90              95

Ala Ile Lys Glu Leu Gln Ser Lys Gly Phe Ala Val Pro Asp Tyr Pro
            100             105             110

Ala Asp Pro Gln Thr Asp Glu Glu Lys Ala Val Arg Glu Arg Tyr Asp
        115             120             125

Arg Ile Lys Gly Ser Ala Val Asn Pro Val Leu Arg Glu Gly Asn Ser
    130             135             140

Asp Arg Arg Ala Pro Lys Ala Val Lys Asn Phe Ala Lys Lys Asn Pro
145             150             155             160

His Ser Met Gly Ala Trp Thr Lys Asp Ser Lys Thr His Val Ala Thr
            165             170             175

Met Gln Ser Gly Asp Phe Phe His Asn Glu Gln Ser Val Ile Val Pro
            180             185             190

Glu Ala Thr Ser Val Ser Ile Val Phe Thr Asp Lys Gln Gly Asn Lys
        195             200             205

Lys Glu Leu Arg Glu Pro Val Ala Leu Lys Ala Gly Glu Ile Ile Asp
    210             215             220

Ala Thr Val Met Ser Lys Lys Ala Leu Leu Ala Phe Leu Ala Glu Gln
225             230             235             240

Val Lys Asp Ala Lys Ala Lys Gly Val Leu Phe Ser Leu His Met Lys
            245             250             255

Ala Thr Met Met Lys Val Ser Asp Pro Ile Ile Phe Gly His Ala Val
            260             265             270

Lys Val Phe Phe Ala Pro Val Phe Glu Lys Phe Gly Asp Lys Leu Ala
        275             280             285

Ala Ala Gly Val Asn Val Asn Asn Gly Phe Gly Asn Leu Leu Ala Asn
    290             295             300
```

```
Leu Asp Lys Leu Asp Ala Asp Thr Arg Thr Ala Val Glu Ala Glu Ile
305             310             315                 320

Ala Ala Val Tyr Ala Ala Asn Pro Asp Leu Ala Met Val Asp Ser Asp
            325             330                 335

Lys Gly Ile Thr Asn Leu His Val Pro Ser Asp Val Ile Val Asp Ala
            340             345                 350

Ser Met Pro Ala Met Ile Arg Asn Ser Gly Arg Met Trp Asp Lys Asn
        355             360             365

Gly Lys Ala Gln Asp Thr Lys Ala Val Ile Pro Asp Ser Ser Tyr Ala
    370             375             380

Gly Val Tyr Gln Ala Thr Ile Asp Phe Cys Arg Glu His Gly Ala Phe
385             390             395                 400

Asp Pro Thr Thr Met Gly Thr Val Pro Asn Val Gly Leu Met Ala Gln
            405             410                 415

Ala Ala Glu Glu Tyr Gly Ser His Asn Lys Thr Phe Glu Ile Glu Ala
        420             425             430

Asp Gly Gln Val Gln Val Ile Asp Ala Ala Gly Lys Val Leu Met Gln
        435             440             445

His Asp Val Glu Ala Gly Asp Ile Trp Arg Met Cys Gln Thr Lys Asp
    450             455             460

Ala Pro Val Lys Asp Trp Val Gln Leu Ala Val Asn Arg Ala Arg Leu
465             470             475                 480

Ser Asn Thr Pro Ala Val Phe Trp Leu Asp Glu Asn Arg Pro His Asp
            485             490                 495

Lys Ser Leu Leu Ala Lys Val Lys Ala Tyr Leu Ala Glu Leu Asp Thr
        500             505             510

Asn Gly Leu Asp Ile Arg Val Leu Ala Pro Glu Glu Ala Ala Lys Phe
        515             520             525

Ser Leu Gly Arg Leu Lys Asn Gly Glu Asp Thr Ile Ser Val Thr Gly
    530             535             540

Asn Val Leu Arg Asp Tyr Leu Thr Asp Leu Phe Pro Ile Leu Glu Leu
545             550             555                 560

Gly Thr Ser Ala Lys Met Leu Ser Ile Val Pro Leu Met Asn Gly Gly
            565             570                 575

Gly Met Phe Glu Thr Gly Ala Gly Gly Ser Ala Pro Lys His Val Gln
        580             585             590

Gln Phe Leu Glu Glu Asn His Leu Arg Trp Asp Ser Leu Gly Glu Phe
    595             600             605

Leu Ala Leu Ala Val Ser Phe Glu His Leu Ala Gln Lys Thr Gly Asn
    610             615             620

Ala Lys Ala Gln Val Leu Ala Asp Thr Leu Asp Ala Ala Thr Glu Lys
625             630             635                 640

Leu Leu Leu Asn Asp Lys Ser Pro Lys Arg Lys Ala Gly Glu Leu Asp
            645             650                 655

Asn Arg Gly Ser His Phe Tyr Leu Thr Leu Tyr Trp Ala Gln Glu Leu
        660             665             670

Ala Ala Gln Asp Lys Asp Ala Glu Leu Lys Ala Ala Phe Thr Pro Leu
```

```
                675                     680                     685

         Ala Ala Ala Leu Thr Ala Asp Glu Ala Lys Ile Val Ala Glu Leu Ser
             690                 695                 700
         Ala Val Gln Gly Lys Ala Ala Asp Ile Gly Gly Tyr Tyr Ala Ala Asn
         705                 710                 715                 720
         Pro Glu Lys Ala Ala Gln Val Met Arg Pro Ser Val Thr Phe Asn Gln
                         725                 730                 735
         Ala Leu Asn Ala Leu
                     740
```

<210> 94
<211> 758
<212> PRT
<213> Neisseria meningitidis

<400> 94

```
Met Thr Thr Leu His Phe Ser Gly Phe Pro Arg Val Gly Ala Phe Arg
1               5               10              15

Glu Leu Lys Phe Ala Gln Glu Lys Tyr Trp Arg Lys Glu Ile Ser Glu
            20              25              30

Gln Glu Leu Leu Ala Val Ala Lys Asp Leu Arg Glu Lys Asn Trp Lys
        35              40              45

His Gln Val Ala Ala Asn Ala Asp Phe Val Ala Val Gly Asp Phe Thr
    50              55              60

Phe Tyr Asp His Ile Leu Asp Leu Gln Val Ala Thr Gly Ala Ile Pro
65              70              75              80

Ala Arg Phe Gly Phe Asp Ser Gln Asn Leu Ser Leu Glu Gln Phe Phe
            85              90              95

Gln Leu Ala Arg Gly Asn Lys Asp Gln Phe Ala Ile Glu Met Thr Lys
        100             105             110

Trp Phe Asp Thr Asn Tyr His Tyr Leu Val Pro Glu Phe His Ala Asp
        115             120             125

Thr Glu Phe Lys Ala Asn Ala Lys His Tyr Val Gln Gln Leu Gln Glu
    130             135             140

Ala Gln Ala Leu Gly Leu Lys Ala Lys Pro Thr Val Val Gly Pro Leu
145             150             155             160

Thr Phe Leu Trp Val Gly Lys Glu Lys Gly Ala Val Glu Phe Asp Arg
            165             170             175

Leu Ser Leu Leu Pro Lys Leu Leu Pro Val Tyr Val Glu Ile Leu Thr
        180             185             190

Ala Leu Val Glu Ala Gly Ala Glu Trp Ile Gln Ile Asp Glu Pro Ala
    195             200             205

Leu Ala Val Asp Leu Pro Lys Glu Trp Val Glu Ala Tyr Lys Asp Val
    210             215             220

Tyr Ala Thr Leu Ser Lys Val Ser Ala Lys Ile Leu Leu Ser Thr Tyr
225             230             235             240

Phe Gly Ser Val Ala Glu His Ala Ala Leu Leu Lys Ala Leu Pro Val
            245             250             255

Asp Gly Leu His Ile Asp Leu Val Arg Ala Pro Glu Gln Leu Asp Ala
            260             265             270
```

```
Phe Ala Asp Tyr Asp Lys Val Leu Ser Ala Gly Val Ile Asp Gly Arg
        275                 280                 285
Asn Ile Trp Arg Ala Asn Leu Asn Lys Val Leu Glu Thr Val Glu Pro
    290                 295                 300
Leu Gln Ala Lys Leu Gly Asp Arg Leu Trp Ile Ser Ser Ser Cys Ser
305             310                 315                 320
Leu Leu His Thr Pro Phe Asp Leu Ser Val Glu Glu Lys Leu Lys Ala
            325                 330                 335
Asn Lys Pro Asp Leu Tyr Ser Trp Leu Ala Phe Thr Leu Gln Lys Thr
            340                 345                 350
Gln Glu Leu Arg Val Leu Lys Ala Ala Leu Asn Glu Gly Arg Asp Ser
        355                 360                 365
Val Ala Glu Glu Leu Ala Ala Ser Gln Ala Ala Ala Asp Ser Arg Ala
    370                 375                 380
Asn Ser Ser Glu Ile His Arg Ala Asp Val Ala Lys Arg Leu Ala Asp
385                 390                 395                 400
Leu Pro Ala Asn Ala Asp Gln Arg Lys Ser Pro Phe Ala Asp Arg Ile
                405                 410                 415
Lys Ala Gln Gln Ala Trp Leu Asn Leu Pro Leu Leu Pro Thr Thr Asn
            420                 425                 430
Ile Gly Ser Phe Pro Gln Thr Thr Glu Ile Arg Gln Ala Arg Ser Ala
            435                 440                 445
Phe Lys Lys Gly Glu Leu Ser Ala Ala Asp Tyr Glu Ala Ala Met Lys
    450                 455                 460
Lys Glu Ile Ala Leu Val Val Glu Glu Gln Glu Lys Leu Asp Leu Asp
465                 470                 475                 480
Val Leu Val His Gly Glu Ala Glu Arg Asn Asp Met Val Glu Tyr Phe
                485                 490                 495
Gly Glu Leu Leu Ser Gly Phe Ala Phe Thr Gln Tyr Gly Trp Val Gln
            500                 505                 510
Ser Tyr Gly Ser Arg Cys Val Lys Pro Pro Ile Ile Phe Gly Asp Val
        515                 520                 525
Ser Arg Pro Glu Ala Met Thr Val Ala Trp Ser Thr Tyr Ala Gln Ser
530                 535                 540
Leu Thr Lys Arg Pro Met Lys Gly Met Leu Thr Gly Pro Val Thr Ile
545                 550                 555                 560
Leu Gln Trp Ser Phe Val Arg Asn Asp Ile Pro Arg Ser Thr Val Cys
            565                 570                 575
Lys Gln Ile Ala Leu Ala Leu Asn Asp Glu Val Leu Asp Leu Glu Lys
            580                 585                 590
Ala Gly Ile Lys Val Ile Gln Ile Asp Glu Pro Ala Ile Arg Glu Gly
        595                 600                 605
Leu Pro Leu Lys Arg Ala Asp Trp Asp Ala Tyr Leu Asn Trp Ala Gly
    610                 615                 620
Glu Ser Phe Arg Leu Ser Ser Ala Gly Cys Glu Asp Ser Thr Gln Ile
625                 630                 635                 640
```

```
His Thr His Met Cys Tyr Ser Glu Phe Asn Asp Ile Leu Pro Ala Ile
                645             650             655

Ala Ala Met Asp Ala Asp Val Ile Thr Ile Glu Thr Ser Arg Ser Asp
            660             665             670

Met Glu Leu Leu Thr Ala Phe Gly Glu Phe Gln Tyr Pro Asn Asp Ile
        675             680             685

Gly Pro Gly Val Tyr Asp Ile His Ser Pro Arg Val Pro Thr Glu Ala
        690             695             700

Glu Val Glu His Leu Leu Arg Lys Ala Ile Glu Val Val Pro Val Glu
705             710             715             720

Arg Leu Trp Val Asn Pro Asp Cys Gly Leu Lys Thr Arg Gly Trp Lys
                725             730             735

Glu Thr Leu Glu Gln Leu Gln Val Met Met Asn Val Thr Arg Lys Leu
            740             745             750

Arg Ala Glu Leu Ala Lys
            755
```

<210> 95
<211> 245
<212> PRT
<213> Neisseria meningitidis

<400> 95

```
Met Ala Leu Gln Asp Arg Thr Gly Gln Lys Val Pro Ser Val Val Phe
1               5                   10                  15

Arg Thr Arg Val Gly Asp Thr Trp Lys Asp Val Ser Thr Asp Asp Leu
            20                  25                  30

Phe Lys Gly Lys Lys Val Val Val Phe Ser Leu Pro Gly Ala Phe Thr
        35                  40                  45

Pro Thr Cys Ser Ser Ser His Leu Pro Arg Tyr Asn Glu Leu Phe Gly
        50                  55                  60

Ala Phe Lys Glu Asn Gly Val Asp Ala Ile Tyr Cys Val Ser Val Asn
65                  70                  75                  80

Asp Thr Phe Val Met Asn Ala Trp Ala Ala Glu Glu Glu Ser Asp Asn
                85                  90                  95

Ile Tyr Met Ile Pro Asp Gly Asn Gly Glu Phe Thr Glu Gly Met Gly
            100                 105                 110

Met Leu Val Gly Lys Glu Asp Leu Gly Phe Gly Lys Arg Ser Trp Arg
            115                 120                 125

Tyr Ser Met Leu Val Asn Asp Gly Val Val Glu Lys Met Phe Ile Glu
    130                 135                 140

Pro Glu Glu Pro Gly Asp Pro Phe Lys Val Ser Asp Ala Asp Thr Met
145                 150                 155                 160

Leu Gln Phe Val Ala Pro Asp Trp Lys Ala Gln Glu Ser Val Ala Ile
                165                 170                 175

Phe Thr Lys Pro Gly Cys Gln Phe Cys Ala Lys Ala Lys Gln Ala Leu
            180                 185                 190

Gln Asp Lys Gly Leu Ser Tyr Glu Glu Ile Val Leu Gly Lys Asp Ala
        195                 200                 205

Thr Val Thr Ser Val Arg Ala Ile Thr Gly Lys Met Thr Ala Pro Gln

        210                 215                 220

Val Phe Ile Gly Gly Lys Tyr Ile Gly Gly Ser Glu Asp Leu Glu Ala
225                 230                 235                 240

Tyr Leu Ala Lys Asn
                245
```

<210> 96
<211> 467
<212> PRT
<213> Neisseria meningitidis

<400> 96

EP 2 279 746 B1

```
Met Lys Lys Ile Gln Ala Asp Val Val Val Ile Gly Gly Gly Thr Ala
1               5                   10                  15

Gly Met Gly Ala Phe Arg Asn Ala Arg Leu His Ser Asp Asn Val Tyr
            20                  25                  30

Leu Ile Glu Asn Asn Val Phe Gly Thr Thr Cys Ala Arg Val Gly Cys
        35                  40                  45

Met Pro Ser Lys Leu Leu Ile Ala Ala Ala Glu Ala Arg His His Ala
    50                  55                  60

Leu His Thr Asp Pro Phe Gly Val His Leu Asp Lys Asp Ser Ile Val
65                  70                  75                  80

Val Asn Gly Glu Glu Val Met Gln Arg Val Lys Ser Glu Arg Asp Arg
                85                  90                  95

Phe Val Gly Phe Val Val Ala Asp Val Glu Glu Trp Pro Ala Asp Lys
            100                 105                 110

Arg Ile Met Gly Ser Ala Lys Phe Ile Asp Glu His Thr Val Gln Ile
            115                 120                 125

Asp Glu His Thr Gln Ile Thr Ala Lys Ser Phe Val Ile Ala Thr Gly
    130                 135                 140

Ser Arg Pro Val Ile Leu Pro Gln Trp Gln Ser Leu Gly Asn Arg Leu
145                 150                 155                 160

Ile Ile Asn Asp Asp Val Phe Ser Trp Asp Thr Leu Pro Lys Arg Val
                165                 170                 175

Ala Val Phe Gly Pro Gly Val Ile Gly Leu Glu Leu Gly Gln Ala Leu
            180                 185                 190

His Arg Leu Gly Val Lys Val Glu Ile Phe Gly Leu Gly Gly Ile Ile
            195                 200                 205

Gly Gly Ile Ser Asp Pro Val Val Ser Asp Glu Ala Asn Ala Val Phe
    210                 215                 220

Gly Glu Glu Leu Lys Leu His Leu Asp Ala Lys Thr Glu Val Lys Leu
225                 230                 235                 240

Asp Ala Asp Gly Asn Val Glu Val His Trp Glu Gln Asp Gly Glu Lys
                245                 250                 255

Gly Val Phe Val Ala Glu Tyr Met Leu Ala Ala Val Gly Arg Arg Pro
            260                 265                 270

Asn Val Asp Asn Ile Gly Leu Glu Asn Ile Asn Ile Glu Lys Asp Ala
            275                 280                 285

Arg Gly Val Pro Val Ala Asp Pro Leu Thr Met Gln Thr Ser Ile Pro
290                 295                 300
```

202

His Ile Phe Ile Ala Gly Asp Ala Ser Asn Gln Leu Pro Leu Leu His
305                     310                 315                 320

Glu Ala Ala Asp Gln Gly Lys Ile Ala Gly Asp Asn Ala Gly Arg Tyr
                325             330                 335

Pro Asn Ile Gly Gly Gly Leu Arg Arg Ser Thr Ile Gly Val Val Phe
            340             345                 350

Thr Ser Pro Gln Ile Gly Phe Val Gly Leu Lys Tyr Ala Gln Val Ala
            355             360                 365

Ala Gln Tyr Gln Ala Asp Glu Phe Val Ile Gly Glu Val Ser Phe Lys
    370             375                 380

Asn Gln Gly Arg Ser Arg Val Met Leu Val Asn Lys Gly His Met Arg
385                 390                 395                 400

Leu Tyr Ala Glu Lys Ala Thr Gly Arg Phe Ile Gly Ala Glu Ile Val
            405             410                 415

Gly Pro Ala Ala Glu His Leu Ala His Leu Leu Ala Trp Ala His Gln
            420             425                 430

Met Lys Met Thr Val Pro Gln Met Leu Asp Met Pro Phe Tyr His Pro
            435             440                 445

Val Ile Glu Glu Gly Leu Arg Thr Ala Leu Arg Asp Ala Asp Ala Lys
    450             455                 460

Leu Lys Ala
465

<210> 97
<211> 942
<212> PRT
<213> Neisseria meningitidis

<400> 97

Met Met Asp Glu Lys Leu Asn Phe Ser Tyr Leu Phe Gly Ser Asn Ala
1               5                   10                  15

Pro Tyr Ile Glu Glu Leu Tyr Glu Ala Phe Leu Glu Asn Pro Asp Ala
            20              25                  30

Val Asp Glu Lys Trp Lys Gln Tyr Phe Thr Asp Leu Ser Lys Gln Pro
        35                  40                  45

Gly Thr Val Ala Val Asp Val Ala His Thr Pro Ile Arg Glu Ser Phe
    50              55                  60

Val Thr Leu Ala Lys Lys Lys Ile Ala Ser Ala Val Ala Gly Gly Ala
65                  70                  75                  80

Asp Glu Ala Met Leu Lys Lys Gln Val Ser Val Leu Arg Leu Ile Ser
                85                  90                  95

Ala Tyr Arg Ile Gln Gly Val Gly Ala Ala Gln Leu Asp Pro Leu Lys
            100                 105                 110

Arg Ile Pro Pro Arg Asp Ile Glu Ala Leu Asp Pro Lys Phe His Gly
        115                 120                 125

Leu Ser Asp Ala Asp Met Ala Leu Gln Phe Asn Met Gly Glu Gly Asp
    130                 135                 140

Phe Ala Asn Arg Gly Lys Leu Pro Leu Ser Gln Ile Ile Ser Asn Leu
145                 150                 155                 160

```
Lys Gln Thr Tyr Cys Gly His Ile Ala Leu Glu Tyr Ile Tyr Ile Pro
              165                 170                 175

Asn Thr Glu Glu Arg Arg Trp Val Arg Asn Tyr Phe Glu Ser Val Leu
              180                 185                 190

Ser Thr Pro His Tyr Asn Ala Asp Gln Lys Arg Arg Ile Leu Lys Glu
              195                 200                 205

Met Thr Ala Ala Glu Thr Leu Glu Arg Tyr Leu His Thr Lys Tyr Val
    210                 215                 220

Gly Gln Lys Arg Phe Gly Val Glu Gly Gly Glu Ser Ala Ile Ala Gly
225                 230                 235                 240

Leu Asn Tyr Leu Ile Gln Asn Ala Gly Lys Asp Gly Val Glu Glu Val
              245                 250                 255

Ile Ile Gly Met Ala His Arg Gly Arg Leu Asn Val Leu Val Asn Ile
              260                 265                 270

Leu Gly Lys Lys Pro Gly Asp Leu Phe Ala Glu Phe Glu Gly Arg Ala
              275                 280                 285

Glu Ile Lys Leu Pro Ser Gly Asp Val Lys Tyr His Met Gly Phe Ser
    290                 295                 300

Ser Asp Ile Ala Thr Pro His Gly Pro Met His Val Ser Leu Ala Phe
305                 310                 315                 320

Asn Pro Ser His Leu Glu Ile Val Asn Pro Val Val Glu Gly Ser Ala
              325                 330                 335

Arg Ala Lys Gln Lys Arg Leu Gly Glu Asn Gly Arg Asp Lys Val Leu
              340                 345                 350

Pro Val Leu Ile His Gly Asp Ser Ala Phe Ile Gly Leu Gly Val Asn
              355                 360                 365

Gln Ala Thr Phe Asn Leu Ser Lys Thr Arg Gly Tyr Thr Thr Gly Gly
    370                 375                 380

Thr Val His Ile Val Ile Asn Asn Gln Ile Gly Phe Thr Thr Ser Asp
385                 390                 395                 400

Ile Arg Asp Thr Arg Ser Thr Val His Cys Thr Asp Ile Ala Lys Met
              405                 410                 415

Val Ser Ala Pro Val Ile His Val Asn Gly Asp Asp Pro Glu Arg Val
              420                 425                 430

Cys Phe Ala Ile Gln Ala Ala Leu Asp Tyr Arg Lys Lys Phe His Lys
              435                 440                 445

Asp Ile Val Ile Asp Val Val Cys Tyr Arg Lys Trp Gly His Asn Glu
    450                 455                 460

Gly Asp Asp Pro Thr Leu Thr Gln Pro Met Met Tyr Lys Lys Val Ser
465                 470                 475                 480

Gln His Pro Gly Ala Arg Ala Leu Tyr Thr Glu Gln Leu Ile Ala Glu
              485                 490                 495

Gly Val Val Thr Gln Ala Glu Ala Asp Gly Tyr Ile Gln Ala Tyr Arg
              500                 505                 510

Asp Ala Leu Asp Lys Gly Glu His Val Glu Gln Thr Thr Leu Ser Asn
    515                 520                 525

Phe Gln Arg Thr Gln Ile Asp Trp Ser Lys Tyr Gln Gly Lys Asp Trp
```

```
                530                      535                      540
            Arg Glu His Ile Glu Thr Gly Leu Pro Ala Ala Asp Ile Glu Arg Leu
            545                 550                 555                 560

            Thr Glu Lys Phe Thr Ala Val Pro Glu Gly Phe Ala Leu His Pro Thr
                            565                 570                 575

            Ala Lys Arg Val Ile Glu Ala Arg Lys Ala Met Ala Ser Gly Lys Gln
                        580                 585                 590

            Ala Ile Asp Trp Gly Met Ala Glu Thr Leu Ala Tyr Ala Ser Leu Val
                    595                 600                 605

            Thr Lys Gly His Gly Val Arg Ile Ser Gly Glu Asp Ser Gly Arg Gly
                610                 615                 620

            Thr Phe Ser His Arg His Ala Val Leu His Asp Gln Lys Arg Glu Lys
            625                 630                 635                 640

            Trp Asp Asp Gly Thr Tyr Val Pro Leu Arg His Met Gly Glu Gly Met
                            645                 650                 655

            Gly Glu Phe Leu Val Ile Asp Ser Ile Leu Asn Glu Glu Ala Val Met
                        660                 665                 670

            Ala Phe Glu Tyr Gly Phe Ala Cys Ser Ala Pro Asp Lys Leu Thr Ile
                    675                 680                 685

            Trp Glu Ala Gln Phe Gly Asp Phe Ala Asn Gly Ala Gln Val Thr Ile
                690                 695                 700

            Asp Gln Phe Leu Ser Ser Gly Glu Thr Lys Trp Gly Arg Leu Cys Gly
            705                 710                 715                 720

            Leu Thr Thr Ile Leu Pro His Gly Tyr Asp Gly Gln Gly Pro Glu His
                            725                 730                 735

            Ser Ser Ala Arg Val Glu Arg Trp Leu Gln Leu Cys Ser Glu Asn Asn
                        740                 745                 750

            Met Gln Val Ile Met Pro Ser Glu Ala Ser Gln Met Phe His Leu Leu
                    755                 760                 765

            Gln Arg Gln Val Leu Gly Ser Tyr Arg Lys Pro Leu Val Ile Phe Met
                770                 775                 780

            Ser Lys Arg Leu Leu Arg Phe Lys Gly Ala Met Ser Pro Leu Glu Asn
            785                 790                 795                 800

            Phe Thr Glu Gly Ser Thr Phe Arg Pro Val Ile Gly Asp Thr Ala Glu
                            805                 810                 815

            Arg Ala Ser Asn Asp Ser Val Lys Arg Val Val Leu Cys Ala Gly Gln
                        820                 825                 830

            Val Tyr Tyr Asp Leu Glu Ala Gly Arg Ala Glu Arg Lys Leu Glu Asp
                    835                 840                 845

            Asp Val Ala Ile Val Arg Val Glu Gln Leu Tyr Pro Phe Pro Tyr Asp
                850                 855                 860

            Glu Val Lys Ala Glu Leu Ala Lys Tyr Pro Asn Ala Lys Ser Val Val
            865                 870                 875                 880

            Trp Ala Gln Glu Glu Pro Lys Asn Gln Gly Ala Phe Tyr Gln Ile Arg
                            885                 890                 895

            His Arg Ile Glu Asp Val Ile Ser Glu Glu Gln Lys Leu Ser Tyr Ala
                        900                 905                 910
```

206

```
Gly Arg Pro Ser Ser Ala Ser Pro Ala Val Gly Tyr Ser Ser Lys His
        915                 920             925

Ile Ala Gln Leu Lys Gln Leu Val Glu Asp Ala Leu Ala Leu
    930                 935             940
```

<210> 98
<211> 393
<212> PRT
<213> Neisseria meningitidis

<400> 98

EP 2 279 746 B1

```
Met Ile Ile Asp Val Lys Val Pro Met Leu Ser Glu Ser Val Ser Glu
1               5               10              15

Gly Thr Leu Leu Glu Trp Lys Lys Lys Val Gly Glu Ala Val Ala Arg
        20              25              30

Asp Glu Ile Leu Ile Asp Ile Glu Thr Asp Lys Val Val Leu Glu Val
        35              40              45

Pro Ser Pro Gln Ala Gly Val Leu Val Glu Ile Val Ala Gln Asp Gly
    50              55              60

Glu Thr Val Val Ala Asp Gln Val Leu Ala Arg Val Asp Thr Ala Ala
65              70              75              80

Thr Ala Ala Ala Glu Ala Pro Ala Ala Ala Pro Ala Glu Ala Ala Pro
            85              90              95

Ala Ala Ala Pro Ala Ala Thr Gln Asn Asn Ala Ala Met Pro Ala Ala
        100             105             110

Ala Lys Leu Ala Ala Glu Thr Gly Val Asp Val Asn Ala Leu Gln Gly
    115             120             125

Ser Gly Arg Asp Gly Arg Val Leu Lys Glu Asp Val Gln Asn Ala Ala
    130             135             140

Ala Lys Pro Ala Gly Ala Ala Ala Pro Ala Val Ala Leu Pro Ala Gly
145             150             155             160

Ala Arg Pro Glu Glu Arg Val Pro Met Ser Arg Leu Arg Ala Arg Val
            165             170             175

Ala Glu Arg Leu Leu Ala Ser Gln Gln Glu Asn Ala Ile Leu Thr Thr
            180             185             190

Phe Asn Glu Val Asn Met Lys Pro Ile Met Asp Leu Arg Ala Lys Tyr
    195             200             205

Lys Glu Lys Phe Glu Lys Glu His Gly Val Lys Leu Gly Phe Met Ser
    210             215             220

Phe Phe Val Lys Ala Ala Val Ala Ala Leu Lys Lys Tyr Pro Val Val
225             230             235             240

Asn Ala Ser Val Asp Gly Lys Asp Ile Val Tyr His Gly Tyr Phe Asp
            245             250             255

Ile Gly Ile Ala Ile Gly Ser Pro Arg Gly Leu Val Val Pro Ile Leu
        260             265             270

Arg Asp Ala Asp Gln Met Ser Ile Ala Asp Ile Glu Gln Ala Ile Val
    275             280             285

Asp Tyr Ala Lys Lys Ala Lys Asp Gly Lys Ile Ala Ile Glu Asp Leu
    290             295             300
```

208

```
Thr Gly Gly Thr Phe Ser Ile Thr Asn Gly Gly Thr Phe Gly Ser Met
305                 310                 315                 320

Met Ser Thr Pro Ile Ile Asn Pro Pro Gln Ser Ala Ile Leu Gly Met
                325                 330                 335

His Ala Thr Lys Glu Arg Ala Val Val Glu Asn Gly Gln Val Val Val
            340                 345                 350

Arg Pro Met Met Tyr Leu Ala Leu Ser Tyr Asp His Arg Ile Ile Asp
            355                 360                 365

Gly Arg Glu Ala Val Leu Thr Leu Val Ala Ile Lys Asp Ala Leu Glu
    370                 375                 380

Asp Pro Ala Arg Leu Leu Leu Asp Leu
385                 390
```

<210> 99
<211> 388
<212> PRT
<213> Neisseria meningitidis

<400> 99

```
Met Asn Leu His Glu Tyr Gln Ala Lys Glu Leu Leu Ala Ser Tyr Gly
1               5                   10                      15

Leu Pro Val Gln Gly Gly Ile Leu Ala His Asn Gly Glu Glu Ala Ala
            20                  25                  30

Ala Ala Tyr Asp Lys Leu Gly Gly Lys Phe Ala Val Val Lys Ala Gln
        35                  40                  45

Val His Ala Gly Gly Arg Gly Lys Ala Gly Gly Val Lys Val Val Lys
    50                  55                  60

Ser Arg Glu Glu Ala Lys Glu Val Ala Glu Ser Leu Ile Gly Thr Asn
65                  70                  75                      80

Leu Val Thr Tyr Gln Thr Asp Ala Asn Gly Gln Pro Val Asn Ser Val
            85                  90                  95

Leu Val Cys Glu Asp Met Tyr Pro Val Gln Thr Glu Leu Tyr Leu Gly
            100                 105                 110

Ala Val Val Asp Arg Ser Thr Arg Arg Ile Thr Phe Met Ala Ser Thr
            115                 120                 125

Glu Gly Gly Val Glu Ile Glu Lys Val Ala Ala Glu Thr Pro Glu Lys
    130                 135                 140

Ile Phe Lys Val Thr Val Asp Pro Leu Val Gly Leu Gln Pro Cys Gln
145                 150                 155                 160

Ala Arg Glu Val Ala Phe Gln Leu Gly Leu Lys Asp Lys Gln Ile Asn
            165                 170                 175

Glu Phe Val Lys Leu Met Thr Gly Ala Tyr Lys Ala Phe Val Glu Asn
            180                 185                 190

Asp Phe Ala Leu Phe Glu Val Asn Pro Leu Ala Val Arg Glu Asn Gly
        195                 200                 205

Ala Leu Ala Cys Val Asp Gly Lys Ile Gly Ile Asp Ser Asn Ala Leu
    210                 215                 220

Tyr Arg Leu Pro Lys Ile Ala Glu Leu Arg Asp Lys Ser Gln Glu Asn
225                 230                 235                 240

Glu Arg Glu Leu Lys Ala Ser Glu Phe Asp Leu Asn Tyr Val Ala Leu
```

                           245                    250                    255

Glu Gly Asn Ile Gly Cys Met Val Asn Gly Ala Gly Leu Ala Met Ala
            260                 265                 270

Thr Met Asp Ile Ile Lys Leu Lys Gly Gly Gln Pro Ala Asn Phe Leu
            275                 280                 285

Asp Val Gly Gly Gly Ala Thr Lys Asp Arg Val Val Glu Ala Phe Lys
            290                 295                 300

Leu Ile Leu Glu Asp Lys Ser Val Gln Gly Val Leu Ile Asn Ile Phe
305                 310                 315                 320

Gly Gly Ile Val Arg Cys Asp Met Ile Ala Glu Ala Ile Val Ala Ala
                325                 330                 335

Val Lys Glu Ile Asn Val Asn Val Pro Val Val Val Arg Leu Glu Gly
            340                 345                 350

Asn Asn Ala Glu Leu Gly Ala Lys Ile Leu Asn Glu Ser Gly Leu Lys
            355                 360                 365

Leu Thr Ser Ala Asp Gly Leu Asn Asp Ala Ala Glu Lys Ile Val Ala
370                 375                 380

Ala Val Asn Ala
385

<210> 100
<211> 296
<212> PRT
<213> Neisseria meningitidis

<400> 100

```
Met Ser Val Leu Ile Asn Lys Asp Thr Lys Val Leu Val Gln Gly Phe
1               5               10              15

Thr Gly Lys Asn Gly Thr Phe His Ser Glu Gln Ala Leu Ala Tyr Gly
            20              25              30

Thr Lys Val Val Gly Gly Val Thr Pro Gly Lys Gly Gly Gln Thr His
        35              40              45

Leu Asn Leu Pro Val Phe Asn Thr Met Lys Glu Ala Val Lys Glu Thr
    50              55              60

Gly Ala Asp Ala Ser Val Ile Tyr Val Pro Ala Pro Phe Val Leu Asp
65              70              75              80

Ser Ile Val Glu Ala Val Asp Ser Gly Val Gly Leu Val Val Val Ile
            85              90              95

Thr Glu Gly Val Pro Thr Leu Asp Met Leu Lys Ala Lys Arg Tyr Leu
            100             105             110

Glu Thr Asn Gly Asn Gly Thr Arg Leu Val Gly Pro Asn Cys Pro Gly
        115             120             125

Val Ile Thr Pro Gly Glu Cys Lys Ile Gly Ile Met Pro Gly His Ile
    130             135             140

His Thr Pro Gly Arg Ile Gly Ile Ile Ser Arg Ser Gly Thr Leu Thr
145             150             155             160

Tyr Glu Ala Val Ala Gln Thr Thr Lys Leu Gly Leu Gly Gln Ser Thr
            165                     170             175

Cys Ile Gly Ile Gly Gly Asp Pro Ile Pro Gly Met Asn Gln Ile Asp
            180             185             190


Ala Leu Lys Leu Phe Gln Glu Asp Pro Asp Thr Asp Ala Ile Ile Met
        195             200             205

Ile Gly Glu Ile Gly Gly Thr Ala Glu Glu Glu Ala Ala Glu Tyr Ile
    210             215             220

Gln Ser Asn Val Ser Lys Pro Val Val Gly Tyr Ile Ala Gly Val Thr
225             230             235             240

Ala Pro Lys Gly Lys Arg Met Gly His Ala Gly Ala Ile Ile Ser Gly
            245             250             255

Gly Lys Gly Thr Ala Glu Glu Lys Phe Ala Ala Phe Glu Lys Ala Gly
        260             265             270

Ile Ala Tyr Thr Arg Ser Pro Ala Glu Leu Gly Thr Thr Met Leu Glu
    275             280             285

Val Leu Lys Ala Lys Gly Leu Ala
290             295
```

<210> 101
<211> 526
<212> PRT
<213> Neisseria meningitidis

<400> 101

```
Met Ser Ser Ile Lys Arg Ala Leu Ile Ser Leu Ser Asp Lys Thr Gly
1               5               10              15

Ala Val Glu Phe Ala Gln Thr Leu His Lys Leu Gly Val Glu Ile Leu
            20              25              30

Ser Thr Gly Gly Thr Ala Lys Leu Leu Ala Asp Ala Gly Val Pro Val
        35              40              45

Ile Glu Val Ala Asp Tyr Thr Gly Phe Pro Glu Met Leu Asp Gly Arg
    50              55              60

Val Lys Thr Leu His Pro Lys Ile His Gly Gly Ile Leu Gly Arg Arg
65              70              75              80

Asp Leu Asp Glu His Val Ala Lys Met Glu Glu His Gly Ile Gly Asn
            85              90              95

Ile Asp Leu Val Cys Val Asn Leu Tyr Leu Phe Ala Ala Thr Ile Ala
        100             105             110

Lys Pro Asn Cys Thr Leu Glu Asp Ala Ile Glu Asn Ile Asp Ile Gly
        115             120             125

Gly Pro Thr Met Val Arg Ser Ala Ala Lys Asn Trp Lys His Val Ala
    130             135             140

Ile Val Thr Asp Thr Ala Asp Phe Pro Ala Ile Ala Ala Glu Leu Glu
145             150             155             160

Ala Asn Asn Gly Ala Leu Ser Asp Lys Thr Arg Phe Asn Leu Ser Arg
            165             170             175

Lys Ala Phe Ser His Thr Ala Gln Tyr Asp Gly Met Ile Ser Asn Tyr
        180             185             190

Leu Thr Ser Leu Ser Asp Asp Val Leu Ser Gly Thr Pro Glu Ile Ala
        195             200             205

Gly Phe Pro Gly Arg Phe Asn Gln Ser Trp Ile Lys Val Gln Asp Met
    210             215             220
```

```
Arg Tyr Gly Glu Asn Pro His Gln Arg Ala Ala Phe Tyr Arg Asp Ile
225             230             235             240

Asp Pro Ala Ala Gly Ser Leu Ala Ala Tyr Lys Gln Leu Gln Gly Lys
            245             250             255

Glu Leu Ser Tyr Asn Asn Ile Ala Asp Ala Asp Ala Ala Trp Glu Ala
            260             265             270

Val Lys Ser Phe Asp Val Pro Ala Cys Val Ile Val Lys His Ala Asn
            275             280             285

Pro Cys Gly Val Ala Ile Ala Ser Asn Thr Leu Asp Ala Tyr Lys Leu
    290             295             300

Ala Tyr Ala Thr Asp Thr Thr Ser Ala Phe Gly Gly Ile Ile Ala Phe
305             310             315             320

Asn Arg Glu Val Asp Gly Ala Thr Val Lys Gln Ile Thr Asp Asn Gln
            325             330             335

Phe Met Glu Val Leu Met Ala Pro Lys Phe Thr Ala Glu Ala Leu Glu
            340             345             350

Ile Ala Ala Ala Lys Lys Asn Val Arg Val Leu Glu Val Pro Leu Glu
            355             360             365

Ala Gly Ala Asn Arg Phe Glu Leu Lys Arg Val Gly Gly Gly Leu Leu
    370             375             380

Val Gln Thr Pro Asp Ile His Arg Ile Ser Arg Ala Asp Leu Lys Val
385             390             395             400

Val Ser Lys Arg Gln Pro Thr Glu Gln Glu Trp Asn Asp Leu Leu Phe
            405             410             415

Val Trp Asn Val Ala Lys Tyr Val Lys Ser Asn Ala Ile Val Phe Gly
            420             425             430

Lys Gly Gly Gln Thr Tyr Gly Ile Gly Ala Gly Gln Met Ser Arg Val
            435             440             445

Asp Ser Thr Arg Ile Ala Ala Arg Lys Ala Gln Asp Ala Gly Leu Asp
    450             455             460

Leu Asn Gly Ala Cys Ala Ala Ser Asp Ala Phe Phe Pro Phe Arg Asp
465             470             475             480

Gly Val Asp Val Ile Ala Glu Gln Gly Ile Lys Ala Ile Ile His Pro
            485             490             495

Ala Gly Ser Met Arg Asp Gln Glu Val Phe Asp Ala Ala Asp Glu His
    500             505             510

Gly Ile Ala Met Val Val Thr Gly Ile Arg His Phe Arg His
    515             520             525
```

<210> 102
<211> 563
<212> PRT
<213> Neisseria meningitidis

<400> 102

Met Ser Ala Ser Gln Leu Leu Ser Arg Leu Thr Gln Thr Val Gly Glu

Lys Tyr Ile Ile Thr Asp Pro Ala Lys Thr Glu Gln Tyr Arg Gln Gly

Tyr Arg Phe Gly Glu Gly Lys Ala Leu Ala Val Val Arg Pro Gly Ser

|  | 35 | | | | 40 | | | | 45 | | |
|---|---|---|---|---|---|---|---|---|---|---|---|
| Ile | Leu<br>50 | Glu | Met | Trp | Lys | Ile<br>55 | Leu | Gln | Ala | Cys | Val<br>60 | Glu | Ala | Asp | Val |

Ile Leu Glu Met Trp Lys Ile Leu Gln Ala Cys Val Glu Ala Asp Val
50 55 60

Ile Val Ile Thr Gln Ala Ala Asn Thr Gly Leu Thr Gly Gly Ser Thr
65 70 75 80

Pro Asp Gly Asn Asp Tyr Asp Arg Asp Ile Val Ile Val Asn Thr Met
85 90 95

Arg Met Asn Ile Ile Gln Thr Ile Asn Asn Asn Glu Gln Val Val Cys
100 105 110

Leu Pro Gly Ser Thr Leu Asn Gln Leu Glu Leu Leu Leu Lys Pro Leu
115 120 125

Gly Arg Glu Pro His Ser Val Ile Gly Ser Ser Cys Ile Gly Ala Ser
130 135 140

Val Leu Gly Gly Val Cys Asn Asn Ser Gly Gly Ala Leu Val Gln Arg
145 150 155 160

Gly Pro Ala Tyr Thr Glu Met Ala Leu Phe Ala Gln Ile Asn Glu Glu
165 170 175

Gly Arg Leu Glu Leu Val Asn His Leu Gly Ile Asp Leu Gly Asn Thr
180 185 190

Pro Glu Glu Ile Leu Thr Asn Leu Gln Gly His His Tyr Gln Asn Lys
195 200 205

Asp Ile Thr Gln Asp Ala Gly Lys Gly His Asp His Ala Tyr Cys Glu
210 215 220

His Val Arg Gln Val Asp Glu Pro Thr Ala Ala Arg Phe Asn Ala Asp
225 230 235 240

Pro Ala Arg His Tyr Glu Ala Ser Gly Cys Ala Gly Lys Leu Met Val
245 250 255

Phe Ala Val Arg Leu Asp Thr Phe Pro Gln Glu Lys Gln Thr Ala Val
260 265 270

Phe Tyr Ile Gly Thr Asn Asp Ile Asn Glu Leu Thr Asp Ile Arg Arg
275 280 285

Ala Ala Leu Gly Glu Phe Glu Ser Leu Pro Val Ser Gly Glu Tyr Ile
290 295 300

His Arg His Ala Phe Asp Ile Ala Asp Val Tyr Gly Lys Asp Thr Phe
305 310 315 320

Tyr Val Ile Lys Lys Phe Gly Thr His Gln Leu Pro Lys Leu Phe Asp
325 330 335

Leu Lys Ala Arg Val Asp Arg Phe Gly Lys Lys Val Ser Phe Leu Pro
340 345 350

Lys His Phe Ser Asp Lys Ala Met Gln Phe Val Ser Lys Phe Leu Pro
355 360 365

Asp His Leu Pro Lys Ser Met Arg Asp Tyr Arg Asp Lys Tyr Glu His
370 375 380

His Leu Ile Leu Lys Met Gly Gly Lys Gly Val Asp Glu Ala Arg Ala
385 390 395 400

Phe Leu Lys Glu Tyr Phe Ala His His Gly Gly Ala Phe Phe Glu Cys
405 410 415

```
        Asn Ala Glu Glu Thr Gln Ala Ala Met Leu His Arg Phe Ala Val Ala
                    420                 425                 430

        Ser Ala Ala Ile Arg Tyr Arg Ala Val His Asp Asp Glu Val Glu Asp
                    435                 440                 445

        Leu Val Ala Leu Asp Ile Ala Leu Arg Arg Asp Asp Arg Asp Trp Phe
            450                 455                 460

        Glu Lys Leu Pro Pro Glu Ile Asp Asn Lys Ile Ile His Lys Leu Tyr
        465                 470                 475                 480

        Tyr Gly His Phe Met Cys His Val Phe His Gln Asp Tyr Ile Ile Lys
                        485                 490                 495

        Lys Gly Asn Asp Cys Met Ala Leu Glu His Glu Met Leu His Leu Leu
                    500                 505                 510

        Asp Gln Arg Gly Ala Gln Tyr Pro Ala Glu His Asn Val Gly His Leu
                    515                 520                 525

        Tyr Glu Ala Lys Pro Ala Leu Lys Gln Phe Tyr Arg Lys Leu Asp Pro
            530                 535                 540

        Thr Asn Ser Phe Asn Pro Gly Val Gly Lys Thr Ser Lys Lys Lys Asn
        545                 550                 555                 560

        Trp Ala Glu
```

<210> 103
<211> 356
<212> PRT
<213> Neisseria meningitidis

<400> 103

```
Met Thr Lys His Ile Ala Ile Leu Arg Gly Asp Gly Ile Gly Pro Glu
1               5               10              15

Ile Val Ala Glu Thr Val Arg Val Leu Asp Lys Leu Ile Ala Gln Gly
        20              25              30

Leu Asp Ala Gly Tyr Glu Tyr Ala Pro Leu Gly Gly Glu Ala Tyr Asp
        35              40              45

Glu Tyr Gly His Pro Tyr Pro Glu Phe Thr Gln Asn Leu Cys Arg Lys
        50              55              60

Ala Asp Ala Val Leu Leu Gly Ala Val Gly Ser Pro Gln Tyr Asp Asn
65              70              75              80

Leu Asp Arg Pro Leu Arg Pro Glu Arg Gly Leu Leu Ala Ile Arg Lys
            85              90              95

Asp Leu Asn Leu Phe Ala Asn Leu Arg Pro Ala Val Leu Tyr Pro Glu
            100             105             110

Leu Ala Asn Ala Ser Thr Leu Lys Pro Glu Ile Val Ala Gly Leu Asp
        115             120             125

Ile Leu Ile Val Arg Glu Leu Thr Gly Asp Ile Tyr Phe Gly Glu Pro
    130             135             140

Arg Gly Ile Arg Val Leu Glu Asn Gly Glu Arg Glu Gly Tyr Asn Thr
145             150             155             160

Met Lys Tyr Ser Glu Ser Glu Ile Arg Arg Ile Ala His Val Ala Phe
            165             170             175

Gln Ser Ala Gln Lys Arg Ser Lys Lys Val Cys Ser Val Gly Lys Ala
            180             185             190

Asn Val Leu Glu Thr Thr Glu Leu Trp Arg Glu Ile Phe Glu Glu Ile
        195             200             205

Gly Lys Glu Tyr Pro Asp Val Glu Leu Ser His Met Tyr Val Asp Asn
    210             215             220

Ala Ala Met Gln Leu Val Arg Ala Pro Lys Gln Phe Asp Val Ile Ala
225             230             235             240

Thr Gly Asn Ile Phe Gly Asp Ile Leu Ser Asp Glu Ala Ser Met Leu
            245             250             255

Thr Gly Ser Ile Gly Met Leu Pro Ser Ala Ser Leu Asp Glu Asn Gly
            260             265             270

Lys Gly Leu Tyr Glu Pro Ser His Gly Ser Ala Pro Asp Ile Ala Gly
        275             280             285

Gln Asn Lys Ala Asn Pro Leu Ala Thr Ile Leu Ser Leu Ala Met Leu
    290             295             300

Leu Arg Tyr Ser Leu Asn Asp Glu Ala Arg Ala Gln Gln Val Glu Asn
305             310             315             320

Ala Val Gln Lys Val Leu Gln Gln Gly Leu Arg Thr Ser Asp Ile Tyr
            325             330             335

Glu Glu Gly Thr Lys Leu Val Ser Cys Ser Glu Met Gly Asp Ala Val
            340             345             350

Leu Ala Ala Leu
            355
```

<210> 104
<211> 258
<212> PRT
<213> Neisseria meningitidis

<400> 104

```
Met Ala Ala Phe Asn Thr Gln Lys Val Leu Ser Val His His Trp Thr
1               5                   10                  15

Asp Ala Tyr Phe Thr Phe Thr Cys Thr Arg Asp Glu Ser Leu Arg Phe
            20              25                  30

Glu Asn Gly Gln Phe Val Met Val Gly Leu Met Val Asp Gly Lys Pro
        35                  40                  45

Leu Met Arg Ala Tyr Ser Val Ala Ser Ala Asn Trp Glu Glu His Leu
    50                  55                  60

Glu Phe Phe Ser Ile Lys Val Gln Asp Gly Pro Leu Thr Ser Arg Leu
65                  70                  75                  80

Gln His Leu Lys Val Gly Asp Asp Val Leu Ile Ser Lys Lys Pro Thr
                85                  90                  95

Gly Thr Leu Val Ala Gly Asp Leu Asn Pro Gly Lys His Leu Tyr Leu
            100                 105                 110

Leu Ser Thr Gly Thr Gly Ile Ala Pro Phe Leu Ser Ile Thr Lys Asp
        115                 120                 125

Pro Glu Ile Tyr Glu Gln Phe Glu Lys Ile Ile Leu Val His Gly Val
    130                 135                 140

Arg Tyr Lys Lys Asp Leu Ala Tyr Tyr Asp Arg Phe Thr Lys Glu Leu
145                 150                 155                 160

Pro Glu His Glu Tyr Leu Gly Asp Leu Val Lys Glu Lys Leu Ile Tyr
                165                 170                 175

Tyr Pro Ile Val Ser Arg Glu Glu Phe Glu His His Gly Arg Leu Thr
            180                 185                 190

Asp Leu Met Val Ser Gly Lys Leu Phe Glu Asp Ile Gly Leu Pro Lys
        195                 200                 205

Ile Asn Pro Gln Asp Asp Arg Ala Met Leu Cys Gly Ser Pro Ala Met
    210                 215                 220

Leu Lys Asp Thr Cys Lys Val Leu Asp Asp Phe Gly Leu Thr Val Ser
225                 230                 235                 240

Pro Lys Thr Gly Val Arg Gly Asp Tyr Leu Ile Glu Arg Ala Phe Val
                245                 250                 255

Asp Gln
```

<210> 105
<211> 469
<212> PRT
<213> Neisseria meningitidis

<400> 105

```
Met Lys Tyr Ile Ser Thr Arg Gly Glu Thr Ala His Lys Pro Phe Ser
1               5               10              15

Glu Val Leu Leu Met Gly Leu Ala Pro Asp Gly Gly Leu Met Leu Pro
        20              25              30

Glu His Tyr Pro Gln Ile Gly Arg Glu Thr Leu Asp Lys Trp Arg Gly
        35              40              45

Leu Ala Tyr Pro Glu Leu Ala Phe Glu Ile Met Arg Leu Phe Val Thr
        50              55              60

Asp Ile Pro Glu Asp Asp Leu Arg Asp Ile Leu Asn Arg Thr Tyr Thr
65              70              75              80

Glu Ala Ala Phe Gly Thr Lys Glu Ile Thr Pro Val Arg Thr Leu Ser
                85              90              95

Asp Gly Ile Lys Ile Gln Ala Leu Ser Asn Gly Pro Thr Leu Ala Phe
            100             105             110

Lys Asp Met Ala Met Gln Phe Leu Gly Asn Ala Phe Glu Tyr Val Leu
        115             120             125

Asn Lys Glu Gly Lys Lys Leu Asn Ile Leu Gly Ala Thr Ser Gly Asp
    130             135             140

Thr Gly Ser Ala Ala Glu Tyr Ala Leu Arg Gly Lys Lys Gly Val Asn
145             150             155             160

Val Phe Met Leu Ser Pro Asp Gly Lys Met Ser Ala Phe Gln Arg Ala
                165             170             175

Gln Met Tyr Ser Leu Gln Asp Glu Asn Ile His Asn Ile Ala Val Lys
        180             185             190

Gly Met Phe Asp Asp Cys Gln Asp Ile Val Lys Ala Val Gln Asn Asp
    195             200             205

Ala Ala Phe Lys Glu Lys Tyr His Ile Gly Thr Val Asn Ser Ile Asn
    210             215             220
```

```
Trp Gly Arg Ile Val Ala Gln Val Val Tyr Tyr Phe Ala Gly Tyr Phe
225             230             235             240

Asn Ala Thr Ser Ser Asn Asp Glu Thr Val Ser Phe Cys Val Pro Ser
                245             250             255

Gly Asn Phe Gly Asn Val Cys Ala Gly His Ile Ala Lys Gln Met Gly
            260             265             270

Leu Pro Ile Arg Arg Leu Ile Val Ala Thr Asn Glu Asn Asp Val Leu
        275             280             285

Asp Glu Phe Phe Lys Thr Gly Ala Tyr Arg Pro Arg Asn Ser Ala His
    290             295             300

Thr Tyr Val Thr Ser Ser Pro Ser Met Asp Ile Ser Lys Ala Ser Asn
305             310             315             320

Phe Glu Arg Phe Val Phe Asp Leu Met Asp Arg Asp Pro Ala Glu Ile
            325             330             335

Asn Thr Leu Trp Ala Glu Val Ala Ala Gly Lys Gly Phe Asp Leu Arg
            340             345             350

Phe Ala Leu Asp Lys Val Gly Gly Lys Tyr Gly Phe Thr Ser Gly Lys
        355             360             365

Ser Thr His Ala Asp Arg Leu Ala Thr Ile Lys Gln Val Tyr Glu Gln
    370             375             380

Asp Gln Glu Leu Ile Asp Pro His Thr Ala Asp Gly Val Lys Val Ala
385             390             395             400

Arg Glu Val Arg Glu Glu Gly Glu Met Val Val Cys Leu Glu Thr Ala
            405             410             415

Leu Ala Ala Lys Phe Asp Ala Thr Ile Arg Glu Ala Val Gly Asp Ala
        420             425             430

Ala Ile Pro Arg Pro Ala Ala Leu Glu Gly Leu Glu Asn Leu Pro Gln
        435             440             445

Arg Val Gln Thr Val Pro Asn Ser Ala Asp Ala Val Lys Gly Ile Ile
    450             455             460

Glu Gln Thr Leu Ala
465
```

<210> 106
<211> 272
<212> PRT
<213> Neisseria meningitidis

<400> 106

```
Met Lys Lys Thr Val Phe Thr Cys Ala Met Ile Ala Leu Thr Gly Thr
1               5               10              15

Ala Ala Ala Ala Gln Glu Leu Gln Thr Ala Asn Glu Phe Thr Val His
        20              25              30

Thr Asp Leu Ser Ser Ile Ser Ser Thr Arg Ala Phe Leu Lys Glu Lys
        35              40              45

His Lys Ala Ala Lys His Ile Ser Val Arg Ala Asp Ile Pro Phe Asp
    50              55              60

Ala Asn Gln Gly Ile Arg Leu Glu Ala Gly Phe Gly Arg Ser Lys Lys
65              70              75              80

Asn Ile Ile Asn Leu Glu Thr Asp Glu Asn Lys Leu Gly Lys Thr Lys
            85              90              95

Asn Val Lys Leu Pro Thr Gly Val Pro Glu Asn Arg Ile Asp Leu Tyr
            100             105             110

Thr Gly Tyr Thr Tyr Thr Gln Thr Leu Ser Asp Ser Leu Asn Phe Arg
        115             120             125

Val Gly Ala Gly Leu Gly Phe Glu Ser Ser Lys Asp Ser Ile Lys Thr
    130             135             140

Thr Lys His Thr Leu His Ser Ser Arg Gln Ser Trp Leu Ala Lys Val
145             150             155             160

His Ala Asp Leu Leu Ser Gln Leu Gly Asn Gly Trp Tyr Ile Asn Pro
            165             170             175

Trp Ser Glu Val Lys Phe Asp Leu Asn Ser Arg Tyr Lys Leu Asn Thr
            180             185             190

Gly Val Thr Asn Leu Lys Lys Asp Ile Asn Gln Lys Thr Asn Gly Trp
        195             200             205

Gly Phe Gly Leu Gly Ala Asn Ile Gly Lys Lys Leu Gly Glu Ser Ala
    210             215             220

Ser Ile Glu Ala Gly Pro Phe Tyr Lys Gln Arg Thr Tyr Lys Glu Ser
225             230             235             240

Gly Glu Phe Ser Val Thr Thr Lys Ser Gly Asp Val Ser Leu Thr Ile
            245             250             255

Pro Lys Thr Ser Ile Arg Glu Tyr Gly Leu Arg Val Gly Ile Lys Phe
            260             265             270
```

<210> 107
<211> 517
<212> PRT
<213> Neisseria meningitidis

<400> 107

```
Met Thr Gln Thr Asn Arg Val Ile Ile Phe Asp Thr Thr Leu Arg Asp
1               5                   10                  15

Gly Glu Gln Ser Pro Gly Ala Ala Met Thr Lys Glu Glu Lys Ile Arg
            20                  25                  30

Val Ala Arg Gln Leu Glu Lys Leu Gly Val Asp Ile Ile Glu Ala Gly
            35                  40                  45

Phe Ala Ala Ala Ser Pro Gly Asp Phe Glu Ala Val Asn Ala Ile Ala
    50                  55                  60

Lys Thr Ile Thr Lys Ser Thr Val Cys Ser Leu Ser Arg Ala Ile Glu
65                  70                  75                  80

Arg Asp Ile Arg Gln Ala Gly Glu Ala Val Ala Pro Ala Pro Lys Lys
            85                  90                  95

Arg Ile His Thr Phe Ile Ala Thr Ser Pro Ile His Met Glu Tyr Lys
            100                 105                 110

Leu Lys Met Lys Pro Lys Gln Val Ile Glu Ala Ala Val Lys Ala Val
            115                 120                 125

Lys Ile Ala Arg Glu Tyr Thr Asp Asp Val Glu Phe Ser Cys Glu Asp
    130                 135                 140
```

```
Ala Leu Arg Ser Glu Ile Asp Phe Leu Ala Glu Ile Cys Gly Ala Val
145             150             155             160

Ile Glu Ala Gly Ala Thr Thr Ile Asn Ile Pro Asp Thr Val Gly Tyr
            165             170             175

Ser Ile Pro Tyr Lys Thr Glu Glu Phe Phe Arg Glu Leu Ile Ala Lys
            180             185             190

Thr Pro Asn Gly Gly Lys Val Val Trp Ser Ala His Cys His Asn Asp
        195             200             205

Leu Gly Leu Ala Val Ala Asn Ser Leu Ala Ala Leu Lys Gly Gly Ala
        210             215             220

Arg Gln Val Glu Cys Thr Val Asn Gly Leu Gly Glu Arg Ala Gly Asn
225             230             235             240

Ala Ser Val Glu Glu Ile Val Met Ala Leu Lys Val Arg His Asp Leu
            245             250             255

Phe Gly Leu Glu Thr Gly Ile Asp Thr Thr Gln Ile Val Pro Ser Ser
            260             265             270

Lys Leu Val Ser Thr Ile Thr Gly Tyr Pro Val Gln Pro Asn Lys Ala
            275             280             285

Ile Val Gly Ala Asn Ala Phe Ser His Glu Ser Gly Ile His Gln Asp
    290             295             300

Gly Val Leu Lys His Arg Glu Thr Tyr Glu Ile Met Ser Ala Glu Ser
305             310             315             320

Val Gly Trp Ala Thr Asn Arg Leu Ser Leu Gly Lys Leu Ser Gly Arg
            325             330             335

Asn Ala Phe Lys Thr Lys Leu Ala Asp Leu Gly Ile Glu Leu Glu Ser
            340             345             350

Glu Glu Ala Leu Asn Ala Ala Phe Ala Arg Phe Lys Glu Leu Ala Asp
        355             360             365

Lys Lys Arg Glu Ile Phe Asp Glu Asp Leu His Ala Leu Val Ser Asp
    370             375             380

Glu Met Gly Ser Met Asn Ala Glu Ser Tyr Lys Phe Ile Ser Gln Lys
385             390             395             400

Ile Ser Thr Glu Thr Gly Glu Glu Pro Arg Ala Asp Ile Val Phe Ser
            405             410             415

Ile Lys Gly Glu Glu Lys Arg Ala Ser Ala Thr Gly Ser Gly Pro Val
            420             425             430

Asp Ala Ile Phe Lys Ala Ile Glu Ser Val Ala Gln Ser Gly Ala Ala
        435             440             445

Leu Gln Ile Tyr Ser Val Asn Ala Val Thr Gln Gly Thr Glu Ser Gln
    450             455             460

Gly Glu Thr Ser Val Arg Leu Ala Arg Gly Asn Arg Val Val Asn Gly
465             470             475             480

Gln Gly Ala Asp Thr Asp Val Leu Val Ala Thr Ala Lys Ala Tyr Leu
            485             490             495

Ser Ala Leu Ser Lys Leu Glu Phe Ser Ala Ala Lys Pro Lys Ala Gln
            500             505             510

Gly Ser Gly Thr Ile
```

515

<210> 108
<211> 376
<212> PRT
<213> Neisseria meningitidis

<400> 108

**225**

```
Met Ile Lys Ile Ser Thr Arg Phe Asp Ala Gly Ser Val Val Val Lys
1               5                   10                  15

Asp Leu Thr Asp Pro Ser Asn Ile Arg Leu Ala Leu Arg Pro Asp Asn
            20                  25                  30

Ala Ser Asp Phe Ala Gln Trp Phe Tyr Phe Arg Leu Gln Gly Ala Ala
            35                  40                  45

Tyr Gln Asn Cys Ile Met His Phe Glu Asn Ala Ala Glu Ala Ala Tyr
    50                  55                  60

Pro Lys Gly Trp Glu Gly Tyr Gln Ala Cys Ala Ser Tyr Asp Arg Arg
65                  70                  75                  80

Asn Trp Phe Arg Val Pro Thr Ser Tyr Glu Asn Gly Val Leu Thr Val
            85                  90                  95

Asn His Thr Pro Leu Ser Asn Ser Val Tyr Tyr Ala Tyr Phe Glu Pro
            100                 105                 110

Tyr Ser Glu Glu Gln His Leu Asn Leu Leu Gly Asp Ala Gln Gly Ser
    115                 120                 125

Gly Leu Cys Arg Ile Asp Asp Leu Gly Ser Thr Val Gln Gly Arg Asp
    130                 135                 140

Ile Asn Leu Leu Thr Ile Gly Asn Gln Val Glu Ser Asp Leu Lys Ile
145                 150                 155                 160

Trp Ile Thr Ala Arg Gln His Pro Gly Glu Thr Met Ala Glu Trp Phe
            165                 170                 175

Ile Glu Gly Leu Leu Gly Arg Leu Leu Asp Pro Gln Asp Pro Thr Ala
            180                 185                 190

Arg Ala Leu Leu Asp Arg Ala Thr Phe Tyr Ile Val Pro Asn Met Asn
            195                 200                 205

Pro Asp Gly Ser Ala Leu Gly Asn Leu Arg Thr Asn Ala Ala Gly Ala
    210                 215                 220

Asn Leu Asn Arg Glu Trp Glu Asn Pro Thr Val Glu Lys Ser Pro Glu
225                 230                 235                 240

Val Phe Phe Val Arg Glu Lys Met Leu Glu Thr Gly Val Asp Leu Phe
            245                 250                 255

Leu Asp Ile His Gly Asp Glu Gly Leu Pro Phe Val Phe Val Ala Gly
            260                 265                 270

Thr Glu Gly Val Pro Asn Tyr Asn Pro Arg Ile Ala Ala Leu Glu Ala
    275                 280                 285

Gln Phe Lys Asn Ala Leu Leu Asn Ala Ser Pro Asp Phe Gln Asp Glu
    290                 295                 300

Tyr Gly Tyr Glu Lys Asp Ala Pro Gly Glu Ala Asn Met Thr Leu Ala
305                 310                 315                 320

Thr Asn Trp Val Gly Asn Arg Phe Asn Cys Leu Ala Tyr Thr Leu Glu
            325                 330                 335
```

Met Pro Phe Lys Asp Asn Ala Asn Leu Pro Asp Asp Asp Phe Gly Trp
340                     345                 350

Asn Gly Gln Arg Ser Leu Arg Leu Gly Glu Ala Ala Leu Ser Ala Ile
        355             360                 365

Leu Asn Val Ile Gly Asp Leu Arg
370             375

<210> 109
<211> 239
<212> PRT
<213> Neisseria meningitidis

<400> 109

Met Ala Thr Leu Ser Asp Lys Thr Ile Leu Val Thr Gly Ala Ser Gln
1               5               10              15

Gly Leu Gly Glu Gln Val Ala Lys Ala Tyr Ala Ala Ala Gly Ala Thr
            20              25              30

Val Ile Leu Val Ala Arg His Gln Lys Lys Leu Glu Lys Val Tyr Asp
        35              40              45

Ala Ile Val Glu Ala Gly Tyr Pro Glu Pro Phe Ala Ile Cys Phe Asp
        50              55              60

Leu Ile Ser Ala Glu Glu Lys Glu Phe Glu His Phe Ala Ala Thr Ile
65              70              75              80

Ala Glu Ala Thr Gln Gly Lys Leu Asp Gly Ile Val His Cys Ala Gly
            85              90              95

Tyr Phe Tyr Ala Leu Ser Pro Leu Asp Phe Gln Thr Val Ala Glu Trp
        100             105             110

Val Asn Gln Tyr Arg Ile Asn Thr Val Ala Pro Met Gly Leu Thr Arg
        115             120             125

Ala Leu Phe Pro Leu Leu Lys Gln Ser Pro Asp Ala Ser Val Ile Phe
        130             135             140

Val Gly Glu Ser His Gly Glu Thr Pro Lys Ala Tyr Trp Gly Gly Phe
145             150             155             160

Gly Ala Ser Lys Ala Ala Leu Asn Tyr Leu Cys Lys Val Ala Ala Asp
            165             170             175

Glu Trp Glu Arg Phe Gly Asn Leu Arg Ala Asn Val Leu Val Pro Gly
            180             185             190

Pro Ile Asn Ser Pro Gln Arg Ile Lys Ser His Pro Gly Glu Ala Lys
        195             200             205

Ser Glu Arg Lys Ser Tyr Gly Asp Val Leu Pro Ala Phe Val Trp Trp
        210             215             220

Ala Ser Ala Glu Ser Lys Gly Arg Ser Gly Glu Ile Val Tyr Leu
225             230             235

<210> 110
<211> 620
<212> PRT
<213> Neisseria meningitidis

<400> 110

```
Met Ala Leu Leu Gln Ile Ser Glu Pro Gly Met Ser Ala Ala Pro His
1               5                   10                  15
```

```
Arg His Arg Leu Ala Ala Gly Ile Asp Leu Gly Thr Thr Asn Ser Leu
            20                  25              30

Val Ala Thr Val Arg Ser Gly Ser Ala Ala Cys Leu Pro Asp Ala Glu
            35              40              45

Gly Arg Val Thr Leu Pro Ser Val Val Arg Tyr Leu Glu Asn Gly Gly
    50              55              60

Ile Glu Val Gly Lys Thr Ala Leu Ser Ala Gln Lys Thr Asp Pro Leu
65              70              75              80

Asn Thr Val Ser Ser Ala Lys Arg Leu Ile Gly Arg Thr Leu Ala Asp
            85              90              95

Leu His Gln Asn Thr His Tyr Leu Pro Tyr Arg Phe Gly Asp Asn Gln
            100             105             110

Arg Val Ile Glu Leu His Thr Arg Gln Gly Val Lys Thr Pro Val Glu
        115             120             125

Val Ser Ala Glu Ile Leu Lys Thr Leu Lys Ser Arg Ala Glu Glu Thr
    130             135             140

Leu Gly Gly Asp Leu Val Gly Val Val Ile Thr Val Pro Ala Tyr Phe
145             150             155             160

Asp Asp Ala Gln Arg Gln Ala Thr Lys Asp Ala Ala Arg Leu Ala Gly
            165             170             175

Leu Asn Val Leu Arg Leu Leu Asn Glu Pro Thr Ala Ala Ala Ile Ala
        180             185             190

Tyr Gly Leu Asp Asn Ala Ser Glu Gly Thr Phe Val Val Tyr Asp Leu
        195             200             205

Gly Gly Gly Thr Phe Asp Val Ser Val Leu Gln Leu Thr Lys Gly Leu
    210             215             220

Phe Glu Val Lys Ala Thr Gly Gly Asn Ser Ala Leu Gly Gly Asp Asp
225             230             235             240

Phe Asp His Arg Leu Phe Cys Arg Leu Leu Glu Gln Asn Gly Leu Ser
            245             250             255

Gln Leu Asn Glu Gln Asp Ser Gln Leu Leu Leu Ser Leu Val Arg Ala
            260             265             270

Ala Lys Glu Gln Leu Thr Thr Gln Thr Glu Ala Arg Ile Gln Ala Thr
        275             280             285

Leu Ser Asp Gly Met Ala Ile Asp Thr Ser Ile Ser Arg Ala Glu Phe
    290             295             300

His Asn Leu Thr Gln His Leu Val Met Lys Thr Leu Glu Pro Val Thr
305             310             315             320

Gln Ala Leu Lys Asp Ala Gly Val Gly Lys Asn Glu Val Lys Gly Val
            325             330             335

Ile Met Val Gly Gly Ser Thr Arg Met Leu His Val Gln Gln Ala Val
            340             345             350

Ala Thr Phe Phe Gly Gln Thr Pro Leu Asn Asn Leu Asn Pro Asp Glu
        355             360             365

Val Val Ala Leu Gly Ala Ala Ile Gln Ala Asn Val Leu Ala Gly Asn
    370             375             380

Lys Thr Asp Gly Glu Trp Leu Leu Leu Asp Val Thr Pro Leu Ser Leu
```

```
              385                    390                  395                    400
        Gly Leu Glu Thr Tyr Gly Gly Leu Ala Glu Lys Ile Ile Pro Arg Asn
                        405                 410                 415

        Ser Thr Ile Pro Thr Ala Arg Ala Gln Asp Phe Thr Thr Phe Lys Asp
                        420                 425                 430

        Gly Gln Thr Ala Met Thr Ile His Val Val Gln Gly Glu Arg Glu Leu
                        435                 440                 445

        Val Ala Asp Cys Arg Ser Leu Ala Lys Phe Thr Leu Arg Gly Ile Pro
            450                 455                 460

        Pro Met Ala Ala Gly Ala Ala Arg Ile Arg Val Thr Phe Gln Ile Asp
        465                 470                 475                 480

        Ala Asp Gly Leu Leu Ser Val Ser Ala Gln Glu Gln Ser Thr Gly Val
                        485                 490                 495

        Gln Ala Gln Ile Glu Val Lys Pro Ser Tyr Gly Leu Asp Asp Asp Thr
                        500                 505                 510

        Ile Thr Gln Met Leu Lys Asp Ser Met Ser Asn Ala Ala Glu Asp Met
                        515                 520                 525

        Ala Ala Arg Ala Arg Ala Glu Ala Val Val Glu Ala Glu Ser Leu Thr
            530                 535                 540

        Asp Ala Val Asn Ala Ala Leu Glu Leu Asp Ser Asp Leu Leu Asp Ala
        545                 550                 555                 560

        Glu Glu Leu Gln Gln Ile Arg Gln Gly Ile Ala Asp Leu Gln Gly Arg
                        565                 570                 575

        Leu Lys Asp Gly Lys Ala Glu Asp Ile Arg Ala Ala Ala Lys Leu
                        580                 585                 590

        Gly Ser Ile Thr Asp Asn Phe Ala Ala Lys Arg Met Asn Arg Asn Ile
                595                 600                 605

        Gln Arg Ala Leu Thr Gly Gln Ser Val Asp Asn Ile
            610                 615                 620
```

<210> 111
<211> 619
<212> PRT
<213> Neisseria meningitidis

<400> 111

```
Met Pro Glu Tyr Arg Ser Lys Thr Ser Thr His Gly Arg Asn Met Ala
1                   5               10                  15

Gly Ala Arg Ala Leu Trp Arg Ala Thr Gly Val Met Glu Thr Asp Phe
            20              25                  30

Gly Lys Pro Ile Ile Ala Val Ala Asn Ser Phe Thr Gln Phe Val Pro
        35              40                  45

Gly His Val His Leu His Asn Met Gly Gln Leu Val Ala Arg Glu Ile
        50              55                  60

Glu Lys Ala Gly Ala Ile Ala Lys Glu Phe Asn Thr Ile Ala Ile Asp
65              70              75                  80

Asp Gly Ile Ala Met Gly His Ser Gly Met Leu Tyr Ser Leu Pro Ser
            85              90                  95

Arg Asp Leu Ile Ala Asp Ser Ile Glu Tyr Met Val Asn Ala His Cys
        100             105                 110
```

```
Ala Asp Ala Leu Val Cys Ile Ser Asn Cys Asp Lys Ile Thr Pro Gly
        115                 120                 125
Met Leu Ile Ala Ala Met Arg Leu Asn Ile Pro Thr Ile Phe Val Ser
    130                 135                 140
Gly Gly Pro Met Glu Ala Gly Lys Val Ile Gly Val Ala Asn Ile Gln
145                 150                 155                 160
Pro Glu Arg Arg Leu Asp Leu Ile Asp Ala Met Ile Glu Ser Ala Asp
                165                 170                 175
Asp Asn Val Ser Asn Arg Gln Val Glu Glu Val Glu Gln Asn Ala Cys
            180                 185                 190
Pro Thr Cys Gly Ser Cys Ser Gly Met Phe Thr Ala Asn Ser Met Asn
        195                 200                 205
Cys Leu Thr Glu Ala Leu Gly Leu Ser Leu Pro Gly Asn Gly Ser Tyr
    210                 215                 220
Leu Ala Thr His Ala Gly Arg Lys Glu Leu Phe Leu Glu Ala Gly Arg
225                 230                 235                 240
Met Ile Val Glu Ile Thr Lys Arg Tyr Tyr Glu Gln Asn Asp Glu Thr
                245                 250                 255
Val Leu Pro Arg Ser Ile Ala Thr Lys Lys Ala Phe Glu Asn Ala Met
            260                 265                 270
Thr Met Asp Ile Ala Met Gly Gly Ser Thr Asn Thr Ile Leu His Leu
            275                 280                 285
Leu Ala Val Ala Asn Glu Ala Gly Val Asp Phe Lys Met Ala Asp Ile
    290                 295                 300
Asp Arg Leu Ser Arg Val Val Pro Cys Ile Cys Lys Thr Ala Pro Asn
305                 310                 315                 320
Asn His Asp Tyr Tyr Met Glu Asp Val His Arg Ala Gly Gly Ile Phe
                325                 330                 335
Ala Ile Leu Lys Glu Leu Asp Lys Ala Gly Lys Leu His Thr Asp Val
            340                 345                 350
His Thr Ile His Ala Pro Thr Leu Lys Asp Ala Ile Glu Gln Trp Asp
            355                 360                 365
Val Thr Asn Pro Glu Asn Thr Arg Ala Ile Glu Arg Phe Lys Ala Ala
    370                 375                 380
Pro Gly Gly Val Arg Thr Thr Gln Ala Phe Ser Gln Asn Arg Met Trp
385                 390                 395                 400
Lys Thr Leu Asp Leu Asp Arg Glu Lys Gly Cys Ile Arg Asp Val Ala
                405                 410                 415
His Ala Tyr Ser Gln Asp Gly Gly Leu Ala Val Leu Phe Gly Asn Ile
            420                 425                 430
Ala Glu Arg Gly Cys Val Val Lys Thr Ala Gly Val Asp Glu Ser Ile
        435                 440                 445
Leu Lys Phe Thr Gly Arg Ala Arg Val Phe Glu Ser Gln Glu Asp Ala
    450                 455                 460
Val Glu Gly Ile Leu Gly Asn Gln Ile Val Ala Gly Asp Ile Val Ile
465                 470                 475                 480
```

```
Ile Arg Tyr Glu Gly Pro Lys Gly Gly Pro Gly Met Gln Glu Met Leu
                485                 490                 495
Tyr Pro Thr Ser Tyr Leu Lys Ser Lys Gly Leu Gly Lys Ala Cys Ala
            500             505             510
Leu Leu Thr Asp Gly Arg Phe Ser Gly Gly Thr Ser Gly Leu Ser Ile
        515             520             525
Gly His Ala Ser Pro Glu Ala Ala Glu Gly Gly Ala Ile Gly Leu Val
    530             535             540
His Glu Gly Asp Thr Val Glu Ile Asp Ile Pro Asn Arg Ser Ile His
545             550             555             560
Leu Ala Ile Ser Asp Glu Glu Leu Ala Ala Arg Arg Ala Glu Met Glu
            565             570             575
Ala Arg Gly Ser Lys Ala Trp Lys Pro Lys Asn Arg Asp Arg Tyr Val
        580             585             590
Ser Ala Ala Leu Arg Ala Tyr Gly Ala Met Ala Thr Ser Ala Asp Lys
        595             600             605
Gly Ala Val Arg Asp Val Ala Gln Ile Glu Arg
    610             615
```

<210> 112
<211> 487
<212> PRT
<213> Neisseria meningitidis

<400> 112

```
Met Arg Ile Val Glu Lys Ala Tyr Thr Phe Asp Asp Val Leu Leu Val
1               5                   10              15

Pro Ala His Ser Thr Val Leu Pro Arg Asp Val Lys Leu Gln Thr Lys
            20              25              30

Leu Thr Arg Glu Ile Thr Leu Asn Leu Pro Leu Leu Ser Ala Ala Met
        35              40              45

Asp Thr Val Thr Glu Ala Arg Leu Ala Ile Ser Met Ala Gln Glu Gly
    50              55              60

Gly Ile Gly Ile Ile His Lys Asn Met Pro Pro Glu Met Gln Ala Arg
65              70              75              80

Ala Val Ser Lys Val Lys Arg His Glu Ser Gly Val Val Lys Asp Pro
            85              90              95

Val Thr Val Ala Pro Thr Thr Leu Ile Arg Glu Val Leu Glu Met Arg
            100             105             110

Ala Gln Arg Lys Arg Lys Met Ser Gly Leu Pro Val Val Glu Asn Gly
        115             120             125

Lys Val Val Gly Ile Val Thr Asn Arg Asp Leu Arg Phe Glu Asn Arg
    130             135             140

Val Asp Leu Pro Val Ser Ala Ile Met Thr Pro Arg Glu Arg Leu Val
145             150             155             160

Thr Val Pro Glu Gly Thr Ser Ile Asp Glu Ala Arg Glu Leu Met His
            165             170             175

Thr His Lys Val Glu Arg Val Leu Val Leu Asn Glu Lys Asp Glu Leu
        180             185             190

Lys Gly Leu Ile Thr Val Lys Asp Ile Leu Lys Thr Thr Glu Phe Pro
```

EP 2 279 746 B1

|  | 195 | 200 | 205 |

Asn Ala Asn Lys Asp Ser Glu Gly Arg Leu Arg Val Gly Ala Ala Val
    210            215            220

Gly Thr Gly Gly Asp Thr Glu Glu Arg Val Lys Ala Leu Val Glu Ala
225            230            235            240

Gly Val Asp Val Ile Val Val Asp Thr Ala His Gly His Ser Gln Gly
            245            250            255

Val Ile Asp Arg Val Arg Trp Val Lys Glu Thr Tyr Pro His Ile Gln
        260            265            270

Val Ile Gly Gly Asn Ile Ala Thr Ala Lys Ala Ala Leu Asp Leu Val
        275            280            285

Ala Ala Gly Ala Asp Ala Val Lys Val Gly Ile Gly Pro Gly Ser Ile
    290            295            300

Cys Thr Thr Arg Ile Val Ala Gly Val Gly Val Pro Gln Leu Thr Ala
305            310            315            320

Ile His Asn Val Ala Glu Ala Leu Lys Gly Thr Gly Val Pro Leu Ile
            325            330            335

Ala Asp Gly Gly Ile Arg Phe Ser Gly Asp Ile Ala Lys Ala Leu Ala
        340            345            350

Ala Gly Ala Tyr Ser Val Met Leu Gly Gly Met Phe Ala Gly Thr Glu
        355            360            365

Glu Ala Pro Gly Glu Ile Glu Leu Tyr Gln Gly Arg Ser Tyr Lys Ser
370            375            380

Tyr Arg Gly Met Gly Ser Leu Gly Ala Met Ser Gln Gly Ser Ala Asp
385            390            395            400

Arg Tyr Phe Gln Asp Lys Thr Asp Ser Thr Asp Lys Tyr Val Pro Glu
            405            410            415

Gly Ile Glu Gly Arg Val Pro Tyr Lys Gly Pro Ile Val Asn Ile Ile
        420            425            430

His Gln Leu Thr Gly Gly Leu Arg Ser Ser Met Gly Tyr Leu Gly Cys
        435            440            445

Ala Asn Ile Ala Glu Met His Glu Lys Ala Glu Phe Val Glu Ile Thr
    450            455            460

Ser Ala Gly Met Ser Glu Ser His Val His Asp Val Gln Ile Thr Lys
465            470            475            480

Glu Ala Pro Asn Tyr His Arg
            485

<210> 113
<211> 157
<212> PRT
<213> Neisseria meningitidis

<400> 113

235

```
Met Lys Gly Asp Arg Leu Val Ile Arg Glu Leu Asn Lys Asn Leu Gly
1               5                   10                  15

Leu Leu Leu Val Thr Ile Asn Gln Tyr Phe Leu His Ala Arg Ile Leu
            20                  25                  30

Lys Asn Trp Gly Phe Glu Glu Leu Gly Glu His Phe Phe Lys Gln Ser
        35                  40                  45

Ile Val Glu Met Lys Ala Ala Asp Asp Leu Ile Glu Arg Ile Leu Phe
        50                  55                  60

Leu Glu Gly Leu Pro Asn Leu Gln Glu Leu Gly Lys Leu Leu Ile Gly
65                  70                  75                  80

Glu Ser Thr Glu Glu Ile Ile Ala Cys Asp Leu Thr Lys Glu Gln Glu
                85                  90                  95

Lys His Glu Ala Leu Leu Ala Ala Ile Ala Thr Ala Glu Ala Gln Gln
                100                 105                 110

Asp Tyr Val Ser Arg Asp Leu Leu Glu Lys Gln Lys Asp Thr Asn Glu
        115                 120                 125

Glu His Ile Asp Trp Leu Glu Thr Gln Gln Glu Leu Ile Gly Lys Ile
        130                 135                 140

Gly Leu Pro Asn Tyr Leu Gln Thr Ala Ala Gln Glu Asp
145                 150                 155
```

<210> 114
<211> 435
<212> PRT
<213> Neisseria meningitidis

<400> 114

```
Met Lys Pro Val Asn Ile Gly Leu Leu Gly Leu Gly Thr Val Gly Gly
1               5               10              15

Gly Thr Ala Ala Val Leu Arg Asp Asn Ala Glu Glu Ile Ser Arg Arg
            20              25              30

Leu Gly Arg Glu Ile Arg Ile Ser Ala Val Cys Asp Leu Ser Glu Glu
        35              40              45

Lys Ala Arg Gln Thr Cys Pro Ser Ala Ala Phe Val Lys Asp Pro Phe
    50              55              60

Glu Leu Val Ala Arg Glu Asp Val Asp Val Val Val Glu Leu Phe Gly
65              70              75              80

Gly Thr Gly Ile Ala Lys Asp Ala Val Leu Lys Ala Ile Glu Asn Gly
            85              90              95

Lys His Ile Val Thr Ala Asn Lys Lys Leu Leu Ala Glu Tyr Gly Asn
        100             105             110

Glu Ile Phe Pro Leu Ala Glu Lys Gln Asn Val Ile Val Gln Phe Glu
        115             120             125

Ala Ala Val Ala Gly Gly Ile Pro Ile Ile Lys Ala Leu Arg Glu Gly
    130             135             140

Leu Ala Ala Asn Arg Ile Lys Ser Ile Ala Gly Ile Ile Asn Gly Thr
145             150             155             160

Ser Asn Phe Ile Leu Ser Glu Met Arg Glu Lys Gly Ser Ala Phe Ala
            165             170             175

Asp Val Leu Lys Glu Ala Gln Ala Leu Gly Tyr Ala Glu Ala Asp Pro
            180             185             190

Thr Phe Asp Ile Glu Gly Asn Asp Ala Gly His Lys Ile Thr Ile Met
        195             200             205

Ser Ala Leu Ala Phe Gly Thr Pro Met Asn Phe Ser Ala Cys Tyr Leu
    210             215             220
```

```
Glu Gly Ile Ser Lys Leu Asp Ser Arg Asp Ile Lys Tyr Ala Glu Glu
225             230             235             240

Leu Gly Tyr Arg Ile Lys Leu Leu Gly Ile Thr Arg Lys Thr Gly Lys
            245             250             255

Gly Ile Glu Leu Arg Val His Pro Thr Leu Ile Pro Glu Ser Arg Leu
            260             265             270

Leu Ala Asn Val Asn Gly Val Met Asn Ala Val Arg Val Asn Ala Asp
            275             280             285

Met Val Gly Glu Thr Leu Tyr Tyr Gly Ala Gly Ala Gly Ala Leu Pro
        290             295             300

Thr Ala Ser Ala Val Val Ala Asp Ile Ile Asp Ile Ala Arg Leu Val
305             310             315             320

Glu Ala Asp Thr Ala His Arg Val Pro His Leu Ala Phe Gln Pro Ala
            325             330             335

Gln Val Gln Ala Gln Thr Ile Leu Pro Met Asp Glu Ile Thr Ser Ser
            340             345             350

Tyr Tyr Leu Arg Val Gln Ala Lys Asp Glu Pro Gly Thr Leu Gly Gln
            355             360             365

Ile Ala Ala Leu Leu Ala Gln Glu Asn Val Ser Ile Glu Ala Leu Ile
            370             375             380

Gln Lys Gly Val Ile Asp Gln Thr Thr Ala Glu Ile Val Ile Leu Thr
385             390             395             400

His Ser Thr Val Glu Lys His Ile Lys Ser Ala Ile Ala Ala Ile Glu
            405             410             415

Ala Leu Asp Cys Val Glu Lys Pro Ile Thr Met Ile Arg Met Glu Ser
            420             425             430

Leu His Asp
        435
```

<210> 115

<211> 820

<212> PRT

<213> Neisseria meningitidis

<400> 115

238

```
Met Thr Gln Lys Glu Lys His Phe Glu Glu Tyr Ala Ala Leu Ala Thr
1                5                    10                15

Leu Pro Leu Arg Asp Val Val Val Tyr Pro His Met Val Leu Pro Leu
            20                25                    30

Phe Val Gly Arg Pro Lys Ser Ile Ala Ala Leu Glu Asn Ala Ile Thr
        35                40                    45

Arg Glu Glu Pro Val Phe Leu Leu Ala Gln Thr Asp Ala Ala Val Glu
    50                55                60

Glu Pro Ile Ala Ala Asp Leu Tyr Gln Thr Gly Thr Val Ala Gln Val
65                70                75                    80

Leu Gln Val Leu Lys Leu Pro Asp Gly Thr Val Lys Val Leu Val Glu
            85                90                    95

Gly Leu Tyr Arg Gly Arg Val Leu Thr Ile Glu Asp Thr Gly Gly Leu
        100                105                110

Phe Val Ser His Ile Glu Thr Val Val Glu Glu Asp Thr Gly Gly Asn
```

```
                  115                    120                    125

     Thr Asp Leu Glu Ala Val Arg Arg Thr Leu Leu Ala Gln Phe Glu Gln
         130                    135                    140

     Tyr Ala Lys Leu Asn Lys Lys Ile Pro Ala Glu Ile Ile Gly Ser Ile
     145                    150                    155                    160

     Asn Gly Ile Ala Glu Asn Ser Arg Leu Thr Asp Thr Val Ala Ala His
                     165                    170                    175

     Leu Gln Leu Lys Leu Ala Gln Arg Gln Gln Ile Leu Glu Ile Pro Glu
                 180                    185                    190

     Ile Gly Lys Arg Met Glu Phe Leu Leu Ala Gln Leu Glu Ser Glu Leu
                 195                    200                    205

     Asp Ile Met Gln Ala Glu Lys Arg Ile Arg Gly Arg Val Lys Arg Gln
         210                    215                    220

     Met Glu Lys Ser Gln Arg Glu Tyr Tyr Leu Asn Glu Gln Ile Lys Ala
     225                    230                    235                    240

     Ile His Lys Glu Leu Gly Glu Glu Asp Glu Asn Gly Glu Leu Asp Ala
                     245                    250                    255

     Leu Glu Ala Asp Ile Lys Lys Ala Gly Met Thr Lys Glu Ala Glu Glu
                 260                    265                    270

     Lys Cys Leu Ser Glu Leu Lys Lys Leu Lys Met Met Pro Pro Met Ser
             275                    280                    285

     Ala Glu Ser Thr Val Val Arg Asn Tyr Ile Asp Thr Leu Leu Glu Leu
         290                    295                    300

     Pro Trp Lys Lys Lys Ser Arg Val Ser Lys Asp Ile Ala Lys Ala Gly
     305                    310                    315                    320

     Leu Val Leu Asp Ala Asp His Tyr Gly Leu Glu Lys Val Lys Glu Arg
                     325                    330                    335

     Ile Leu Glu Tyr Leu Ala Val Gln Lys Arg Met Asp Lys Leu Lys Gly
                 340                    345                    350

     Pro Ile Leu Cys Leu Val Gly Pro Pro Gly Val Gly Lys Thr Ser Leu
             355                    360                    365

     Gly Glu Ser Ile Ala Lys Ala Thr Gly Arg Lys Tyr Val Arg Met Ala
         370                    375                    380

     Leu Gly Gly Val Arg Asp Glu Ser Glu Ile Arg Gly His Arg Arg Thr
     385                    390                    395                    400

     Tyr Ile Gly Ser Met Pro Gly Lys Ile Leu Gln Asn Met Ala Lys Ala
                     405                    410                    415

     Gly Val Lys Asn Pro Leu Phe Leu Leu Asp Glu Ile Asp Lys Leu Gly
                 420                    425                    430

     Asn Asp Phe Arg Gly Asp Pro Ala Ser Ala Leu Leu Glu Val Leu Asp
         435                    440                    445

     Pro Glu Gln Asn Asn Lys Phe Ala Asp His Tyr Ala Glu Val Asp Tyr
         450                    455                    460

     Asp Leu Ser Asp Val Met Phe Ile Ala Thr Ser Asn Ser Leu Asn Ile
     465                    470                    475                    480

     Pro Thr Pro Leu Leu Asp Arg Met Glu Ile Ile Arg Leu Ser Gly Tyr
                     485                    490                    495
```

240

```
Thr Glu Asp Glu Lys Ile Asn Ile Ala Met Gln Tyr Leu Val Pro Lys
            500                   505                   510

Gln Met Lys Arg Asn Gly Val Lys Glu Gly Glu Leu Ala Ile Glu Glu
            515                   520                   525

Ser Ala Val Arg Asp Ile Ile Arg Tyr Tyr Thr Arg Glu Ala Gly Val
            530                   535                   540

Arg Ser Leu Asp Arg Glu Ile Ala Lys Ile Cys Arg Lys Val Val Met
545                   550                   555                   560

Gln Ile Thr Leu Asp Glu Asp Lys Lys Arg Leu Ser Glu Thr Lys Lys
            565                   570                   575

Thr Ser Lys Ala Lys Pro Lys Ala Val Lys Val Asn Glu Lys Asn Leu
            580                   585                   590

His Asp Tyr Leu Gly Val Arg Arg Phe Asp Tyr Gly Val Ala Glu Ser
            595                   600                   605

Glu Asn Arg Ile Gly Gln Val Thr Gly Leu Ala Trp Thr Glu Val Gly
            610                   615                   620

Gly Glu Leu Leu Thr Val Glu Ala Ala Ala Leu Pro Gly Lys Gly Val
625                   630                   635                   640

Ile Gln Cys Thr Gly Gln Leu Gly Asp Val Met Lys Glu Ser Val Ser
            645                   650                   655

Ala Ala Trp Ser Val Val Arg Ser Arg Ala Glu Ser Val Gly Leu Ala
            660                   665                   670

Pro Asp Phe Tyr Glu Lys Lys Asp Ile His Ile His Val Pro Glu Gly
            675                   680                   685

Ala Thr Pro Lys Asp Gly Pro Ser Ala Gly Ile Ala Met Thr Leu Ala
            690                   695                   700

Ala Val Ser Ala Phe Thr Lys Ile Pro Val Arg Ala Asp Val Ala Met
705                   710                   715                   720

Thr Gly Glu Ile Thr Leu Arg Gly Glu Val Leu Pro Ile Gly Gly Leu
            725                   730                   735

Lys Glu Lys Leu Leu Ala Ala Leu Arg Gly Gly Ile Lys His Val Leu
            740                   745                   750

Ile Pro Lys Asp Asn Val Lys Asp Leu Glu Glu Ile Pro Glu Asn Val
            755                   760                   765

Lys Thr Gly Leu Thr Ile His Pro Val Lys Trp Ile Asp Glu Val Leu
            770                   775                   780

Ala Leu Gly Leu Glu Ser Gln Pro Glu Pro Trp Ala Glu Pro Ser Gly
785                   790                   795                   800

Ala Glu Ala Ala Ala Glu Ser Ala Ser Lys Pro Lys Pro Arg Ser Arg
            805                   810                   815

Ala Thr Lys His
            820
```

<210> 116

<211> 542

<212> PRT

<213> Neisseria meningitidis

<400> 116

EP 2 279 746 B1

```
Met Ile Ser Thr Asn Gly Ile Thr Met Gln Phe Gly Ala Lys Pro Leu
1               5               10              15

Phe Glu Asn Val Ser Val Lys Phe Gly Glu Gly Asn Arg Tyr Gly Leu
            20              25              30

Ile Gly Ala Asn Gly Ser Gly Lys Ser Thr Phe Met Lys Ile Leu Gly
        35              40              45

Gly Asp Leu Glu Gln Thr Ala Gly Glu Val Ala Ile Glu Asn Gly Val
    50              55              60

Arg Leu Gly Lys Leu Arg Gln Asp Gln Phe Ala Tyr Glu Asp Met Arg
65              70              75              80

Val Leu Asp Val Val Met Met Gly His Thr Glu Met Trp Ala Ala Met
            85              90              95

Thr Glu Arg Asp Ala Ile Tyr Ala Asn Pro Glu Ala Thr Glu Asp Asp
            100             105             110

Tyr Met Lys Ala Ala Glu Leu Glu Ala Lys Phe Ala Glu Tyr Asp Gly
        115             120             125

Tyr Thr Ala Glu Ala Arg Ala Ala Glu Leu Leu Ser Gly Val Gly Ile
    130             135             140

Ser Glu Asp Leu His Asn Ala Lys Met Ala Glu Val Ala Pro Gly Phe
145             150             155             160

Lys Leu Arg Val Leu Leu Ala Gln Ala Leu Phe Ser Lys Pro Asp Val
            165             170             175

Leu Leu Leu Asp Glu Pro Thr Asn Asn Leu Asp Ile Asn Thr Ile Arg
            180             185             190

Trp Leu Glu Gly Val Leu Asn Gln Tyr Asp Ser Thr Met Ile Ile Ile
        195             200             205

Ser His Asp Arg His Phe Leu Asn Glu Val Cys Thr His Met Ala Asp
    210             215             220

Leu Asp Tyr Asn Thr Ile Thr Ile Tyr Pro Gly Asn Tyr Asp Asp Tyr
225             230             235             240

Met Leu Ala Ser Ala Gln Ser Arg Glu Arg Ala Leu Lys Asp Asn Ala
            245             250             255

Lys Ala Lys Glu Lys Leu Gln Glu Leu Gln Glu Phe Val Ala Arg Phe
        260             265             270

Ser Ala Asn Lys Ser Lys Ala Arg Gln Ala Thr Ser Arg Leu Lys Gln
        275             280             285

Ala Asp Lys Ile Lys Ser Glu Met Val Glu Val Lys Pro Ser Thr Arg
    290             295             300

Gln Asn Pro Tyr Ile Arg Phe Glu Ala Asp Glu Lys Ala Lys Leu His
305             310             315             320

Arg Gln Ala Val Glu Val Glu Lys Leu Ala Lys Arg Phe Glu Thr Gln
            325             330             335

Leu Phe Lys Asn Leu Asn Phe Ile Leu Glu Ala Gly Gln Arg Leu Ala
        340             345             350

Ile Ile Gly Pro Asn Gly Ala Gly Lys Ser Thr Leu Leu Lys Leu Leu
    355             360             365

Ala Gly Ala Tyr Asn Pro Glu Tyr Ser Asp Gly Leu Leu Pro Asp Glu
```

243

|  | 370 | | | | | 375 | | | | | 380 | | | |
|---|---|---|---|---|---|---|---|---|---|---|---|---|---|---|

Gly Thr Ile Lys Trp Ala Glu Lys Ala Ser Val Gly Tyr Tyr Pro Gln
385                     390                     395                     400

Asp His Glu Asn Asp Phe Asp Val Asp Met Asp Leu Ser Glu Trp Met
                    405                     410                     415

Arg Gln Trp Gly Gln Glu Gly Asp Asp Glu Gln Val Ile Arg Gly Thr
              420                     425                     430

Leu Gly Arg Leu Leu Phe Gly Ser Asn Asp Val Val Lys Lys Val Lys
          435                     440                     445

Val Leu Ser Gly Gly Glu Lys Gly Arg Met Leu Tyr Gly Lys Leu Leu
          450                     455                     460

Leu Leu Lys Pro Asn Val Leu Val Met Asp Glu Pro Thr Asn His Met
465                     470                     475                     480

Asp Met Glu Ser Ile Glu Ser Leu Asn Met Ala Leu Glu Lys Tyr Asn
                485                     490                     495

Gly Thr Leu Ile Phe Val Ser His Asp Arg Gln Phe Val Ser Ser Leu
          500                     505                     510

Ala Thr Gln Ile Ile Glu Leu Asp Gly Lys Gly Gly Tyr Glu His Tyr
          515                     520                     525

Leu Gly Asp Tyr Glu Ser Tyr Leu Glu Lys Lys Gly Val Ala
          530                     535                     540

<210> 117
<211> 344
<212> PRT
<213> Neisseria meningitidis

<400> 117

```
Met Ser Thr Ser Leu Ser Tyr Arg Asp Ala Gly Val Asp Ile Asp Ala
1               5               10              15

Gly Asp Gln Leu Val Glu Asn Ile Lys Pro Phe Ala Lys Arg Thr Met
            20              25              30

Arg Pro Glu Val Leu Gly Asp Leu Gly Gly Phe Gly Ala Leu Val Glu
        35              40              45

Ile Gly Lys Lys Tyr Gln Asn Pro Val Leu Val Ser Gly Thr Asp Gly
    50              55              60

Val Gly Thr Lys Leu Lys Leu Ala Phe Asp Trp Asp Lys His Asp Thr
65              70              75              80

Val Gly Ile Asp Leu Val Ala Met Ser Val Asn Asp Ile Leu Val Gln
            85              90              95

Gly Ala Glu Pro Leu Phe Phe Leu Asp Tyr Phe Ala Cys Gly Lys Leu
            100             105             110

Asp Val Pro Arg Ala Thr Asp Val Ile Lys Gly Ile Ala Gln Gly Cys
        115             120             125

Glu Glu Ser Gly Cys Ala Leu Ile Gly Gly Glu Thr Ala Glu Met Pro
    130             135             140

Gly Met Tyr Pro Val Gly Glu Tyr Asp Leu Ala Gly Phe Ala Val Gly
145             150             155             160

Val Val Glu Lys Glu Asn Val Ile Thr Gly Arg Ser Ile Gly Val Gly
            165             170             175

Asp Val Val Leu Gly Leu Ala Ser Asn Gly Ala His Ser Asn Gly Tyr
            180             185             190

Ser Leu Ile Arg Lys Ile Ile Glu Arg Asp Asn Pro Asp Leu Asp Ala
        195             200             205

Glu Phe Asp Asn Gly Lys Thr Leu Arg Glu Ala Val Ile Ala Pro Thr
    210             215             220

Arg Leu Tyr Val Lys Pro Ile Leu Ala Ala Leu Glu Lys Phe Thr Ile
225             230             235             240

Lys Gly Met Ala His Ile Thr Gly Gly Gly Ile Thr Glu Asn Val Pro
            245             250             255

Arg Val Leu Pro Glu Asn Thr Val Ala Gln Ile Asp Ala Lys Ser Trp
        260             265             270

Glu Leu Pro Lys Leu Phe Gln Trp Leu Gln Lys Ala Gly Asn Val Glu
        275             280             285

Thr Gln Glu Met Tyr Arg Thr Phe Asn Cys Gly Ile Gly Met Val Val
    290             295             300

Ile Val Ala Ala Glu Asp Ala Asp Ala Val Gln Gly Leu Leu Gly Glu
305             310             315             320

Gln Gly Glu Thr Val Tyr Arg Leu Gly Leu Ile Arg Glu Arg Gln Gly
            325             330             335

Asp Glu His Gln Thr Gln Val Ala
            340
```

<210> 118

<211> 561
<212> PRT
<213> Neisseria meningitidis

<400> 118

```
Met Ser Met Glu Asn Phe Ala Gln Leu Leu Glu Glu Ser Phe Thr Leu
1               5               10              15

Gln Glu Met Asn Pro Gly Glu Val Ile Thr Ala Glu Val Val Ala Ile
        20              25              30

Asp Gln Asn Phe Val Thr Val Asn Ala Gly Leu Lys Ser Glu Ser Leu
        35              40              45

Ile Asp Val Ala Glu Phe Lys Asn Ala Gln Gly Glu Ile Glu Val Lys
    50              55              60

Val Gly Asp Phe Val Thr Val Thr Ile Glu Ser Val Glu Asn Gly Phe
65              70              75              80

Gly Glu Thr Lys Leu Ser Arg Glu Lys Ala Lys Arg Ala Ala Asp Trp
                85              90              95

Ile Ala Leu Glu Glu Ala Met Glu Asn Gly Asp Ile Leu Ser Gly Ile
            100             105             110

Ile Asn Gly Lys Val Lys Gly Gly Leu Thr Val Met Ile Ser Ser Ile
        115             120             125

Arg Ala Phe Leu Pro Gly Ser Leu Val Asp Val Arg Pro Val Lys Asp
    130             135             140

Thr Ser His Phe Glu Gly Lys Glu Ile Glu Phe Lys Val Ile Lys Leu
145             150             155             160
```

```
Asp Lys Lys Arg Asn Asn Val Val Val Ser Arg Arg Ala Val Leu Glu
            165             170             175

Ala Thr Leu Gly Glu Glu Arg Lys Ala Leu Leu Glu Asn Leu Gln Glu
            180             185             190

Gly Ser Val Ile Lys Gly Ile Val Lys Asn Ile Thr Asp Tyr Gly Ala
            195             200             205

Phe Val Asp Leu Gly Gly Ile Asp Gly Leu Leu His Ile Thr Asp Leu
    210             215             220

Ala Trp Arg Arg Val Lys His Pro Ser Glu Val Leu Glu Val Gly Gln
225             230             235             240

Glu Val Glu Ala Lys Val Leu Lys Phe Asp Gln Glu Lys Gln Arg Val
            245             250             255

Ser Leu Gly Met Lys Gln Leu Gly Glu Asp Pro Trp Ser Gly Leu Thr
            260             265             270

Arg Arg Tyr Pro Gln Gly Thr Arg Leu Phe Gly Lys Val Ser Asn Leu
            275             280             285

Thr Asp Tyr Gly Ala Phe Val Glu Ile Glu Gln Gly Ile Glu Gly Leu
    290             295             300

Val His Val Ser Glu Met Asp Trp Thr Asn Lys Asn Val His Pro Ser
305             310             315             320

Lys Val Val Gln Leu Gly Asp Glu Val Glu Val Met Ile Leu Glu Ile
            325             330             335

Asp Glu Gly Arg Arg Arg Ile Ser Leu Gly Met Lys Gln Cys Gln Ala
            340             345             350

Asn Pro Trp Glu Glu Phe Ala Ala Asn His Asn Lys Gly Asp Lys Ile
            355             360             365

Ser Gly Ala Val Lys Ser Ile Thr Asp Phe Gly Val Phe Val Gly Leu
            370             375             380

Pro Gly Gly Ile Asp Gly Leu Val His Leu Ser Asp Leu Ser Trp Thr
385             390             395             400

Glu Ser Gly Glu Glu Ala Val Arg Lys Tyr Lys Lys Gly Glu Glu Val
            405             410             415

Glu Ala Val Val Leu Ala Ile Asp Val Glu Lys Glu Arg Ile Ser Leu
            420             425             430

Gly Ile Lys Gln Leu Glu Gly Asp Pro Phe Gly Asn Phe Ile Ser Val
            435             440             445

Asn Asp Lys Gly Ser Leu Val Lys Gly Ser Val Lys Ser Val Asp Ala
    450             455             460

Lys Gly Ala Val Ile Ala Leu Ser Asp Glu Val Glu Gly Tyr Leu Pro
465             470             475             480

Ala Ser Glu Phe Ala Ala Asp Arg Val Glu Asp Leu Thr Thr Lys Leu
            485             490             495

Lys Glu Gly Asp Glu Val Glu Ala Val Ile Val Thr Val Asp Arg Lys
            500             505             510

Asn Arg Ser Ile Lys Leu Ser Val Lys Ala Lys Asp Ala Lys Glu Ser
    515             520             525

Arg Glu Ala Leu Asn Ser Val Asn Ala Ala Ala Asn Ala Asn Ala Gly
```

530                     535                     540

Thr Thr Ser Leu Gly Asp Leu Leu Lys Ala Lys Leu Ser Gly Glu Gln
545                     550                     555                     560

Glu

<210> 119
<211> 104
<212> PRT
<213> Neisseria meningitidis

<400> 119

Met Thr Lys Ser Glu Leu Met Val Arg Leu Ala Glu Val Phe Ala Ala
1                   5                       10                      15

Lys Asn Gly Thr His Leu Leu Ala Lys Asp Val Glu Tyr Ser Val Lys
            20                      25                      30

Val Leu Val Asp Thr Met Thr Arg Ser Leu Ala Arg Gly Gln Arg Ile
            35                      40                      45

Glu Ile Arg Gly Phe Gly Ser Phe Asp Leu Asn His Arg Pro Ala Arg
    50                      55                      60

Ile Gly Arg Asn Pro Lys Thr Gly Glu Arg Val Glu Val Pro Glu Lys
65                      70                      75                      80

His Val Pro His Phe Lys Pro Gly Lys Glu Leu Arg Glu Arg Val Asp
                85                      90                      95

Leu Ala Leu Lys Glu Asn Ala Asn
                100

<210> 120
<211> 437
<212> PRT
<213> Neisseria meningitidis

<400> 120

```
Met Met Ser Val Thr Val Glu Thr Leu Glu Asn Leu Glu Arg Lys Val
1               5               10                  15

Val Leu Ser Leu Pro Trp Ser Glu Ile Asn Ala Glu Thr Asp Lys Lys
            20              25                  30

Leu Lys Gln Thr Gln Arg Arg Ala Lys Ile Asp Gly Phe Arg Pro Gly
        35              40                  45

Lys Ala Pro Leu Lys Met Ile Ala Gln Met Tyr Gly Ala Ser Ala Gln
    50              55                  60

Asn Asp Val Ile Asn Glu Leu Val Gln Arg Arg Phe Tyr Asp Val Ala
65              70              75                  80

Val Ala Gln Glu Leu Lys Val Ala Gly Phe Pro Arg Phe Glu Gly Val
            85              90                  95

Glu Glu Gln Asp Asp Lys Glu Ser Phe Lys Val Ala Ala Ile Phe Glu
        100             105                 110

Val Phe Pro Glu Val Val Ile Gly Asp Leu Ser Ala Gln Glu Val Glu
        115             120                 125

Lys Val Thr Ala Ser Val Gly Asp Ala Glu Val Asp Gln Thr Val Glu
    130             135                 140

Ile Leu Arg Lys Gln Arg Thr Arg Phe Asn His Val Glu Arg Glu Ala
145             150                 155                 160
```

```
Arg Asn Gly Asp Arg Val Ile Ile Asp Phe Glu Gly Lys Ile Asp Gly
                165             170             175

Glu Pro Phe Ala Gly Gly Ala Ser Lys Asn Tyr Ala Phe Val Leu Gly
            180             185             190

Ala Ser Gln Met Leu Pro Glu Phe Glu Ala Gly Val Val Gly Met Lys
        195             200             205

Ala Gly Glu Ser Lys Asp Val Thr Val Asn Phe Pro Glu Asp Tyr His
    210             215             220

Gly Lys Asp Val Ala Gly Lys Thr Ala Val Phe Thr Ile Thr Leu Asn
225             230             235             240

Asn Val Ser Glu Ala Thr Leu Pro Glu Val Asp Ala Asp Phe Ala Lys
                245             250             255

Ala Leu Gly Ile Ala Asp Gly Asp Val Ala Lys Met Arg Glu Glu Val
            260             265             270

Gln Lys Asn Val Ser Arg Glu Val Glu Arg Arg Val Asn Glu Gln Thr
        275             280             285

Lys Glu Ser Val Met Asn Ala Leu Leu Lys Ala Val Glu Leu Lys Ala
    290             295             300

Pro Val Ala Leu Val Asn Glu Glu Ala Ala Arg Leu Ala Asn Glu Met
305             310             315             320

Lys Gln Asn Phe Val Asn Gln Gly Met Ala Asp Ala Ala Asn Leu Asp
                325             330             335

Leu Pro Leu Asp Met Phe Lys Glu Gln Ala Glu Arg Arg Val Ser Leu
            340             345             350

Gly Leu Ile Leu Ala Lys Leu Val Asp Glu Asn Lys Leu Glu Pro Thr
        355             360             365

Glu Glu Gln Ile Lys Ala Val Val Ala Asn Phe Ala Glu Ser Tyr Glu
    370             375             380

Asp Pro Gln Glu Val Ile Asp Trp Tyr Tyr Ala Asp Pro Ser Arg Leu
385             390             395             400

Gln Ala Pro Thr Ser Leu Ala Val Glu Ser Asn Val Val Asp Phe Val
            405             410             415

Leu Gly Lys Ala Lys Val Asn Glu Lys Ala Leu Ser Phe Asp Glu Val
        420             425             430

Met Gly Ala Gln Ala
        435
```

<210> 121
<211> 150
<212> PRT
<213> Neisseria meningitidis

<400> 121

EP 2 279 746 B1

```
Met Gln Ile Ile Leu Leu Glu Lys Ile Gly Gly Leu Gly Asn Leu Gly
1               5               10              15

Asp Ile Val Thr Val Lys Asn Gly Tyr Ala Arg Asn Phe Leu Ile Pro
            20              25              30

Ala Gly Lys Ala Lys Arg Ala Thr Glu Ala Asn Met Lys Glu Phe Glu
        35              40              45


Ala Arg Arg Ala Glu Leu Glu Ala Lys Gln Ala Glu Ile Leu Ala Asp
    50              55              60

Ala Arg Val Arg Gln Glu Lys Leu Asp Gly Gln Thr Val Thr Val Ala
65              70              75              80

Gln Lys Ala Gly Val Asp Gly Arg Leu Phe Gly Ser Val Thr Asn Ala
                85              90              95

Asp Ile Ala Ala Ala Ile Val Ala Ala Gly Ile Glu Ala Val Lys Ala
            100             105             110

Asn Val Arg Leu Pro Asn Gly Pro Leu Lys Ala Val Gly Glu Tyr Glu
        115             120             125

Val Glu Val Ala Leu His Thr Asp Ala Val Ala Lys Ile Thr Val Ala
    130             135             140

Val Val Ala Ala Thr Glu
145             150
```

<210> 122
<211> 122
<212> PRT
<213> Neisseria meningitidis

<400> 122

```
Met Arg His Tyr Glu Ile Val Phe Ile Val His Pro Asp Gln Ser Glu
1               5               10              15

Gln Val Pro Ala Met Val Glu Arg Tyr Lys Thr Met Ile Ala Glu Ala
            20              25              30

Asn Gly Lys Ile His Arg Leu Glu Asp Trp Gly Arg Arg Gln Leu Ala
        35              40              45

Tyr Pro Ile Asn Lys Ile His Lys Ala His Tyr Val Leu Met Asn Ile
    50              55              60

Glu Thr Thr Pro Glu Val Val Glu Glu Leu Glu Thr Ala Phe Arg Phe
65              70              75              80

Asn Asp Ala Ile Leu Arg His Leu Thr Ile Lys Thr Lys His Ala Val
            85              90              95

Thr Glu Ala Ser Pro Met Leu Gly Gly Glu Lys Ala Lys Asn Leu Leu
            100             105             110

Ser Gly Ala Ser Glu Glu Ala Val Ala Gln
        115             120
```

<210> 123
<211> 316

251

EP 2 279 746 B1

<212> PRT
<213> Neisseria meningitidis

<400> 123

Met Ser Gln His Arg Lys Leu Ile Ile Leu Gly Ser Gly Pro Ala Gly
1               5                   10                  15

Tyr Thr Ala Ala Val Tyr Ala Ala Arg Ala Asn Leu Asn Pro Val Ile
            20                  25                  30

Ile Thr Gly Ile Ala Gln Gly Gly Gln Leu Met Thr Thr Thr Glu Val
        35                  40                  45

Asp Asn Trp Pro Ala Asp Ala Asp Gly Val Gln Gly Pro Glu Leu Met
        50                  55                  60

Ala Arg Phe Leu Ala His Ala Glu Arg Phe Gly Thr Glu Ile Ile Phe

65                  70                  75                  80

Asp Gln Ile Asn Ala Val Asp Leu Gln Lys Arg Pro Phe Thr Leu Lys
                85                  90                  95

Gly Asp Met Gly Glu Tyr Thr Cys Asp Ala Leu Ile Val Ala Thr Gly
            100                 105                 110

Ala Ser Ala Lys Tyr Leu Gly Leu Pro Ser Glu Glu Ala Phe Ala Gly
        115                 120                 125

Lys Gly Val Ser Ala Cys Ala Thr Cys Asp Gly Phe Phe Tyr Lys Asn
    130                 135                 140

Gln Asp Val Ala Val Val Gly Gly Gly Asn Thr Ala Val Glu Glu Ala
145                 150                 155                 160

Leu Tyr Leu Ala Asn Ile Ala Lys Thr Val Thr Leu Ile His Arg Arg
                165                 170                 175

Ser Glu Phe Arg Ala Glu Lys Ile Met Ile Asp Lys Leu Met Lys Arg
            180                 185                 190

Val Glu Glu Gly Lys Ile Ile Leu Lys Leu Glu Ser Asn Leu Gln Glu
            195                 200                 205

Val Leu Gly Asp Asp Arg Gly Val Asn Gly Ala Leu Leu Lys Asn Asn
    210                 215                 220

Asp Gly Ser Glu Gln Gln Ile Ala Val Ser Gly Ile Phe Ile Ala Ile
225                 230                 235                 240

Gly His Lys Pro Asn Thr Asp Ile Phe Lys Gly Gln Leu Glu Met Asp
                245                 250                 255

Glu Ala Gly Tyr Leu Lys Thr Lys Gly Gly Thr Ala Asp Asn Val Gly
            260                 265                 270

Ala Thr Asn Ile Glu Gly Val Trp Ala Ala Gly Asp Val Lys Asp His
            275                 280                 285

Thr Tyr Arg Gln Ala Ile Thr Ser Ala Ala Ser Gly Cys Gln Ala Ala
        290                 295                 300

Leu Asp Ala Glu Arg Trp Leu Gly Ser Gln Asn Ile
305                 310                 315

252

<210> 124
<211> 238
<212> PRT
<213> Neisseria meningitidis

<400> 124

```
Met Thr Asp Thr Ala Glu Asn Gln Thr Gln Asn Asn Trp Gln Ala Gly
1               5                   10                  15

His Pro Arg Ser Ile Arg Ser Phe Val Leu Arg Gln Ser His Met Thr
            20                  25                  30

Ala Ala Gln Gln Arg Ala Ile Asp Thr Leu Trp Asp Ser Phe Gly Ile
        35                  40                  45

Asp Tyr Gln Ala Thr Pro Ala Asp Leu Asp Ala Arg Phe Gly Ser Ser
    50                  55                  60

Arg Pro Lys Ile Leu Glu Ile Gly Phe Gly Met Gly Thr Ala Thr Ala
65                  70                  75                  80

Glu Ile Ala Arg Arg Leu Pro Glu Thr Asp Phe Leu Ala Ile Asp Val
                85                  90                  95

His Gly Pro Gly Val Gly Asn Leu Leu Lys Leu Ile Asp Glu Asn His
            100                 105                 110

Leu Glu Asn Ile Arg Val Met Arg His Asp Ala Val Glu Val Val Glu
            115                 120                 125

Asn Met Leu Gln Asp Gly Ser Leu Asp Gly Ile His Ile Phe Phe Pro
    130                 135                 140

Asp Pro Trp His Lys Lys Arg His His Lys Arg Arg Leu Ile Gln Ala
145                 150                 155                 160

Pro Phe Ile Ala Lys Leu Leu Pro Lys Leu Lys Thr Gly Gly Tyr Ile
                165                 170                 175

His Leu Ala Thr Asp Trp Glu Glu Tyr Ala Gln Gln Met Leu Glu Val
            180                 185                 190

Leu Ser Ser Phe Asp Ser Leu Gln Asn Thr Ala Ala Asp Tyr Ala Pro
            195                 200                 205

Thr Pro Asp Tyr Arg Pro Glu Thr Lys Phe Glu Ala Arg Gly Lys Arg
210                 215                 220

Leu Gly His Gly Val Trp Asp Leu Val Phe Lys Arg Ile Gly
225                 230                 235
```

<210> 125
<211> 570
<212> PRT
<213> Neisseria meningitidis

<400> 125

```
Met Lys Ala Ser Gln Phe Phe Ile Ser Thr Leu Lys Glu Ala Pro Ala
1               5               10              15

Glu Ala Ala Leu Ala Ser His Lys Leu Met Ile Arg Ala Gly Leu Ile
            20              25              30

Lys Ala Asn Ala Ser Gly Leu Tyr Thr Trp Met Pro Met Gly Leu Arg
        35              40              45

Val Leu Arg Lys Val Glu Asn Val Val Arg Glu Glu Met Ala Arg Ala
    50              55              60

Gly Ser Val Glu Leu Leu Met Pro Val Val Gln Pro Ala Glu Leu Trp
65              70              75              80

Gln Glu Ser Gly Arg Trp Glu Phe Tyr Gly Lys Glu Leu Leu Arg Leu
            85              90              95

Lys Asp Arg His Asp Arg Asp Phe Cys Met Gly Pro Thr Cys Glu Glu
        100             105             110

Val Ile Ala Asp Ile Val Arg Lys Glu Ile Asn Ser Tyr Lys Gln Leu
    115             120             125

Pro Lys Asn Phe Tyr His Ile Gln Thr Lys Phe Arg Asp Glu Val Arg
    130             135             140

Pro Arg Phe Gly Val Met Arg Ala Arg Glu Phe Val Met Lys Asp Ala
145             150             155             160

Tyr Ser Phe His Ala Asp Tyr Ala Ser Leu Gln Thr Thr Tyr Gln Asp
            165             170             175

Met Tyr Asp Ala Tyr Cys Arg Ile Phe Thr Arg Leu Gly Leu Ala Phe
            180             185             190
```

Arg Pro Val Ala Ala Asp Thr Gly Ser Ile Gly Gly Thr Gly Ser His
    195                200             205

Glu Phe Gln Val Leu Ala Glu Ser Gly Glu Asp Val Ile Ala Tyr Ser
    210               215             220

Asp Thr Ser Asp Tyr Ala Ala Asn Ile Glu Leu Ala Pro Thr Leu Pro
225            230          235            240

Leu Lys Gly Glu Arg Ala Ala Ala Gln Ala Glu Leu Val Lys Val His
          245          250            255

Thr Pro Asn Val Lys Thr Ile Asp Ser Leu Val Asp Phe Leu Ser Ile
          260          265          270

Pro Ile Glu Lys Thr Leu Lys Ser Ile Val Val Glu Gly Glu Asn Glu
          275          280          285

Gly Glu Leu Ile Leu Leu Leu Leu Arg Gly Asp His Glu Phe Asn Asp
    290              295          300

Ile Lys Ala Glu Lys Leu Ala Gly Val Lys Ser Pro Leu Thr Met Ala
305            310          315            320

Ser Pro Ala Ala Ile Val Glu Gln Phe Gly Ala Asn Gly Gly Ser Leu
          325          330          335

Gly Pro Val Gly Phe Ala Gly Lys Val Tyr Ala Asp Phe Ala Thr Glu
          340          345          350

Lys Gly Ala Asp Trp Val Ile Gly Ala Asn Glu Asp Asp Tyr His Tyr
          355          360          365

Thr Gly Phe Asn Phe Gly Arg Asp Ala Ala Glu Pro Glu Phe Val Asp
      370            375          380

Leu Arg Asn Val Val Glu Gly Asp Glu Ser Pro Asp Gly Gln Gly Arg
385            390          395            400

Leu Lys Leu Ala Arg Gly Ile Glu Val Gly His Val Phe Gln Leu Arg
          405          410          415

Asp Lys Tyr Thr Gln Ala Met Asn Val Ser Phe Leu Asp Asn Asn Gly
          420          425          430

Lys Ser Gln Ile Met Glu Met Gly Cys Tyr Gly Ile Gly Ile Thr Arg
          435          440          445

Val Val Ala Ala Ala Ile Glu Gln Asn Asn Asp Glu Lys Gly Ile Ile
      450            455          460

Trp Thr Lys Ala Met Ala Pro Phe Glu Val Val Ile Val Pro Met Asn
465            470          475            480

Tyr Lys Lys Ser Asp Thr Val Arg Glu Ala Ala Asp Lys Ile Tyr Ala
          485          490          495

Glu Leu Leu Ala Ala Gly Ala Asp Val Leu Leu Asp Asp Arg Asp Glu
          500          505          510

Arg Ala Gly Val Leu Leu Asn Asp Ser Glu Leu Leu Gly Ile Pro His
          515          520          525

Arg Ile Val Ile Gly Asp Arg Ala Leu Lys Glu Gly Asn Val Glu Tyr
      530            535          540

Ala Glu Arg Arg Asp Asn Glu Ala Gln Ala Val Ala Ile Gly Glu Ile
545            550          555          560

Val Ala Arg Val Thr Ala Ser Leu Asn Ala

565                                    570

<210> 126
<211> 887
<212> PRT
<213> Neisseria meningitidis

<400> 126

```
Met Ser Thr Gln Leu His Asp Val Asp Pro Ile Glu Thr Gln Glu Trp
1             5               10                  15

Leu Asp Ala Leu Ser Ser Val Leu Glu Tyr Glu Gly Gly Glu Arg Ala
            20              25              30

Gln Tyr Leu Leu Glu Asn Leu Val Lys Tyr Cys Arg Asp Lys Gly Val
        35              40              45

Arg Met Pro His Gly Thr Thr Thr Pro Tyr Leu Asn Thr Val Ser Val
    50              55              60

Glu Asn Glu Lys Gly Ile Pro Gly Asp Gln Asn Ile Glu His Arg Ile
65              70              75              80

Arg Ala Phe Val Arg Trp Asn Ala Ala Ala Ile Val Leu Arg Ala Gly
            85              90              95

Lys Lys Asp Leu Glu Leu Gly Gly His Ile Ala Ser Phe Gln Ser Ala
        100             105             110

Ala Thr Met Tyr Glu Val Gly Phe Asn His Phe Trp Lys Ala Lys Gly
        115             120             125

Glu Gly Glu Glu Gly Asp Leu Val Phe Phe Gln Gly His Val Ala Pro
    130             135             140

Gly Ile Tyr Ala Arg Ala Phe Val Glu Gly Arg Leu Thr Glu Asp Gln
145             150             155             160

Leu Asn Asn Phe Arg Gln Glu Val Asp Gly His Gly Leu Pro Ser Tyr
            165             170             175

Pro His Pro His Leu Leu Pro Asp Phe Trp Gln Phe Pro Thr Val Ser
        180             185             190

Met Gly Leu Gly Pro Ile Met Ala Ile Tyr Gln Ala Arg Phe Leu Lys
        195             200             205

Tyr Leu Glu Ser Arg Gly Leu Ala Lys Thr Lys Gly Arg Lys Val Trp
    210             215             220

Cys Phe Cys Gly Asp Gly Glu Met Asp Glu Pro Glu Ser Gln Gly Ala
225             230             235             240

Ile Ala Leu Ala Ala Arg Glu Gly Leu Asp Asn Leu Ile Phe Val Ile
            245             250             255

Asn Cys Asn Leu Gln Arg Leu Asp Gly Pro Val Arg Gly Asn Gly Lys
        260             265             270

Ile Ile Gln Glu Leu Glu Gly Asn Phe Ala Gly Ala Gly Trp Asn Val
    275             280             285

Val Lys Val Ile Trp Gly Arg Arg Trp Asp Arg Leu Leu Ala Lys Asp
    290             295             300

Lys Asp Gly Ile Leu Arg Gln Arg Met Glu Glu Cys Leu Asp Gly Asp
305             310             315             320

Tyr Gln Thr Tyr Lys Ser Lys Asp Gly Ala Tyr Val Arg Glu His Phe
            325             330             335
```

257

```
Phe Asn Thr Pro Glu Leu Lys Ala Leu Val Ala Asp Met Thr Asp Glu
            340             345             350

Gln Leu Trp Ala Leu Asn Arg Gly Gly His Asp Pro Gln Lys Val Tyr
            355             360             365

Asn Ala Tyr Asp Arg Ala Ala Asn His Ala Asp Gly Lys Pro Thr Val
    370             375             380

Ile Leu Ala Lys Thr Ile Lys Gly Tyr Gly Met Gly Ala Ser Gly Glu
385             390             395             400

Gly Gln Asn Val Ala His Gln Ala Lys Lys Met Asp Lys Ala Ser Leu
            405             410             415

Lys Gln Phe Arg Asp Arg Phe Asp Ile Pro Val Thr Asp Glu Gln Ile
            420             425             430

Glu Ser Gly Asp Leu Pro Tyr Leu Thr Phe Ala Pro Asp Thr Glu Glu
            435             440             445

Tyr Lys Tyr Leu His Ala Arg Arg Asp Ala Leu Gly Gly Tyr Leu Pro
    450             455             460

Gln Arg Lys Pro Thr Gln Glu Val Leu Glu Val Pro Glu Leu Ser Ala
465             470             475             480

Phe Asp Ala Gln Leu Lys Ser Ser Gly Glu Arg Glu Phe Ser Thr Thr
            485             490             495

Met Ala Phe Val Arg Ile Leu Ser Thr Leu Leu Lys Asp Lys Lys Ile
            500             505             510

Gly Lys Arg Val Val Pro Ile Val Pro Asp Glu Ser Arg Thr Phe Gly
            515             520             525

Met Glu Gly Met Phe Arg Gln Tyr Gly Ile Trp Asn Pro Lys Gly Gln
    530             535             540

Gln Tyr Thr Pro Gln Asp Lys Asp Gln Leu Met Phe Tyr Lys Glu Ser
545             550             555             560

Val Asp Gly Gln Ile Leu Gln Glu Gly Ile Asn Glu Pro Gly Ala Met
            565             570             575

Ala Asp Trp Ile Ala Ala Ala Thr Ser Tyr Ala Asn Ser Asn Phe Ala
            580             585             590

Met Ile Pro Phe Tyr Ile Tyr Tyr Ser Met Phe Gly Phe Gln Arg Ile
    595             600             605

Gly Asp Leu Ala Trp Ala Ala Gly Asp Met His Ala Arg Gly Phe Leu
    610             615             620

Leu Gly Gly Thr Ala Gly Arg Thr Thr Leu Asn Gly Glu Gly Leu Gln
625             630             635             640

His Glu Asp Gly His Ser His Ile Gln Ala Asp Leu Ile Pro Asn Cys
            645             650             655

Val Ser Tyr Asp Pro Thr Phe Gln Tyr Glu Val Ala Val Ile Val Gln
    660             665             670

Asp Gly Leu Arg Arg Met Tyr Ala Asn Asn Glu Asp Val Phe Tyr Tyr
    675             680             685

Ile Thr Leu Met Asn Glu Asn Tyr Thr His Pro Asp Met Pro Glu Gly
    690             695             700
```

```
Ala Glu Gln Asp Ile Leu Lys Gly Met Tyr Leu Leu Lys Ala Gly Gly
705             710             715                 720

Lys Gly Asp Lys Lys Val Gln Leu Met Gly Ser Gly Thr Ile Leu Gln
                725             730                 735

Glu Val Ile Ala Gly Ala Glu Leu Leu Lys Ala Asp Phe Gly Val Glu
            740             745             750

Ala Asp Ile Trp Ser Cys Pro Ser Phe Asn Leu Leu His Arg Asp Ala
            755             760             765

Val Glu Val Glu Arg Phe Asn Arg Leu His Pro Leu Glu Ala Glu Lys
        770             775             780

Val Pro Phe Val Thr Ser Gln Leu Gln Gly His Asp Gly Pro Val Ile
785             790             795                 800

Ala Ala Thr Asp Tyr Ile Arg Ser Tyr Ala Asp Arg Ile Arg Ala Tyr
                805             810             815

Ile Pro Asn Asp Tyr His Val Leu Gly Thr Asp Gly Phe Gly Arg Ser
            820             825             830

Asp Ser Arg Ala Asn Leu Arg Arg Phe Phe Glu Val Asp Arg Tyr Asn
        835             840             845

Val Ala Val Ala Ala Leu Ala Ala Leu Ala Glu Gln Gly Lys Val Ser
    850             855             860

Lys Glu Thr Val Gln Gln Ala Ile Glu Lys Tyr Gly Ile Lys Ala Asp
865             870             875                 880

Ser Ala Pro Ser Trp Lys Arg
            885
```

<210> 127
<211> 535
<212> PRT
<213> Neisseria meningitidis

<400> 127

```
Met Ser Ile Val Glu Ile Lys Val Pro Asp Ile Gly Gly His Glu Asn
1               5               10              15

Val Asp Ile Ile Ala Val Glu Val Lys Ala Gly Asp Thr Ile Ala Val
            20              25              30

Asp Asp Thr Leu Ile Thr Leu Glu Thr Asp Lys Ala Thr Met Asp Val
        35              40              45

Pro Ala Asp Ala Ala Gly Val Val Lys Glu Val Lys Val Lys Val Gly
    50              55              60

Asp Lys Ile Ser Glu Gly Gly Val Ile Leu Thr Val Glu Thr Gly Ala
65              70              75              80

Ala Ala Glu Ala Ala Pro Ala Ala Ala Glu Ala Gln Pro Ala Pro
        85              90              95

Ala Ala Ala Pro Ala Ala Ala Gly Gly Ala Thr Val Gln Val Ala Val
        100             105             110

Pro Asp Ile Gly Gly His Thr Asp Val Asp Val Ile Ala Val Glu Ile
        115             120             125

Lys Val Gly Asp Thr Val Ala Glu Asp Asp Thr Leu Ile Thr Leu Glu
    130             135             140

Thr Asp Lys Ala Thr Met Asp Val Pro Cys Thr Ala Ala Gly Val Val
```

|        | 145  |     |     |     | 150  |     |     |     | 155  |     |     |     | 160  |     |
|--------|------|-----|-----|-----|------|-----|-----|-----|------|-----|-----|-----|------|-----|

Lys Ala Val Phe Leu Lys Val Gly Asp Lys Val Ser Glu Gly Ser Ala
        165             170             175

Ile Ile Glu Val Glu Thr Val Gly Ser Ala Ala Ala Ala Pro Ala Gln
        180             185             190

Ala Ala Gln Ala Ala Ala Pro Ala Ala Ala Pro Pro Pro Thr Ala Ala
        195             200             205

Ala Ala Pro Ala Ala Ala Pro Ala Pro Ser Ala Pro Ala Ala Ala Lys
        210             215             220

Ile Asp Glu Ala Ala Phe Ala Lys Ala His Ala Gly Pro Ser Ala Arg
225             230             235             240

Lys Leu Ala Arg Glu Leu Gly Val Asp Leu Gly Gln Val Lys Gly Thr
        245             250             255

Gly Leu Lys Gly Arg Ile Met Gly Asp Asp Ile Lys Ala Phe Val Lys
        260             265             270

Ser Val Met Gln Gly Gly Ala Ala Lys Pro Ala Ala Ala Ser Ala Ser
        275             280             285

Leu Gly Gly Gly Leu Asp Leu Leu Pro Trp Pro Lys Val Asp Phe Ser
        290             295             300

Lys Phe Gly Asn Val Glu Val Lys Glu Leu Ser Arg Ile Lys Lys Ile
305             310             315             320

Ser Gly Gln Asn Leu Ser Arg Asn Trp Val Val Ile Pro His Val Thr
        325             330             335

Val His Glu Glu Ala Asp Met Thr Glu Leu Glu Glu Phe Arg Lys Gln
        340             345             350

Leu Asn Lys Glu Trp Glu Arg Glu Gly Val Lys Leu Ser Pro Leu Ala
        355             360             365

Phe Ile Ile Lys Ala Ser Val Ser Ala Leu Lys Ala Phe Pro Glu Phe
        370             375             380

Asn Ala Ser Leu Asp Gly Asp Asn Leu Val Leu Lys Asn Tyr Phe Asn
385             390             395             400

Ile Gly Phe Ala Ala Asp Thr Pro Asn Gly Leu Val Val Pro Val Ile
        405             410             415

Lys Asp Val Asp Gln Lys Gly Leu Lys Gln Ile Ser Gln Glu Leu Thr
        420             425             430

Glu Leu Ser Lys Lys Ala Arg Glu Gly Lys Leu Lys Pro Gln Glu Met
        435             440             445

Gln Gly Ala Cys Phe Thr Ile Ser Ser Leu Gly Gly Ile Gly Gly Thr
        450             455             460

Gly Phe Thr Pro Ile Val Asn Ala Pro Glu Val Ala Ile Leu Gly Val
465             470             475             480

Cys Lys Ser Gln Ile Lys Pro Val Trp Asn Gly Lys Glu Phe Ala Pro
        485             490             495

Arg Leu Met Cys Pro Leu Ser Leu Ser Phe Asp His Arg Val Ile Asp
        500             505             510

Gly Ala Ala Gly Met Arg Phe Thr Val Phe Leu Ala Lys Leu Leu Lys
        515             520             525

```
Asp Phe Arg Arg Ile Thr Leu
    530                 535
```

<210> 128
<211> 145
<212> PRT
<213> Neisseria meningitidis


<400> 128

```
Met Gly Asn Phe Leu Tyr Arg Gly Ile Ser Cys Gln Gln Asp Glu Gln
1               5               10                  15

Asn Asn Gly Gln Leu Lys Pro Lys Gly Asn Lys Ala Glu Val Ala Ile
            20                  25                  30

Arg Tyr Asp Gly Lys Phe Lys Tyr Asp Gly Lys Ala Thr His Gly Pro
        35                  40                  45

Ser Val Lys Asn Ala Val Tyr Ala His Gln Ile Glu Thr Gly Leu Tyr
        50                  55                  60

Asp Gly Cys Tyr Ile Ser Thr Thr Thr Asp Lys Glu Ile Ala Lys Lys
65                  70                  75                  80

Phe Ala Thr Ser Ser Gly Ile Glu Asn Gly Tyr Ile Tyr Val Leu Asn
            85                  90                  95

Arg Asp Leu Phe Gly Gln Tyr Ser Ile Phe Glu Tyr Glu Val Glu His
            100                 105                 110

Pro Glu Asn Pro Asn Glu Lys Glu Val Thr Ile Arg Ala Glu Asp Cys
            115                 120                 125

Gly Cys Ile Pro Glu Glu Val Ile Ile Ala Lys Glu Leu Ile Glu Ile
    130                 135                 140

Asn
145
```

<210> 129
<211> 594
<212> PRT
<213> Neisseria meningitidis


<400> 129

Met Ala Leu Val Glu Leu Lys Val Pro Asp Ile Gly Gly His Glu Asn
1                5                10               15

Val Asp Ile Ile Ala Val Glu Val Asn Val Gly Asp Thr Ile Ala Val
            20              25              30

Asp Asp Thr Leu Ile Thr Leu Glu Thr Asp Lys Ala Thr Met Asp Val
        35              40              45

Pro Ala Glu Val Ala Gly Val Val Lys Glu Val Lys Val Lys Val Gly
    50              55              60

Asp Lys Ile Ser Glu Gly Gly Leu Ile Val Val Val Glu Ala Glu Gly
65              70              75              80

Thr Ala Ala Ala Pro Lys Ala Glu Ala Ala Ala Pro Ala Gln Glu
            85              90              95

Ala Pro Lys Ala Ala Ala Pro Ala Pro Gln Ala Ala Gln Phe Gly Gly
        100             105             110

Ser Ala Asp Ala Glu Tyr Asp Val Val Val Leu Gly Gly Gly Pro Gly
        115             120             125

Gly Tyr Ser Ala Ala Phe Ala Ala Ala Asp Glu Gly Leu Lys Val Ala
130             135             140

Ile Val Glu Arg Tyr Lys Thr Leu Gly Gly Val Cys Leu Asn Val Gly
145             150             155             160

Cys Ile Pro Ser Lys Ala Leu Leu His Asn Ala Ala Val Ile Asp Glu
165             170             175

Val Arg His Leu Ala Ala Asn Gly Ile Lys Tyr Pro Glu Pro Glu Leu
180             185             190

Asp Ile Asp Met Leu Arg Ala Tyr Lys Asp Gly Val Val Ser Arg Leu
195             200             205

Thr Gly Gly Leu Ala Gly Met Ala Lys Ser Arg Lys Val Asp Val Ile
210             215             220

Gln Gly Asp Gly Gln Phe Leu Asp Pro His His Leu Glu Val Ser Leu
225             230             235             240

Thr Ala Gly Asp Ala Tyr Glu Gln Ala Ala Pro Thr Gly Glu Lys Lys
245             250             255

Ile Val Ala Phe Lys Asn Cys Ile Ile Ala Ala Gly Ser Arg Val Thr
260             265             270

Lys Leu Pro Phe Ile Pro Glu Asp Pro Arg Ile Ile Asp Ser Ser Gly
275             280             285

Ala Leu Ala Leu Lys Glu Val Pro Gly Lys Leu Leu Ile Ile Gly Gly
290             295             300

Gly Ile Ile Gly Leu Glu Met Gly Thr Val Tyr Ser Thr Leu Gly Ser
305             310             315             320

Arg Leu Asp Val Val Glu Met Met Asp Gly Leu Met Gln Gly Ala Asp
325             330             335

Arg Asp Leu Val Lys Val Trp Gln Lys Gln Asn Glu Tyr Arg Phe Asp
340             345             350

Asn Ile Met Val Asn Thr Lys Thr Val Ala Val Glu Pro Lys Glu Asp
355             360             365

Gly Val Tyr Val Thr Phe Glu Gly Ala Asn Ala Pro Lys Glu Pro Gln
370             375             380

Arg Tyr Asp Ala Val Leu Val Ala Ala Gly Arg Ala Pro Asn Gly Lys
385             390             395             400

Leu Ile Ser Ala Glu Lys Ala Gly Val Ala Val Thr Asp Arg Gly Phe
405             410             415

Ile Glu Val Asp Lys Gln Met Arg Thr Asn Val Pro His Ile Tyr Ala
420             425             430

Ile Gly Asp Ile Val Gly Gln Pro Met Leu Ala His Lys Ala Val His
435             440             445

Glu Gly His Val Ala Ala Glu Asn Cys Ala Gly His Lys Ala Tyr Phe
450             455             460

Asp Ala Arg Val Ile Pro Gly Val Ala Tyr Thr Ser Pro Glu Val Ala
465             470             475             480

Trp Val Gly Glu Thr Glu Leu Ser Ala Lys Ala Ser Gly Arg Lys Ile
485             490             495

Thr Lys Ala Asn Phe Pro Trp Ala Ala Ser Gly Arg Ala Ile Ala Asn

```
                    500                    505                    510
        Gly Cys Asp Asn Gly Phe Thr Lys Leu Ile Phe Asp Ala Glu Thr Gly
                515                    520                    525

        Arg Ile Ile Gly Gly Gly Ile Val Gly Pro Asn Gly Gly Asp Met Ile
            530                    535                    540

        Gly Glu Val Cys Leu Ala Ile Glu Met Gly Cys Asp Ala Ala Asp Ile
        545                    550                    555                    560

        Gly Lys Thr Ile His Pro His Pro Thr Leu Gly Glu Ser Ile Gly Met
                565                    570                    575

        Ala Ala Glu Val Ala Leu Gly Thr Cys Thr Asp Leu Pro Pro Gln Lys
                580                    585                    590

        Lys Lys
```

<210> 130
<211> 261
<212> PRT
<213> Neisseria meningitidis

<400> 130

```
Met Asn Pro Phe Leu Asn Thr Ala Phe Lys Ala Ala Arg Arg Ala Gly
1               5                   10                  15

Gln Met Met Ile Arg Ala Ala Gly Asn Leu Asp Ala Val Lys Thr Asp
            20                  25                  30

Ser Lys Ala Phe Asn Asp Phe Val Ser Asp Val Asp Arg Asn Ser Glu
        35                  40                  45

Ile Ile Leu Val Glu Ala Leu Lys Glu Ala Tyr Pro His His Lys Ile
        50                  55                  60

Thr Cys Glu Glu Ser Gly Ser His Gly Lys Ala Ala Ala Glu Tyr Glu
65                  70                  75                  80

Trp Ile Ile Asp Pro Leu Asp Gly Thr Thr Asn Phe Leu His Gly His
            85                  90                  95

Pro Gln Tyr Ala Ile Ser Met Ala Leu Leu His Lys Gly Val Leu Gln
            100                 105                 110

Glu Ala Leu Val Tyr Ala Pro Glu Arg Asn Asp Val Tyr Met Ala Ser
        115                 120                 125

Arg Gly Lys Gly Ala Leu Leu Asn Asp Arg Arg Ile Arg Val Ser Asn
    130                 135                 140

Arg Ile Glu Leu Asn Arg Cys Leu Ile Gly Thr Gly Phe Pro Val Val
145                 150                 155                 160

Asp Gln Ser Met Met Asp Lys Tyr Leu Ala Ile Leu Lys Asp Phe Leu
            165                 170                 175

Ala Lys Thr Ala Gly Gly Arg Arg Glu Gly Ala Ala Ser Leu Asp Leu
        180                 185                 190

Cys Ala Val Ala Thr Gly Arg Phe Asp Gly Phe Phe Glu Phe Asn Leu
        195                 200                 205

Lys Pro Trp Asp Ile Ala Ala Gly Ala Leu Ile Val Gln Glu Ala Gly
    210                 215                 220

Gly Ile Val Thr Asp Met Ser Gly Glu Asp Gly Trp Leu Glu Ser Gly
225                 230                 235                 240

Asp Ile Val Ala Ala Asn Pro Lys Val Leu Ala Gln Met Leu Lys Ile
            245                 250                 255

Ile Ser Ala His Val
            260
```

<210> 131
<211> 481
<212> PRT
<213> Neisseria meningitidis

<400> 131

```
Met Thr Gln Tyr Thr Leu Lys Gln Ala Ser Val Leu Leu Gln Ser Lys
1           5                   10                  15
Gln Ile Ser Ala Val Glu Leu Ala Ser Ala Tyr Leu Ala Ala Ile Ala
            20              25                  30
Glu Lys Asn Pro Ala Leu Asn Gly Tyr Ile Thr Ile Asp Gln Asp Lys
        35                  40                  45
Thr Leu Ala Glu Ala Arg Ala Ala Asp Glu Arg Ile Ala Gln Gly Asn
    50              55                  60
Ala Ser Ala Leu Thr Gly Val Pro Val Ala Tyr Lys Asp Ile Phe Cys
65              70                  75                  80
Gln Thr Gly Trp Arg Ser Ala Cys Ala Ser Lys Met Leu Asp Asn Phe
                85                  90                  95
Ile Ser Pro Tyr Thr Ala Thr Val Val Gln Asn Leu Leu Asp Glu Gly
            100                 105                 110
Met Val Thr Leu Gly Arg Thr Asn Met Asp Glu Phe Ala Met Gly Ser
            115                 120                 125
Thr Asn Glu Asn Ser Phe Tyr Gly Ala Ala Lys Asn Pro Trp Asn Leu
    130                 135                 140
Glu His Val Pro Gly Gly Ser Ser Gly Gly Ser Ala Ala Val Val Ala
145                 150                 155                 160
Ala Arg Leu Ala Pro Ala Ala Leu Gly Ser Asp Thr Gly Gly Ser Ile
                165                 170                 175
Arg Gln Pro Ala Ser His Cys Gly Ile Thr Gly Ile Lys Pro Thr Tyr
            180                 185                 190
Gly Thr Val Ser Arg Phe Gly Met Val Ala Tyr Ala Ser Ser Phe Asp
            195                 200                 205
Gln Thr Gly Pro Met Ala Gln Thr Ala Glu Asp Cys Ala Ile Leu Leu
    210                 215                 220
Asn Ala Met Ala Gly Phe Asp Pro Lys Asp Ser Thr Ser Leu Glu Arg
225                 230                 235                 240
Glu Lys Glu Asp Tyr Thr Arg Asp Leu Asn Gln Pro Leu Lys Gly Leu
            245                 250                 255
Lys Ile Gly Leu Pro Lys Glu Tyr Phe Gly Glu Gly Asn Ser Ala Asp
            260                 265                 270
Val Leu Thr Ala Leu Gln Asn Thr Ile Asp Leu Leu Lys Ala Gln Gly
            275                 280                 285
Ala Glu Leu Ile Glu Val Ser Leu Pro Gln Thr Lys Leu Ser Ile Pro
            290                 295                 300
```

```
Ala Tyr Tyr Val Leu Ala Ser Ala Glu Ala Ser Thr Asn Leu Ser Arg
305                 310             315                 320

Tyr Asp Gly Val Arg Tyr Gly His Arg Ala Ala Gln Phe Ala Asp Leu
            325             330                 335

Glu Glu Met Tyr Gly Lys Thr Arg Ala Glu Gly Phe Gly Ser Glu Val
        340             345             350

Lys Arg Arg Ile Met Ile Gly Thr Tyr Val Leu Ser His Gly Tyr Tyr
        355             360             365

Asp Ala Tyr Tyr Leu Lys Ala Gln Lys Leu Arg Arg Leu Val Ala Asp
    370             375             380

Asp Phe Gln Thr Ala Phe Ala Arg Cys Asp Leu Ile Leu Ala Pro Thr
385             390             395                 400

Ala Pro Thr Ala Ala Pro Lys Ile Gly Ala Asp Ala Ser Pro Val Glu
            405             410             415

Thr Tyr Leu Ser Asp Ile Tyr Thr Ile Ala Val Asn Leu Ala Gly Leu
        420             425             430

Pro Ala Leu Thr Leu Pro Ala Gly Phe Ser Gly Gly Gly Leu Pro Val
        435             440             445

Gly Val Gln Leu Val Gly Asn Tyr Phe Ala Glu Ala Lys Ile Leu Gly
    450             455             460

Ala Ala His Gln Ile Gln Leu Asn Ser Asp Trp His Gly Lys Arg Pro
465             470             475                 480

Glu
```

<210> 132
<211> 476
<212> PRT
<213> Neisseria meningitidis

<400> 132

```
Met Thr Trp Glu Thr Val Ile Gly Leu Glu Ile His Val Gln Leu Asn
1               5               10              15

Thr Lys Ser Lys Ile Phe Ser Gly Ala Ser Thr Ala Phe Gly Ala Glu
            20              25              30

Pro Asn Ala His Ala Ser Val Val Glu Cys Ala Leu Pro Gly Val Leu
        35              40              45

Pro Val Met Asn Arg Glu Val Val Glu Lys Ala Ile Lys Leu Gly Leu
    50              55              60

Ala Leu Asp Ala Lys Ile Asn Gln Lys Asn Val Phe Asp Arg Lys Asn
65              70              75              80

Tyr Phe Tyr Pro Asp Leu Pro Lys Gly Tyr Gln Ile Ser Gln Leu Asp
            85              90              95

Leu Pro Ile Val Glu His Gly Lys Leu Glu Ile Val Val Gly Asp Asp
            100             105             110

Val Lys Thr Ile Asn Val Thr Arg Ala His Met Glu Glu Asp Ala Gly
        115             120             125

Lys Ser Val His Glu Gly Leu Asn Gly Ala Thr Gly Ile Asp Leu Asn
    130             135             140

Arg Ala Gly Thr Pro Leu Leu Glu Val Val Ser Glu Pro Glu Met Arg
```

|  |  |  |  |  |  |  |  |  |  |  |  |  |  |  |  |
|---|---|---|---|---|---|---|---|---|---|---|---|---|---|---|---|
| 145 |  |  |  |  | 150 |  |  |  | 155 |  |  |  | 160 |  |  |

Ser Ala Ala Glu Ala Val Ala Tyr Ala Lys Ala Leu His Ser Leu Val
165 170 175

Thr Trp Leu Asp Ile Cys Asp Gly Asn Met Ala Glu Gly Ser Phe Arg
180 185 190

Val Asp Ala Asn Val Ser Val Arg Pro Lys Gly Gln Glu Glu Phe Gly
195 200 205

Thr Arg Arg Glu Ile Lys Asn Leu Asn Ser Phe Arg Phe Leu Glu Gln
210 215 220

Ala Ile Asn Tyr Glu Ala Glu Ala Gln Ile Glu Ile Leu Glu Asp Gly
225 230 235 240

Gly Lys Val Gln Gln Ala Thr Met Leu Phe Asp Pro Glu Lys Gly Glu
245 250 255

Thr Arg Val Met Arg Leu Lys Glu Asp Ala His Asp Tyr Arg Tyr Phe
260 265 270

Pro Asp Pro Asp Leu Leu Pro Val Ile Ile Ser Asp Ala Gln Met Gln
275 280 285

Lys Ala Lys Ala Glu Met Pro Glu Leu Pro Lys Glu Met Ala Ala Arg
290 295 300

Phe Val Ala Asp Tyr Gly Val Ser Glu Tyr Asp Ala Arg Leu Leu Thr
305 310 315 320

Ala Ser Arg Ala Gln Ala Ala Tyr Phe Glu Glu Ala Ala Lys Glu Ser
325 330 335

Gly Gln Gly Lys Leu Thr Ala Asn Trp Met Asn Gly Glu Leu Ala Ala
340 345 350

Ala Leu Asn Lys Glu Gly Met Glu Leu Ala Asp Ser Pro Ile Thr Ala
355 360 365

Pro Arg Leu Ala Ala Leu Val Gly Lys Ile Ala Asp Gly Thr Leu Ser
370 375 380

Ser Lys Leu Ala Lys Lys Ala Phe Glu Ala Met Trp Ala Glu Pro Glu
385 390 395 400

Ala Thr Ile Ala Glu Ile Ile Glu Lys His Gly Leu Gln Gln Met Thr
405 410 415

Asp Thr Gly Glu Ile Glu Ala Met Val Asp Glu Val Leu Ala Asn Asn
420 425 430

Ala Lys Ala Val Glu Gln Phe Lys Ser Gly Asn Glu Lys Ala Leu Asn
435 440 445

Ala Ile Val Gly Gln Val Met Lys Ala Ser Lys Gly Lys Ala Asn Pro
450 455 460

Ala Gln Val Gln Glu Leu Ile Lys Ala Lys Leu Ala
465 470 475

<210> 133
<211> 414
<212> PRT
<213> Neisseria meningitidis

<400> 133

```
Met Ser Asn Glu Asn Arg Thr Cys Ser Phe Cys Gly Lys Ser Lys Ser
1               5                   10                  15
```

His Val Lys His Leu Ile Glu Gly Glu Asn Ala Phe Ile Cys Asp Glu
            20                    25                    30

Cys Val Ser Asn Cys Ile Glu Ile Leu His Glu Asp Gly Asn Asp Gly
            35                    40                    45

Thr Pro Ser Glu Ser Ala Gly Gly Glu Pro Glu Glu Ser Gly Lys Leu
    50                    55                    60

Pro Thr Pro Ala Glu Ile Val Ala Asn Leu Asn Asp His Val Ile Gly
65                    70                    75                    80

Gln Glu Gln Ala Lys Lys Ala Leu Ala Val Ser Val Tyr Asn His Tyr
                85                    90                    95

Lys Arg Leu Arg His Pro Lys Ala Gly Ala Asn Val Glu Leu Ser Lys
            100                    105                    110

Ser Asn Ile Leu Leu Ile Gly Pro Thr Gly Ser Gly Lys Thr Leu Leu
            115                    120                    125

Ala Gln Ser Leu Ala Arg Lys Leu Asp Val Pro Phe Val Met Ala Asp
    130                    135                    140

Ala Thr Thr Leu Thr Glu Ala Gly Tyr Val Gly Glu Asp Val Glu Gln
145                    150                    155                    160

Ile Ile Thr Lys Leu Leu Gly Lys Cys Asp Phe Asp Val Glu Lys Ala
                165                    170                    175

Gln Arg Gly Ile Val Tyr Ile Asp Glu Ile Asp Lys Ile Ser Arg Lys
            180                    185                    190

Ser Asp Asn Pro Ser Ile Thr Arg Asp Val Ser Gly Glu Gly Val Gln
            195                    200                    205

Gln Ala Leu Leu Lys Leu Ile Glu Gly Thr Val Ala Ser Val Pro Pro
    210                    215                    220

Gln Gly Gly Arg Lys His Pro Asn Gln Glu Phe Ile Asn Val Asp Thr
225                    230                    235                    240

Thr Asn Ile Leu Phe Ile Cys Gly Gly Ala Phe Ala Gly Leu Glu Lys
                245                    250                    255

Val Ile Arg Gln Arg Thr Glu Lys Gly Gly Ile Gly Phe Gly Ala Ser
            260                    265                    270

Val His Ser Lys Asp Glu Asn Ala Asp Ile Thr Lys Leu Phe Gly Ile
    275                    280                    285

Val Glu Pro Glu Asp Leu Ile Lys Phe Gly Leu Ile Pro Glu Leu Ile
    290                    295                    300

Gly Arg Leu Pro Val Ile Ala Thr Leu Glu Glu Leu Asp Glu Asp Ala
305                    310                    315                    320

Leu Ile Asn Ile Leu Thr Glu Pro Lys Asn Ala Leu Val Lys Gln Tyr
                325                    330                    335

Gln Ala Leu Phe Gly Met Glu Asn Val Glu Leu Glu Phe Glu Glu Gly
            340                    345                    350

Ala Leu Arg Ser Ile Ala Arg Gln Ala Met Glu Arg Lys Thr Gly Ala
            355                    360                    365

Arg Gly Leu Arg Ser Ile Val Glu Arg Cys Leu Leu Asp Thr Met Tyr
    370                    375                    380

```
Arg Leu Pro Asp Leu Lys Gly Leu Lys Lys Val Val Val Gly Lys Ala
385                 390             395                 400

Val Ile Glu Glu Gly Arg Glu Pro Glu Leu Val Phe Glu Ser
                405                 410
```

<210> 134
<211> 404
<212> PRT
<213> Neisseria meningitidis

<400> 134

```
Met Thr Val Lys Thr Pro Val Tyr Leu Asp Tyr Ala Ala Thr Pro Pro
1               5               10              15

Val Asp Lys Arg Val Ala Glu Lys Met Ile Pro Tyr Leu Thr Glu Thr
            20              25              30

Phe Gly Asn Pro Ala Ser Asn Ser His Ser Phe Gly Trp Glu Ala Glu
        35              40              45

Glu Ala Val Glu Lys Ala Arg Ala Asp Ile Ala Ala Leu Ile Asn Ala
    50              55              60

Asp Ser Lys Glu Ile Val Phe Thr Ser Gly Ala Thr Glu Ser Asn Asn
65              70              75              80

Leu Ala Ile Lys Gly Ala Ala His Phe Tyr Lys Ser Lys Gly Asn His
            85              90              95

Leu Ile Thr Val Lys Thr Glu His Lys Ala Val Leu Asp Thr Met Arg
        100             105             110

Glu Leu Glu Arg Gln Gly Tyr Glu Val Thr Tyr Leu Asp Val Gln Glu
    115             120             125

Asn Gly Leu Val Asp Leu Asp Val Leu Lys Ala Ala Ile Arg Glu Asp
    130             135             140

Thr Ile Leu Val Ser Val Met Trp Val Asn Asn Glu Ile Gly Val Val
145             150             155             160

Gln Asp Ile Pro Ala Ile Gly Glu Ile Cys Arg Glu Arg Lys Ile Ile
            165             170             175

Phe His Val Asp Ala Ala Gln Ala Cys Gly Lys Val Pro Val Asp Val
        180             185             190

Glu Ala Ala Lys Val Asp Leu Leu Ser Met Ser Gly His Lys Val Tyr
    195             200             205

Gly Pro Lys Gly Ile Gly Ala Leu Tyr Val Arg Arg Lys Pro Arg Val
    210             215             220

Arg Leu Glu Ala Gln Met His Gly Gly Gly His Glu Arg Gly Phe Arg
225             230             235             240

Ser Gly Thr Leu Pro Thr His Gln Ile Val Gly Met Gly Glu Ala Phe
            245             250             255

Arg Ile Ala Lys Glu Glu Leu Ala Gln Asp Thr Ala His Tyr Leu Lys
            260             265             270

Leu Arg Asp Ile Phe Leu Lys Gly Ile Glu Gly Ile Glu Glu Val Tyr
        275             280             285

Ile Asn Gly Asp Leu Glu His Arg Val Pro Asn Asn Leu Asn Val Ser
    290             295             300

Phe Asn Phe Val Glu Gly Glu Ser Leu Ile Met Ala Val Lys Glu Leu
```

```
        305                     310               315                 320
        Ala Val Ser Ser Gly Ser Ala Cys Thr Ser Ala Ser Leu Glu Pro Ser
                        325             330             335
        Tyr Val Leu Arg Ala Leu Gly Arg Asn Asp Glu Leu Ala His Ser Ser
                        340             345             350
        Leu Arg Ile Thr Phe Gly Arg Met Thr Thr Glu Glu Glu Val Gln Phe
                        355             360             365
        Ala Ala Glu Leu Ile Lys Ser Lys Ile Gly Lys Leu Arg Glu Leu Ser
                370             375             380
        Pro Leu Trp Glu Met Phe Lys Asp Gly Ile Asp Leu Asn Ser Ile Glu
        385             390             395             400
        Trp Ala Ala His
```

<210> 135
<211> 328
<212> PRT
<213> Neisseria meningitidis

<400> 135

```
Met Ser Ser Thr Pro Asn Lys Gln Ala Gly Tyr Pro Arg Leu Val Ala
1               5                   10              15

Asp Ile Gly Gly Thr Asn Ala Arg Phe Ala Leu Glu Thr Ala Pro Arg
            20              25              30

Val Ile Glu Lys Ala Ala Val Leu Pro Cys Lys Asp Tyr Asp Thr Val
            35              40              45

Thr Asp Ala Val Arg Ala Tyr Leu Asn Gln Ser Gly Ala Thr Ala Val
        50              55              60

Arg His Ala Ala Phe Ala Ile Ala Asn Pro Ile Leu Gly Asp Trp Val
65              70              75              80

Gln Met Thr Asn His His Trp Ala Phe Ser Ile Glu Thr Thr Arg Gln
                85              90              95

Thr Leu Gly Leu Asp Thr Leu Ile Leu Leu Asn Asp Phe Thr Ala Gln
            100             105             110

Ala Leu Ala Val Thr Gln Thr Ser Ser Lys Asp Leu Met Gln Val Gly
        115             120             125

Gly Gln Lys Pro Val Glu Phe Ala Pro Lys Ala Val Ile Gly Pro Gly
    130             135             140

Thr Gly Leu Gly Val Ser Gly Leu Val His Ser His Ala Gly Trp Val
145             150             155             160

Ala Leu Ala Gly Glu Gly Gly His Thr Ser Phe Pro Pro Phe Asp Asp
            165             170             175

Met Glu Val Leu Ile Trp Gln Tyr Ala Lys Asn Lys Tyr Gly His Val
            180             185             190

Ser Ala Glu Arg Phe Leu Ser Gly Ala Gly Leu Ser Leu Val Tyr Glu
        195             200             205

Ala Leu Ala Ala Lys Gln Lys Ala Lys Pro Ala Lys Leu Met Pro Ser
    210             215             220

Glu Ile Thr Glu Lys Ala Leu Ser Gly Ala Ser Pro Leu Cys Arg Gln
225             230             235             240

Thr Leu Asp Ile Phe Cys Ala Met Leu Gly Thr Val Ala Ser Asn Leu
            245             250             255

Ala Leu Thr Leu Gly Ala Arg Gly Gly Val Tyr Leu Cys Gly Gly Ile
        260             265             270

Ile Pro Arg Val Leu Glu Tyr Phe Lys Thr Ser Pro Phe Arg Ser Arg
        275             280             285

Phe Glu Asn Lys Gly Arg Phe Glu Ala Tyr Leu Ala Ala Ile Pro Val
    290             295             300

Tyr Val Val Leu Ser Glu Phe Pro Gly Ile Ser Gly Ala Ala Ala Ala
305             310             315             320

Leu Asp Asn His Leu Arg Asn Val
            325
```

<210> 136

<211> 481

<212> PRT

<213> Neisseria meningitidis

<400> 136

```
Met Ser Thr Gln Thr Asn Phe Asp Leu Val Leu Phe Gly Ala Thr Gly
1               5                   10                  15

Asp Leu Ala Met Arg Lys Leu Leu Pro Cys Leu Tyr Gln Ala His Val
            20                  25                  30

Ala Gly Leu Leu His Pro Glu Gly Arg Ile Leu Gly Val Ser Arg Ser
        35                  40                  45

Glu Leu Asp Thr Glu Gly Phe Leu Ala Lys Val Glu Thr Ser Ser Lys
    50                  55                  60

Ile His Val Lys Glu Asn Phe Ser Asp Glu Ala Trp Ala Ser Phe Val
65                  70                  75                  80

Glu Arg Phe Ala Tyr Leu Lys Val Asp Val Thr Gln Pro Asp Asp Phe
                85                  90                  95

Ala Ala Leu Gly Asp Leu Val Lys Ala Arg Lys Glu Thr Asp Asn Val
            100                 105                 110

Val Ile Tyr Leu Ser Thr Ala Pro Lys Phe Phe Ala Gln Ala Cys Glu
        115                 120                 125

Asn Leu Ala Ala Ile Gly Leu Asn Ala Asp Asn Val Arg Val Val Leu
    130                 135                 140

Glu Lys Pro Leu Gly Thr Asp Leu Ala Ser Ser Gln Gln Ile Asn Thr
145                 150                 155                 160

Asp Val Ala Arg Tyr Phe Lys Glu Gly Gln Ile Tyr Arg Ile Asp His
                165                 170                 175

Tyr Leu Gly Lys Glu Ser Leu Gln Asn Leu Leu Ala Leu Arg Phe Ala
            180                 185                 190

Asn Val Met Phe Glu Pro Leu Trp Asn Asn Lys Tyr Ile Glu Ser Val
            195                 200                 205

Gln Leu Thr Ile Ala Glu Gln Leu Gly Val Glu Glu Arg Gly Glu Phe
    210                 215                 220

Tyr Asp Ile Thr Gly Ala Leu Arg Asp Met Val Gln Asn His Leu Met
225                 230                 235                 240
```

```
Gln Met Leu Cys Met Thr Ala Met Glu Ala Pro Ala Ser Leu Asp Ala
                245             250             255

Asp Ala Val Arg Asp Glu Lys Val Lys Val Ile Lys Ser Leu Lys Pro
            260             265             270

Leu Thr Val Glu Ser Val Asn Glu Asn Val Val Arg Gly Gln Tyr Thr
        275             280             285

Ala Ala Arg Gly Met Asn Gly Tyr Leu Glu Glu Ile Asn Val Pro Gln
    290             295             300

Asp Ser Phe Thr Glu Thr Tyr Val Ala Ile Lys Ala Glu Ile Glu Asn
305             310             315             320

Glu Arg Trp Lys Gly Val Pro Phe Tyr Leu Arg Thr Gly Lys Arg Met
            325             330             335

Ala Gly Lys Val Ala Glu Ile Val Leu Asn Phe Lys Asp Leu Asn Ser
        340             345             350

His Ile Phe Glu Gly Ser Arg Thr Ala Pro Asn Arg Leu Val Ile Glu
        355             360             365

Leu Gln Pro Tyr Glu Ser Val Arg Leu Tyr Thr Gln Met Lys Thr Pro
370             375             380

Gly Ala Gly Asn Lys Val Glu Thr Val Pro Leu Ala Thr Asp Leu Gly
385             390             395             400

Lys Ala Leu Glu Gly Arg Arg Ala Glu Ala Tyr Glu Arg Leu Leu Leu
            405             410             415

Asp Val Ile Asn Gly Lys Leu Ala Leu Phe Asn Arg Arg Asp Glu Leu
            420             425             430

Glu Ala Ala Trp Glu Tyr Val Met Pro Ile Leu Glu Asn Trp Thr Asn
        435             440             445

Asn Thr Thr Pro Pro His Gly Tyr Gly Ala His Ser Trp Gly Pro Glu
    450             455             460

Ala Ala Arg Glu Leu Leu Ala Arg Asp Gly His Lys Trp His Glu Glu
465             470             475             480

Gln
```

<210> 137
<211> 503
<212> PRT
<213> Neisseria meningitidis

<400> 137

```
Met Ser Glu Gln Asn His Pro Gln Thr Glu Pro Gln Leu Asp Glu Asn
1               5               10                  15

Gln Ile Ile Ala Leu Arg Arg Glu Lys Leu His Asn Ile Arg Gln Gln
            20              25                  30

Arg Asn Ala Tyr Pro Asn Asp Phe Lys Arg Asp Ser Phe Ala Ala Asp
        35              40                  45

Leu His Ala Gln Tyr Gly Glu Ile Gly Lys Glu Glu Leu Asp Pro Gln
    50              55                  60

Gly Ile Pro Val Lys Val Ala Gly Arg Met Met Leu Lys Arg Gln Met
65              70                  75                  80

Gly Lys Ala Ser Phe Ala Thr Ile Gln Asp Val Ser Gly Gln Ile Gln
```

```
                        85                        90                        95
      Leu Tyr Leu Asn Asn Lys Gly Val Ser Gln Glu Val Leu Asp Asp Phe
                  100                     105                     110

      Asn His Trp Asp Leu Gly Asp Ile Val Gly Ala Glu Gly Thr Leu Phe
                  115                     120                     125

      Lys Thr Asn His Gly Glu Leu Thr Val Arg Val Ser Gly Ile Arg Leu
                  130                     135                     140

      Leu Ser Lys Ser Leu Arg Pro Leu Pro Asp Lys His Lys Gly Leu Ser
      145                     150                     155                 160

      Asp Gln Glu Thr Lys Tyr Arg Gln Arg Tyr Val Asp Leu Ile Ala Asn
                      165                     170                     175

      Glu Glu Ser Arg Asn Thr Phe Ile Lys Arg Ser Gln Ile Ile Gln Ser
                  180                     185                     190

      Val Arg Asn Phe Met Val Gly Glu His Tyr Leu Glu Val Glu Thr Pro
                  195                     200                     205

      Met Met His Pro Ile Pro Gly Gly Ala Thr Ala Lys Pro Phe Val Thr
          210                     215                     220

      His His Asn Ala Leu Asp Ile Pro Leu Tyr Leu Arg Ile Ala Pro Glu
      225                     230                     235                 240

      Leu Tyr Leu Lys Arg Leu Val Val Gly Gly Leu Glu Arg Val Phe Glu
                      245                     250                     255

      Ile Asn Arg Ser Phe Arg Asn Glu Gly Met Ser Val Arg His Asn Pro
                  260                     265                     270

      Glu Phe Thr Met Ile Glu Phe Tyr Glu Ala Phe Ser Asp Tyr Glu Arg
              275                     280                     285

      Met Met Gln Met Ala Glu Asp Ile Ile Arg Asn Ala Ser Arg Thr Val
          290                     295                     300

      Asn Gly Thr Ala Asn Ile Thr Tyr Asn Gly Lys Glu Val Asp Leu Glu
      305                     310                     315                 320

      Ser Pro Phe Glu Arg Leu Thr Ile Leu Glu Ala Ile Lys Lys Tyr Asn
                      325                     330                     335

      Pro His Tyr Thr Asp Glu Gln Leu Asn Asp Ala Glu Trp Leu Lys Lys
                  340                     345                     350

      Glu Ile Val Lys His Gly Glu Ser Leu Pro Pro Ser Pro Gly Ile Gly
                  355                     360                     365

      Ser Leu Gln Leu Ala Leu Phe Glu Gly Cys Ala Glu Gly Lys Leu Trp
          370                     375                     380

      Asn Pro Thr Phe Ile Val Asp Tyr Pro Val Glu Val Ser Pro Leu Ala
      385                     390                     395                 400

      Arg Ala Ser Asp Thr Lys Gln Gly Leu Thr Glu Arg Phe Glu Leu Phe
                      405                     410                     415

      Val Val Gly Arg Glu Leu Ala Asn Gly Tyr Ser Glu Leu Asn Asp Pro
                  420                     425                     430

      Glu Asp Gln Ala Glu Arg Phe Lys Ala Gln Val Val Gln Lys Asp Ala
              435                     440                     445

      Gly Asp Asp Glu Ala Met His Tyr Asp Ala Asp Tyr Ile Arg Ala Met
          450                     455                     460
```

280

```
Glu Phe Gly Leu Pro Pro Thr Gly Gly Cys Gly Ile Gly Ile Asp Arg
465             470             475             480

Leu Val Met Leu Leu Thr Asp Ser Gln Thr Ile Arg Asp Val Ile Leu
              485             490             495

Phe Pro Gln Met Arg Pro Glu
              500
```

<210> 138
<211> 392
<212> PRT
<213> Neisseria meningitidis

<400> 138

Met Arg Lys Lys Leu Thr Ala Leu Val Leu Ser Ala Leu Pro Leu Ala
1                5                  10                  15

Ala Val Ala Asp Val Ser Leu Tyr Gly Glu Ile Lys Ala Gly Val Glu
                20                  25                  30

Gly Arg Asn Tyr Gln Leu Gln Leu Thr Glu Ala Gln Ala Ala Asn Gly
        35                  40                  45

Gly Ala Ser Gly Gln Val Lys Val Thr Lys Val Thr Lys Ala Lys Ser
        50                  55                  60

Arg Ile Arg Thr Lys Ile Ser Asp Phe Gly Ser Phe Ile Gly Phe Lys
65                  70                  75                  80

Gly Ser Glu Asp Leu Gly Asp Gly Leu Lys Ala Val Trp Gln Leu Glu
                85                  90                  95

Gln Asp Val Ser Val Ala Gly Gly Gly Ala Thr Gln Trp Gly Asn Arg
        100                 105                 110

Glu Ser Phe Ile Gly Leu Ala Gly Glu Phe Gly Thr Leu Arg Ala Gly
        115                 120                 125

Arg Val Ala Asn Gln Phe Asp Asp Ala Ser Gln Ala Ile Asp Pro Trp
    130                 135                 140

Asp Ser Asn Asn Asp Val Ala Ser Gln Leu Gly Ile Phe Lys Arg His
145                 150                 155                 160

Asp Asp Met Pro Val Ser Val Arg Tyr Asp Ser Pro Glu Phe Ser Gly
            165                 170                 175

Phe Ser Gly Ser Val Gln Phe Val Pro Ile Gln Asn Ser Lys Ser Ala
        180                 185                 190

Tyr Thr Pro Ala Tyr Tyr Thr Lys Asn Thr Asn Asn Asn Leu Thr Leu
        195                 200                 205

Val Pro Ala Val Val Gly Lys Pro Gly Ser Asp Val Tyr Tyr Ala Gly
    210                 215                 220

Leu Asn Tyr Lys Asn Gly Gly Phe Ala Gly Asn Tyr Ala Phe Lys Tyr
225                 230                 235                 240

Ala Arg His Ala Asn Val Gly Arg Asn Ala Phe Glu Leu Phe Leu Ile
            245                 250                 255

Gly Ser Gly Ser Asp Gln Ala Lys Gly Thr Asp Pro Leu Lys Asn His
        260                 265                 270

Gln Val His Arg Leu Thr Gly Gly Tyr Glu Glu Gly Gly Leu Asn Leu
        275                 280                 285

282

Ala Leu Ala Ala Gln Leu Asp Leu Ser Glu Asn Gly Asp Lys Thr Lys
    290             295             300

Asn Ser Thr Thr Glu Ile Ala Ala Thr Ala Ser Tyr Arg Phe Gly Asn
305             310             315             320

Ala Val Pro Arg Ile Ser Tyr Ala His Gly Phe Asp Phe Ile Glu Arg
            325             330             335

Gly Lys Lys Gly Glu Asn Thr Ser Tyr Asp Gln Ile Ile Ala Gly Val
            340             345             350

Asp Tyr Asp Phe Ser Lys Arg Thr Ser Ala Ile Val Ser Gly Ala Trp
        355             360             365

Leu Lys Arg Asn Thr Gly Ile Gly Asn Tyr Thr Gln Ile Asn Ala Ala
    370             375             380

Ser Val Gly Leu Arg His Lys Phe
385             390

<210> 139
<211> 348
<212> PRT
<213> Neisseria meningitidis

<400> 139

```
Met Ser Asp Asp Lys Ser Lys Ala Leu Ala Ala Ala Leu Ala Gln Ile
1               5               10              15

Glu Lys Ser Phe Gly Lys Gly Ala Ile Met Lys Met Asp Gly Ser Gln
            20              25              30

Gln Glu Glu Asn Leu Glu Val Ile Ser Thr Gly Ser Leu Gly Leu Asp
        35              40              45

Leu Ala Leu Gly Val Gly Gly Leu Pro Arg Gly Arg Ile Val Glu Ile
    50              55              60

Phe Gly Pro Glu Ser Ser Gly Lys Thr Thr Leu Cys Leu Glu Ala Val
65              70              75              80

Ala Gln Cys Gln Lys Asn Gly Gly Val Cys Ala Phe Val Asp Ala Glu
            85              90              95

His Ala Phe Asp Pro Val Tyr Ala Arg Lys Leu Gly Val Lys Val Glu
            100             105             110

Glu Leu Tyr Leu Ser Gln Pro Asp Thr Gly Glu Gln Ala Leu Glu Ile
        115             120             125

Cys Asp Thr Leu Val Arg Ser Gly Gly Ile Asp Met Val Val Val Asp
    130             135             140

Ser Val Ala Ala Leu Val Pro Lys Ala Glu Ile Glu Gly Asp Met Gly
145             150             155             160

Asp Ser His Val Gly Leu Gln Ala Arg Leu Met Ser Gln Ala Leu Arg
            165             170             175

Lys Leu Thr Gly His Ile Lys Lys Thr Asn Thr Leu Val Val Phe Ile
        180             185             190

Asn Gln Ile Arg Met Lys Ile Gly Val Met Phe Gly Ser Pro Glu Thr
        195             200             205

Thr Thr Gly Gly Asn Ala Leu Lys Phe Tyr Ser Ser Val Arg Leu Asp
    210             215             220

Ile Arg Arg Thr Gly Ser Ile Lys Lys Gly Glu Glu Val Leu Gly Asn

225             230             235             240

Glu Thr Arg Val Lys Val Ile Lys Asn Lys Val Ala Pro Pro Phe Arg
            245             250             255

Gln Ala Glu Phe Asp Ile Leu Tyr Gly Glu Gly Ile Ser Trp Glu Gly
            260             265             270

Glu Leu Ile Asp Ile Gly Val Lys Asn Asp Ile Ile Asn Lys Ser Gly
        275             280             285

Ala Trp Tyr Ser Tyr Asn Gly Ala Lys Ile Gly Gln Gly Lys Asp Asn
    290             295             300

Val Arg Val Trp Leu Lys Glu Asn Pro Glu Val Ala Asn Glu Ile Asp
305             310             315             320

Ala Lys Ile Arg Ala Leu Asn Gly Val Glu Met His Ile Thr Glu Gly
            325             330             335

Thr Gln Asp Glu Thr Asp Gly Glu Arg Pro Glu Glu
        340             345
```

284

<210> 140
<211> 379
<212> PRT
<213> Neisseria meningitidis

<400> 140

```
Met Gln Asn Ile Phe Asp Pro Leu Val Ile Arg Gly Lys Ser Leu Thr
1               5                   10                  15

Pro Ile Val Gln Gly Gly Met Gly Val Gly Val Ser Ala Ser Gly Leu
            20                  25                  30

Ser Ser Ala Val Ala Arg Glu Asn Gly Ile Gly Thr Ile Ala Ser Val
        35                  40                  45

Asp Leu Arg His Leu His Glu Asp Leu Leu Ala Glu Ser Gln Ile Asn
    50                  55                  60

Pro Ser Glu Glu Lys Tyr Thr Ser Leu Asn Cys Thr Ala Leu Asp Arg
65                  70                  75                  80

Glu Ile Gln Lys Ala Lys Ser Ala Ser Glu Gly Lys Gly Leu Ile Ala
            85                  90                  95

Val Asn Val Met Lys Ala Val Lys Asp His Ala Ala Tyr Val Arg Gln
            100                 105                 110

Ala Cys Glu Ser Gly Ala Asp Ala Val Val Met Gly Ala Gly Leu Pro
        115                 120                 125

Leu Asp Leu Pro Glu Met Thr Glu Gly Tyr His Lys Asp Val Ala Leu
    130                 135                 140

Leu Pro Ile Leu Ser Glu Ser Arg Gly Ile Asn Ile Val Leu Lys Arg
145                 150                 155                 160

Trp Met Lys Lys Gly Ile Leu Pro Asp Ala Ile Val Val Glu His Pro
                165                 170                 175

Ala His Ala Ala Gly His Leu Gly Ala Ser Thr Val Glu Gly Val Asn
            180                 185                 190

Asp Ala Lys Phe Asp Phe Lys Arg Val Ile Glu Glu Thr Phe Glu Val
            195                 200                 205

Phe Lys Ser Leu Gly Leu Glu Ser Glu Lys Ile Pro Leu Ile Leu Ala
    210                 215                 220
```

```
Gly Gly Met Ala Asn Phe Glu Lys Val Lys Thr Ala Leu Lys Asn Trp
225             230             235             240

Gly Ala Ser Ala Val Gln Ile Gly Thr Ala Phe Ala Val Thr Glu Glu
            245             250             255

Gly Asp Ala His Leu Asn Phe Lys Lys Thr Leu Ala Gly Ala Glu Thr
            260             265             270

Glu Lys Val Val Glu Phe Met Ser Val Ala Gly Leu Pro Ala Arg Gly
            275             280             285

Val Arg Thr Lys Phe Leu Asp Ser Tyr Ile Lys Arg Glu Ser Lys Leu
            290             295             300

Gln Thr Asn Ala Lys Ala Asp Pro Arg Arg Cys Thr Gln Gly Leu Asn
305             310             315             320

Cys Leu Thr Ser Cys Gly Leu Arg Asp Gly Leu Ser Lys Ala Gly Gln
            325             330             335

Phe Cys Ile Asp Ile Gln Leu Ala Ala Ala Phe Arg Gly Glu Val Asp
            340             345             350

Lys Gly Leu Phe Phe Arg Gly Lys Asp Arg Cys Pro Ser Ala Met Pro
            355             360             365

Ser Ala Pro Ser Ala Arg Arg Tyr Asn Ile Cys
    370             375
```

<210> 141
<211> 659
<212> PRT
<213> Neisseria meningitidis

<400> 141

```
Met Ser Gln Leu Ala Asn Ala Ile Arg Phe Leu Ser Ala Asp Ala Val
1               5                   10              15

Gln Lys Ala Asn Ser Gly His Pro Gly Ala Pro Met Gly Met Ala Glu
            20              25              30

Met Ala Glu Thr Leu Trp Thr Lys Phe Leu Asn His Asn Pro Ala Asn
        35              40              45

Pro Lys Phe Tyr Asn Arg Asp Arg Phe Val Leu Ser Asn Gly His Ala
        50              55              60

Ser Met Leu Leu Tyr Ser Leu Leu His Leu Thr Gly Tyr Asn Leu Ser
65              70              75              80

Ile Glu Asp Leu Lys Asn Phe Arg Gln Leu His Ser Lys Thr Pro Gly
            85              90              95

His Pro Glu Tyr Gly Tyr Thr Asp Gly Val Glu Thr Thr Thr Gly Pro
        100             105             110

Leu Gly Gln Gly Ile Ala Asn Ala Val Gly Met Ala Leu Ala Glu Lys
        115             120             125

Ile Leu Ala Ala Glu Phe Asn Lys Asp Gly Leu Asn Ile Val Asp His
    130             135             140

Tyr Thr Tyr Val Phe Met Gly Asp Gly Cys Leu Met Glu Gly Val Ser
145             150             155             160

His Glu Ala Cys Ser Leu Ala Gly Thr Leu Gly Leu Gly Lys Leu Ile
            165             170             175
```

```
Val Leu Tyr Asp Asp Asn Asn Ile Ser Ile Asp Gly Lys Val Asp Gly
            180                 185             190

Trp Phe Thr Glu Asn Ile Pro Gln Arg Phe Glu Ser Tyr Gly Trp His
        195                 200             205

Val Val Pro Asn Val Asn Gly His Asp Thr Ala Ala Ile Gln Ala Ala
    210                 215                 220

Ile Glu Ala Ala Arg Ala Glu Thr Gly Lys Pro Ser Ile Ile Cys Cys
225                 230                 235                 240

Lys Thr Leu Ile Gly Lys Gly Ser Ala Asn Lys Glu Gly Ser His Lys
                245                 250                 255

Thr His Gly Ala Pro Leu Gly Ala Asp Glu Ile Glu Ala Thr Arg Lys
            260                 265                 270

His Leu Gly Trp Thr Tyr Pro Ala Phe Glu Ile Pro Gln Glu Ile Tyr
        275                 280                 285

Asp Ala Trp Asn Ala Lys Glu Gln Gly Ala Lys Leu Glu Ala Asp Trp
    290                 295                 300

Asn Glu Leu Phe Ala Gln Tyr Gln Ala Lys Tyr Pro Ala Glu Ala Ala
305                 310                 315                 320

Glu Phe Val Arg Arg Met Asp Lys Lys Leu Pro Asp Asn Phe Asp Glu
                325                 330                 335

Tyr Val Gln Ala Ala Leu Lys Glu Val Cys Ala Lys Ala Glu Thr Ile
            340                 345                 350

Ala Thr Arg Lys Ala Ser Gln Asn Ser Ile Glu Ile Leu Ala Lys Glu
        355                 360                 365

Leu Pro Glu Leu Val Gly Gly Ser Ala Asp Leu Thr Pro Ser Asn Leu
    370                 375                 380

Thr Asp Trp Ser Asn Ser Val Ser Val Thr Arg Asp Lys Gly Gly Asn
385                 390                 395                 400

Tyr Ile His Tyr Gly Val Arg Glu Phe Gly Met Gly Ala Ile Met Asn
            405                 410                 415

Gly Leu Val Leu His Gly Gly Val Lys Pro Phe Gly Ala Thr Phe Leu
            420                 425                 430

Met Phe Ser Glu Tyr Glu Arg Asn Ala Leu Arg Met Ala Ala Leu Met
        435                 440                 445

Lys Ile Asn Pro Val Phe Val Phe Thr His Asp Ser Ile Gly Leu Gly
    450                 455                 460

Glu Asp Gly Pro Thr His Gln Pro Ile Glu Gln Thr Ala Thr Leu Arg
465                 470                 475                 480

Leu Ile Pro Asn Met Asp Val Trp Arg Pro Cys Asp Thr Ala Glu Ser
            485                 490                 495

Leu Val Ala Trp Ala Glu Ala Val Lys Ala Ala Asp His Pro Ser Cys
        500                 505                 510

Leu Ile Phe Ser Arg Gln Asn Leu Lys Phe Gln Ala Arg Ser Glu Gln
    515                 520                 525

Gln Leu Asn Asp Ile Lys Arg Gly Gly Tyr Val Ile Ser Glu Ala Gln
    530                 535                 540

Gly Asn Ala Gln Ala Val Ile Ile Ala Thr Gly Ser Glu Val Glu Leu
```

```
       545                    550                  555                   560
       Ala Leu Glu Ala Gln Lys Ala Leu Ala Ala Gln Asn Ile Ala Val Arg
                       565                   570                   575

       Val Val Ser Met Pro Ser Thr Asn Val Phe Asp Arg Gln Asp Ala Ala
                       580                   585                   590

       Tyr Gln Ala Ala Val Leu Pro Glu Gly Leu Pro Arg Ile Ala Val Glu
                   595                   600                   605

       Ala Gly His Ala Asp Gly Trp Tyr Lys Tyr Val Gly Leu Asn Gly Ala
                   610                   615                   620

       Val Val Gly Ile Asn Arg Phe Gly Glu Ser Ala Pro Ala Asp Leu Leu
       625                   630                   635               640

       Phe Lys Ala Phe Gly Phe Thr Val Asp Asn Val Val Asp Thr Val Lys
                       645                   650                   655

       Ser Val Leu
```

<210> 142
<211> 336
<212> PRT
<213> Neisseria meningitidis

<400> 142

```
Met Ser Lys Lys Arg Val Leu Thr Gly Val Thr Thr Thr Gly Ile Pro
1               5                   10                  15

His Leu Gly Asn Tyr Val Gly Ala Ile Arg Pro Ala Val Arg Ala Ala
            20                  25                  30

Gln Asn Leu Asp Thr Glu Ser Phe Leu Phe Leu Ala Asp Tyr His Gly
        35                  40                  45

Ile Ile Lys Cys His Glu Pro Glu Met Ile His Gln Ser Thr Gln Ala
        50                  55                  60

Val Ala Ala Thr Trp Leu Ala Cys Gly Leu Asp Pro Glu Arg Thr Thr
65              70                  75                  80

Phe Tyr Arg Gln Ser Asp Thr Pro Glu Val Met Glu Leu Asn Trp Ile
            85                  90                  95

Leu Thr Cys Ile Thr Ala Lys Gly Leu Met Asn Arg Ala His Ala Tyr
            100                 105                 110

Lys Ala Ala Val Gln Ala Asn Ala Glu Asn Gly Gln Glu Asp Pro Asp
        115                 120                 125

Phe Gly Val Glu Met Gly Leu Phe Ser Tyr Pro Ile Leu Met Thr Ala
    130                 135                 140

Asp Ile Leu Met Phe Asn Ala Asn Glu Val Pro Val Gly Arg Asp Gln
145                 150                 155                 160

Ile Gln His Val Glu Met Ala Arg Asp Ile Ala Gly Arg Phe Asn His
            165                 170                 175

Arg Phe Arg Glu Leu Phe Thr Leu Pro Glu Val Lys Ile Asp Glu Asn
            180                 185                 190

Val Glu Leu Leu Val Gly Leu Asp Gly Arg Lys Met Ser Lys Ser Tyr
            195                 200                 205

Gly Asn Thr Ile Pro Leu Trp Glu Asn Asp Lys Lys Thr Gln Lys Ser
    210                 215                 220

Val Asn Lys Ile Ile Thr Asn Met Lys Glu Pro Gly Glu Pro Lys Gln
225                 230                 235                 240

Pro Asp Glu Ser Pro Leu Phe Glu Ile Tyr Lys Ala Phe Ser Thr Pro
            245                 250                 255

Ser Glu Thr Val Glu Phe Thr Lys Met Leu Ala Asp Gly Leu Ala Trp
            260                 265                 270

Gly Glu Ala Lys Lys Leu Leu Ala Ala Lys Ile Asn Ala Glu Leu Ala
        275                 280                 285

Glu Pro Arg Glu Arg Tyr Asn Glu Leu Thr Ala Asp Pro Ser Gln Ile
    290                 295                 300

Glu Glu Ile Leu Gln Ala Gly Ala Ala Lys Ala Arg Lys Glu Ala Arg
305                 310                 315                 320

Glu Leu Leu Asp Lys Val Arg Asp Ala Val Gly Ile Arg Pro Leu Lys
            325                 330                 335
```

<210> 143
<211> 859
<212> PRT

<213> Neisseria meningitidis

<400> 143

```
Met Arg Tyr Asp Lys Leu Thr Ala Lys Phe Gln Gln Ala Leu Ala Glu
1               5                   10              15
Ala Gln Ser Leu Ala Leu Ala Ala Asp Gly Ser Tyr Leu Glu Ala Gly
            20                  25                  30
Phe Val Leu Lys Ala Leu Leu Asp Asp Gln Asn Ser Gly Ala Ala Ala
            35                  40                  45
Leu Leu Ala His Ala Gly Val Asn Val Pro Gln Val Lys Gln Arg Leu
        50                  55                  60
Gln Gln His Leu Asn Ser Leu Pro Lys Val Ser Gly Gln Gly Gly Asp
65                  70                  75                  80
Ile Leu Pro Ser Arg Glu Leu Gln Ala Val Leu Asn Leu Met Asp Lys
                85                  90                  95
Ala Ala Thr Lys Arg Ser Asp Ala Tyr Ile Ala Ser Glu Leu Phe Leu
            100                 105                 110
Leu Ala Leu Val Gln Gln Asn Asp Ala Thr Gly Lys Ile Leu Lys Glu
            115                 120                 125
Ala Gly Ala Thr Glu Gln Asn Ile Asn Ala Ala Ile Asp Ala Val Arg
    130                 135                 140
Gly Gly Gln Asn Val Asn Asp Ala Asn Ala Glu Asp Gln Arg Asp Ala
145                 150                 155                 160
Leu Lys Lys Tyr Thr Leu Asp Leu Thr Gln Arg Ala Arg Asp Gly Lys
                165                 170                 175
Leu Asp Pro Val Ile Gly Arg Asp Asp Glu Ile Arg Arg Ala Ile Gln
                180                 185                 190
Val Leu Gln Arg Arg Thr Lys Asn Asn Pro Val Leu Ile Gly Glu Pro
            195                 200                 205
Gly Val Gly Lys Thr Ala Ile Val Glu Gly Leu Ala Gln Arg Ile Val
    210                 215                 220
```

```
Asn Gly Glu Val Pro Glu Ser Leu Arg Asn Lys Arg Leu Leu Val Leu
225             230             235             240

Asp Leu Ala Ala Leu Ile Ala Gly Ala Lys Tyr Arg Gly Glu Phe Glu
            245             250             255

Glu Arg Leu Lys Gly Val Leu Asn Asp Leu Ala Lys Asp Asp Gly Asn
            260             265             270

Thr Leu Ile Phe Ile Asp Glu Ile His Thr Leu Val Gly Ala Gly Lys
            275             280             285

Thr Asp Gly Ala Met Asp Ala Gly Asn Met Leu Lys Pro Ala Leu Ala
    290             295             300

Arg Gly Glu Leu His Cys Ile Gly Ala Thr Thr Leu Asp Glu Tyr Arg
305             310             315             320

Gln Tyr Ile Glu Lys Asp Ala Ala Leu Glu Arg Arg Phe Gln Lys Val
            325             330             335

Leu Val Gly Glu Pro Ser Val Glu Asp Thr Ile Ala Ile Leu Arg Gly
            340             345             350

Leu Gln Glu Arg Tyr Glu Ile His His Gly. Ile Asp Ile Thr Asp Pro
    355             360             365

Ala Ile Val Ala Ala Ala Glu Leu Ser Asp Arg Tyr Ile Thr Asp Arg
370             375             380

Phe Leu Pro Asp Lys Ala Ile Asp Leu Ile Asp Glu Ala Ala Ser Arg
385             390             395             400

Val Lys Met Glu Lys Glu Thr Lys Pro Glu Ala Met Asp Lys Ile Asp
            405             410             415

Arg Arg Leu Ile Gln Leu Arg Met Glu Lys Ala His Val Glu Lys Glu
            420             425             430

Lys Asp Asp Ala Ser Lys Lys Arg Leu Glu Leu Ile Asp Glu Glu Ile
    435             440             445

Asn Gly Leu Gln Lys Glu Tyr Ala Asp Leu Asp Glu Ile Trp Lys Ala
    450             455             460

Glu Lys Ala Ile Ser Asp Gly Ala Ala Asn Ile Lys Lys Gln Ile Asp
465             470             475             480

Glu Val Lys Ile Lys Ile Glu Gln Ala Lys Arg Gln Gly Asp Leu Ala
            485             490             495

Leu Ala Ser Lys Leu Met Tyr Glu Asp Leu Glu His Leu Glu Lys Gln
            500             505             510

Arg Ala Ala Ala Glu Arg Ala Asp Thr Asp Ser Thr Lys Pro Ala Asn
    515             520             525

Lys Leu Leu Arg Asn Asn Val Gly Ala Glu Glu Ile Ala Glu Val Val
    530             535             540

Ser Arg Met Thr Gly Ile Pro Val Ser Lys Met Met Glu Gly Glu Arg
545             550             555             560

Asp Lys Leu Leu Lys Met Glu Glu Val Leu His Arg Arg Val Val Gly
            565             570             575

Gln Asp Glu Ala Val Arg Ala Val Ser Asp Ala Ile Arg Arg Ser Arg
            580             585             590
```

```
        Ser Gly Leu Ala Asp Pro Asn Lys Pro Tyr Gly Ser Phe Leu Phe Leu
                595             600             605

        Gly Pro Thr Gly Val Gly Lys Thr Glu Leu Cys Lys Ala Leu Ala Gly
            610             615             620

        Phe Leu Phe Asp Ser Glu Asp His Leu Ile Arg Ile Asp Met Ser Glu
        625             630             635             640

        Tyr Met Glu Lys His Ala Val Ala Arg Leu Ile Gly Ala Pro Pro Gly
                        645             650             655

        Tyr Val Gly Tyr Glu Glu Gly Gly Tyr Leu Thr Glu Gln Val Arg Arg
                    660             665             670

        Lys Pro Tyr Ser Val Ile Leu Leu Asp Glu Val Glu Lys Ala His Pro
                    675             680             685

        Asp Val Phe Asn Ile Leu Leu Gln Val Leu Asp Asp Gly Arg Leu Thr
            690             695             700

        Asp Gly Gln Gly Arg Thr Val Asp Phe Lys Asn Thr Val Ile Val Met
        705             710             715             720

        Thr Ser Asn Ile Gly Ser Gln His Ile Gln Gln Met Gly Ile Gln Asp
                    725             730             735

        Tyr Glu Ala Val Lys Glu Val Val Met Glu Asp Val Lys Glu His Phe
                    740             745             750

        Arg Pro Glu Met Ile Asn Arg Ile Asp Glu Val Val Val Phe His Gly
                    755             760             765

        Leu Asp Gln Asp Asn Ile Arg Asn Ile Ala Lys Ile Gln Leu Lys Gly
            770             775             780

        Leu Glu Lys Arg Leu Glu Lys Gln Asn Leu Arg Leu Ala Val Ser Asp
        785             790             795             800

        Ala Ala Leu Asp Ile Ile Ala Lys Ala Gly Phe Asp Pro Ile Tyr Gly
                        805             810             815

        Ala Arg Pro Leu Lys Arg Ala Ile Gln Ser Glu Ile Glu Asn Pro Leu
                    820             825             830

        Ala Lys Ala Leu Leu Ala Gly Asn Tyr Ala Pro Glu Ser Glu Ile Arg
                    835             840             845

        Val Glu Ala Asp Gly Asp Arg Leu Lys Phe Ala
            850             855
```

<210> 144
<211> 572
<212> PRT
<213> Neisseria meningitidis

<400> 144

```
Met Asn Leu His Gln Thr Val Glu His Glu Ala Ala Ala Ala Phe Ala
1               5               10              15

Ala Ala Gly Ile Ala Asp Ser Pro Ile Val Leu Gln Pro Thr Lys Asn
            20              25              30

Ala Glu His Gly Asp Phe Gln Ile Asn Gly Val Met Gly Ala Ala Lys
        35              40              45

Lys Ala Lys Gln Asn Pro Arg Glu Leu Ala Gln Lys Val Ala Glu Ala
    50              55              60

Leu Ala Asp Asn Ala Val Ile Glu Ser Ala Glu Val Ala Gly Pro Gly
```

```
    65                    70                    75                    80
    Phe Ile Asn Leu Arg Leu Arg Pro Glu Phe Leu Ala Gln Asn Ile Gln
                    85                    90                    95
    Thr Ala Leu Asn Asp Ala Arg Phe Gly Val Ala Lys Thr Asp Lys Pro
                100                   105                   110
    Gln Thr Val Val Ile Asp Tyr Ser Ser Pro Asn Leu Ala Lys Glu Met
                115                   120                   125
    His Val Gly His Leu Arg Ser Ser Ile Ile Gly Asp Ser Ile Ser Arg
        130                   135                   140
    Val Leu Ala Phe Met Gly Asn Thr Val Ile Arg Gln Asn His Val Gly
    145                   150                   155                   160
    Asp Trp Gly Thr Gln Phe Gly Met Leu Val Ala Tyr Leu Val Glu Gln
                    165                   170                   175
    Gln Lys Asp Asn Ala Ala Phe Glu Leu Ala Asp Leu Glu Gln Phe Tyr
                180                   185                   190
    Arg Ala Ala Lys Val Arg Phe Asp Glu Asp Pro Ala Phe Ala Asp Thr
                195                   200                   205
    Ala Arg Glu Tyr Val Val Lys Leu Gln Gly Gly Asp Glu Thr Val Leu
        210                   215                   220
    Ala Leu Trp Lys Gln Phe Val Asp Ile Ser Leu Ser His Ala Gln Ala
    225                   230                   235                   240
    Val Tyr Asp Thr Leu Gly Leu Lys Leu Arg Pro Glu Asp Val Ala Gly
                    245                   250                   255
    Glu Ser Lys Tyr Asn Asp Asp Leu Gln Pro Val Val Asp Asp Leu Val
                260                   265                   270
    Gln Lys Gly Leu Ala Val Glu Asp Asp Gly Ala Lys Val Val Phe Leu
                275                   280                   285
    Asp Glu Phe Lys Asn Lys Glu Gly Glu Pro Ala Ala Phe Ile Val Gln
                290                   295                   300
    Lys Gln Gly Gly Gly Phe Leu Tyr Ala Ser Thr Asp Leu Ala Cys Leu
    305                   310                   315                   320
    Arg Tyr Arg Ile Gly Arg Leu Lys Ala Asp Arg Leu Leu Tyr Val Val
                    325                   330                   335
    Asp His Arg Gln Ala Leu His Phe Glu Gln Leu Phe Thr Thr Ser Arg
                340                   345                   350
    Lys Ala Gly Tyr Leu Pro Glu Asn Val Gly Ala Ala Phe Ile Gly Phe
                355                   360                   365
    Gly Thr Met Met Gly Lys Asp Gly Lys Pro Phe Lys Thr Arg Ser Gly
        370                   375                   380
    Asp Thr Val Lys Leu Val Asp Leu Leu Thr Glu Ala Val Glu Arg Ala
    385                   390                   395                   400
    Thr Ala Leu Val Lys Glu Lys Asn Pro Glu Leu Gly Ala Asp Glu Ala
                    405                   410                   415
    Ala Lys Ile Gly Lys Thr Val Gly Ile Gly Ala Val Lys Tyr Ala Asp
                420                   425                   430
    Leu Ser Lys Asn Arg Thr Ser Asp Tyr Val Phe Asp Trp Asp Ala Met
                435                   440                   445
```

```
Leu Ser Phe Glu Gly Asn Thr Ala Pro Tyr Leu Gln Tyr Ala Tyr Thr
    450             455         460

Arg Val Gln Ser Val Phe Arg Lys Ala Gly Glu Trp Asp Ala Asn Ala
465             470         475                 480

Pro Thr Val Leu Thr Glu Pro Leu Glu Lys Gln Leu Ala Ala Glu Leu
            485             490             495

Leu Lys Phe Glu Asp Val Leu Gln Ser Val Ala Asp Thr Ala Tyr Pro
        500             505             510

His Tyr Leu Ala Ala Tyr Leu Tyr Gln Ile Ala Thr Leu Phe Ser Arg
        515             520             525

Phe Tyr Glu Ala Cys Pro Ile Leu Lys Ala Glu Gly Ala Ser Arg Asn
        530             535             540

Ser Arg Leu Gln Leu Ala Lys Leu Thr Gly Asp Thr Leu Lys Gln Gly
545             550             555             560

Leu Asp Leu Leu Gly Ile Asp Val Leu Asp Val Met
            565             570
```

<210> 145
<211> 398
<212> PRT
<213> Neisseria meningitidis

<400> 145

```
Met Ser Gln Lys Leu Ile Leu Val Leu Asn Cys Gly Ser Ser Ser Leu
1               5                   10              15
Lys Gly Ala Val Leu Asp Asn Gly Ser Gly Glu Val Leu Leu Ser Cys
            20              25              30
Leu Ala Glu Lys Leu Asn Leu Pro Asp Ala Tyr Ile Thr Phe Lys Val
        35              40              45
Asn Gly Glu Lys His Lys Val Asp Leu Ser Ala His Pro Asp His Thr
    50              55              60
Gly Ala Val Glu Ala Leu Met Glu Glu Leu Lys Ala His Gly Leu Asp
65              70              75              80
Ser Arg Ile Gly Ala Ile Gly His Arg Val Val Ser Gly Gly Glu Leu
            85              90              95
Tyr Ser Glu Ser Ile Leu Val Asp Asp Glu Val Ile Ala Gly Ile Glu
        100             105             110
Lys Cys Ile Pro Leu Ala Pro Leu His Asn Pro Ala His Leu Leu Gly
    115             120             125
Leu Arg Ala Ala Gln Ser Ile Phe Lys Gly Leu Pro Asn Val Val Val
    130             135             140
Phe Asp Thr Ser Phe His Gln Thr Met Pro Glu Val Ala Tyr Lys Tyr
145             150             155             160
Ala Val Pro Gln Glu Leu Tyr Glu Lys Tyr Gly Leu Arg Arg Tyr Gly
            165             170             175
Ala His Gly Thr Ser Tyr Arg Phe Val Ala Asp Glu Thr Ala Arg Phe
        180             185             190
Leu Gly Lys Asp Lys Lys Asp Leu Arg Met Val Ile Ala His Leu Gly
    195             200             205
```

```
Asn Gly Ala Ser Ile Thr Ala Val Ala Asn Gly Glu Ser Arg Asp Thr
    210             215             220

Ser Met Gly Leu Thr Pro Leu Glu Gly Leu Val Met Gly Thr Arg Ser
225             230             235             240

Gly Asp Ile Asp Pro Ser Val Phe Gly Phe Leu Ala Glu Asn Ala Asn
            245             250             255

Met Thr Ile Ala Gln Ile Thr Glu Met Leu Asn Lys Lys Ser Gly Leu
            260             265             270

Leu Gly Ile Ser Gly Leu Ser Asn Asp Cys Arg Thr Ile Glu Glu Glu
        275             280             285

Ala Ala Lys Gly His Lys Gly Ala Lys Leu Ala Leu Asp Met Phe Ile
    290             295             300

Tyr Arg Leu Ala Lys Tyr Ile Gly Ser Met Ala Val Ala Ala Gly Gly
305             310             315             320

Leu Asp Ala Leu Val Phe Thr Gly Gly Ile Gly Glu Asn Ser Asp Ile
            325             330             335

Ile Arg Glu Arg Val Ile Gly Tyr Leu Gly Phe Leu Gly Leu Asn Ile
        340             345             350

Asp Gln Glu Ala Asn Leu Lys Ala Arg Phe Gly Asn Ala Gly Val Ile
        355             360             365

Thr Thr Ala Asp Ser Lys Ala Val Ala Val Val Ile Pro Thr Asn Glu
370             375             380

Glu Leu Met Ile Ala His Asp Thr Ala Arg Leu Ser Gly Leu
385             390             395
```

<210> 146
<211> 183
<212> PRT
<213> Neisseria meningitidis

<400> 146

EP 2 279 746 B1

```
Met Lys Ala Tyr Leu Ala Leu Ile Ser Ala Ala Val Ile Gly Leu Ala
1               5               10              15

Ala Cys Ser Gln Glu Pro Ala Ala Pro Ala Ala Glu Ala Thr Pro Ala
            20              25              30

Ala Glu Ala Pro Ala Ser Glu Ala Pro Ala Ala Glu Ala Ala Pro Ala
        35              40              45

Asp Ala Ala Glu Ala Pro Ala Ala Gly Asn Cys Ala Ala Thr Val Glu
        50              55              60

Ser Asn Asp Asn Met Gln Phe Asn Thr Lys Asp Ile Gln Val Ser Lys
65              70              75              80

Ala Cys Lys Glu Phe Thr Ile Thr Leu Lys His Thr Gly Thr Gln Pro
            85              90              95

Lys Ala Ser Met Gly His Asn Leu Val Ile Ala Lys Ala Glu Asp Met
            100             105             110

Asp Gly Val Phe Lys Asp Gly Val Gly Ala Ala Asp Thr Asp Tyr Val
            115             120             125

Lys Pro Asp Asp Ala Arg Val Val Ala His Thr Lys Leu Ile Gly Gly
    130             135             140

Gly Glu Glu Ala Ser Leu Thr Leu Asp Pro Ala Lys Leu Ala Asp Gly

145             150             155             160

Glu Tyr Lys Phe Ala Cys Thr Phe Pro Gly His Gly Ala Leu Met Asn
            165             170             175

Gly Lys Val Thr Leu Val Asp
            180
```

<210> 147
<211> 916
<212> PRT
<213> Neisseria meningitidis

<400> 147

Met Leu Thr Asn Ile Ala Lys Lys Ile Phe Gly Ser Arg Asn Asp Arg
1               5                   10                  15

Leu Leu Lys Gln Tyr Arg Lys Ser Val Ala Arg Ile Asn Ala Leu Glu
            20              25              30

Glu Gln Met Gln Ala Leu Ser Asp Ala Asp Leu Gln Ala Lys Thr Ala
        35              40              45

Glu Phe Lys Gln Arg Leu Ala Asp Gly Gln Thr Leu Asp Gly Ile Leu
    50              55              60

Pro Glu Ala Phe Ala Val Cys Arg Glu Ala Ser Arg Arg Thr Leu Gly
65              70              75              80

Met Arg His Phe Asp Val Gln Leu Ile Gly Gly Met Val Leu His Asp
            85              90              95

Gly Lys Ile Ala Glu Met Arg Thr Gly Glu Gly Lys Thr Leu Val Ala
        100             105             110

Thr Leu Ala Val Tyr Leu Asn Ala Leu Ala Gly Lys Gly Val His Val
    115             120             125

Val Thr Val Asn Asp Tyr Leu Ala Ser Arg Asp Ala Gly Ile Met Glu
    130             135             140

Pro Leu Tyr Asn Phe Leu Gly Leu Thr Val Gly Val Ile Ile Ser Asp
145             150             155             160

Met Gln Pro Phe Asp Arg Gln Asn Ala Tyr Ala Ala Asp Ile Thr Tyr
            165             170             175

Gly Thr Asn Asn Glu Phe Gly Phe Asp Tyr Leu Arg Asp Asn Met Val
        180             185             190

Thr Asp Gln Tyr Asp Lys Val Gln Arg Glu Leu Asn Phe Ala Val Val
    195             200             205

Asp Glu Val Asp Ser Ile Leu Ile Asp Glu Ala Arg Thr Pro Leu Ile
    210             215             220

Ile Ser Gly Gln Ala Asp Asp Asn Ile Gln Leu Tyr Gln Ile Met Asn
225             230             235             240

Thr Val Pro Pro His Leu Val Arg Gln Glu Thr Glu Glu Gly Glu Gly
            245             250             255

Asp Tyr Trp Val Asp Glu Lys Ala His Gln Val Ile Leu Ser Glu Ala
            260             265             270

Gly His Glu His Ala Glu Gln Ile Leu Thr Gln Met Gly Leu Leu Ala
    275             280             285

Glu Asn Asp Ser Leu Tyr Ser Ala Ala Asn Ile Ala Leu Met His His
    290             295             300

```
Leu Met Ala Ala Leu Arg Ala His Ser Leu Phe His Lys Asp Gln His
305                 310             315                 320

Tyr Val Ile Gln Asp Gly Glu Ile Val Ile Val Asp Glu Phe Thr Gly
                325             330                 335

Arg Leu Met Ser Gly Arg Arg Trp Ser Glu Gly Leu His Gln Ala Val
                340             345                 350

Glu Ala Lys Glu Gly Val Glu Ile Lys Arg Glu Asn Gln Thr Leu Ala
                355             360                 365

Ser Ile Thr Phe Gln Asn Tyr Phe Arg Leu Tyr Thr Lys Leu Ser Gly
    370             375             380

Met Thr Gly Thr Ala Asp Thr Glu Ala Phe Glu Phe Gln Ser Ile Tyr
385             390             395                 400

Asn Leu Glu Thr Val Ile Ile Pro Thr Asn Arg Pro Val Gln Arg Lys
                405             410                 415

Asp Phe Asn Asp Gln Ile Phe Arg Ser Ala Glu Glu Lys Phe Glu Ala
                420             425             430

Val Val Lys Asp Ile Glu Glu Cys His Lys Arg Gly Gln Pro Val Leu
        435             440             445

Val Gly Thr Thr Ser Ile Glu Asn Ser Glu Leu Val Ser Lys Leu Leu
    450             455             460

Thr Gln Ala Gly Leu Pro His Asn Val Leu Asn Ala Lys Glu His Glu
465             470             475                 480

Arg Glu Ala Leu Ile Val Ala Gln Ala Gly Lys Val Gly Ala Ile Thr
                485             490             495

Val Ala Thr Asn Met Ala Gly Arg Gly Thr Asp Ile Val Leu Gly Gly
    500             505             510

Asn Leu Lys His Gln Thr Asp Ala Ile Arg Ala Asp Glu Thr Leu Ser
    515             520             525

Asp Glu Glu Lys Gln Ala Gln Ile Ala Ala Leu Glu Asp Gly Trp Gln
    530             535             540

Ala Glu His Asp Lys Val Met Glu Ala Gly Gly Leu His Ile Ile Gly
545             550             555                 560

Thr Glu Arg His Glu Ser Arg Arg Ile Asp Asn Gln Leu Arg Gly Arg
                565             570             575

Ser Gly Arg Gln Gly Asp Pro Gly Ser Ser Arg Phe Tyr Leu Ser Phe
                580             585             590

Glu Asp Pro Leu Leu Arg Leu Phe Ala Leu Asp Arg Ala Ala Ala Ile
                595             600             605

Leu Asn Arg Leu Ala Pro Glu Arg Gly Val Ala Ile Glu His Asn Leu
    610             615             620

Leu Thr Arg Gln Ile Glu Gly Ala Gln Arg Lys Val Glu Gly Arg Asn
625             630             635                 640

Phe Asp Met Arg Lys Gln Val Leu Glu Tyr Asp Asp Val Ala Asn Glu
                645             650             655

Gln Arg Lys Val Ile Tyr Ser Gln Arg Asn Glu Ile Leu Thr Ser Lys
                660             665             670
```

```
Asp Ile Ser Asp Leu Met Gln Glu Ile Arg Ser Asp Val Val Ser Asp
        675             680             685

Leu Val Asp Thr Tyr Met Pro Pro Asp Ser Met Glu Glu Gln Trp Asp
        690             695             700

Ile Pro Thr Leu Glu Asn Arg Leu Ala Ala Glu Phe Arg Leu His Glu
705             710             715             720

Asp Ile Gln Ser Trp Leu Lys Ala Asp Asn Ala Ile Asp Gly Gln Asp
            725             730             735

Ile Lys Glu Arg Leu Ile Glu Arg Ile Glu Asn Glu Tyr Ala Ala Lys
        740             745             750

Thr Glu Leu Val Gly Lys Gln Ala Met Ala Asp Phe Glu Arg Asn Val
        755             760             765

Met Leu Gln Val Ile Asp Asn Gln Trp Arg Glu His Leu Ala Ala Met
    770             775             780

Asp Tyr Leu Arg Gln Gly Ile His Leu Arg Ser Tyr Ala Gln Lys Asn
785             790             795             800

Pro Lys Gln Glu Tyr Lys Arg Glu Ala Phe Thr Met Phe Gln Asp Leu
            805             810             815

Trp Asn Gly Ile Lys Phe His Ile Ala Ser Leu Leu Thr Ser Val Gln
            820             825             830

Ile Glu Gln Asn Pro Val Ala Val Val Glu Glu Gln Pro Ile Gly Asn
        835             840             845

Ile Gln Ser Ile His Ser Glu Ser Pro Asp Met Glu Glu Leu Leu Gly
    850             855             860

Gln Ser Gln Thr Asp Leu Val Thr Glu Ala Phe Asn Pro Asp Gly Thr
865             870             875             880

Asp Phe Ser Pro Glu Ala Leu Glu Ala Arg Gly Gln Ile Val His Arg
            885             890             895

Asn Asp Pro Cys Pro Cys Gly Ser Gly Leu Lys Tyr Lys Gln Cys His
            900             905             910

Gly Lys Leu Ala
            915
```

<210> 148
<211> 562
<212> PRT
<213> Neisseria meningitidis

<400> 148

Met Leu Asn Lys Asp Gln Phe Ala Asp Asn His Phe Ile Arg Thr Ile
1               5                   10                  15

Ile Glu Glu Asp Leu Glu Ser Gly Lys His Thr Ala Val Gln Thr Arg
            20                  25                  30

Phe Pro Pro Glu Pro Asn Gly Tyr Leu His Ile Gly His Ala Lys Ser
        35                  40                  45

Ile Cys Leu Asn Phe Gly Leu Ala Tyr Ile Tyr Asp Gly Leu Cys Asn
        50                  55                  60

Leu Arg Phe Asp Asp Thr Asn Pro Glu Lys Glu Asn Asp Glu Tyr Val
65                  70                  75                  80

Asn Ala Ile Lys Glu Asp Val Glu Trp Leu Gly Phe His Trp Ala Gly

<pre>
                        85                        90                        95
        Glu Pro Arg Phe Ala Ser Asn Tyr Phe Asp Gln Leu Tyr Asp Tyr Ala
                    100                 105                 110
        Val Gly Leu Ile Lys Asp Gly Lys Ala Tyr Val Asp Asp Leu Thr Pro
                    115                 120                 125
        Glu Glu Met Arg Glu Tyr Arg Gly Thr Leu Thr Glu Ala Gly Lys Asn
                    130                 135                 140
        Ser Pro Tyr Arg Asp Arg Ser Val Glu Glu Asn Leu Asp Leu Phe Thr
        145                 150                 155                 160
        Arg Met Lys Asn Gly Glu Phe Pro Asp Gly Ser Lys Thr Leu Arg Leu
                        165                 170                 175
        Lys Ile Asp Met Ala Ser Gly Asn Ile Asn Met Arg Asp Pro Val Ile
                    180                 185                 190
        Tyr Arg Ile Arg Arg Ala His His His Asn Thr Gly Asp Lys Trp Cys
                    195                 200                 205
        Ile Tyr Pro Met Tyr Asp Tyr Thr His Cys Ile Ser Asp Ala Ile Glu
            210                 215                 220
        Gly Ile Thr His Ser Leu Cys Thr Leu Glu Phe Glu Ala His Arg Pro
        225                 230                 235                 240
        Leu Tyr Asp Cys Val Leu Asp Asn Ile Pro Ala Pro His Ala Thr Arg
                        245                 250                 255
        Pro Arg Gln Tyr Glu Phe Ser Arg Leu Glu Leu Leu Tyr Thr Ile Thr
                    260                 265                 270
        Ser Lys Arg Lys Leu Asn Gln Leu Val Val Glu Lys His Val Ser Gly
                    275                 280                 285
        Trp Asp Asp Pro Arg Met Pro Thr Ile Ser Gly Met Arg Arg Arg Gly
            290                 295                 300
        Tyr Thr Pro Glu Gly Leu Arg Leu Phe Ala Lys Arg Ala Gly Ile Ser
        305                 310                 315                 320
        Lys Ser Glu Asn Ile Val Asp Met Ser Val Leu Glu Gly Ala Ile Arg
                        325                 330                 335
        Glu Glu Leu Glu Asn Ser Ala Pro Arg Leu Met Ala Val Leu Asn Pro
                    340                 345                 350
        Leu Lys Val Thr Leu Thr Asn Phe Glu Thr Gly Arg Thr Gln Ser Arg
                    355                 360                 365
        Arg Ala Ala Phe His Pro Asn His Glu Glu Met Gly Glu Arg Glu Val
            370                 375                 380
        Pro Ile Ser Gln Thr Ile Tyr Ile Glu Ala Asp Asp Phe Ala Glu Asn
        385                 390                 395                 400
        Pro Pro Lys Gly Phe Lys Arg Leu Ile Pro Gly Gly Glu Val Arg Leu
                        405                 410                 415
        Arg His Gly Tyr Val Ile Lys Cys Asp Glu Val Val Lys Asp Glu Ala
                    420                 425                 430
        Gly Asn Val Val Glu Leu Lys Cys Ser Ile Asp His Asp Thr Leu Gly
                    435                 440                 445
        Lys Asn Pro Glu Gly Arg Lys Val Lys Gly Val Ile His Trp Val Ser
            450                 455                 460
</pre>

304

```
Ala Glu His Ala Ala Glu Ile Lys Val Arg Leu Tyr Asp Arg Leu Phe
465             470             475                 480

Thr Val Glu Arg Pro Asp Ala Val Arg Gly Glu Asp Gly Glu Tyr Leu
            485             490                 495

Pro Phe Thr Asp Phe Leu Asn Pro Glu Ser Val Lys Glu Ile Thr Ala
        500             505                 510

Tyr Ala Glu Pro Ala Ala Lys Asp Leu Pro Ala Glu Ser Arg Trp Gln
        515             520                 525

Phe Glu Arg Ile Gly Tyr Phe Val Thr Asp Arg Lys Asp His Gly Lys
    530             535                 540

Asp Thr Pro Val Phe Asn Arg Thr Val Thr Leu Lys Asp Ser Trp Gln
545             550                 555                 560

Pro Lys
```

<210> 149
<211> 861
<212> PRT
<213> Neisseria meningitidis

<400> 149

```
Met Leu Glu Ala Tyr Arg Lys Ala Ala Ala Glu Arg Ala Ala Leu Gly
1               5                   10                  15

Ile Pro Ala Leu Pro Leu Asn Ala Gln Gln Thr Ala Asp Leu Val Glu
            20                  25                  30

Leu Leu Lys Ser Pro Pro Ala Gly Glu Gly Glu Phe Leu Val Glu Leu
        35                  40                  45

Leu Ala His Arg Val Pro Pro Gly Val Asp Asp Ala Ala Lys Val Lys
    50                  55                  60

Ala Ser Phe Leu Ala Ala Val Ala Glu Gly Ser Ala Ser Ser Pro Leu
65                  70                  75                  80

Ile Ser Pro Glu Tyr Ala Thr Glu Leu Leu Gly Thr Met Leu Gly Gly
            85                  90                  95

Tyr Asn Ile His Ala Leu Ile Glu Leu Leu Asp Asp Asp Lys Leu Ala
            100                 105                 110

Ser Ile Ala Ala Lys Gly Leu Lys His Thr Leu Leu Met Phe Asp Ser
        115                 120                 125

Phe His Asp Val Gln Glu Lys Ala Glu Lys Gly Asn Lys Tyr Ala Gln
    130                 135                 140

Glu Val Leu Gln Ser Trp Ala Asp Ala Glu Trp Phe Ala Ser Arg Ala
145                 150                 155                 160

Lys Val Pro Glu Lys Ile Thr Val Thr Val Phe Lys Val Asp Gly Glu
            165                 170                 175

Thr Asn Thr Asp Asp Leu Ser Pro Ala Pro Asp Ala Trp Ser Arg Pro
            180                 185                 190

Asp Ile Pro Leu His Ala Leu Ala Met Leu Lys Asn Pro Arg Asp Gly
        195                 200                 205

Ile Thr Pro Asp Lys Pro Gly Glu Val Gly Pro Ile Lys Leu Leu Glu
    210                 215                 220
```

Glu Leu Lys Ala Lys Gly His Pro Val Ala Tyr Val Gly Asp Val Val
225               230               235               240

Gly Thr Gly Ser Ser Arg Lys Ser Ala Thr Asn Ser Val Ile Trp His
          245               250               255

Thr Gly Glu Asp Ile Pro Phe Val Pro Asn Lys Arg Phe Gly Gly Val
          260               265               270

Cys Leu Gly Gly Lys Ile Ala Pro Ile Phe Phe Asn Thr Gln Glu Asp
      275               280               285

Ser Gly Ala Leu Pro Ile Glu Val Asp Val Ser Ala Leu Lys Met Gly
      290               295               300

Asp Val Val Asp Ile Leu Pro Tyr Glu Gly Lys Ile Val Lys Asn Gly
305               310               315               320

Glu Thr Val Ala Glu Phe Glu Leu Lys Ser Gln Val Leu Leu Asp Glu
              325               330               335

Val Gln Ala Gly Gly Arg Ile Asn Leu Ile Ile Gly Arg Gly Leu Thr
          340               345               350

Ala Lys Ala Arg Glu Ala Leu Lys Leu Pro Ala Ser Thr Ala Phe Arg
          355               360               365

Leu Pro Gln Ala Pro Ala Glu Ser Lys Ala Gly Phe Thr Leu Ala Gln
      370               375               380

Lys Met Val Gly Arg Ala Cys Gly Leu Pro Glu Gly Gln Gly Val Arg
385               390               395               400

Pro Gly Thr Tyr Cys Glu Pro Arg Met Thr Thr Val Gly Ser Gln Asp
              405               410               415

Thr Thr Gly Pro Met Thr Arg Asp Glu Leu Lys Asp Leu Ala Cys Leu
          420               425               430

Gly Phe Ser Ala Asp Met Val Met Gln Ser Phe Cys His Thr Ala Ala
      435               440               445

Tyr Pro Lys Pro Val Asp Val Lys Thr His Lys Glu Leu Pro Ala Phe
      450               455               460

Ile Ser Thr Arg Gly Gly Val Ser Leu Arg Pro Gly Asp Gly Val Ile
465               470               475               480

His Ser Trp Leu Asn Arg Leu Leu Leu Pro Asp Thr Val Gly Thr Gly
              485               490               495

Gly Asp Ser His Thr Arg Phe Pro Ile Gly Ile Ser Phe Pro Ala Gly
          500               505               510

Ser Gly Leu Val Ala Phe Ala Ala Ala Thr Gly Val Met Pro Leu Asp
      515               520               525

Met Pro Glu Ser Val Leu Val Arg Phe Ser Gly Lys Leu Gln Pro Gly
      530               535               540

Val Thr Leu Arg Asp Leu Val Asn Ala Ile Pro Leu Tyr Ala Ile Lys
545               550               555               560

Gln Gly Leu Leu Thr Val Ala Lys Ala Gly Lys Lys Asn Ile Phe Ser
              565               570               575

Gly Arg Ile Leu Glu Ile Glu Gly Leu Pro Asp Leu Lys Val Glu Gln
          580               585               590

Ala Phe Glu Leu Thr Asp Ala Ser Ala Glu Arg Ser Ala Ala Gly Cys

```
                595                     600                     605

        Thr Val Lys Leu Asn Lys Glu Pro Ile Ile Glu Tyr Met Lys Ser Asn
            610                 615                 620

        Val Val Leu Met Lys Asn Met Ile Ala Asn Gly Tyr Gln Asp Pro Arg
        625                 630                 635                 640

        Thr Leu Glu Arg Arg Ile Lys Ala Met Glu Lys Trp Leu Ala Asn Pro
                        645                 650                 655

        Glu Leu Leu Glu Ala Asp Lys Asp Ala Glu Tyr Ala Ala Val Ile Glu
                        660                 665                 670

        Ile Asn Met Asp Asp Ile Lys Glu Pro Ile Ile Ala Cys Pro Asn Asp
                675                 680                 685

        Pro Asp Asp Val Cys Phe Met Ser Glu Arg Ser Gly Thr Lys Ile Asp
            690                 695                 700

        Glu Val Phe Ile Gly Ser Cys Met Thr Asn Ile Gly His Phe Arg Ala
        705                 710                 715                 720

        Ala Ser Lys Leu Leu Glu Gly Lys Ala Asp Thr Pro Val Arg Leu Trp
                        725                 730                 735

        Ile Ala Pro Pro Thr Lys Met Asp Ala Lys Gln Leu Ser Asp Glu Gly
                        740                 745                 750

        His Tyr Gly Val Leu Gly Arg Ala Gly Ala Arg Met Glu Met Pro Gly
                        755                 760                 765

        Cys Ser Leu Cys Met Gly Asn Gln Ala Gln Val Arg Glu Gly Ala Thr
                770                 775                 780

        Val Met Ser Thr Ser Thr Arg Asn Phe Pro Asn Arg Leu Gly Lys Asn
        785                 790                 795                 800

        Thr Phe Val Tyr Leu Gly Ser Ala Glu Leu Ala Ala Ile Cys Ser Lys
                        805                 810                 815

        Leu Gly Lys Ile Pro Thr Val Glu Glu Tyr Gln Ala Asn Ile Gly Ile
                        820                 825                 830

        Ile Asn Glu Gln Gly Asp Lys Ile Tyr Arg Tyr Met Asn Phe Asn Glu
                    835                 840                 845

        Ile Asp Ser Tyr Asn Glu Val Ala Glu Thr Val Asn Val
            850                 855                 860
```

<210> 150
<211> 337
<212> PRT
<213> Neisseria meningitidis

<400> 150

```
Met Gln Val Tyr Tyr Asp Lys Asp Ala Asp Leu Ser Leu Ile Lys Gly
1               5                   10                  15

Lys Thr Val Ala Ile Ile Gly Tyr Gly Ser Gln Gly His Ala His Ala
              20                25                  30

Ala Asn Leu Lys Asp Ser Gly Val Asn Val Val Ile Gly Leu Arg Gln
          35                40                  45

Gly Ser Ser Trp Lys Lys Ala Glu Ala Ala Gly His Val Val Lys Thr
        50                55                  60

Val Ala Glu Ala Thr Lys Glu Ala Asp Val Val Met Leu Leu Leu Pro
65                  70                  75                  80

Asp Glu Thr Met Pro Ala Val Tyr His Ala Glu Val Thr Ala Asn Leu
                85                  90                  95

Lys Glu Gly Ala Thr Leu Ala Phe Ala His Gly Phe Asn Val His Tyr
              100                 105                 110

Asn Gln Ile Val Pro Arg Ala Asp Leu Asp Val Ile Met Val Ala Pro
          115                 120                 125

Lys Gly Pro Gly His Thr Val Arg Ser Glu Tyr Lys Arg Gly Gly Gly
        130                 135                 140

Val Pro Ser Leu Ile Ala Val Tyr Gln Asp Asn Ser Gly Lys Ala Lys
145                 150                 155                 160

Asp Ile Ala Leu Ser Tyr Ala Ala Ala Asn Gly Gly Thr Lys Gly Gly
              165                 170                 175

Val Ile Glu Thr Thr Phe Arg Glu Glu Thr Glu Thr Asp Leu Phe Gly
              180                 185                 190

Glu Gln Ala Val Leu Cys Gly Gly Val Val Glu Leu Ile Lys Ala Gly
          195                 200                 205

Phe Glu Thr Leu Thr Glu Ala Gly Tyr Ala Pro Glu Met Ala Tyr Phe
    210                 215                 220

Glu Cys Leu His Glu Met Lys Leu Ile Val Asp Leu Ile Phe Glu Gly
225                 230                 235                 240

Gly Ile Ala Asn Met Asn Tyr Ser Ile Ser Asn Asn Ala Glu Tyr Gly
              245                 250                 255

Glu Tyr Val Thr Gly Pro Glu Val Val Asn Ala Ser Ser Lys Glu Ala
              260                 265                 270

Met Arg Asn Ala Leu Lys Arg Ile Gln Thr Gly Glu Tyr Ala Lys Met
        275                 280                 285

Phe Ile Gln Glu Gly Asn Val Asn Tyr Ala Ser Met Thr Ala Arg Arg
    290                 295                 300

Arg Leu Asn Ala Asp His Gln Val Glu Lys Val Gly Ala Gln Leu Arg
305                 310                 315                 320

Ala Met Met Pro Trp Ile Thr Ala Asn Lys Leu Val Asp Gln Asp Lys
              325                 330                 335

Asn
```

<210> 151
<211> 575

<212> PRT
<213> Neisseria meningitidis

<400> 151

```
Met Gln Leu Ser Gly Ala Gln Ile Ile Val Gln Ser Leu Lys Ala Glu
1               5                   10                  15

Gly Val Glu Tyr Val Phe Gly Tyr Pro Gly Gly Ala Val Ile Glu Ile
                20                  25                  30

Tyr Asp Ala Leu Phe Gln Leu Asn Lys Phe Lys His Ile Leu Thr Arg
            35                  40                  45

His Glu Gln Ala Ala Val His Ala Ala Asp Ala Tyr Ala Arg Val Ser
            50                  55                  60
```

Gly Lys Val Gly Val Ala Leu Val Thr Ser Gly Pro Gly Val Thr Asn
65            70              75              80

Ala Leu Thr Gly Ile Ala Thr Ala Tyr Thr Asp Ser Ile Pro Met Val
            85          90              95

Val Ile Ser Gly Gln Val Gly Asn Ser Leu Ile Gly Thr Asp Ala Phe
            100         105             110

Gln Glu Val Asp Thr Val Gly Ile Thr Arg Pro Cys Val Lys His Asn
        115             120             125

Phe Leu Val Thr Asp Ile Asn Glu Leu Ala Glu Thr Ile Lys Lys Ala
    130             135             140

Phe Gln Ile Ala Ala Ser Gly Arg Pro Gly Pro Val Val Val Asp Val
145             150             155             160

Pro Lys Asp Val Thr Gln Ala Met Ala Lys Phe Ser Tyr Pro Gln Glu
            165             170             175

Asp Ile Phe Ile Arg Ser Tyr Gln Pro Val Val Gln Gly His Ile Gly
            180             185             190

Gln Ile Lys Lys Ala Val Gln Met Leu Ala Ser Ala Lys Arg Pro Val
        195             200             205

Val Tyr Phe Gly Gly Gly Val Val Leu Gly Asn Ala Ser Glu Glu Leu
    210             215             220

Thr Arg Phe Val Arg Met Thr Gly Ala Pro Cys Thr Gly Thr Leu Met
225             230             235             240

Gly Leu Gly Ala Tyr Pro Ser Gly Asp Arg Gln Phe Leu Gly Met Leu
            245             250             255

Gly Met His Gly Thr Tyr Glu Ala Asn Leu Ala Met Gln Asn Ala Asp
            260             265             270

Val Val Leu Ala Val Gly Ala Arg Phe Asp Asp Arg Val Val Ser Val
        275             280             285

Pro Ser Lys Phe Phe Glu Lys Ala Lys Lys Val Ile His Ile Asp Val
    290             295             300

Asp Pro Ser Ser Ile Ala Lys Arg Val Lys Val Asp Ile Pro Ile Val
305             310             315             320

Gly Asp Val Lys Asn Val Leu Ser Glu Met Val Ala Leu Trp Gln Lys
            325             330             335

Gln Glu Ser Val Pro Ser Glu Asp Ala Leu Gly Lys Trp Trp Lys Thr
            340             345             350

Ile Glu Glu Trp Arg Ser Arg Asp Cys Leu Trp Phe Asp Asn Gly Ser
        355             360             365

Glu Ile Ile Lys Pro Gln Tyr Val Ile Gln Lys Leu Ala Glu Ile Thr
    370             375             380

Gly Asn Ser Ala Ile Ile Thr Ser Asp Val Gly Gln His Gln Met Phe
385             390             395             400

Ala Ala Gln Tyr Tyr Pro Phe Glu Arg Pro Arg Gln Trp Leu Asn Ser
            405             410             415

Gly Gly Leu Gly Thr Met Gly Val Gly Leu Pro Tyr Ala Ile Gly Ala
            420             425             430

Lys Leu Ala Ala Pro Asp Gln Asp Val Phe Cys Ile Thr Gly Asp Gly

311

```
           435                    440                    445

    Ser Ile Gln Met Asn Ile Gln Glu Leu Ser Thr Cys Phe Gln Tyr Arg
        450                 455             460

    Ile Pro Val Asn Val Ile Thr Leu Asn Asn Gly Tyr Leu Gly Met Val
    465                 470             475                 480

    Arg Gln Trp Gln Glu Ile Tyr Tyr Gly Gly Arg Glu Ser Glu Thr Tyr
                    485             490                 495

    Phe Asp Ser Leu Pro Asp Phe Val Lys Leu Ala Glu Ala Tyr Gly His
                500             505             510

    Ile Gly Ile Arg Val Asp Lys Lys Ser Asp Val Glu Gly Ala Leu Leu
            515             520             525

    Glu Ala Leu Asn Gln Lys Asp Arg Leu Val Phe Ile Asp Phe Leu Thr
        530             535             540

    Asp Gln Lys Gln Asn Val Met Pro Met Val Gly Asn Gly Lys Gly Leu
    545             550             555                 560

    Asp Glu Met Val Leu Pro Pro His Met Arg Ala Asp Gly Lys Ala
                565             570             575
```

<210> 152
<211> 429
<212> PRT
<213> Neisseria meningitidis

<400> 152

Met Lys Lys Leu Asn Thr Gln Ser Pro Asp Phe Gln Ala Gly Leu Lys
1               5                   10                  15

Ala Leu Leu Ala Phe Glu Thr Ala Gln Asn Pro Glu Thr Glu Arg Ile
            20                  25                  30

Val Ala Asp Ile Cys Ala Asp Val Gln Lys Arg Gly Asp Ala Ala Leu
            35                  40                  45

Ile Glu Tyr Thr Asn Lys Phe Asp Gln Thr Asn Ala Lys Ser Ile Asp
        50                  55                  60

Asp Leu Ile Leu Thr Gln Ala Asp Leu Asn Ala Ala Phe Glu Arg Ile
65                  70                  75                  80

Pro Asn Asp Val Gln Thr Ala Leu Gln Thr Ala Ala Arg Arg Val Glu
            85                  90                  95

Ser Tyr His Gln Arg Gln Lys Met Glu Ser Trp Ser Tyr Thr Asp Glu
            100                 105                 110

Asp Gly Thr Leu Leu Gly Gln Gln Ile Thr Pro Leu Asp Arg Val Gly
        115                 120                 125

Ile Tyr Val Pro Gly Gly Lys Ala Ala Tyr Pro Ser Ser Val Ile Met
        130                 135                 140

Asn Ala Met Pro Ala His Val Ala Gly Val Lys Glu Ile Ile Met Val
145                 150                 155                 160

Val Pro Thr Pro Lys Gly Glu Arg Asn Asp Ile Val Leu Ala Ala Ala
            165                 170                 175

Tyr Val Ala Gly Val Thr Lys Val Phe Thr Val Gly Gly Ala Gln Ala
        180                 185                 190

Val Ala Ala Leu Ala Tyr Gly Thr Glu Thr Ile Pro Gln Val Asp Lys
        195                 200                 205

```
Ile Thr Gly Pro Gly Asn Ala Phe Val Ala Ala Ala Lys Arg Arg Val
    210             215             220

Phe Gly Val Val Gly Ile Asp Met Val Ala Gly Pro Ser Glu Ile Leu
225             230             235                 240

Val Ile Ala Asp Gly Thr Thr Pro Ala Asp Trp Val Ala Met Asp Leu
            245             250             255

Phe Ser Gln Ala Glu His Asp Glu Ile Ala Gln Ala Ile Leu Ile Gly
            260             265             270

Thr Ser Gln Ala Tyr Leu Asp Glu Val Glu Ala Ala Met Asp Arg Leu
            275             280             285

Ile Glu Thr Met Pro Arg Arg Asp Ile Ile Glu Ala Ser Leu Gly Asn
    290             295             300

Arg Gly Ala Met Ile Leu Ala Lys Asp Leu Asp Glu Ala Cys Glu Ile
305             310             315             320

Ala Asn Tyr Ile Ser Pro Glu His Leu Glu Leu Ser Val Glu Asn Pro
            325             330             335

Gln Glu Trp Ala Lys Lys Ile Arg His Ala Gly Ala Ile Phe Met Gly
            340             345             350

Arg Tyr Thr Gly Glu Ser Leu Gly Asp Tyr Cys Ala Gly Pro Asn His
        355             360             365

Val Leu Pro Thr Ser Arg Thr Ala Arg Phe Ser Ser Pro Leu Gly Thr
    370             375             380

Tyr Asp Phe Gln Lys Arg Ser Ser Leu Ile Gln Val Ser Glu Gln Gly
385             390             395             400

Ala Gln Lys Leu Gly Glu Thr Ala Ser Val Leu Ala His Gly Glu Ser
            405             410             415

Leu Thr Ala His Ala Arg Ala Ala Glu Phe Arg Met Lys
            420             425
```

<210> 153
<211> 305
<212> PRT
<213> Neisseria meningitidis

<400> 153

```
Met Asn Leu Thr Lys Thr Gln Arg Gln Leu His Asn Phe Leu Thr Leu
1               5               10              15

Ala Gln Glu Ala Gly Ser Leu Ser Lys Leu Ala Lys Leu Cys Gly Tyr
            20              25              30

Arg Thr Pro Val Ala Leu Tyr Lys Leu Lys Gln Arg Leu Glu Lys Gln
        35              40              45

Ala Glu Asp Pro Asp Ala Arg Gly Ile Arg Pro Ser Leu Met Ala Lys
    50              55              60

Leu Glu Lys His Thr Gly Lys Pro Lys Gly Trp Leu Asp Arg Lys His
65              70              75              80

Arg Glu Arg Thr Val Pro Glu Thr Ala Ala Glu Ser Thr Gly Thr Ala
            85              90              95

Glu Thr Gln Ile Ala Glu Thr Ala Ser Ala Ala Gly Cys Arg Ser Val
        100             105             110

Thr Val Asn Arg Asn Thr Cys Glu Thr Gln Ile Thr Val Ser Ile Asn
        115             120             125

Leu Asp Gly Ser Gly Lys Ser Arg Leu Asp Thr Gly Val Pro Phe Leu
    130             135             140

Glu His Met Ile Asp Gln Ile Ala Arg His Gly Met Ile Asp Ile Asp
145             150             155             160

Ile Ser Cys Lys Gly Asp Leu His Ile Asp Asp His His Thr Ala Glu
            165             170             175

Asp Ile Gly Ile Thr Leu Gly Gln Ala Ile Arg Gln Ala Leu Gly Asp
            180             185             190

Lys Lys Gly Ile Arg Arg Tyr Gly His Ser Tyr Val Pro Leu Asp Glu
            195             200             205

Ala Leu Ser Arg Val Val Ile Asp Leu Ser Gly Arg Pro Gly Leu Val
    210             215             220

Tyr Asn Ile Glu Phe Thr Arg Ala Leu Ile Gly Arg Phe Asp Val Asp
225             230             235             240

Leu Phe Glu Glu Phe Phe His Gly Ile Val Asn His Ser Met Met Thr
            245             250             255

Leu His Ile Asp Asn Leu Ser Gly Lys Asn Ala His His Gln Ala Glu
        260             265             270

Thr Val Phe Lys Ala Phe Gly Arg Ala Leu Arg Met Ala Val Glu His
        275             280             285

Asp Pro Arg Met Ala Gly Gln Thr Pro Ser Thr Lys Gly Thr Leu Thr
    290             295             300

Ala
305
```

```
<210> 154
<211> 874
<212> PRT
<213> Neisseria meningitidis
```

<400> 154

```
Met Lys Thr Ser Glu Leu Arg Gln Lys Phe Leu Lys Phe Phe Glu Thr
1               5                   10                  15

Lys Gly His Thr Val Val Arg Ser Ser Ser Leu Val Pro His Asp Asp
            20                  25                  30

Pro Thr Leu Leu Phe Thr Asn Ala Gly Met Asn Gln Phe Lys Asp Val
            35                  40                  45

Phe Leu Gly Phe Asp Lys Arg Pro Tyr Ser Arg Ala Thr Thr Ala Gln
    50                  55                  60

Lys Cys Val Arg Ala Gly Gly Lys His Asn Asp Leu Glu Asn Val Gly
65                  70                  75                  80

Tyr Thr Ala Arg His His Thr Phe Phe Glu Met Met Gly Asn Phe Ser
                85                  90                  95

Phe Gly Asp Tyr Phe Lys Arg Asp Ala Ile His Phe Ala Trp Glu Phe
            100                 105                 110

Leu Thr Ser Pro Glu Trp Leu Asn Ile Pro Lys Asp Lys Leu Leu Ala
            115                 120                 125

Thr Val Tyr Ala Glu Asp Asp Glu Ala Tyr Asn Ile Trp Leu Asn Glu
```

|  | 130 |  |  |  | 135 |  |  |  | 140 |  |  |  |  |
|---|---|---|---|---|---|---|---|---|---|---|---|---|---|

Ile Gly Met Pro Ser Glu Arg Ile Val Arg Ile Gly Asp Asn Lys Gly
145                150                155                160

Ala Lys Tyr Ala Ser Asp Asn Phe Trp Gln Met Gly Asp Thr Gly Pro
                165                170                175

Cys Gly Pro Cys Ser Glu Ile Phe Tyr Asp His Gly Glu Glu Ile Trp
                180                185                190

Gly Gly Ile Pro Gly Ser Pro Glu Glu Asp Gly Asp Arg Trp Ile Glu
                195                200                205

Ile Trp Asn Cys Val Phe Met Gln Phe Asn Arg Asp Glu Gln Gly Asn
210                215                220

Met Asn Pro Leu Pro Lys Pro Ser Val Asp Thr Gly Met Gly Leu Glu
225                230                235                240

Arg Ile Ala Ala Val Met Gln His Val His Ser Asn Tyr Glu Ile Asp
                245                250                255

Leu Phe Gln Asp Leu Leu Lys Ala Val Ala Arg Glu Thr Gly Ala Pro
                260                265                270

Phe Arg Met Glu Glu Pro Ser Leu Lys Val Ile Ala Asp His Ile Arg
                275                280                285

Ser Cys Ser Phe Leu Ile Ala Asp Gly Val Leu Pro Ser Asn Glu Gly
                290                295                300

Arg Gly Tyr Val Leu Arg Arg Ile Ile Arg Arg Ala Val Arg His Gly
305                310                315                320

Tyr Lys Leu Gly Gln Ser Lys Pro Phe Phe His Lys Leu Val Ala Asp
                325                330                335

Leu Val Lys Glu Met Gly Gly Ala Tyr Pro Glu Leu Lys Glu Lys Gln
                340                345                350

Ala Gln Ile Glu Glu Ala Leu Lys Asn Glu Glu Ser Arg Phe Ala Gln
                355                360                365

Thr Leu Glu Thr Gly Met Ala Leu Leu Glu Asn Ala Leu Val Lys Gly
                370                375                380

Gly Lys Thr Leu Gly Gly Glu Ile Ile Phe Lys Leu Tyr Asp Thr Tyr
385                390                395                400

Gly Phe Pro Tyr Asp Leu Thr Ala Asp Ile Cys Arg Glu Arg Asn Ile
                405                410                415

Glu Pro Asp Glu Ala Gly Phe Glu Arg Glu Met Glu Ala Gln Arg Ala
                420                425                430

Arg Ala Arg Ala Ala Gln Ser Phe Lys Ala Asn Ala Gln Leu Pro Tyr
                435                440                445

Asp Gly Gln Asp Thr Glu Phe Lys Gly Tyr Ser Glu Arg Gln Thr Glu
                450                455                460

Ser Lys Val Leu Ala Leu Tyr Lys Asp Gly Glu Gln Val Asn Glu Leu
465                470                475                480

Asn Glu Gly Asp Ser Gly Ala Val Val Ile Asp Phe Thr Pro Phe Tyr
                485                490                495

Ala Glu Ser Gly Gly Gln Val Gly Asp Val Gly Tyr Ile Phe Ser Gly
                500                505                510

```
Glu Asn Arg Phe Glu Val Arg Asp Thr Gln Lys Ile Lys Ala Ala Val
        515                 520                 525

Phe Gly Gln Phe Gly Val Gln Thr Ser Gly Arg Leu Lys Val Gly Asp
        530                 535                 540

Ser Val Thr Ala Lys Val Asp Asp Glu Ile Arg Asn Ala Asn Met Arg
545                 550                 555                 560

Asn His Ser Ala Thr His Leu Met His Lys Ala Leu Arg Asp Val Leu
                565                 570                 575

Gly Arg His Val Glu Gln Lys Gly Ser Leu Val Thr Ala Glu Ser Thr
        580                 585                 590

Arg Phe Asp Ile Ser His Pro Gln Ala Val Thr Ala Glu Glu Ile Ala
        595                 600                 605

Glu Val Glu Arg Arg Val Asn Glu Ala Ile Leu Ala Asn Val Ala Val
    610                 615                 620

Asn Ala Ala Ile Met Ser Met Glu Asp Ala Gln Lys Thr Gly Ala Met
625                 630                 635                 640

Met Leu Phe Gly Glu Lys Tyr Gly Glu Glu Val Arg Val Leu Gln Met
                645                 650                 655

Gly Gly Phe Ser Thr Glu Leu Cys Gly Gly Thr His Val Ser Arg Thr
            660                 665                 670

Gly Asp Ile Gly Leu Phe Lys Ile Ile Ser Glu Gly Gly Ile Ala Ala
        675                 680                 685

Gly Val Arg Arg Ile Glu Ala Ile Thr Gly Leu Asn Ala Leu Lys Trp
    690                 695                 700

Ala Gln Glu Gln Glu Arg Leu Val Lys Asp Ile Ile Ala Glu Thr Lys
705                 710                 715                 720

Ala Gln Thr Glu Lys Asp Val Leu Ala Lys Ile Gln Ala Gly Ala Ala
                725                 730                 735

His Ala Lys Ala Leu Glu Lys Glu Leu Ala Arg Ala Lys Ala Glu Leu
            740                 745                 750

Ala Val His Ala Gly Ala Lys Leu Leu Asp Asp Ala Lys Asp Leu Gly
            755                 760                 765

Ala Ala Lys Leu Val Ala Ala Gln Ile Glu Ala Asp Ala Ala Ala Leu
770                 775                 780

Arg Glu Ile Val Thr Asp Leu Thr Gly Lys Ser Asp Asn Ala Val Ile
785                 790                 795                 800

Leu Leu Ala Ala Val Asn Asp Gly Lys Val Ser Leu Cys Ala Gly Val
                805                 810                 815

Ser Lys Pro Leu Thr Gly Lys Val Lys Ala Gly Asp Leu Val Lys Phe
            820                 825                 830

Ala Ala Glu Gln Val Gly Gly Lys Gly Gly Gly Arg Pro Asp Leu Ala
        835                 840                 845

Gln Ala Gly Gly Thr Asp Ala Asp Lys Leu Pro Ala Val Leu Asp Ser
        850                 855                 860

Val Lys Asp Trp Val Gly Ala Lys Leu Val
865                 870
```

<210> 155
<211> 227
<212> PRT
<213> Neisseria meningitidis

<400> 155

```
        Met Glu Leu Val Phe Ile Arg His Gly Gln Ser Glu Trp Asn Ala Lys
        1               5                   10                  15

        Asn Leu Phe Thr Gly Trp Arg Asp Val Lys Leu Ser Glu Gln Gly Leu
                    20                  25                  30

        Ala Glu Ala Ala Ala Ala Gly Lys Lys Leu Lys Glu Asn Gly Tyr Glu
                    35                  40                  45

        Phe Asp Ile Ala Phe Thr Ser Val Leu Thr Arg Ala Ile Lys Thr Cys
                50                  55                  60

        Asn Ile Val Leu Glu Glu Ser Asp Gln Leu Phe Val Pro Gln Ile Lys
        65                  70                  75                  80

        Thr Trp Arg Leu Asn Glu Arg His Tyr Gly Gln Leu Gln Gly Leu Asp
                        85                  90                  95

        Lys Lys Gln Thr Ala Glu Gln Tyr Gly Asp Glu Gln Val Arg Ile Trp
                    100                 105                 110

        Arg Arg Ser Tyr Asp Thr Leu Pro Pro Leu Leu Asp Lys Asp Asp Glu
                    115                 120                 125

        Phe Ser Ala His Lys Asp Arg Arg Tyr Ala His Leu Pro Ala Asp Val
                130                 135                 140

        Val Pro Asp Gly Glu Asn Leu Lys Val Thr Leu Glu Arg Val Leu Pro
        145                 150                 155                 160

        Phe Trp Glu Asp Gln Ile Ala Pro Ala Ile Leu Ser Gly Lys Arg Val
                        165                 170                 175

        Leu Val Ala Ala His Gly Asn Ser Leu Arg Ala Leu Ala Lys His Ile
                    180                 185                 190

        Glu Gly Ile Ser Asp Glu Asp Ile Met Gly Leu Glu Ile Pro Thr Gly
                    195                 200                 205

        Gln Pro Leu Val Tyr Lys Leu Asp Asp Asn Leu Lys Val Ile Glu Lys
            210                 215                 220

        Phe Tyr Leu
        225
```

<210> 156
<211> 177
<212> PRT
<213> Neisseria meningitidis

<400> 156

Met Gly Ile Tyr Asp Phe Gln Met Lys Asp Ala Glu Gly Asn Ala Val
1                   5                   10                  15

Asp Leu Ser Gly Tyr Arg Gly Lys Val Leu Leu Ile Val Asn Thr Ala
            20              25                  30

Thr Arg Cys Gly Leu Thr Pro Gln Tyr Glu Ala Leu Gln Lys Leu Tyr
            35              40                  45

Ala Gln Tyr Thr Ala Glu Gly Leu Glu Ile Leu Asp Phe Pro Cys Asn
    50              55                  60

Gln Phe Arg Glu Gln Ala Pro Glu Ser Ser Gly Glu Ile Ala Gln Val

65                  70                  75                  80

Cys Met Met Lys Phe Gly Thr Lys Phe Lys Ile Phe Asp Lys Ile Glu
            85              90                  95

Val Asn Gly Ala Asn Thr Ala Pro Leu Tyr Ala Tyr Leu Lys Ser Val
            100             105                 110

Lys Pro Gln Asp Lys Gly Asn His Leu Phe Lys Asp Phe Val Leu Lys
        115             120                 125

Leu Ala Ala Leu Gly Glu Lys Arg Asp Glu Gly Asp Ile Lys Trp Asn
    130             135                 140

Phe Thr Lys Phe Leu Val Asn Arg Asp Gly Glu Val Val Glu Arg Phe
145             150                 155                 160

Ala Pro Ser Val Thr Pro Glu Glu Ile Glu Ala Asp Ile Arg Ala Leu
            165             170                 175

Leu

<210> 157
<211> 201
<212> PRT
<213> Neisseria meningitidis

<400> 157

```
        Leu Leu Phe Ser Ser Leu Leu Phe Ser Ser Leu Leu Phe Ser Ser Leu
        1               5               10              15

        Leu Phe Ser Ser Ala Ala Gln Ala Ala Ser Glu Asp Ser Ser Arg Ser
                    20              25              30

        Pro Tyr Tyr Val Gln Ala Asp Leu Ala Tyr Ala Ala Glu Arg Ile Thr
                    35              40              45

        His Asp Tyr Pro Lys Pro Thr Gly Thr Asp Lys Asp Lys Ile Ser Thr
                50              55              60

        Val Ser Asp Tyr Phe Arg Asn Ile Arg Ala His Ser Tyr His Pro Arg
        65              70              75              80

        Val Ser Val Gly Tyr Asp Phe Gly Gly Trp Arg Ile Ala Ala Asp Tyr
                        85              90              95

        Ala Ser Tyr Arg Lys Trp Asn Asn Asn Lys Tyr Ser Val Asn Ile Lys
                    100             105             110

        Lys Gly Arg Glu Thr Gln Asp Asn Arg Glu Glu Leu Lys Thr Glu Asn
                    115             120             125

        Gln Glu Asn Gly Ser Phe His Ala Ala Ser Ser Leu Gly Leu Ser Ala
                130             135             140

        Ile Tyr Asp Phe Lys Leu Asn Asp Lys Phe Asp Lys Phe Lys Pro Tyr
        145             150             155             160

        Ile Gly Ala Arg Val Ala Tyr Gly His Val Lys His Gln Val His Ser
                    165             170             175

        Val Glu Thr Lys Thr Thr Ile Val Thr Ser Lys Pro Ala Ala Thr Ser
                    180             185             190

        Pro Gln Gly Gly Pro Ile Ile Glu Thr
                    195             200
```

<210> 158
<211> 368
<212> PRT
<213> Neisseria meningitidis

<400> 158

```
Met Ser Leu Tyr Pro Ile Tyr Asn Phe Ser Ala Gly Pro Ala Val Leu
1               5               10              15

Pro Glu Ala Val Leu Glu Thr Ala Arg Gln Glu Met Leu Asp Tyr Asn
            20              25              30

Gly Thr Gly Phe Pro Val Met Ala Met Ser His Arg Ser Glu Met Phe
        35              40              45

Leu Ser Ile Leu His His Ala Glu Gln Asp Leu Arg Gln Leu Leu Lys
    50              55              60

Val Pro Asp Asn Tyr Lys Ile Leu Phe Leu Gln Gly Gly Ala Thr Thr
65              70              75              80

Gln Phe Asn Met Ala Ala Met Asn Leu Ala His Gly Phe Arg Thr Ala
            85              90              95

Asp Ala Val Val Thr Gly Asn Trp Ser Arg Ile Ala Tyr Glu Gln Met
            100             105             110

Ser Arg Leu Thr Asp Thr Glu Ile Arg Leu Ala Ala His Gly Gly Glu
        115             120             125

Gln Phe Asp Tyr Leu Asp Leu Pro Pro Val Glu Thr Trp Asp Val Ala
    130             135             140

Pro Asp Ser Ala Phe Val His Phe Ala Val Asn Glu Thr Val Asn Gly
145             150             155             160

Leu Gln Tyr Arg Glu Val Pro Cys Leu Ser Glu Gly Met Pro Pro Leu
            165             170             175

Val Cys Asp Met Ser Ser Glu Ile Leu Ser Arg Glu Phe Asp Val Ala
            180             185             190

Asp Tyr Gly Leu Ile Tyr Ala Gly Ala Gln Lys Asn Ile Gly Pro Ala
        195             200             205

Gly Val Thr Val Val Ile Val Arg Glu Asp Leu Leu Glu Arg Cys Pro
    210             215             220

Asn Asp Ile Pro Asp Val Phe Asn Tyr Arg Ser His Ile Asn Arg Asp
225             230             235             240

Gly Met Tyr Asn Thr Pro Ser Thr Tyr Ala Ile Tyr Met Ser Gly Leu
            245             250             255

Val Phe Arg Trp Leu Gln Ala Gln Gly Gly Val Lys Lys Ile Glu Ala
            260             265             270

Val Asn Arg Leu Lys Ala Gln Thr Leu Tyr Glu Thr Ile Asp Gly Ser
        275             280             285

Asp Gly Phe Tyr Ile Asn Arg Ile Arg Pro Asn Ala Arg Ser Lys Met
    290             295             300

Asn Val Val Phe Gln Thr Gly Asp Glu Glu Leu Asp Arg Arg Phe Val
305             310             315             320

Leu Glu Ala Glu Leu Gln Gly Leu Cys Leu Leu Lys Gly Tyr Lys Thr
            325             330             335

Val Gly Gly Met Arg Ala Ser Ile Tyr Asn Ala Met Pro Leu Glu Gly
            340             345             350
```

322

```
Val Arg Ala Leu Ala Asp Phe Met Arg Asp Phe Gln Arg Arg Tyr Gly
          355                 360                 365
```

<210> 159
<211> 500
<212> PRT
<213> Neisseria meningitidis

<400> 159

```
Met Ser Arg Glu Met Leu Gln Leu Ala Glu Ala Leu Ala Ser Glu Lys
1               5               10              15

Asn Val Asp Ala Glu Val Val Phe Gln Ala Leu Glu Phe Ala Leu Ser
            20              25              30

Thr Ala Ala Lys Lys Lys Ala Asp Arg Glu His Met Asp Val Arg Val
        35              40              45

Gln Ile Asn Arg Asp Thr Gly Glu Tyr Gln Thr Phe Arg Arg Trp Leu
    50              55              60

Ile Val Ala Asp Glu Asp Tyr Thr Tyr Pro Asp Val Glu Lys Thr Ile
65              70              75              80

Glu Glu Ile Gln Glu Glu Ile Pro Gly Thr Thr Ile Gln Ile Gly Glu
            85              90              95

Tyr Tyr Glu Glu Gln Leu Pro Asn Glu Gly Phe Gly Arg Gln Ala Ala
        100             105             110

Gln Thr Ala Lys Gln Ile Ile Leu Gln Arg Ile Arg Asp Ala Glu Arg
    115             120             125

Glu Gln Asn Leu Asn Glu Phe Leu Ala Val Lys Glu Asp Ile Val Ser
    130             135             140

Gly Thr Val Lys Arg Val Glu Arg His Gly Ile Ile Val Glu Val Val
145             150             155             160

Ala Gly Lys Leu Asp Ala Leu Ile Pro Arg Asp Gln Met Ile Pro Arg
            165             170             175

Glu Asn Phe Arg Ser Gly Asp Arg Ile Arg Ala Leu Phe Leu Arg Val
            180             185             190

Glu Glu Ile Gly Asn Thr Gly Arg Lys Gln Val Ile Leu Ser Arg Thr
            195             200             205

Ser Gly Asp Phe Leu Val Lys Leu Tyr Ala Asn Glu Val Pro Glu Ile
    210             215             220

Ala Asp Gly Met Leu Glu Ile Arg Ala Val Ala Arg Asp Pro Gly Gln
225             230             235             240

Arg Ala Lys Val Ala Val Lys Ala Asn Asp Gln Arg Ile Asp Pro Gln
            245             250             255

Gly Thr Cys Ile Gly Val Arg Gly Ser Arg Val Asn Ala Val Ser Asn
            260             265             270

Glu Leu Ser Gly Glu Arg Ile Asp Val Val Leu Trp Ser Pro Glu Pro
    275             280             285

Ala Gln Phe Val Met Ser Ala Leu Ser Pro Ala Glu Val Ser Arg Ile
    290             295             300

Val Ile Asp Glu Asp Lys His Ala Val Asp Val Ile Val Ala Glu Asp
305             310             315             320
```

324

```
Gln Leu Ala Leu Ala Ile Gly Arg Gly Gly Gln Asn Val Arg Leu Ala
                325             330                 335

Ser Asp Leu Thr Gly Trp Gln Leu Asn Ile Met Thr Ser Ala Glu Ala
                340             345                 350

Asp Glu Arg Asn Ala Ala Glu Asp Ala Ala Ile Arg Arg Leu Phe Met
            355             360             365

Asp His Leu Asn Val Asp Glu Glu Thr Ala Asp Val Leu Val Gln Glu
        370             375             380

Gly Phe Ala Thr Leu Glu Glu Val Ala Tyr Val Pro Ala Ala Glu Leu
385             390             395                 400

Leu Ala Ile Glu Gly Phe Asp Glu Glu Ile Val Asp Met Leu Arg Asn
                405             410                 415

Arg Ala Arg Asp Ala Ile Leu Thr Met Ala Ile Ala Ala Glu Glu Lys
            420             425                 430

Leu Gly Glu Val Ser Asp Asp Met Arg Asn Leu Glu Gly Ile Asp Ala
            435             440                 445

Asp Met Leu Arg Ser Leu Ala Glu Ala Gly Ile Thr Thr Arg Asp Asp
    450             455             460

Leu Ala Glu Leu Ala Val Asp Glu Leu Ile Glu Ile Thr Gly Val Asn
465             470             475                 480

Glu Glu Thr Ala Lys Ala Val Ile Leu Thr Ala Arg Glu His Trp Phe
                485             490                 495

Thr Glu Asp Lys
            500
```

<210> 160
<211> 303
<212> PRT
<213> Neisseria meningitidis

<400> 160

```
Met Val His Ile Met Phe Asn Gln Ala Ala Gln Glu Leu Thr Thr Ile
1               5                   10                  15

Arg Asp Ile Leu Arg Phe Ala Val Ser Arg Phe Asn Glu Ala Gly Leu
            20                  25                  30

Phe Phe Gly His Gly Thr Asp Asn Ala His Asp Glu Ala Ala Tyr Leu
        35                  40                  45

Ile Leu His Thr Leu Asn Leu Pro Leu Asp Met Leu Ala Pro Tyr Leu
    50                  55                  60

Asp Ala Lys Leu Leu Glu Ala Glu Lys Glu Glu Val Leu Ala Val Ile
65                  70                  75                  80

Glu Arg Arg Ala Val Glu His Ile Pro Ala Ala Tyr Leu Thr His Gln
                85                  90                  95

Ala Trp Gln Gly Glu Phe Asp Phe Tyr Val Asp Glu Arg Val Ile Ile
            100                 105                 110

Pro Arg Ser Phe Ile Tyr Glu Leu Leu Gly Asp Gly Leu Arg Pro Trp
        115                 120                 125

Ile Glu Tyr Asp Glu Leu Val His Asn Ala Leu Asp Leu Cys Thr Gly
    130                 135                 140

Ser Gly Cys Leu Ala Ile Gln Met Ala His His Tyr Pro Asp Ala Gln

145                 150                 155                 160

Ile Asp Ala Val Asp Val Ser Leu Asp Ala Leu Glu Val Ala Gly Ile
            165                 170                 175

Asn Val Glu Asp Tyr Gly Leu Glu Glu Arg Ile Arg Leu Ile His Thr
            180                 185                 190

Asp Leu Phe Glu Gly Leu Glu Gly Thr Tyr Asp Leu Ile Val Ser Asn
        195                 200                 205

Pro Pro Tyr Val Asp Ala Glu Ser Val Glu Leu Leu Pro Glu Glu Tyr
    210                 215                 220

Leu His Glu Pro Glu Leu Ala Leu Gly Ser Gly Ala Asp Gly Leu Asp
225                 230                 235                 240

Ala Thr Arg Gln Ile Leu Leu Asn Ala Ala Lys Phe Leu Asn Pro Lys
            245                 250                 255

Gly Val Leu Leu Val Glu Ile Gly His Asn Arg Asp Val Leu Glu Ala
        260                 265                 270

Ala Tyr Pro Glu Leu Pro Phe Thr Trp Leu Glu Thr Ser Gly Gly Asp
        275                 280                 285

Gly Phe Val Phe Leu Leu Thr Arg Glu Gln Leu Leu Gly Glu Glu
    290                 295                 300
```

<210> 161
<211> 431
<212> PRT
<213> Neisseria meningitidis

<400> 161

```
Met Leu Asp Ile Gln Leu Leu Arg Ser Asn Thr Ala Ala Val Ala Glu
1               5               10              15

Arg Leu Ala Arg Arg Gly Tyr Asp Phe Asp Thr Ala Arg Phe Asp Thr
        20              25              30

Leu Glu Glu Arg Arg Lys Ser Val Gln Val Lys Thr Glu Glu Leu Gln
        35              40              45

Ala Ser Arg Asn Ser Ile Ser Lys Gln Ile Gly Ala Leu Lys Gly Gln
        50              55              60

Gly Lys His Glu Glu Ala Gln Ala Ala Met Asn Gln Val Ala Gln Ile
65              70              75              80

Lys Thr Asp Leu Glu Gln Ala Ala Ala Asp Leu Asp Ala Val Gln Lys
                85              90              95

Glu Leu Asp Ala Trp Leu Leu Ser Ile Pro Asn Leu Pro His Glu Ser
            100             105             110

Val Pro Ala Gly Lys Asp Glu Thr Glu Asn Val Glu Val Arg Lys Val
        115             120             125

Gly Thr Pro Arg Glu Phe Asp Phe Glu Ile Lys Asp His Val Asp Leu
    130             135             140

Gly Glu Pro Leu Gly Leu Asp Phe Glu Gly Gly Ala Lys Leu Ser Gly
145             150             155             160

Ala Arg Phe Thr Val Met Arg Gly Gln Ile Ala Arg Leu His Arg Ala
            165             170             175

Leu Ala Gln Phe Met Leu Asp Thr His Thr Leu Gln His Gly Tyr Thr
            180             185             190
```

```
Glu His Tyr Thr Pro Tyr Ile Val Asp Asp Thr Thr Leu Gln Gly Thr
        195                 200                 205

Gly Gln Leu Pro Lys Phe Ala Glu Asp Leu Phe His Val Thr Arg Gly
        210                 215                 220

Gly Asp Glu Thr Lys Thr Thr Gln Tyr Leu Ile Pro Thr Ala Glu Val
225                 230                 235                 240

Thr Leu Thr Asn Thr Val Ala Asp Ser Ile Ile Pro Ser Glu Gln Leu
                245                 250                 255

Pro Leu Lys Leu Thr Ala His Ser Pro Cys Phe Arg Ser Glu Ala Gly
        260                 265                 270

Ser Tyr Gly Lys Asp Thr Arg Gly Leu Ile Arg Gln His Gln Phe Asp
        275                 280                 285

Lys Val Glu Met Val Gln Ile Val His Pro Glu Lys Ser Tyr Glu Thr
290                 295                 300

Leu Glu Glu Met Val Gly His Ala Glu Asn Ile Leu Lys Ala Leu Glu
305                 310                 315                 320

Leu Pro Tyr Arg Val Ile Thr Leu Cys Thr Gly Asp Met Gly Phe Gly
                325                 330                 335

Ala Ala Lys Thr Tyr Asp Leu Glu Val Trp Val Pro Ala Gln Asn Thr
        340                 345                 350

Tyr Arg Glu Ile Ser Ser Cys Ser Asn Cys Glu Asp Phe Gln Ala Arg
        355                 360                 365

Arg Leu Lys Ala Arg Phe Lys Asp Glu Asn Gly Lys Asn Arg Leu Val
370                 375                 380

His Thr Leu Asn Gly Ser Gly Leu Ala Val Gly Arg Thr Leu Val Ala
385                 390                 395                 400

Val Leu Glu Asn His Gln Asn Ala Asp Gly Ser Ile Asn Ile Pro Ala
                405                 410                 415

Ala Leu Gln Pro Tyr Met Gly Gly Val Ala Lys Leu Glu Val Lys
        420                 425                 430
```

<210> 162
<211> 285
<212> PRT
<213> Neisseria meningitidis

<400> 162

```
Met Ala Arg His Val Trp Gln Ala Gly Arg Phe Glu Ile Gly Leu Asp
1               5               10              15

Lys Pro Lys Ile Met Gly Ile Val Asn Leu Thr Pro Asp Ser Phe Ser
            20              25              30

Asp Gly Gly Val Tyr Ser Gln Asn Ala Gln Thr Ala Leu Ala His Ala
        35              40              45

Glu Gln Leu Leu Lys Glu Gly Ala Asp Ile Leu Asp Ile Gly Gly Glu
    50              55              60

Ser Thr Arg Ser Gly Ala Asp Tyr Val Ser Pro Glu Glu Glu Trp Ala
65              70              75              80

Arg Val Glu Pro Val Leu Ala Glu Val Ala Gly Trp Gly Val Pro Ile
            85              90              95

Ser Leu Asp Thr Arg Arg Thr Val Ile Met Glu Lys Ala Leu Ala Leu
        100             105             110

Gly Gly Ile Asp Ile Ile Asn Asp Val Ala Ala Leu Asn Asp Glu Gly
        115             120             125

Ala Val Glu Leu Leu Ala Arg Gln Ala Asp Thr Gly Ile Cys Leu Met
    130             135             140

His Met Gln Gly Leu Pro Lys Thr Met Gln Ile Asn Pro Lys Tyr Gln
145             150             155             160

Asp Val Val Gly Glu Val Ala Arg Tyr Leu Lys Ala Arg Ser Ala Glu
            165             170             175

Cys Ile Ala Ala Gly Ile Ala Pro Gln Arg Ile Ile Leu Asp Pro Gly
        180             185             190

Phe Gly Ser Gly Phe Gly Lys Pro Leu Gln His Asn Ile Ala Leu Met
        195             200             205

Arg His Leu Pro Glu Leu Met Ala Glu Thr Gly Phe Pro Leu Leu Ile
    210             215             220

Gly Val Ser Arg Lys Ser Thr Ile Gly Glu Leu Thr Gly Glu Ala Asn
225             230             235             240

Ala Ala Glu Arg Val His Gly Ser Val Ala Ala Ala Leu Ala Ser Val
            245             250             255

Ala Arg Gly Ala Gln Ile Val Arg Val His Asp Val Lys Ala Thr Ala
        260             265             270

Asp Ala Leu Lys Val Trp Glu Ala Leu Gly Ile Asn Leu
        275             280             285
```

<210> 163
<211> 444
<212> PRT
<213> Neisseria meningitidis

<400> 163

Met Thr Asp Leu Asn Thr Leu Phe Ala Asn Leu Lys Gln Arg Asn Pro
1               5                   10                  15

Asn Gln Glu Pro Phe His Gln Ala Val Glu Glu Val Phe Met Ser Leu
            20              25                  30

Asp Pro Phe Leu Ala Lys Asn Pro Lys Tyr Thr Gln Gln Ser Leu Leu
        35              40                  45

Glu Arg Ile Val Glu Pro Glu Arg Val Val Met Phe Arg Val Thr Trp
    50              55                  60

Gln Asp Asp Lys Gly Gln Val Gln Val Asn Arg Gly Tyr Arg Val Gln
65              70                  75                  80

Met Ser Ser Ala Ile Gly Pro Tyr Lys Gly Gly Leu Arg Phe His Pro
            85                  90                  95

Thr Val Asp Leu Gly Val Leu Lys Phe Leu Ala Phe Glu Gln Val Phe
        100             105                 110

Lys Asn Ala Leu Thr Thr Leu Pro Met Gly Gly Gly Lys Gly Gly Ser
        115             120                 125

Asp Phe Asp Pro Lys Gly Lys Ser Asp Ala Glu Val Met Arg Phe Cys
    130             135                 140

Gln Ala Phe Met Thr Glu Leu Tyr Arg His Ile Gly Ala Asp Thr Asp

```
       145                    150                    155                    160
       Val Pro Ala Gly Asp Ile Gly Val Gly Gly Arg Glu Ile Gly Tyr Leu
                       165                 170                 175

       Phe Gly Gln Tyr Lys Lys Ile Arg Asn Glu Phe Ser Ser Val Leu Thr
                       180                 185                 190

       Gly Lys Gly Leu Glu Trp Gly Gly Ser Leu Ile Arg Pro Glu Ala Thr
                   195                 200                 205

       Gly Tyr Gly Cys Val Tyr Phe Ala Gln Ala Met Leu Gln Thr Arg Asn
           210                 215                 220

       Asp Ser Phe Glu Gly Lys Arg Val Leu Ile Ser Gly Ser Gly Asn Val
       225                 230                 235                 240

       Ala Gln Tyr Ala Ala Glu Lys Ala Ile Gln Leu Gly Ala Lys Val Leu
                       245                 250                 255

       Thr Val Ser Asp Ser Asn Gly Phe Val Leu Phe Pro Asp Ser Gly Met
                   260                 265                 270

       Thr Glu Ala Gln Leu Ala Ala Leu Ile Glu Leu Lys Glu Val Arg Arg
                   275                 280                 285

       Glu Arg Val Ala Thr Tyr Ala Lys Glu Gln Gly Leu Gln Tyr Phe Glu
           290                 295                 300

       Lys Gln Lys Pro Trp Gly Val Ala Ala Glu Ile Ala Leu Pro Cys Ala
       305                 310                 315                 320

       Thr Gln Asn Glu Leu Asp Glu Glu Ala Ala Lys Thr Leu Leu Ala Asn
                   325                 330                 335

       Gly Cys Tyr Val Val Ala Glu Gly Ala Asn Met Pro Ser Thr Leu Gly
                   340                 345                 350

       Ala Val Glu Gln Phe Ile Lys Ala Gly Ile Leu Tyr Ala Pro Gly Lys
                   355                 360                 365

       Ala Ser Asn Ala Gly Gly Val Ala Thr Ser Gly Leu Glu Met Ser Gln
           370                 375                 380

       Asn Ala Ile Arg Leu Ser Trp Thr Arg Glu Glu Val Asp Gln Arg Leu
       385                 390                 395                 400

       Phe Gly Ile Met Gln Ser Ile His Glu Ser Cys Leu Lys Tyr Gly Lys
                       405                 410                 415

       Val Gly Asp Thr Val Asn Tyr Val Asn Gly Ala Asn Ile Ala Gly Phe
                   420                 425                 430

       Val Lys Val Ala Asp Ala Met Leu Ala Gln Gly Phe
                   435                 440
```

<210> 164
<211> 412
<212> PRT
<213> Neisseria meningitidis

<400> 164

331

Met Ala Phe Tyr Ala Phe Lys Ala Met Arg Ala Ala Ala Leu Ala Ala
1               5                   10                  15

Ala Val Ala Leu Val Leu Ser Ser Cys Gly Lys Gly Gly Asp Ala Ala
            20                  25                  30

Gln Gly Gly Gln Pro Ala Gly Arg Glu Ala Pro Ala Pro Val Val Gly
            35                  40                  45

```
Val Val Thr Val His Pro Gln Thr Val Ala Leu Thr Val Glu Leu Pro
    50                  55                  60
Gly Arg Leu Glu Ser Leu Arg Thr Ala Asp Val Arg Ala Gln Val Gly
65                  70                  75                  80
Gly Ile Ile Gln Lys Arg Leu Phe Gln Glu Gly Ser Tyr Val Arg Ala
                85                  90                  95
Gly Gln Pro Leu Tyr Gln Ile Asp Ser Ser Thr Tyr Glu Ala Gly Leu
            100                 105                 110
Glu Ser Ala Arg Ala Gln Leu Ala Thr Ala Gln Ala Thr Leu Ala Lys
        115                 120                 125
Ala Asp Ala Asp Leu Ala Arg Tyr Lys Pro Leu Val Ala Ala Glu Ala
        130                 135                 140
Val Ser Arg Gln Glu Tyr Asp Ala Ala Val Thr Ala Lys Arg Ser Ala
145                 150                 155                 160
Glu Ala Gly Val Lys Ala Ala Gln Ala Ala Ile Lys Ser Ala Gly Ile
                165                 170                 175
Ser Leu Asn Arg Ser Arg Ile Thr Ala Pro Ile Ser Gly Phe Ile Gly
            180                 185                 190
Gln Ser Lys Val Ser Glu Gly Thr Leu Leu Asn Ala Gly Asp Ala Thr
        195                 200                 205
Val Leu Ala Thr Ile Arg Gln Thr Asn Pro Met Tyr Val Asn Val Thr
210                 215                 220
Gln Ser Ala Ser Glu Val Met Lys Leu Arg Arg Gln Ile Ala Glu Gly
225                 230                 235                 240
Lys Leu Leu Ala Ala Asp Gly Val Ile Ala Val Gly Ile Lys Phe Asp
                245                 250                 255
Asp Gly Thr Val Tyr Pro Glu Lys Gly Arg Leu Leu Phe Ala Asp Pro
            260                 265                 270
Ala Val Asn Glu Ser Thr Gly Gln Ile Thr Leu Arg Ala Ala Val Pro
        275                 280                 285
Asn Asp Gln Asn Ile Leu Met Pro Gly Leu Tyr Val Arg Val Leu Met
290                 295                 300
Asp Gln Val Ala Val Asp Asn Ala Phe Val Val Pro Gln Gln Ala Val
305                 310                 315                 320
Thr Arg Gly Ala Lys Asp Thr Val Met Ile Val Asn Ala Gln Gly Gly
                325                 330                 335
Met Glu Pro Arg Glu Val Thr Val Ala Gln Gln Gln Gly Thr Asn Trp
            340                 345                 350
Ile Val Thr Ser Gly Leu Lys Asp Gly Asp Lys Val Val Val Glu Gly
        355                 360                 365
Ile Ser Ile Ala Gly Ile Thr Gly Ala Lys Lys Val Thr Pro Lys Glu
    370                 375                 380
Trp Ala Ser Ser Glu Asn Gln Ala Ala Ala Pro Gln Ser Gly Val Gln
385                 390                 395                 400
Thr Ala Ser Glu Ala Lys Pro Ala Ser Glu Ala Lys
                405                 410
```

<210> 165
<211> 190
<212> PRT
<213> Neisseria meningitidis

<400> 165

```
Met Ile Met Ala Lys Lys Ile Ser Ile Leu Val Gly Ser Leu Arg Arg
1               5                   10                  15

Ala Ser Phe Ala Arg Lys Val Ala Leu Asn Ala Ala Glu Met Phe Pro
            20                  25                  30

Glu Gly Trp Gln Ala Glu Ile Val Glu Ile Gly His Leu Pro Leu Tyr
        35                  40                  45

Asn Phe Asp Tyr Asp Asp Pro Ala Val Glu Asp Val Pro Leu Pro Glu
    50                  55                  60

Ser Tyr Thr Ala Phe Arg Glu Thr Ile Lys Ala Ser Asp Gly Ile Leu
65                  70                  75                  80

Phe Val Thr Ser Glu Asn Asn Arg Thr Ile Pro Ala Cys Leu Lys Asn
                85                  90                  95

Ala Val Asp Ile Gly Ser Lys Pro Asn Ala Asp Val Ala Trp Lys Asn
            100                 105                 110

Lys Pro Ala Gly Ile Ile Ser His Ser Val Gly Lys Met Gly Gly Tyr
            115                 120                 125

Ser Ser Gln Lys Asn Leu Arg Leu Ala Leu Ser Tyr Phe Asp Met Pro
    130                 135                 140

Val Thr Gly Gln Pro Glu Val Phe Leu Gly Asn Ser Pro Thr Leu Phe
145                 150                 155                 160

Asp Glu Asn Gly Lys Leu Ile Asp Ser Ala Arg Asp Phe Val Gln Ser
            165                 170                 175

Tyr Ile Asn Gln Phe Val Gly Leu Ile Glu Arg Asn Ala Lys
            180                 185                 190
```

<210> 166
<211> 199
<212> PRT
<213> Neisseria meningitidis

<400> 166

```
Met Asn Asn Leu Ile Lys Ile Asn Leu Leu Pro Tyr Arg Glu Glu Met
1               5                   10                  15

Asn Lys Arg Lys Gln Gln Gln Phe Lys Thr Leu Met Tyr Gly Ala Val
            20              25                  30

Leu Thr Gly Val Ala Ala Val Ala Ala Thr Tyr Leu Phe Ile Asp Asn
        35              40                  45

Met Ile Asn Asn Gln Ser Glu Arg Asn Thr Leu Leu Glu Thr Ser Ile
    50              55                  60

Ala His Leu Asp Thr Glu Leu Ser Glu Ile Gln Lys Leu Lys Gln Glu
65              70                  75                  80

Lys Asp Ala Phe Leu Ile Lys Lys Asn Lys Ile Glu Glu Leu Gln Leu
                85              90                  95

Lys Arg Leu Gln Ala Ala Lys Ile Leu Asp Ser Leu Asn Glu Ala Val
            100             105                 110

Pro Gly Ser Thr Tyr Leu Thr Ser Leu Asp Ala Val Thr Ala Asp Ser

        115                 120         -       125

Tyr Arg Leu Ser Gly Arg Thr Ser Ser Asp Asn Arg Val Ala Ala Met
    130             135                 140

Met Arg Ala Met Pro Asn Thr Gly Ile Phe Lys Gln Pro Glu Leu Leu
145             150                 155                 160

Ser Ile Lys Lys Asn Asn Ser His Gln Glu Phe Thr Leu Gln Ala Thr
            165                 170                 175

Leu Gln Pro Ile Val Lys Ala Ala Glu Ser Lys Glu Asn Pro Ala Ser
        180                 185                 190

Gly Asn Ala Gln Glu Ala Asn
        195
```

<210> 167
<211> 215
<212> PRT
<213> Neisseria meningitidis

<400> 167

```
Met Ala Ser Lys Ser Ser Lys Thr Asn Leu Asp Leu Asn Asn Leu His
1               5               10              15

Leu Leu Asn Leu Pro Ala Arg Leu Phe Ile Ala Leu Leu Ala Val Ala
            20              25              30

Ala Val Leu Gly Leu Gly Tyr Ala Gly Leu Phe Lys Ser Gln Met Glu
        35              40              45

Ser Leu Glu Glu Tyr Glu Ala Lys Glu Thr Glu Leu Lys Asn Thr Tyr
    50              55              60

Lys Gln Lys Ser Ile Asp Ala Ala Ser Leu Asn Asn Leu Arg Asp Glu
65              70              75              80

Leu Ala Ser Ile Arg Ser Ala Phe Asp Ile Met Leu Lys Gln Leu Pro
            85              90              95

Thr Asp Ala Glu Ile Pro Asn Leu Val Gln Glu Leu His Gln Ala Gly
        100             105             110

Ser Ser Asn Gly Leu Arg Leu Asp Ser Val Met Pro Gln Pro Pro Val
        115             120             125

Asp Asp Gly Pro Ile Lys Arg Leu Pro Tyr Ser Ile Ser Ile Thr Gly
    130             135             140

Asn Tyr Glu Gln Ile Ser Gln Phe Thr Arg Asp Val Gly Ser Leu Ser
145             150             155             160

Arg Ile Ile Thr Leu Glu Ser Leu Lys Ile Ala Gln Ser Pro Glu Asn
            165             170             175

Gly Gly Asn Pro Asp Gly Lys Ser Ser Ile Leu Asn Leu Ser Ala Ile
        180             185             190

Ala Thr Thr Tyr Gln Ala Lys Ser Val Glu Glu Leu Ala Ala Glu Ala
        195             200             205

Ala Gln Asn Ala Glu Gln Lys
    210             215
```

<210> 168
<211> 181
<212> PRT
<213> Neisseria meningitidis

<400> 168

```
Met Lys His Tyr Ala Leu Leu Ile Ser Phe Leu Ala Leu Ser Ala Cys
1                5               10              15

Ser Gln Gly Ser Glu Asp Leu Asn Glu Trp Met Ala Gln Thr Arg Arg
             20              25              30

Glu Ala Lys Ala Glu Ile Ile Pro Phe Gln Ala Pro Thr Leu Pro Val
         35              40              45

Ala Pro Val Tyr Ser Pro Pro Gln Leu Thr Gly Pro Asn Ala Phe Asp
     50              55              60

Phe Arg Arg Met Glu Thr Asp Lys Lys Gly Glu Asn Ala Pro Asp Thr
65               70              75              80

Lys Arg Ile Lys Glu Thr Leu Glu Lys Phe Ser Leu Glu Asn Met Arg
             85              90              95

Tyr Val Gly Ile Leu Lys Ser Gly Gln Lys Val Ser Gly Phe Ile Glu
             100             105             110

Ala Glu Gly Tyr Val Tyr Thr Val Gly Val Gly Asn Tyr Leu Gly Gln
         115             120             125

Asn Tyr Gly Arg Ile Glu Ser Ile Thr Asp Asp Ser Ile Val Leu Asn
    130             135             140

Glu Leu Ile Glu Asp Ser Thr Gly Asn Trp Val Ser Arg Lys Ala Glu
145             150             155             160

Leu Leu Leu Asn Ser Ser Asp Lys Asn Thr Glu Gln Ala Ala Ala Pro
             165             170             175

Ala Ala Glu Gln Asn
             180
```

<210> 169
<211> 769
<212> PRT
<213> Neisseria meningitidis

<400> 169

```
Met Asn Thr Lys Leu Thr Lys Ile Ile Ser Gly Leu Phe Val Ala Thr
1               5                   10              15

Ala Ala Phe Gln Thr Ala Ser Ala Gly Asn Ile Thr Asp Ile Lys Val
            20              25              30

Ser Ser Leu Pro Asn Lys Gln Lys Ile Val Lys Val Ser Phe Asp Lys
        35              40              45

Glu Ile Val Asn Pro Thr Gly Phe Val Thr Ser Ser Pro Ala Arg Ile
    50              55              60

Ala Leu Asp Phe Glu Gln Thr Gly Ile Ser Met Asp Gln Gln Val Leu
65              70              75              80

Glu Tyr Ala Asp Pro Leu Leu Ser Lys Ile Ser Ala Ala Gln Asn Ser
            85              90              95

Ser Arg Ala Arg Leu Val Leu Asn Leu Asn Lys Pro Gly Gln Tyr Asn
        100             105             110

Thr Glu Val Arg Gly Asn Lys Val Trp Ile Phe Ile Asn Glu Ser Asp
        115             120             125

Asp Thr Val Ser Ala Pro Ala Arg Pro Ala Val Lys Ala Ala Pro Ala
    130             135             140
```

Ala Pro Ala Lys Gln Gln Ala Ala Ala Pro Ser Thr Lys Ser Ala Val
145             150             155             160

Ser Val Ser Glu Pro Phe Thr Pro Ala Lys Gln Gln Ala Ala Ala Pro
            165             170             175

Phe Thr Glu Ser Val Val Ser Val Ser Ala Pro Phe Ser Pro Ala Lys
            180             185             190

Gln Gln Ala Ala Ala Ser Ala Lys Gln Gln Ala Ala Ala Pro Ala Lys
        195             200             205

Gln Gln Ala Ala Ala Pro Ala Lys Gln Gln Ala Ala Ala Pro Ala Lys
    210             215             220

Gln Thr Asn Ile Asp Phe Arg Lys Asp Gly Lys Asn Ala Gly Ile Ile
225             230             235             240

Glu Leu Ala Ala Leu Gly Phe Ala Gly Gln Pro Asp Ile Ser Gln Gln
            245             250             255

His Asp His Ile Ile Val Thr Leu Lys Asn His Thr Leu Pro Thr Thr
            260             265             270

Leu Gln Arg Ser Leu Asp Val Ala Asp Phe Lys Thr Pro Val Gln Lys
        275             280             285

Val Thr Leu Lys Arg Leu Asn Asn Asp Thr Gln Leu Ile Ile Thr Thr
    290             295             300

Ala Gly Asn Trp Glu Leu Val Asn Lys Ser Ala Ala Pro Gly Tyr Phe
305             310             315             320

Thr Phe Gln Val Leu Pro Lys Lys Gln Asn Leu Glu Ser Gly Gly Val
            325             330             335

Asn Asn Ala Pro Lys Thr Phe Thr Gly Arg Lys Ile Ser Leu Asp Phe
        340             345             350

Gln Asp Val Glu Ile Arg Thr Ile Leu Gln Ile Leu Ala Lys Glu Ser
    355             360             365

Gly Met Asn Ile Val Ala Ser Asp Ser Val Asn Gly Lys Met Thr Leu
    370             375             380

Ser Leu Lys Asp Val Pro Trp Asp Gln Ala Leu Asp Leu Val Met Gln
385             390             395             400

Ala Arg Asn Leu Asp Met Arg Gln Gln Gly Asn Ile Val Asn Ile Ala
            405             410             415

Pro Arg Asp Glu Leu Leu Ala Lys Asp Lys Ala Leu Leu Gln Ala Glu
            420             425             430

Lys Asp Ile Ala Asp Leu Gly Ala Leu Tyr Ser Gln Asn Phe Gln Leu
    435             440             445

Lys Tyr Lys Asn Val Glu Glu Phe Arg Ser Ile Leu Arg Leu Asp Asn
    450             455             460

Ala Asp Thr Thr Gly Asn Arg Asn Thr Leu Ile Ser Gly Arg Gly Ser
465             470             475             480

Val Leu Ile Asp Pro Ala Thr Asn Thr Leu Ile Val Thr Asp Thr Arg
            485             490             495

Ser Val Ile Glu Lys Phe Arg Lys Leu Ile Asp Glu Leu Asp Val Pro
    500             505             510

Ala Gln Gln Val Met Ile Glu Ala Arg Ile Val Glu Ala Ala Asp Gly

339

```
                    515                      520                      525
        Phe Ser Arg Asp Leu Gly Val Lys Phe Gly Ala Thr Gly Lys Lys Lys
            530                 535                 540
        Leu Lys Asn Asp Thr Ser Ala Phe Gly Trp Gly Val Asn Ser Gly Phe
        545                 550                 555                 560
        Gly Gly Asp Asp Lys Trp Gly Ala Glu Thr Lys Ile Asn Leu Pro Ile
                        565                 570                 575
        Thr Ala Ala Ala Asn Ser Ile Ser Leu Val Arg Ala Ile Ser Ser Gly
                    580                 585                 590
        Ala Leu Asn Leu Glu Leu Ser Ala Ser Glu Ser Leu Ser Lys Thr Lys
                595                 600                 605
        Thr Leu Ala Asn Pro Arg Val Leu Thr Gln Asn Arg Lys Glu Ala Lys
            610                 615                 620
        Ile Glu Ser Gly Tyr Glu Ile Pro Phe Thr Val Thr Ser Ile Ala Asn
        625                 630                 635                 640
        Gly Gly Ser Ser Thr Asn Thr Glu Leu Lys Lys Ala Val Leu Gly Leu
                    645                 650                 655
        Thr Val Thr Pro Asn Ile Thr Pro Asp Gly Gln Ile Ile Met Thr Val
                    660                 665                 670
        Lys Ile Asn Lys Asp Ser Pro Ala Gln Cys Ala Ser Gly Asn Gln Thr
                675                 680                 685
        Ile Leu Cys Ile Ser Thr Lys Asn Leu Asn Thr Gln Ala Met Val Glu
            690                 695                 700
        Asn Gly Gly Thr Leu Ile Val Gly Gly Ile Tyr Glu Glu Asp Asn Gly
        705                 710                 715                 720
        Asn Thr Leu Thr Lys Val Pro Leu Leu Gly Asp Ile Pro Val Ile Gly
                    725                 730                 735
        Asn Leu Phe Lys Thr Arg Gly Lys Lys Thr Asp Arg Arg Glu Leu Leu
                740                 745                 750
        Ile Phe Ile Thr Pro Arg Ile Met Gly Thr Ala Gly Asn Ser Leu Arg
                755                 760                 765
        Tyr
```

<210> 170
<211> 431
<212> PRT
<213> Neisseria meningitidis

<400> 170

```
Met Ser Val Ile Gln Asp Leu Gln Ser Arg Gly Leu Ile Ala Gln Thr
1               5               10              15

Thr Asp Ile Glu Ala Leu Asp Ala Leu Leu Asn Glu Gln Lys Ile Ala
            20              25              30

Leu Tyr Cys Gly Phe Asp Pro Thr Ala Asp Ser Leu His Ile Gly His
        35              40              45

Leu Leu Pro Val Leu Ala Leu Arg Arg Phe Gln Gln Ala Gly His Thr
    50              55              60

Pro Ile Ala Leu Val Gly Gly Ala Thr Gly Met Ile Gly Asp Pro Ser
65              70              75              80
```

```
Phe Lys Ala Ala Glu Arg Ser Leu Asn Ser Ala Glu Thr Val Ala Gly
                 85                  90                  95

Trp Val Glu Ser Ile Arg Asn Gln Leu Thr Pro Phe Leu Ser Phe Glu
            100                 105                 110

Gly Gly Asn Ala Ala Ile Met Ala Asn Asn Ala Asp Trp Phe Gly Ser
        115                 120                 125

Met Asn Cys Leu Asp Phe Leu Arg Asp Ile Gly Lys His Phe Ser Val
    130                 135                 140

Asn Ala Met Leu Asn Lys Glu Ser Val Lys Gln Arg Ile Asp Arg Asp
145                 150                 155                 160

Gly Ala Gly Ile Ser Phe Thr Glu Phe Ala Tyr Ser Leu Leu Gln Gly
            165                 170                 175

Tyr Asp Phe Ala Glu Leu Asn Lys Arg His Gly Ala Val Leu Glu Ile
        180                 185                 190

Gly Gly Ser Asp Gln Trp Gly Asn Ile Thr Ala Gly Ile Asp Leu Thr
        195                 200                 205

Arg Arg Leu His Gln Lys Gln Val Phe Gly Leu Thr Leu Pro Leu Val
210                 215                 220

Thr Lys Ser Asp Gly Thr Lys Phe Gly Lys Thr Glu Gly Gly Ala Val
225                 230                 235                 240

Trp Leu Asn Ala Lys Lys Thr Ser Pro Tyr Gln Phe Tyr Gln Phe Trp
            245                 250                 255

Leu Lys Val Ala Asp Ala Asp Val Tyr Lys Phe Leu Lys Tyr Phe Thr
            260                 265                 270

Phe Leu Ser Ile Glu Glu Ile Asp Ala Ile Glu Ala Lys Asp Lys Ala
        275                 280                 285

Ser Gly Ser Lys Pro Glu Ala Gln Arg Ile Leu Ala Glu Glu Met Thr
    290                 295                 300

Arg Leu Ile His Gly Glu Glu Ala Leu Ala Ala Ala Gln Arg Ile Ser
305                 310                 315                 320

Glu Ser Leu Phe Ala Glu Asp Gln Ser Ser Leu Thr Glu Ser Asp Phe
            325                 330                 335

Glu Gln Leu Ala Leu Asp Gly Leu Pro Ala Phe Glu Val Ser Asp Gly
        340                 345                 350

Ile Asn Val Val Glu Ala Leu Val Lys Thr Gly Leu Ala Ser Ser Asn
        355                 360                 365

Lys Glu Ala Arg Gly Phe Val Asn Ser Lys Ala Val Leu Leu Asn Gly
    370                 375                 380

Lys Pro Ala Glu Ala Asn Asn Pro Asn His Ala Ala Glu Arg Pro Asp
385                 390                 395                 400

Asp Ala Cys Leu Leu Asn Gly Glu His Lys Arg Phe Gly Lys Tyr Thr
            405                 410                 415

Ile Leu Arg Arg Gly Lys Arg Asn His Ala Leu Leu Val Trp Lys
            420                 425                 430
```

<210> 171
<211> 363

<212> PRT
<213> Neisseria meningitidis

<400> 171

```
Met Ser Leu Lys Cys Gly Ile Val Gly Leu Pro Asn Val Gly Lys Ser
1               5                   10                  15
Thr Leu Phe Asn Ala Leu Thr Gln Ser Gly Ile Glu Ala Ala Asn Tyr
            20                  25                  30
Pro Phe Cys Thr Ile Glu Pro Asn Val Gly Ile Val Glu Val Pro Asp
        35                  40                  45
Pro Arg Met Ala Glu Leu Ala Lys Ile Val Asn Pro Gln Lys Met Gln
    50                  55                  60
Pro Ala Ile Val Glu Phe Val Asp Ile Ala Gly Leu Val Ala Gly Ala
65                  70                  75                  80
Ser Lys Gly Glu Gly Leu Gly Asn Gln Phe Leu Ala Asn Ile Arg Glu
                85                  90                  95
Thr Asp Ala Ile Val Asn Val Val Arg Cys Phe Asp Asp Asp Asn Ile
            100                 105                 110
Val His Val Ala Gly Arg Val Asp Pro Ile Ala Asp Ile Glu Thr Ile
        115                 120                 125
Gly Thr Glu Leu Ala Leu Ala Asp Leu Ala Ser Val Glu Lys Ala Ile
    130                 135                 140
Val Arg Glu Glu Lys Arg Ala Arg Ser Gly Asp Lys Asp Ala Gln Lys
145                 150                 155                 160
Leu Val Asp Leu Cys Lys Lys Leu Leu Pro His Leu Asp Glu Gly Lys
                165                 170                 175
Pro Val Arg Ser Phe Gly Leu Asp Ala Glu Glu Arg Ala Met Leu Lys
            180                 185                 190
Pro Leu Phe Leu Leu Thr Ala Lys Pro Ala Met Tyr Val Gly Asn Val
        195                 200                 205
Ala Glu Asp Gly Phe Glu Asn Asn Pro His Leu Asp Arg Leu Lys Glu
    210                 215                 220
Leu Ala Ala Lys Glu Asn Ala Pro Val Val Ala Val Cys Ala Ala Met
225                 230                 235                 240
Glu Ser Glu Ile Ala Glu Leu Glu Asp Asp Glu Lys Ala Glu Phe Leu
            245                 250                 255
Ala Glu Met Gly Leu Glu Glu Pro Gly Leu Asn Arg Leu Ile Arg Ala
        260                 265                 270
Gly Tyr Asp Leu Leu Gly Leu Gln Thr Tyr Phe Thr Ala Gly Val Lys
        275                 280                 285
Glu Val Arg Ala Trp Thr Ile His Lys Gly Asp Thr Ala Pro Gln Ala
    290                 295                 300
Ala Gly Val Ile His Thr Asp Phe Glu Arg Gly Phe Ile Arg Ala Gln
305                 310                 315                 320
Val Ile Ser Tyr Asp Asp Phe Val Ser Leu Gly Gly Glu Ala Lys Ala
            325                 330                 335
Lys Glu Ala Gly Lys Met Arg Val Glu Gly Lys Glu Tyr Val Val Gln
        340                 345                 350
Asp Gly Asp Val Met His Phe Leu Phe Asn Val
```

355                    360

<210> 172
<211> 146
<212> PRT
<213> Neisseria meningitidis

<400> 172

```
Met Pro Thr Gln Ser Lys His Ala Ser Ile Asn Ile Gly Leu Ile Gln
1               5               10                  15

Ala Arg Glu Ala Leu Met Thr Gln Phe Arg Pro Ile Leu Asn Gln Ala
            20              25                  30

Asn Ile Thr Asp Gln Gln Trp Arg Ile Ile Arg Leu Leu Ala Glu Asn
        35              40                  45

Gly Thr Leu Asp Phe Gln Asp Leu Ala Asn Gln Ala Cys Ile Leu Arg
    50                  55                  60

Pro Ser Leu Thr Gly Ile Leu Thr Arg Leu Glu Lys Ala Gly Leu Val
65                  70                  75                  80

Val Arg Leu Lys Pro Ser Asn Asp Gln Arg Arg Val Phe Leu Lys Leu
                85              90                  95

Thr Ala Glu Gly Glu Lys Leu Tyr Glu Glu Ile Gly Glu Glu Val Asp
            100             105                 110

Glu Arg Tyr Asp Ala Ile Glu Glu Val Leu Gly Arg Glu Lys Met Leu
        115                 120                 125

Leu Leu Lys Asp Leu Leu Ala Glu Leu Ala Lys Ile Glu Asp Ala Leu
    130                 135                 140

Asn Ser
145
```

<210> 173
<211> 377
<212> PRT
<213> Neisseria meningitidis

<400> 173

```
Met Ser Thr Pro Ala Leu Leu Val Leu Ala Asp Gly Ser Val Phe His
1               5                   10                  15

Gly Thr Ser Ile Gly Tyr Glu Gly Ser Thr Ser Gly Glu Val Val Phe
            20              25                  30

Asn Thr Ser Met Thr Gly Tyr Gln Glu Ile Leu Thr Asp Pro Ser Tyr
        35                  40                  45

Cys Lys Gln Ile Val Thr Leu Thr Tyr Pro His Ile Gly Asn Thr Gly
        50              55                  60

Thr Asn Ala Glu Asp Glu Glu Ser Arg Ser Val Tyr Ala Ala Gly Leu
65              70                  75                  80

Ile Ile Arg Asp Leu Pro Leu Leu His Ser Asn Phe Arg Ala Ser Glu
            85                  90                  95

Ser Leu His Asp Tyr Leu Val Arg Asn Lys Thr Val Ala Ile Ala Asp
            100                 105                 110

Ile Asp Thr Arg Arg Leu Thr Thr Leu Leu Arg Glu Lys Gly Ala Gln
        115                 120                 125

Gly Gly Ala Ile Leu Thr Gly Ala Asp Ala Thr Ile Glu Lys Ala Gln
        130                 135                 140
```

346

```
Glu Leu Ile Ala Ala Phe Gly Ser Met Val Gly Lys Asp Leu Ala Lys
145                 150                 155                 160

Glu Val Ser Cys Thr Glu Thr Tyr Glu Trp Thr Glu Gly Glu Trp Ala
            165                 170                 175

Leu Gly Lys Gly Phe Val Thr Pro Asp Glu Gln Pro Tyr His Val Val
            180                 185                 190

Ala Tyr Asp Phe Gly Val Lys Thr Asn Ile Leu Arg Met Leu Ala Ser
            195                 200                 205

Arg Gly Cys Arg Leu Thr Val Val Pro Ala Gln Thr Ser Ala Glu Asp
    210                 215                 220

Val Leu Ala Leu Asn Pro Asp Gly Val Phe Leu Ser Asn Gly Pro Gly
225                 230                 235                 240

Asp Pro Glu Pro Cys Thr Tyr Ala Ile Lys Ala Val Gln Lys Leu Ile
            245                 250                 255

Glu Ser Gly Lys Pro Ile Phe Gly Ile Cys Leu Gly His Gln Leu Ile
            260                 265                 270

Ser Leu Ala Ile Gly Ala Lys Thr Leu Lys Met Arg Phe Ser His His
            275                 280                 285

Gly Ala Asn His Pro Val Gln Asp Leu Asp Ser Gly Lys Val Val Ile
    290                 295                 300

Thr Ser Gln Asn His Gly Phe Ala Val Asp Ala Asp Thr Leu Pro Ala
305                 310                 315                 320

Asn Ala Arg Ile Thr His Lys Ser Leu Phe Asp Asn Thr Leu Gln Gly
            325                 330                 335

Ile Glu Leu Thr Asp Lys Pro Val Phe Cys Phe Gln Gly His Pro Glu
            340                 345                 350

Ala Ser Pro Gly Pro Gln Asp Val Gly Tyr Leu Phe Asp Lys Phe Ile
            355                 360                 365

Gly Asn Met Lys Ala Ala Lys Arg Ala
370                 375
```

<210> 174  
<211> 213  
<212> PRT  
<213> Neisseria meningitidis

<400> 174

```
Met Ser Ile Pro Ile Asn Phe Asn Asn Leu Lys Tyr Leu Leu Asn Asp
1               5                   10                  15

Met Arg Asn Lys Asn Arg Ile Ile Glu Ala Phe Pro Phe Asn Tyr Asn
            20                  25                  30

Gln Arg Gln Tyr Ala Val Ile Leu Thr Arg Tyr Lys Pro Asp Glu Pro
        35                  40                  45

Arg Pro Asp Asp Tyr Ala Gln Ala Lys Leu Glu Phe Phe Asn Leu Asn
    50                  55                  60

Ser Glu Asn Ser Ile Phe Ala Tyr Ala Asp Phe Tyr Glu Val His Phe
65                  70                  75                  80

Lys Ser Ala Thr Asp Phe Ile Asn Phe Phe Lys Ile Asn Val Gln Ala
            85                  90                  95


Gly Ala Ala Lys Ile Arg Glu Ile Phe Gln Ser Phe Ser Asn Leu Phe
            100                 105                 110

Ala Asp Phe Ile Pro Thr Gln Thr Lys Lys Asp Leu Asp Ile Ile Tyr
        115                 120                 125

Lys Lys Ile Val Ala Thr Arg Leu Glu Pro Asn Ser Pro Asn Thr Ile
    130                 135                 140

Tyr Cys Tyr Asp Val Arg Arg Asn Gly Lys Asp Lys Ala Gly Lys Pro
145                 150                 155                 160

Asn Arg Arg Ser Val Glu Asn Ser Glu Lys Ala Lys Ile Leu Arg Pro
            165                 170                 175

Glu Leu Tyr Glu Lys Phe Lys Ala Asp Ser Asn Tyr Ser Phe Phe Phe
            180                 185                 190

Ser Asp Asn Pro Ser Asp Glu Lys Thr Asp Ala Glu Ile Ile Arg Glu
            195                 200                 205

Val Thr Asn Arg Gln
            210
```

<210> 175
<211> 1071
<212> PRT
<213> Neisseria meningitidis

<400> 175

```
Met Pro Lys Arg Thr Asp Leu Lys Ser Ile Leu Ile Ile Gly Ala Gly
1               5               10              15

Pro Ile Val Ile Gly Gln Ala Cys Glu Phe Asp Tyr Ser Gly Ala Gln
            20              25              30

Ala Cys Lys Ala Leu Arg Glu Glu Gly Tyr Lys Val Ile Leu Val Asn
        35              40              45

Ser Asn Pro Ala Thr Ile Met Thr Asp Pro Glu Met Ala Asp Val Thr
    50              55              60

Tyr Ile Glu Pro Ile Met Trp Gln Thr Val Glu Lys Ile Ile Ala Lys
65              70              75              80

Glu Arg Pro Asp Ala Ile Leu Pro Thr Met Gly Gly Gln Thr Ala Leu
            85              90              95

Asn Cys Ala Leu Asp Leu Ala Arg Asn Gly Val Leu Ala Lys Tyr Asn
            100             105             110

Val Glu Leu Ile Gly Ala Thr Glu Asp Ala Ile Asp Lys Ala Glu Asp
        115             120             125

Arg Gly Arg Phe Lys Glu Ala Met Glu Lys Ile Gly Leu Ser Cys Pro
    130             135             140

Lys Ser Phe Val Cys His Thr Met Asn Glu Ala Leu Ala Ala Gln Glu
145             150             155             160

Gln Val Gly Phe Pro Thr Leu Ile Arg Pro Ser Phe Thr Met Gly Gly
            165             170             175

Ser Gly Gly Gly Ile Ala Tyr Asn Lys Asp Glu Phe Leu Ala Ile Cys
            180             185             190

Glu Arg Gly Phe Asp Ala Ser Pro Thr His Glu Leu Leu Ile Glu Gln
    195             200             205

Ser Val Leu Gly Trp Lys Glu Tyr Glu Met Glu Val Val Arg Asp Lys
```

```
                 210                    215                    220
Asn Asp Asn Cys Ile Ile Ile Cys Ser Ile Glu Asn Phe Asp Pro Met
225             230                235                240

Gly Val His Thr Gly Asp Ser Ile Thr Val Ala Pro Ala Gln Thr Leu
                245                250                255

Thr Asp Lys Glu Tyr Gln Ile Met Arg Asn Ala Ser Leu Ala Val Leu
            260                265                270

Arg Glu Ile Gly Val Asp Thr Gly Gly Ser Asn Val Gln Phe Ala Val
            275                280                285

Asn Pro Ala Asn Gly Glu Met Ile Val Ile Glu Met Asn Pro Arg Val
        290                295                300

Ser Arg Ser Ser Ala Leu Ala Ser Lys Ala Thr Gly Phe Pro Ile Ala
305                310                315                320

Lys Val Ala Ala Lys Leu Ala Val Gly Phe Thr Leu Asp Glu Leu Arg
                325                330                335

Asn Asp Ile Thr Gly Gly Lys Thr Pro Ala Ser Phe Glu Pro Ser Ile
                340                345                350

Asp Tyr Val Val Thr Lys Ile Pro Arg Phe Ala Phe Glu Lys Phe Pro
        355                360                365

Ala Ala Asp Asp Arg Leu Thr Thr Gln Met Lys Ser Val Gly Glu Val
    370                375                380

Met Ala Met Gly Arg Thr Ile Gln Glu Ser Phe Gln Lys Ala Leu Arg
385                390                395                400

Gly Leu Glu Thr Gly Leu Cys Gly Phe Asn Pro Arg Ser Glu Asp Lys
                405                410                415

Ala Glu Ile Arg Arg Glu Leu Ala Asn Pro Gly Pro Glu Arg Met Leu
            420                425                430

Phe Val Ala Asp Ala Phe Arg Ala Gly Phe Thr Leu Glu Glu Ile His
        435                440                445

Glu Ile Cys Ala Ile Asp Pro Trp Phe Leu Ala Gln Ile Glu Asp Leu
    450                455                460

Met Lys Glu Glu Lys Ala Val Ser Asp Gly Ile Leu Ser Asp Leu Asp
465                470                475                480

Phe Ala Ala Leu Arg Arg Leu Lys Arg Lys Gly Phe Ser Asp Lys Arg
            485                490                495

Leu Ala Gln Leu Leu Asn Val Ser Glu Lys Glu Val Arg Glu His Arg
        500                505                510

Tyr Ala Leu Lys Leu His Pro Val Tyr Lys Arg Val Asp Thr Cys Ala
        515                520                525

Ala Glu Phe Ala Thr Glu Thr Ala Tyr Leu Tyr Ser Thr Tyr Glu Glu
    530                535                540

Glu Cys Glu Ser Arg Pro Ser Asp Arg Lys Lys Val Met Ile Leu Gly
545                550                555                560

Gly Gly Pro Asn Arg Ile Gly Gln Gly Ile Glu Phe Asp Tyr Cys Cys
                565                570                575

Val His Ala Ala Leu Ala Leu Arg Glu Ser Gly Phe Glu Thr Ile Met
        580                585                590
```

350

Val Asn Cys Asn Pro Glu Thr Val Ser Thr Asp Phe Asp Thr Ser Asp
595 600 605

Arg Leu Tyr Phe Glu Pro Leu Thr Leu Glu Asp Val Leu Glu Ile Val
610 615 620

Arg Thr Glu Asn Pro Trp Gly Val Ile Val His Tyr Gly Gly Gln Thr
625 630 635 640

Pro Leu Lys Leu Ala Asn Ala Leu Val Glu Asn Gly Val Asn Ile Ile
645 650 655

Gly Thr Ser Ala Asp Ser Ile Asp Ala Ala Glu Asp Arg Glu Arg Phe
660 665 670

Gln Lys Val Leu Asn Asp Leu Gly Leu Arg Gln Pro Pro Asn Arg Ile
675 680 685

Ala His Asn Glu Glu Glu Ala Leu Val Lys Ala Glu Glu Ile Gly Tyr
690 695 700

Pro Leu Val Val Arg Pro Ser Tyr Val Leu Gly Gly Arg Ala Met Gln
705 710 715 720

Val Val His Ser Ala Glu Glu Leu Gln Lys Tyr Met Arg Glu Ala Val
725 730 735

Gln Val Ser Glu Asp Ser Pro Val Leu Leu Asp Phe Phe Leu Asn Asn
740 745 750

Ala Ile Glu Val Asp Val Asp Cys Val Ser Asp Gly Lys Asp Val Val
755 760 765

Ile Gly Gly Ile Met Gln His Val Glu Gln Ala Gly Ile His Ser Gly
770 775 780

Asp Ser Gly Cys Ser Leu Pro Pro Tyr Ser Leu Ser Glu Glu Ile Gln
785 790 795 800

Asp Glu Ile Arg Arg Gln Thr Lys Ala Met Ala Tyr Ala Leu Gly Val
805 810 815

Val Gly Leu Met Asn Val Gln Phe Ala Val Gln Asp Gly Val Val Phe
820 825 830

Val Leu Glu Val Asn Pro Arg Ala Ser Arg Thr Val Pro Phe Val Ser
835 840 845

Lys Ala Thr Gly Val Pro Leu Ala Lys Val Gly Ala Arg Cys Met Ala
850 855 860

Gly Ile Ser Leu Lys Glu Gln Gly Val Glu Lys Glu Val Val Pro Asp
865 870 875 880

Phe Tyr Ala Val Lys Glu Ala Val Phe Pro Phe Ile Lys Phe Pro Gly
885 890 895

Val Asp Thr Ile Leu Gly Pro Glu Met Arg Ser Thr Gly Glu Val Met
900 905 910

Gly Val Gly Ala Ser Phe Gly Glu Ala Tyr Tyr Lys Ala Gln Leu Gly
915 920 925

Ala Gly Glu Arg Leu Asn Pro Thr Gly Lys Ile Phe Leu Ser Val Arg
930 935 940

Glu Glu Asp Lys Glu Arg Val Ile Lys Thr Ala Lys Asn Phe Gln Val
945 950 955 960

```
Leu Gly Tyr Gly Ile Cys Ala Thr Arg Gly Thr Ala Gln Tyr Leu Thr
                965               970               975

Glu His Gly Leu Ile Val Gln Thr Ile Asn Lys Val Pro Glu Gly Arg
            980               985               990

Pro His Ile Gly Asp Ala Leu Lys Asn Gly Glu Ile Ala Leu Val Val
        995              1000              1005

Asn Thr Val Ser Ser Asp Pro Gln Ser Val Ser Asp Ser His Ile Ile
    1010              1015              1020

Arg Gln Ser Ala Leu Gln Gln Arg Val Pro Gln Tyr Thr Thr Thr Ala
1025              1030              1035              1040

Gly Gly Glu Ala Met Ser Glu Gly Ala Lys Ser Arg Asp His Leu Gly
            1045              1050              1055

Val Tyr Ser Val Gln Glu Leu His Gly Arg Leu Lys Asn Arg Asn
        1060              1065              1070
```

<210> 176
<211> 346
<212> PRT
<213> Neisseria meningitidis

<400> 176

```
Met Asp Ile Ser Asp Phe Asp Phe Thr Leu Pro Glu Lys Leu Ile Ala
1               5                   10                  15

Gln His Pro Pro Glu Val Arg Gly Ser Ser Arg Leu Leu Val Ala Leu
            20                  25                  30

Pro Asp Met Pro Leu Gln Asp Arg Val Phe Gly Asp Leu Pro Asp Tyr
        35                  40                  45

Val Glu Ala Gly Asp Val Leu Val Phe Asn Asn Thr Lys Val Met Lys
    50                  55                  60

Ala Arg Leu Phe Gly Gln Lys Asp Ser Gly Gly Arg Ile Glu Ala Leu
65                  70                  75                  80

Ile Glu Arg Val Leu Asp Asn His Thr Ala Leu Ala His Ile Arg Ser
                85                  90                  95

Ser Lys Ser Pro Lys Pro Gly Met Gly Leu Val Phe Glu Gly Gly Ile
            100                 105                 110

Arg Ala Val Thr Val Gly Arg Glu Gly Glu Leu Phe Cys Leu Arg Phe
        115                 120                 125

Glu Gly Gly Glu Thr Val Tyr Glu Leu Leu Glu Gln Asn Gly His Leu
    130                 135                 140

Pro Leu Pro Pro Tyr Ile Glu Arg Ala Ala Asp Ala Asp Asp Asp Ser
145                 150                 155                 160

Arg Tyr Gln Thr Val Tyr Ala Lys Tyr Gln Gly Ala Val Ala Ala Pro
            165                 170                 175

Thr Ala Gly Leu His Phe Thr Glu Glu Leu Leu His Arg Leu Lys Asp
        180                 185                 190

Lys Gly Ala Val Thr Ala Glu Val Thr Leu His Val Gly Ala Gly Thr
        195                 200                 205

Phe Gln Pro Val Arg Val Asp Lys Ile Glu Glu His Lys Met His Ser
    210                 215                 220

Glu Trp Phe Glu Val Pro Ser Glu Thr Ala Ala Ala Val Glu Ala Ala

225                 230                 235                 240

Lys Ala Arg Gly Asn Lys Val Trp Ala Val Gly Thr Thr Ser Met Arg
            245                 250                 255

Ala Leu Glu Ser Ala Ala Arg Ala Thr Gly Arg Leu Lys Ala Gly Gln
        260                 265                 270

Gly Asp Thr Asp Ile Phe Ile Thr Pro Gly Tyr Arg Phe Asn Val Val
        275                 280                 285

Asp Arg Leu Val Thr Asn Phe His Leu Pro Lys Ser Thr Leu Leu Met
    290                 295                 300

Leu Val Ser Ala Phe Ser Gly Met Gly His Ile Arg Ala Ala Tyr Arg
305                 310                 315                 320

His Ala Val Glu Arg Glu Tyr Arg Phe Phe Ser Tyr Gly Asp Ala Met
            325                 330                 335

Val Leu Gly Arg Asn Glu Gly Val Val Arg
            340                 345
```

<210> 177
<211> 453
<212> PRT
<213> Neisseria meningitidis

<400> 177

```
Met Leu Lys Lys Val Leu Ile Ala Asn Arg Gly Glu Ile Ala Leu Arg
1               5                   10                  15

Val Leu Arg Ala Cys Arg Glu Met Gly Ile Ala Thr Val Ala Val His
            20                  25                  30

Ser Glu Ala Asp Lys Asp Ser Leu His Val Lys Leu Ala Asp Glu Ser
        35                  40                  45

Val Cys Ile Gly Pro Ala Ala Ser Ala Gln Ser Tyr Leu Asn Val Pro
    50                  55                  60

Ala Ile Ile Ala Ala Ala Glu Val Ser Cys Ala Asp Ala Val His Pro
65                  70                  75                  80

Gly Tyr Gly Phe Leu Ala Glu Asn Ala Asp Phe Ala Glu Gln Val Glu
                85                  90                  95

Gln Ser Gly Phe Thr Phe Ile Gly Pro Lys Pro Asp Thr Ile Arg Leu
            100                 105                 110

Met Gly Asp Lys Val Ser Ala Lys His Ala Met Ile Ala Ala Gly Val
        115                 120                 125

Pro Cys Val Pro Gly Ser Asp Gly Ala Leu Pro Asp Asp Gly Glu Glu
    130                 135                 140

Ile Leu Lys Ile Ala Asp Lys Val Gly Tyr Pro Val Ile Ile Lys Ala
145                 150                 155                 160

Ser Gly Gly Gly Gly Gly Arg Gly Met Arg Val Val Glu Lys Lys Glu
                165                 170                 175

Asp Leu Leu Gln Ser Val Glu Met Thr Lys Ala Glu Ala Gly Ala Ala
            180                 185                 190

Phe Gly Asn Pro Met Val Tyr Met Glu Arg Tyr Leu Gln Arg Pro Arg
            195                 200                 205

His Val Glu Ile Gln Val Ile Ala Asp Glu His Gly Asn Ala Ile Tyr
    210                 215                 220
```

```
Leu Ala Glu Arg Asp Cys Ser Leu Gln Arg Arg His Gln Lys Val Ile
225             230             235             240

Glu Glu Ala Pro Ala Pro Phe Ile Thr Glu Lys Glu Arg Ala Lys Ile
            245             250             255

Gly Asn Ala Cys Ala Asp Ala Cys Lys Arg Ile Gly Tyr Arg Gly Ala
            260             265             270

Gly Thr Phe Glu Phe Leu Tyr Glu Asp Gly Glu Phe Phe Phe Ile Glu
            275             280             285

Met Asn Thr Arg Val Gln Val Glu His Pro Val Thr Glu Leu Ile Thr
290             295             300

Gly Val Asp Ile Val Gln Glu Gln Leu Arg Ile Ala Ala Gly Leu Pro
305             310             315             320

Leu Gln Tyr Lys Gln Lys Asp Ile Gln Val Glu Gly His Ala Phe Glu
            325             330             335

Cys Arg Ile Asn Ala Glu Asp Pro Tyr Asn Phe Ile Pro Ser Pro Gly
            340             345             350

Leu Ile Glu Ser Cys His Leu Pro Gly Gly Phe Gly Ile Arg Val Asp
            355             360             365

Ser His Ile Tyr Gln Gly Tyr Arg Ile Pro Pro Tyr Tyr Asp Ser Leu
    370             375             380

Ile Gly Lys Ile Cys Val His Gly Lys Thr Arg Glu Gln Ala Met Ala
385             390             395             400

Lys Met Arg Val Ala Leu Ala Glu Leu Ala Val Thr Gly Ile Lys Thr
            405             410             415

Asn Thr Pro Leu His Arg Asp Leu Phe Ala Asp Ala Gly Phe Gln Lys
            420             425             430

Gly Gly Val Ser Ile His Tyr Leu Glu His Trp Leu Glu Asp Arg Lys
            435             440             445

Ala Lys Gln Asp Lys
    450
```

<210> 178
<211> 295
<212> PRT
<213> Neisseria meningitidis

<400> 178

```
Met Pro Tyr Gln Gln Ile Thr Val Asn Val Asn Asp Ala Val Ala Glu
1               5               10                  15

Arg Leu Ala Asp Ala Leu Met Glu His Gly Ala Leu Ser Ala Ala Ile
            20              25                  30

Glu Asp Ala Tyr Ala Gly Thr Gln Asn Glu Gln Ala Ile Phe Gly Glu
        35              40                  45

Pro Gly Met Pro Ala Glu Gln Ile Trp Gln Gln Ser Lys Val Ile Ala
    50              55                  60

Leu Phe Gly Glu His Asp Glu Ala Ala Ala Ile Ile Gln Thr Ala Thr
65              70                  75                  80

Gln Glu Cys Gly Leu Lys Asp Leu Ala Tyr Thr Gly Glu Thr Ile Glu
            85                  90                  95

Asp Gln Asp Trp Val Arg Leu Thr Gln Ser Gln Phe Asp Pro Ile Arg
            100                 105                 110

Ile Ser Asp Arg Leu Trp Ile Thr Pro Ser Trp His Glu Val Pro Glu
        115             120                 125

Gly Ser Ala Val Asn Leu Arg Leu Asp Pro Gly Leu Ala Phe Gly Thr
    130             135                 140

Gly Ser His Pro Thr Thr Arg Leu Cys Leu Lys Trp Leu Asp Thr Gln
145             150                 155                 160

Leu Lys Asn Gly Glu Ser Val Leu Asp Tyr Gly Cys Gly Ser Gly Ile
            165                 170                 175

Leu Thr Ile Ala Ala Leu Lys Leu Gly Ala Gly Phe Ala Val Gly Val
        180             185                 190

Asp Ile Asp Glu Gln Ala Val Arg Ala Gly Lys Asp Asn Ala Ala Gln
    195             200                 205

Asn Asn Val Asp Ala Gln Phe Phe Leu Pro Asp Gly Leu Pro Gln Gly
    210             215                 220

Gln Phe Asp Val Val Val Ala Asn Ile Leu Ala Asn Pro Leu Arg Met
225             230             235                 240

Leu Gly Glu Met Leu Ala Ala Arg Thr Lys Gln Gly Gly Arg Ile Val
            245             250                 255

Leu Ser Gly Leu Leu Asp Glu Gln Ala Glu Glu Leu Gly Gly Ile Tyr
        260             265                 270

Ser Gln Trp Phe Asp Leu Asp Pro Ala Glu Thr Glu Glu Gly Trp Ala
    275             280                 285

Arg Leu Ser Gly Val Lys Arg
    290             295
```

<210> 179
<211> 427
<212> PRT
<213> Neisseria meningitidis

<400> 179

```
Met Asn Arg Asn Glu Ile Leu Phe Asp Arg Ala Lys Ala Ile Ile Pro
1               5               10              15

Gly Gly Val Asn Ser Pro Val Arg Ala Phe Gly Ser Val Gly Gly Val
            20              25              30

Pro Arg Phe Ile Lys Lys Ala Glu Gly Ala Tyr Val Trp Asp Glu Asn
        35              40              45

Gly Thr Arg Tyr Thr Asp Tyr Val Gly Ser Trp Gly Pro Ala Ile Val
        50              55              60

Gly His Ala His Pro Glu Val Val Glu Thr Val Cys Glu Ala Ala Leu
65              70              75              80

Gly Gly Leu Ser Phe Gly Ala Pro Thr Glu Gly Glu Ile Val Ile Ala
            85              90              95

Glu Glu Ile Ala Lys Ile Met Pro Ser Val Glu Arg Leu Arg Leu Val
            100             105             110

Ser Ser Gly Thr Glu Ala Thr Met Thr Ala Ile Arg Leu Ala Arg Gly
            115             120             125

Phe Thr Gly Arg Asp Lys Ile Ile Lys Phe Glu Gly Cys Tyr His Gly
```

```
              130                    135              140

     His Ser Asp Ser Leu Leu Val Lys Ala Gly Ser Gly Leu Leu Thr Phe
     145             150                 155                 160

     Gly Asn Pro Ser Ser Ala Gly Val Pro Ala Asp Phe Thr Lys His Thr
                     165                 170                 175

     Leu Val Leu Glu Tyr Asn Asn Ile Ala Gln Leu Glu Glu Ala Phe Ala
                 180                 185                 190

     Gln Ser Gly Asn Glu Ile Ala Cys Val Ile Val Glu Pro Phe Val Gly
             195                 200                 205

     Asn Met Asn Leu Val Arg Pro Thr Glu Ala Phe Val Lys Ala Leu Arg
         210                 215                 220

     Gly Leu Thr Glu Lys Tyr Gly Ala Val Leu Ile Tyr Asp Glu Val Met
     225                 230                 235                 240

     Thr Gly Phe Arg Val Ala Leu Gly Gly Ala Gln Ser Leu His Gly Ile
                     245                 250                 255

     Thr Pro Asp Leu Thr Thr Met Gly Lys Val Ile Gly Gly Gly Met Pro
                 260                 265                 270

     Leu Ala Ala Phe Gly Gly Arg Lys Asp Ile Met Glu Cys Ile Ser Pro
             275                 280                 285

     Leu Gly Gly Val Tyr Gln Ala Gly Thr Leu Ser Gly Asn Pro Ile Ala
         290                 295                 300

     Val Ala Ala Gly Leu Lys Thr Leu Glu Ile Ile Gln Arg Glu Gly Phe
     305                 310                 315                 320

     Tyr Glu Asn Leu Thr Ala Arg Thr Glu Gln Leu Val Gln Gly Phe Arg
                     325                 330                 335

     Thr Ala Ala Asp Ala Ala Gly Ile Glu Phe Thr Ala Asp Ser Val Gly
                 340                 345                 350

     Gly Met Phe Gly Leu Tyr Phe Ala Ala His Ala Pro Arg Asn Tyr Ala
             355                 360                 365

     Asp Met Ala Arg Ser Asn Ile Glu Gly Phe Lys Gln Phe Phe His Gly
         370                 375                 380

     Met Leu Asp Arg Asn Val Ala Phe Gly Pro Ser Ala Tyr Glu Ala Gly
     385                 390                 395                 400

     Phe Val Ser Ala Ala His Thr Pro Glu Leu Ile Asp Glu Thr Val Ala
                     405                 410                 415

     Val Ala Val Glu Val Phe Lys Ala Met Ala Glu
                 420                 425
```

<210> 180
<211> 518
<212> PRT
<213> Neisseria meningitidis

<400> 180

358

```
Met Thr Leu Ala Glu Phe Trp Pro Leu Cys Leu Arg Arg Leu His Asp
1               5               10                  15

Met Leu Pro Gln Gly Gln Phe Ala Gln Trp Ile Ala Pro Leu Thr Val
            20              25                  30

Gly Glu Glu Gly Gly Val Trp Val Val Tyr Gly Lys Asn Gln Phe Ala
        35              40                  45
```

```
Cys Asn Met Leu Lys Ser Gln Phe Ala Gly Lys Ile Glu Ala Val Arg
    50                  55                  60

Glu Glu Leu Ala Ala Gly Arg Ser Ala Phe Val Phe Lys Pro Gly Glu
65                  70                  75                  80

Gly Val Arg Tyr Glu Met Ala Ala Val Glu Gly Ala Val Glu Pro Ala
                85                  90                  95

Glu Pro Ser Leu His Ala Val Ser Glu Gly Met Pro Val Gln Glu Val
                100                 105                 110

Leu Leu Asp Glu Leu Pro Ser Glu Glu Pro Val Lys Pro Ala Ala Ser
        115                 120                 125

Lys Thr Ala Ala Asp Ile Leu Ala Glu Arg Met Lys Asn Leu Pro His
    130                 135                 140

Glu Pro Arg Gln Ala Ala Gly Ser Ala Ser Arg Pro Glu Ser Val Ala
145                 150                 155                 160

Val Ala Lys Ala Arg Thr Asp Val Gln Arg Asp Ala Glu Glu Ala Arg
                165                 170                 175

Tyr Glu Gln Thr Asn Leu Ser Pro Asp Tyr Thr Phe Asp Thr Leu Val
                180                 185                 190

Glu Gly Lys Gly Asn Arg Leu Ala Ala Ala Ala Gln Ala Ile Ala
                195                 200                 205

Glu Ser Pro Gly Gln Ser Tyr Asn Pro Phe Phe Leu Tyr Gly Ser Thr
210                 215                 220

Gly Leu Gly Lys Thr His Leu Val Gln Ala Val Gly Asn Glu Leu Leu
225                 230                 235                 240

Lys Asn Arg Pro Asp Ala Lys Val Arg Tyr Met His Ser Asp Asp Tyr
                245                 250                 255

Ile Arg Ser Phe Met Lys Ala Val Arg Asn Asn Thr Tyr Asp Val Phe
            260                 265                 270

Lys Gln Gln Tyr Lys Gln Tyr Asp Leu Leu Ile Ile Asp Asp Ile Gln
    275                 280                 285

Phe Ile Lys Gly Lys Asp Arg Thr Met Glu Glu Phe Phe Tyr Leu Tyr
    290                 295                 300

Asn His Phe His Asn Glu Lys Lys Gln Leu Ile Leu Thr Cys Asp Val
305                 310                 315                 320

Leu Pro Ala Lys Ile Glu Gly Met Asp Asp Arg Leu Lys Ser Arg Phe
                325                 330                 335

Ser Trp Gly Leu Thr Leu Glu Leu Glu Pro Pro Glu Leu Glu Met Arg
                340                 345                 350

Ile Ala Ile Leu Gln Lys Lys Ala Glu Ala Ala Gly Ile Ser Ile Glu
            355                 360                 365

Asp Glu Ala Ala Leu Phe Ile Ala Asn Leu Ile Arg Ser Asn Val Arg
    370                 375                 380

Glu Leu Glu Gly Ala Phe Asn Arg Val Gly Ala Ser Ser Arg Phe Met
385                 390                 395                 400

Asn Arg Pro Val Ile Asp Ile Asp Leu Ala Arg Thr Ala Leu Gln Asp
                405                 410                 415
```

360

```
Ile Ile Ala Glu Lys His Lys Val Ile Thr Ala Asp Ile Ile Ile Asp
            420             425         430

Ala Val Ala Lys Tyr Tyr Arg Ile Lys Ile Ser Asp Val Leu Gly Lys
            435             440         445

Lys Arg Thr Arg Asn Ile Ala Arg Pro Arg Gln Val Ala Met Ser Leu
    450             455         460

Thr Lys Glu Leu Thr Thr Leu Ser Leu Pro Ser Ile Gly Asp Ser Phe
465             470         475             480

Gly Gly Arg Asp His Thr Thr Val Met His Gly Ile Arg Ala Val Ala
            485             490         495

Lys Leu Arg Glu Glu Asp Pro Glu Leu Ala Gln Asp Tyr Glu Lys Leu
            500         505         510

Leu Ile Leu Ile Gln Asn
            515
```

<210> 181
<211> 521
<212> PRT
<213> Neisseria meningitidis

<400> 181

```
Met Thr Gln Asp Lys Ile Leu Ile Leu Asp Phe Gly Ser Gln Val Thr
1             5                   10              15

Gln Leu Ile Ala Arg Arg Val Arg Glu Ala His Val Tyr Cys Glu Leu
            20              25              30

His Ser Phe Asp Met Pro Leu Asp Glu Ile Lys Ala Phe Asn Pro Lys
        35              40              45

Gly Ile Ile Leu Ser Gly Gly Pro Asn Ser Val Tyr Glu Ser Asp Tyr
        50              55              60

Gln Ala Asp Thr Gly Ile Phe Asp Leu Gly Ile Pro Val Leu Gly Ile
65              70              75              80

Cys Tyr Gly Met Gln Phe Met Ala His His Leu Gly Gly Glu Val Gln
                85              90              95

Pro Gly Asn Gln Arg Glu Phe Gly Tyr Ala Gln Val Lys Thr Ile Asp
            100             105             110

Ser Glu Leu Thr Arg Gly Ile Gln Asp Gly Glu Pro Asn Thr Leu Asp
        115             120             125

Val Trp Met Ser His Gly Asp Lys Val Ser Lys Leu Pro Asp Gly Phe
    130             135             140

Ala Val Ile Gly Asn Thr Pro Ser Cys Pro Ile Ala Met Met Glu Asn
145             150             155             160

Ala Glu Lys Gln Phe Tyr Gly Ile Gln Phe His Pro Glu Val Thr His
            165             170             175

Thr Lys Gln Gly Arg Ala Leu Leu Asn Arg Phe Val Leu Asp Ile Cys
            180             185             190

Gly Ala Gln Pro Gly Trp Thr Met Pro Asn Tyr Ile Glu Glu Ala Val
            195             200             205

Ala Lys Ile Arg Glu Gln Val Gly Ser Asp Glu Val Ile Leu Gly Leu
    210             215             220

Ser Gly Gly Val Asp Ser Ser Val Ala Ala Ala Leu Ile His Arg Ala
```

```
          225                    230              235                    240

          Ile Gly Asp Gln Leu Thr Cys Val Phe Val Asp His Gly Leu Leu Arg
                          245                 250                 255

          Leu Asn Glu Ser Lys Met Val Met Asp Met Phe Ala Arg Asn Leu Gly
                      260                 265                 270

          Val Lys Val Ile His Val Asp Ala Glu Gly Gln Phe Met Ala Lys Leu
                      275                 280                 285

          Ala Gly Val Thr Asp Pro Glu Lys Lys Arg Lys Ile Ile Gly Ala Glu
                  290                 295                 300

          Phe Ile Glu Val Phe Asp Ala Glu Glu Lys Lys Leu Thr Asn Ala Lys
          305                 310                 315                 320

          Trp Leu Ala Gln Gly Thr Ile Tyr Pro Asp Val Ile Glu Ser Ala Gly
                          325                 330                 335

          Ala Lys Thr Lys Lys Ala His Ala Ile Lys Ser His His Asn Val Gly
                      340                 345                 350

          Gly Leu Pro Glu Asn Met Lys Leu Lys Leu Leu Glu Pro Leu Arg Asp
                      355                 360                 365

          Leu Phe Lys Asp Glu Val Arg Glu Leu Gly Val Ala Leu Gly Leu Pro
              370                 375                 380

          Arg Glu Met Val Tyr Arg His Pro Phe Pro Gly Pro Gly Leu Gly Val
          385                 390                 395                 400

          Arg Ile Leu Gly Glu Val Lys Lys Glu Tyr Ala Asp Leu Leu Arg Gln
                          405                 410                 415

          Ala Asp Asp Ile Phe Ile Gln Glu Leu Arg Asn Thr Thr Asp Glu Asn
                      420                 425                 430

          Gly Thr Ser Trp Tyr Asp Leu Thr Ser Gln Ala Phe Ala Val Phe Leu
                      435                 440                 445

          Pro Val Lys Ser Val Gly Val Met Gly Asp Gly Arg Thr Tyr Asp Tyr
              450                 455                 460

          Val Ile Ala Leu Arg Ala Val Ile Thr Ser Asp Phe Met Thr Ala His
          465                 470                 475                 480

          Trp Ala Glu Leu Pro Tyr Ser Leu Leu Gly Lys Val Ser Asn Arg Ile
                          485                 490                 495

          Ile Asn Glu Val Lys Gly Ile Asn Arg Val Val Tyr Asp Val Ser Gly
                      500                 505                 510

          Lys Pro Pro Ala Thr Ile Glu Trp Glu
                      515                 520
```

<210> 182
<211> 248
<212> PRT
<213> Neisseria meningitidis

<400> 182

```
Met Ser Thr Gln Asp Leu Asn Gly Lys Ile Ala Leu Val Thr Gly Ala
1               5               10              15

Ser Arg Gly Ile Gly Ala Ala Ile Ala Asp Thr Leu Ala Ala Ala Gly
            20              25              30

Ala Lys Val Ile Gly Thr Ala Thr Ser Glu Ser Gly Ala Ala Ala Ile
        35              40              45


Ser Glu Arg Leu Ala Gln Trp Gly Gly Glu Gly Arg Val Leu Asn Ser
    50              55              60

Ala Glu Pro Glu Thr Ile Glu Ser Leu Ile Ala Asp Ile Glu Lys Ala
65              70              75              80

Phe Gly Lys Leu Asp Ile Leu Val Asn Asn Ala Gly Ile Thr Arg Asp
                85              90              95

Asn Leu Leu Met Arg Met Lys Glu Glu Glu Trp Asp Asp Ile Met Gln
            100             105             110

Val Asn Leu Lys Ser Val Phe Arg Ala Ser Lys Ala Val Leu Arg Gly
            115             120             125

Met Met Lys Gln Arg Ser Gly Arg Ile Ile Asn Ile Thr Ser Val Val
    130             135             140

Gly Val Met Gly Asn Ala Gly Gln Thr Asn Tyr Ala Ala Ala Lys Ala
145             150             155             160

Gly Leu Ile Gly Phe Ser Lys Ser Met Ala Arg Glu Val Gly Ser Arg
                165             170             175

Gly Ile Thr Val Asn Cys Val Ala Pro Gly Phe Ile Asp Thr Asp Met
            180             185             190

Thr Arg Ala Leu Pro Glu Glu Thr Arg Gln Thr Phe Thr Ala Gln Thr
    195             200             205

Ala Leu Gly Arg Phe Gly Asp Ala Gln Asp Ile Ala Asp Ala Val Leu
    210             215             220

Phe Leu Ala Ser Asp Gln Ala Lys Tyr Ile Thr Gly Gln Thr Leu His
225             230             235             240

Val Asn Gly Gly Met Leu Met Pro
            245
```

<210> 183
<211> 687
<212> PRT
<213> Neisseria meningitidis

<400> 183

Met Met Thr Gln Thr Leu Leu Ile Glu Leu Leu Thr Glu Glu Leu Pro
1           5               10              15

Pro Lys Ala Leu Asn Asn Leu Gly Asn His Phe Ala Ala Ser Val Ala
            20              25              30

Glu Gly Leu Glu Lys Ala Gln Leu Val Asp Gly Ala Ala Glu Phe Thr
            35              40              45

Ala Tyr Ala Ser Pro Arg Arg Leu Ala Val Gln Val Lys Asn Val Lys
        50              55              60

Ala Val Gln Ala Asp Gln Lys Ile Val Lys Lys Gly Pro Ala Val Ala
65              70              75              80

Asn Ala Met Lys Asp Gly Ala Pro Thr Lys Ala Leu Glu Gly Phe Ala
                85              90              95

Arg Gly Ala Gly Ala Lys Ile Glu Asp Leu Thr Ile Val His Asp Gly
            100             105             110

Lys Gln Asp Val Tyr Ala Tyr Glu Tyr Val Gln Ile Gly Lys Pro Leu
            115             120             125

Gly Gly Leu Leu Glu Asp Ile Ile Asn Gln Ala Val Lys Lys Leu Pro
    130                     135                 140

Ile Pro Lys Val Met Arg Trp Gly Ser Ser Thr Phe Thr Phe Val Arg
145                 150                 155                 160

Pro Val His Gly Leu Val Val Leu His Gly Gly Asp Ile Val Asn Val
                165                 170                 175

Ser Val Leu Gly Leu Gln Ser Gly Asn Lys Thr Leu Gly His Arg Phe
                180                 185                 190

Leu Ser Asp Gly Glu Ile Thr Ile Glu Asn Ala Asp Ser Tyr Ala Ala
        195                 200                 205

Gln Met Arg Glu Gln Gly Lys Val Val Ala Ser Phe Ala Glu Arg Lys
    210                 215                 220

Ala Ala Ile Gln Thr Val Leu Glu Gly Gln Ala Arg Arg Leu Asn Ala
225                 230                 235                 240

Thr Ala Ala Ala Asp Glu Ala Leu Leu Asp Glu Val Thr Ala Leu Val
                245                 250                 255

Glu Trp Pro Val Val Leu Glu Ala Gly Phe Glu Glu His Phe Leu Ala
                260                 265                 270

Val Pro Gln Glu Cys Leu Ile Leu Thr Met Gln Gln Asn Gln Lys Tyr
        275                 280                 285

Phe Pro Leu Leu Asp Gln Asn Gly Lys Leu Met Asn Arg Phe Leu Leu
    290                 295                 300

Val Ser Asn Leu Gln Thr Glu Asp Pro Ser His Ile Ile Gln Gly Asn
305                 310                 315                 320

Glu Arg Val Leu Arg Ala Arg Leu Ser Asp Ala Glu Phe Phe Tyr Lys
                325                 330                 335

Gln Asp Gln Lys Ala Thr Leu Glu Ser Arg Leu Pro Lys Leu Thr Asn
        340                 345                 350

Val Val Tyr His Asn Lys Ile Gly Ser Gln Ala Glu Arg Ile Glu Arg
        355                 360                 365

Leu Gln Ser Ile Ala Ala His Ile Ala Lys Ala Leu Gly Ala Asp Ala
    370                 375                 380

Ala Ala Ala Glu Arg Ala Ala Arg Leu Ala Lys Ala Asp Leu Val Thr
385                 390                 395                 400

Glu Met Val Gly Glu Phe Pro Glu Leu Gln Gly Thr Met Gly Lys Tyr
                405                 410                 415

Tyr Ala Arg Leu Asp Gly Glu Thr Glu Glu Ile Thr Glu Ala Val Glu
        420                 425                 430

Gln His Tyr Gln Pro Arg Phe Ala Gly Asp Asn Leu Pro Glu Gly Lys
        435                 440                 445

Ile Ala Ala Ala Val Ala Leu Ala Asp Lys Leu Glu Thr Leu Val Gly
    450                 455                 460

Ile Trp Gly Ile Gly Leu Ile Pro Thr Gly Asp Lys Asp Pro Tyr Ala
465                 470                 475                 480

Leu Arg Arg Ala Ala Leu Gly Ile Leu Arg Met Leu Met Gln Tyr Gly
                485                 490                 495

Leu Asp Val Asn Glu Leu Ile Gln Thr Ala Phe Asn Ser Phe Pro Gln

```
                500                    505                    510
    Gly Leu Leu Asn Glu Lys Thr Pro Ser Glu Thr Ala Asp Phe Met Gln
            515                  520                  525

    Ala Arg Leu Ala Val Leu Leu Gln Asn Asp Tyr Pro Gln Asp Ile Val
        530              535                  540

    Ala Ala Val Leu Ala Lys Gln Pro Arg Arg Leu Asp Asp Leu Thr Ala
    545              550                  555                  560

    Lys Leu Gln Ala Val Ala Ala Phe Lys Gln Leu Pro Glu Ala Ala Ala
                565                  570                  575

    Leu Ala Ala Ala Asn Lys Arg Val Gln Asn Leu Leu Lys Lys Ala Asp
                580                  585                  590

    Ala Glu Leu Gly Ala Val Asn Glu Ser Leu Leu Gln Gln Asp Glu Glu
            595                  600                  605

    Lys Ala Leu Phe Ala Ala Ala Gln Gly Leu Gln Pro Lys Ile Ala Ala
        610                  615                  620

    Ala Val Ala Glu Gly Asn Phe Gln Thr Ala Leu Ser Glu Leu Ala Ser
    625                  630                  635                  640

    Val Lys Pro Gln Val Asp Ala Phe Phe Asp Gly Val Met Val Met Ala
                645                  650                  655

    Glu Asp Ala Ala Val Lys Gln Asn Arg Leu Asn Leu Leu Asn Arg Leu
                660                  665                  670

    Ala Glu Gln Met Asn Ala Val Ala Asp Ile Ala Leu Leu Gly Glu
            675                  680                  685
```

<210> 184
<211> 465
<212> PRT
<213> Neisseria meningitidis

<400> 184

```
Met Ser Gln Gly Lys Ile Val Gln Ile Ile Gly Ala Val Val Asp Val
1               5                   10              15

Glu Phe Pro Arg Asp Met Ile Pro Arg Val Tyr Asp Ala Leu Lys Leu
            20              25              30

Asp Glu Asn Gly Leu Thr Leu Glu Val Gln Gln Leu Leu Gly Asp Gly
        35              40              45

Val Val Arg Ala Ile Ala Met Gly Ser Ser Asp Gly Leu Lys Arg Gly
    50              55              60

Met Thr Val Ser Asn Thr Gly Ala Pro Ile Thr Val Pro Val Gly Lys
65              70              75              80

Gly Thr Leu Gly Arg Ile Val Asp Val Leu Gly Thr Pro Val Asp Glu
            85              90              95

Ala Gly Pro Ile Asp Thr Asp Lys Ser Arg Ala Ile His Gln Ala Ala
        100             105             110

Pro Lys Phe Asp Glu Leu Ser Ser Thr Thr Glu Leu Leu Glu Thr Gly
        115             120             125

Ile Lys Val Ile Asp Leu Leu Cys Pro Phe Ala Lys Gly Gly Lys Val
130             135             140

Gly Leu Phe Gly Gly Ala Gly Val Gly Lys Thr Val Asn Met Met Glu
145             150             155             160
```

```
        Leu Ile Asn Asn Ile Ala Lys Ala His Ser Gly Leu Ser Val Phe Ala
                        165             170                 175

        Gly Val Gly Glu Arg Thr Arg Glu Gly Asn Asp Phe Tyr His Glu Met
                    180             185                 190

        Lys Asp Ser Asn Val Leu Asp Lys Val Ala Met Val Tyr Gly Gln Met
                195                 200                 205

        Asn Glu Pro Pro Gly Asn Arg Leu Arg Val Ala Leu Thr Gly Leu Thr
            210                 215                 220

        Met Ala Glu Tyr Phe Arg Asp Glu Lys Asp Glu Asn Gly Lys Gly Arg
        225                 230                 235                 240

        Asp Val Leu Phe Phe Val Asp Asn Ile Tyr Arg Tyr Thr Leu Ala Gly
                        245             250                 255

        Thr Glu Val Ser Ala Leu Leu Gly Arg Met Pro Ser Ala Val Gly Tyr
                    260             265                 270

        Gln Pro Thr Leu Ala Glu Glu Met Gly Arg Leu Gln Glu Arg Ile Thr
                    275             280                 285

        Ser Thr Gln Thr Gly Ser Ile Thr Ser Ile Gln Ala Val Tyr Val Pro
            290                 295                 300

        Ala Asp Asp Leu Thr Asp Pro Ser Pro Ala Thr Thr Phe Ala His Leu
        305                 310                 315                 320

        Asp Ala Thr Val Val Leu Ser Arg Asp Ile Ala Ser Leu Gly Ile Tyr
                        325             330                 335

        Pro Ala Val Asp Pro Leu Asp Ser Thr Ser Arg Gln Leu Asp Pro Met
                    340             345                 350

        Val Leu Gly Gln Glu His Tyr Asp Val Ala Arg Gly Val Gln Ser Thr
                    355             360                 365

        Leu Gln Lys Tyr Lys Glu Leu Arg Asp Ile Ile Ala Ile Leu Gly Met
            370                 375                 380

        Asp Glu Leu Ser Asp Glu Asp Lys Leu Thr Val Met Arg Ala Arg Lys
        385                 390                 395                 400

        Ile Gln Arg Phe Leu Ser Gln Pro Phe His Val Ala Glu Val Phe Thr
                        405             410                 415

        Gly Ser Pro Gly Lys Tyr Val Ala Leu Arg Asp Thr Ile Ala Gly Phe
                    420             425                 430

        Lys Ala Ile Leu Asn Gly Glu Tyr Asp His Leu Pro Glu Gln Ala Phe
                    435             440                 445

        Tyr Met Val Gly Ser Ile Glu Glu Ala Val Glu Lys Ala Lys Thr Leu
        450                 455                 460

        Asn
        465
```

<210> 185

<211> 515

<212> PRT

<213> Neisseria meningitidis

<400> 185

Met Gln Leu Asn Pro Ala Glu Ile Ser Asp Leu Ile Lys Ala Lys Ile
1                   5                   10                  15

```
Glu Asn Leu Ser Val Asn Ala Glu Val Arg Thr Cys Gly Thr Val Ile
            20                  25                  30

Ser Val Thr Asp Gly Ile Val Arg Ile His Gly Leu Ser Asp Ala Met
            35                  40                  45

Gln Gly Glu Met Leu Glu Phe Pro Gly Asn Thr Phe Gly Leu Ala Met
    50                  55                  60

Asn Leu Glu Arg Asp Ser Val Gly Ala Val Val Leu Gly Glu Tyr Glu
65                  70                  75                  80

His Ile Lys Glu Gly Asp Thr Val Thr Cys Thr Gly Arg Ile Leu Glu
                85                  90                  95

Val Pro Val Gly Arg Glu Leu Val Gly Arg Val Val Asp Ala Leu Gly
                100                 105                 110

Arg Pro Ile Asp Gly Lys Gly Pro Ile Asn Thr Thr Leu Thr Ala Pro
        115                 120                 125

Ile Glu Lys Ile Ala Pro Gly Val Ile Ala Arg Lys Ser Val Asp Gln
    130                 135                 140

Pro Met Gln Thr Gly Leu Lys Ala Ile Asp Ser Met Val Pro Val Gly
145                 150                 155                 160

Arg Gly Gln Arg Glu Leu Ile Ile Gly Asp Arg Gln Thr Gly Lys Thr
                165                 170                 175

Ala Val Ala Leu Asp Ala Ile Val Asn Gln Lys Gly Thr Gly Val Ile
            180                 185                 190

Cys Ile Tyr Val Ala Ile Gly Gln Lys Ala Ser Ser Ile Ala Asn Val
        195                 200                 205

Val Arg Lys Leu Glu Glu His Gly Ala Met Glu His Thr Ile Val Val
    210                 215                 220

Ala Ala Thr Ala Ser Glu Ala Ala Ala Leu Gln Tyr Ile Ala Pro Tyr
225                 230                 235                 240

Ser Gly Cys Thr Met Gly Glu Phe Phe Arg Asp Arg Gly Glu Asp Ala
                245                 250                 255

Leu Ile Val Tyr Asp Asp Leu Ser Lys Gln Ala Val Ala Tyr Arg Gln
            260                 265                 270

Ile Ser Leu Leu Leu Arg Arg Pro Pro Gly Arg Glu Ala Tyr Pro Gly
        275                 280                 285

Asp Val Phe Tyr Leu His Ser Arg Leu Leu Glu Arg Ala Ala Arg Val
    290                 295                 300

Asn Glu His Glu Val Glu Lys Leu Thr Asn Gly Glu Val Lys Gly Lys
305                 310                 315                 320

Thr Gly Ser Leu Thr Ala Leu Pro Ile Ile Glu Thr Gln Ala Gly Asp
                325                 330                 335

Val Ser Ala Phe Val Pro Thr Asn Val Ile Ser Ile Thr Asp Gly Gln
            340                 345                 350

Ile Phe Leu Glu Thr Asp Leu Phe Asn Ala Gly Ile Arg Pro Ala Ile
        355                 360                 365

Asn Ala Gly Ile Ser Val Ser Arg Val Gly Gly Ala Ala Gln Thr Lys
    370                 375                 380

Val Ile Lys Lys Leu Gly Gly Gly Ile Arg Leu Ala Leu Ala Gln Tyr
```

```
     385                      390                      395                      400
     Arg Glu Leu Ala Ala Phe Ser Gln Phe Ala Ser Asp Leu Asp Glu Ala
                     405                 410                 415

     Thr Arg Lys Gln Leu Glu His Gly Glu Val Val Thr Glu Leu Met Lys
                     420                 425                 430

     Gln Lys Gln Phe Ser Thr Leu Asn Thr Ala Glu Met Ala Leu Thr Leu
                     435                 440                 445

     Trp Ala Ile Asn Asn Gly Ser Tyr Ser Asp Val Pro Val Ala Lys Ala
         450                 455                 460

     Leu Ala Phe Glu Ser Glu Phe Leu Ser Phe Val Arg Thr Gln His Pro
     465                 470                 475                 480

     Glu Val Leu Glu Ala Val Asn Ala Ser Gly Ala Met Ser Asp Glu Ser
                     485                 490                 495

     Glu Lys Thr Leu Glu Ala Ala Met Lys Ser Phe Lys Ser Ser Tyr Ala
                     500                 505                 510

     Tyr Gln Ala
             515
```

<210> 186
<211> 177
<212> PRT
<213> Neisseria meningitidis

<400> 186

```
Met Ala Glu Phe Ala Thr Ile Ala Arg Pro Tyr Ala Lys Ala Leu Phe
1               5                   10              15

Gly Leu Ala Gln Glu Lys Asn Gln Ile Glu Ser Trp Leu Gly Gly Leu
            20              25                  30

Glu Lys Leu Ala Ala Val Val Gln Glu Gly Lys Val Ala Ser Leu Ile
        35              40                  45

Asp Arg Pro Glu Thr Asn Ala Ser Glu Lys Ala Asp Ile Leu Ile Asp
        50              55              60

Leu Val Gly Leu Lys Asp Lys Glu Leu Lys Asn Phe Val Ile Val Leu
65              70                  75                  80

Ala Gly Gln Lys Arg Leu Ser Ile Leu Pro Glu Val Tyr Ala Gln Tyr
            85              90                  95

Gln Asp Leu Thr Leu Ser Phe Asn His Ile Lys Ser Ala Val Ile Tyr
        100             105                 110

Ser Ala Tyr Pro Leu Thr Asp Lys Gln Val Gly Glu Leu Val Gln Met
        115             120                 125

Leu Asn Lys Arg Phe Asp Ser Glu Leu Lys Ile Ser Val Glu Ile Glu
    130             135                 140

Pro Glu Leu Ile Gly Gly Ile Lys Val Glu Val Gly Asp Gln Val Leu
145             150                 155                 160

Asp Leu Ser Val Gln Gly Lys Leu Ser Ala Leu Tyr Thr Thr Met Thr
            165             170                 175

Asn
```

<210> 187
<211> 156
<212> PRT
<213> Neisseria meningitidis

<400> 187

```
Met Asn Ile Asn Ala Thr Leu Phe Ala Gln Ile Ile Val Phe Phe Gly
1               5                   10                  15

Leu Val Trp Phe Thr Met Lys Phe Val Trp Pro Pro Ile Ala Lys Ala
            20                  25                  30

Leu Asp Glu Arg Ala Ala Lys Val Ala Glu Gly Leu Ala Ala Ala Glu
        35                  40                  45

Arg Gly Lys Ser Asp Phe Glu Gln Ala Glu Lys Lys Val Ala Glu Leu
    50                  55                  60

Leu Ala Glu Gly Arg Asn Gln Val Ser Glu Met Val Ala Asn Ala Glu
65                  70                  75                  80

Lys Arg Ala Ala Lys Ile Val Glu Glu Ala Lys Glu Gln Ala Ser Ser
                85                  90                  95

Glu Ala Ala Arg Ile Ala Ala Gln Ala Lys Ala Asp Val Glu Gln Glu
            100                 105                 110

Leu Phe Arg Ala Arg Glu Ser Leu Arg Glu Gln Val Ala Val Leu Ala
        115                 120                 125

Val Lys Gly Ala Glu Ser Ile Leu Arg Ser Glu Val Asp Ala Ser Lys
    130                 135                 140

His Ala Lys Leu Leu Asp Thr Leu Lys Gln Glu Leu
145                 150                 155
```

<210> 188
<211> 201
<212> PRT
<213> Neisseria meningitidis

<400> 188

```
Met Met Thr Leu Tyr Ser Gly Ile Thr Cys Pro Phe Ser His Arg Cys
1               5                   10                  15

Arg Phe Val Leu Tyr Glu Lys Gly Met Asp Phe Glu Ile Lys Asp Val
            20                  25                  30

Asp Ile Tyr Asn Lys Pro Glu Asp Leu Ala Val Met Asn Pro Tyr Asn
        35                  40                  45

Gln Val Pro Val Leu Val Glu Arg Asp Leu Val Leu His Glu Ser Asn
    50                  55                  60

Ile Ile Asn Glu Tyr Ile Asp Glu Arg Phe Pro His Pro Gln Leu Met
65                  70                  75                  80

Pro Gly Asp Pro Val Met Arg Gly Arg Gly Arg Leu Val Leu Tyr Arg
                85                  90                  95

Met Glu Lys Glu Leu Phe Asn His Val Gln Val Leu Glu Asn Pro Ala
            100                 105                 110

Ala Thr Asn Lys Glu Gln Ala Lys Ala Arg Glu Ala Ile Gly Asn Gly
        115                 120                 125

Leu Thr Met Leu Ala Pro Ser Phe Ser Lys Ser Lys Tyr Ile Leu Gly
    130                 135                 140

Glu Asp Phe Ser Met Ile Asp Val Ala Leu Ala Pro Leu Leu Trp Arg
145                 150                 155                 160
```

374

Leu Asp His Tyr Asp Val Lys Leu Gly Lys Ser Ala Ala Pro Leu Leu
165 170 175

Lys Tyr Ala Glu Arg Ile Phe Gln Arg Glu Ala Phe Ile Glu Ala Leu
180 185 190

Thr Pro Ala Glu Lys Ala Met Arg Lys
195 200

<210> 189
<211> 266
<212> PRT
<213> Neisseria meningitidis

<400> 189

Met Ser Pro Ser Pro Phe Ile Glu Met Lys Asp Val Ala Phe Ala Tyr
1 5 10 15

Gly Asp Arg Pro Ile Leu Lys Asn Ile Asn Phe Ser Ile Pro Gln Gly
20 25 30

Asn Phe Ala Ala Val Met Gly Gly Ser Gly Ser Gly Lys Thr Thr Leu
35 40 45

Met Arg Leu Ile Thr Gly Gln Ile Arg Pro Gln Ser Gly Gln Val Leu
50 55 60

Ile Glu Gly Arg Asp Leu Ala Gly Phe Ser Ala Asp Glu Leu Tyr Glu
65 70 75 80

His Arg Arg Arg Met Gly Val Leu Phe Gln His Gly Ala Leu Phe Thr
85 90 95

Asp Leu Ser Val Phe Asp Asn Ile Ala Phe Pro Met Arg Glu Leu Thr
100 105 110

Arg Leu Pro Glu Ala Val Ile Arg Asp Leu Val Leu Leu Lys Leu Asn
115 120 125

Ala Val Gly Leu Arg Gly Val Glu Asn Leu Met Pro Ser Glu Leu Ser
130 135 140

Gly Gly Met Ser Arg Arg Val Ala Leu Ala Arg Thr Ile Ala Leu Asp
145 150 155 160

Pro Glu Ile Met Leu Tyr Asp Glu Pro Phe Thr Gly Leu Asp Pro Ile
165 170 175

Ser Leu Gly Val Ile Ala His Leu Ile Ser Arg Val Asn Lys Ala Leu
180 185 190

Arg Ser Thr Ser Ile Met Val Thr His Asp Ile Glu Lys Ser Leu Glu
195 200 205

Ile Val Asp Gln Val Ile Phe Leu Ala His Gly Glu Ile Met Phe Ser
210 215 220

Gly Ser Pro Gln Glu Met Arg Glu Leu Asp Ser Pro Trp Val Arg Gln
225 230 235 240

Phe Val Gly Gly Leu Ala Asp Gly Pro Val Ala Tyr Arg Tyr Pro Ala
245 250 255

Gln Thr Ser Leu Gln Gln Asp Leu Leu Gly
260 265

<210> 190
<211> 480
<212> PRT
<213> Neisseria meningitidis

<400> 190

```
Met Lys Gln Leu Ala Met Tyr Ile Asn Gly Arg Phe Glu Asn Asp Phe
1               5                   10                  15

Asn Gly Glu Trp Arg Asp Val Leu Asn Pro Ser Thr Glu Glu Ala Ile
            20                  25                  30

Ala Arg Glu Pro Lys Gly Gly Lys Ala Asp Val Asp Arg Ala Val Ala
        35                  40                  45

Ala Ala Arg Ala Ala Gln Pro Ala Trp Glu Arg Leu Pro Ala Val Glu
    50                  55                  60

Arg Gly Ala Tyr Leu Arg Lys Ile Ala Gln Gly Ile Arg Glu Arg Ala
65                  70                  75                  80

Asp Glu Leu Thr Asp Thr Ile Val Ala Glu Gly Gly Lys Thr Lys Asp
                85                  90                  95

Leu Ala Arg Val Glu Val Met Phe Thr Ala Asp Tyr Leu Asp Tyr Gln
            100                 105                 110

Ala Glu Trp Ala Arg Arg Tyr Glu Gly Glu Ile Ile Gln Ser Asp Arg
        115                 120                 125

Pro Arg Glu Asn Ile Leu Leu Phe Lys Arg Pro Leu Gly Val Ile Ala
    130                 135                 140

Gly Ile Leu Pro Trp Asn Phe Pro Phe Phe Leu Ile Ala Arg Lys Met
145                 150                 155                 160

Gly Pro Ala Leu Val Thr Gly Asn Thr Ile Val Val Lys Pro Ser Ser
                165                 170                 175

Val Thr Pro Ile Asn Cys His Ile Phe Ala Glu Ile Val Asp Ala Val
            180                 185                 190

Gly Leu Pro Ala Gly Val Phe Asn Val Val Asn Gly Pro Gly Ala Glu
        195                 200                 205

Ile Gly Asn Ala Leu Ser Ala His Pro Gln Val Asp Met Val Ser Leu
    210                 215                 220

Thr Gly Ser Val Glu Ala Gly Arg Gln Val Met Glu Ala Ala Ser Ala
225                 230                 235                 240

Asn Ile Thr Lys Val Ser Leu Glu Leu Gly Gly Lys Ala Pro Ala Ile
            245                 250                 255

Val Leu Lys Asp Ala Asp Leu Asp Leu Ala Val Lys Ser Ile Leu Ala
        260                 265                 270

Ser Arg Val Gly Asn Thr Gly Gln Ile Cys Asn Cys Ala Glu Arg Val
        275                 280                 285

Tyr Val His Ser Ser Leu Lys Asp Ala Phe Ile Glu Lys Met Thr Ala
    290                 295                 300

Ala Met Lys Gly Val Arg Tyr Gly Asn Pro Ala Glu Ala Glu Ala Gly
305                 310                 315                 320

Ala Leu Glu Met Gly Pro Leu Ile Glu Glu Arg Ala Val Lys Ala Val
            325                 330                 335

Ala Glu Lys Val Glu Arg Ala Val Lys Gln Gly Ala Lys Leu Val Cys
        340                 345                 350

Gly Gly Lys Arg Ala Glu Gly Arg Gly Tyr Phe Phe Glu Pro Thr Leu
        355                 360                 365
```

```
Leu Thr Asp Thr Asp Asn Ser Met Asp Ile Met Lys Glu Glu Thr Phe
    370                 375                 380

Gly Pro Val Leu Pro Val Ser Ala Phe Asp Thr Leu Asp Gln Val Ile
385                 390                 395                 400

Ala Leu Ala Asn Asp Cys Glu Phe Gly Leu Thr Ser Ser Val Tyr Thr
                405                 410                 415

Thr Asn Leu Asn Glu Ala Phe Tyr Val Thr Arg Arg Leu Gln Phe Gly
                420                 425                 430

Glu Thr Tyr Ile Asn Arg Glu Asn Phe Glu Ala Met Gln Gly Phe His
        435                 440                 445

Ala Gly Trp Lys Lys Ser Gly Ile Gly Gly Ala Asp Gly Lys His Gly
    450                 455                 460

Leu Glu Glu Tyr Leu Gln Thr Gln Val Val Tyr Leu Glu Thr Asp Ile
465                 470                 475                 480
```

<210> 191
<211> 938
<212> PRT
<213> Neisseria meningitidis

<400> 191

```
Met Ser Asn Arg Pro Thr Leu Leu Leu Val Asp Gly Ser Ser Tyr Leu
1               5                   10                  15

Tyr Arg Ala Tyr His Ala Met Gly Gln Asn Leu Thr Ala Pro Asp Gly
            20              25              30

Ala Pro Thr Gly Ala Leu Tyr Gly Val Leu Asn Met Leu Arg Arg Leu
        35              40                  45

Arg Ser Glu Tyr Pro His Asp Tyr Cys Ala Val Val Phe Asp Ala Lys
    50              55                  60

Gly Lys Asn Phe Arg His Gln Met Phe Glu Glu Tyr Lys Ala Thr Arg
65              70              75                      80

Pro Pro Met Pro Asp Asp Leu Arg Pro Gln Ala Glu Ala Leu Pro Asp
            85              90                  95

Leu Val Arg Leu Thr Gly Trp Pro Val Leu Val Ile Gly Gln Val Glu
        100             105             110

Ala Asp Asp Val Ile Gly Thr Leu Ala Lys Gln Gly Ala Glu His Gly
    115             120             125

Leu Arg Val Ile Val Ser Thr Gly Asp Lys Asp Met Ala Gln Leu Val
    130             135             140

Asp Glu Arg Val Thr Leu Val Asn Thr Met Ser Ser Glu Thr Leu Asp
145             150             155             160

Ile Glu Gly Val Lys Ala Lys Phe Gly Val Arg Pro Asp Gln Ile Arg
            165             170             175

Asp Tyr Leu Ala Leu Met Gly Asp Lys Val Asp Asn Val Pro Gly Val
        180             185             190

Glu Lys Cys Gly Pro Lys Thr Ala Val Lys Trp Leu Glu Ala Tyr Gly
        195             200             205

Ser Leu Ala Gly Val Met Glu His Ala Ser Glu Ile Lys Gly Lys Val
    210             215             220
```

```
Gly Glu Asn Leu Gln Ala Ala Leu Pro Gln Leu Pro Leu Ser Tyr Asp
225             230             235             240

Leu Val Thr Ile Lys Thr Asp Val Asp Leu His Ala Glu Leu Ser Asp
            245             250             255

Gly Ile Glu Ser Leu Arg Arg Thr Thr Pro Lys Trp Ala Gln Leu Val
        260             265             270

Val Asp Phe Lys Arg Trp Gly Phe Arg Thr Trp Leu Lys Glu Ala Glu
        275             280             285

Ser Asn Met Asn Thr Gly Ser Thr Asp Asp Leu Phe Gly Ser Asp Ser
    290             295             300

Ile Gly Glu Gln Ala Ala Leu Asn Ala Glu Met Pro Phe Glu Lys Gln
305             310             315             320

Ala Glu Lys Ala Thr Ala Pro Glu Lys Leu Asp Tyr Gln Ala Val Thr
            325             330             335

Thr Glu Ala Gln Phe Ala Ala Leu Leu Asp Lys Leu Ser Arg Ala Asp
        340             345             350

Thr Ile Gly Ile Asp Thr Glu Thr Thr Ser Leu Asp Ala Met Asn Ala
        355             360             365

Ser Leu Val Gly Ile Ser Ile Ala Phe Gln Ala Gly Glu Ala Val Tyr
    370             375             380

Ile Pro Val Gly His Ser Leu Thr Ala Ala Pro Glu Gln Leu Asp Leu
385             390             395             400

Gln Asp Val Leu Gly Arg Leu Lys Pro His Leu Gly Asn Pro Ala Leu
        405             410             415

Lys Lys Ile Gly Gln Asn Leu Lys Tyr Asp Gln His Val Phe Ala Asn
        420             425             430

Tyr Gly Ile Ala Leu Asn Gly Ile Ala Gly Asp Ala Met Leu Ala Ser
        435             440             445

Tyr Ile Ile Glu Ser His Leu Gly His Gly Leu Asp Glu Leu Ser Glu
    450             455             460

Arg Trp Leu Gly Leu Glu Thr Ile Thr Tyr Glu Ser Leu Cys Gly Lys
465             470             475             480

Gly Ala Lys Gln Ile Gly Phe Ala Asp Val Ala Ile Gly Gln Ala Thr
            485             490             495

Glu Tyr Ala Ala Gln Asp Ala Asp Phe Ala Leu Arg Leu Glu Ala His
        500             505             510

Leu Arg Ala Gln Met Asp Glu Lys Gln Leu Glu Met Tyr Glu Lys Met
        515             520             525

Glu Leu Pro Val Ala Gln Val Leu Phe Glu Met Glu Arg Asn Gly Val
    530             535             540

Gln Ile Asp Arg Ala Glu Leu Ala Arg Gln Ser Ala Glu Leu Gly Ala
545             550             555             560

Glu Leu Met Lys Leu Glu Gln Glu Ala Tyr Ala Ala Ala Gly Gln Pro
            565             570             575

Phe Asn Leu Asn Ser Pro Lys Gln Leu Gln Glu Ile Leu Phe Asp Lys
            580             585             590
```

```
Met Gly Ile Pro Thr Lys Gly Leu Lys Lys Thr Ala Lys Gly Gly Ile
        595             600             605

Ser Thr Asn Glu Ala Val Leu Glu Gln Leu Ala Pro Asp Tyr Pro Leu
    610             615             620

Pro Lys Ile Ile Leu Gln Asn Arg Ser Leu Ala Lys Leu Lys Ser Thr
625             630             635             640

Tyr Thr Asp Lys Leu Pro Glu Met Ile Ser Pro Lys Asp Gly Arg Val
            645             650             655

His Thr Thr Tyr Ala Gln Ala Val Ala Ile Thr Gly Arg Leu Ala Ser
            660             665             670

Asn Asn Pro Asn Leu Gln Asn Ile Pro Ile Arg Thr Glu Glu Gly Arg
        675             680             685

Lys Val Arg Arg Ala Phe Thr Ala Pro Gln Gly Ser Val Ile Val Ser
    690             695             700

Ala Asp Tyr Ser Gln Ile Glu Leu Arg Ile Met Ala His Leu Ser Gly
705             710             715             720

Asp Lys Thr Leu Ile Ala Ala Phe Gln Asn Gly Glu Asp Val His Arg
            725             730             735

Arg Thr Ala Ala Glu Val Phe Gly Thr Ala Pro Glu Asn Val Ser Ser
            740             745             750

Glu Gln Arg Arg Tyr Ala Lys Ser Ile Asn Phe Gly Leu Ile Tyr Gly
        755             760             765

Met Gly Gln Tyr Gly Leu Ala Lys Ser Leu Gly Ile Asp Asn Leu Ser
    770             775             780

Ala Lys Asn Phe Ile Asp Arg Tyr Phe Ala Arg Tyr Pro Gly Val Ala
785             790             795             800

Glu Tyr Met Gln Arg Thr Lys Glu Gln Ala Ala Ala Gln Gly Tyr Val
            805             810             815

Glu Thr Leu Phe Gly Arg Arg Leu Tyr Leu Pro Asp Ile Arg Asn Lys
        820             825             830

Asn Ala Asn Ala Arg Ala Gly Ala Glu Arg Ala Ala Ile Asn Ala Pro
    835             840             845

Met Gln Gly Thr Ala Ser Asp Leu Ile Lys Arg Ala Met Ile Asp Val
    850             855             860

Ser Arg Trp Leu Ser Glu Cys Glu Ala Ser Pro Trp Asp Glu Leu Leu
865             870             875             880

Gln Ser Lys Leu Ile Met Gln Val His Asp Glu Leu Val Leu Glu Val
            885             890             895

Val Glu Thr Glu Leu Asp Phe Val Lys Glu Lys Leu Pro Gln Ile Met
            900             905             910

Ala Lys Val Asp Gly Gly Leu Leu Asp Val Pro Leu Val Ala Glu Val
            915             920             925

Gly Val Gly Glu Asn Trp Glu Glu Ala His
    930             935
```

<210> 192
<211> 1320

<212> PRT
<213> Neisseria meningitidis

<400> 192

```
Met Ser Val Val Leu Pro Leu Arg Gly Val Thr Ala Leu Ser Asp Phe
1               5               10              15

Arg Val Glu Lys Leu Leu Gln Lys Ala Ala Ala Leu Gly Leu Pro Glu
            20              25              30

Val Lys Leu Ser Ser Glu Phe Trp Tyr Phe Val Gly Ser Glu Lys Ala
        35              40              45

Leu Asp Ala Ala Thr Val Glu Lys Leu Gln Ala Leu Leu Ala Ala Gln
    50              55              60

Ser Val Glu Gln Thr Pro Lys Ala Arg Glu Gly Leu His Leu Phe Leu
65              70              75              80

Val Thr Pro Arg Leu Gly Thr Ile Ser Pro Trp Ala Ser Lys Ala Thr
            85              90              95

Asn Ile Ala Glu Asn Cys Gly Leu Ala Gly Ile Glu Arg Ile Glu Arg
            100             105             110

Gly Met Ala Val Trp Leu Glu Gly Arg Leu Asn Asp Glu Gln Lys Gln
    115             120             125

Gln Trp Ala Ala Leu Leu His Asp Arg Met Thr Glu Ser Val Leu Pro
    130             135             140

Asp Phe Gln Thr Ala Ser Lys Leu Phe His His Leu Glu Ser Glu Thr
145             150             155             160

Phe Ser Gly Val Asp Val Leu Gly Gly Gly Lys Glu Ala Leu Val Lys
            165             170             175

Ala Asn Thr Glu Met Gly Leu Ala Leu Ser Ala Asp Glu Ile Asp Tyr
            180             185             190

Leu Val Glu Asn Tyr Gln Ala Leu Gln Arg Asn Pro Ser Asp Val Glu
    195             200             205

Leu Met Met Phe Ala Gln Ala Asn Ser Glu His Cys Arg His Lys Ile
    210             215             220

Phe Asn Ala Asp Phe Ile Leu Asn Gly Glu Lys Gln Pro Lys Ser Leu
225             230             235             240

Phe Gly Met Ile Arg Asp Thr His Asn Ala His Pro Glu Gly Thr Val
            245             250             255

Val Ala Tyr Lys Asp Asn Ser Ser Val Ile Glu Gly Ala Lys Ile Glu
        260             265             270

Arg Phe Tyr Pro Asn Ala Ala Glu Asn Gln Gly Tyr Arg Phe His Glu
    275             280             285

Glu Asp Thr His Ile Ile Met Lys Val Glu Thr His Asn His Pro Thr
290             295             300

Ala Ile Ala Pro Phe Ala Gly Ala Ala Thr Gly Ala Gly Gly Glu Ile
305             310             315             320

Arg Asp Glu Gly Ala Thr Gly Lys Gly Ser Arg Pro Lys Ala Gly Leu
            325             330             335

Thr Gly Phe Thr Val Ser Asn Leu Asn Ile Pro Asp Leu Lys Gln Pro
            340             345             350

Trp Glu Gln Asp Tyr Gly Lys Pro Glu His Ile Ser Ser Pro Leu Asp
    355             360             365
```

```
Ile Met Ile Glu Gly Pro Ile Gly Gly Ala Ala Phe Asn Asn Glu Phe
    370             375             380

Gly Arg Pro Asn Leu Leu Gly Tyr Phe Arg Thr Phe Glu Glu Lys Phe
385             390             395             400

Asp Gly Gln Val Arg Gly Tyr His Lys Pro Ile Met Ile Ala Gly Gly
                405             410             415

Leu Gly Ser Ile Gln Ala Gln Gln Thr His Lys Asp Glu Ile Pro Glu
            420             425             430

Gly Ala Leu Leu Ile Gln Leu Gly Gly Pro Gly Met Leu Ile Gly Leu
        435             440             445

Gly Gly Gly Ala Ala Ser Ser Met Asp Thr Gly Thr Asn Asp Ala Ser
    450             455             460

Leu Asp Phe Asn Ser Val Gln Arg Gly Asn Pro Glu Ile Glu Arg Arg
465             470             475             480

Ala Gln Glu Val Ile Asp Arg Cys Trp Gln Leu Gly Gly Lys Asn Pro
                485             490             495

Ile Ile Ser Ile His Asp Val Gly Ala Gly Gly Leu Ser Asn Ala Phe
            500             505             510

Pro Glu Leu Val Asn Asp Ala Arg Arg Gly Ala Val Phe Lys Leu Arg
        515             520             525

Glu Val Pro Leu Glu Glu His Gly Leu Asn Pro Leu Gln Ile Trp Cys
    530             535             540

Asn Glu Ser Gln Glu Arg Tyr Val Leu Ser Ile Leu Glu Lys Asp Leu
545             550             555             560

Asp Ala Phe Arg Ala Ile Cys Glu Arg Glu Arg Cys Pro Phe Ala Val
                565             570             575

Val Gly Thr Ala Thr Asp Asp Gly His Leu Lys Val Arg Asp Asp Leu
            580             585             590

Phe Ala Asn Asn Pro Val Asp Leu Pro Leu Asn Val Leu Leu Gly Lys
        595             600             605

Leu Pro Lys Thr Thr Arg Thr Asp Lys Thr Val Ala Pro Ser Lys Lys
    610             615             620

Pro Phe His Ala Gly Asp Ile Asp Ile Thr Glu Ala Ala Tyr Arg Val
625             630             635             640

Leu Arg Leu Pro Ala Val Ala Ala Lys Asn Phe Leu Ile Thr Ile Gly
                645             650             655

Asp Arg Ser Val Gly Gly Leu Thr His Arg Asp Gln Met Val Gly Lys
            660             665             670

Tyr Gln Thr Pro Val Ala Asp Cys Ala Val Thr Met Met Gly Phe Asn
        675             680             685

Thr Tyr Arg Gly Glu Ala Met Ser Met Gly Glu Lys Pro Thr Val Ala
    690             695             700

Leu Phe Asp Ala Pro Ala Ser Gly Arg Met Cys Val Gly Glu Ala Ile
705             710             715             720

Thr Asn Ile Ala Ala Val Asn Ile Gly Asp Ile Gly Asn Ile Lys Leu
            725             730             735
```

Ser Ala Asn Trp Met Ala Ala Cys Gly Asn Glu Gly Glu Asp Glu Lys
740 745 750

Leu Tyr Arg Thr Val Glu Ala Val Ser Lys Ala Cys Gln Ala Leu Asp
755 760 765

Leu Ser Ile Pro Val Gly Lys Asp Ser Leu Ser Met Lys Thr Val Trp
770 775 780

Gln Asp Gly Glu Glu Lys Lys Ser Val Val Ser Pro Leu Ser Leu Ile
785 790 795 800

Ile Ser Ala Phe Ala Pro Val Lys Asp Val Arg Lys Thr Val Thr Pro
805 810 815

Glu Leu Lys Asn Val Glu Asp Ser Val Leu Leu Phe Val Asp Leu Gly
820 825 830

Phe Gly Lys Ala Arg Met Gly Gly Ser Ala Phe Gly Gln Val Tyr Asn
835 840 845

Asn Met Ser Gly Asp Ala Pro Asp Leu Asp Asp Thr Gly Arg Leu Lys
850 855 860

Ala Phe Tyr Ser Val Ile Gln Gln Leu Val Ala Glu Asn Lys Leu Leu
865 870 875 880

Ala Tyr His Asp Arg Ser Asp Gly Gly Leu Phe Ala Val Leu Val Glu
885 890 895

Met Ala Phe Ala Gly Arg Cys Gly Leu Asp Ile Asp Leu Asn Leu Leu
900 905 910

Leu Ala Gln Thr Phe Ile Thr Asn His Thr Ala Leu Ser Gln Ser Leu
915 920 925

Arg Thr Glu Glu Val Lys Ala Leu Ala Glu Trp Gln Glu Thr Ile Ala
930 935 940

Arg Thr Leu Phe Asn Glu Glu Leu Gly Ala Val Ile Gln Val Arg Lys
945 950 955 960

Gln Asp Val Ala Asp Ile Ile Asn Leu Phe Tyr Gln Gln Gln Leu His
965 970 975

His Asn Val Phe Glu Ile Gly Thr Leu Thr Asp Glu Asn Thr Leu Ile
980 985 990

Ile Arg Asp Gly Gln Thr His Leu Ile Ser Asp Asn Leu Ile Lys Leu
995 1000 1005

Gln Gln Thr Trp Gln Glu Thr Ser His Gln Ile Gln Arg Leu Arg Asp
1010 1015 1020

Asn Pro Ala Cys Ala Asp Ser Glu Phe Ala Leu Ile Gly Asp Asn Glu
1025 1030 1035 1040

Arg Ser Ala Leu Phe Ala Asp Val Lys Phe Asp Val Asn Glu Asp Ile
1045 1050 1055

Ala Ala Pro Phe Ile Asn Ser Gly Ala Lys Pro Lys Ile Ala Ile Leu
1060 1065 1070

Arg Glu Gln Gly Val Asn Gly Gln Ile Glu Met Ala Ala Ala Phe Thr
1075 1080 1085

Arg Ala Gly Phe Asp Ala Tyr Asp Val His Met Ser Asp Leu Met Ala
1090 1095 1100

Gly Arg Ile His Leu Ala Asp Phe Lys Met Leu Ala Ala Cys Gly Gly

```
        1105                    1110                    1115                    1120
    Phe Ser Tyr Gly Asp Val Leu Gly Ala Gly Glu Gly Trp Ala Lys Ser
                    1125                    1130                    1135

    Ile Leu Phe His Pro Ala Leu Arg Asp Gln Phe Ala Ala Phe Phe Ala
                1140                    1145                    1150

    Asp Pro Asp Thr Leu Thr Leu Gly Val Cys Asn Gly Cys Gln Met Val
                1155                    1160                    1165

    Ser Asn Leu Ala Glu Ile Ile Pro Gly Thr Ala Gly Trp Pro Lys Phe
        1170                    1175                    1180

    Lys Arg Asn Leu Ser Glu Gln Phe Glu Ala Arg Leu Ser Met Val His
    1185                    1190                    1195                    1200

    Val Pro Lys Ser Ala Ser Leu Ile Leu Asn Glu Met Gln Gly Ser Ser
                    1205                    1210                    1215

    Leu Pro Val Val Val Ser His Gly Glu Gly Arg Ala Asp Phe Ala Leu
                1220                    1225                    1230

    His Gly Gly Asn Ile Ser Ala Asp Leu Gly Ile Ala Leu Gln Tyr Ile
                1235                    1240                    1245

    Asp Gly Gln Asn Gln Val Thr Gln Thr Tyr Pro Leu Asn Pro Asn Gly
        1250                    1255                    1260

    Ser Pro Gln Gly Ile Ala Gly Val Thr Asn Ala Asp Gly Arg Ile Thr
    1265                    1270                    1275                    1280

    Ile Met Met Pro His Pro Glu Arg Val Tyr Arg Ala Ala Gln Met Ser
                    1285                    1290                    1295

    Trp Lys Pro Glu Gly Trp Thr Glu Leu Ser Gly Trp Tyr Arg Leu Phe
                1300                    1305                    1310

    Ala Gly Ala Arg Lys Ala Leu Gly
            1315                1320
```

<210> 193
<211> 302
<212> PRT
<213> Neisseria meningitidis

<400> 193

```
Met Asn Gln Thr Ala Ile Asn Arg Ala Asp Val Arg Thr Arg Phe Ile
1               5                   10                  15

Phe Asp Asp Met Pro Val Arg Gly Leu His Val Arg Leu Glu Asn Val
            20                  25                  30

Trp Gln His Ile Val Lys Gln Lys Asn Tyr Pro Ala Ala Ile Arg Arg
        35                  40                  45

Ala Leu Gly Glu Leu Leu Ala Ala Gly Val Leu Leu Ser Gly Asn Leu
        50                  55                  60

Lys Asn Glu Gly Thr Leu Ile Val Gln Val Gln Gly Arg Gly Arg Leu
65                  70                  75                  80

Lys Met Leu Val Ala Glu Ala Ala Ser Asp Arg Thr Val Arg Ala Thr
                85                  90                  95

Ala Arg Trp Asp Glu Thr Ala Glu Ile Ala Asp Asp Glu Ser Leu Gly
            100                 105                 110

Asp Leu Leu Gly Glu Gly Gly Val Phe Val Leu Thr Leu Gln Pro Lys
        115                 120                 125

Asp Gly Glu Pro Trp Gln Gly Val Val Pro Leu Glu Gly Gly Gly Ile
    130                 135                 140

Ala Gln Met Leu Val Asn Tyr Met Lys Arg Ser Glu Gln Leu Asp Thr
145                 150                 155                 160

His Ile Val Leu Ser Ala Ser Asp Glu Ala Ala Gly Gly Leu Leu Val
            165                 170                 175

Gln Arg Leu Pro Glu Glu Val Leu Asp Glu Glu Ala Trp Glu His Val
        180                 185                 190

Ser Thr Leu Ala Arg Thr Leu Thr Ala Glu Glu Leu Ala Gly Leu Asp
        195                 200                 205

Ala Gln His Val Leu Tyr Arg Leu Phe His Glu Thr Pro Pro Arg Val
    210                 215                 220

Phe Glu Pro Glu Thr Phe Glu Phe Ser Cys Thr Cys Ser Arg Gly Lys
225                 230                 235                 240

Val Ser Asp Met Leu Leu Met Leu Gly Gly Glu Glu Val Gly Gly Val
            245                 250                 255

Val Val Glu Gln Gly Ser Ile Glu Val Asp Cys Asp Phe Cys His Ser
            260                 265                 270

Lys Tyr Val Phe Asp Glu Thr Asp Val Asn Ala Leu Phe Gly Glu Asp
        275                 280                 285

Val Val Gly Val Ala Lys Gly Leu Pro Arg His Thr Val Gln
        290                 295                 300
```

<210> 194  
<211> 331  
<212> PRT  
<213> Neisseria meningitidis

<400> 194

```
Met Lys Lys Ser Leu Ile Ala Leu Thr Leu Ala Ala Leu Pro Val Ala
1               5               10              15

Ala Met Ala Asp Val Thr Leu Tyr Gly Thr Ile Lys Ala Gly Val Glu
            20              25              30

Thr Ser Arg Ser Val Phe His Gln Asn Gly Gln Val Thr Glu Val Thr
        35              40                      45

Thr Ala Thr Gly Ile Val Asp Leu Gly Ser Lys Ile Gly Phe Lys Gly
    50                  55                  60

Gln Glu Asp Leu Gly Asn Gly Leu Lys Ala Ile Trp Gln Val Glu Gln
65              70                  75                  80

Lys Ala Ser Ile Ala Gly Thr Asp Ser Gly Trp Gly Asn Arg Gln Ser
            85                  90                  95

Phe Ile Gly Leu Lys Gly Gly Phe Gly Lys Leu Arg Val Gly Arg Leu
            100             105             110

Asn Ser Val Leu Lys Asp Thr Gly Asp Ile Asn Pro Trp Asp Ser Lys
        115             120             125

Ser Asp Tyr Leu Gly Val Asn Lys Ile Ala Glu Pro Glu Ala Arg Leu
    130             135             140

Ile Ser Val Arg Tyr Asp Ser Pro Glu Phe Ala Gly Leu Ser Gly Ser
145             150             155             160

Val Gln Tyr Ala Leu Asn Asp Asn Ala Gly Arg His Asn Ser Glu Ser
            165             170             175

Tyr His Ala Gly Phe Asn Tyr Lys Asn Gly Gly Phe Phe Val Gln Tyr
            180             185             190

Gly Gly Ala Tyr Lys Arg His His Gln Val Gln Glu Gly Leu Asn Ile
        195             200             205

Glu Lys Tyr Gln Ile His Arg Leu Val Ser Gly Tyr Asp Asn Asp Ala
    210             215             220

Leu Tyr Ala Ser Val Ala Val Gln Gln Gln Asp Ala Lys Leu Thr Asp
225             230             235             240

Ala Ser Asn Ser His Asn Ser Gln Thr Glu Val Ala Ala Thr Leu Ala
            245             250             255

Tyr Arg Phe Gly Asn Val Thr Pro Arg Val Ser Tyr Ala His Gly Phe
        260             265             270

Lys Gly Leu Val Asp Asp Ala Asp Ile Gly Asn Glu Tyr Asp Gln Val
        275             280             285

Val Val Gly Ala Glu Tyr Asp Phe Ser Lys Arg Thr Ser Ala Leu Val
    290             295             300

Ser Ala Gly Trp Leu Gln Glu Gly Lys Gly Glu Asn Lys Phe Val Ala
305             310             315             320

Thr Ala Gly Gly Val Gly Leu Arg His Lys Phe
            325             330
```

<210> 195
<211> 143
<212> PRT

388

<213> Neisseria meningitidis

<400> 195

```
Met Lys Thr Phe Ser Ala Lys Pro His Glu Val Lys Arg Glu Trp Phe
1               5                   10                  15

Val Ile Asp Ala Gln Asp Lys Val Leu Gly Arg Val Ala Ala Glu Val
            20                  25                  30

Ala Ser Arg Leu Arg Gly Lys His Lys Pro Glu Tyr Thr Pro His Val
            35                  40                  45

Asp Thr Gly Asp Tyr Ile Ile Val Ile Asn Ala Asp Lys Leu Arg Val
        50              55                  60

Thr Gly Ala Lys Phe Glu Asp Lys Lys Tyr Phe Arg His Ser Gly Phe
65                  70                  75                  80

Pro Gly Gly Ile Tyr Glu Arg Thr Phe Arg Glu Met Gln Glu Gln Phe
                85                  90                  95

Pro Gly Arg Ala Leu Glu Gln Ala Val Lys Gly Met Leu Pro Lys Gly
            100                 105                 110

Pro Leu Gly Tyr Ala Met Ile Lys Lys Leu Lys Val Tyr Ala Gly Ala
            115                 120                 125

Glu His Ala His Ala Ala Gln Gln Pro Lys Val Leu Glu Leu Lys
        130                 135                 140
```

<210> 196
<211> 371
<212> PRT
<213> Neisseria meningitidis

<400> 196

```
Met Lys Val Gly Phe Val Gly Trp Arg Gly Met Val Gly Ser Val Leu
1             5                 10                15

Met Gln Arg Met Lys Glu Glu Asn Asp Phe Ala His Ile Pro Glu Ala
            20              25              30

Phe Phe Phe Thr Thr Ser Asn Val Gly Gly Ala Ala Pro Asp Phe Gly
        35              40              45

Gln Ala Ala Lys Thr Leu Leu Asp Ala Asn Asn Val Ala Glu Leu Ala
        50          55              60

Lys Met Asp Ile Ile Val Thr Cys Gln Gly Gly Asp Tyr Thr Lys Ser
65              70              75              80

Val Phe Gln Ala Leu Arg Asp Ser Gly Trp Asn Gly Tyr Trp Ile Asp
            85              90              95

Ala Ala Ser Ser Leu Arg Met Lys Asp Asp Ala Ile Ile Val Leu Asp
        100             105             110

Pro Val Asn Arg Asp Val Leu Asp Asn Gly Leu Lys Asn Gly Val Lys
        115             120             125

Asn Tyr Ile Gly Gly Asn Cys Thr Val Ser Leu Met Leu Met Ala Leu
    130             135             140

Gly Gly Leu Phe Gln Asn Asp Leu Val Glu Trp Ala Thr Ser Met Thr
145             150             155             160

Tyr Gln Ala Ala Ser Gly Ala Gly Ala Lys Asn Met Arg Glu Leu Ile
            165             170             175

Ser Gly Met Gly Ala Val His Ala Gln Val Ala Asp Ala Leu Ala Asp
        180             185             190

Pro Ala Gly Ser Ile Leu Asp Ile Asp Arg Lys Val Ser Asp Phe Leu
    195             200             205

Arg Ser Glu Asp Tyr Pro Lys Ala Asn Phe Gly Val Pro Leu Ala Gly
    210             215             220

Ser Leu Ile Pro Trp Ile Asp Val Asp Leu Gly Asn Gly Gln Ser Lys
225             230             235             240

Glu Glu Trp Lys Gly Gly Val Glu Thr Asn Lys Ile Leu Gly Arg Ser
            245             250             255

Asp Asn Pro Thr Val Ile Asp Gly Leu Cys Val Arg Val Gly Ala Met
        260             265             270

Arg Cys His Ser Gln Ala Ile Thr Leu Lys Leu Lys Lys Asp Leu Pro
        275             280             285

Val Ser Glu Ile Glu Thr Ile Leu Ala Gly Ala Asn Asp Trp Val Lys
    290             295             300

Val Ile Pro Asn Glu Lys Glu Ala Ser Ile His Glu Leu Thr Pro Ala
305             310             315             320

Lys Val Thr Gly Thr Leu Ser Val Pro Val Gly Arg Ile Arg Lys Leu
            325             330             335

Gly Met Gly Gly Glu Tyr Ile Ser Ala Phe Thr Val Gly Asp Gln Leu
        340             345             350

Leu Trp Gly Ala Ala Glu Pro Leu Arg Arg Val Leu Arg Ile Val Leu
        355             360             365
```

390

```
Gly Ser Leu
    370
```

<210> 197
<211> 384
<212> PRT
<213> Neisseria meningitidis

<400> 197

```
Gly Ser Leu
```

Met Lys Phe Ile Asp Glu Ala Lys Ile Glu Val Ala Ala Gly Lys Gly
1               5               10                  15

Gly Asn Gly Ala Thr Ser Phe Arg Arg Glu Lys Phe Val Pro Arg Gly
            20              25                  30

Gly Pro Asp Gly Gly Asp Gly Gly Lys Gly Gly Ser Val Trp Ala Glu
        35              40              45

Ala Asp Glu Asn Thr Asn Thr Leu Val Glu Tyr Arg Phe Val Lys Arg
    50              55              60

Tyr Gln Ala Lys Asn Gly Glu Lys Gly His Gly Ser Asp Arg Tyr Gly
65              70              75                  80

Ala Gly Ala Asp Asp Ile Val Leu Lys Met Pro Val Gly Thr Leu Ile
            85              90                  95

Arg Asp Leu Asp Thr Gly Glu Thr Val Ala Asp Leu Thr Tyr His Gly
        100             105             110

Gln Arg Val Cys Leu Ala Lys Gly Gly Lys Gly Gly Leu Gly Asn Ile
        115             120             125

His Phe Lys Ser Ser Val Asn Arg Ala Pro Lys Gln Ser Thr Pro Gly
    130             135             140

Glu Glu Gly Glu Ala Arg Ser Leu Gln Leu Glu Leu Lys Val Leu Ala
145             150             155             160

Asp Val Gly Leu Leu Gly Met Pro Asn Ala Gly Lys Ser Thr Leu Ile
            165             170             175

Thr Ala Val Ser Ala Ala Arg Pro Lys Ile Ala Asn Tyr Pro Phe Thr
            180             185             190

Thr Leu His Pro Asn Leu Gly Val Val Arg Ile Asp Glu Asn His Ser
        195             200             205

Phe Val Met Ala Asp Ile Pro Gly Leu Ile Glu Gly Ala Ala Glu Gly
    210             215             220

Ala Gly Leu Gly His Arg Phe Leu Lys His Leu Ser Arg Thr Gly Leu
225             230             235             240

Leu Leu His Val Val Asp Leu Ala Pro Phe Asp Glu Thr Val Asn Pro
            245             250             255

Ala Glu Glu Ala Leu Ala Ile Ile Asn Glu Leu Arg Lys Tyr Asp Glu
            260             265             270

Glu Leu Tyr Gly Lys Pro Arg Trp Leu Val Leu Asn Lys Leu Asp Met
        275             280             285

Leu Asp Glu Glu Glu Ala Gln Thr Arg Thr Ala Ala Phe Leu Glu Ala
    290             295             300

Val Gly Trp Asp Tyr Pro Lys Pro Asp Asp Arg Phe Gln Phe Asp Met
305             310             315             320

```
Glu Thr Pro Arg Leu Phe Gln Ile Ser Ala Leu Thr His Gln Gly Thr
                325             330             335

Gln Glu Leu Val His Gln Ile Asn Gln Tyr Leu Thr Glu Lys Lys Arg
                340             345             350

Ile Glu Ala Glu Lys Ala Glu Ala Glu Lys Ala Ala Ala Asn Val Glu
        355             360             365

Ile Ile Glu Gln Gln Pro Lys Thr Asp Thr Gly Val Phe Lys Pro Glu
        370             375             380
```

<210> 198
<211> 242
<212> PRT
<213> Neisseria meningitidis

<400> 198

```
Met Ser Gln Ile Thr Met Arg Gln Met Ile Glu Ala Gly Val His Phe
1           5               10              15

Gly His Gln Thr Arg Phe Trp Asn Pro Lys Met Ala Gln Tyr Ile Phe
            20              25              30

Gly Ala Arg Asn Lys Ile His Ile Val Asn Leu Glu Lys Thr Leu Pro
        35              40              45

Met Phe Gln Asp Ala Gln Glu Ala Val Arg Arg Leu Val Ala Asn Lys
    50              55              60

Gly Thr Val Leu Phe Val Gly Thr Lys Arg Gln Ala Arg Asp Ile Ile
65              70              75              80

Arg Glu Glu Ala Thr Arg Ala Gly Met Pro Phe Val Asp Tyr Arg Trp
            85              90              95

Leu Gly Gly Met Leu Thr Asn Tyr Lys Thr Val Lys Gln Ser Ile Lys
            100             105             110

Arg Leu Glu Glu Lys Thr Ala Ala Leu Glu Asn Ala Ala Glu Ser Gly
        115             120             125

Phe Ser Lys Lys Glu Ile Leu Glu Met Gln Arg Asp Val Glu Lys Leu
    130             135             140

Glu Arg Ser Leu Gly Gly Ile Lys Asn Met Lys Gly Leu Pro Asp Ala
145             150             155             160

Ile Phe Val Ile Asp Thr Gly Tyr Gln Lys Gly Thr Leu Val Glu Ala
            165             170             175

Glu Lys Leu Gly Ile Pro Val Ile Ala Val Val Asp Thr Asn Asn Ser
            180             185             190

Pro Asp Gly Val Lys Tyr Val Ile Pro Gly Asn Asp Asp Ser Ala Lys
            195             200             205

Ala Ile Arg Leu Tyr Cys Arg Gly Ile Ala Asp Ala Val Leu Glu Gly
    210             215             220

Lys Asn Gln Ala Leu Gln Glu Thr Val Ala Ala Ala Gln Glu Ala Ala
225             230             235             240

Ala Glu
```

<210> 199
<211> 284
<212> PRT
<213> Neisseria meningitidis

<400> 199

```
Met Ala Glu Ile Thr Ala Lys Met Val Ala Asp Leu Arg Ala Ala Thr
1               5                   10                  15

Gly Leu Gly Met Met Glu Cys Lys Lys Ala Leu Val Glu Ala Glu Gly
            20              25                  30

Asn Phe Asp Lys Ala Glu Glu Ile Leu Arg Ile Lys Ser Gly Ala Lys
            35                  40                  45

Ala Gly Lys Leu Ala Gly Arg Thr Ala Ala Glu Gly Val Leu Ala Tyr
    50              55                  60

Ala Ile Asn Gly Asn Val Gly Ala Leu Val Glu Val Asn Cys Glu Thr
65              70                  75                  80

Asp Phe Val Ala Lys Asp Ala Gly Phe Val Glu Phe Ala Asn Phe Val
            85                  90                  95

Ala Lys Thr Ala Ala Glu Lys Lys Pro Ala Ser Val Glu Glu Leu Ser
            100                 105                 110

Glu Leu Val Glu Ala Glu Arg Lys Ala Ile Ile Ala Lys Leu Gly Glu
            115                 120                 125

Asn Met Ser Val Arg Arg Phe Gln Val Ile Asp Thr Ala Asn Gln Leu
    130                 135                 140

Val Ala Tyr Ile His Gly Ala Leu Ala Thr Glu Gly Val Leu Val Glu
145                 150                 155                 160

Tyr Lys Gly Ser Glu Asp Val Ala Arg Lys Ile Gly Met His Ile Val
            165                 170                 175

Ala Ala Lys Pro Gln Cys Val Ser Glu Ala Glu Val Asp Ala Glu Thr
            180                 185                 190

Val Glu Lys Glu Arg His Ile Tyr Thr Glu Gln Ala Ile Ala Ser Gly
            195                 200                 205

Lys Pro Ala Asp Ile Ala Ala Lys Met Val Glu Gly Arg Ile Arg Lys
    210                 215                 220

Phe Leu Ala Glu Ile Thr Leu Asn Gly Gln Ala Phe Val Met Asn Pro
225                 230                 235                 240

Asp Gln Thr Val Ala Gln Phe Ser Lys Glu Asn Gly Thr Glu Val Ile
            245                 250                 255

Ser Phe Val Arg Tyr Lys Val Gly Asp Gly Ile Glu Lys Lys Ala Val
            260                 265                 270

Asp Tyr Ala Ala Glu Val Ala Ala Ala Ala Lys Val
            275                 280
```

<210> 200
<211> 239
<212> PRT
<213> Neisseria meningitidis

<400> 200

```
Met Thr Gln Gln Ile Lys Tyr Lys Arg Val Leu Leu Lys Leu Ser Gly
1               5                   10                  15

Glu Ser Leu Met Gly Ser Asp Pro Phe Gly Ile Asn His Asp Thr Ile
            20              25              30

Val Gln Thr Val Gly Glu Ile Ala Glu Val Val Lys Met Gly Val Gln
        35                  40                  45

Val Gly Ile Val Val Gly Gly Gly Asn Ile Phe Arg Gly Val Ser Ala
    50                  55                  60

Gln Ala Gly Ser Met Asp Arg Ala Thr Ala Asp Tyr Met Gly Met Met
65              70                  75                  80

Ala Thr Val Met Asn Ala Leu Ala Leu Lys Asp Ala Phe Glu Thr Leu
                85                  90                  95

Gly Ile Lys Ala Arg Val Gln Ser Ala Leu Ser Met Gln Gln Ile Ala
            100                 105                 110

Glu Thr Tyr Ala Arg Pro Lys Ala Ile Gln Tyr Leu Glu Glu Gly Lys
        115                 120                 125

Val Val Ile Phe Ala Ala Gly Thr Gly Asn Pro Phe Phe Thr Thr Asp
    130                 135                 140

Thr Ala Ala Ala Leu Arg Gly Ala Glu Met Asn Cys Asp Val Met Leu
145             150                 155                 160

Lys Ala Thr Asn Val Asp Gly Val Tyr Thr Ala Asp Pro Lys Lys Asp
                165                 170                 175

Pro Ser Ala Thr Arg Tyr Glu Thr Ile Thr Phe Asp Glu Ala Leu Leu
            180                 185                 190

Lys Asn Leu Lys Val Met Asp Ala Thr Ala Phe Ala Leu Cys Arg Glu
            195                 200                 205

Arg Lys Leu Asn Ile Val Val Phe Gly Ile Ala Lys Glu Gly Ser Leu
    210                 215                 220

Lys Arg Val Ile Thr Gly Glu Asp Glu Gly Thr Leu Val His Cys
225                 230                 235
```

<210> 201
<211> 447
<212> PRT
<213> Neisseria meningitidis

<400> 201

```
Met Ser Gln Asn His Thr Ile Leu Gln Ser Leu Pro Val Gly Gln Lys
1               5                   10                  15

Val Gly Ile Ala Phe Ser Gly Gly Leu Asp Thr Ser Ala Ala Leu Leu
            20                  25                  30

Trp Met Lys Leu Lys Gly Ala Leu Pro Tyr Ala Tyr Thr Ala Asn Leu
        35                  40                  45

Gly Gln Pro Asp Glu Asp Asp Tyr Asn Ala Ile Pro Lys Lys Ala Met
    50                  55                  60

Glu Tyr Gly Ala Glu Asn Ala Arg Leu Ile Asp Cys Arg Ala Gln Leu
65                  70                  75                  80

Ala His Glu Gly Ile Ala Ala Ile Gln Cys Gly Ala Phe His Val Ser
            85                  90                  95

Thr Gly Gly Ile Ala Tyr Phe Asn Thr Thr Pro Leu Gly Arg Ala Val
            100                 105                 110

Thr Gly Thr Met Leu Val Ser Ala Met Lys Glu Asp Asp Val Asn Ile
            115                 120                 125

Trp Gly Asp Gly Ser Thr Tyr Lys Gly Asn Asp Ile Glu Arg Phe Tyr
    130                 135                 140
```

```
Arg Tyr Gly Leu Leu Thr Asn Pro Ala Leu Lys Ile Tyr Lys Pro Trp
145                 150                 155                 160

Leu Asp Gln Gln Phe Ile Asp Glu Leu Gly Gly Arg His Glu Met Ser
                165                 170                 175

Glu Phe Leu Ile Ala Asn Gly Phe Asn Tyr Lys Met Ser Val Glu Lys
            180                 185                 190

Ala Tyr Ser Thr Asp Ser Asn Met Leu Gly Ala Thr His Glu Ala Lys
        195                 200                 205

Asp Leu Glu Phe Leu Asn Ser Gly Ile Lys Ile Val Lys Pro Ile Met
    210                 215                 220

Gly Val Ala Phe Trp Asp Glu Asn Val Glu Val Ser Pro Glu Glu Val
225                 230                 235                 240

Ser Val Arg Phe Glu Glu Gly Val Pro Val Ala Leu Asn Gly Lys Glu
                245                 250                 255

Tyr Ala Asp Pro Val Glu Leu Phe Leu Glu Ala Asn Arg Ile Gly Gly
                260                 265                 270

Arg His Gly Leu Gly Met Ser Asp Gln Ile Glu Asn Arg Ile Ile Glu
        275                 280                 285

Ala Lys Ser Arg Gly Ile Tyr Glu Ala Pro Gly Met Ala Leu Phe His
    290                 295                 300

Ile Ala Tyr Glu Arg Leu Val Thr Gly Ile His Asn Glu Asp Thr Ile
305                 310                 315                 320

Glu Gln Tyr Arg Ile Asn Gly Leu Arg Leu Gly Arg Leu Leu Tyr Gln
                325                 330                 335

Gly Arg Trp Phe Asp Ser Gln Ala Leu Met Leu Arg Glu Thr Ala Gln
            340                 345                 350

Arg Trp Val Ala Lys Ala Val Thr Gly Glu Val Thr Leu Glu Leu Arg
        355                 360                 365

Arg Gly Asn Asp Tyr Ser Ile Leu Asn Thr Glu Ser Pro Asn Leu Thr
    370                 375                 380

Tyr Gln Pro Glu Arg Leu Ser Met Glu Lys Val Glu Asp Ala Ala Phe
385                 390                 395.                400

Thr Pro Leu Asp Arg Ile Gly Gln Leu Thr Met Arg Asn Leu Asp Ile
                405                 410                 415

Thr Asp Thr Arg Val Lys Leu Gly Ile Tyr Ser Gln Ser Gly Leu Leu
            420                 425                 430

Ser Leu Gly Glu Gly Ser Val Leu Pro Gln Leu Gly Asn Lys Gln
        435                 440                 445
```

<210> 202
<211> 367
<212> PRT
<213> Neisseria meningitidis

<400> 202

```
Met Glu Ala Glu Val Ile Asn Gln Leu Asn Asn Thr Leu Asn Asp Leu
1               5               10              15

Glu Lys Arg Ser Glu Asp Ile Arg Val Tyr Met Asp Tyr Gln Gly Lys
            20              25              30

Lys Asp Arg Leu Glu Glu Val Ile Gly Leu Ser Glu Asp Pro Glu Leu
        35              40              45

Trp Asn Asp Pro Lys Arg Ala Gln Glu Ile Gly Lys Glu Arg Lys Ile
    50              55              60

Leu Glu Gly Ile Val Leu Thr Leu Asp Asn Ile Ala Ser Gly Ile Glu
65              70              75              80

Asp Asn Arg Met Leu Ile Glu Met Thr Val Glu Glu Asn Asp Glu Glu
            85              90              95

Gly Phe Ala Ala Val Gln Glu Asp Val Ala Gly Leu Glu Lys Gln Met
        100             105             110

Ala Asp Leu Glu Phe Lys Arg Met Phe Asn Gln Pro Ala Asp Pro Asn
        115             120             125

Asn Cys Phe Ile Asp Ile Thr Ala Gly Ala Gly Gly Thr Glu Ala Glu
    130             135             140

Asp Trp Ala Gly Met Leu Phe Arg Met Tyr Ser Arg Tyr Ala Glu Arg
145             150             155             160

Lys Gly Phe Arg Ile Glu Ile Leu Glu Glu Asp Asp Gly Glu Ile Ala
            165             170             175

Gly Ile Asn Arg Ala Thr Ile Arg Val Glu Gly Glu Tyr Ala Tyr Gly
        180             185             190

Leu Leu Arg Thr Glu Thr Gly Val His Arg Leu Val Arg Tyr Ser Pro
        195             200             205

Phe Asp Ser Asn Asn Lys Arg His Thr Ser Phe Ala Ser Val Phe Val
    210             215             220

Tyr Pro Glu Ile Asp Asp Ser Ile Glu Ile Glu Ile Asn Pro Ala Asp
225             230             235             240

Leu Arg Ile Asp Thr Tyr Arg Ala Ser Gly Ala Gly Gly Gln His Ile
            245             250             255

Asn Lys Thr Asp Ser Ala Val Arg Ile Thr His Glu Pro Thr Gly Ile
        260             265             270

Val Val Gln Cys Gln Asn Asp Arg Ser Gln His Ala Asn Lys Ala Ala
        275             280             285

Ala Met Glu Met Leu Lys Ser Lys Leu Tyr Glu Leu Glu Met Arg Lys
    290             295             300

Arg Asn Glu Glu Lys Gln Ala Leu Glu Glu Gly Lys Ser Asp Val Gly
305             310             315             320

Trp Gly Ser Gln Ile Arg Ser Tyr Val Leu Asp Ser Ser Arg Ile Lys
            325             330             335

Asp Leu Arg Thr Gly Tyr Glu Val Gly Asn Thr Lys Ala Val Leu Asp
        340             345             350

Gly Asp Leu Asp Gly Phe Ile Glu Ala Ser Leu Lys Gln Gly Val
        355             360             365
```

<210> 203
<211> 311
<212> PRT
<213> Neisseria meningitidis

<400> 203

Met Ser Val Leu Ile Ile Val Glu His Asp Asn Lys Gln Leu Asn Pro

```
         1                5                      10                     15
        Thr Thr Leu His Ala Val Thr Ala Ala Ala Lys Leu Gly Lys Val Asp
                    20                  25                  30
        Leu Leu Val Ala Gly Asn Gly Ala Ser Ala Val Val Glu Phe Ala Lys
                    35                  40                  45
        Gln Val Ala Gly Val Lys Lys Val Leu Val Ala Asp Ala Ala His Tyr
                50                  55                  60
        Ala Glu Gly Leu Ala Glu Glu Leu Ala Pro Leu Val Val Lys Leu Ala
        65                  70                  75                  80
        Ala Asp Tyr Arg Tyr Val Ala Ala Thr Ala Thr Thr Phe Gly Lys Asn
                        85                  90                  95
        Leu Leu Pro Arg Val Ala Ala Leu Leu Asp Val Pro Gln Ile Ser Asp
                    100                 105                 110
        Leu Thr Glu Ile Val Asp Asn Thr Thr Phe Val Arg Pro Ile Tyr Ala
                    115                 120                 125
        Gly Asn Ala Phe Glu Thr Val Gln Ala Asp Ser Glu Lys Leu Val Leu
            130                 135                 140
        Thr Phe Arg Ala Thr Val Phe Asp Ala Val Ala Ala Gln Gly Gly Asn
        145                 150                 155                 160
        Ala Glu Val Ile Asn Val Glu Ala Thr Pro Ala Gln Asn Leu Ser Arg
                        165                 170                 175
        Phe Val Asn Arg Gln Leu Ser His Ser Asp Arg Pro Glu Leu Thr Gln
                    180                 185                 190
        Ala Lys Val Ile Val Ser Gly Gly Arg Ala Leu Gly Ser Ala Glu Lys
                    195                 200                 205
        Phe Asn Glu Val Leu Thr Pro Leu Ala Asp Val Leu Gly Ala Ala Ile
            210                 215                 220
        Gly Ala Ser Arg Ala Ala Val Asp Ala Glu Tyr Ala Pro Asn Asp Ala
        225                 230                 235                 240
        Gln Val Gly Gln Thr Gly Lys Val Val Ala Pro Gln Leu Tyr Phe Ala
                    245                 250                 255
        Ile Gly Ile Ser Gly Ala Ile Gln His Val Ala Gly Met Gln Asp Ser
                    260                 265                 270
        Lys Val Ile Val Ala Ile Asn Lys Asp Ala Asp Ala Pro Ile Phe Asn
                    275                 280                 285
        Val Ala Asp Tyr Gly Leu Val Gly Asp Leu Phe Glu Ile Val Pro Gln
            290                 295                 300
        Leu Thr Glu Val Leu Lys Asn
        305                 310
```

<210> 204
<211> 249
<212> PRT
<213> Neisseria meningitidis

<400> 204

```
Met Lys Ala Leu Val Ala Val Lys Arg Val Val Asp Tyr Asn Val Lys
1               5               10              15

Val Arg Val Lys Ala Asp Gly Ser Asp Val Asp Ile Gly Asn Val Lys
            20              25              30

Met Ser Met Asn Pro Phe Asp Glu Ile Ala Val Glu Glu Ala Val Arg
        35              40              45

Leu Lys Glu Ala Gly Lys Val Ser Glu Ile Val Ala Val Ser Leu Gly
    50              55              60

Glu Lys Lys Cys Glu Glu Thr Leu Arg Thr Ala Leu Ala Met Gly Ala
65              70              75              80

Asp Arg Ala Ile His Val Glu Thr Asp Thr Lys Leu Glu Ser Leu Ala
            85              90              95

Val Ala Lys Leu Leu Lys Ala Val Ala Asp Lys Glu Asn Pro Gln Ile
        100             105             110

Phe Phe Leu Gly Lys Gln Ala Ile Asp Asp Asp Ala Asn Gln Val Ala
    115             120             125

Gln Met Leu Ala Ala Leu Leu Asn Ala Ala Gln Gly Thr Phe Ala Ser
    130             135             140

Lys Val Gln Ile Glu Gly Asp Glu Val Gln Ile Val Arg Glu Ile Asp
145             150             155             160

Gly Gly Glu Glu Thr Ile Ala Leu Lys Leu Pro Ala Val Ile Ser Ala
            165             170             175

Asp Leu Arg Leu Asn Glu Pro Arg Phe Val Lys Leu Pro Asn Ile Met
        180             185             190

Ala Ala Lys Lys Lys Pro Leu Glu Lys Leu Thr Pro Asp Asp Leu Val
    195             200             205

Ala Asp Ile Ser Pro Arg Leu Lys Thr Val Lys Phe Ala Glu Pro Lys
    210             215             220

Ala Arg Gln Ala Gly Val Lys Val Ala Ser Val Ala Glu Leu Val Glu
225             230             235             240

Lys Leu Lys Asn Glu Ala Lys Val Ile
            245
```

<210> 205

<211> 334

<212> PRT

<213> Neisseria meningitidis

<400> 205

```
Met Ser Ile Lys Val Ala Ile Asn Gly Phe Gly Arg Ile Gly Arg Leu
1               5                   10                  15

Ala Leu Arg Gln Ile Glu Lys Ala His Asp Ile Glu Val Val Ala Val
            20              25                  30

Asn Asp Leu Thr Pro Ala Glu Met Leu Leu His Leu Phe Lys Tyr Asp
        35                  40                  45

Ser Thr Gln Gly Arg Phe Gln Gly Thr Ala Glu Leu Lys Asp Asp Ala
    50                  55                  60

Ile Val Val Asn Gly Lys Glu Ile Lys Val Phe Ala Asn Pro Asn Pro
65                  70                  75                  80

Glu Glu Leu Pro Trp Gly Glu Leu Gly Val Asp Val Ile Leu Glu Cys
            85                  90                  95

Thr Gly Phe Phe Thr Asn Lys Thr Lys Ala Glu Ala His Ile Arg Ala
            100                 105                 110

Gly Ala Arg Lys Val Val Ile Ser Ala Pro Gly Gly Asn Asp Val Lys
            115                 120                 125

Thr Val Val Tyr Gly Val Asn Gln Asp Ile Leu Asp Gly Ser Glu Thr
    130                 135                 140

Val Ile Ser Ala Ala Ser Cys Thr Thr Asn Cys Leu Ala Pro Met Ala
145                 150                 155                 160

Ala Val Leu Gln Lys Glu Phe Gly Val Val Glu Gly Leu Met Thr Thr
            165                 170                 175

Ile His Ala Tyr Thr Gly Asp Gln Asn Thr Leu Asp Ala Pro His Arg
            180                 185                 190

Lys Gly Asp Leu Arg Arg Ala Arg Ala Ala Ala Leu Asn Ile Val Pro
            195                 200                 205

Asn Ser Thr Gly Ala Ala Lys Ala Ile Gly Leu Val Ile Pro Glu Leu
    210                 215                 220

Asn Gly Lys Leu Asp Gly Ser Ala Gln Arg Val Pro Val Ala Ser Gly
225                 230                 235                 240

Ser Leu Thr Glu Leu Val Ser Ile Leu Glu Arg Pro Val Thr Lys Glu
            245                 250                 255

Glu Ile Asn Ala Ala Met Lys Ala Ala Ala Ser Glu Ser Tyr Gly Tyr
            260                 265                 270

Asn Glu Asp Gln Ile Val Ser Ser Asp Val Val Gly Ile Glu Tyr Gly
            275                 280                 285

Ser Leu Phe Asp Ala Thr Gln Thr Arg Val Met Thr Val Gly Gly Lys
    290                 295                 300

Gln Leu Val Lys Thr Val Ala Trp Tyr Asp Asn Glu Met Ser Tyr Thr
305                 310                 315                 320

Cys Gln Leu Val Arg Thr Leu Glu Tyr Phe Ala Gly Lys Ile
            325                 330
```

<210> 206
<211> 223
<212> PRT
<213> Neisseria meningitidis

<400> 206

```
Met Lys Thr Val Ser Thr Ala Val Val Leu Ala Ala Ala Ala Val Ser
1               5               10                  15
Leu Thr Gly Cys Ala Thr Glu Ser Ser Arg Ser Leu Glu Val Glu Lys
            20              25                  30
Val Ala Ser Tyr Asn Thr Gln Tyr His Gly Val Arg Thr Pro Ile Ser
            35              40                  45
Val Gly Thr Phe Asp Asn Arg Ser Ser Phe Gln Lys Gly Ile Phe Ser
        50              55                  60
Asp Gly Glu Asp Arg Leu Gly Ser Gln Ala Lys Thr Ile Leu Val Thr
65              70                  75                  80
His Leu Gln Gln Thr Asn Arg Phe Asn Val Leu Asn Arg Thr Asn Leu
                85              90                  95
Asn Ala Leu Lys Gln Glu Ser Gly Ile Ser Gly Lys Ala His Asn Leu
            100             105                 110
Lys Gly Ala Asp Tyr Val Val Thr Gly Asp Val Thr Glu Phe Gly Arg

            115                 120                 125
Arg Asp Val Gly Asp His Gln Leu Phe Gly Ile Leu Gly Arg Gly Lys
    130                 135                 140
Ser Gln Ile Ala Tyr Ala Lys Val Ala Leu Asn Ile Val Asn Val Asn
145                 150                 155                 160
Thr Ser Glu Ile Val Tyr Ser Ala Gln Gly Ala Gly Glu Tyr Ala Leu
            165                 170                 175
Ser Asn Arg Glu Ile Ile Gly Phe Gly Gly Thr Ser Gly Tyr Asp Ala
            180             185                 190
Thr Leu Asn Gly Lys Val Leu Asp Leu Ala Ile Arg Glu Ala Val Asn
        195                 200                 205
Ser Leu Val Gln Ala Val Asp Asn Gly Ala Trp Gln Pro Asn Arg
    210                 215                 220
```

<210> 207
<211> 389
<212> PRT
<213> Neisseria meningitidis

<400> 207

```
Met Ser Glu Tyr Leu Phe Thr Ser Glu Ser Val Ser Glu Gly His Pro
1               5                   10                  15

Asp Lys Val Ala Asp Gln Val Ser Asp Ala Ile Leu Asp Ala Ile Leu
            20                  25                  30

Ala Gln Asp Pro Lys Ala Arg Val Ala Ala Glu Thr Leu Val Asn Thr
        35              40                  45

Gly Leu Cys Val Leu Ala Gly Glu Ile Thr Thr Thr Ala Gln Val Asp
    50              55                  60

Tyr Ile Lys Val Ala Arg Glu Thr Ile Lys Arg Ile Gly Tyr Asn Ser
65              70                  75                  80

Ser Glu Leu Gly Phe Asp Ala Asn Gly Cys Ala Val Gly Val Tyr Tyr
            85                  90                  95

Asp Gln Gln Ser Pro Asp Ile Ala Gln Gly Val Asn Glu Gly Glu Gly
        100                 105                 110

Ile Asp Leu Asn Gln Gly Ala Gly Asp Gln Gly Leu Met Phe Gly Tyr
        115                 120                 125

Ala Cys Asp Glu Thr Pro Thr Leu Met Pro Phe Ala Ile Tyr Tyr Ser
    130             135                 140

His Arg Leu Met Gln Arg Gln Ser Glu Leu Arg Lys Asp Gly Arg Leu
145             150                 155                 160

Pro Trp Leu Arg Pro Asp Ala Lys Ala Gln Leu Thr Val Val Tyr Asp
            165                 170                 175

Ser Glu Thr Gly Lys Val Lys Arg Ile Asp Thr Val Val Leu Ser Thr
            180                 185                 190

Gln His Asp Pro Ser Ile Ala Tyr Glu Glu Leu Lys Asn Ala Val Ile
        195                 200                 205

Glu His Ile Ile Lys Pro Val Leu Pro Ser Glu Leu Leu Thr Asp Glu
    210             215                 220

Thr Lys Tyr Leu Ile Asn Pro Thr Gly Arg Phe Val Ile Gly Gly Pro
225             230                 235                 240
```

```
Gln Gly Asp Cys Gly Leu Thr Gly Arg Lys Ile Ile Val Asp Thr Tyr
                245                 250                 255

Gly Gly Ala Ala Pro His Gly Gly Gly Ala Phe Ser Gly Lys Asp Pro
            260             265             270

Ser Lys Val Asp Arg Ser Ala Ala Tyr Ala Cys Arg Tyr Val Ala Lys
        275             280             285

Asn Ile Val Ala Ala Gly Leu Ala Thr Gln Cys Gln Ile Gln Val Ser
    290             295             300

Tyr Ala Ile Gly Val Ala Glu Pro Thr Ser Ile Ser Ile Asp Thr Phe
305             310             315             320

Gly Thr Gly Lys Ile Ser Glu Glu Lys Leu Ile Ala Leu Val Arg Glu
            325             330             335

His Phe Asp Leu Arg Pro Lys Gly Ile Val Gln Met Leu Asp Leu Leu
        340             345             350

Arg Pro Ile Tyr Ser Lys Ser Ala Ala Tyr Gly His Phe Gly Arg Glu
        355             360             365

Glu Pro Glu Phe Thr Trp Glu Arg Thr Asp Lys Ala Ala Ala Leu Arg
    370             375             380

Ala Ala Ala Gly Leu
385
```

<210> 208
<211> 167
<212> PRT
<213> Neisseria meningitidis

<400> 208

```
Met Ala Leu Leu Asn Ile Leu Gln Tyr Pro Asp Glu Arg Leu His Thr
1               5                   10                  15

Val Ala Lys Pro Val Glu Gln Val Asp Glu Arg Ile Arg Lys Leu Ile
            20                  25                  30

Ala Asp Met Phe Glu Thr Met Tyr Glu Ser Arg Gly Ile Gly Leu Ala
        35                  40                  45

Ala Thr Gln Val Asp Val His Glu Arg Val Val Val Met Asp Leu Thr
        50                  55                  60

Glu Asp Arg Ser Glu Pro Arg Val Phe Ile Asn Pro Val Ile Val Glu
65                  70                  75                  80

Lys Asp Gly Glu Thr Thr Tyr Glu Glu Gly Cys Leu Ser Val Pro Gly
                85                  90                  95

Ile Tyr Asp Thr Val Thr Arg Ala Glu Arg Val Lys Val Glu Ala Leu
                100                 105                 110

Asn Glu Lys Gly Glu Lys Phe Thr Leu Glu Ala Asp Gly Leu Leu Ala
            115                 120                 125

Ile Cys Val Gln His Glu Leu Asp His Leu Met Gly Ile Val Phe Val
        130                 135                 140

Glu Arg Leu Ser Gln Leu Lys Gln Gly Arg Ile Lys Thr Lys Leu Lys
145                 150                 155                 160

Lys Arg Gln Lys His Thr Ile
                165
```

<210> 209
<211> 477
<212> PRT
<213> Neisseria meningitidis

<400> 209

```
Met Ser Gln Tyr Asp Val Val Val Ile Gly Ala Gly Pro Gly Gly Tyr
1               5               10              15

Val Ala Ala Ile Arg Ala Ala Gln Leu Gly Phe Lys Thr Ala Cys Val
            20              25              30

Asp Ala Gly Val Asn Lys Ala Gly Asn Ala Pro Ala Leu Gly Gly Thr
        35              40              45

Cys Leu Asn Val Gly Cys Ile Pro Ser Lys Ala Leu Leu Gln Ser Ser
    50              55              60

Glu His Phe His Ala Ala Gln His Glu Phe Ala Glu His Gly Ile Thr
65              70              75              80

Val Gly Asp Val Lys Phe Asp Ala Ala Lys Met Ile Glu Arg Lys Asp
            85              90              95

Ala Ile Val Thr Lys Leu Thr Gly Gly Val Lys Phe Leu Phe Gln Lys
        100             105             110

Asn Lys Val Thr Ser Leu Phe Gly Thr Ala Ser Phe Ala Gly Lys Asn
    115             120             125

Gly Asp Ala Tyr Gln Ile Glu Val Asp Asn Lys Gly Glu Lys Thr Val
    130             135             140

Ile Glu Ala Lys His Val Ile Val Ala Thr Gly Ser Val Pro Arg Pro
145             150             155             160

Leu Pro Gln Val Ala Ile Asp Asn Val Asn Val Leu Asp Asn Glu Gly
            165             170             175

Ala Leu Asn Leu Thr Glu Val Pro Ala Lys Leu Gly Val Ile Gly Ser
    180             185             190

Gly Val Ile Gly Leu Glu Met Gly Ser Val Trp Asn Arg Val Gly Ala
    195             200             205

Glu Val Thr Ile Leu Glu Ala Ala Pro Thr Phe Leu Ala Ala Ala Asp
    210             215             220

Gln Gln Ile Ala Lys Glu Ala Phe Lys Tyr Phe Thr Lys Glu Gln Gly
225             230             235             240

Leu Ser Ile Glu Leu Gly Val Lys Ile Gly Asp Ile Lys Ser Glu Gly
            245             250             255

Lys Gly Val Ser Val Ala Tyr Glu Thr Ala Ala Gly Glu Ala Lys Thr
        260             265             270

Glu Val Phe Asp Lys Leu Ile Val Ala Ile Gly Arg Ile Pro Asn Thr
    275             280             285

Lys Gly Leu Asn Ala Glu Ala Val Gly Leu Glu Lys Asp Glu Arg Gly
    290             295             300

Phe Ile Lys Val Asp Gly Glu Cys Arg Thr Asn Leu Pro Asn Val Trp
305             310             315             320

Ala Ile Gly Asp Val Val Arg Gly Pro Met Leu Ala His Lys Ala Ser
            325             330             335

Asp Glu Gly Val Ala Val Ala Glu Arg Ile Ala Gly Gln Lys Pro His
```

```
                340                        345                        350

      Ile Asp Phe Asn Asn Val Pro Phe Val Ile Tyr Thr Asp Pro Glu Ile
              355                 360                 365

      Ala Trp Val Gly Lys Thr Glu Glu Gln Leu Lys Ala Glu Gly Val Glu
          370                 375                 380

      Tyr Lys Lys Gly Thr Ser Gly Phe Gly Ala Asn Gly Arg Ala Leu Ala
      385                 390                 395                 400

      Met Gly Lys Ala Lys Gly Thr Val Lys Val Leu Ala Asp Ala Lys Thr
                      405                 410                 415

      Asp Arg Ile Leu Gly Val His Met Ile Gly Pro Val Val Ser Glu Leu
                  420                 425                 430

      Val Thr Glu Gly Val Thr Ala Leu Glu Phe Phe Ala Ser Ser Glu Asp
              435                 440                 445

      Ile Ala Arg Ile Ile His Ala His Pro Thr Leu Ser Glu Val Val His
          450                 455                 460

      Glu Ala Ala Leu Ala Ala Asp Lys Arg Ala Leu His Gly
      465                 470                 475
```

<210> 210
<211> 315
<212> PRT
<213> Neisseria meningitidis

<400> 210

```
Met Met Ile Glu Lys Ser Ile Ser Ile Val Asp Gly Lys Glu Tyr Ser
1               5                   10                  15

Val Phe Ala Val Ser His Glu Phe Arg Tyr Thr Phe Asp Glu Pro Ile
            20              25              30

Leu Val Ala Asp Leu Ile Ser Ser Leu Lys Ala Tyr Glu Thr Leu Thr
        35                  40                  45

Ser Ser Tyr Leu Pro Ala Ile Leu Asn Gln Leu Phe Asp Val Lys Ile
        50                  55                  60

Gln Lys Ile Lys Val Ala Val Ser Glu Ile Glu Arg Gly Ser Phe Leu
65                  70                  75                      80

Glu Lys Leu Ile Phe Asn Leu Phe Phe Lys Asp Glu Asp Ala Tyr Asn
                85                  90                  95

Glu Phe Cys Leu Lys Ile Arg Lys Phe Leu Gly Thr Glu Asn Gln Asp
                100                 105                 110

Gly Ser Ile Asn Met Ser Lys Ile Ile Met Phe Ala Met Thr Thr Leu
            115                 120                 125

Leu Gly Val Gly Ala Gly Tyr Leu Leu Phe Lys Asn Pro Pro Gln Glu
    130                 135                 140

Lys Gln Ala Ile Thr Asn Asn Ile Val Thr Val Ile Asn Ala Asp Ser
145                 150                 155                 160

Ser Val Ala Leu Asp Gly Glu His Leu Val Ser Val Val Lys Glu Val
                165                 170                 175

Thr Gly Ser Ser Lys Gln Lys Thr Ala Glu Asn Val Ala Lys Val Tyr
            180                 185                 190

Ala Pro Ala Ser Lys Asn Asn Gly Ser Ile Thr Leu Gly Thr Asp Asp
        195                 200                 205

Val Arg Ile Glu Pro Val Ala Gln Gln Thr Val Ala Thr Leu Pro Lys
    210                 215                 220

Asp Val Asp Leu Arg Asp Thr Pro Leu Thr Glu Asp Tyr Thr Asp Ile
225                 230                 235                 240

Asp Val Gln Ile Arg Ala Thr Asp Arg Asp Lys Asn Ser Gly Trp Tyr
                245                 250                 255

Ala Val Ile Asp Gln Ile Val Pro Ser Arg Val Arg Leu Glu Leu Pro
            260                 265                 270

Glu Asp Ile Asp Leu Asn Arg Leu Ala Asn Asn Ala Thr Ile Arg Ala
        275                 280                 285

Asn Val Thr Val Glu Phe Asp Leu Lys Gln Asn Gly Ser Arg Lys Pro
    290                 295                 300

Lys Lys Ile Ile Leu Thr Ser Leu Ser Thr Asp
305                 310                 315
```

<210> 211
<211> 416
<212> PRT
<213> Neisseria meningitidis

<400> 211

```
Met Phe Ser Lys Ser Val Thr Leu Ala Gln Tyr Asp Pro Asp Leu Ala
1               5                   10              15

Ala Ala Ile Ala Gln Glu Asp Gln Arg Gln Gln Asp His Val Glu Leu
            20              25              30

Ile Ala Ser Glu Asn Tyr Val Ser Cys Ala Val Met Asp Ala Gln Gly
        35              40              45

Ser Gln Leu Thr Asn Lys Tyr Ala Glu Gly Tyr Pro Gly Lys Arg Tyr
    50              55              60

Tyr Gly Gly Cys Glu Tyr Val Asp Ile Val Glu Gln Leu Ala Ile Asp
65              70              75              80

Arg Val Lys Glu Leu Phe Gly Ala Ala Tyr Ala Asn Val Gln Pro His
            85              90              95

Ser Gly Ser Gln Ala Asn Gln Ala Val Tyr Ala Ser Val Leu Lys Pro
            100             105             110

Gly Asp Thr Ile Leu Gly Met Ser Leu Ala His Gly Gly His Leu Thr
        115             120             125

His Gly Ala Ser Val Asn Ile Ser Gly Lys Leu Tyr Asn Ala Val Thr
    130             135             140

Tyr Gly Leu Asp Glu Asn Glu Val Leu Asp Tyr Ala Glu Val Glu Arg
145             150             155             160

Leu Ala Leu Glu His Lys Pro Lys Met Ile Val Ala Gly Ala Ser Ala
            165             170             175

Tyr Ala Leu Gln Ile Asp Trp Ala Lys Phe Arg Glu Ile Ala Asp Lys
        180             185             190

Val Gly Ala Tyr Leu Phe Val Asp Met Ala His Tyr Ala Gly Leu Val
        195             200             205

Ala Gly Gly Glu Tyr Pro Asn Pro Val Pro Phe Cys Asp Phe Val Thr
    210             215             220
```

```
Thr Thr Thr His Lys Thr Leu Arg Gly Pro Arg Gly Gly Val Ile Leu
225             230             235             240

Cys Arg Asp Asn Thr His Glu Lys Ala Leu Asn Ser Ser Ile Phe Pro
            245             250             255

Ser Leu Gln Gly Gly Pro Leu Met His Val Ile Ala Ala Lys Ala Val
        260             265             270

Ala Phe Lys Glu Ala Leu Gln Pro Glu Phe Lys Gln Tyr Ala Lys Gln
        275             280             285

Val Lys Ile Asn Ala Ala Ala Met Ala Glu Glu Leu Val Lys Arg Gly
    290             295             300

Leu Arg Ile Val Ser Gly Arg Thr Glu Ser His Val Phe Leu Val Asp
305             310             315             320

Leu Gln Pro Met Lys Ile Thr Gly Lys Ala Ala Glu Ala Ala Leu Gly
            325             330             335

Lys Ala His Ile Thr Val Asn Lys Asn Ala Ile Pro Asn Asp Pro Glu
            340             345             350

Lys Pro Phe Val Thr Ser Gly Ile Arg Ile Gly Ser Ala Ala Met Thr
        355             360             365

Thr Arg Gly Phe Asn Glu Ala Asp Ala Arg Val Leu Ala Asn Leu Val
    370             375             380

Ala Asp Val Leu Ser Asn Pro Glu Asp Glu Ala Asn Leu Ala Lys Val
385             390             395             400

Arg Lys Gln Val Thr Ala Leu Cys Asn Lys Tyr Pro Val Tyr Gly Ala
            405             410             415
```

<210> 212
<211> 233
<212> PRT
<213> Neisseria meningitidis

<400> 212

```
Met Ser Ala Arg Glu Asn Ile Leu Ala Lys Leu Lys Lys Ala Asp Ala
1               5               10              15

Leu Pro Met Glu Glu Pro Ala Val Phe Asp Tyr Tyr Arg Glu Met Gly
            20              25              30

Val Ser Trp Gly Ser Glu Val Glu Arg Leu Lys His Trp Ala Ala Ala
        35              40              45

Met Arg Ala Val Lys Thr Glu Ile Tyr Trp Val Thr Lys Ser Asn Trp
    50              55              60

Met Gln Val Phe Arg Glu Ala Ala Glu Gly Lys Gly Leu Lys Asn Ile
65              70              75              80

Leu Leu Pro Leu Ala Thr Glu His Gly Gln Ile Ala Arg Ala Ala Leu
            85              90              95

Ala Asp Ser Asn Ile Glu Pro Ile Ala Phe Glu Arg Glu Ile Asp Thr
            100             105             110

Trp Lys Thr Glu Phe Phe Thr Asn Ile Asp Ala Gly Phe Ser Gly Ala
        115             120             125

Gln Cys Gly Ile Ala Arg Thr Gly Thr Leu Met Leu Phe Ser Ser Pro
    130             135             140

Glu Glu Pro Arg Thr Leu Ser Leu Val Pro Pro Val His Phe Cys Leu
145             150             155             160

Phe Asp Thr Ser Lys Met Tyr Asn Glu Phe His Asn Ala Val Glu Gly
            165             170             175

Glu Lys Leu Val Glu Asn Gly Met Pro Thr Asn Val Phe Leu Ile Ser
            180             185             190

Gly Pro Ser Lys Thr Ala Asp Ile Gln Leu Thr Leu Ala Tyr Gly Ala
        195             200             205

His Gly Pro Arg Asp Leu Val Ile Leu Ala Ile Leu Pro Asp His Ile
    210             215             220

Ser Pro Ala Asp Leu Glu Glu Asn Ala
225             230
```

<210> 213
<211> 174
<212> PRT
<213> Neisseria meningitidis

<400> 213

```
Met Ser Leu Asn Lys Val Ile Leu Ile Gly Arg Leu Gly Arg Asp Pro
1               5               10              15

Glu Val Arg Tyr Met Pro Asn Gly Glu Ala Val Cys Asn Phe Ser Val
            20          ,       25              30

Ala Thr Ser Glu Thr Trp Asn Asp Arg Asn Gly Gln Arg Val Glu Arg
        35              40              45

Thr Glu Trp His Asn Ile Thr Met Tyr Arg Lys Leu Ala Glu Ile Ala
    50              55              60

Gly Gln Tyr Leu Lys Lys Gly Gly Leu Val Tyr Leu Glu Gly Arg Ile
65              70              75              80

Gln Ser Arg Lys Tyr Gln Gly Lys Asp Gly Ile Glu Arg Thr Ala Tyr
            85              90              95

Asp Ile Val Ala Asn Glu Met Lys Met Leu Gly Gly Arg Asn Glu Asn
            100             105             110

Ser Gly Gly Ala Pro Tyr Glu Glu Gly Tyr Gly Gln Ser Gln Glu Ala
        115             120             125

Tyr Gln Arg Pro Ala Gln Gln Ser Arg Gln Pro Ala Ser Asp Ala Pro
    130             135             140

Ser His Pro Gln Glu Ala Pro Ala Ala Pro Arg Arg Gln Pro Val Pro
145             150             155             160

Ala Ala Ala Pro Val Glu Asp Ile Asp Asp Asp Ile Pro Phe
            165             170
```

<210> 214
<211> 943
<212> PRT
<213> Neisseria meningitidis

<400> 214

```
Met Asn Lys Lys His Gly Phe Pro Leu Thr Leu Thr Ala Leu Ala Ile
1               5               10              15

Ala Thr Ala Phe Pro Ala Tyr Ala Ala Gln Ala Gly Gly Ala Thr Pro
            20              25              30

Asp Ala Ala Gln Thr Gln Ser Leu Lys Glu Ile Thr Val Arg Ala Ala
```

|  | 35 |  |  |  |  | 40 |  |  |  |  | 45 |  |  |
|---|---|---|---|---|---|---|---|---|---|---|---|---|---|

Lys Val Gly Arg Arg Ser Lys Glu Ala Thr Gly Leu Gly Lys Ile Val
50 55 60

Lys Thr Ser Glu Thr Leu Asn Lys Glu Gln Val Leu Gly Ile Arg Asp
65 70 75 80

Leu Thr Arg Tyr Asp Pro Gly Val Ala Val Val Glu Gln Gly Asn Gly
85 90 95

Ala Ser Gly Gly Tyr Ser Ile Arg Gly Val Asp Lys Asn Arg Val Ala
100 105 110

Val Ser Val Asp Gly Val Ala Gln Ile Gln Ala Phe Thr Val Gln Gly
115 120 125

Ser Leu Ser Gly Tyr Gly Gly Arg Gly Gly Ser Gly Ala Ile Asn Glu
130 135 140

Ile Glu Tyr Glu Asn Ile Ser Thr Val Glu Ile Asp Lys Gly Ala Gly
145 150 155 160

Ser Ser Asp His Gly Ser Gly Ala Leu Gly Gly Ala Val Ala Phe Arg
165 170 175

Thr Lys Glu Ala Ala Asp Leu Ile Ser Asp Gly Lys Ser Trp Gly Ile
180 185 190

Gln Ala Lys Thr Ala Tyr Gly Ser Lys Asn Arg Gln Phe Met Lys Ser
195 200 205

Leu Gly Ala Gly Phe Ser Lys Asp Gly Trp Glu Gly Leu Leu Ile Arg
210 215 220

Thr Glu Arg Gln Gly Arg Glu Thr Arg Pro His Gly Asp Ile Ala Asp
225 230 235 240

Gly Val Glu Tyr Gly Ile Asp Arg Leu Asp Ala Phe Arg Gln Thr Tyr
245 250 255

Asp Ile Lys Arg Lys Thr Arg Glu Pro Phe Phe Ser Val Glu Gly Glu
260 265 270

Arg Glu Ser Lys Pro Val Ala Lys Leu Ala Gly Tyr Gly Lys Tyr Leu
275 280 285

Asn Asn Gln Leu Asn Arg Trp Val Lys Glu Arg Ile Glu Gln Asn Gln
290 295 300

Pro Leu Ser Ala Glu Glu Glu Ala Gln Val Arg Glu Ala Gln Ala Arg
305 310 315 320

His Glu Asn Leu Ser Ala Gln Ala Tyr Thr Gly Gly Gly Arg Ile Leu
325 330 335

Pro Asp Pro Met Asp Tyr Arg Ser Gly Ser Trp Leu Ala Lys Leu Gly
340 345 350

Tyr Arg Phe Gly Gly Arg His Tyr Val Gly Gly Val Phe Glu Asp Thr
355 360 365

Lys Gln Arg Tyr Asp Ile Arg Asp Met Thr Glu Lys Gln Tyr Tyr Gly
370 375 380

Thr Asp Glu Ala Glu Lys Phe Arg Asp Lys Ser Gly Val Tyr Asp Gly
385 390 395 400

Asp Asp Phe Arg Asp Gly Leu Tyr Phe Val Pro Asn Ile Glu Glu Trp
405 410 415

```
Lys Gly Asp Lys Asn Leu Val Arg Gly Ile Gly Leu Lys Tyr Ser Arg
            420                 425             430

Thr Lys Phe Ile Asp Glu His His Arg Arg Arg Arg Met Gly Leu Leu
            435             440             445

Tyr Arg Tyr Glu Asn Glu Ala Tyr Ser Asp Asn Trp Ala Asp Lys Ala
    450             455             460

Val Leu Ser Phe Asp Lys Gln Gly Val Ala Thr Asp Asn Asn Thr Leu
465             470             475                         480

Lys Leu Asn Cys Ala Val Tyr Pro Ala Val Asp Lys Ser Cys Arg Ala
            485             490             495

Ser Ala Asp Lys Pro Tyr Ser Tyr Asp Ser Ser Asp Arg Phe His Tyr
            500             505             510

Arg Glu Gln His Asn Val Leu Asn Ala Ser Phe Glu Lys Ser Leu Lys
        515             520             525

Asn Lys Trp Thr Lys His His Leu Thr Leu Gly Phe Gly Tyr Asp Ala
    530             535             540

Ser Lys Ala Ile Ser Arg Pro Glu Gln Leu Ser His Asn Ala Ala Arg
545             550             555                         560

Ile Ser Glu Ser Thr Gly Phe Asp Glu Asn Asn Gln Asp Lys Tyr Leu
            565             570             575

Leu Gly Lys Pro Glu Val Val Glu Gly Ser Val Cys Gly Tyr Ile Glu
            580             585             590

Thr Leu Arg Ser Arg Lys Cys Val Pro Arg Lys Ile Asn Gly Ser Asn
        595             600             605

Ile His Ile Ser Leu Asn Asp Arg Phe Ser Ile Gly Lys Tyr Phe Asp
    610             615             620

Phe Ser Leu Gly Gly Arg Tyr Asp Arg Lys Asn Phe Thr Thr Ser Glu
625             630             635                         640

Glu Leu Val Arg Ser Gly Arg Tyr Val Asp Arg Ser Trp Asn Ser Gly
            645             650             655

Ile Leu Phe Lys Pro Asn Arg His Phe Ser Val Ser Tyr Arg Ala Ser
            660             665             670

Ser Gly Phe Arg Thr Pro Ser Phe Gln Glu Leu Phe Gly Ile Asp Ile
            675             680             685

Tyr His Asp Tyr Pro Lys Gly Trp Gln Arg Pro Ala Leu Lys Ser Glu
    690             695             700

Lys Ala Ala Asn Arg Glu Ile Gly Leu Gln Trp Lys Gly Asp Phe Gly
705             710             715                         720

Phe Leu Glu Ile Ser Ser Phe Arg Asn Arg Tyr Thr Asp Met Ile Ala
            725             730             735

Val Ala Asp His Lys Thr Lys Leu Pro Asn Gln Ala Gly Gln Leu Thr
            740             745             750

Glu Ile Asp Ile Arg Asp Tyr Tyr Asn Ala Gln Asn Met Ser Leu Gln
        755             760             765

Gly Val Asn Ile Leu Gly Lys Ile Asp Trp Asn Gly Val Tyr Gly Lys
    770             775             780
```

```
Leu Pro Glu Gly Leu Tyr Thr Thr Leu Ala Tyr Asn Arg Ile Lys Pro
785                 790             795                 800

Lys Ser Val Ser Asn Arg Pro Gly Leu Ser Leu Arg Ser Tyr Ala Leu
                805             810                 815

Asp Ala Val Gln Pro Ser Arg Tyr Val Leu Gly Phe Gly Tyr Asp Gln
            820             825             830

Pro Glu Gly Lys Trp Gly Ala Asn Ile Met Leu Thr Tyr Ser Lys Gly
        835             840             845

Lys Asn Pro Asp Glu Leu Ala Tyr Leu Ala Gly Asp Gln Lys Arg Tyr
    850             855             860

Ser Thr Lys Arg Ala Ser Ser Ser Trp Ser Thr Ala Asp Val Ser Ala
865             870             875                 880

Tyr Leu Asn Leu Lys Lys Arg Leu Thr Leu Arg Ala Ala Ile Tyr Asn
            885             890                 895

Ile Gly Asn Tyr Arg Tyr Val Thr Trp Glu Ser Leu Arg Gln Thr Ala
        900             905             910

Glu Ser Thr Ala Asn Arg His Gly Gly Asp Ser Asn Tyr Gly Arg Tyr
    915             920             925

Ala Ala Pro Gly Arg Asn Phe Ser Leu Ala Leu Glu Met Lys Phe
    930             935             940
```

<210> 215
<211> 544
<212> PRT
<213> Neisseria meningitidis

<400> 215

```
Met Thr Lys Phe Ile Phe Val Thr Gly Gly Val Val Ser Ser Leu Gly
1               5               10              15

Lys Gly Ile Ala Ala Ala Ser Ile Ala Ala Ile Leu Glu Ser Arg Gly
            20              25              30

Leu Asn Val Thr Met Leu Lys Leu Asp Pro Tyr Ile Asn Val Asp Pro
        35              40              45

Gly Thr Met Ser Pro Phe Gln His Gly Glu Val Phe Val Thr Asp Asp
    50              55              60

Gly Ala Glu Thr Asp Leu Asp Leu Gly His Tyr Glu Arg Phe Ile Asp
65              70              75              80

Ser Thr Met Thr Arg Arg Asn Ser Phe Ser Thr Gly Gln Val Tyr Glu
            85              90              95

Asn Val Ile Ala Lys Glu Arg Arg Gly Asp Tyr Leu Gly Gly Thr Val
            100             105             110

Gln Val Ile Pro His Ile Thr Asp Glu Ile Lys Arg Arg Ile His Glu
            115             120             125

Gly Ala Ala Gly Tyr Asp Val Ala Ile Val Glu Ile Gly Gly Thr Val
    130             135             140

Gly Asp Ile Glu Ser Leu Pro Phe Leu Glu Ala Ile Arg Gln Met Arg
145             150             155             160

Ser Gln Leu Gly Arg Asn Asn Thr Leu Phe Ala His Leu Ser Tyr Val
            165             170             175

Pro Tyr Ile Ala Ala Ala Gly Glu Ile Lys Thr Lys Pro Thr Gln His
```

```
              180                      185                      190
     Thr Val Lys Glu Met Leu Ser Ile Gly Leu Gln Pro Asp Ile Leu Ile
             195                      200                      205
     Cys Arg Met Asp Arg Thr Met Pro Ala Asp Glu Arg Arg Lys Ile Ala
             210                      215                      220
     Leu Phe Cys Asn Val Glu Glu Arg Ala Ile Val Gly Ser Tyr Asp Val
     225                      230                      235                      240
     Asp Ser Ile Tyr Glu Cys Pro Glu Met Leu His Asp Gln Gly Ile Asp
                          245                      250                      255
     Asn Ile Ile Thr Glu Gln Leu Gln Leu Asn Val Gln Gln Ala Asp Leu
                      260                      265                      270
     Thr Ala Trp Lys Lys Ile Val His Ala Ile Gln Asn Pro Lys His Thr
                  275                      280                      285
     Val Lys Ile Ala Met Val Gly Lys Tyr Val Asp Leu Thr Glu Ser Tyr
             290                      295                      300
     Lys Ser Leu Ile Glu Ala Leu Lys His Ala Gly Ile His Thr Glu Thr
     305                      310                      315                      320
     Asp Val Gln Ile Thr Phe Val Asp Ser Glu Asn Ile Glu Lys Asn Lys
                          325                      330                      335
     Gly Asp Val Ser Met Leu Lys Asp Met Asp Ala Ile Leu Val Pro Gly
                  340                      345                      350
     Gly Phe Gly Ser Arg Gly Val Glu Gly Lys Ile Ala Ala Val Arg Tyr
             355                      360                      365
     Ala Arg Glu Asn Asn Val Pro Tyr Leu Gly Ile Cys Leu Gly Met Gln
         370                      375                      380
     Ile Ala Leu Ile Glu Tyr Ala Arg Asp Val Ala Gly Leu Lys Gly Ala
     385                      390                      395                      400
     Asn Ser Thr Glu Phe Asp Leu Lys Cys Ala Ala Pro Val Val Ala Leu
                          405                      410                      415
     Ile Asp Glu Trp Gln Thr Ala Asp Gly Ser Val Glu Thr Arg Asp Glu
                  420                      425                      430
     Ser Thr Asp Leu Gly Gly Thr Met Arg Leu Gly Ala Gln Glu Val Glu
                  435                      440                      445
     Leu Lys Ala Gly Ser Leu Ala Ala Lys Ile Tyr Gly Ser Gly His Ile
         450                      455                      460
     Arg Glu Arg His Arg His Arg Tyr Glu Val Asn Asn Asn Tyr Val Pro
     465                      470                      475                      480
     Thr Leu Glu Gln Ala Gly Leu Val Ile Gly Gly Val Ser Ala Gly Arg
                          485                      490                      495
     Glu Arg Leu Val Glu Thr Ile Glu Leu Pro Asn His Pro Trp Phe Phe
                  500                      505                      510
     Ala Cys Gln Phe His Pro Glu Phe Thr Ser Asn Pro Arg Lys Gly His
             515                      520                      525
     Pro Leu Phe Thr Ala Phe Val Lys Ala Ala Leu Asn Asn Lys Lys Ala
         530                      535                      540
```

<210> 216
<211> 163
<212> PRT
<213> Neisseria meningitidis

<400> 216

```
Met Arg His Ile Leu Ser Val Leu Ile Glu Asn Glu Ser Gly Ala Met
1               5                   10                  15

Ser Arg Val Val Gly Leu Phe Ser Ala Arg Asp Tyr Asn Ile Asp Ser
            20                  25                  30

Leu Ala Val Ala Pro Thr Glu Asp Lys Thr Leu Ser Arg Met Thr Ile
            35                  40                  45

Val Thr His Gly Asp Glu Gln Val Ile Glu Gln Ile Thr Lys Gln Leu
        50                  55                  60

Asn Lys Leu Ile Glu Val Ile Lys Val Val Asp Leu Asn Glu Ser Arg
65                  70                  75                  80

Phe Val Glu Arg Glu Leu Met Leu Val Lys Val Arg Ala Ala Gly Lys
                85                  90                  95

Asp Arg Asp Glu Phe Leu Arg Leu Thr Glu Ile Tyr Arg Gly Ser Ile
            100                 105                 110

Ile Asp Val Thr Asp Arg Ser Tyr Thr Ile Glu Ile Thr Gly Ser Thr
            115                 120                 125

Asp Lys Leu Asp Ser Phe Leu Glu Thr Val Gly Arg Ala Gln Ile Leu
        130                 135                 140

Glu Thr Val Arg Thr Gly Ala Ala Gly Ile Gly Arg Gly Glu Arg Ile
145                 150                 155                 160

Leu Lys Ile
```

<210> 217
<211> 371
<212> PRT
<213> Neisseria meningitidis

<400> 217

```
Met Arg Leu Phe Lys Ser Leu Lys Asn Pro Lys Lys Thr Asp Ala Lys
1               5                   10              15

Leu Pro Lys Lys Ser Ser Gly Leu Asn Asn Arg Ala Ala Ile Gly Ile
            20                  25              30

Asp Ile Asp Gln His Ser Ile Lys Met Val Gln Leu Ser Gly Arg Ser
        35              40              45

Leu Asn Gln Ile Gln Leu Glu Lys Tyr Val Ile Ala Lys Leu Pro Lys
    50              55              60

Asn Ile Ile Gln Gly Asn Lys Val Gln Asn Tyr Asp Gln Leu Val Thr
65              70              75              80

Tyr Leu Gln Gln Ala Tyr Ala Lys Leu Gly Thr Ser Cys Lys Asn Ile
            85              90              95

Ile Ala Ser Val Pro Gln Asn Leu Ala Thr Ile Glu Gln Leu Thr Tyr
            100             105             110

Thr Asp Lys Asp Ala Glu Leu Asp Leu Gln Gly Phe Val Glu Ser Ser
        115             120             125

Ile Ser Glu Val Ser Ser Ile Ser Leu Glu Glu Ala Asn Tyr Asp Tyr
    130             135             140
```

```
Gln Val Leu Ser Gln Ser Ala Ala Gly Glu Ala Val Leu Ala Val Ala
145             150             155                     160

Ser Arg Lys Asp Glu Ile Glu Pro Leu Ile Asp Ala Phe Asn Ala Ala
                165             170                     175

Gly Met Lys Leu Ser Ala Leu Asp Val Asp Ile Phe Gly Gln Tyr Asn
                180             185                     190

Ala Tyr Ala Leu Trp Ile Asn His Phe Ala Pro Glu Leu Ala Ala Glu
            195             200             205

Lys Val Ala Ile Phe Gly Val Tyr Ala Ala Gln Thr Tyr Ala Leu Val
    210             215             220

Ile Gln Asp Gly Lys Ile Leu Tyr Lys Gln Glu Thr Ser Val Ser Glu
225             230             235             240

Glu Gln Leu Asn Gln Leu Ile Gln Arg Thr Tyr Gln Val Thr Glu Glu
            245             250             255

Lys Ala Glu Glu Ile Ile Asn Ser Pro Gln Lys Pro Ser Asp Tyr Gln
            260             265             270

Glu Ser Val Ala Asn Tyr Phe Asn Gln Gln Ile Thr Gln Glu Ile Gln
            275             280             285

Arg Val Leu Gln Phe Tyr Tyr Thr Thr Gln Thr Ala Asp Asp Met Thr
    290             295             300

Asp Ile Lys His Ile Leu Leu Thr Gly Glu Ala Ala Arg Gln Glu Gly
305             310             315             320

Ile Ala Gln Thr Val Ala Ser Gln Thr Asn Ala Asp Val Gln Cys Val
            325             330             335

His Pro Ala Arg Tyr Phe Ala Asp Asn Leu Lys Thr Asp Lys Gln Gln
            340             345             350

Phe Glu Leu Asp Ala Pro Thr Leu Thr Arg Ala Phe Gly Leu Ala Val
            355             360             365

Arg Gly Leu
    370
```